(19) Europäisches Patentamt
European Patent Office
Office européen des brevets

(11) **EP 4 585 587 A1**

(12) **EUROPEAN PATENT APPLICATION**
published in accordance with Art. 153(4) EPC

(43) Date of publication:
**16.07.2025 Bulletin 2025/29**

(21) Application number: **23862513.1**

(22) Date of filing: **08.09.2023**

(51) International Patent Classification (IPC):
***C07D 213/64*** *(2006.01)* ***C07D 401/02*** *(2006.01)*
***C07D 205/02*** *(2006.01)* ***A61K 31/397*** *(2006.01)*
***A61K 31/4412*** *(2006.01)* ***A61P 29/00*** *(2006.01)*

(52) Cooperative Patent Classification (CPC):
**A61K 31/397; A61K 31/4412; A61P 29/00; C07D 205/02; C07D 213/64; C07D 401/02**

(86) International application number:
**PCT/CN2023/117732**

(87) International publication number:
**WO 2024/051819 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.09.2022 CN 202211104699**
**11.11.2022 CN 202211391154**
**05.01.2023 CN 202310000075**
**23.02.2023 CN 202310157111**
**16.05.2023 CN 202310546078**
**21.07.2023 CN 202310900169**

(71) Applicant: **Xizang Haisco Pharmaceutical Co., Ltd.**
**Lhoka, Tibet 856099 (CN)**

(72) Inventors:
- **ZHANG, Chen**
**Tibet 856000 (CN)**
- **HE, Ping**
**Tibet 856000 (CN)**
- **WANG, Le**
**Tibet 856000 (CN)**
- **XUAN, Zhaoli**
**Tibet 856000 (CN)**
- **WEI, Qi**
**Tibet 856000 (CN)**
- **TANG, Pingming**
**Tibet 856000 (CN)**
- **HE, Haiqing**
**Tibet 856000 (CN)**
- **ZHONG, Yajun**
**Tibet 856000 (CN)**
- **YU, Yan**
**Tibet 856000 (CN)**
- **LI, Yao**
**Tibet 856000 (CN)**
- **NI, Jia**
**Tibet 856000 (CN)**
- **YAN, Pangke**
**Tibet 856000 (CN)**

(74) Representative: **IP TRUST SERVICES**
**Europole - Le Grenat**
**3, avenue Doyen Louis Weil**
**38000 Grenoble (FR)**

# (54) PROPIONIC ACID DERIVATIVE AND USE THEREOF IN MEDICINE

(57) A compound of general formula (I) or a stereoisomer, mesomer/racemate, deuterated product, solvate, prodrug, metabolite, pharmaceutically acceptable salt, or co-crystal thereof, an intermediate thereof, a preparation method therefor, and a use in the preparation of a drug for treating diseases related to integrin α4β7 activity or expression.

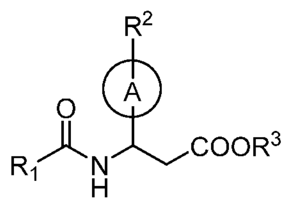

(I)

EP 4 585 587 A1

## Description

### Technical Field

The present invention relates to a compound of general formula (I) or a stereoisomer, mesomer/racemate, deuterated product, solvate, prodrug, metabolite, pharmaceutically acceptable salt, or co-crystal thereof, an intermediate thereof, a preparation method therefor, and a use in the preparation of a drug for treating diseases related to integrin $\alpha 4\beta 7$ activity or expression.

### Background Art

The integrin family comprises dimers formed by two subunits: $\alpha$ (120-185 KD) and $\beta$ (90-110 KD). Mammalian integrins involve 18 $\alpha$-subunits and 8 $\beta$-subunits. More than 20 integrins can be formed from their various combinations. $\alpha 4\beta 7$ is a member of the integrin family. It is currently established that inflammatory bowel diseases related to $\alpha 4\beta 7$ include Crohn's disease, ulcerative colitis, etc. The main ligand of $\alpha 4\beta 7$ is mucosal addressin cell adhesion molecule-1 (MADCAM-1). MAdCAM-1 is a transmembrane glycoprotein molecule selectively expressed in the high endothelial veins of mucosal lymphoid organs and the veins of the intestinal lamina propria. In inflammation, a variety of cytokines can promote high expression of MAdCAM-1 by endothelial cells, and then MAdCAM-1 mediates the migration of $\alpha 4\beta 7$-expressing leukocytes to the site of inflammation. Targeting either integrin $\alpha 4\beta 7$ or MAdCAM-1 can reduce intestinal inflammation. There are currently no specific small-molecule compounds on the market that target $\alpha 4\beta 7$-mediated inflammation. Natalizumab in clinical use is a humanized monoclonal antibody that targets the $\alpha 4$ subunit and is used primarily to treat multiple sclerosis and Crohn's disease. However, the side effect of PML (progressive multifocal leukoencephalopathy) has occurred during its clinical use. Therefore, there is a need to develop a small-molecule compound, which can inhibit integrin $\alpha 4\beta 7$ protein, for use in the treatment of diseases related to an integrin $\alpha 4\beta 7$ activity or expression level.

### Summary of the Invention

The present invention develops an integrin $\alpha 4\beta 7$ inhibitor with novel structure, good drug efficacy, better selectivity for $\alpha 4\beta 1/\alpha 4\beta 7$, better stability of liver microsomes, greater safety, and no significant inhibitory activity against hERG or CYP. These compounds have good pharmacokinetic properties and good safety and can be used to treat diseases related to integrin $\alpha 4\beta 7$, such as inflammatory bowel diseases.

An object of the present invention is to provide a compound capable of inhibiting integrin $\alpha 4\beta 7$, or a stereoisomer, mesomer/racemate, deuterated product, solvate, prodrug, metabolite, pharmaceutically acceptable salt, or co-crystal thereof, an intermediate thereof, a preparation method therefor, and a use in the preparation of a drug for the treatment of diseases related to integrin $\alpha 4\beta 7$ activity or expression.

The present invention provides a compound of general formula (I) or a stereoisomer, mesomer/racemate, deuterated product, solvate, prodrug, metabolite, pharmaceutically acceptable salt, or co-crystal thereof, wherein

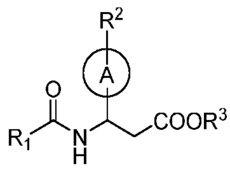
(I)

in some embodiments, $R^1$ is selected from $-CHR^{1a}R^{1b}$ or $-NR^{1a}R^{1b}$;
in some embodiments, $R^1$ is selected from

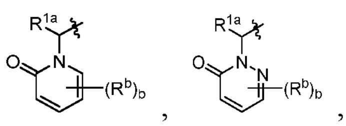

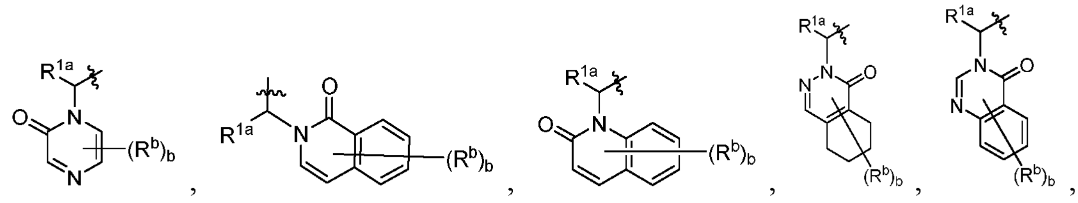

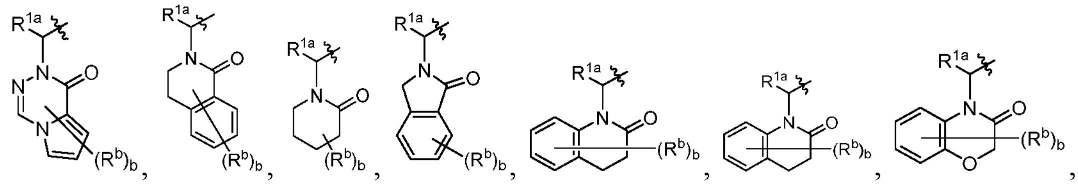

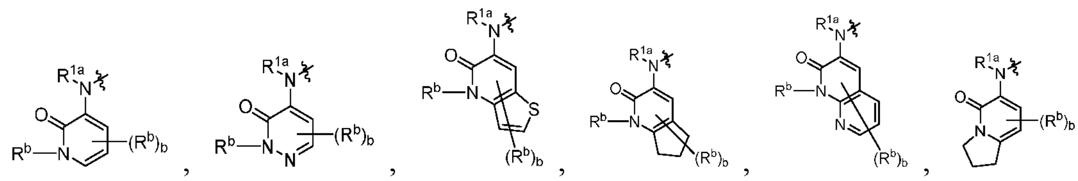

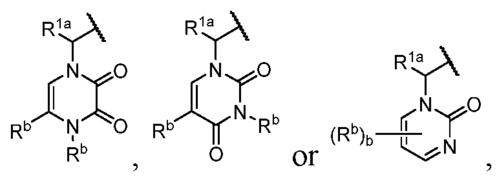

or

and b is selected from 0, 1, 2 or 3;
in some embodiments, $R^1$ is selected from

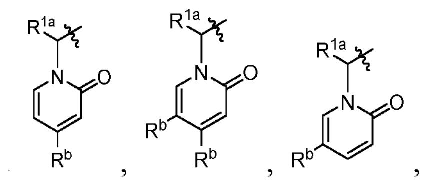

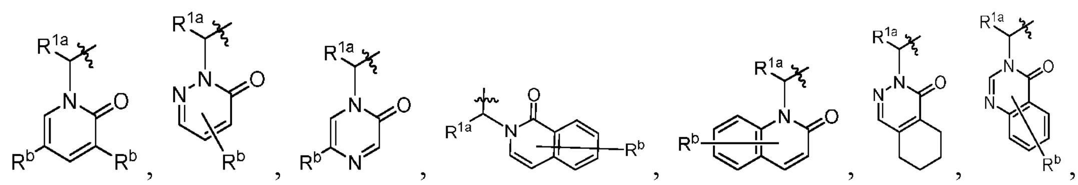

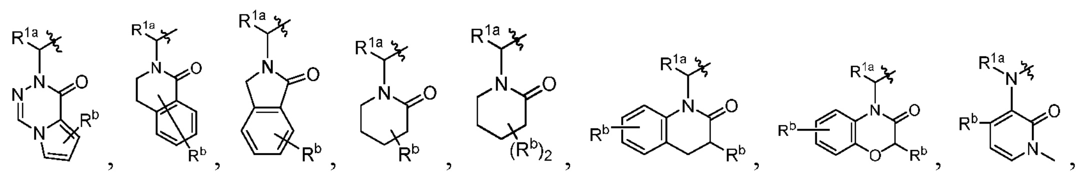

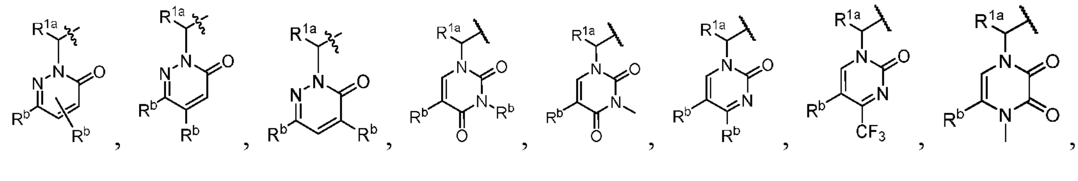

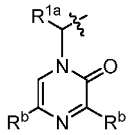, 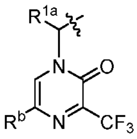, 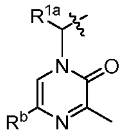, 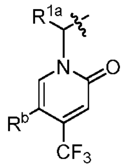, 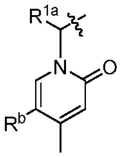, 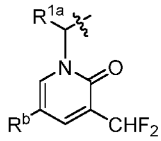 or 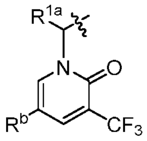;

in some embodiments, $R^1$ is selected from

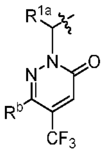, 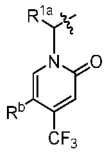, 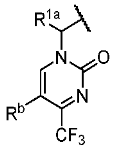, 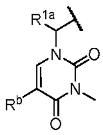

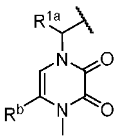, 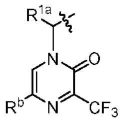, 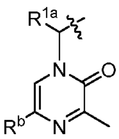, 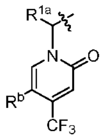, 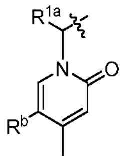, 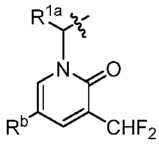 or 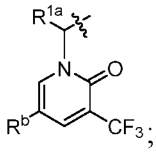;

in some embodiments, $R^1$ is selected from

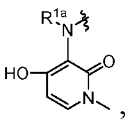, 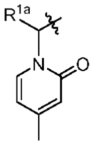, 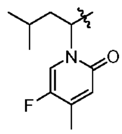, 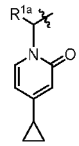,

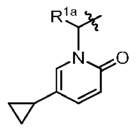, 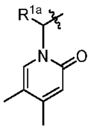, 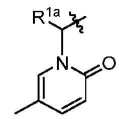, 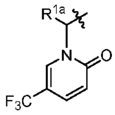, 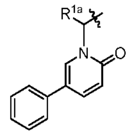, 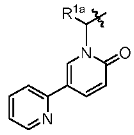, 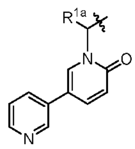,

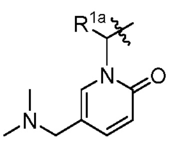, 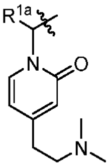, 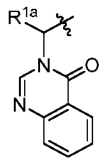, 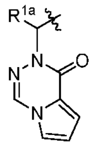, 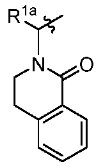, 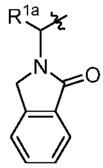, 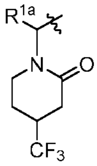, 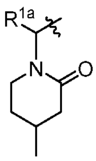,

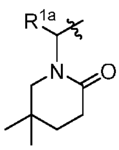, 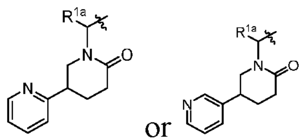 or;

in some embodiments, $R^1$ is selected from

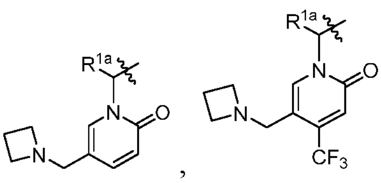,

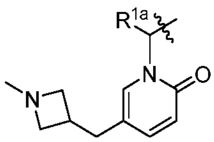 , 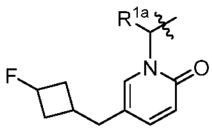 , 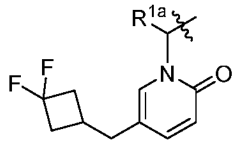 , 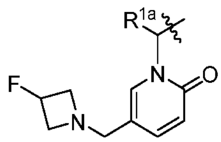 ,

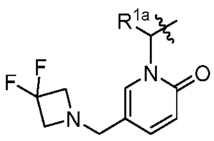 , 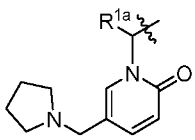 , 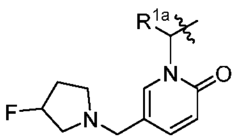 , 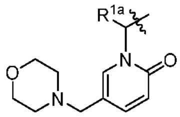 , 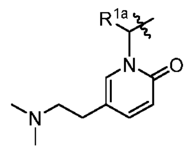 ,

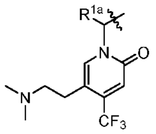 , 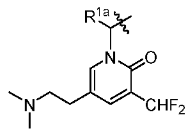 , 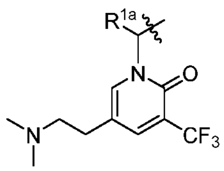 , 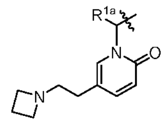 , 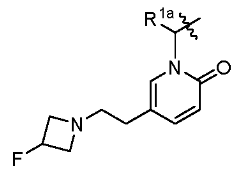 ,

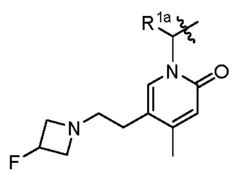 , 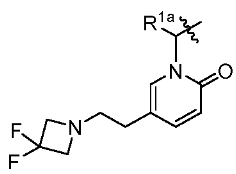 , 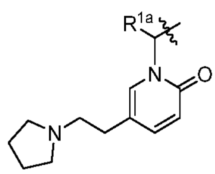 , 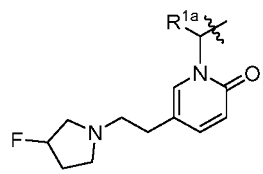 ,

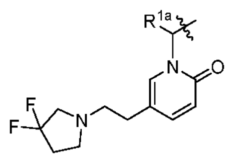 , 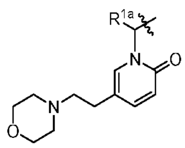 , 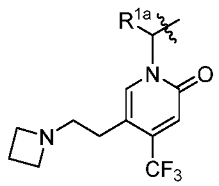 , 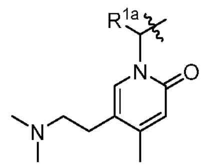 ,

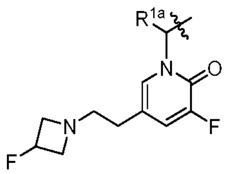 , 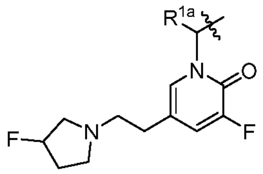 or 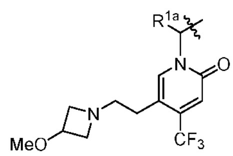 ;

; in some embodiments, $R^1$ is selected from

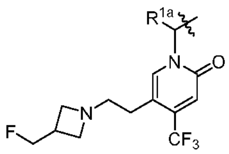 or 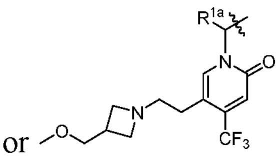

; in some embodiments, $R^1$ is selected from

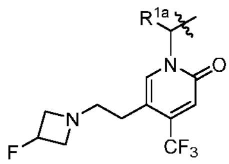 or 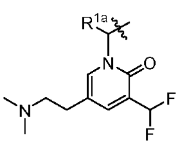 ;

in some embodiments, $R^1$ is selected from

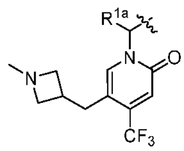, 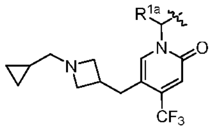, 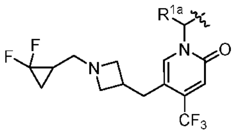, 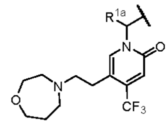,

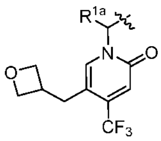, 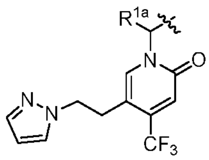, 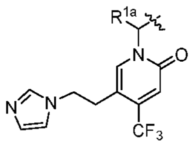, 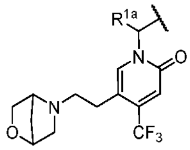, 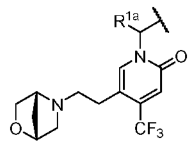,

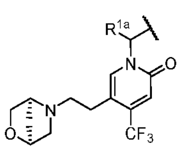, 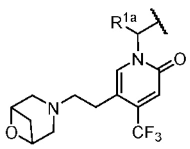, 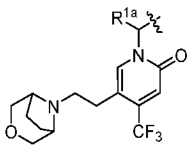, 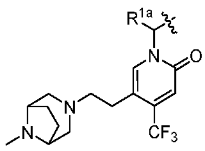, 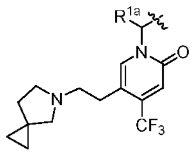,

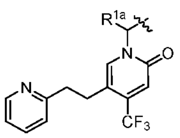, 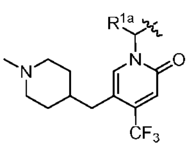, 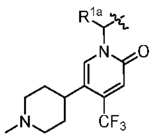, 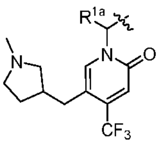, 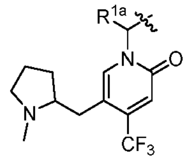,

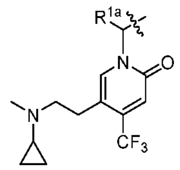, 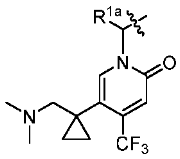, 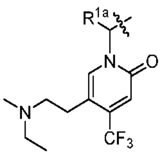, 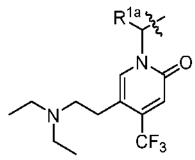, 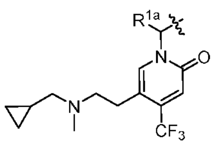 or

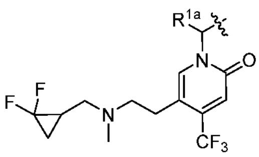;

in some embodiments, $R^1$ is selected from

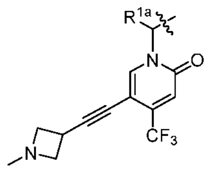,

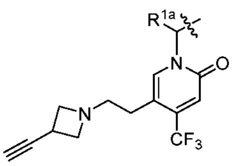, 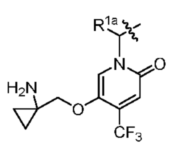, 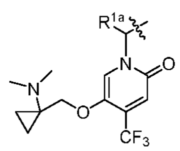, 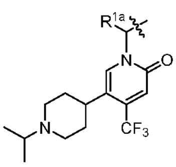, 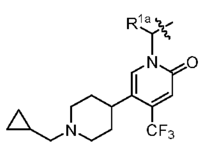,

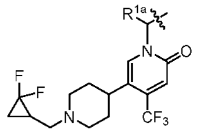, 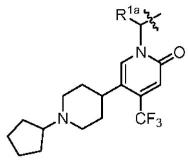 or 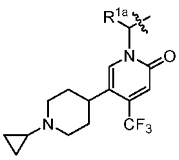;

in some embodiments, $R^1$ is selected from

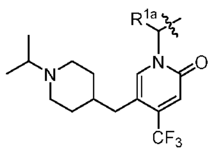, 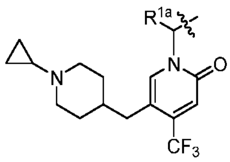, 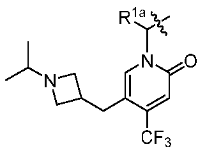, 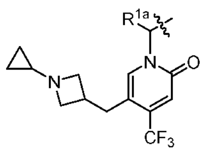,

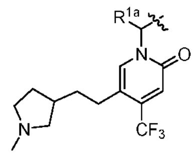 or 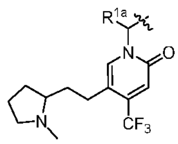;

in some embodiments, $R^1$ is selected from

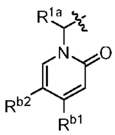, 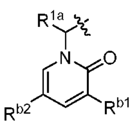,

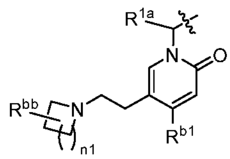, 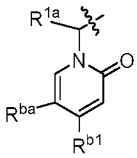, 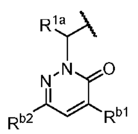, 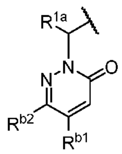, 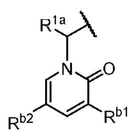, 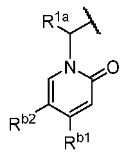,

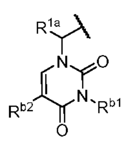 or 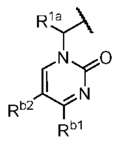;

in some embodiments, $R^1$ is selected from

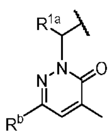,

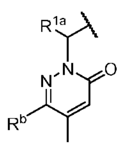, 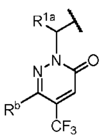, 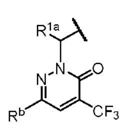, 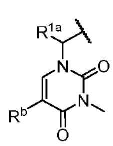, 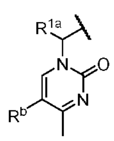, 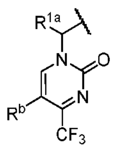, 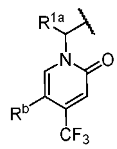, 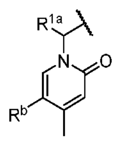,

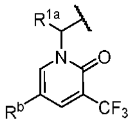, 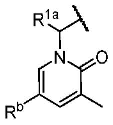 or 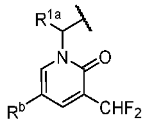;

in some embodiments, $R^{b1}$ is selected from H, F, $CH_2F$, $CHF_2$, $CF_3$, methyl or $-CH_2CH_2N(CH_3)_2$;
in some embodiments, $R^{b2}$ and $R^b$ are each independently selected from $R^{ba}$,

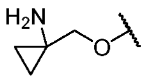, 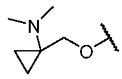, 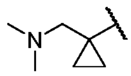,

or one of the following optionally substituted groups: ethynyl, cyclopropyl, cyclobutyl, cyclopentyl, cyclohexyl, azetidinyl, azolidinyl, azinanyl, oxetanyl, oxolanyl, oxanyl, morpholinyl, phenyl, pyridyl, - $CH_2NH(CH_2CH_3)$, $-CH_2N(CH_2CH_3)_2$, $-CH_2CH_2NH(CH_3)$, $-CH_2CH_2N(CH_3)_2$, - $CH_2CH_2NH(CH_2CH_3)$, $-CH_2CH_2N(CH_2CH_3)_2$, $-CH_2CH_2N(CH_3)(CH_2CH_3)$, $-CH_2$-cyclopropyl, $-CH_2$-cyclobutyl, $-CH_2$-cyclopentyl, $-CH_2$-cyclohexyl, $-CH_2$-azetidinyl, $-CH_2$-azolidinyl, $-CH_2$-azinanyl, $-CH_2$-oxetanyl, $-CH_2$-oxolanyl, $-CH_2$-oxanyl, $-CH_2$-morpholinyl, $-CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, - $CH_2CH_2$-azinanyl, $-CH_2CH_2$-oxetanyl, $-CH_2CH_2$-oxolanyl, $-CH_2CH_2$-oxanyl, - $CH_2CH_2$-morpholinyl, $-CH_2$-phenyl, $-CH_2$-pyridyl, $-CH_2CH_2$-phenyl, $-CH_2CH_2$-pyridyl or

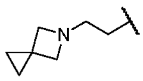,

which, when substituted, is substituted with 1 to 4 substituents selected from deuterium, F, Cl, Br, OH, =O, cyano, $CONH_2$, $CONHCH_3$, $CON(CH_3)_2$, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;
in some embodiments, $R^{b2}$ and $R^b$ are each independently selected from $R^{ba}$,

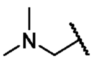, 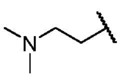, 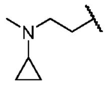, 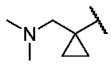, 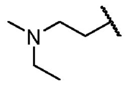, 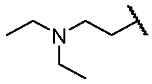, 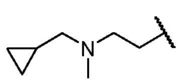,

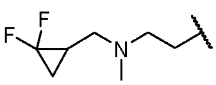, 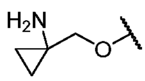, 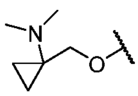, 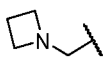, 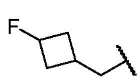, 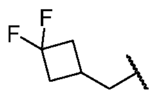,

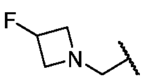, 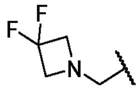, 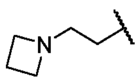, 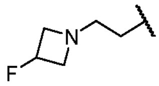, 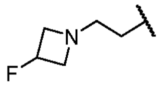, 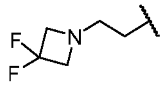,

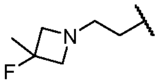, 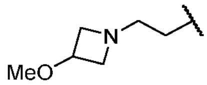, 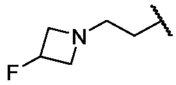, 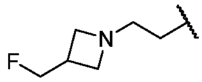, 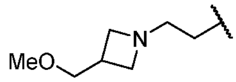,

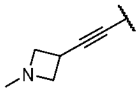, 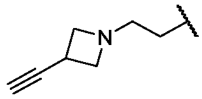, 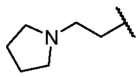, 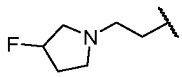, 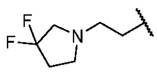, 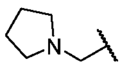,

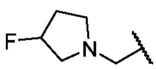, 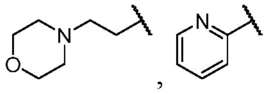, , 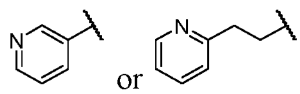 or ;

in some embodiments, $R^{ba}$ is selected from

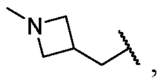, 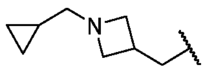,

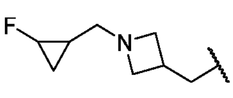, 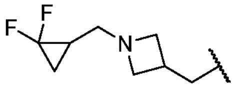, 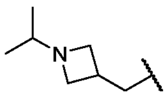, 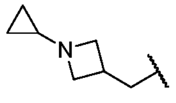, 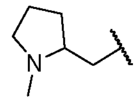,

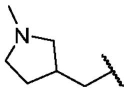, 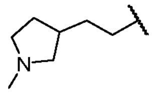, 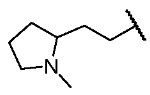, 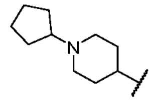, 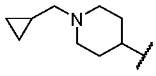, 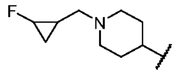,

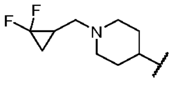, 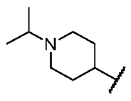, 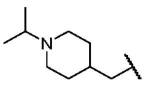, 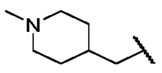, 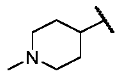, 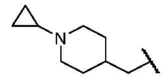,

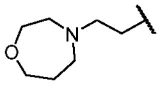, 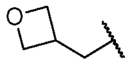, 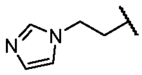, 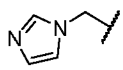, 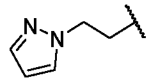, 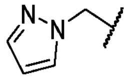, 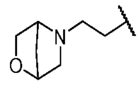,

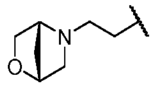, 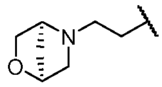, 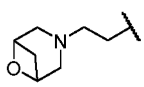, 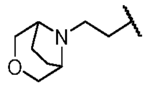, 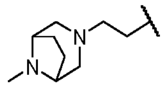 or 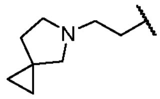;

in some embodiments, $R^{b1}$ is selected from H, $C_{1-4}$ alkyl or $C_{3-6}$ cycloalkyl, wherein the alkyl or cycloalkyl is optionally substituted with 0 to 4 substituents selected from deuterium, halogen, OH, CN, $NHC_{1-4}$ alkyl, $N(C_{1-4}$ alkyl$)_2$, $C_{1-4}$ alkyl or $C_{1-4}$ alkoxy;

in some embodiments, $R^{b2}$ is selected from H, $R^{ba}$, or one of the following substituted or unsubstituted groups: $C_{1-4}$ alkyl, $C_{2-4}$ alkynyl, $C_{1-4}$ alkoxy, $-CH_2CH_2-NHC_{1-4}$ alkyl, $-CH_2CH_2-N(C_{1-4}$ alkyl$)_2$, $-CH_2CH_2-C_{3-6}$ cycloalkyl, $-CH_2CH_2$-3- to 7-membered heterocycloalkyl, wherein the $CH_2$, alkyl, cycloalkyl or heterocycloalkyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heteroaryl or heterocycloalkyl contains 1 to 4 heteroatoms selected from O, S or N;

in some embodiments, $R^{1a}$ is selected from $C_{1-6}$ alkyl, wherein the alkyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ cycloalkyl;

in some embodiments, $R^{1a}$ is selected from methyl, ethyl, propyl, butyl, isobutyl, sec-butyl, tert-butyl, $-CH_2$-cyclopropyl or $-CH_2$-cyclobutyl;

in some embodiments, $R^{1a}$ is selected from

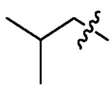, 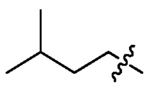, or 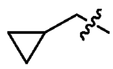;

in some embodiments, $R^{1b}$ is selected from $C_{4-10}$ carbocyclic ring or 5- to 10-membered heterocyclic ring, wherein the carbocyclic ring or heterocyclic ring is optionally substituted with 0 to 4 $R^b$, and the heterocyclic ring contains 1 to 4 heteroatoms selected from O, S or N;

in some embodiments, each $R^b$ is independently selected from deuterium, halogen, OH, =O, cyano, COOH, $NH_2$, $-C_{0-4}$ alkyl-$NHC_{1-6}$ alkyl, $-C_{0-4}$ alkyl-$N(C_{1-6}$ alkyl$)_2$, $C_{1-6}$ alkyl, $C_{2-6}$ alkynyl, $C_{1-6}$ alkoxy, $-C_{0-4}$ alkyl-$C_{3-10}$ carbocyclic ring, $-C_{0-4}$ alkyl-3- to 10-membered heterocyclic ring or $R^{ba}$, wherein the alkyl, alkynyl, alkoxy, carbocyclic ring or heterocyclic ring is optionally substituted with 0 to 4 substituents selected from deuterium, halogen, OH, =O, cyano, COOH, $CONH_2$, $CONHC_{1-6}$ alkyl, $CON(C_{1-6}$ alkyl$)_2$, $NH_2$, $NHC_{1-6}$ alkyl, $N(C_{1-6}$ alkyl$)_2$, $N(C_{1-6}$ alkyl$)(C_{3-6}$ cycloalkyl), $NH(C_{3-6}$ cycloalkyl), $C_{1-6}$ alkyl, $C_{2-6}$ alkynyl, $C_{1-6}$ alkoxy, halogen-substituted $C_{1-6}$ alkyl, halogen-substituted $C_{1-6}$

alkoxy, $C_{1-6}$ alkoxyalkyl or $R^k$, and the heterocyclic ring contains 1 to 4 heteroatoms selected from O, S or N;

in some embodiments, each $R^b$ is independently selected from deuterium, halogen, OH, =O, cyano, COOH, $NH_2$, $-C_{0-4}$ alkyl-$NHC_{1-4}$ alkyl, $-C_{0-4}$ alkyl-$N(C_{1-4}$ alkyl$)_2$, $C_{1-4}$ alkyl, $C_{1-4}$ alkoxy, $-C_{0-4}$ alkyl-$C_{3-6}$ carbocyclic ring, $-C_{0-4}$ alkyl-3- to 7-membered heterocyclic ring or $R^{ba}$, wherein the alkyl, alkoxy, carbocyclic ring or heterocyclic ring is optionally substituted with 0 to 4 substituents selected from deuterium, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heterocyclic ring contains 1 to 4 heteroatoms selected from O, S or N;

in some embodiments, $R^b$ is selected from $R^{ba}$;

In some embodiments, each $R^b$ is independently selected from deuterium, halogen, OH, =O, cyano, $C_{1-4}$ alkyl, $C_{2-4}$ alkynyl, $C_{1-4}$ alkoxy, phenyl, 5- to 6-membered heteroaryl, $-CH_2NHC_{1-4}$ alkyl, $-CH_2N(C_{1-4}$ alkyl$)_2$, $-CH_2CH_2-NHC_{1-4}$ alkyl, $-CH_2CH_2-N(C_{1-4}$ alkyl$)_2$, $C_{3-6}$ cycloalkyl, 3- to 7-membered heterocycloalkyl, $-CH_2-C_{3-6}$ cycloalkyl, $-CH_2$-3- to 7-membered heterocycloalkyl, $-CH_2CH_2-C_{3-6}$ cycloalkyl, $-CH_2CH_2$-3- to 7-membered heterocycloalkyl or $R^{ba}$, wherein the $CH_2$, alkyl, alkynyl, cycloalkyl or heterocycloalkyl is optionally substituted with 0 to 4 substituents selected from deuterium, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heteroaryl or heterocycloalkyl contains 1 to 4 heteroatoms selected from O, S or N;

in some embodiments, each $R^b$ is independently selected from deuterium, F, Cl, Br, I, OH, =O, cyano or $R^{ba}$, or each $R^b$ is independently selected from one of the following substituted or unsubstituted groups: methyl, ethyl, ethynyl, methoxy, ethoxy, cyclopropyl, cyclobutyl, cyclopentyl, cyclohexyl, azetidinyl, azolidinyl, azinanyl, oxetanyl, oxolanyl, oxanyl, morpholinyl, phenyl, pyridine, $-CH_2NH(CH_2CH_3)$, $-CH_2N(CH_2CH_3)_2$, $-CH_2CH_2NH(CH_3)$, $-CH_2CH_2N(CH_3)_2$, $-CH_2CH_2NH(CH_2CH_3)$, $-CH_2CH_2N(CH_2CH_3)_2$, $-CH_2CH_2N(CH_3)(CH_2CH_3)$, $-CH_2$-cyclopropyl, $-CH_2$-cyclobutyl, $-CH_2$-cyclopentyl, $-CH_2$-cyclohexyl, $-CH_2$-azetidinyl, $-CH_2$-azolidinyl, $-CH_2$-azinanyl, $-CH_2$-oxetanyl, $-CH_2$-oxolanyl, $-CH_2$-oxanyl, $-CH_2$-morpholinyl, $-CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, $-CH_2CH_2$-oxetanyl, $-CH_2CH_2$-oxolanyl, $-CH_2CH_2$-oxanyl or $-CH_2CH_2$-morpholinyl, which, when substituted, is substituted with 0 to 4 substituents selected from deuterium, halogen, OH, =O, cyano, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$;

in some embodiments, each $R^b$ is independently selected from deuterium, F, Cl, Br, OH, cyano or $R^{ba}$, or each $R^b$ is independently selected from one of the following substituted or unsubstituted groups: methyl, ethyl, ethynyl, methoxy, ethoxy, cyclopropyl, cyclobutyl, cyclopentyl, cyclohexyl, azetidinyl, azolidinyl, azinanyl, oxetanyl, oxolanyl, oxanyl, morpholinyl, phenyl, pyridine, $-CH_2NH(CH_2CH_3)$, $-CH_2N(CH_2CH_3)_2$, $-CH_2CH_2NH(CH_3)$, $-CH_2CH_2N(CH_3)_2$, $-CH_2CH_2NH(CH_2CH_3)$, $-CH_2CH_2N(CH_2CH_3)_2$, $-CH_2CH_2N(CH_3)(CH_2CH_3)$, $-CH_2$-cyclopropyl, $-CH_2$-cyclobutyl, $-CH_2$-cyclopentyl, $-CH_2$-cyclohexyl, $-CH_2$-azetidinyl, $-CH_2$-azolidinyl, $-CH_2$-azinanyl, $-CH_2$-oxetanyl, $-CH_2$-oxolanyl, $-CH_2$-oxanyl, $-CH_2$-morpholinyl, $-CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, $-CH_2CH_2$-oxetanyl, $-CH_2CH_2$-oxolanyl, $-CH_2CH_2$-oxanyl or $-CH_2CH_2$-morpholinyl, which, when substituted, is substituted with 0 to 4 substituents selected from deuterium, F, Cl, Br, OH, =O, cyano, $CONH_2$, $CONHCH_3$, $CON(CH_3)_2$, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;

in some embodiments, $R^b$ is selected from $R^{b2}$;

in some embodiments, $R^{ba}$ is selected from $-C_{0-4}$ alkyl-7- to 12-membered heterocyclic ring, $-C_{0-4}$ alkyl-4- to 6-membered heterocyclic ring connected via a carbon atom,

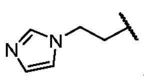, 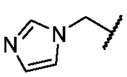, 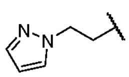, 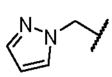 or 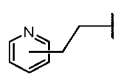,

and the $R^{ba}$ is optionally substituted with 1 to 4 substituents selected from H, halogen, OH, =O, cyano, COOH, $CONH_2$, $CONHC_{1-6}$ alkyl, $CON(C_{1-6}$ alkyl$)_2$, $NH_2$, $NHC_{1-6}$ alkyl, $N(C_{1-6}$ alkyl$)_2$, $C_{1-6}$ alkyl, $C_{2-6}$ alkynyl, $C_{1-6}$ alkoxy, halogen-substituted $C_{1-6}$ alkyl, halogen-substituted $C_{1-6}$ alkoxy, $C_{1-6}$ alkoxyalkyl or $R^k$, wherein the heterocyclic ring contains 1 to 4 heteroatoms selected from O, S or N;

in some embodiments, $R^{ba}$ is selected from $-C_{0-2}$ alkyl-7- to 8-membered monocyclic heterocycloalkyl, $-C_{0-2}$ alkyl-7- to 11-membered spiro heterocycloalkyl, $-C_{0-2}$ alkyl-7- to 11-membered bridged heterocycloalkyl, $-C_{0-2}$ alkyl-4- to 6-membered monocyclic heterocycloalkyl connected via a carbon atom,

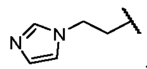,

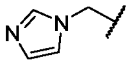, 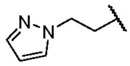, 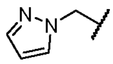 or 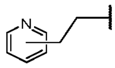,

and the $R^{ba}$ is optionally substituted with 1 to 4 substituents selected from H, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, wherein the heterocycloalkyl contains 1 to 4 heteroatoms selected from O, S or N;

in some embodiments, $R^{ba}$ is selected from 7- to 8-membered monocyclic heterocycloalkyl, 7- to 11-membered spiro heterocycloalkyl, 7- to 11-membered bridged heterocycloalkyl, 4- to 6-membered monocyclic heterocycloalkyl connected via a carbon atom, $-CH_2$-7- to 8-membered monocyclic heterocycloalkyl, $-CH_2$-7- to 11-membered spiro heterocycloalkyl, $-CH_2$-7- to 11-membered bridged heterocycloalkyl, $-CH_2$-4- to 6-membered monocyclic heterocycloalkyl connected via a carbon atom, $-CH_2CH_2$-7- to 8-membered monocyclic heterocycloalkyl, - $CH_2CH_2$-7- to 11-membered spiro heterocycloalkyl, $-CH_2CH_2$-7- to 11-membered bridged heterocycloalkyl, $-CH_2CH_2$-4- to 6-membered monocyclic heterocycloalkyl connected via a carbon atom,

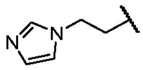, 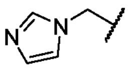, 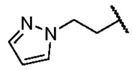,

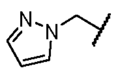 or 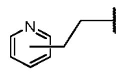,

and the $R^{ba}$ is optionally substituted with 1 to 4 substituents selected from H, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, wherein the heterocycloalkyl contains 1 to 4 heteroatoms selected from O, S or N;

in some embodiments, $R^{ba}$ is selected from one of the following optionally substituted groups:

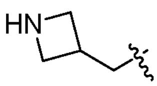, 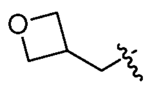, 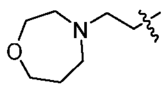, 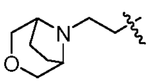, 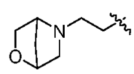,

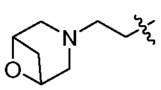, 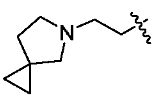, 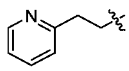, 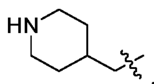, 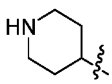, 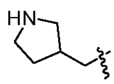, 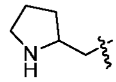,

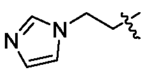, 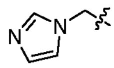, 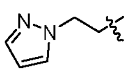, 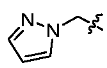, 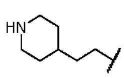, 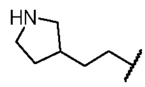 or 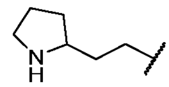,

which, when substituted, is optionally substituted with 1 to 4 substituents selected from deuterium, halogen, OH, =O, cyano, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$;

in some embodiments, $R^{ba}$ is selected from one of the following optionally substituted groups:

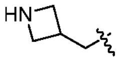, 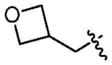, 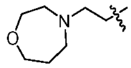, 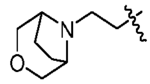, 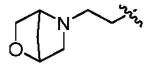,

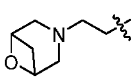, 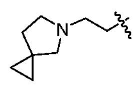, 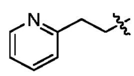, 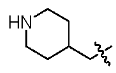, 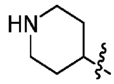, 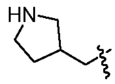, 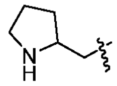,

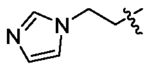, 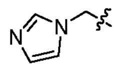, 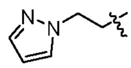, 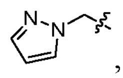, 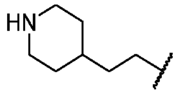, 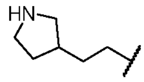 or 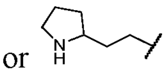,

which, when substituted, is optionally substituted with 1 to 4 substituents selected from deuterium, F, Cl, Br, OH, =O, cyano, $CONH_2$, $CONHCH_3$, $CON(CH_3)_2$, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;

in some embodiments, $R^k$ is selected from -$C_{1-4}$ alkyl-$NH_2$, -$C_{1-4}$ alkyl-$NHC_{1-6}$ alkyl, -$C_{1-4}$ alkyl-N($C_{1-6}$ alkyl)$_2$, -$C_{0-4}$ alkyl-$C_{3-10}$ carbocyclic ring or -$C_{0-4}$ alkyl-3-to 10-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-6}$ alkyl, N($C_{1-6}$ alkyl)$_2$, $C_{1-6}$ alkyl, $C_{1-6}$ alkoxy, halogen-substituted $C_{1-6}$ alkyl, halogen-substituted $C_{1-6}$ alkoxy or $C_{1-6}$ alkoxyalkyl, and the heterocyclic ring contains 1 to 4 heteroatoms selected from O, S or N;

in some embodiments, $R^k$ is selected from -$C_{1-2}$ alkyl-$NH_2$, -$C_{1-2}$ alkyl-$NHC_{1-4}$ alkyl, -$C_{1-2}$ alkyl-N($C_{1-4}$ alkyl)$_2$, -$C_{0-2}$ alkyl-$C_{3-6}$ carbocyclic ring or -$C_{0-2}$ alkyl-3- to 6-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-4}$ alkyl, N($C_{1-4}$ alkyl)$_2$, $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl, and the heterocyclic ring contains 1 to 4 heteroatoms selected from O, S or N;

in some embodiments, $R^k$ is selected from -$C_{1-4}$ alkyl-$NH_2$, -$C_{1-4}$ alkyl-$NHC_{1-4}$ alkyl, -$C_{1-4}$ alkyl-N($C_{1-4}$ alkyl)$_2$, -$C_{0-4}$ alkyl-$C_{3-6}$ carbocyclic ring or -$C_{0-4}$ alkyl-3- to 6-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-4}$ alkyl, N($C_{1-4}$ alkyl)$_2$, $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl, and the heterocyclic ring contains 1 to 4 heteroatoms selected from O, S or N;

in some embodiments, $R^k$ is selected from -$CH_2N(CH_3)_2$, -$CH_2$-cyclopropyl, - $CH_2$-cyclobutyl, cyclopropyl, cyclobutyl, cyclopentyl, cyclohexyl, azetidinyl, azolidinyl, azinanyl or oxetanyl, wherein the cyclopropyl, cyclobutyl, cyclopentyl, cyclohexyl, azetidinyl, azolidinyl, azinanyl or oxetanyl is optionally substituted with 1 to 4 substituents selected from deuterium, halogen, OH, =O, cyano, $NH_2$, $NHC_{1-4}$ alkyl, N($C_{1-4}$ alkyl)$_2$, N($C_{1-4}$ alkyl)($C_{3-6}$ cycloalkyl), NH($C_{3-6}$ cycloalkyl), $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl;

in some embodiments, $R^k$ is selected from -$CH_2N(CH_3)_2$, -$CH_2$-cyclopropyl, - $CH_2$-cyclobutyl, cyclopropyl, cyclobutyl, cyclopentyl, cyclohexyl, azetidinyl, azolidinyl, azinanyl or oxetanyl, wherein the cyclopropyl, cyclobutyl, cyclopentyl, cyclohexyl, azetidinyl, azolidinyl, azinanyl or oxetanyl is optionally substituted with 1 to 4 substituents selected from deuterium, F, Cl, Br, OH, =O, cyano, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, methoxy, ethoxy, methoxymethyl, ethoxymethyl or methoxyethyl;

in some embodiments, $R^2$ is selected from $C_{1-6}$ alkyl, $C_{6-10}$ aromatic ring, 5- to 10-membered heteroaromatic ring, $C_{3-10}$ carbocyclic ring or 5- to 10-membered heterocyclic ring, and the $R^2$ is optionally substituted with 0 to 4 $R^{2a}$, wherein the heteroaromatic ring or heterocyclic ring contains 1 to 4 heteroatoms selected from O, S or N;

in some embodiments, $R^2$ is selected from $C_{1-4}$ alkyl, benzene ring, naphthalene ring, 5- to 6-membered heteroaromatic ring, 9- to 10-membered heteroaromatic ring, $C_{3-10}$ non-aromatic carbocyclic ring, 5- to 10-membered non-aromatic heterocyclic ring, benzo $C_{4-6}$ carbocyclyl or benzo 4- to 6-membered heterocyclyl, and the $R^2$ is optionally substituted with 0 to 4 $R^{2a}$, wherein the heteroaromatic ring or heterocyclic ring contains 1 to 4 heteroatoms selected from O, S or N;

in some embodiments, $R^2$ is selected from benzene ring, naphthalene ring, 5-to 6-membered heteroaromatic ring, 9-to 10-membered heteroaromatic ring, $C_{3-6}$ cycloalkyl, 3- to 7-membered heterocycloalkyl, benzo $C_{4-6}$ carbocyclyl or benzo 4-to 6-membered heterocyclyl, and the $R^2$ is optionally substituted with 0 to 4 $R^{2a}$, wherein the heteroaromatic ring or heterocycloalkyl contains 1 to 4 heteroatoms selected from O, S or N; in some embodiments, $R^2$ is selected from benzene ring, pyridyl, pyridonyl, pyrazinyl, pyrimidyl, thienyl, thiazolyl, furyl, oxazolyl, pyrrolyl, pyrazolyl, imidazolyl, cyclopropyl, cyclobutyl, cyclopentyl, cyclohexyl, azetidinyl, azolidinyl, azinanyl, oxetanyl, oxolanyl, oxanyl, morpholinyl,

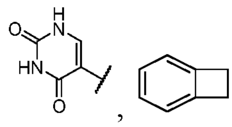 , ,

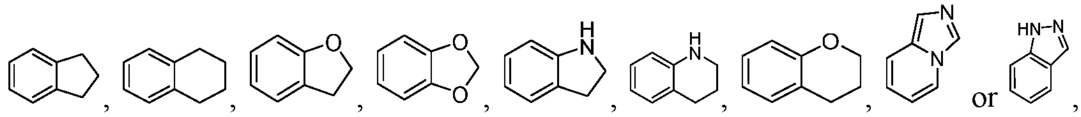 , , , , , , , , or ,

and the $R^2$ is optionally substituted with 0 to 4 $R^{2a}$; in some embodiments, $R^2$ is selected from

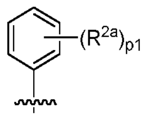 ,

wherein p1 is selected from 0, 1, 2, 3 or 4;
in some embodiments, $R^2$ is selected from

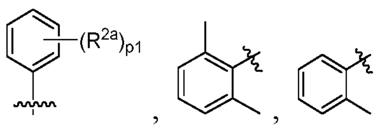 , , ,

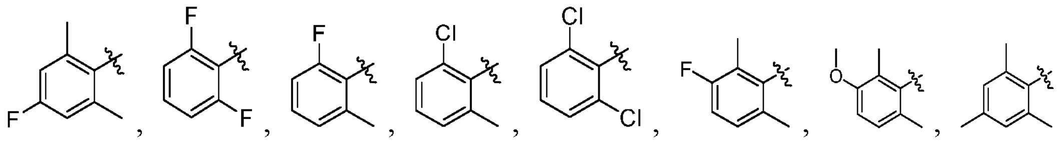 , , , , , , , ,

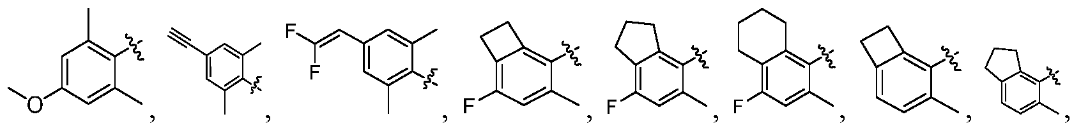 , , , , , , , , , ,

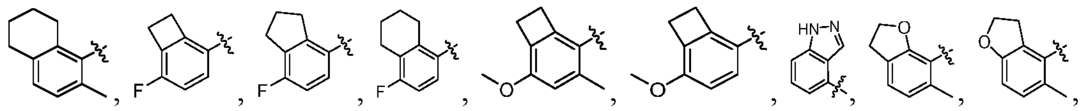 , , , , , , , , , ,

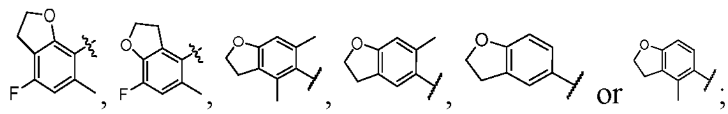 , , , , or ;

in some embodiments, $R^2$ is selected from phenyl, pyridyl, pyridonyl, azolidinyl, morpholinyl or

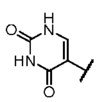 ,

and the $R^2$ is optionally substituted with 0 to 4 substituents selected from deuterium, F, Cl, Br, OH, $CF_3$, cyano, methyl, ethyl, methoxy, ethoxy, cyclopropyl or cyclobutyl;
in some embodiments, $R^2$ is selected from

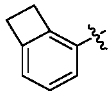, 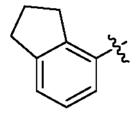, 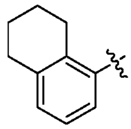,

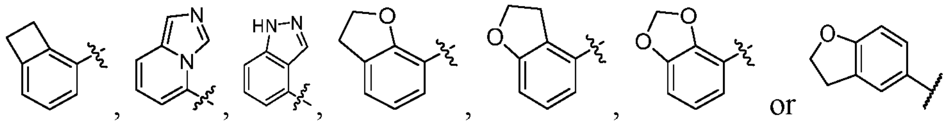

, , , , , , or ,

and the $R^2$ is optionally substituted with 1 to 4 substituents selected from deuterium, $CD_3$, - $OCD_3$, F, Cl, Br, OH, $CF_3$, cyano, methyl, ethyl, methoxy, ethoxy, cyclopropyl or cyclobutyl;

in some embodiments, $R^2$ is selected from

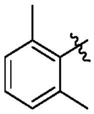, 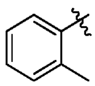, 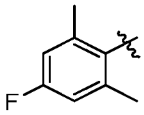, 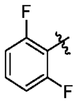,

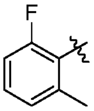, 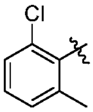, 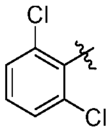, 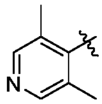, 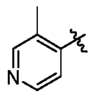, 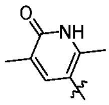, 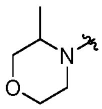, 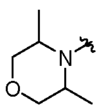,

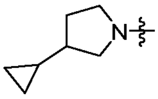, 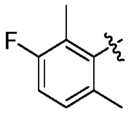, 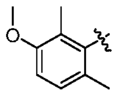, 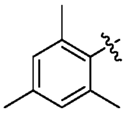, 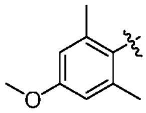, 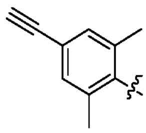,

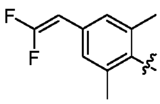, 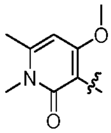, 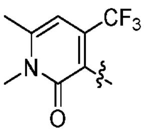 or 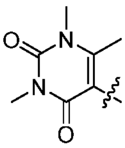;

in some embodiments, $R^2$ is selected from

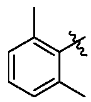;

in some embodiments, $R^2$ is selected from

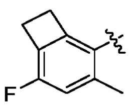,

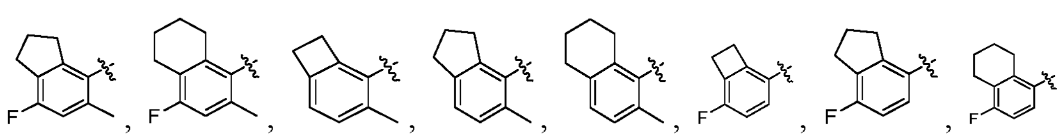

, , , , , , , ,

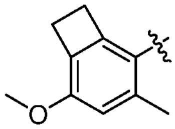, 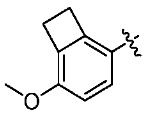, 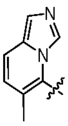, 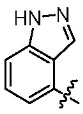, 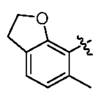, 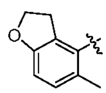, 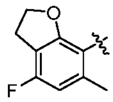 or 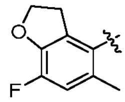;

in some embodiments, $R^2$ is selected from

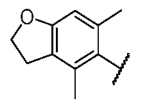, 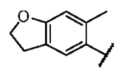, 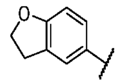,

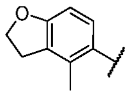 or 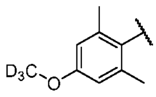;

in some embodiments, each $R^{2a}$ is independently selected from deuterium, halogen, OH, cyano, =O, $C_{1-6}$ alkyl, $C_{2-6}$ alkenyl, $C_{2-6}$ alkynyl, $C_{1-6}$ alkoxy, $C_{3-6}$ cycloalkyl or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl or $C_{1-6}$ alkoxy, and the heterocyclyl contains 1 to 3 heteroatoms selected from N, O or S;

in some embodiments, each $R^{2a}$ is independently selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl or $C_{1-4}$ alkoxy, and the heterocyclyl contains 1 to 3 heteroatoms selected from N, O or S;

in some embodiments, $R^{2a}$ is selected from deuterium, halogen, OH, CN, $C_{1-4}$ alkyl, $C_{2-4}$ alkenyl, $C_{2-4}$ alkynyl, $C_{1-4}$ alkoxy, $C_{3-6}$ cycloalkyl or 4- to 6-membered heterocycloalkyl, wherein the alkyl, alkenyl, alkynyl, alkoxy, cycloalkyl or heterocycloalkyl is optionally substituted with 0 to 4 substituents selected from deuterium, halogen, OH, CN, $C_{1-4}$ alkyl, or $C_{1-4}$ alkoxy, and the heterocycloalkyl contains 1 to 4 heteroatoms selected from O, S or N;

in some embodiments, each $R^{2a}$ is independently selected from deuterium, halogen, OH, cyano, =O, $C_{1-4}$ alkyl, $C_{2-4}$ alkenyl, $C_{2-4}$ alkynyl, $C_{1-4}$ alkoxy or $C_{3-6}$ cycloalkyl, wherein the alkyl, alkenyl, alkynyl, alkoxy or cycloalkyl is optionally substituted with 0 to 4 substituents selected from deuterium, halogen, OH, =O, cyano, $C_{1-4}$ alkyl or $C_{1-4}$ alkoxy;

in some embodiments, each $R^{2a}$ is independently selected from deuterium, F, Cl, Br, I, OH, =O, cyano, methyl, ethyl, ethenyl, ethynyl, methoxy, ethoxy, cyclopropyl or cyclobutyl, wherein the methyl, ethyl, ethenyl, ethynyl, methoxy, ethoxy, cyclopropyl or cyclobutyl is optionally substituted with 0 to 4 substituents selected from deuterium, halogen, OH, =O, cyano, $C_{1-4}$ alkyl or $C_{1-4}$ alkoxy;

in some embodiments, $R^{2a}$ is selected from deuterium, F, Cl, Br, I, OH, cyano, methyl, ethyl, methoxy or ethoxy;

in some embodiments, $R^{2a}$ and $R^{2a}$ are directly linked to form $C_{4-7}$ carbocyclic ring or 4- to 7-membered heterocyclic ring, wherein the carbocyclic ring or heterocyclic ring is optionally substituted with 1 to 4 substituents selected from deuterium, halogen, OH, cyano, =O, $C_{1-6}$ alkyl, $C_{2-6}$ alkenyl, $C_{2-6}$ alkynyl, $C_{1-6}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;

in some embodiments, $R^{2a}$ and $R^{2a}$ are directly linked to form $C_{4-7}$ carbocyclic ring or 4- to 7-membered heterocyclic ring, wherein the carbocyclic ring or heterocyclic ring is optionally substituted with 1 to 4 substituents selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;

in some embodiments, $R^{2a}$ and $R^{2a}$ are directly linked to form 4-membered carbocyclic ring, 5-membered carbocyclic ring, 6-membered carbocyclic ring, 4-membered heterocyclic ring, 5-membered heterocyclic ring or 6-membered heterocyclic ring, wherein the carbocyclic ring or heterocyclic ring is optionally substituted with 1 to 4 substituents selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano (such as F, Cl, Br, I, OH or cyano);

in some embodiments, $R^3$ is selected from H or $C_{1-6}$ alkyl, wherein the alkyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ carbocyclic ring;

in some embodiments, $R^3$ is selected from H or $C_{1-4}$ alkyl, wherein the alkyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, $C_{1-4}$ alkoxy or $C_{3-6}$ carbocyclic ring;
in some embodiments, $R^3$ is selected from H or $C_{1-4}$ alkyl, wherein the alkyl is optionally substituted with 0 to 4 substituents selected from deuterium, halogen, $C_{1-4}$ alkyl, $C_{1-4}$ alkoxy or benzene ring;
in some embodiments, $R^3$ is selected from H, methyl, ethyl, propyl, butyl, isobutyl, sec-butyl, tert-butyl or benzyl;
in some embodiments, ring A is selected from $C_{8-10}$ fused carbocyclic ring (such as benzo $C_{4-6}$ carbocyclic ring), and the ring A is optionally substituted with 0 to 4 $R^{a5}$; in some embodiments, ring A is selected from

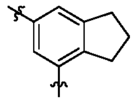,

and the ring A is optionally substituted with 0 to 4 $R^{a5}$; in some embodiments, ring A is selected from

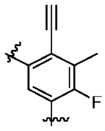, 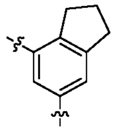, 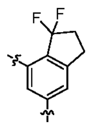, 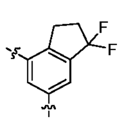, 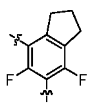, 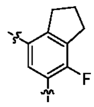, 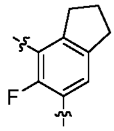, 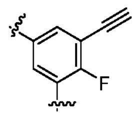,

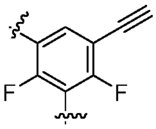, 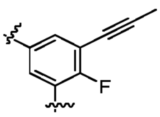, 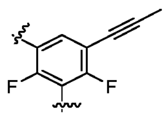, 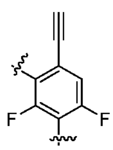, 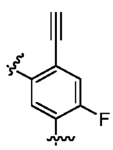, 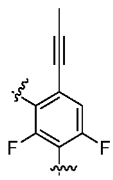 or 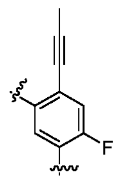;

in some embodiments, ring A is selected from

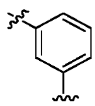,

and the ring A is substituted with one substituent selected from $C_{2-6}$ alkenyl or $C_{2-6}$ alkynyl and optionally substituted with 1 to 3 substituents selected from $R^{a5}$; in some embodiments, ring A is selected from

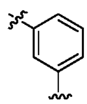,

and the ring A is substituted with one $R^{ak}$ and optionally substituted with 1 to 3 substituents selected from $R^{a5}$; in some embodiments, ring A is selected from

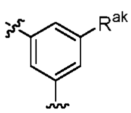,

and the ring A is optionally substituted with 1 to 3 substituents selected from $R^{a5}$;
in some embodiments, $R^{ak}$ is selected from $C_{2-4}$ alkynyl (such as ethynyl, propynyl or propargyl);
in some embodiments, ring A is selected from

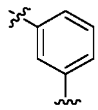

,

and the ring A is substituted with one substituent selected from ethenyl, ethynyl, propynyl or propargyl and optionally substituted with 1 to 3 substituents selected from $R^{a5}$;

in some embodiments, $R^{a5}$ is selected from halogen, OH, cyano, $C_{1-6}$ alkyl, $C_{2-6}$ alkenyl, $C_{2-6}$ alkynyl, $C_{1-6}$ alkoxy, $C_{3-6}$ cycloalkyl, or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ cycloalkyl, and the heterocyclyl contains 1 to 3 heteroatoms selected from N, O or S;

in some embodiments, $R^{a5}$ is selected from halogen, OH, cyano, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocyclyl, wherein the alkyl, alkenyl, alkynyl, alkoxy, cycloalkyl or heterocyclyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, halogen-substituted $C_{1-4}$ alkyl, cyano-substituted $C_{1-4}$ alkyl, $C_{1-4}$ alkoxy or $C_{3-6}$ cycloalkyl, and the heterocyclyl contains 1 to 3 heteroatoms selected from N, O or S;

in some embodiments, $R^{a5}$ is selected from halogen, OH, cyano, $C_{1-4}$ alkyl, $C_{2-4}$ alkynyl, $C_{1-4}$ alkoxy or $C_{3-6}$ cycloalkyl, wherein the alkyl, alkynyl, alkoxy, cycloalkyl or heterocyclyl is optionally substituted with 0 to 4 substituents selected from deuterium, halogen, OH, =O, cyano, $C_{1-4}$ alkyl, $C_{1-4}$ alkoxy or $C_{3-6}$ cycloalkyl;

in some embodiments, each $R^{a5}$ is independently selected from F, Cl, Br, I, OH, CN, ethynyl, propynyl, propargyl, methyl, ethyl, cyclopropyl, methoxy or ethoxy, wherein the ethynyl, propynyl, propargyl, methyl, ethyl, cyclopropyl, methoxy or ethoxy is optionally substituted with 1 to 4 substituents selected from deuterium, halogen, OH, =O, cyano, $C_{1-4}$ alkyl, $C_{1-4}$ alkoxy or $C_{3-6}$ cycloalkyl;

in some embodiments, each $R^{a5}$ is independently selected from F, Cl, Br, I, OH, CN, ethynyl, methyl, or ethyl;

in some embodiments, each $R^{a5}$ is independently selected from H, halogen, OH, =O, cyano, COOH, $NH_2$, $NHC_{1-6}$ alkyl, $N(C_{1-6}$ alkyl$)_2$, $C_{1-6}$ alkyl, halogen-substituted $C_{1-6}$ alkyl, cyano-substituted $C_{1-6}$ alkyl, $C_{1-6}$ alkoxy or $C_{3-6}$ cycloalkyl;

in some embodiments, each $R^{a5}$ is independently selected from H, halogen, OH, =O, cyano, COOH, $NH_2$, $NHC_{1-4}$ alkyl, $N(C_{1-4}$ alkyl$)_2$, $C_{1-4}$ alkyl, halogen-substituted $C_{1-4}$ alkyl, cyano-substituted $C_{1-4}$ alkyl, $C_{1-4}$ alkoxy or $C_{3-6}$ cycloalkyl;

in some embodiments, each $R^{a5}$ is independently selected from F, Cl, Br, OH, cyano, $CF_3$, methyl, ethyl, methoxy, ethoxy, cyclopropyl or cyclobutyl;

in some embodiments, ring A is selected from

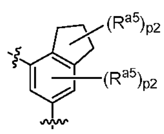

,

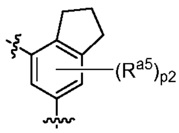

or

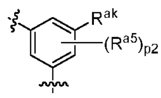

,

the upper left part of which is directly connected to $R^2$, wherein p2 is selected from 0, 1 or 2;

in some embodiments, $R^{1b}$ is selected from

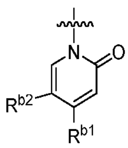

,

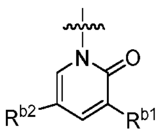

,

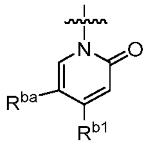, 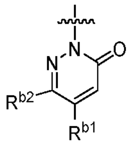, 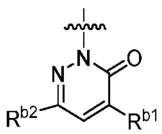, 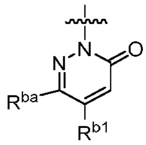, 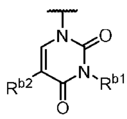, 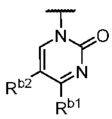, 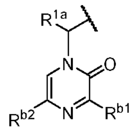

or

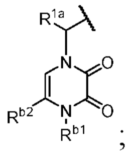;

in some embodiments, $R^{1b}$ is selected from

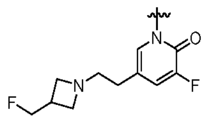

or

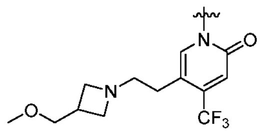;

in some embodiments, each $R^{b1}$ is independently selected from H, halogen, CN, $C_{1-6}$ alkyl, or $C_{3-6}$ cycloalkyl, wherein the alkyl or cycloalkyl is optionally substituted with 0 to 4 substituents selected from deuterium, halogen, OH, CN, $NHC_{1-6}$ alkyl, $N(C_{1-6}$ $alkyl)_2$, $C_{1-4}$ alkyl or $C_{1-6}$ alkoxy;

in some embodiments, each $R^{b1}$ is independently selected from H, halogen, CN, $C_{1-4}$ alkyl, or $C_{3-6}$ cycloalkyl, wherein the alkyl or cycloalkyl is optionally substituted with 0 to 4 substituents selected from deuterium, halogen, OH, CN, $NHC_{1-4}$ alkyl, $N(C_{1-4}$ $alkyl)_2$, $C_{1-4}$ alkyl or $C_{1-4}$ alkoxy;

in some embodiments, the compound is not the structure shown in Table E-2 or the stereoisomer thereof;

in some embodiments, the compound of general formula (I) is selected from a compound of general formula (I-a), (I-b), (I-a-1) or (I-b-1),

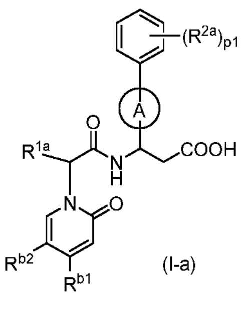
(I-a)

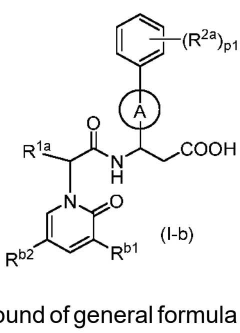
(I-b)

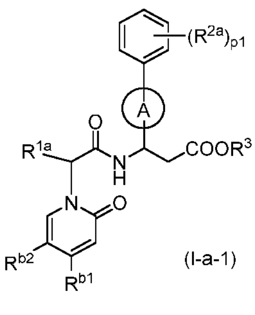
(I-a-1)

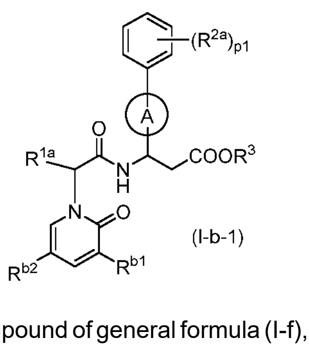
(I-b-1)

in some embodiments, the compound of general formula (I) is selected from a compound of general formula (I-f), (I-g), (I-f-1), (I-g-1), (I-h), (I-i), (I-h-1) or (I-i-1),

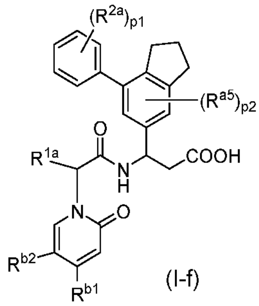
(I-f)
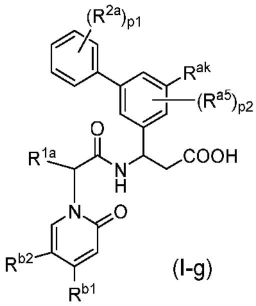
(I-g)
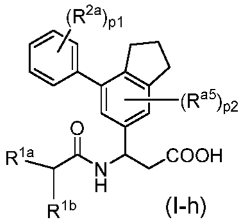
(I-h)
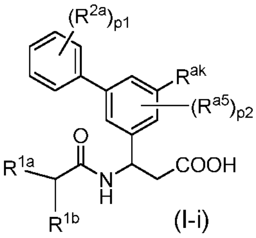
(I-i)

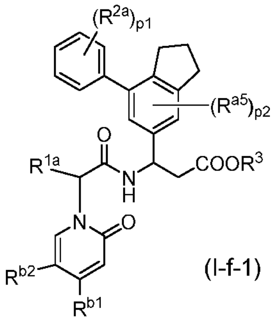
(I-f-1)
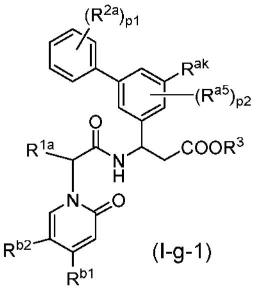
(I-g-1)
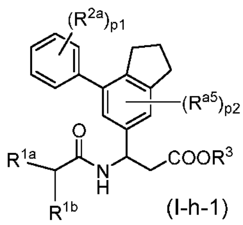
(I-h-1)
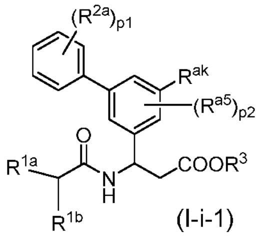
(I-i-1)

in some embodiments, in general formula (I-a), (I-b), (I-a-1), (I-b-1), (I-f), (I-g), (I-f-1), (I-g-1), (I-h), (I-i), (I-h-1) or (I-i-1), the definitions of $R^{2a}$, $R^{1a}$, $R^{ak}$, $R^{a5}$, $R^{b2}$, $R^{b1}$ and $R^3$ are the same as those in any one of the preceding embodiments;
in some embodiments, in general formula (I-a) or (I-b), the definition of ring A is the same as that in any one of the preceding embodiments of ring A;
in some embodiments, in general formula (I), (I-a), (I-b), (I-a-1), (I-b-1), (I-f), (I-g), (I-f-1), (I-g-1), (I-h), (I-i), (I-h-1) or (I-i-1), p1 is selected from 0, 1, 2, 3 or 4;
in some embodiments, in general formula (I), (I-a), (I-b), (I-a-1), (I-b-1), (I-f), (I-g), (I-f-1), (I-g-1), (I-h), (I-i), (I-h-1) or (I-i-1), $R^{2a}$ is selected from deuterium, halogen, OH, CN, $C_{1-4}$ alkyl, $C_{1-4}$ alkoxy, $C_{3-6}$ cycloalkyl or 4- to 6-membered heterocycloalkyl, wherein the alkyl, alkoxy, cycloalkyl or heterocycloalkyl is optionally substituted with 0 to 4 substituents selected from deuterium, halogen, OH, CN, $C_{1-4}$ alkyl, or $C_{1-4}$ alkoxy, and the heterocycloalkyl contains 1 to 4 heteroatoms selected from O, S or N;
in some embodiments, in general formula (I), (I-a), (I-b), (I-a-1), (I-b-1), (I-f), (I-g), (I-f-1), (I-g-1), (I-h), (I-i), (I-h-1) or (I-i-1), $R^{1a}$ is selected from

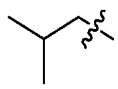
,

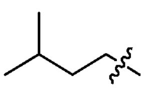
or
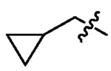
;

in some embodiments, in general formula (I), (I-a), (I-b), (I-a-1), (I-b-1), (I-f), (I-g), (I-f-1), (I-g-1), (I-h), (I-i), (I-h-1) or (I-i-1), $R^{b1}$ is selected from H, $C_{1-4}$ alkyl or $C_{3-6}$ cycloalkyl, wherein the alkyl or cycloalkyl is optionally substituted with 0 to 4 substituents selected from deuterium, halogen, OH, CN, $NHC_{1-4}$ alkyl, $N(C_{1-4}$ alkyl$)_2$, $C_{1-4}$ alkyl or $C_{1-4}$ alkoxy;
in some embodiments, in general formula (I), (I-a), (I-b), (I-f), (I-g), (I-f-1), (I-g-1), (I-h), (I-i), (I-h-1) or (I-i-1), $R^{b1}$ is selected from H, F, $CH_2F$, $CHF_2$, $CF_3$, methyl or $-CH_2CH_2N(CH_3)_2$;
in some embodiments, in general formula (I), (I-a), (I-b), (I-f), (I-g), (I-f-1), (I-g-1), (I-h), (I-i), (I-h-1) or (I-i-1), $R^{b2}$ is selected from H, $R^{ba}$, or one of the following substituted or unsubstituted groups: $C_{1-4}$ alkyl, $C_{2-4}$ alkynyl, $C_{1-4}$ alkoxy, $-CH_2CH_2-NHC_{1-4}$ alkyl, $-CH_2CH_2-N(C_{1-4}$ alkyl$)_2$, $-CH_2CH_2-C_{3-6}$ cycloalkyl, - $CH_2CH_2$-3- to 7-membered heterocycloalkyl, wherein the $CH_2$, alkyl, cycloalkyl or heterocycloalkyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heteroaryl or heterocycloalkyl contains 1 to 4 heteroatoms selected from O, S or N;

in some embodiments, in general formula (I), (I-a), (I-b), (I-f), (I-g), (I-f-1), (I-g-1), (I-h), (I-i), (I-h-1) or (I-i-1), $R^{b2}$ is selected from H, $R^{ba}$, or one of the following substituted or unsubstituted groups: methyl, ethyl, ethynyl, methoxy, ethoxy, $-CH_2NH(CH_2CH_3)$, $-CH_2N(CH_2CH_3)_2$, $-CH_2CH_2NH(CH_3)$, $-CH_2CH_2N(CH_3)_2$, $-CH_2CH_2NH(CH_2CH_3)$, $-CH_2CH_2N(CH_2CH_3)_2$, $-CH_2CH_2N(CH_3)(CH_2CH_3)$, $-CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, $-CH_2CH_2$-oxetanyl, $-CH_2CH_2$-oxolanyl, $-CH_2CH_2$-oxanyl or $-CH_2CH_2$-morpholine, which, when substituted, is substituted with 1, 2 or 3 substituents selected from deuterium, F, Cl, Br, OH, =O, cyano, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;

in some embodiments, in general formula (I-a), (I-b), (I-f), (I-g), (I-f-1), (I-g-1), (I-h), (I-i), (I-h-1) or (I-i-1), the definitions of $R^{ba}$ and $R^k$ are the same as those in any one of the preceding embodiments;

in some embodiments, in general formula (I), (I-a), (I-b), (I-f), (I-g), (I-f-1), (I-g-1), (I-h), (I-i), (I-h-1) or (I-i-1), $R^{2a}$ is selected from deuterium, $-CD_3$, $-OCD_3$, F, Cl, Br, I, OH, cyano, methyl, ethyl, methoxy or ethoxy;

in some embodiments, in general formula (I), (I-f), (I-g), (I-f-1), (I-g-1), (I-h), (I-i), (I-h-1) or (I-i-1), p2 is selected from 0, 1 or 2;

in some embodiments, in general formula (I), (I-g), (I-g-1), (I-i) or (I-i-1), $R^{ak}$ is selected from $C_{2-4}$ alkynyl;

in some embodiments, in general formula (I), (I-f), (I-g), (I-f-1), (I-g-1), (I-h), (I-i), (I-h-1) or (I-i-1), $R^{a5}$ is selected from F, Cl, Br, I, OH, cyano, methyl, ethyl, methoxy, ethoxy or ethynyl;

in some embodiments, in general formula (I), n1 is selected from 1, 2 or 3.

As a first embodiment of the present invention, provided is the compound of preceding general formula (I) or a stereoisomer, mesomer/racemate, deuterated product, solvate, prodrug, metabolite, pharmaceutically acceptable salt, or co-crystal thereof, wherein

$R^1$ is selected from $-CHR^{1a}R^{1b}$ or $-NR^{1a}R^{1b}$;

$R^{1a}$ is selected from $C_{1-6}$ alkyl, wherein the alkyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ cycloalkyl;

$R^{1b}$ is selected from $C_{4-10}$ carbocyclic ring or 5- to 10-membered heterocyclic ring, wherein the carbocyclic ring or heterocyclic ring is optionally substituted with 0 to 4 $R^b$, and the heterocyclic ring contains 1 to 4 heteroatoms selected from O, S or N;

each $R^b$ is independently selected from deuterium, halogen, OH, =O, cyano, COOH, $NH_2$, $-C_{0-4}$ alkyl-$NHC_{1-6}$ alkyl, $-C_{0-4}$ alkyl-$N(C_{1-6}$ alkyl$)_2$, $C_{1-6}$ alkyl, $C_{2-6}$ alkynyl, $C_{1-6}$ alkoxy, $-C_{0-4}$ alkyl-$C_{3-10}$ carbocyclic ring, $-C_{0-4}$ alkyl-3- to 10-membered heterocyclic ring or $R^{ba}$, wherein the alkyl, alkynyl, alkoxy, carbocyclic ring or heterocyclic ring is optionally substituted with 0 to 4 substituents selected from deuterium, halogen, OH, =O, cyano, COOH, $CONH_2$, $CONHC_{1-6}$ alkyl, $CON(C_{1-6}$ alkyl$)_2$, $NH_2$, $NHC_{1-6}$ alkyl, $N(C_{1-6}$ alkyl$)_2$, $N(C_{1-6}$ alkyl$)(C_{3-6}$ cycloalkyl), $NH(C_{3-6}$ cycloalkyl), $C_{1-6}$ alkyl, $C_{2-6}$ alkynyl, $C_{1-6}$ alkoxy, halogen-substituted $C_{1-6}$ alkyl, halogen-substituted $C_{1-6}$ alkoxy, $C_{1-6}$ alkoxyalkyl or $R^k$, and the heterocyclic ring contains 1 to 4 heteroatoms selected from O, S or N;

$R^{ba}$ is selected from $-C_{0-4}$ alkyl-7- to 12-membered heterocyclic ring, $-C_{0-4}$ alkyl-4- to 6-membered heterocyclic ring connected via a carbon atom,

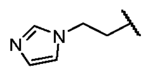
,

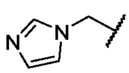
,
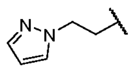
,
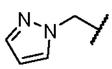
or
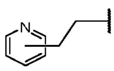
,

and the $R^{ba}$ is optionally substituted with 1 to 4 substituents selected from H, halogen, OH, =O, cyano, COOH, $CONH_2$, $CONHC_{1-6}$ alkyl, $CON(C_{1-6}$ alkyl$)_2$, $NH_2$, $NHC_{1-6}$ alkyl, $N(C_{1-6}$ alkyl$)_2$, $C_{1-6}$ alkyl, $C_{2-6}$ alkynyl, $C_{1-6}$ alkoxy, halogen-substituted $C_{1-6}$ alkyl, halogen-substituted $C_{1-6}$ alkoxy, $C_{1-6}$ alkoxyalkyl or $R^k$, wherein the heterocyclic ring contains 1 to 4 heteroatoms selected from O, S or N;

$R^k$ is selected from $-C_{1-4}$ alkyl-$NH_2$, $-C_{1-4}$ alkyl-$NHC_{1-6}$ alkyl, $-C_{1-4}$ alkyl-$N(C_{1-6}$ alkyl$)_2$, $-C_{0-4}$ alkyl-$C_{3-10}$ carbocyclic ring or $-C_{0-4}$ alkyl-3- to 10-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy, halogen-substituted $C_{1-6}$ alkyl, halogen-substituted $C_{1-6}$ alkoxy or $C_{1-6}$ alkoxyalkyl, and the heterocyclic ring contains 1 to 4 heteroatoms selected from O, S or N;

$R^2$ is selected from $C_{1-6}$ alkyl, $C_{6-10}$ aromatic ring, 5- to 10-membered heteroaromatic ring, $C_{3-10}$ carbocyclic ring or 5- to 10-membered heterocyclic ring, and the $R^2$ is optionally substituted with 0 to 4 $R^{2a}$, wherein the heteroaromatic ring or heterocyclic ring contains 1 to 4 heteroatoms selected from O, S or N;

each $R^{2a}$ is independently selected from deuterium, halogen, OH, cyano, =O, $C_{1-6}$ alkyl, $C_{2-6}$ alkenyl, $C_{2-6}$ alkynyl, $C_{1-6}$ alkoxy, $C_{3-6}$ cycloalkyl or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl or $C_{1-6}$ alkoxy, and the heterocyclyl contains 1 to 3 heteroatoms selected from N, O or S;

as an option, $R^{2a}$ and $R^{2a}$ are directly linked to form $C_{4-7}$ carbocyclic ring or 4-to 7-membered heterocyclic ring, wherein the carbocyclic ring or heterocyclic ring is optionally substituted with 1 to 4 substituents selected from deuterium, halogen, OH, cyano, =O, $C_{1-6}$ alkyl, $C_{2-6}$ alkenyl, $C_{2-6}$ alkynyl, $C_{1-6}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;

$R^3$ is selected from H or $C_{1-6}$ alkyl, wherein the alkyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ carbocyclic ring;

ring A is selected from $C_{8-10}$ fused carbocyclic ring, and the ring A is optionally substituted with 0 to 4 $R^{a5}$;

or ring A is selected from

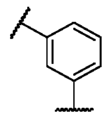,

and the ring A is substituted with one substituent selected from $C_{2-6}$ alkenyl or $C_{2-6}$ alkynyl and optionally substituted with 1 to 3 $R^{a5}$;

$R^{a5}$ is selected from halogen, OH, cyano, $C_{1-6}$ alkyl, $C_{2-6}$ alkenyl, $C_{2-6}$ alkynyl, $C_{1-6}$ alkoxy, $C_{3-6}$ cycloalkyl, or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ cycloalkyl, and the heterocyclyl contains 1 to 3 heteroatoms selected from N, O or S.

As a second embodiment of the present invention, provided is the compound of preceding general formula (I) or a stereoisomer, mesomer/racemate, deuterated product, solvate, prodrug, metabolite, pharmaceutically acceptable salt, or co-crystal thereof, wherein

$R^1$ is selected from

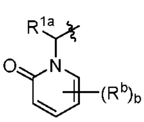, 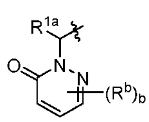, 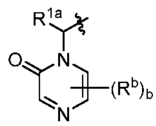, 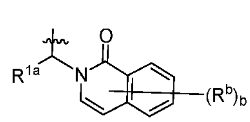,

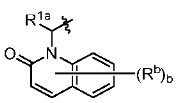, 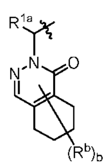, 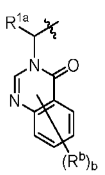, 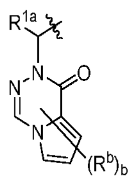, 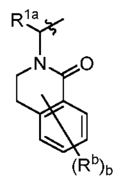, 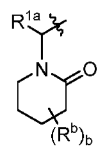, 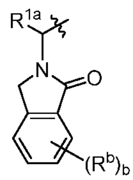,

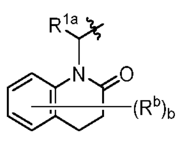, 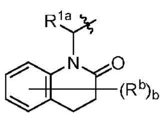, 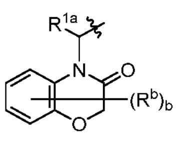, 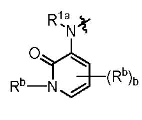, 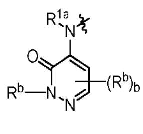, 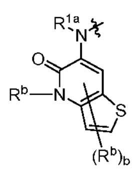,

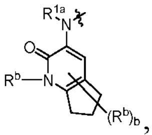, 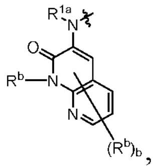, 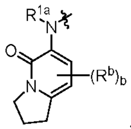, 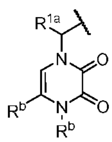, 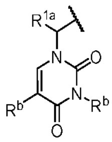 or 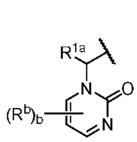;

b is selected from 0, 1, 2 or 3;

each $R^b$ is independently selected from deuterium, halogen, OH, =O, cyano, COOH, $NH_2$, $-C_{0-4}$ alkyl-$NHC_{1-4}$ alkyl, $-C_{0-4}$ alkyl-$N(C_{1-4}$ alkyl$)_2$, $C_{1-4}$ alkyl, $C_{2-4}$ alkynyl, $C_{1-4}$ alkoxy, $-C_{0-4}$ alkyl-$C_{3-6}$ carbocyclic ring, $-C_{0-4}$ alkyl-3- to 7-membered heterocyclic ring or $R^{ba}$, wherein the alkyl, alkynyl, alkoxy, carbocyclic ring or heterocyclic ring is optionally substituted with 0 to 4 substituents selected from deuterium, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heterocyclic ring contains 1 to 4 heteroatoms selected from O, S or N;

$R^{ba}$ is selected from $-C_{0-2}$ alkyl-7- to 8-membered monocyclic heterocycloalkyl, $-C_{0-2}$ alkyl-7- to 11-membered spiro heterocycloalkyl, $-C_{0-2}$ alkyl-7- to 11-membered bridged heterocycloalkyl, $-C_{0-2}$ alkyl-4- to 6-membered monocyclic heterocycloalkyl connected via a carbon atom,

, 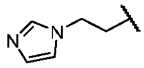, 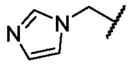,

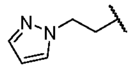, 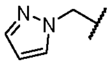 or 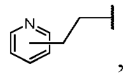,

and the $R^{ba}$ is optionally substituted with 1 to 4 substituents selected from H, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, wherein the heterocycloalkyl contains 1 to 4 heteroatoms selected from O, S or N;

$R^k$ is selected from $-C_{1-2}$ alkyl-$NH_2$, $-C_{1-2}$ alkyl-$NHC_{1-4}$ alkyl, $-C_{1-2}$ alkyl-$N(C_{1-4}$ alkyl$)_2$, $-C_{0-2}$ alkyl-$C_{3-6}$ carbocyclic ring or $-C_{0-2}$ alkyl-3- to 6-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl, and the heterocyclic ring contains 1 to 4 heteroatoms selected from O, S or N;

$R^2$ is selected from $C_{1-4}$ alkyl, benzene ring, naphthalene ring, 5- to 6-membered heteroaromatic ring, 9- to 10-membered heteroaromatic ring, $C_{3-10}$ non-aromatic carbocyclic ring, 5- to 10-membered non-aromatic heterocyclic ring, benzo $C_{4-6}$ carbocyclyl or benzo 4- to 6-membered heterocyclyl, and the $R^2$ is optionally substituted with 0 to 4 $R^{2a}$, wherein the heteroaromatic ring or heterocyclic ring contains 1 to 4 heteroatoms selected from O, S or N;

each $R^{2a}$ is independently selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl or $C_{1-4}$ alkoxy, and the heterocyclyl contains 1 to 3 heteroatoms selected from N, O or S;

as an option, $R^{2a}$ and $R^{2a}$ are directly linked to form $C_{4-7}$ carbocyclic ring or 4-to 7-membered heterocyclic ring, wherein the carbocyclic ring or heterocyclic ring is optionally substituted with 1 to 4 substituents selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;

$R^3$ is selected from H or $C_{1-4}$ alkyl, wherein the alkyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, $C_{1-4}$ alkoxy or $C_{3-6}$ carbocyclic ring;

ring A is selected from benzo $C_{4-6}$ carbocyclic ring, and the ring A is optionally substituted with 0 to 4 $R^{a5}$;

or ring A is selected from

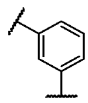,

and the ring A is substituted with one substituent selected from $C_{2-4}$ alkenyl or $C_{2-4}$ alkynyl and optionally substituted with 1 to 3 substituents selected from $R^{a5}$;

$R^{a5}$ is selected from halogen, OH, cyano, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocyclyl, wherein the alkyl, alkenyl, alkynyl, alkoxy, cycloalkyl or heterocyclyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, halogen-substituted $C_{1-4}$ alkyl, cyano-substituted $C_{1-4}$ alkyl, $C_{1-4}$ alkoxy or $C_{3-6}$ cycloalkyl, and the heterocyclyl contains 1 to 3 heteroatoms selected from N, O or S;

the definitions of the remaining substituents are consistent with those in the first embodiment of the present invention.

As a third embodiment of the present invention, provided is the compound of preceding general formula (I) or a stereoisomer, mesomer/racemate, deuterated product, solvate, prodrug, metabolite, pharmaceutically acceptable salt, or co-crystal thereof, wherein

each $R^b$ is independently selected from deuterium, halogen, OH, =O, cyano, $C_{1-4}$ alkyl, $C_{2-4}$ alkynyl, $C_{1-4}$ alkoxy, phenyl, 5- to 6-membered heteroaryl, - $CH_2NHC_{1-4}$ alkyl, $-CH_2N(C_{1-4}$ alkyl$)_2$, $-CH_2CH_2-NHC_{1-4}$ alkyl, $-CH_2CH_2-N(C_{1-4}$ alkyl$)_2$, phenyl, 5- to 6-membered heteroaryl, $C_{3-6}$ cycloalkyl, 3- to 7-membered heterocycloalkyl, $-CH_2$-phenyl, $-CH_2$-5- to 6-membered heteroaryl, $-CH_2-C_{3-6}$ cycloalkyl, $-CH_2$-3- to 7-membered heterocycloalkyl, $-CH_2CH_2$-phenyl, $-CH_2CH_2$-5- to 6-membered heteroaryl, $-CH_2CH_2-C_{3-6}$ cycloalkyl, $-CH_2CH_2$-3- to 7-membered heterocycloalkyl or $R^{ba}$, wherein the $CH_2$, alkyl, alkynyl, phenyl, heteroaryl, cycloalkyl or heterocycloalkyl is optionally substituted with 0 to 4 substituents selected from deuterium, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heteroaryl or heterocycloalkyl contains 1 to 4 heteroatoms selected from O, S or N;

$R^{ba}$ is selected from 7- to 8-membered monocyclic heterocycloalkyl, 7- to 11-membered spiro heterocycloalkyl, 7- to 11-membered bridged heterocycloalkyl, 4-to 6-membered monocyclic heterocycloalkyl connected via a carbon atom, $-CH_2$-7-to 8-membered monocyclic heterocycloalkyl, $-CH_2$-7- to 11-membered spiro heterocycloalkyl, $-CH_2$-7- to 11-membered bridged heterocycloalkyl, $-CH_2$-4- to 6-membered monocyclic heterocycloalkyl connected via a carbon atom, $-CH_2CH_2$-7-to 8-membered monocyclic heterocycloalkyl, $-CH_2CH_2$-7- to 11-membered spiro heterocycloalkyl, $-CH_2CH_2$-7- to 11-membered bridged heterocycloalkyl, - $CH_2CH_2$-4- to 6-membered monocyclic heterocycloalkyl connected via a carbon atom,

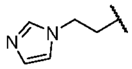, 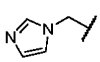, 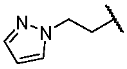, 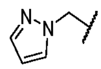 or 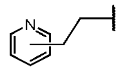,

and the $R^{ba}$ is optionally substituted with 1 to 4 substituents selected from H, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, wherein the heterocycloalkyl contains 1 to 4 heteroatoms selected from O, S or N;

$R^k$ is selected from $-C_{1-4}$ alkyl-$NH_2$, $-C_{1-4}$ alkyl-$NHC_{1-4}$ alkyl, $-C_{1-4}$ alkyl-$N(C_{1-4}$ alkyl$)_2$, $-C_{0-4}$ alkyl-$C_{3-6}$ carbocyclic ring or $-C_{0-4}$ alkyl-3- to 6-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl, and the heterocyclic ring contains 1 to 4 heteroatoms selected from O, S or N;

$R^2$ is selected from benzene ring, naphthalene ring, 5- to 6-membered heteroaromatic ring, 9- to 10-membered heteroaromatic ring, $C_{3-6}$ cycloalkyl, 3- to 7-membered heterocycloalkyl, benzo $C_{4-6}$ carbocyclyl or benzo 4- to 6-membered heterocyclyl, and the $R^2$ is optionally substituted with 0 to 4 $R^{2a}$, wherein the heteroaromatic ring or heterocycloalkyl contains 1 to 4 heteroatoms selected from O, S or N;

each $R^{2a}$ is independently selected from deuterium, halogen, OH, cyano, =O, $C_{1-4}$ alkyl, $C_{2-4}$ alkenyl, $C_{2-4}$ alkynyl, $C_{1-4}$ alkoxy or $C_{3-6}$ cycloalkyl, wherein the alkyl, alkenyl, alkynyl, alkoxy or cycloalkyl is optionally substituted with 0 to 4 substituents selected from deuterium, halogen, OH, =O, cyano, $C_{1-4}$ alkyl or $C_{1-4}$ alkoxy;

as an option, $R^{2a}$ and $R^{2a}$ are directly linked to form 4-membered carbocyclic ring, 5-membered carbocyclic ring, 6-membered carbocyclic ring, 4-membered heterocyclic ring, 5-membered heterocyclic ring or 6-membered heterocyclic ring, wherein the carbocyclic ring or heterocyclic ring is optionally substituted with 1 to 4 substituents selected

from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;
$R^3$ is selected from H or $C_{1-4}$ alkyl, wherein the alkyl is optionally substituted with 0 to 4 substituents selected from deuterium, halogen, $C_{1-4}$ alkyl, $C_{1-4}$ alkoxy or benzene ring;
ring A is selected from

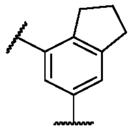,

and the ring A is optionally substituted with 0 to 4 $R^5$;
or ring A is selected from

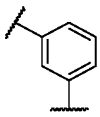,

and the ring A is substituted with one substituent selected from $C_{2-4}$ alkenyl or $C_{2-4}$ alkynyl and optionally substituted with 1 to 3 substituents selected from $R^{a5}$;
$R^{a5}$ is selected from halogen, OH, cyano, $C_{1-4}$ alkyl, $C_{2-4}$ alkynyl, $C_{1-4}$ alkoxy or $C_{3-6}$ cycloalkyl, wherein the alkyl, alkynyl, alkoxy, cycloalkyl or heterocyclyl is optionally substituted with 0 to 4 substituents selected from deuterium, halogen, OH, =O, cyano, $C_{1-4}$ alkyl, $C_{1-4}$ alkoxy or $C_{3-6}$ cycloalkyl;
the definitions of the remaining substituents are the same as those in any one of the first or second embodiment of the present invention.

As a forth embodiment of the present invention, provided is the compound of preceding general formula (I) or a stereoisomer, mesomer/racemate, deuterated product, solvate, prodrug, metabolite, pharmaceutically acceptable salt, or co-crystal thereof, wherein

$R^{1a}$ is selected from methyl, ethyl, propyl, butyl, isobutyl, sec-butyl, tert-butyl, $-CH_2$-cyclopropyl or $-CH_2$-cyclobutyl;
each $R^b$ is independently selected from deuterium, F, Cl, Br, I, OH, =O, cyano or $R^{ba}$, or each $R^b$ is independently selected from one of the following substituted or unsubstituted groups: methyl, ethyl, ethynyl, methoxy, ethoxy, cyclopropyl, cyclobutyl, cyclopentyl, cyclohexyl, azetidinyl, azolidinyl, azinanyl, oxetanyl, oxolanyl, oxanyl, morpholinyl, phenyl, pyridine, $-CH_2NH(CH_2CH_3)$, - $CH_2N(CH_2CH_3)_2$, $-CH_2CH_2NH(CH_3)$, $-CH_2CH_2N(CH_3)_2$, $-CH_2CH_2NH(CH_2CH_3)$, - $CH_2CH_2N(CH_2CH_3)_2$, $-CH_2CH_2N(CH_3)(CH_2CH_3)$, $-CH_2$-cyclopropyl, $-CH_2$-cyclobutyl, $-CH_2$-cyclopentyl, $-CH_2$-cyclohexyl, $-CH_2$-azetidinyl, $-CH_2$-azolidinyl, $-CH_2$-azinanyl, $-CH_2$-oxetanyl, $-CH_2$-oxolanyl, $-CH_2$-oxanyl, $-CH_2$-morpholinyl, - $CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, - $CH_2CH_2$-oxetanyl, $-CH_2CH_2$-oxolanyl, $-CH_2CH_2$-oxanyl, $-CH_2CH_2$-morpholinyl, - $CH_2$-phenyl, $-CH_2$-pyridyl, $-CH_2CH_2$-phenyl or $-CH_2CH_2$-pyridyl, which, when substituted, is substituted with 0 to 4 substituents selected from deuterium, halogen, OH, =O, cyano, $CONH_2$, $CONHC_{1-4}$ alkyl, $CON(C_{1-4}\ alkyl)_2$, $NH_2$, $NHC_{1-4}$ alkyl, $N(C_{1-4}\ alkyl)_2$, $N(C_{1-4}\ alkyl)(C_{3-6}\ cycloalkyl)$, $NH(C_{3-6}\ cycloalkyl)$, $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$;
$R^{ba}$ is selected from one of the following optionally substituted groups:

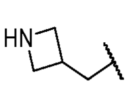, 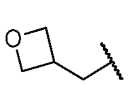, 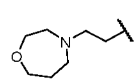, 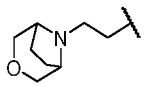, 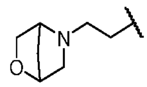, 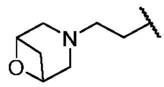,

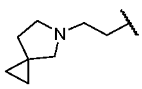, 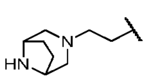, 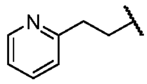, 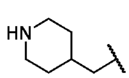, 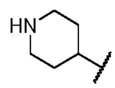, 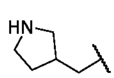, 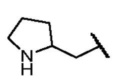,

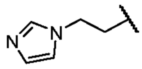, 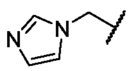, 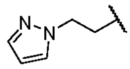, 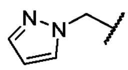, 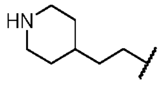, 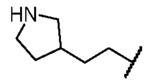 or

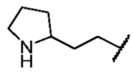,

which, when substituted, is optionally substituted with 1 to 4 substituents selected from deuterium, halogen, OH, =O, cyano, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$;

$R^k$ is selected from $-CH_2N(CH_3)_2$, $-CH_2$-cyclopropyl, $-CH_2$-cyclobutyl, cyclopropyl, cyclobutyl, cyclopentyl, cyclohexyl, azetidinyl, azolidinyl, azinanyl or oxetanyl, wherein the cyclopropyl, cyclobutyl, cyclopentyl, cyclohexyl, azetidinyl, azolidinyl or azinanyl is optionally substituted with 1 to 4 substituents selected from deuterium, halogen, OH, =O, cyano, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl;

$R^2$ is selected from benzene ring, pyridyl, pyridonyl, pyrazinyl, pyrimidyl, thienyl, thiazolyl, furyl, oxazolyl, pyrrolyl, pyrazolyl, imidazolyl, cyclopropyl, cyclobutyl, cyclopentyl, cyclohexyl, azetidinyl, azolidinyl, azinanyl, oxetanyl, oxolanyl, oxanyl, morpholinyl,

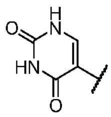, 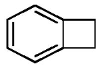, 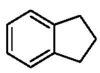, 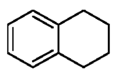, 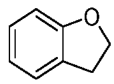,

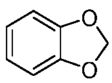, 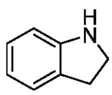, 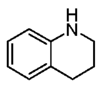, 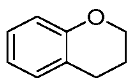, 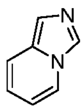 or 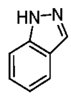,

and the $R^2$ is optionally substituted with 0 to 4 $R^{2a}$;

each $R^{2a}$ is independently selected from deuterium, F, Cl, Br, I, OH, =O, cyano, methyl, ethyl, ethenyl, ethynyl, methoxy, ethoxy, cyclopropyl or cyclobutyl, wherein the methyl, ethyl, ethenyl, ethynyl, methoxy, ethoxy, cyclopropyl or cyclobutyl is optionally substituted with 0 to 4 substituents selected from deuterium, halogen, OH, =O, cyano, $C_{1-4}$ alkyl or $C_{1-4}$ alkoxy;

$R^3$ is selected from H, methyl, ethyl, propyl, butyl, isobutyl, sec-butyl, tert-butyl or benzyl;

ring A is selected from

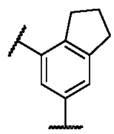,

and the ring A is optionally substituted with 0 to 4 $R^{a5}$;

or ring A is selected from

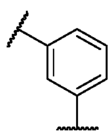,

and the ring A is substituted with one substituent selected from ethenyl, ethynyl, propynyl or propargyl and optionally substituted with 1 to 3 substituents selected from $R^{a5}$;

each $R^{a5}$ is independently selected from F, Cl, Br, I, OH, CN, ethynyl, propynyl, propargyl, methyl, ethyl, cyclopropyl, methoxy or ethoxy, wherein the ethynyl, propynyl, propargyl, methyl, ethyl, cyclopropyl, methoxy or ethoxy is

optionally substituted with 1 to 4 substituents selected from deuterium, halogen, OH, =O, cyano, $C_{1-4}$ alkyl, $C_{1-4}$ alkoxy or $C_{3-6}$ cycloalkyl;

the definitions of the remaining substituents are the same as those in any one of the first, second or third embodiment of the present invention.

As a fifth embodiment of the present invention, provided is the compound of preceding general formula (I) or a stereoisomer, mesomer/racemate, deuterated product, solvate, prodrug, metabolite, pharmaceutically acceptable salt, or co-crystal thereof, wherein

$R^1$ is selected from

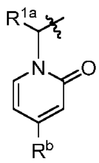, 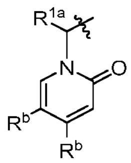, 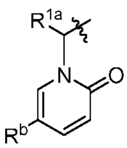, 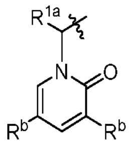, 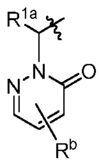,

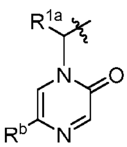, 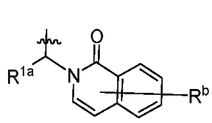, 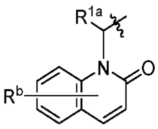, 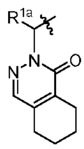, 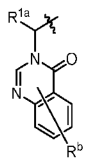, 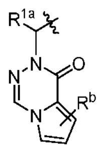, 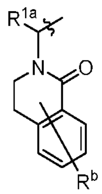, 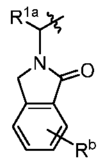,

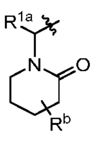, 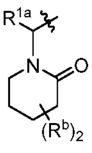, 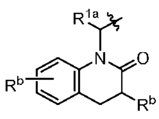, 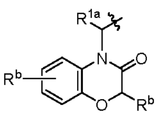, 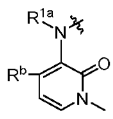, 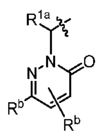, 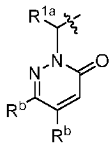, 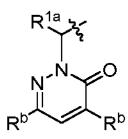,

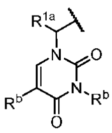, 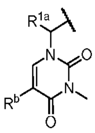, 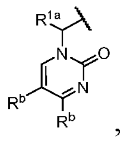, 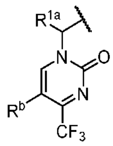, 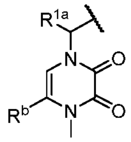, 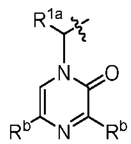, 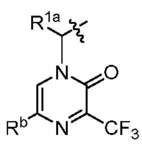, 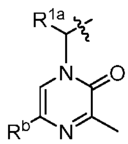,

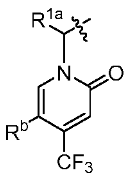, 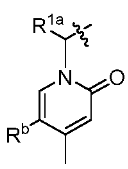, 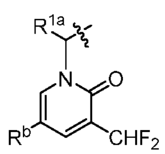 or 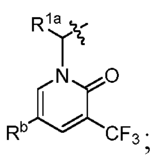;

each $R^b$ is independently selected from deuterium, F, Cl, Br, OH, cyano or $R^{ba}$, or each $R^b$ is independently selected from one of the following substituted or unsubstituted groups: methyl, ethyl, ethynyl, methoxy, ethoxy, cyclopropyl, cyclobutyl, cyclopentyl, cyclohexyl, azetidinyl, azolidinyl, azinanyl, oxetanyl, oxolanyl, oxanyl, morpholinyl, phenyl, pyridyl, $-CH_2NH(CH_2CH_3)$, - $CH_2N(CH_2CH_3)_2$, $-CH_2CH_2NH(CH_3)$, $-CH_2CH_2N(CH_3)_2$, $-CH_2CH_2NH(CH_2CH_3)$, - $CH_2CH_2N(CH_2CH_3)_2$, $-CH_2CH_2N(CH_3)(CH_2CH_3)$, $-CH_2$-cyclopropyl, $-CH_2$-cyclobutyl, $-CH_2$-cyclopentyl, $-CH_2$-cyclohexyl, $-CH_2$-azetidinyl, $-CH_2$-azolidinyl, $-CH_2$-azinanyl, $-CH_2$-oxetanyl, $-CH_2$-oxolanyl, $-CH_2$-oxanyl, $-CH_2$-morpholinyl, - $CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, - $CH_2CH_2$-oxetanyl, $-CH_2CH_2$-oxolanyl, $-CH_2CH_2$-oxanyl, $-CH_2CH_2$-morpholinyl, - $CH_2$-phenyl, $-CH_2$-pyridyl, $-CH_2CH_2$-phenyl, $-CH_2CH_2$-pyridyl or

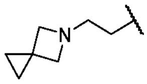,

which, when substituted, is substituted with 0 to 4 substituents selected from deuterium, F, Cl, Br, OH, =O, cyano, $CONH_2$, $CONHCH_3$, $CON(CH_3)_2$, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxy methyl, ethoxymethyl, methoxyethyl or $R^k$;
$R^{ba}$ is selected from one of the following optionally substituted groups:

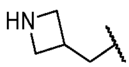, 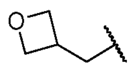, 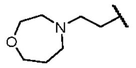, 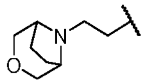, 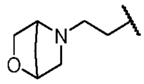, 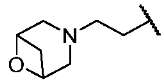,

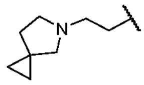, 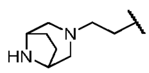, 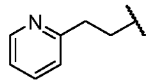, 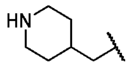, 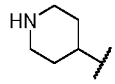, 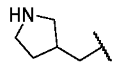, 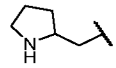,

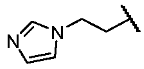, 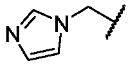, 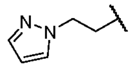, 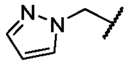, 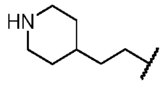, 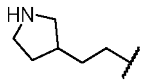

or

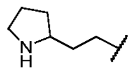,

which, when substituted, is optionally substituted with 1 to 4 substituents selected from deuterium, F, Cl, Br, OH, =O, cyano, $CONH_2$, $CONHCH_3$, $CON(CH_3)_2$, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;
$R^k$ is selected from $-CH_2N(CH_3)_2$, $-CH_2$-cyclopropyl, $-CH_2$-cyclobutyl, cyclopropyl, cyclobutyl, cyclopentyl, cyclohexyl, azetidinyl, azolidinyl, azinanyl or oxetanyl, wherein the cyclopropyl, cyclobutyl, cyclopentyl, cyclohexyl, azetidinyl, azolidinyl, azinanyl or oxetanyl is optionally substituted with 1 to 4 substituents selected from deuterium, F, Cl, Br, OH, =O, cyano, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, methoxy, ethoxy, methoxymethyl, ethoxymethyl or methoxyethyl;
$R^2$ is selected from phenyl, pyridyl, pyridonyl, azolidinyl, morpholinyl or

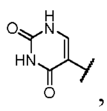,

and the $R^2$ is optionally substituted with 0 to 4 substituents selected from deuterium, F, Cl, Br, OH, $CF_3$, cyano, methyl, ethyl, methoxy, ethoxy, cyclopropyl or cyclobutyl;
or $R^2$ is selected from

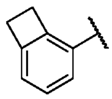, 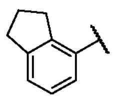, 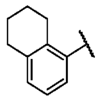, 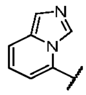, 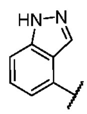, 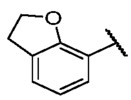,

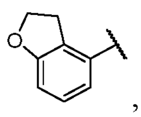, 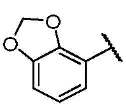 or 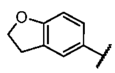,

and the $R^2$ is optionally substituted with 1 to 4 substituents selected from deuterium, $CD_3$, $-OCD_3$, F, Cl, Br, OH, $CF_3$,

cyano, methyl, ethyl, methoxy, ethoxy, cyclopropyl or cyclobutyl;
ring A is selected from

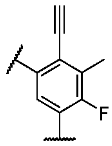, 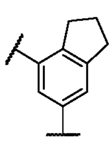, 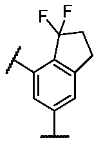, 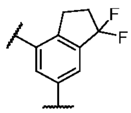, 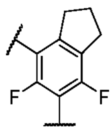,

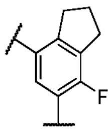, 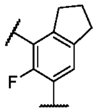, 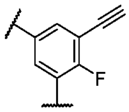, 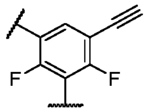, 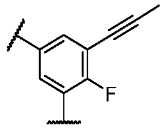, 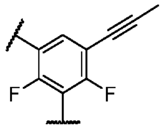, 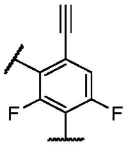,

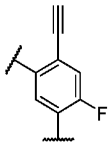, 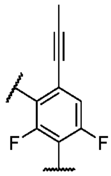 or 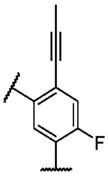;

the definitions of the remaining substituents are the same as those in any one of the first, second, third or fourth embodiment of the present invention.

As a sixth embodiment of the present invention, provided is the compound of above general formula (I) or a stereoisomer, mesomer/racemate, deuterated product, solvate, prodrug, metabolite, pharmaceutically acceptable salt, or co-crystal thereof, wherein

$R^1$ is selected from

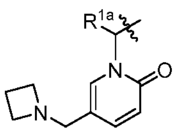, 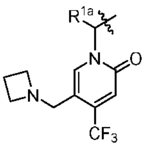, 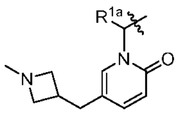, 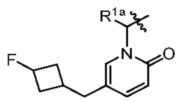,

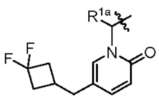, 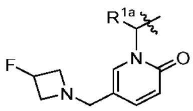, 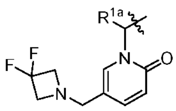, 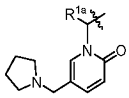, 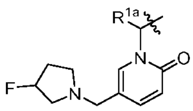,

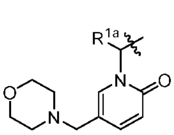, 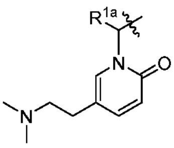, 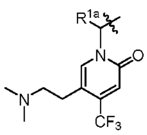, 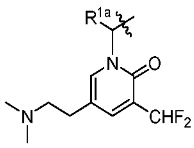, 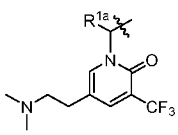,

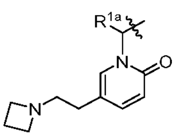, 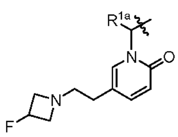, 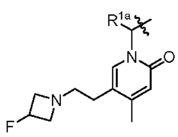, 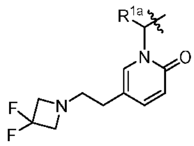, 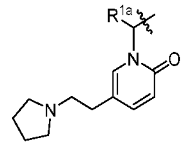,

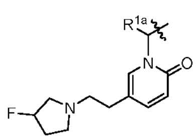, 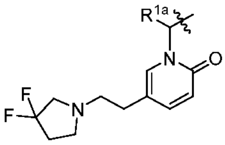, 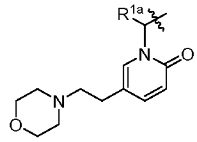, 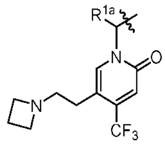, 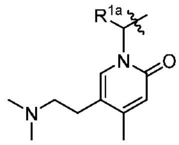,

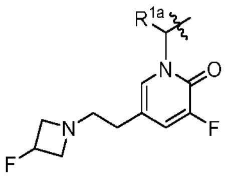, 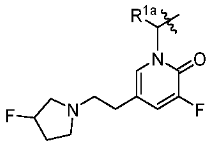 or 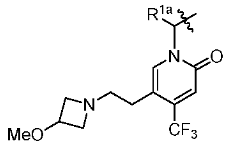;

or $R^1$ is selected from

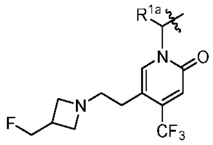 or 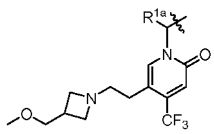;

or $R^1$ is selected from

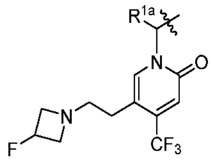 or 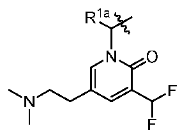;

or $R^1$ is selected from

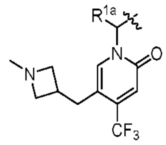, 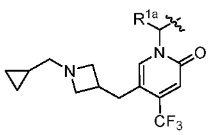, 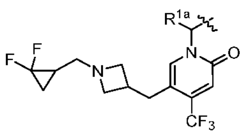,

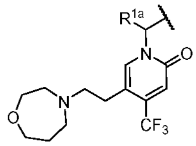, 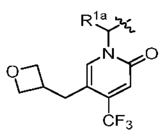, 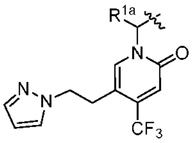, 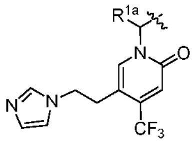, 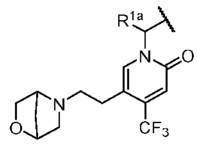,

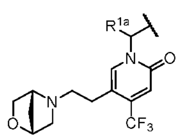, 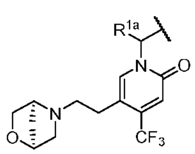, 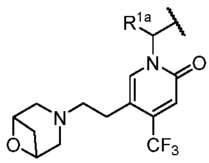, 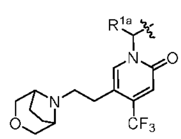, 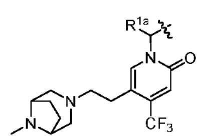,

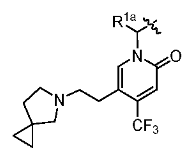, 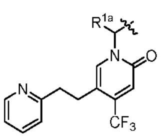, 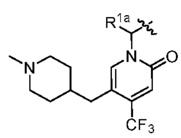, 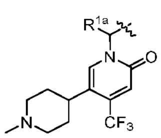, 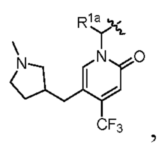, 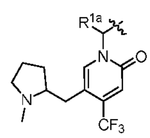,

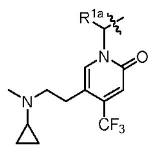, 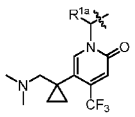, 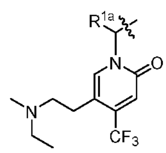, 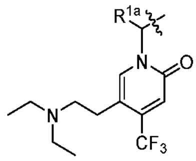, 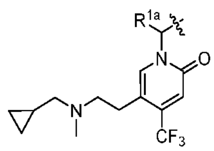

or

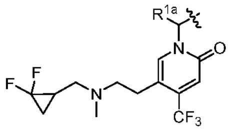;

or $R^1$ is selected from

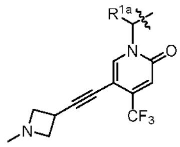, 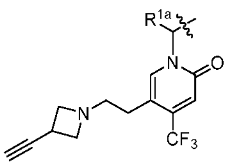, 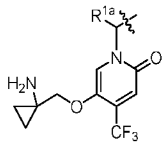,

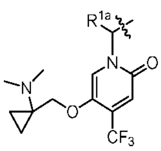, 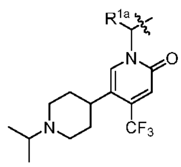, 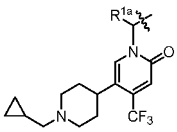, 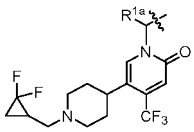, 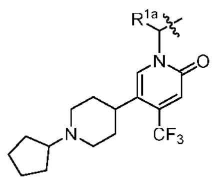

or

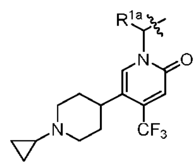;

or $R^1$ is selected from

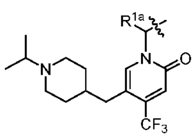, 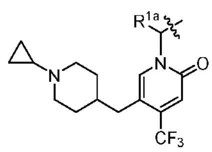, 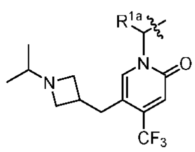,

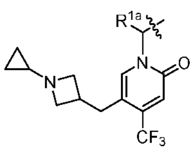, 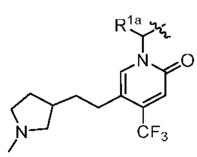 or 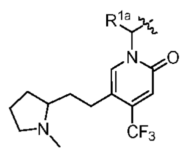;

or $R^1$ is selected from

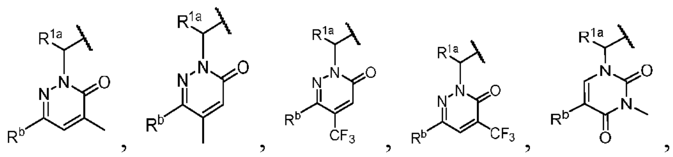

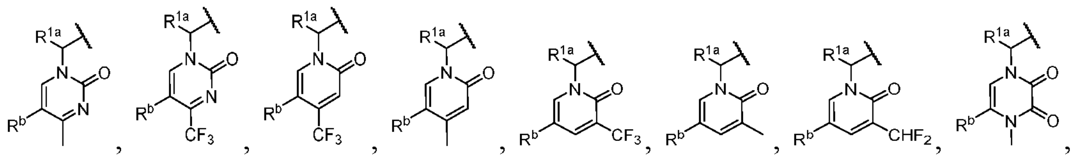

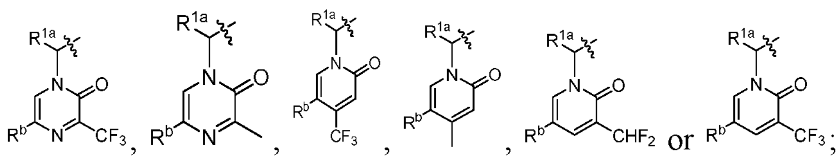
or ;

$R^2$ is selected from

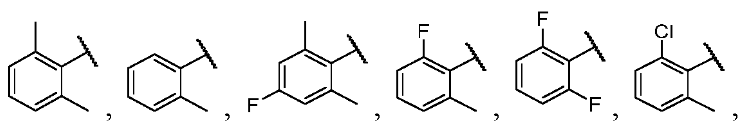

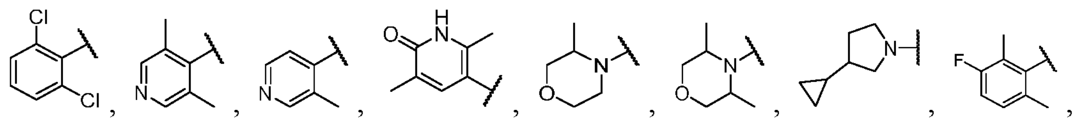

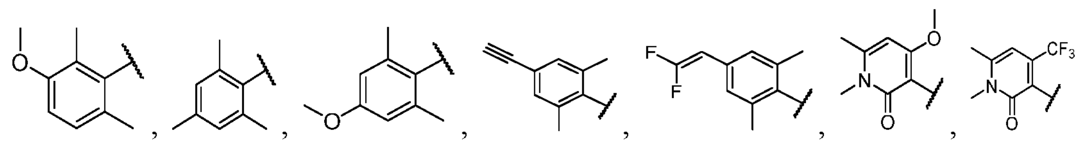

or

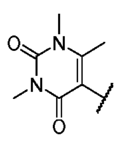;

or $R^2$ is selected from

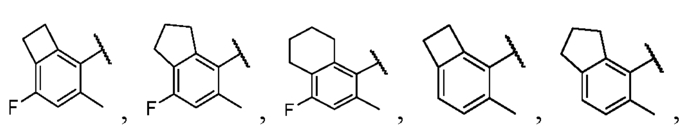

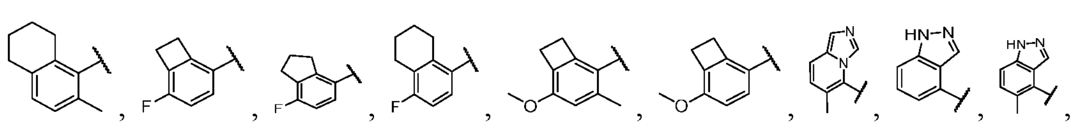

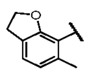, 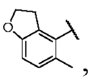, 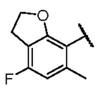 or 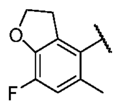;

or $R^2$ is selected from

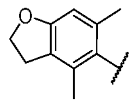, 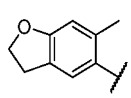, 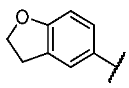, 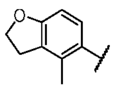 or

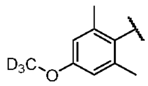;

$R^{1a}$ is selected from

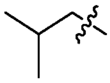, 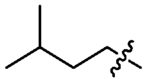 or 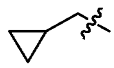;

the definitions of the remaining substituents are the same as those in any one of the first, second, third, fourth or fifth embodiment of the present invention.

As a seventh embodiment of the present invention, provided is the compound of above general formula (I) or a stereoisomer, mesomer/racemate, deuterated product, solvate, prodrug, metabolite, pharmaceutically acceptable salt, or co-crystal thereof, wherein the compound is selected from a compound of general formula (I-a), (I-b), (I-a-1) or (I-b-1),

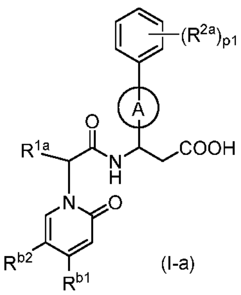 (I-a) 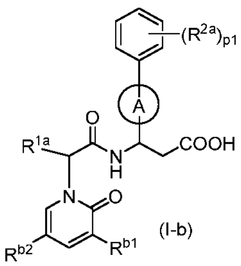 (I-b) 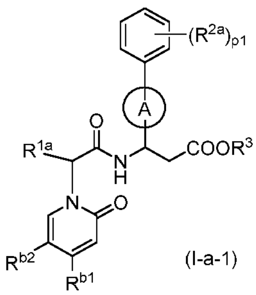 (I-a-1) 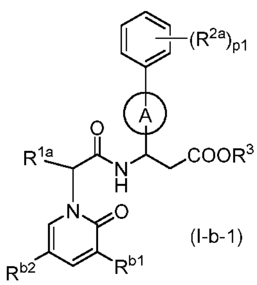 (I-b-1)

the definitions of ring A and $R^3$ are the same as those in any one of the second, third, fourth, fifth or sixth embodiment of the present invention;
p1 is selected from 0, 1, 2, 3 or 4;
$R^{2a}$ is selected from deuterium, halogen, OH, CN, $C_{1-4}$ alkyl, $C_{1-4}$ alkoxy, $C_{3-6}$ cycloalkyl or 4- to 6-membered heterocycloalkyl, wherein the alkyl, alkoxy, cycloalkyl or heterocycloalkyl is optionally substituted with 0 to 4 substituents selected from deuterium, halogen, OH, CN, $C_{1-4}$ alkyl or $C_{1-4}$ alkoxy, and the heterocycloalkyl contains 1 to 4 heteroatoms selected from O, S or N;
$R^{1a}$ is selected from

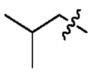, 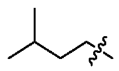 or 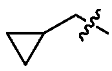;

$R^{b1}$ is selected from H, $C_{1-4}$ alkyl or $C_{3-6}$ cycloalkyl, wherein the alkyl or cycloalkyl is optionally substituted with 0 to 4 substituents selected from deuterium, halogen, OH, CN, $NHC_{1-4}$ alkyl, $N(C_{1-4}$ alkyl$)_2$, $C_{1-4}$ alkyl or $C_{1-4}$ alkoxy;
$R^{b2}$ is selected from H, $R^{ba}$, or one of the following substituted or unsubstituted groups: $C_{1-4}$ alkyl, $C_{2-4}$ alkynyl, $C_{1-4}$ alkoxy, $-CH_2CH_2-NHC_{1-4}$ alkyl, $-CH_2CH_2-N(C_{1-4}$ alkyl$)_2$, $-CH_2CH_2-C_{3-6}$ cycloalkyl, $-CH_2CH_2$-3- to 7-membered heterocycloalkyl, wherein the $CH_2$, alkyl, cycloalkyl or heterocycloalkyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $N(C_{1-4}$ alkyl$)(C_{3-6}$

cycloalkyl), NH($C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heteroaryl or heterocycloalkyl contains 1 to 4 heteroatoms selected from O, S or N;

n1 is selected from 1, 2 or 3;

the definitions of $R^{ba}$ and $R^k$ are the same as those in any one of the second, third, fourth, fifth or sixth embodiment of the present invention.

As an eighth embodiment of the present invention, provided is the compound of above general formula (I) or a stereoisomer, mesomer/racemate, deuterated product, solvate, prodrug, metabolite, pharmaceutically acceptable salt, or co-crystal thereof, wherein the compound is selected from a compound of general formula (I-a), (I-b), (I-a-1) or (I-b-1),

$R^{2a}$ is selected from deuterium, $CD_3$, $-OCD_3$, F, Cl, Br, I, OH, cyano, methyl, ethyl, methoxy or ethoxy;

$R^{b1}$ is selected from H, F, $CH_2F$, $CHF_2$, $CF_3$, methyl or $-CH_2CH_2N(CH_3)_2$;

$R^{b2}$ is selected from H, $R^{ba}$, or one of the following substituted or unsubstituted groups: methyl, ethyl, ethynyl, methoxy, ethoxy, $-CH_2NH(CH_2CH_3)$, $-CH_2N(CH_2CH_3)_2$, $-CH_2CH_2NH(CH_3)$, $-CH_2CH_2N(CH_3)_2$, $-CH_2CH_2NH(CH_2CH_3)$, $-CH_2CH_2N(CH_2CH_3)_2$, $-CH_2CH_2N(CH_3)(CH_2CH_3)$, $-CH_2CH_2$-cyclopropyl, - $CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, $-CH_2CH_2$-oxetanyl, $-CH_2CH_2$-oxolanyl, $-CH_2CH_2$-oxanyl or $-CH_2CH_2$-morpholine, which, when substituted, is substituted with 1, 2 or 3 substituents selected from deuterium, F, Cl, Br, OH, =O, cyano, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;

the definitions of the remaining groups are the same as those in the seventh embodiment of the present invention.

As a ninth embodiment of the present invention, provided is the compound of above general formula (I) or a stereoisomer, mesomer/racemate, deuterated product, solvate, prodrug, metabolite, pharmaceutically acceptable salt, or co-crystal thereof, wherein the compound is selected from a compound of general formula (I-f), (I-g), (I-f-1), (I-g-1), (I-h), (I-i), (I-h-1) or (I-i-1):

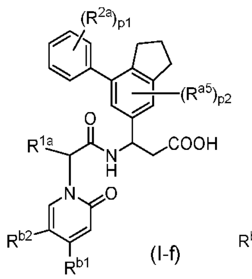
(I-f)
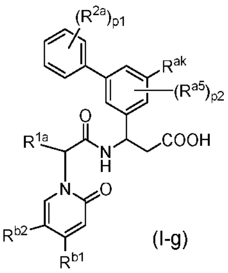
(I-g)
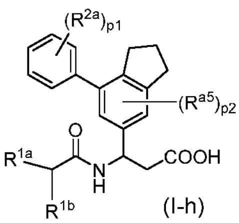
(I-h)
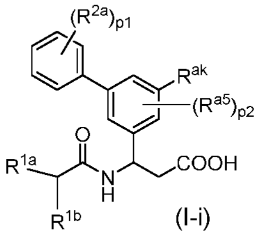
(I-i)

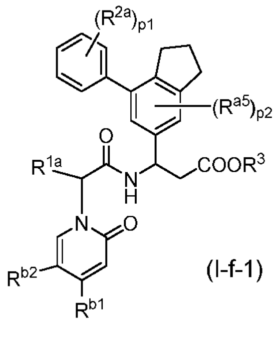
(I-f-1)
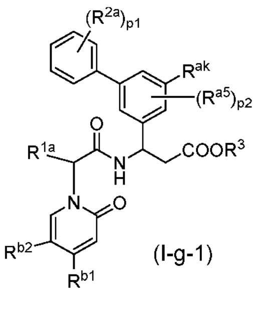
(I-g-1)
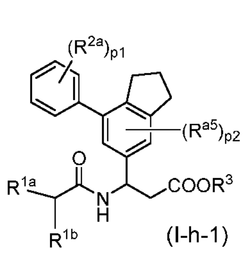
(I-h-1)
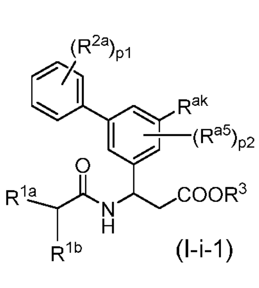
(I-i-1)

$R^{1b}$ is selected from

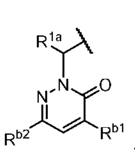
,
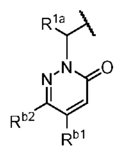
,
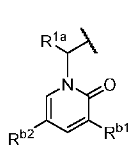
,
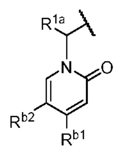
,
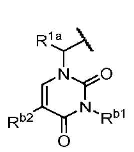
,

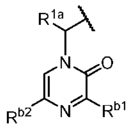 or 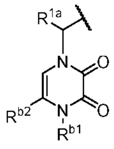;

wherein p2 is selected from 0, 1 or 2;

$R^{ak}$ is selected from $C_{2-4}$ alkynyl;

the definition of $R^{a5}$ is the same as that in any one of the first, second, third or fourth embodiment of the present invention;

the definitions of the remaining groups are the same as those in the seventh or eighth embodiment of the present invention.

As a tenth embodiment of the present invention, provided is the compound of above general formula (I) or a stereoisomer, mesomer/racemate, deuterated product, solvate, prodrug, metabolite, pharmaceutically acceptable salt, or co-crystal thereof, wherein the compound is selected from a compound of general formula (I-f), (I-g), (I-f-1), (I-g-1), (I-h), (I-i), (I-h-1) or (I-i-1);

$R^{ak}$ is selected from ethynyl, propynyl or propargyl;

$R^{a5}$ is selected from F, Cl, Br, I, OH, cyano, methyl, ethyl, methoxy, ethoxy or ethynyl;

the definitions of the remaining groups are the same as those in the ninth embodiment of the present invention.

As a tenth embodiment of the present invention, provided is the compound of above general formula (I) or a stereoisomer, mesomer/racemate, deuterated product, solvate, prodrug, metabolite, pharmaceutically acceptable salt, or co-crystal thereof, wherein the compound is selected from a compound of general formula (I-j) or (I-k);

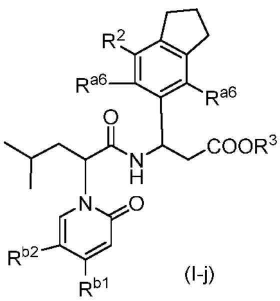
(I-j)

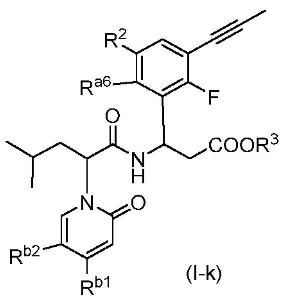
(I-k)

$R^{a6}$ is selected from H or F;

$R^2$ is selected from phenyl or

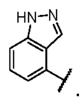;

and the $R^2$ is optionally substituted with 1 to 4 substituents selected from halogen, $C_{1-4}$ alkyl, $C_{1-4}$ alkoxy, 1 to 4 halogen(s)-substituted $C_{1-4}$ alkyl, 1 to 4 halogen(s)-substituted $C_{1-4}$ alkoxy, 1 to 4 deuterium(s)-substituted $C_{1-4}$ alkyl or 1 to 4 deuterium(s)-substituted $C_{1-4}$ alkoxy, preferably,

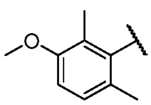, 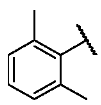, 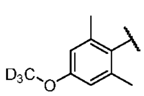, 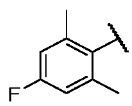, 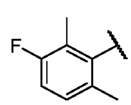, 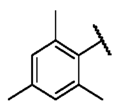,

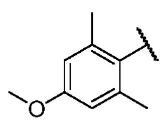 or 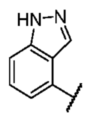;

$R^{b1}$ is selected from $CH_2F$, $CHF_2$, $CF_3$ or methyl, preferably $CF_3$;
$R^{b2}$ is selected from $-CH_2CH_2N(CH_3)_2$, $-CH_2CH_2NH(CH_2CH_3)$, $-CH_2$-azetidinyl, $-CH_2$-azolidinyl, $-CH_2$-azinanyl, $-CH_2$-morpholinyl, $-CH_2CH_2$-azetidinyl, $-CH_2CH_2$-azolidinyl, $-CH_2CH_2$-azinanyl or

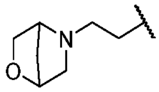,

and the $R^{b2}$ is optionally substituted with 1 to 4 substituents selected from deuterium, F, Cl, Br, $NHCH_2CH_3$, $N(CH_2CH_3)_2$, $CH_2F$, $CHF_2$, $CF_3$, methyl, ethyl, isopropyl, methoxy, ethoxy, $-CH_2$-cyclopropyl or

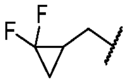,

preferably

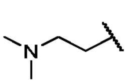, 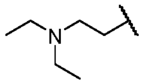,

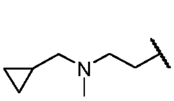, 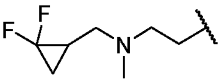, 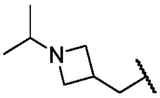, 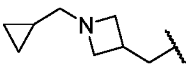, 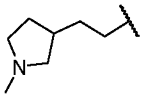,

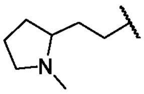, 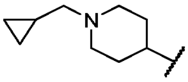, 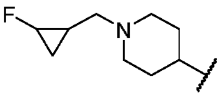, 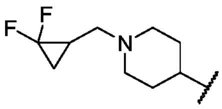, 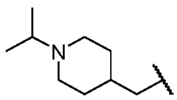,

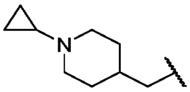, 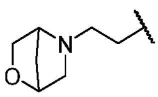, 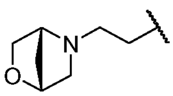, 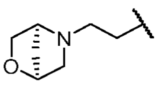, 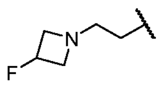,

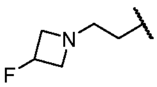, 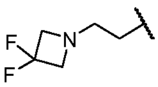, 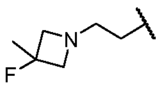, 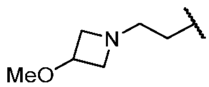 or 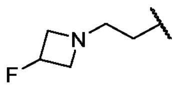.

The present invention relates to some specific compounds of general formula (I), wherein the compounds are selected from Table E-1.

The present invention relates to some specific compounds of general formula (I), wherein the compounds are selected from Table E-3, Table E-4 or table E-5.

Table E-1

| | | | | | |
|---|---|---|---|---|---|
| 1 | 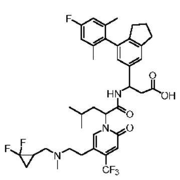 | 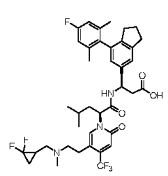 | 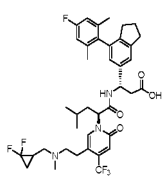 | 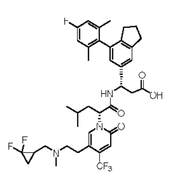 | 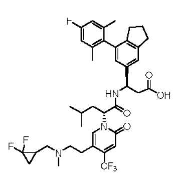 |
| 2 | 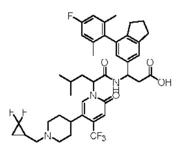 | 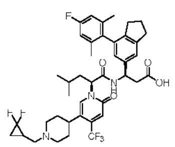 | 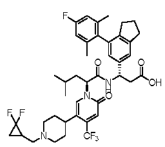 | 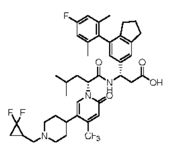 | 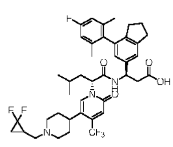 |

(continued)

| | | | | | |
|---|---|---|---|---|---|
| 3 | 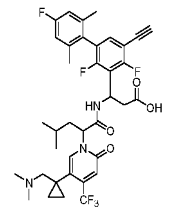 | 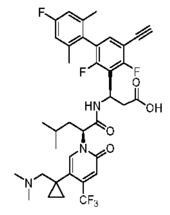 | 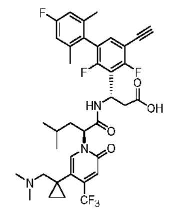 | 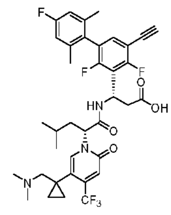 | 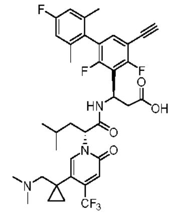 |
| 4 | 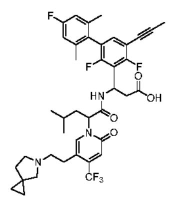 | 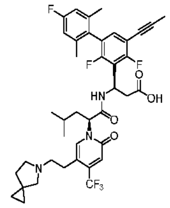 | 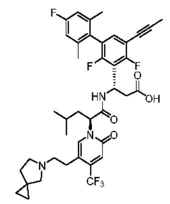 | 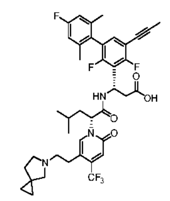 | 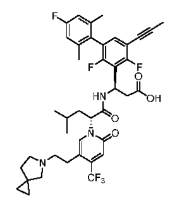 |
| 5 | 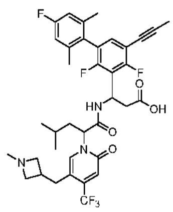 | 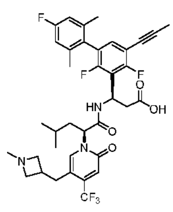 | 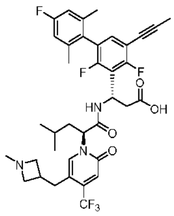 | 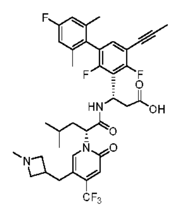 | 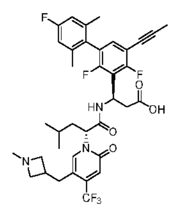 |
| 6 | 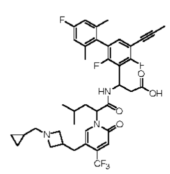 | 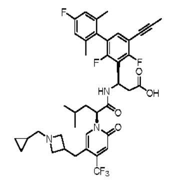 | 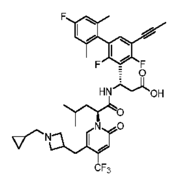 | 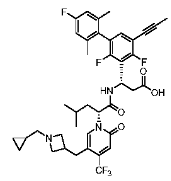 | 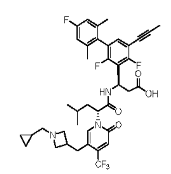 |
| 7 | 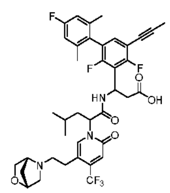 | 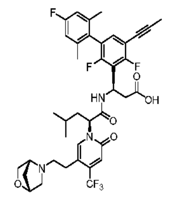 | 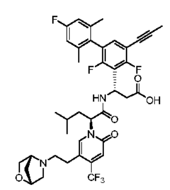 | 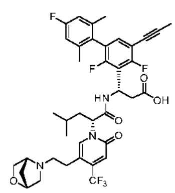 | 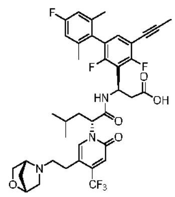 |
| 8 | 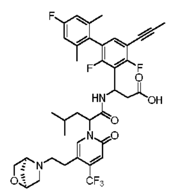 | 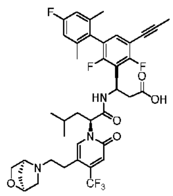 | 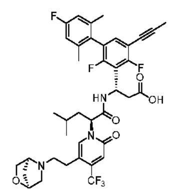 | 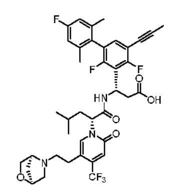 | 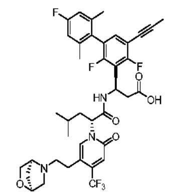 |
| 9 | 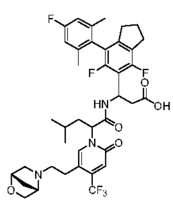 | 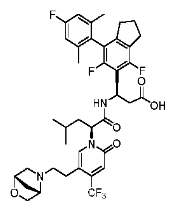 | 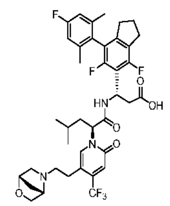 | 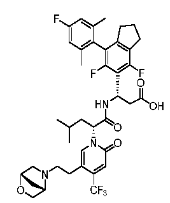 | 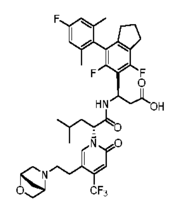 |

(continued)

| | | | | | |
|---|---|---|---|---|---|
| 10 | 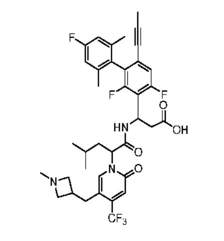 | 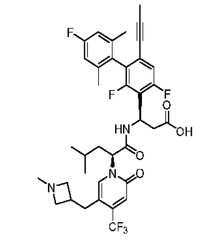 | 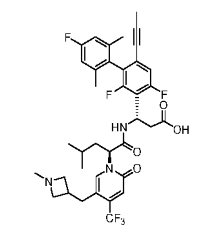 | 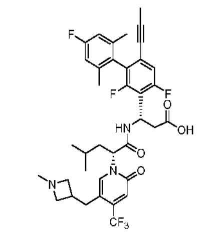 | 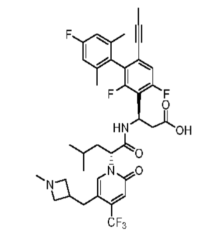 |
| 11 | 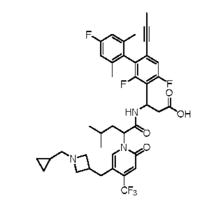 | 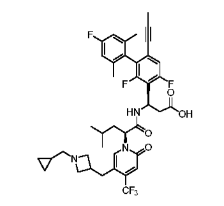 | 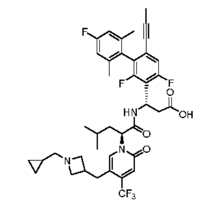 | 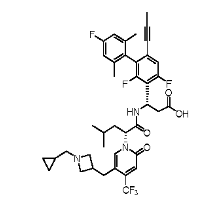 | 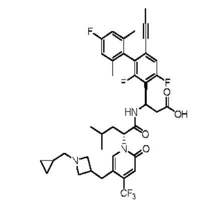 |
| 12 | 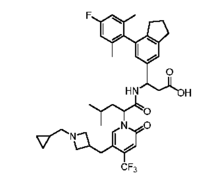 | 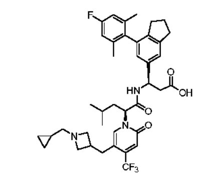 | 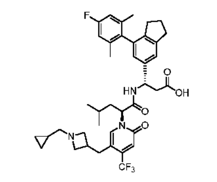 | 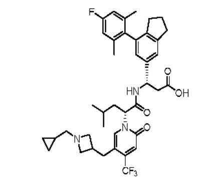 | 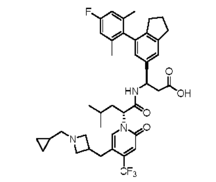 |
| 13 | 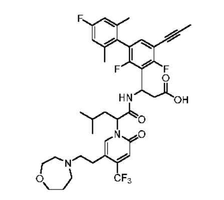 | 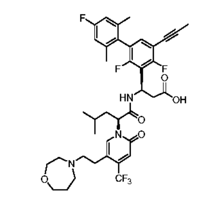 | 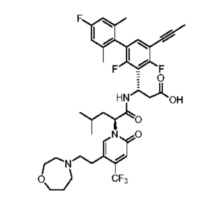 | 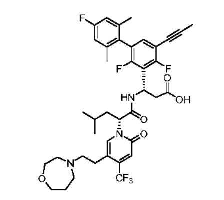 | 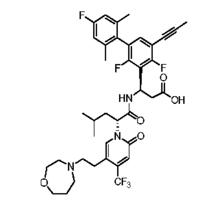 |
| 14 | 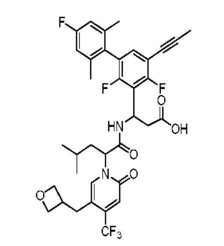 | 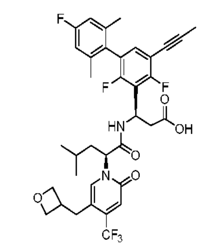 | 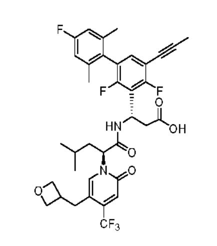 | 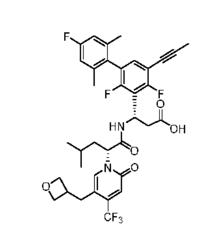 | 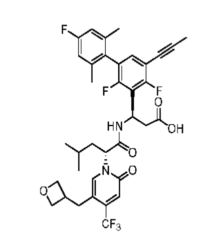 |
| 15 | 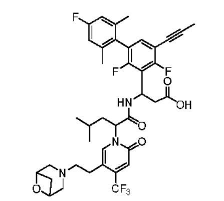 | 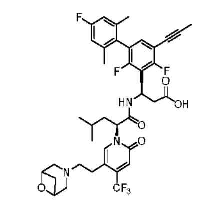 | 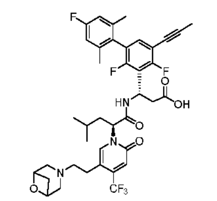 | 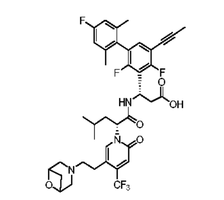 | 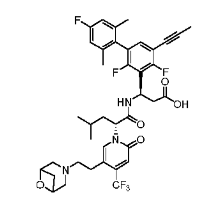 |
| 16 | 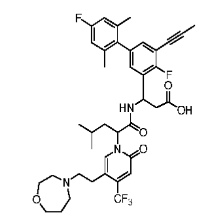 | 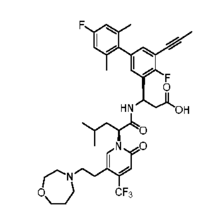 | 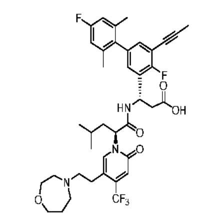 | 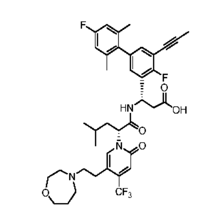 | 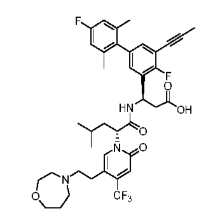 |

(continued)

| | | | | | |
|---|---|---|---|---|---|
| 17 | 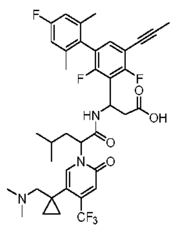 | 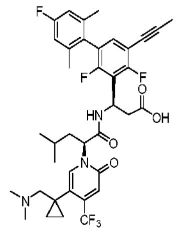 | 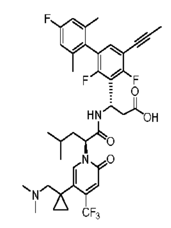 | 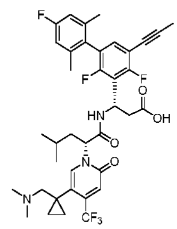 | 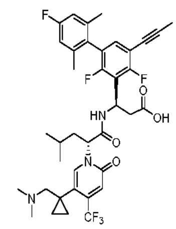 |
| 18 | 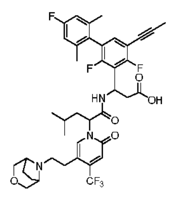 | 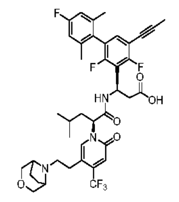 | 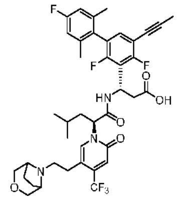 | 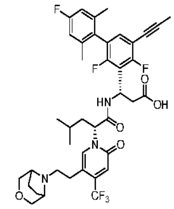 | 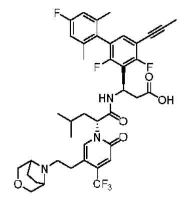 |
| 19 | 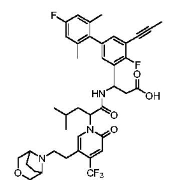 | 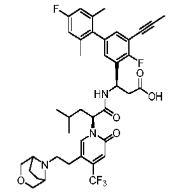 | 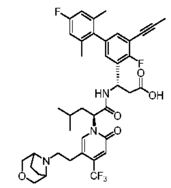 | 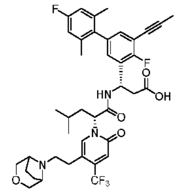 | 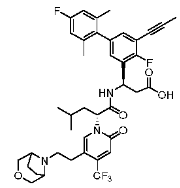 |
| 20 | 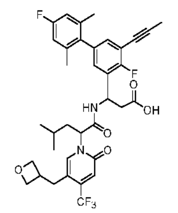 | 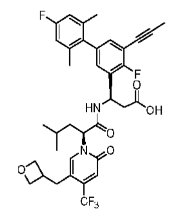 | 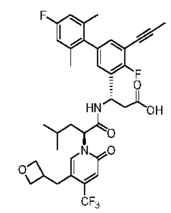 | 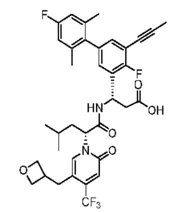 | 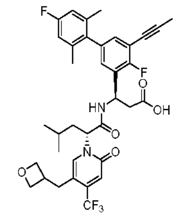 |
| 21 | 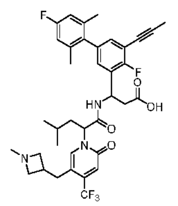 | 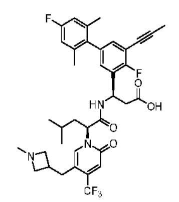 | 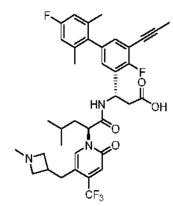 | 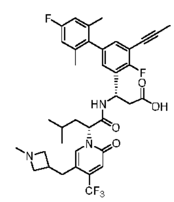 | 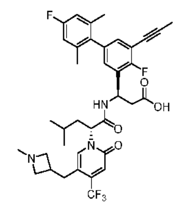 |
| 22 | 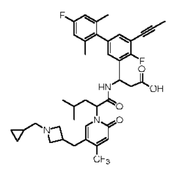 | 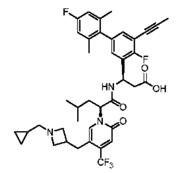 | 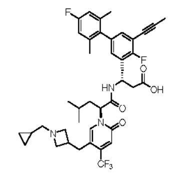 | 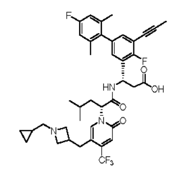 | 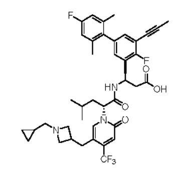 |
| 23 | 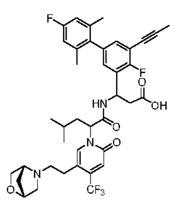 | 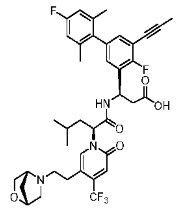 | 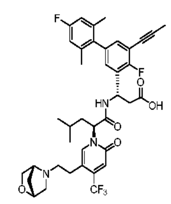 | 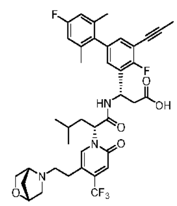 | 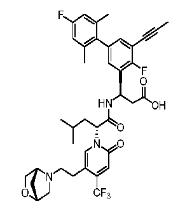 |

(continued)

| | | | | | |
|---|---|---|---|---|---|
| 24 | 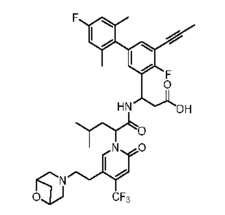 | 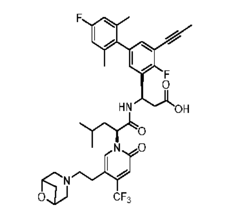 | 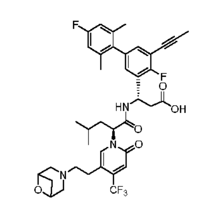 | 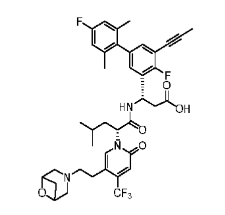 | 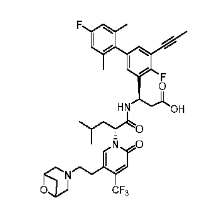 |
| 25 | 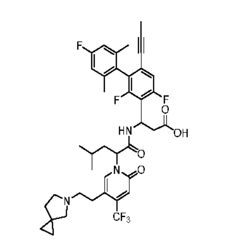 | 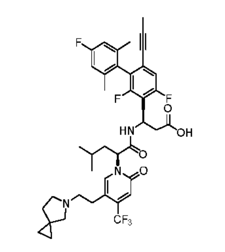 | 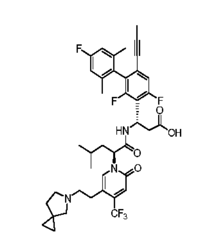 | 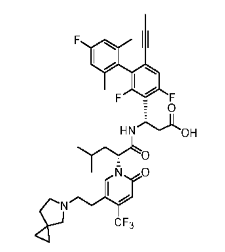 | 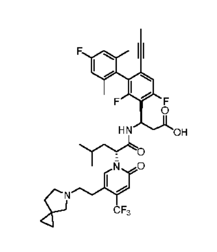 |
| 26 | 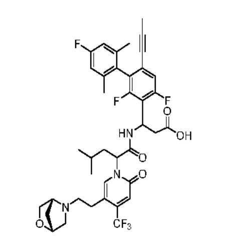 | 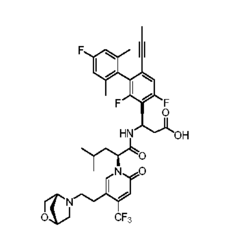 | 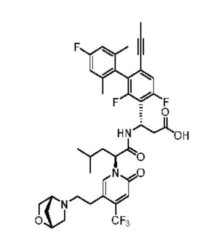 | 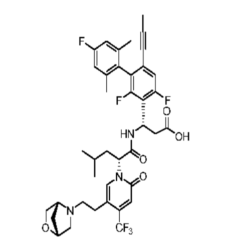 | 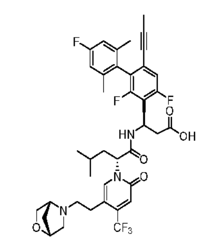 |
| 27 | 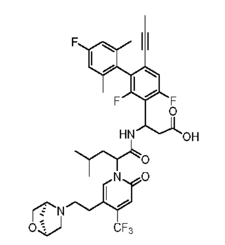 | 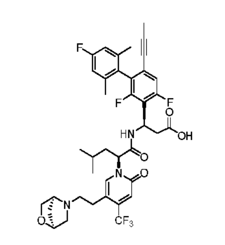 | 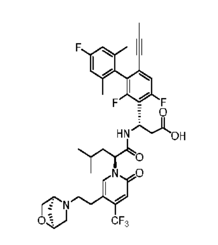 | 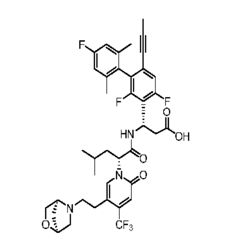 | 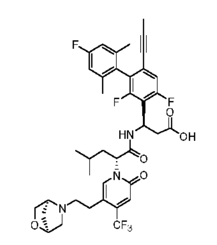 |
| 28 | 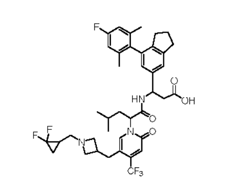 | 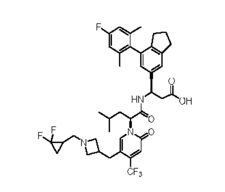 | 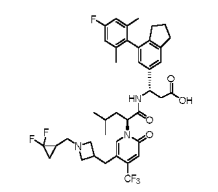 | 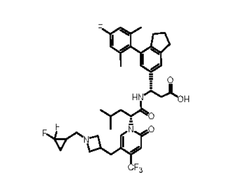 | 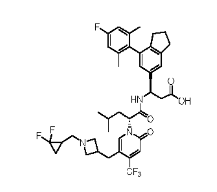 |
| 29 | 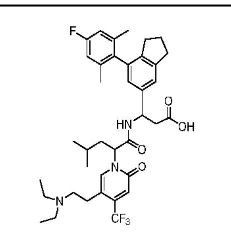 | 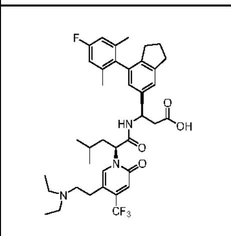 | 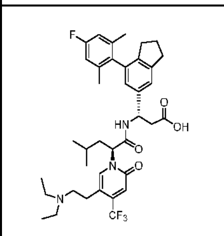 | 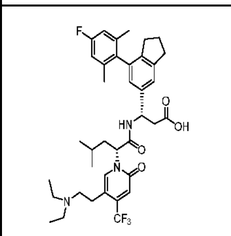 | 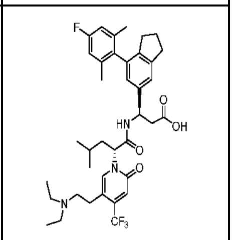 |
| 30 | 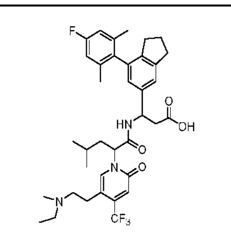 | 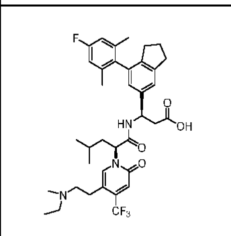 | 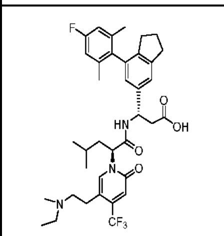 | 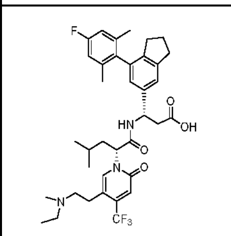 | 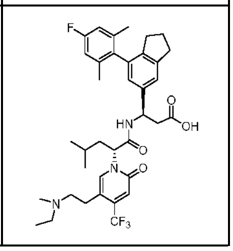 |

(continued)

| | | | | | |
|---|---|---|---|---|---|
| 31 | 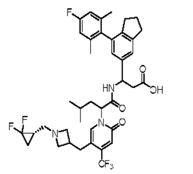 | 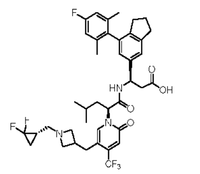 | 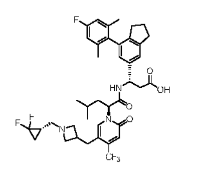 | 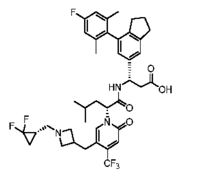 | 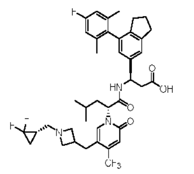 |
| 32 | 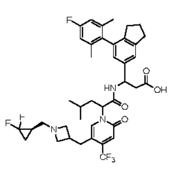 | 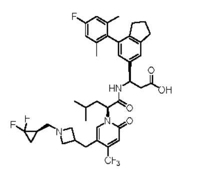 | 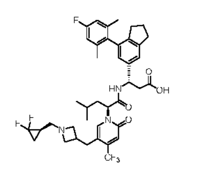 | 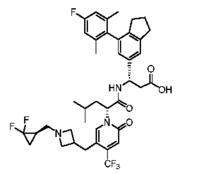 | 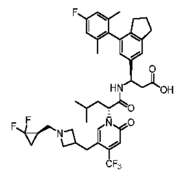 |
| 33 | 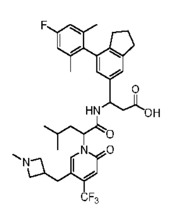 | 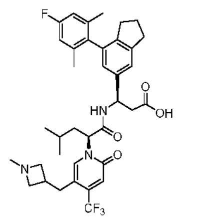 | 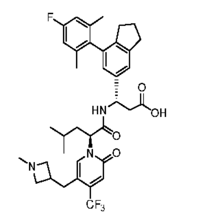 | 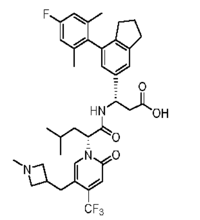 | 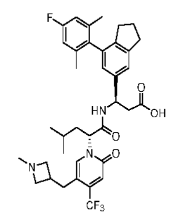 |
| 34 | 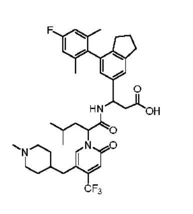 | 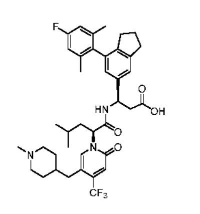 | 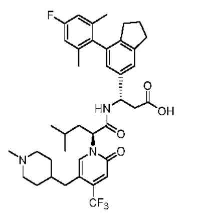 | 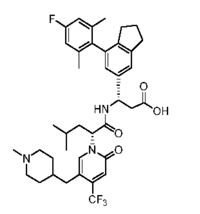 | 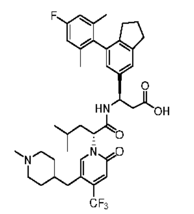 |
| 35 | 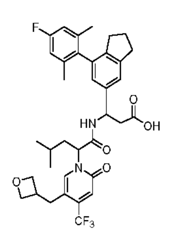 | 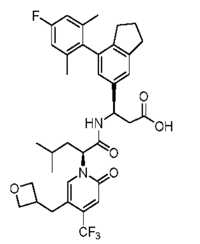 | 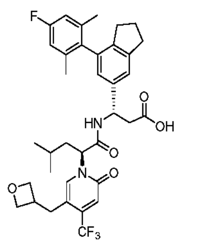 | 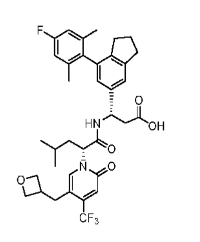 | 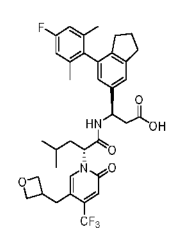 |
| 36 | 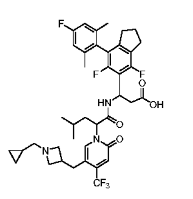 | 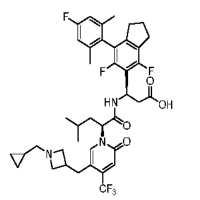 | 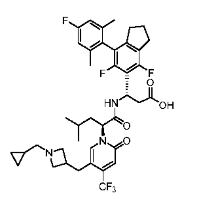 | 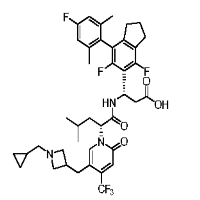 | 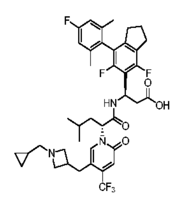 |
| 37 | 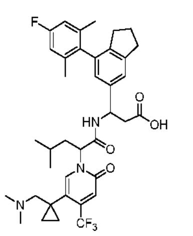 | 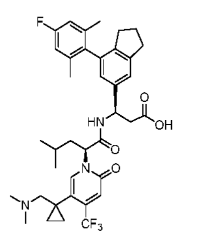 | 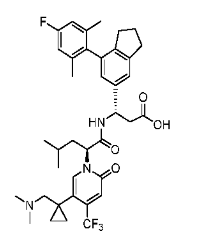 | 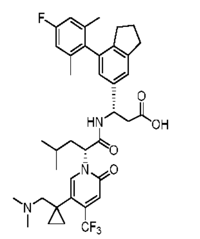 | 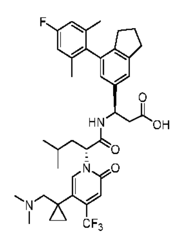 |

(continued)

| | | | | | |
|---|---|---|---|---|---|
| 38 | 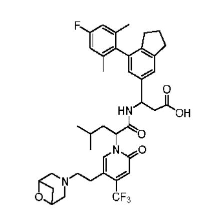 | 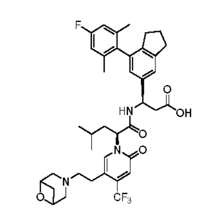 | 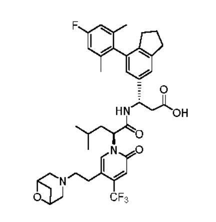 | 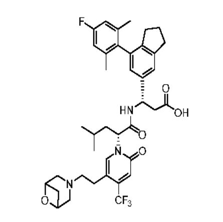 | 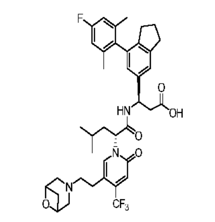 |
| 39 | 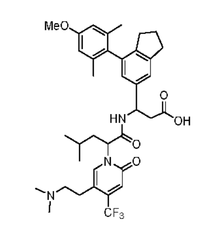 | 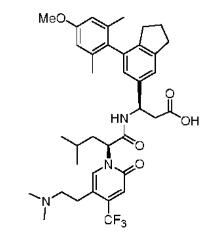 | 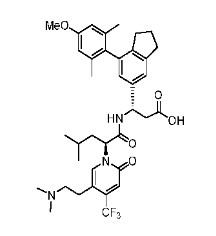 | 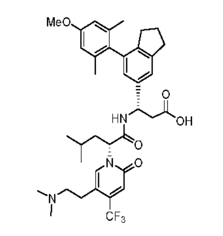 | 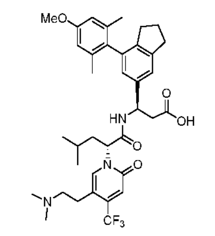 |
| 40 | 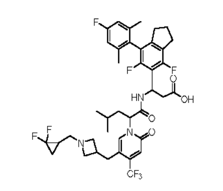 | 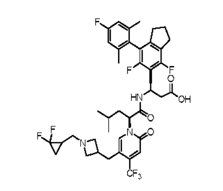 | 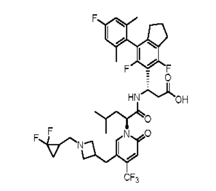 | 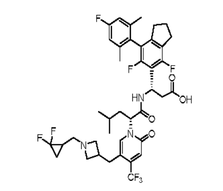 | 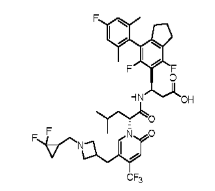 |
| 41 | 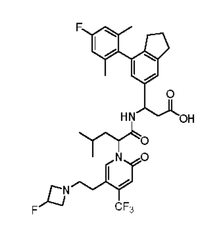 | 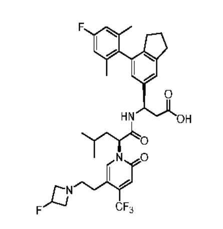 | 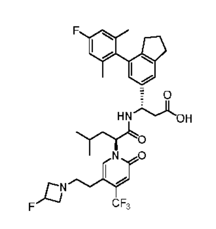 | 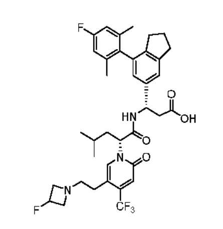 | 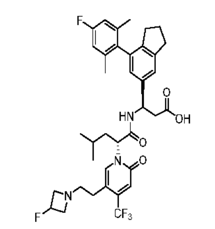 |
| 42 | 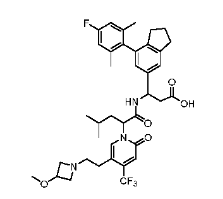 | 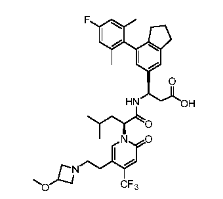 | 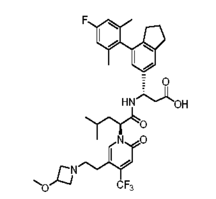 | 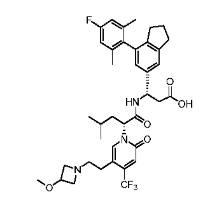 | 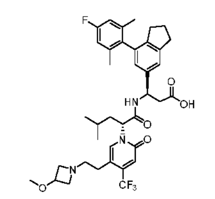 |
| 43 | 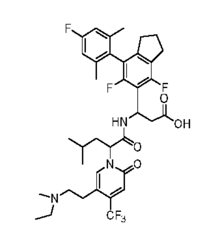 | 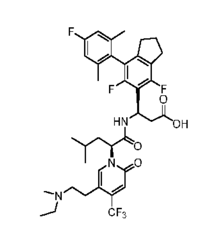 | 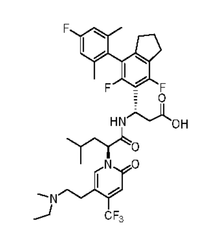 | 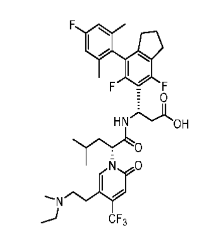 | 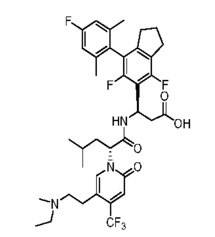 |
| 44 | 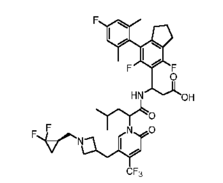 | 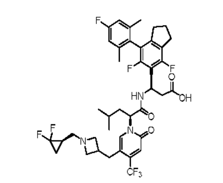 | 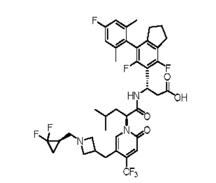 | 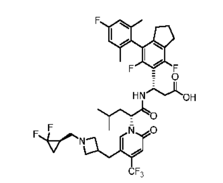 | 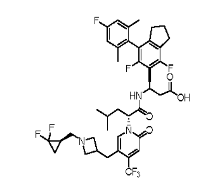 |

(continued)

| | | | | | |
|---|---|---|---|---|---|
| 45 | 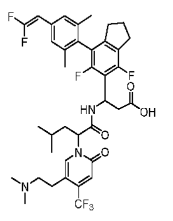 | 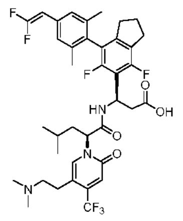 | 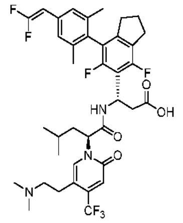 | 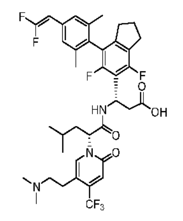 | 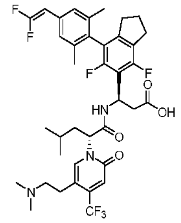 |
| 46 | 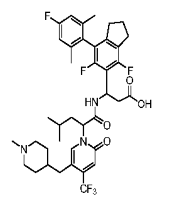 | 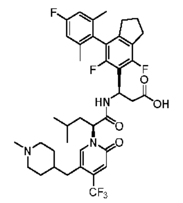 | 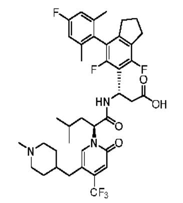 | 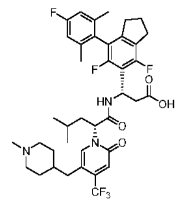 | 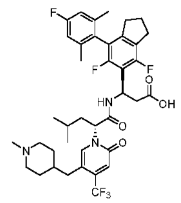 |
| 47 | 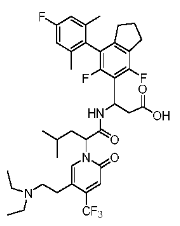 | 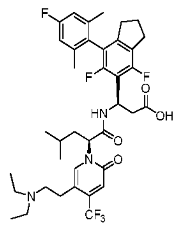 | 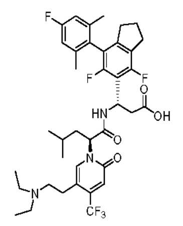 | 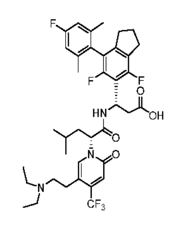 | 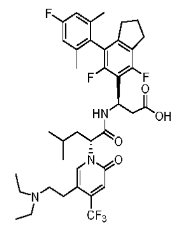 |
| 48 | 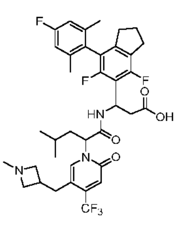 | 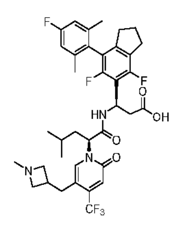 | 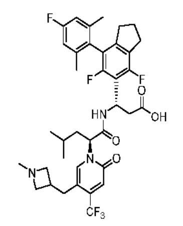 | 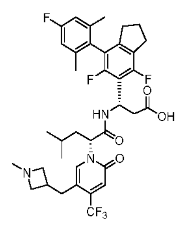 | 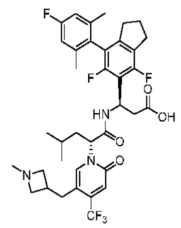 |
| 49 | 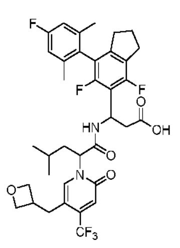 | 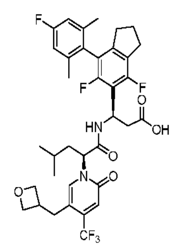 | 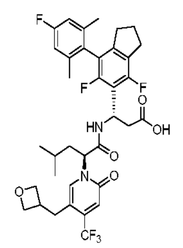 | 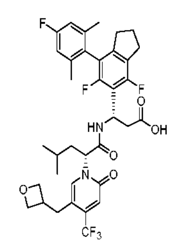 | 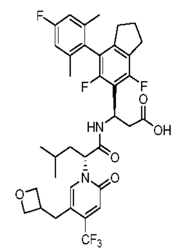 |
| 50 | 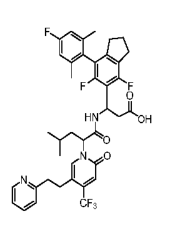 | 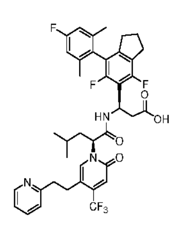 | 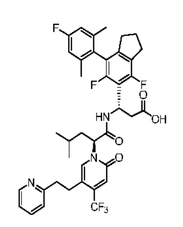 | 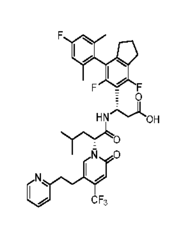 | 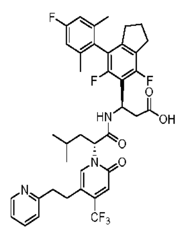 |
| 51 | 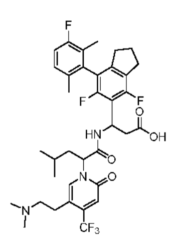 | 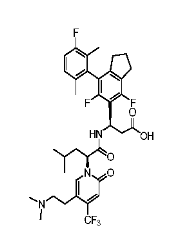 | 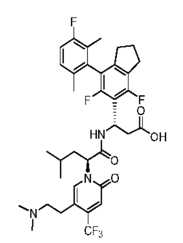 | 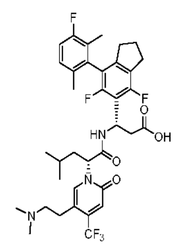 | 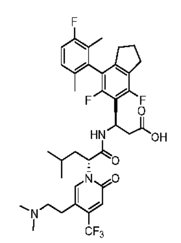 |

(continued)

| | | | | | |
|---|---|---|---|---|---|
| 52 | 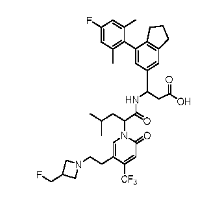 | 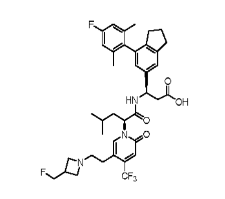 | 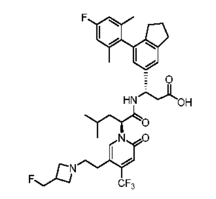 | 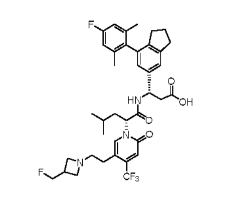 | 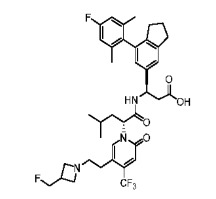 |
| 53 | 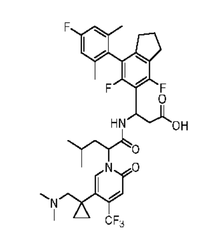 | 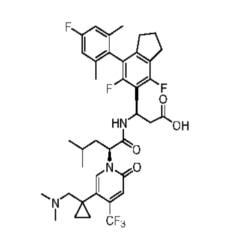 | 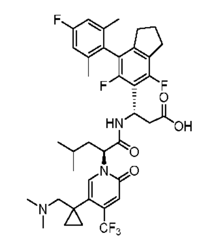 | 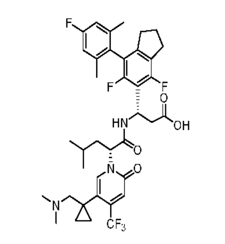 | 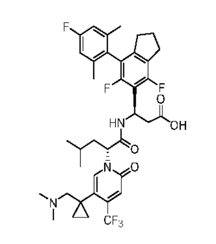 |
| 54 | 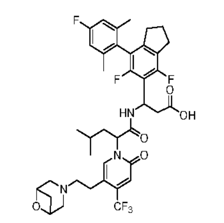 | 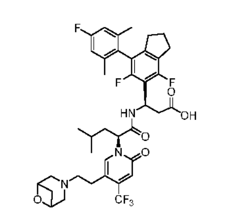 | 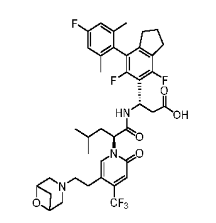 | 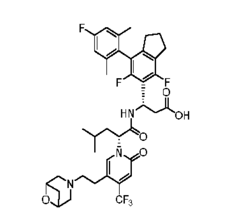 | 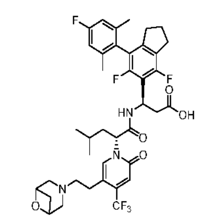 |
| 55 | 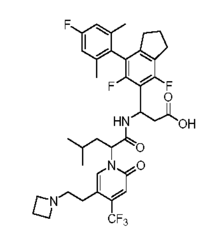 | 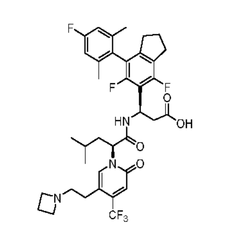 | 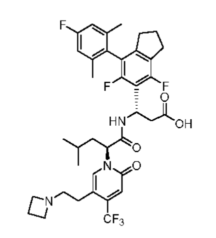 | 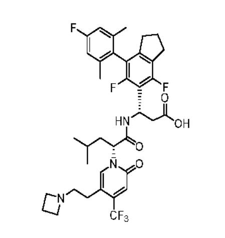 | 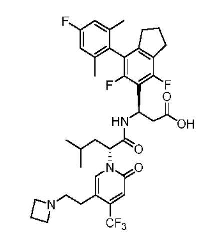 |
| 56 | 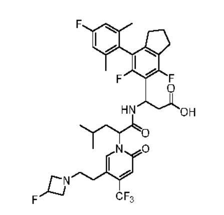 | 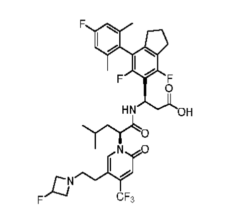 | 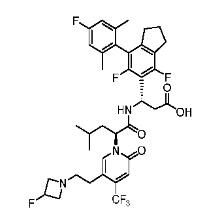 | 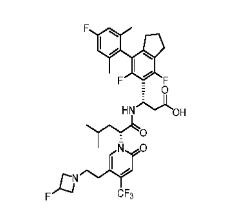 | 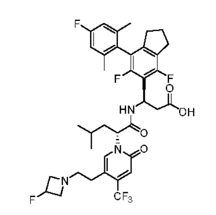 |
| 57 | 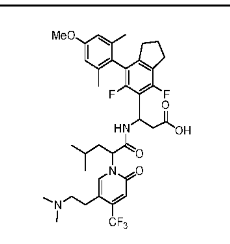 | 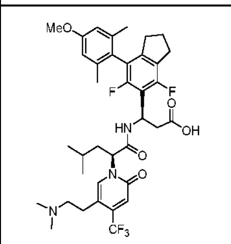 | 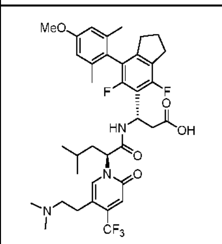 | 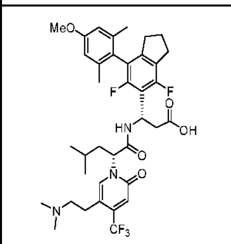 | 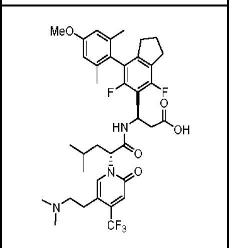 |
| 58 | 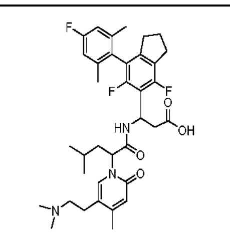 | 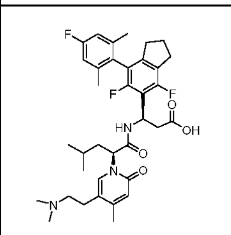 | 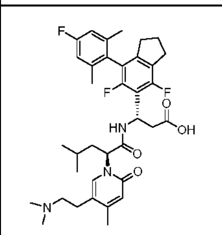 | 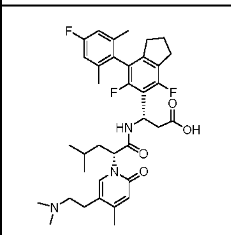 | 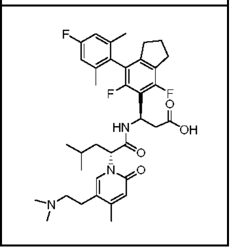 |

(continued)

| | | | | | |
|---|---|---|---|---|---|
| 59 | 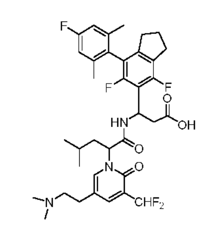 | 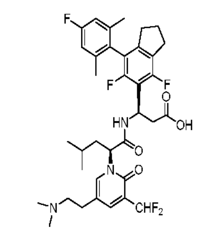 | 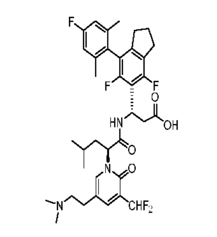 | 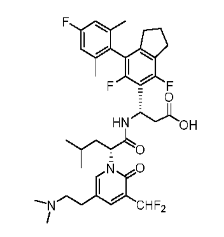 | 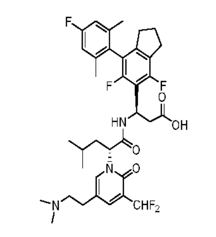 |
| 60 | 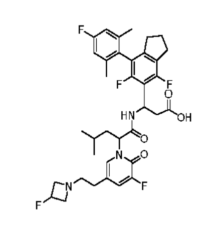 | 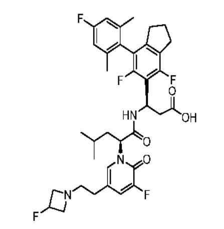 | 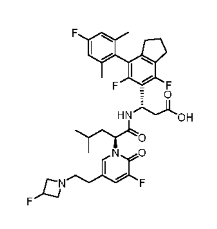 | 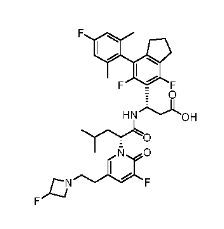 | 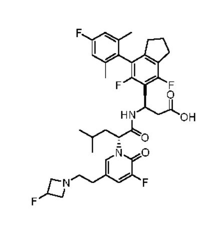 |
| 61 | 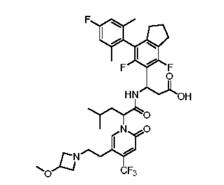 | 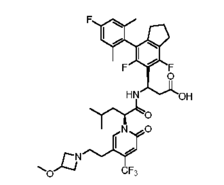 | 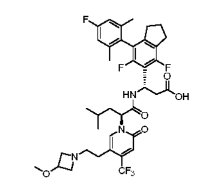 | 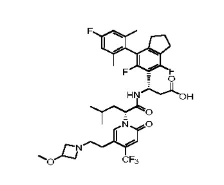 | 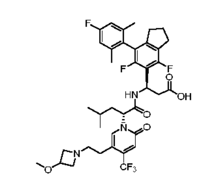 |
| 62 | 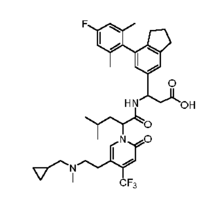 | 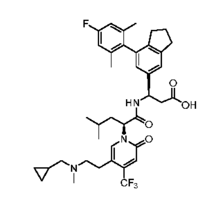 | 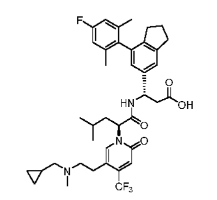 | 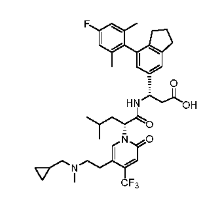 | 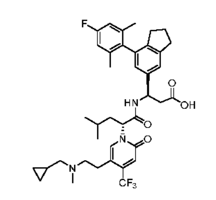 |
| 63 | 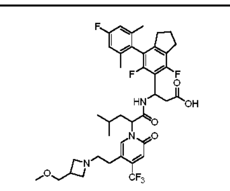 | 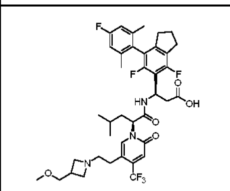 | 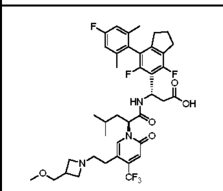 | 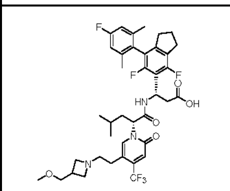 | 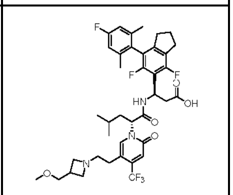 |
| 64 | 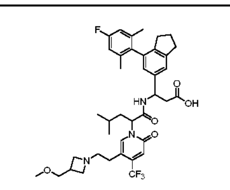 | 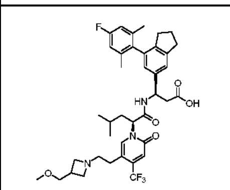 | 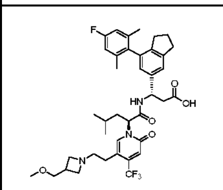 | 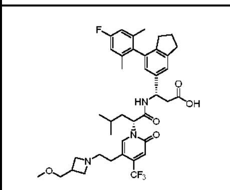 | 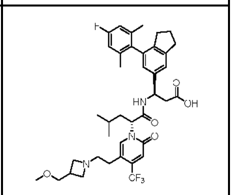 |
| 65 | 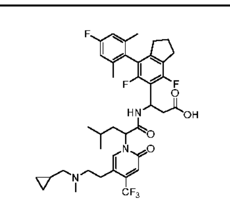 | 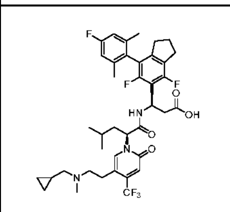 | 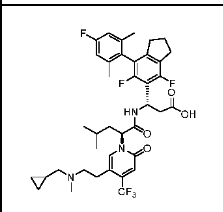 | 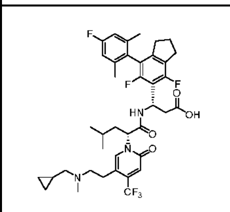 | 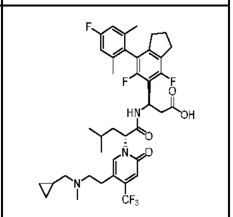 |
| 66 | 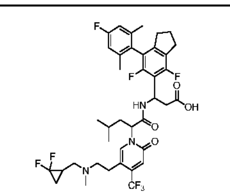 | 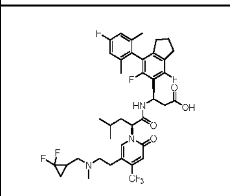 | 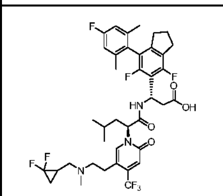 | 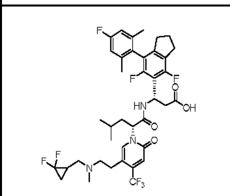 | 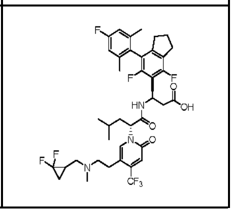 |

(continued)

| | | | | | |
|---|---|---|---|---|---|
| 67 | 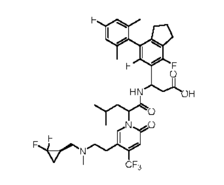 | 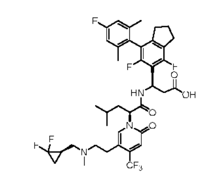 | 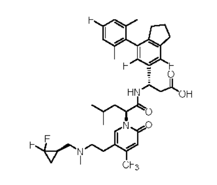 | 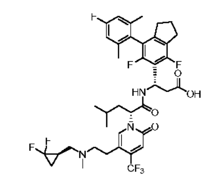 | 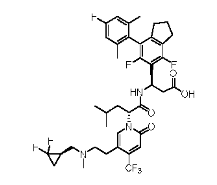 |
| 68 | 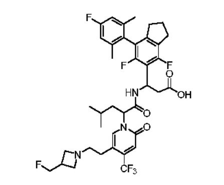 | 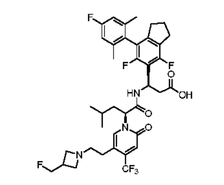 | 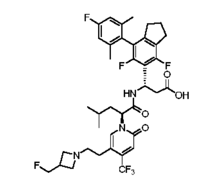 | 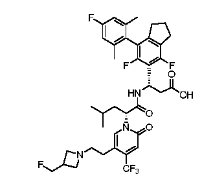 | 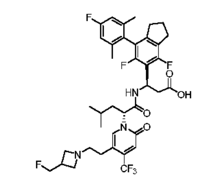 |
| 69 | 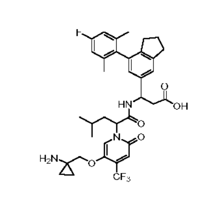 | 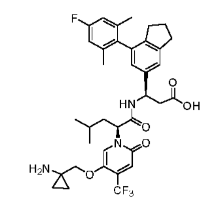 | 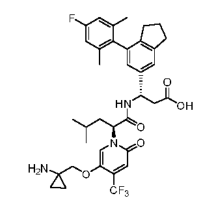 | 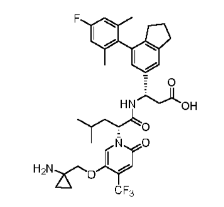 | 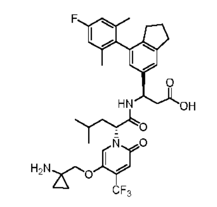 |
| 70 | 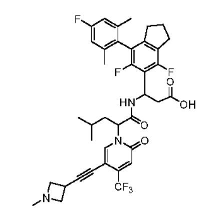 | 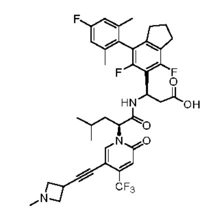 | 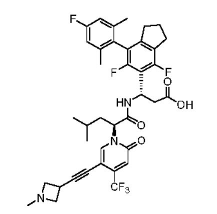 | 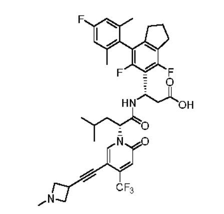 | 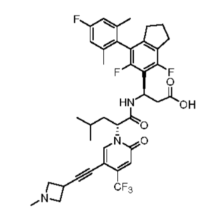 |
| 71 | 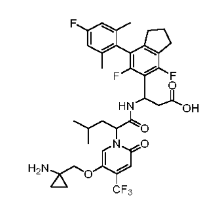 | 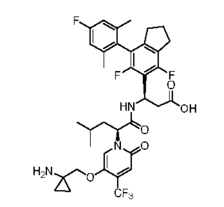 | 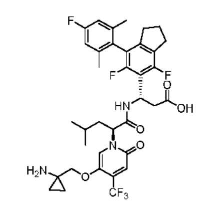 | 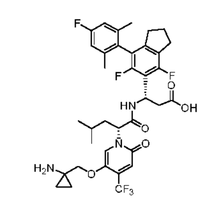 | 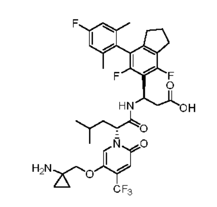 |
| 72 | 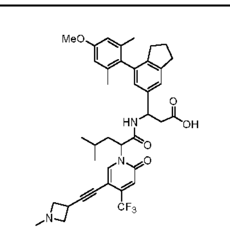 | 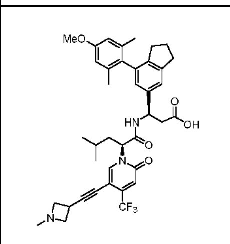 | 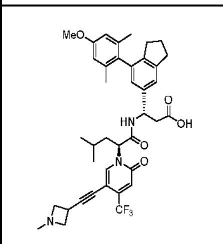 | 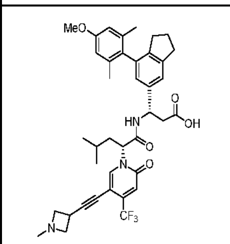 | 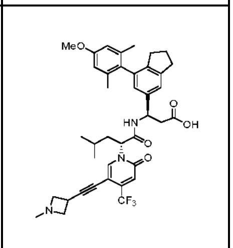 |
| 73 | 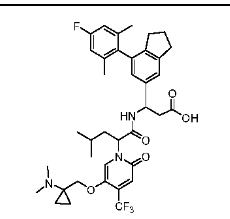 | 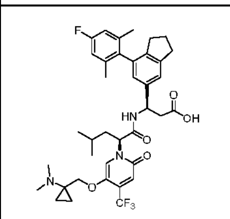 | 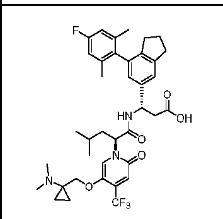 | 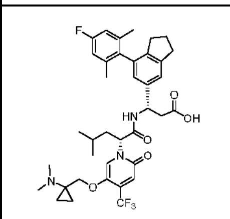 | 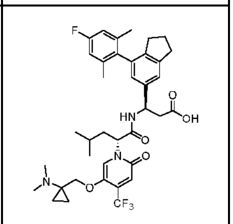 |
| 74 | 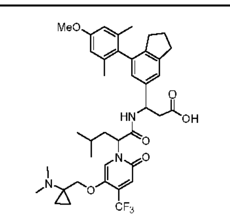 | 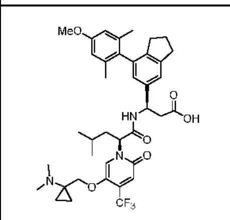 | 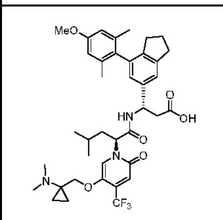 | 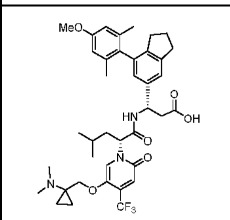 | 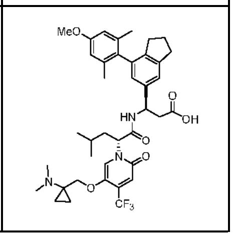 |

(continued)

| | | | | | |
|---|---|---|---|---|---|
| 75 | 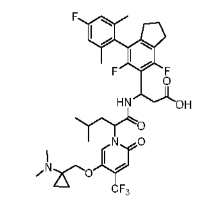 | 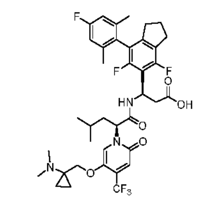 | 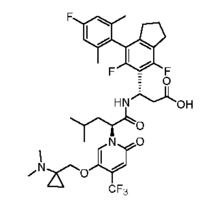 | 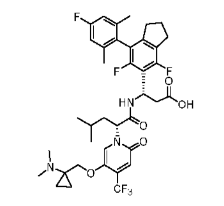 | 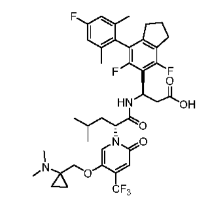 |
| 76 | 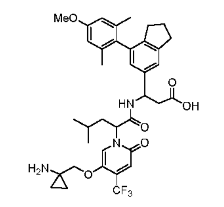 | 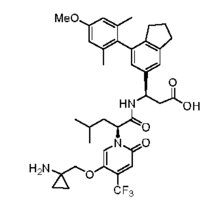 | 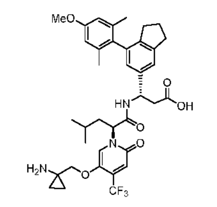 | 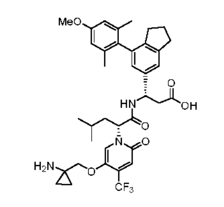 | 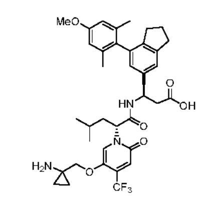 |
| 77 | 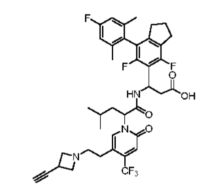 | 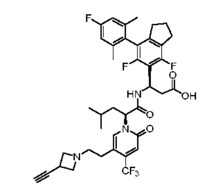 | 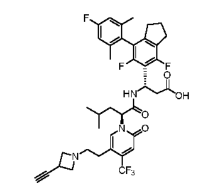 | 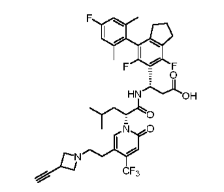 | 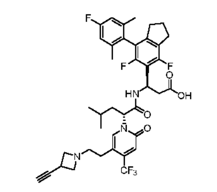 |
| 78 | 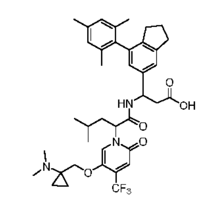 | 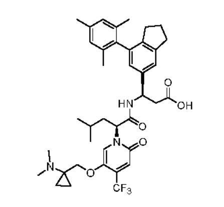 | 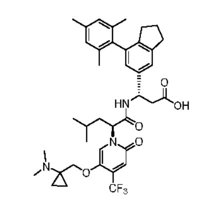 | 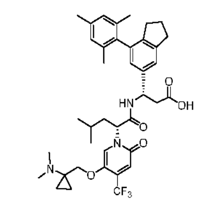 | 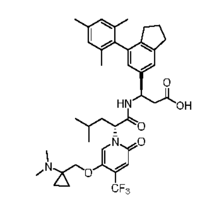 |
| 79 | 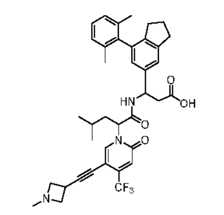 | 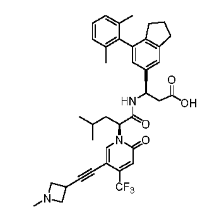 | 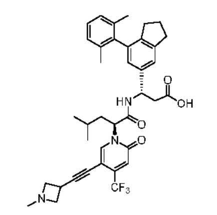 | 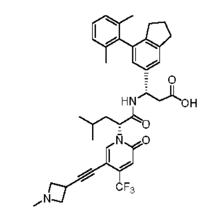 | 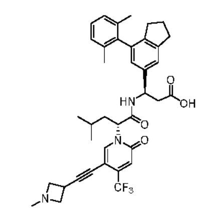 |
| 80 | 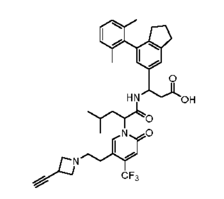 | 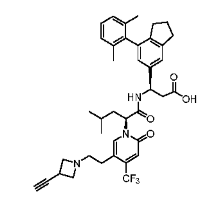 | 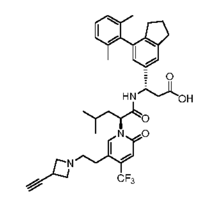 | 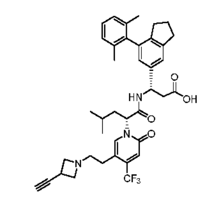 | 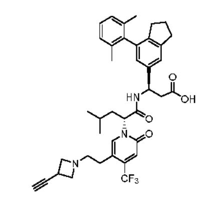 |
| 81 | 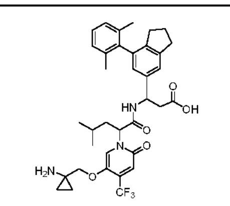 | 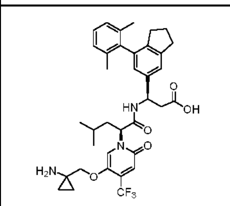 | 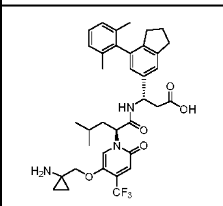 | 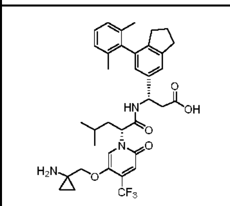 | 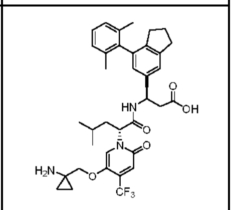 |
| 82 | 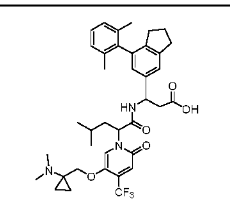 | 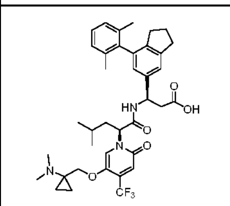 | 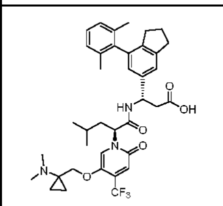 | 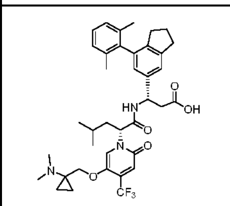 | 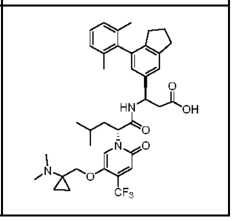 |

(continued)

| | | | | | |
|---|---|---|---|---|---|
| 83 | 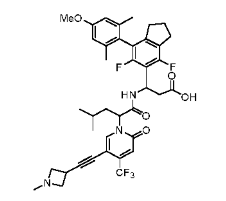 | 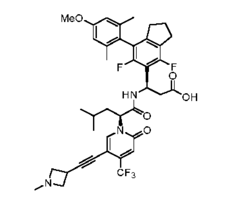 | 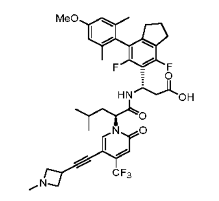 | 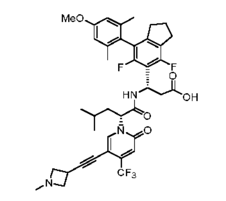 | 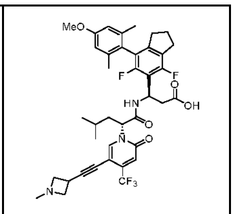 |
| 84 | 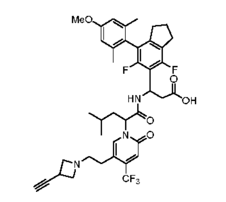 | 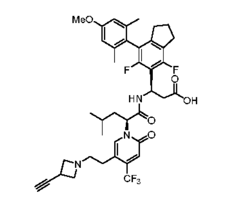 | 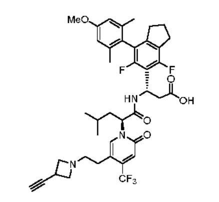 | 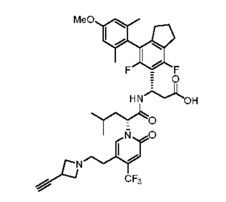 | 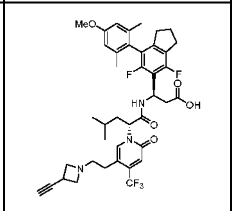 |
| 85 | 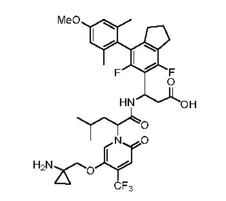 | 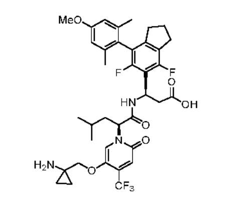 | 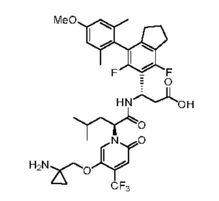 | 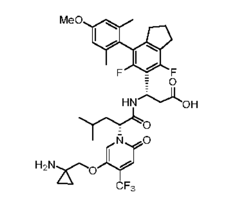 | 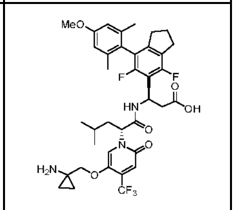 |
| 86 | 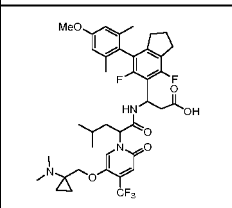 | 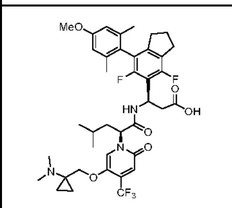 | 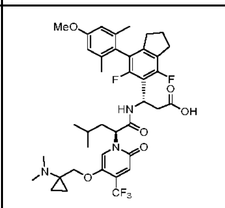 | 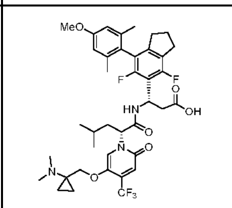 | 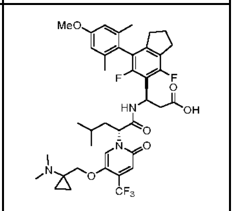 |
| 87 | 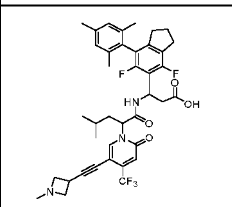 | 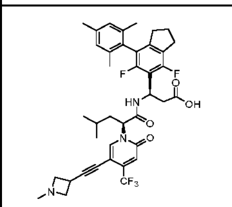 | 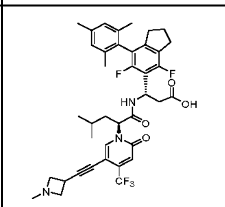 | 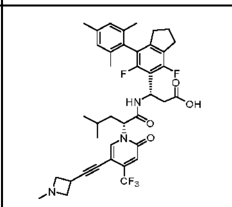 | 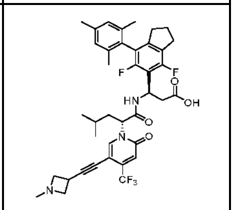 |
| 88 | 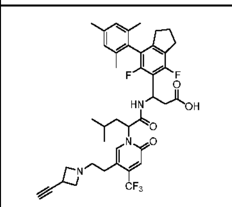 | 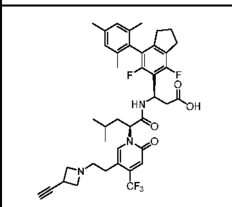 | 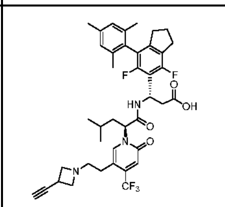 | 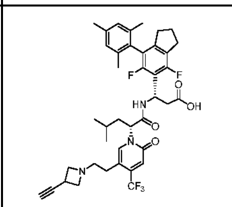 | 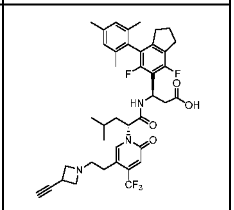 |
| 89 | 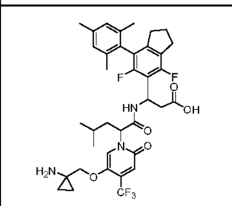 | 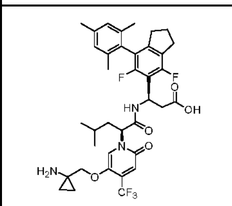 | 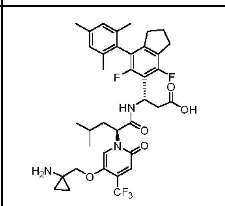 | 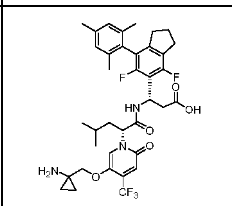 | 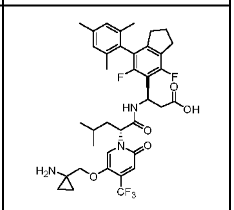 |
| 90 | 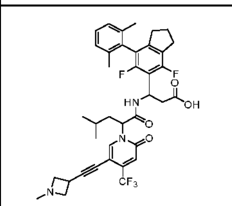 | 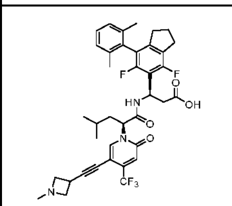 | 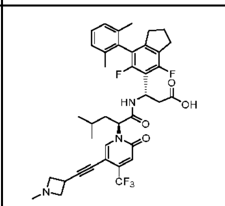 | 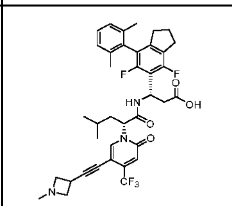 | 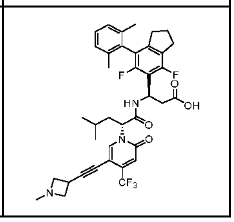 |

(continued)

| | | | | | |
|---|---|---|---|---|---|
| 91 | 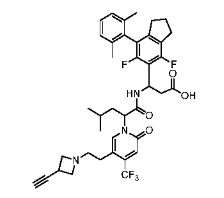 | 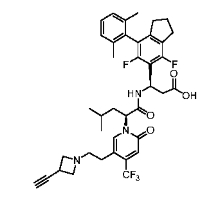 | 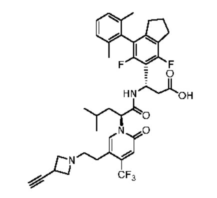 | 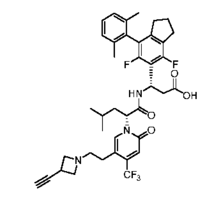 | 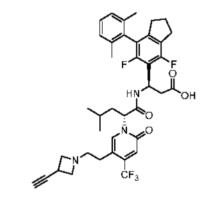 |
| 92 | 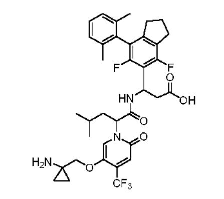 | 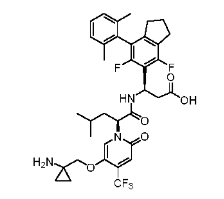 | 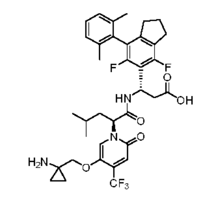 | 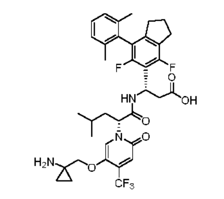 | 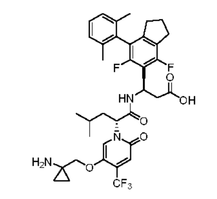 |
| 93 | 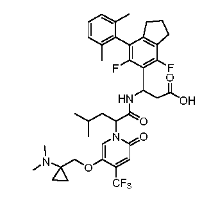 | 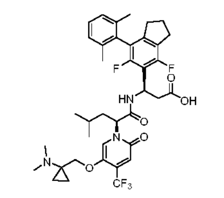 | 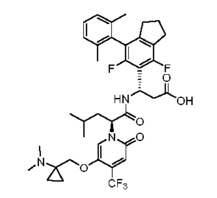 | 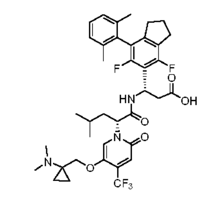 | 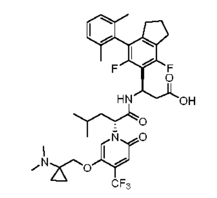 |
| 94 | 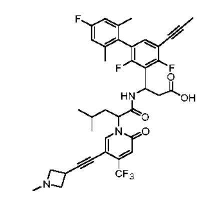 | 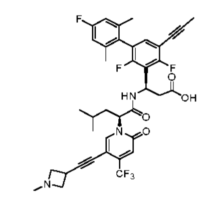 | 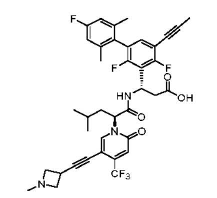 | 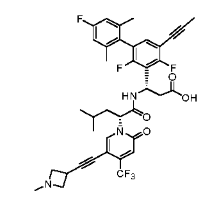 | 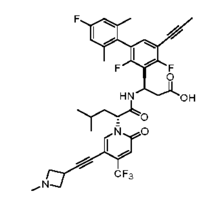 |
| 95 | 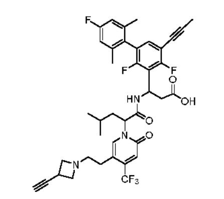 | 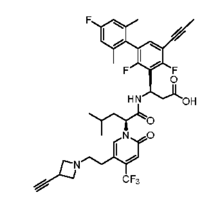 | 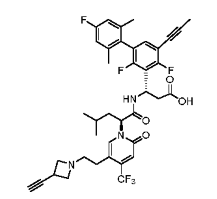 | 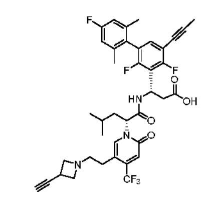 | 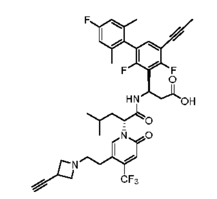 |
| 96 | 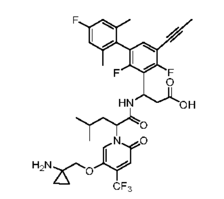 | 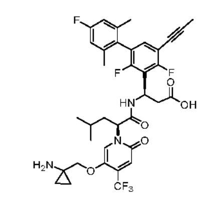 | 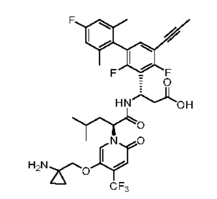 | 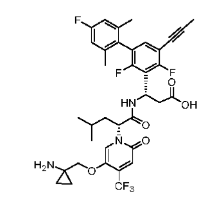 | 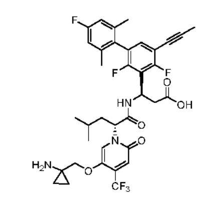 |
| 97 | 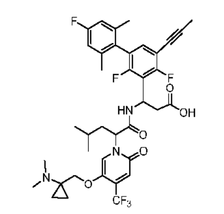 | 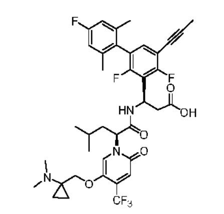 | 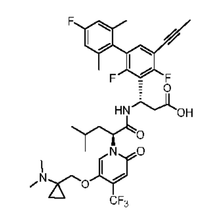 | 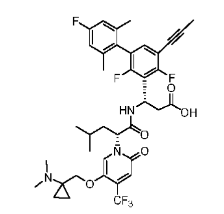 | 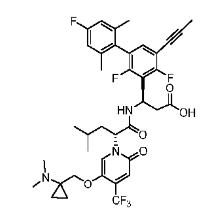 |
| 98 | 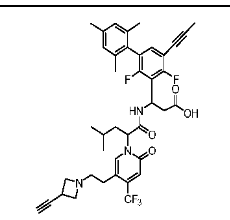 | 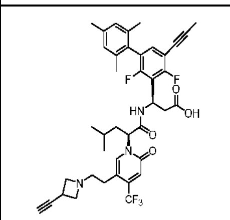 | 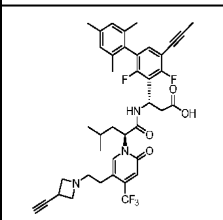 | 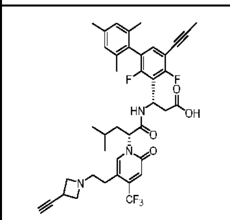 | 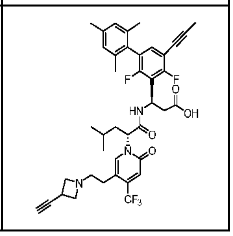 |

(continued)

| | | | | | |
|---|---|---|---|---|---|
| 99 | 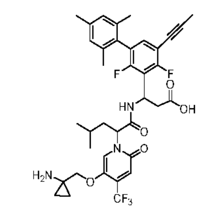 | 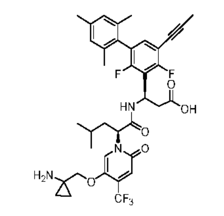 | 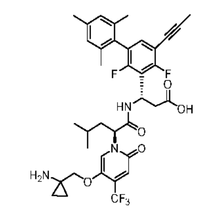 | 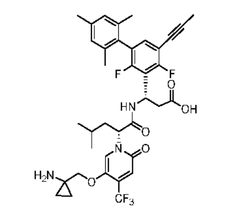 | 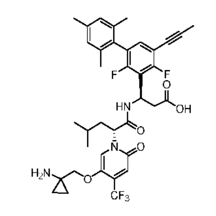 |
| 100 | 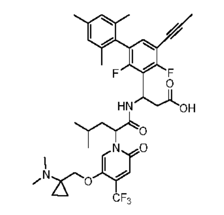 | 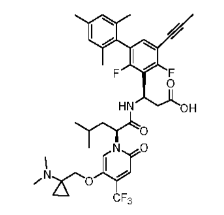 | 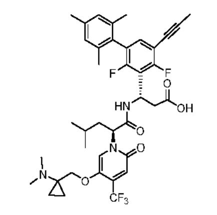 | 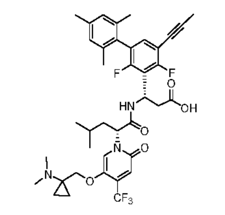 | 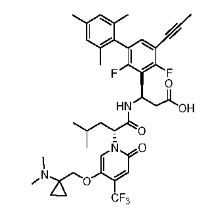 |
| 101 | 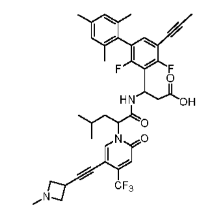 | 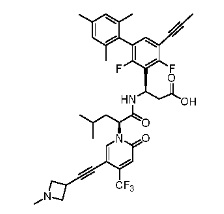 | 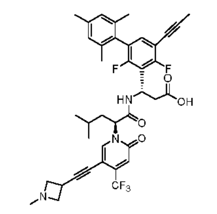 | 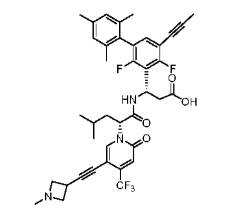 | 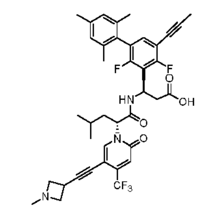 |
| 102 | 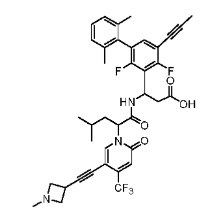 | 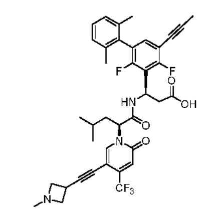 | 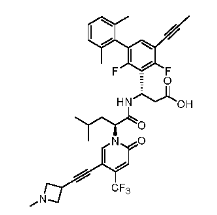 | 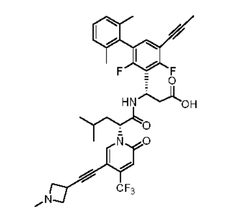 | 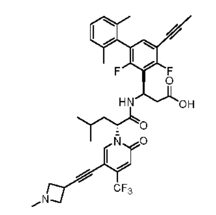 |
| 103 | 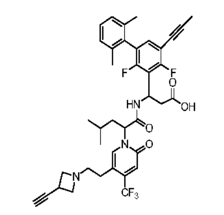 | 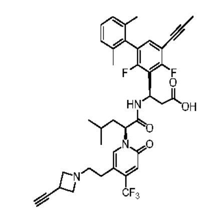 | 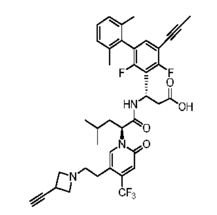 | 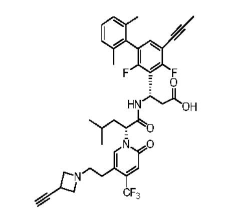 | 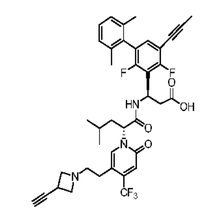 |
| 104 | 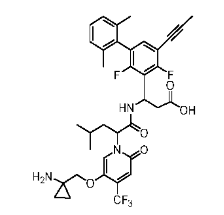 | 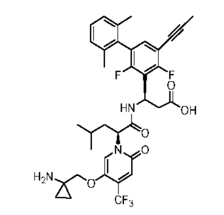 | 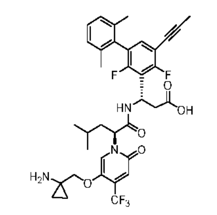 | 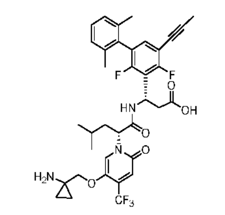 | 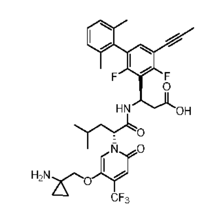 |
| 105 | 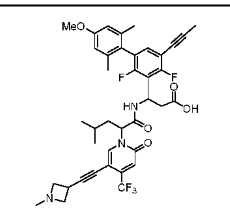 | 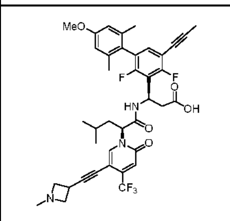 | 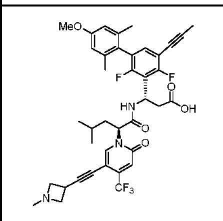 | 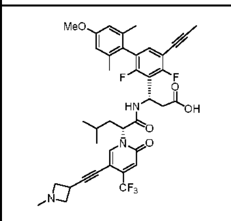 | 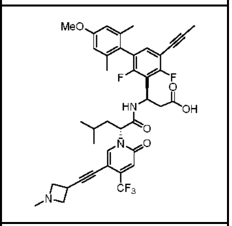 |
| 106 | 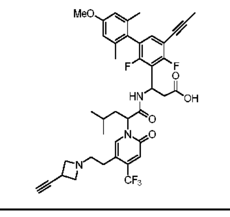 | 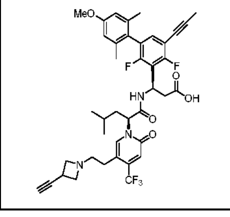 | 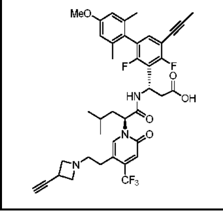 | 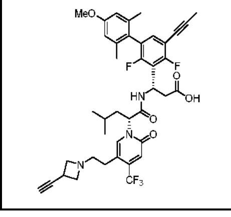 | 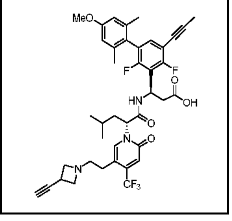 |

(continued)

| | | | | | |
|---|---|---|---|---|---|
| 107 | 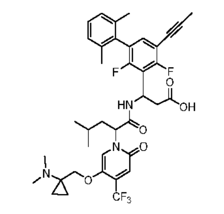 | 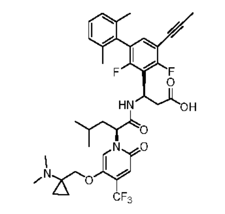 | 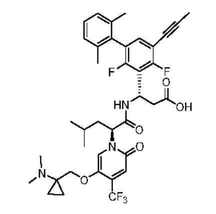 | 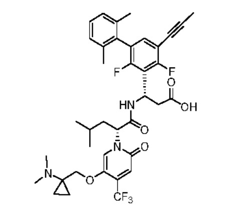 | 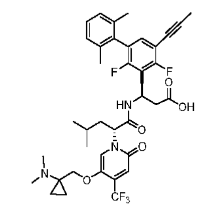 |
| 108 | 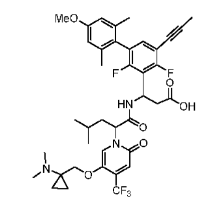 | 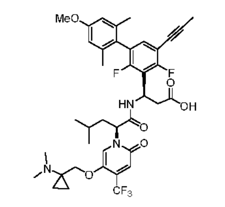 | 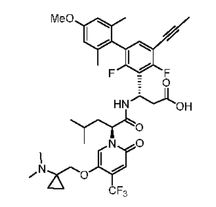 | 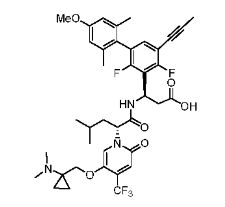 | 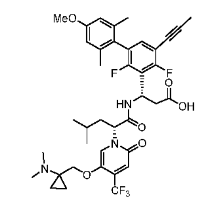 |
| 109 | 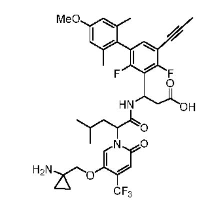 | 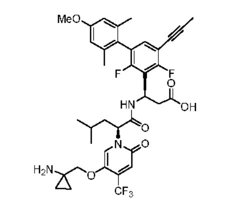 | 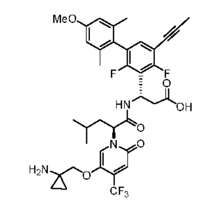 | 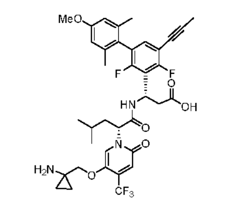 | 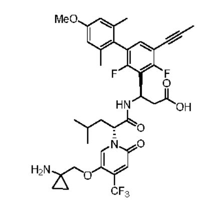 |
| 110 | 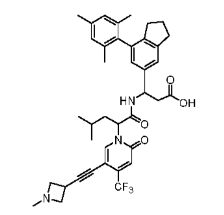 | 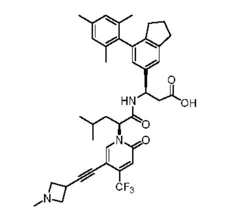 | 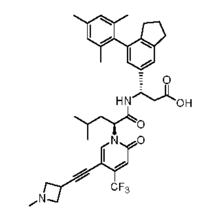 | 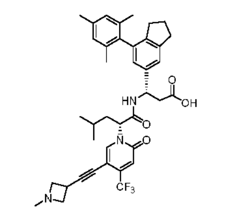 | 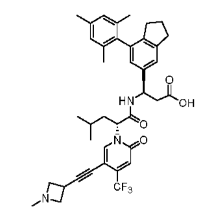 |
| 111 | 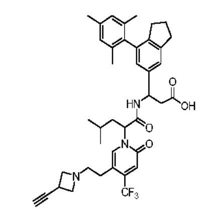 | 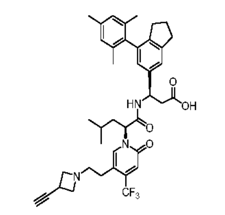 | 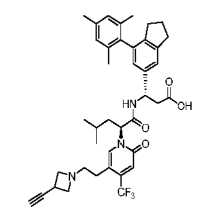 | 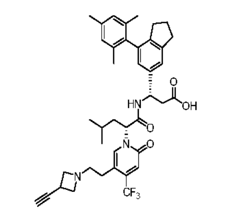 | 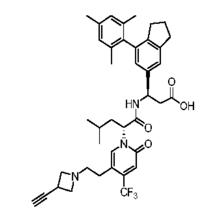 |
| 112 | 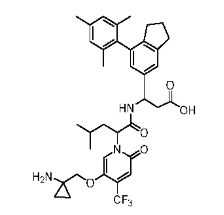 | 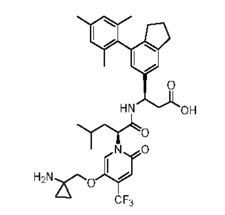 | 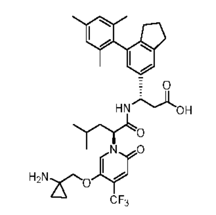 | 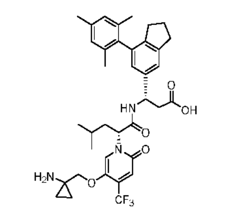 | 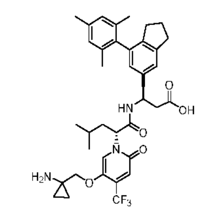 |
| 113 | 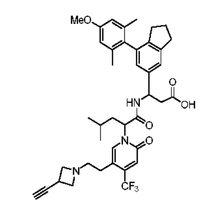 | 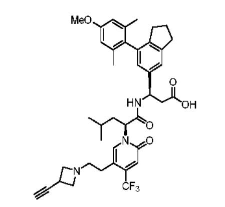 | 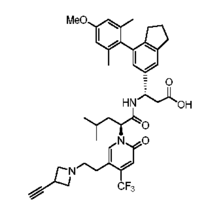 | 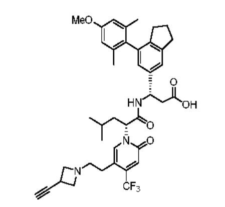 | 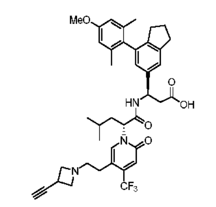 |
| 114 | 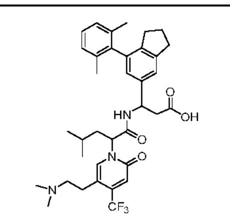 | 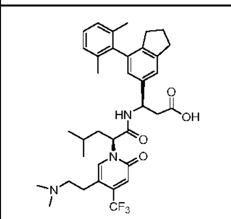 | 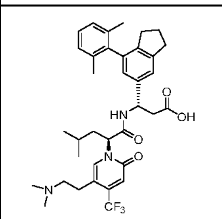 | 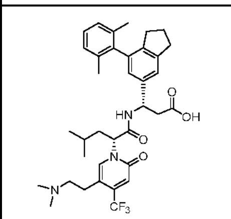 | 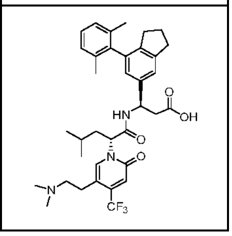 |

(continued)

| | | | | | |
|---|---|---|---|---|---|
| 115 | 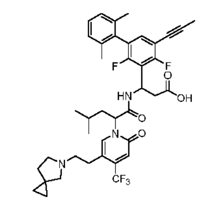 | 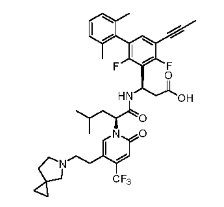 | 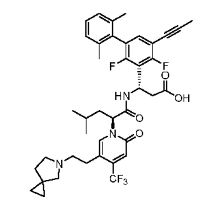 | 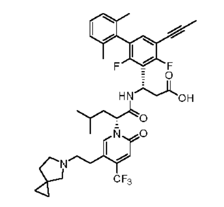 | 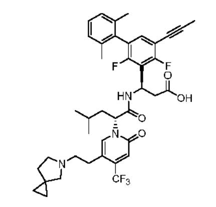 |
| 116 | 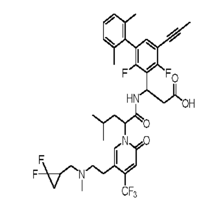 | 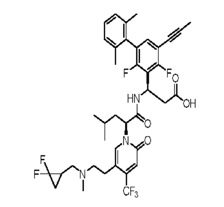 | 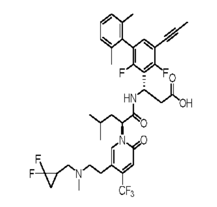 | 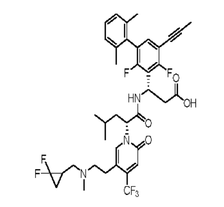 | 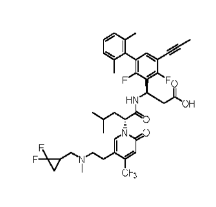 |
| 117 | 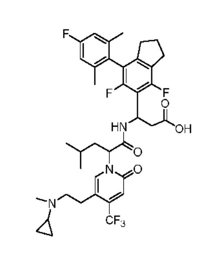 | 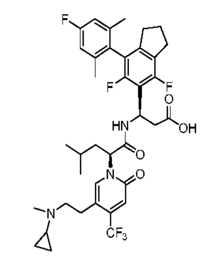 | 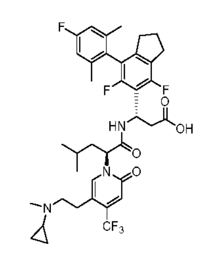 | 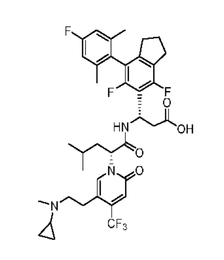 | 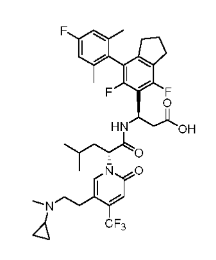 |
| 118 | 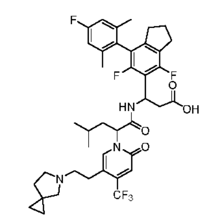 | 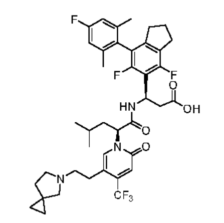 | 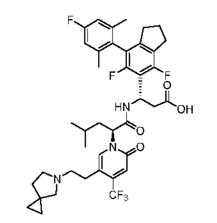 | 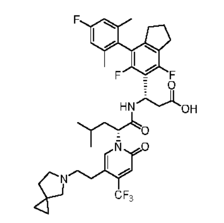 | 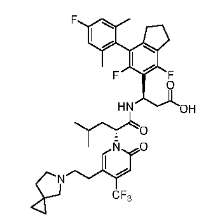 |
| 119 | 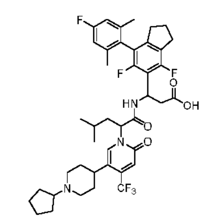 | 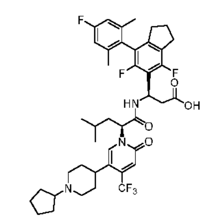 | 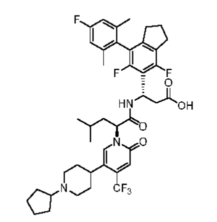 | 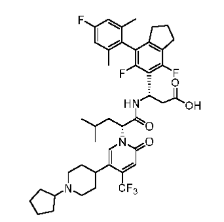 | 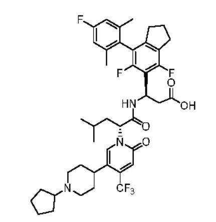 |
| 120 | 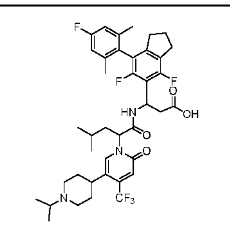 | 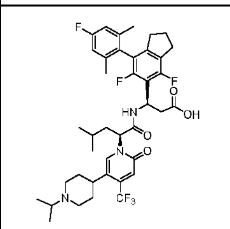 | 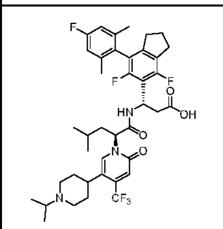 | 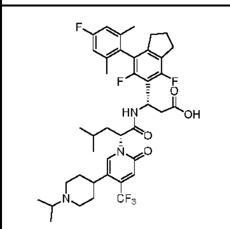 | 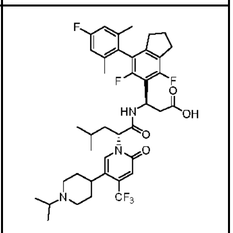 |
| 121 | 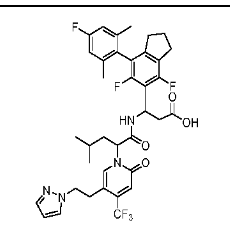 | 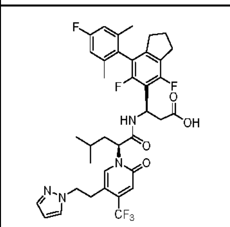 | 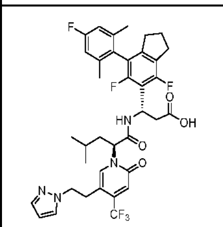 | 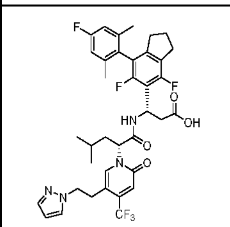 | 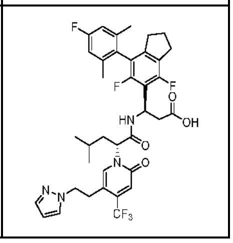 |

(continued)

| | | | | | |
|---|---|---|---|---|---|
| 122 | 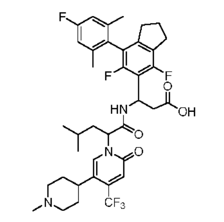 | 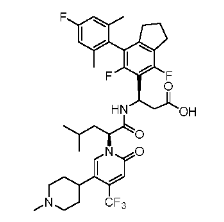 | 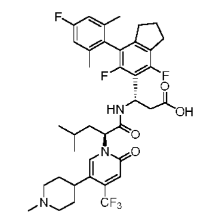 | 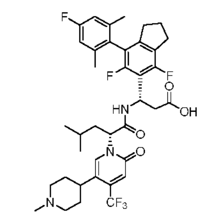 | 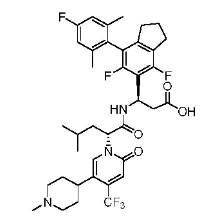 |
| 123 | 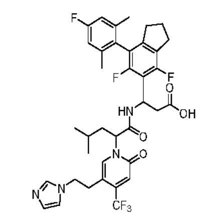 | 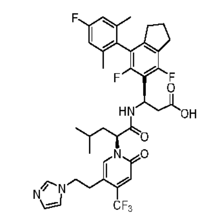 | 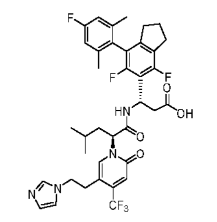 | 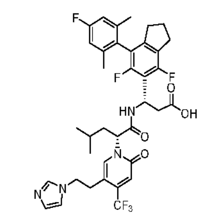 | 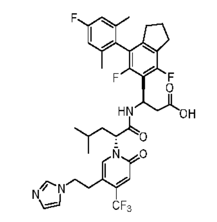 |
| 124 | 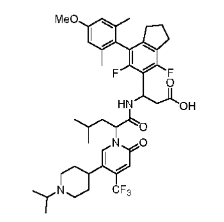 | 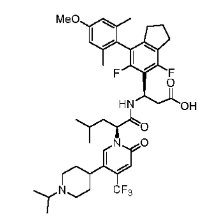 | 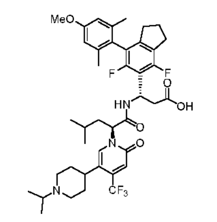 | 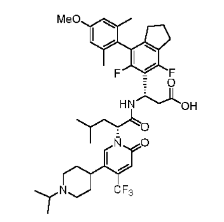 | 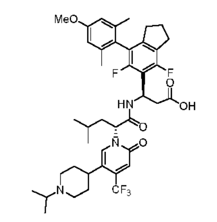 |
| 125 | 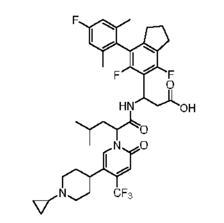 | 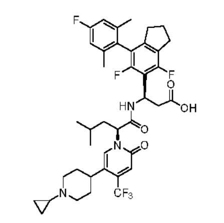 | 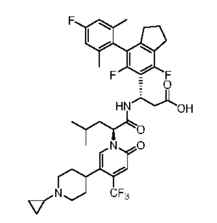 | 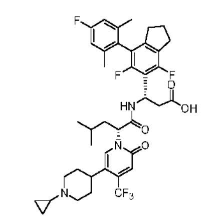 | 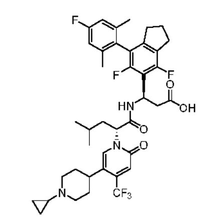 |
| 126 | 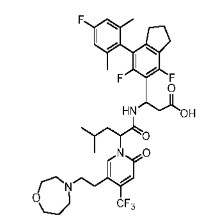 | 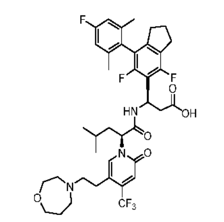 | 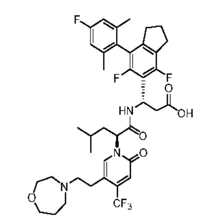 | 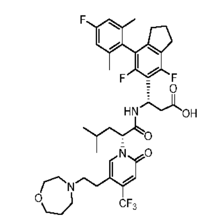 | 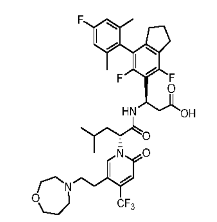 |
| 127 | 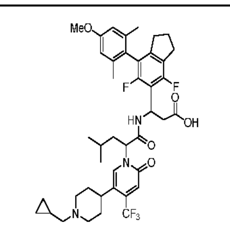 | 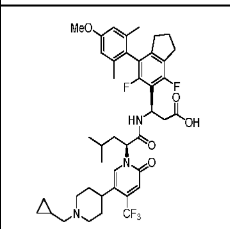 | 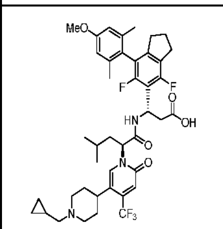 | 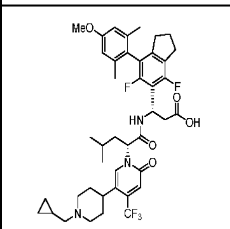 | 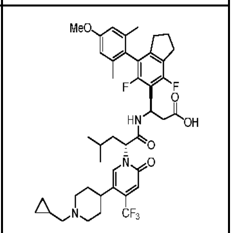 |
| 128 | 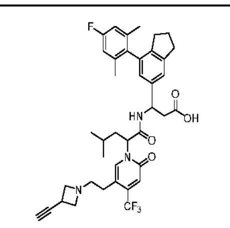 | 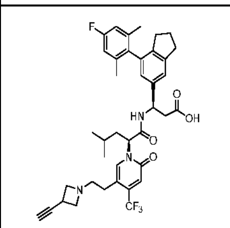 | 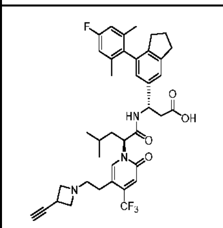 | 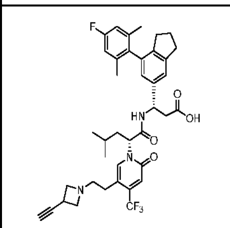 | 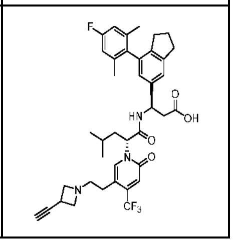 |

(continued)

| | | | | | |
|---|---|---|---|---|---|
| 129 | 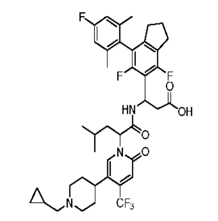 | 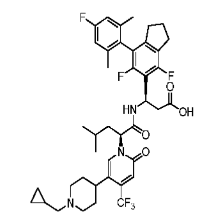 | 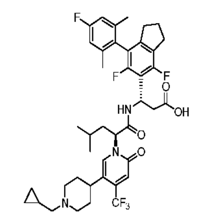 | 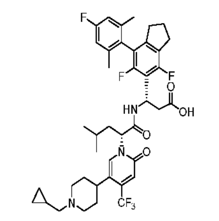 | 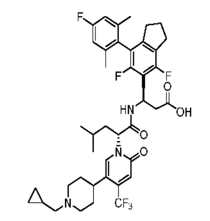 |
| 130 | 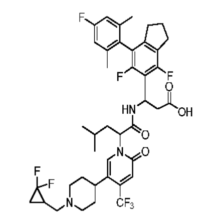 | 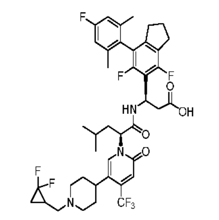 | 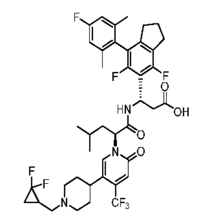 | 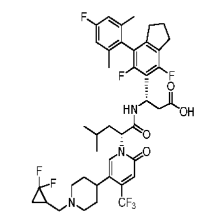 | 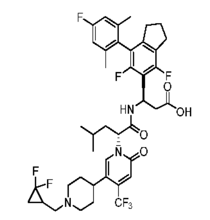 |
| 131 | 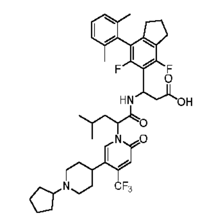 | 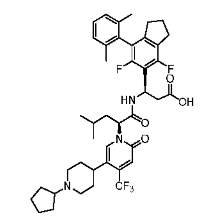 | 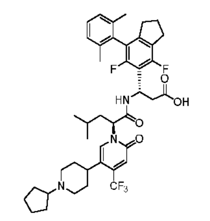 | 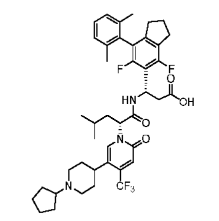 | 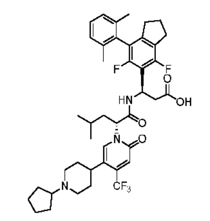 |
| 132 | 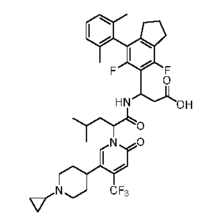 | 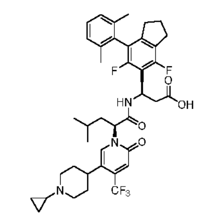 | 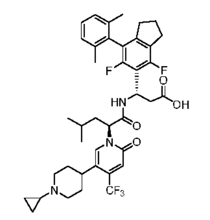 | 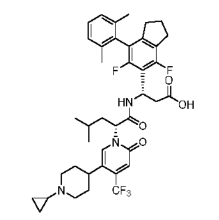 | 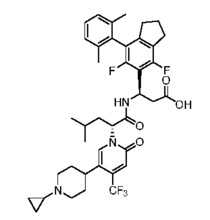 |
| 133 | 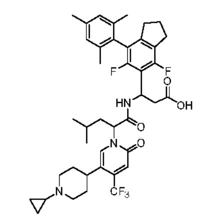 | 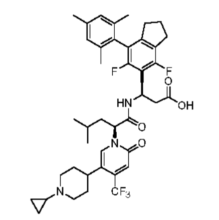 | 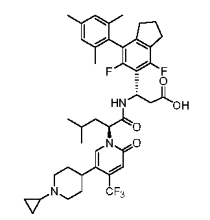 | 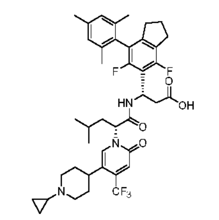 | 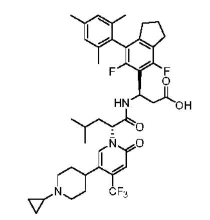 |
| 134 | 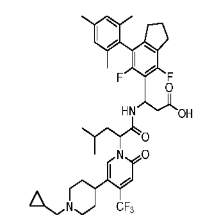 | 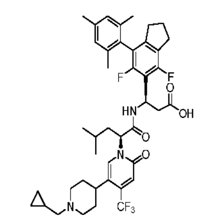 | 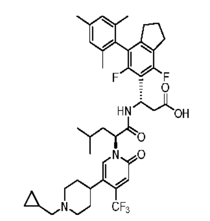 | 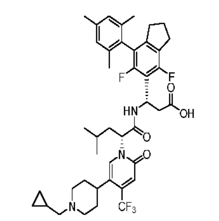 | 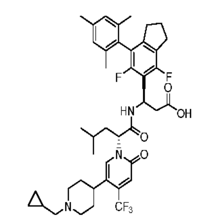 |
| 135 | 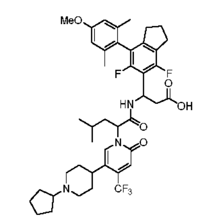 | 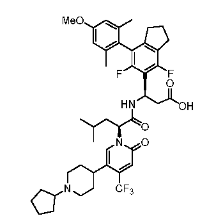 | 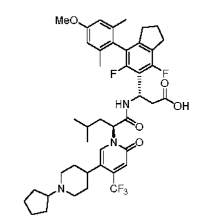 | 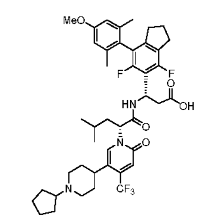 | 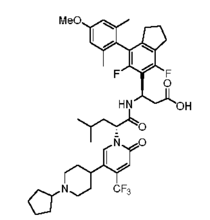 |

(continued)

| | | | | | |
|---|---|---|---|---|---|
| 136 | 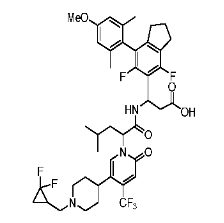 | 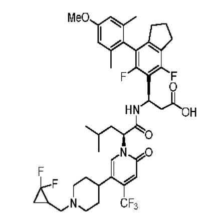 | 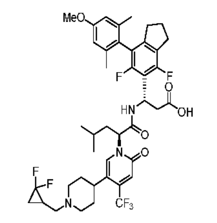 | 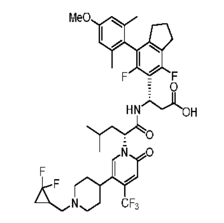 | 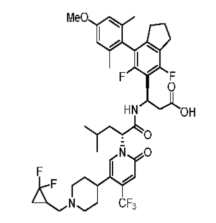 |
| 137 | 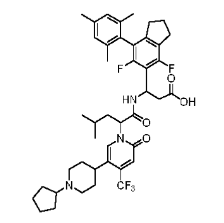 | 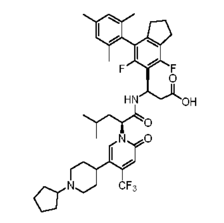 | 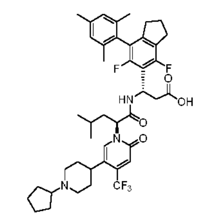 | 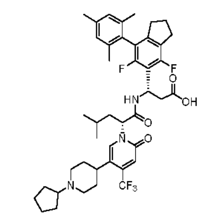 | 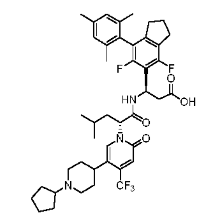 |
| 138 | 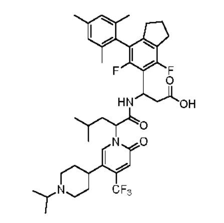 | 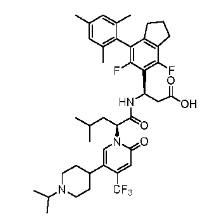 | 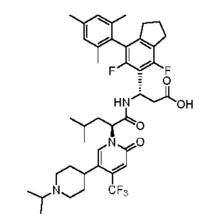 | 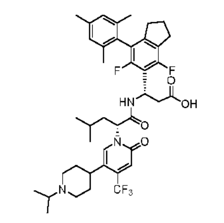 | 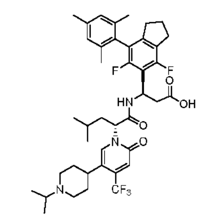 |
| 139 | 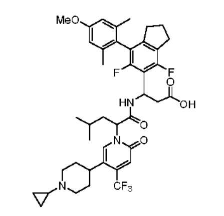 | 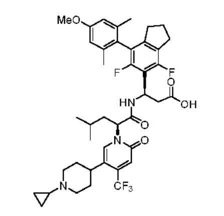 | 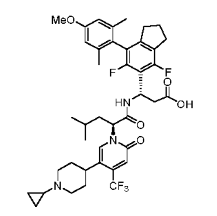 | 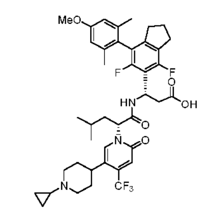 | 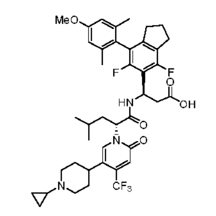 |
| 140 | 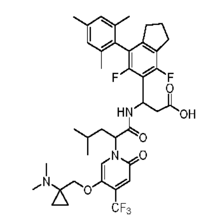 | 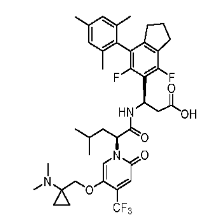 | 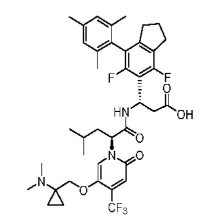 | 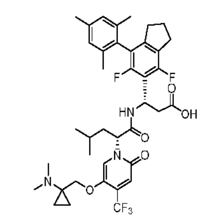 | 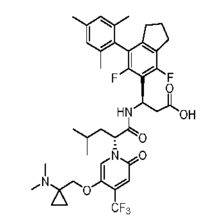 |
| 141 | 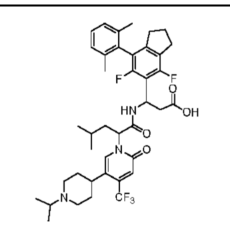 | 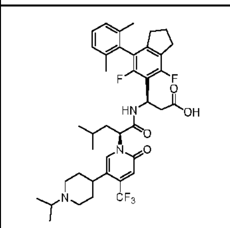 | 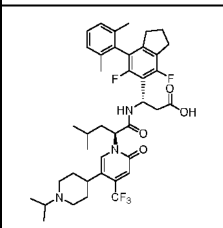 | 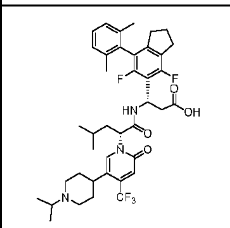 | 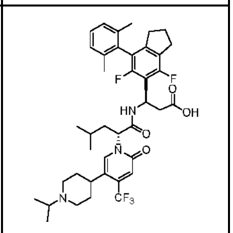 |
| 142 | 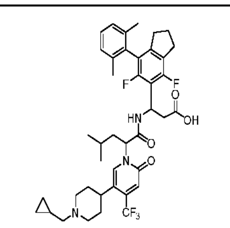 | 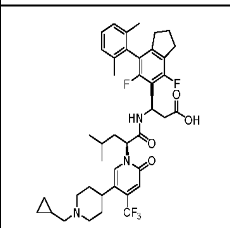 | 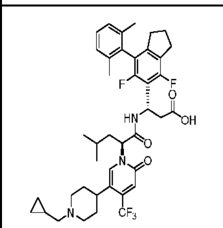 | 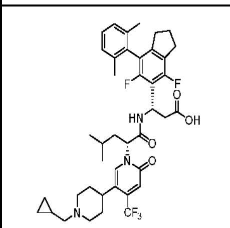 | 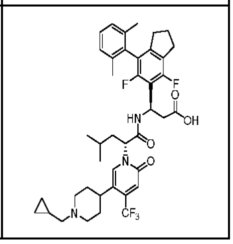 |

(continued)

| | | | | | |
|---|---|---|---|---|---|
| 143 | 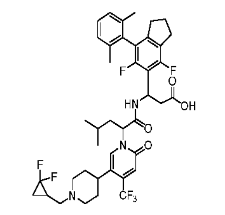 | 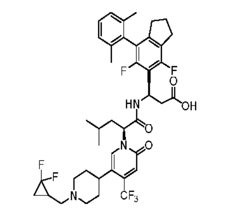 | 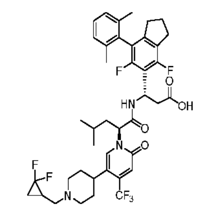 | 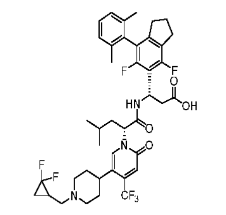 | 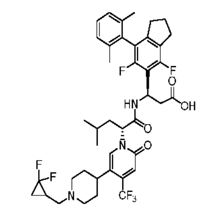 |
| 144 | 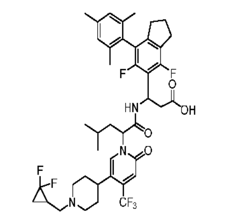 | 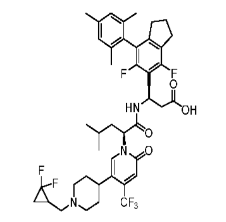 | 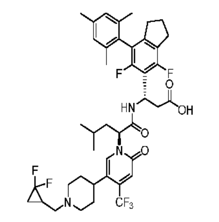 | 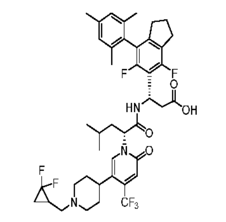 | 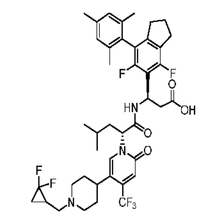 |
| 145 | 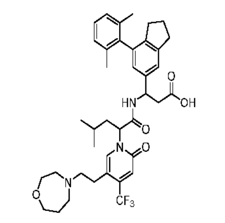 | 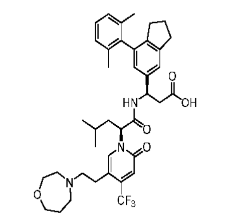 | 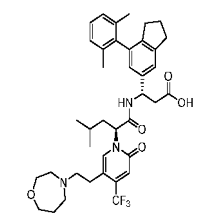 | 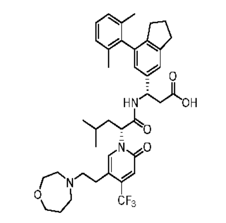 | 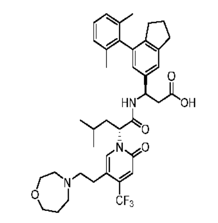 |
| 146 | 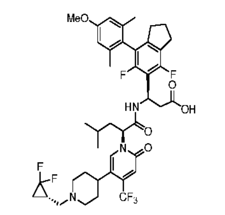 | 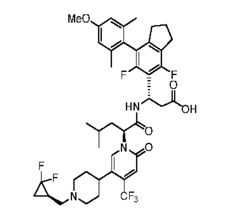 | 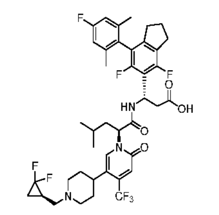 | 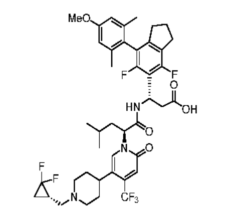 | 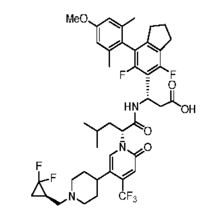 |
| 147 | 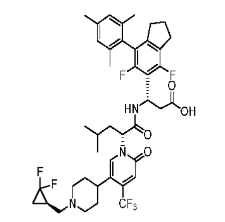 | 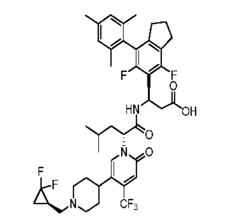 | 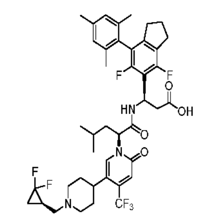 | 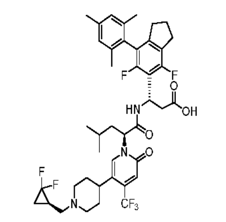 | 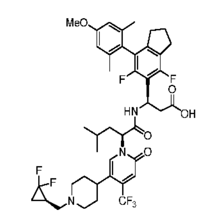 |
| 148 | 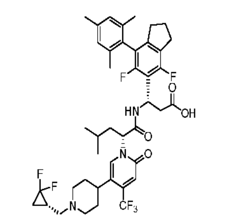 | 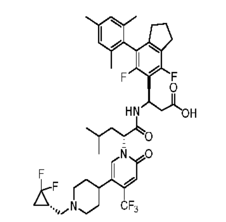 | 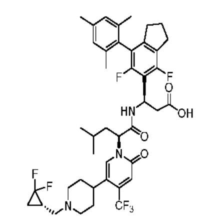 | 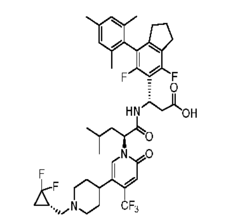 | 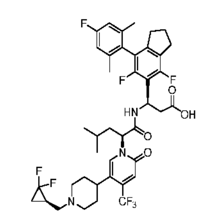 |
| 149 | 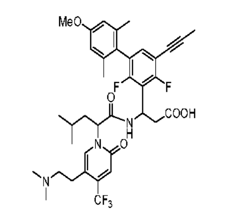 | 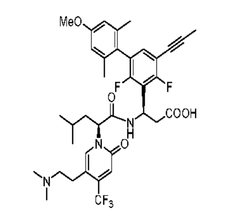 | 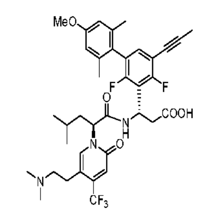 | 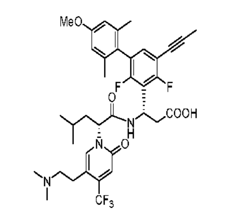 | 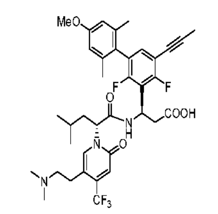 |

(continued)

| | | | | | |
|---|---|---|---|---|---|
| 150 | 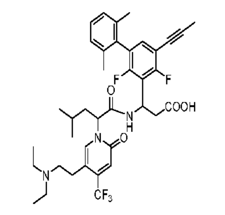 | 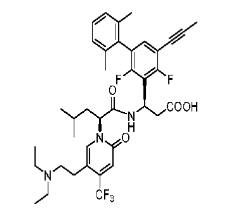 | 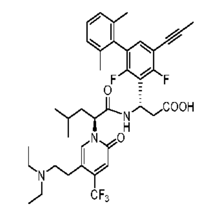 | 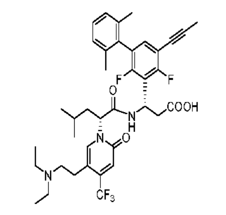 | 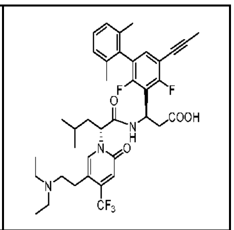 |
| 151 | 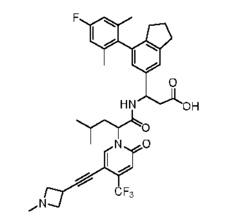 | 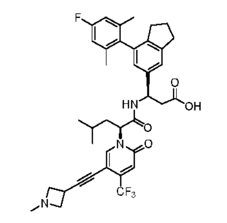 | 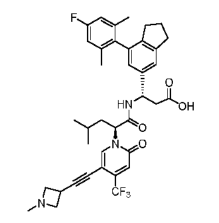 | 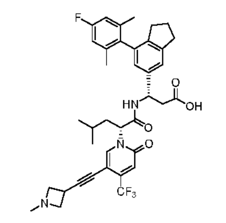 | 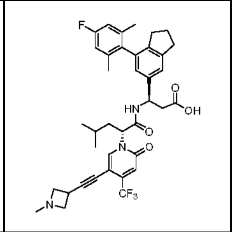 |
| 152 | 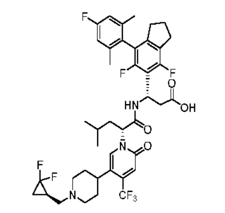 | 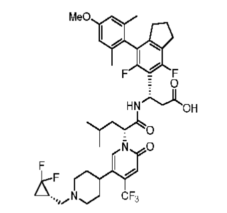 | 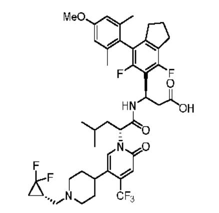 | 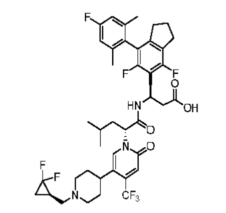 | 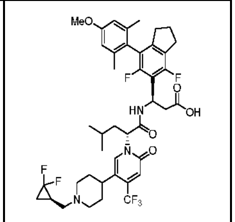 |
| 153 | 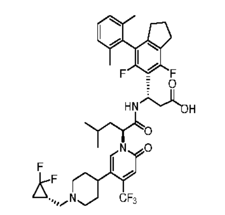 | 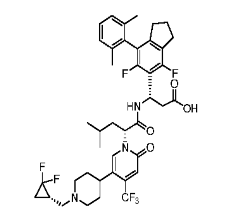 | 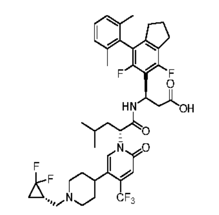 | 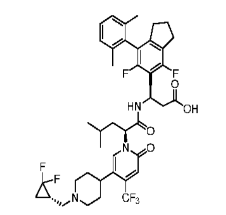 | 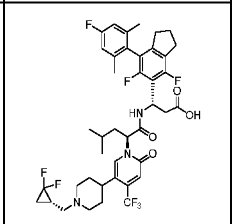 |
| 154 | 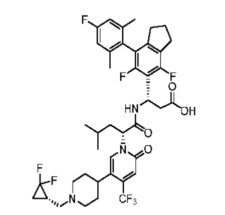 | 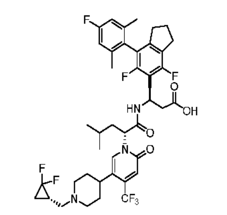 | | | |
| 155 | 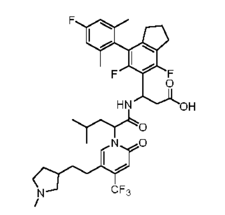 | 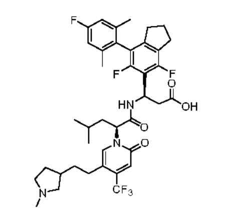 | 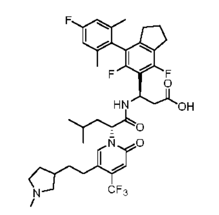 | 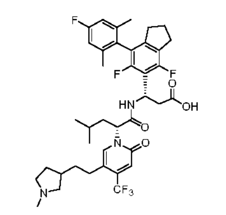 | 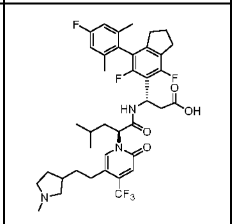 |
| 156 | 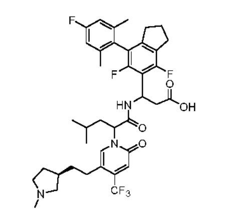 | 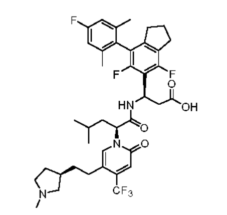 | 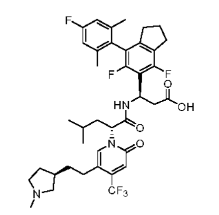 | 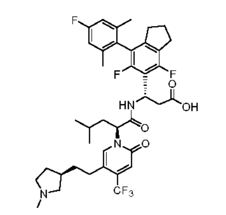 | 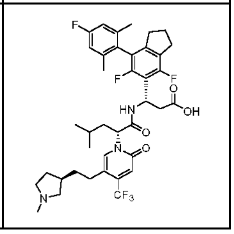 |

(continued)

| | | | | | |
|---|---|---|---|---|---|
| 157 | 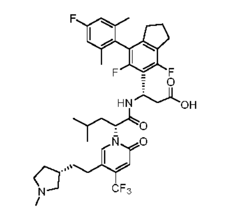 | 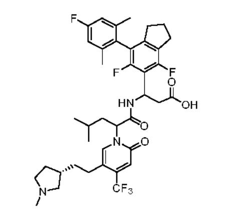 | 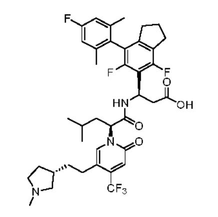 | 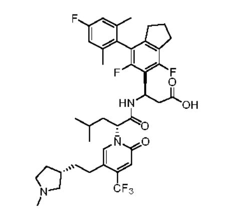 | 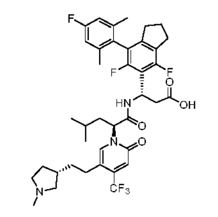 |
| 158 | 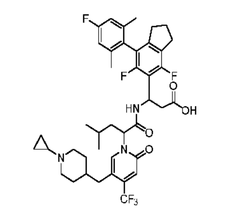 | 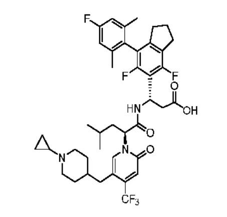 | 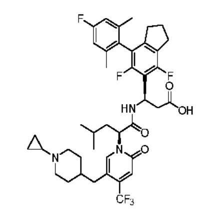 | 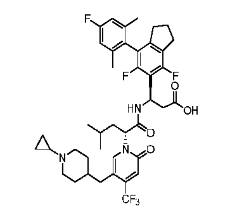 | 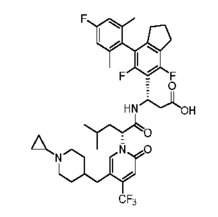 |
| 159 | 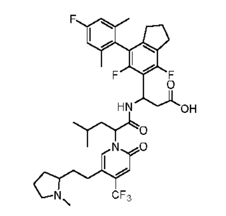 | 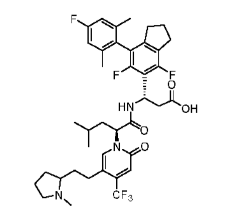 | 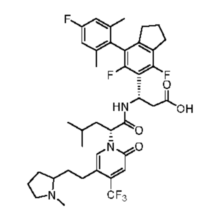 | 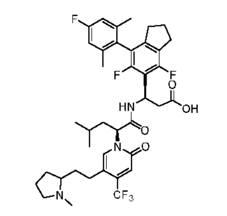 | 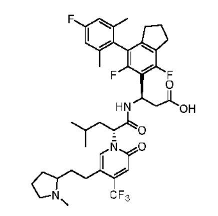 |
| 160 | 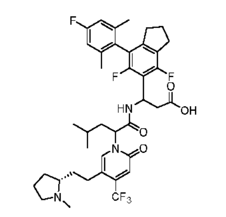 | 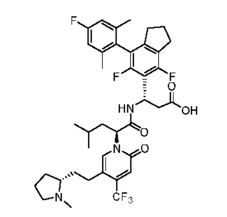 | 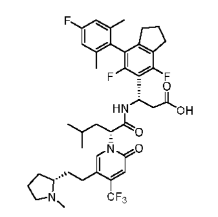 | 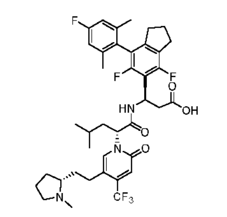 | 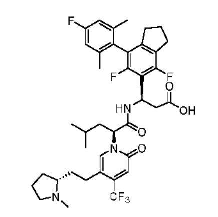 |
| 161 | 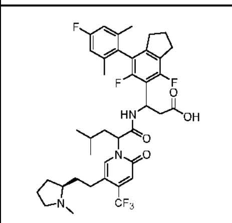 | 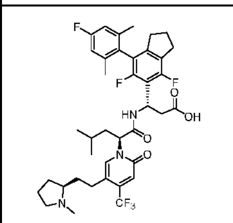 | 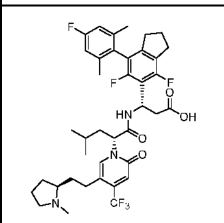 | 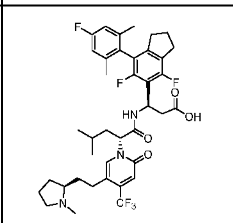 | 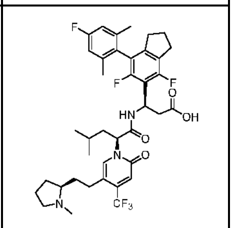 |
| 162 | 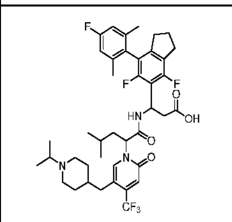 | 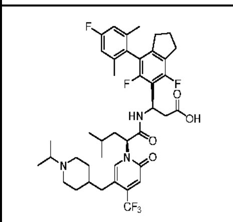 | 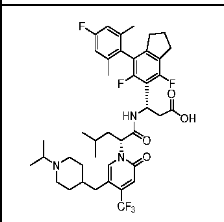 | 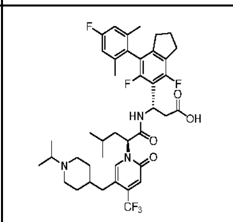 | 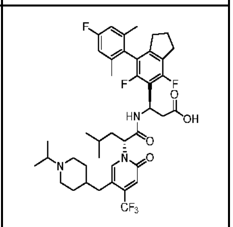 |
| 163 | 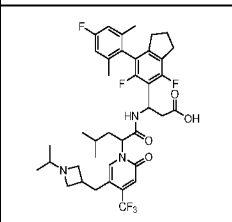 | 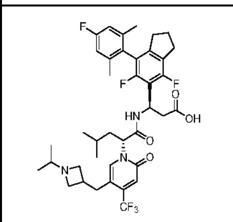 | 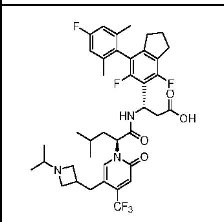 | 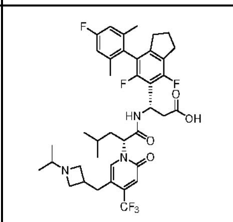 | 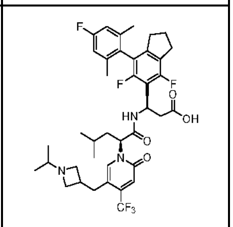 |
| 164 | 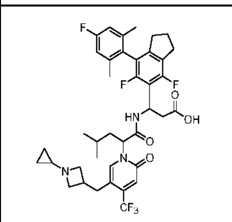 | 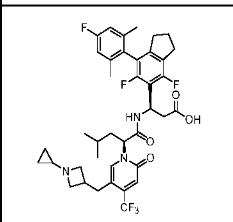 | 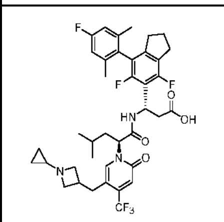 | 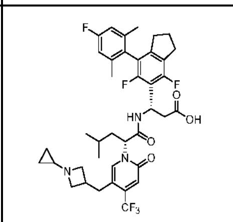 | 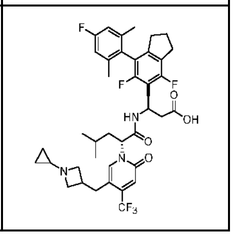 |

(continued)

| | | | | | |
|---|---|---|---|---|---|
| 165 | 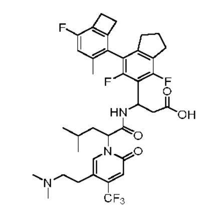 | 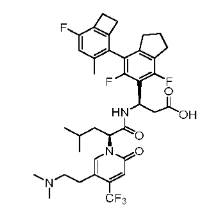 | 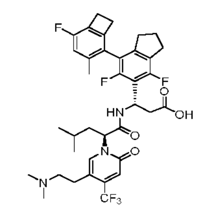 | 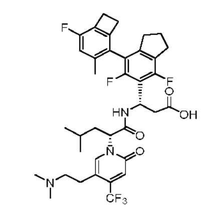 | 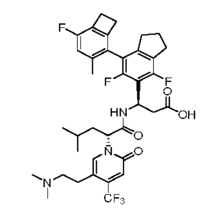 |
| 166 | 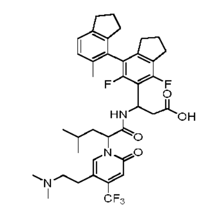 | 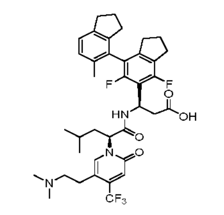 | 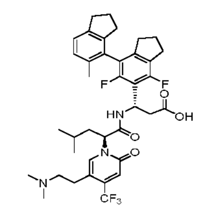 | 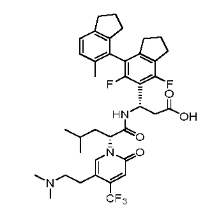 | 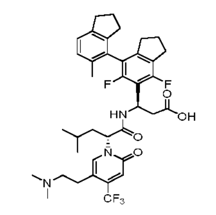 |
| 167 | 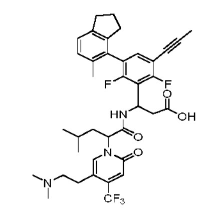 | 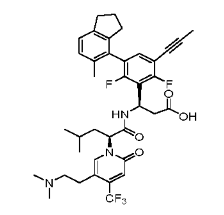 | 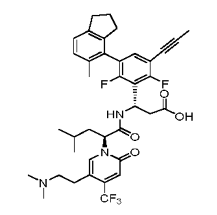 | 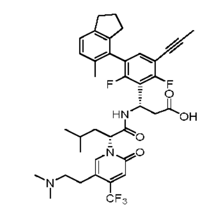 | 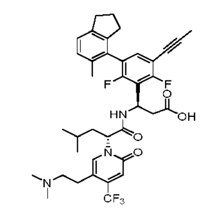 |
| 168 | 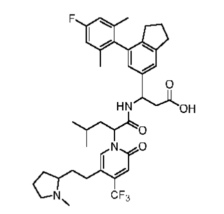 | 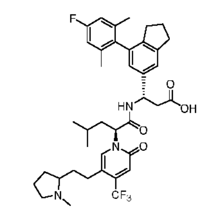 | 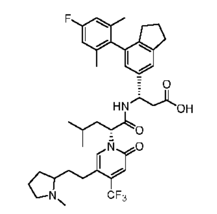 | 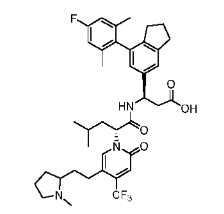 | 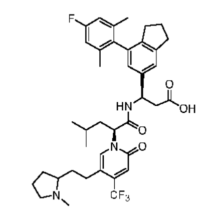 |
| 169 | 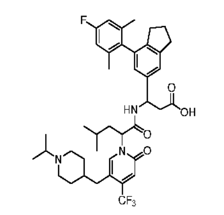 | 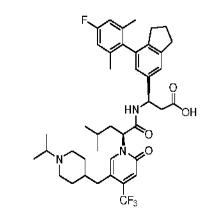 | 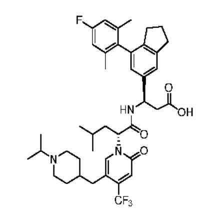 | 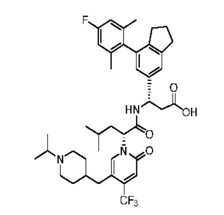 | 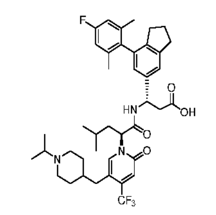 |
| 170 | 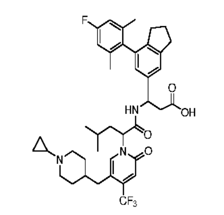 | 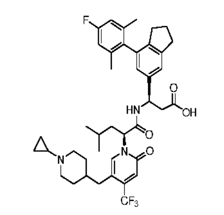 | 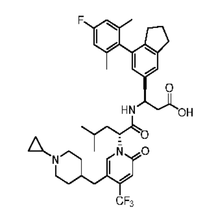 | 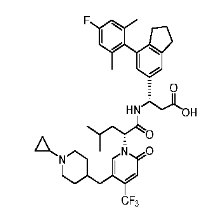 | 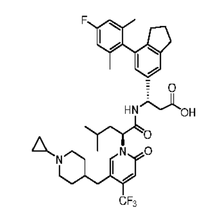 |
| 171 | 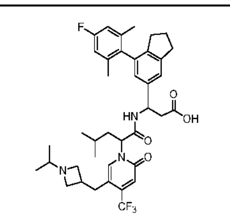 | 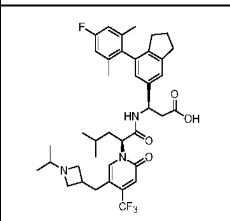 | 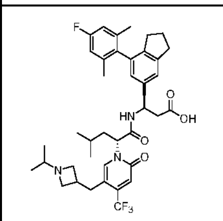 | 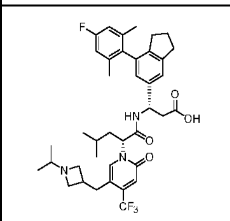 | 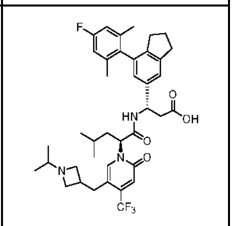 |
| 172 | 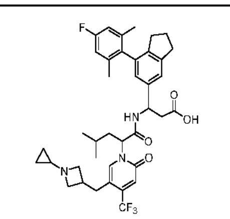 | 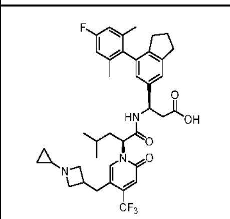 | 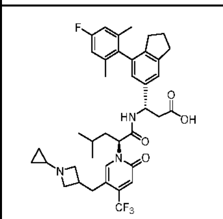 | 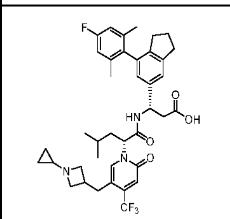 | 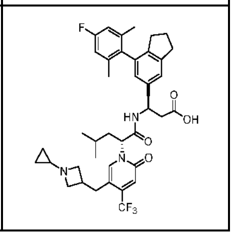 |

(continued)

| | | | | | |
|---|---|---|---|---|---|
| 173 | 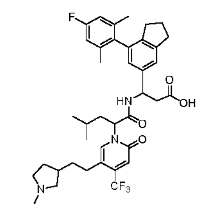 | 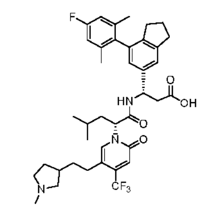 | 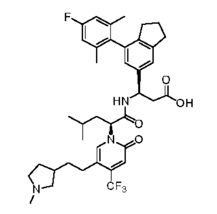 | 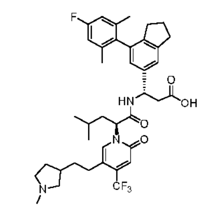 | 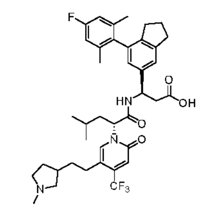 |
| 174 | 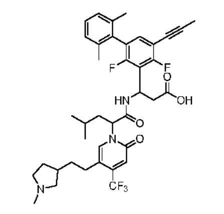 | 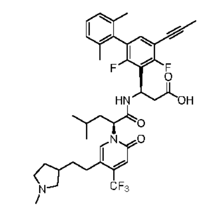 | 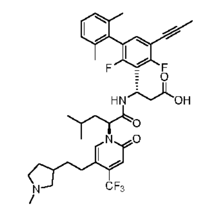 | 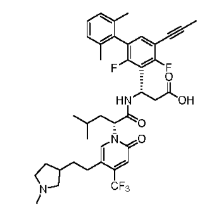 | 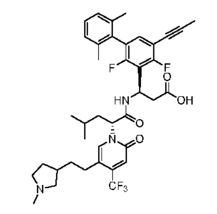 |
| 175 | 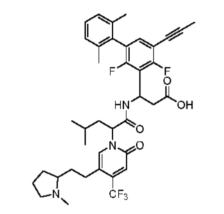 | 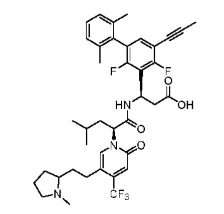 | 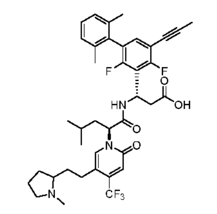 | 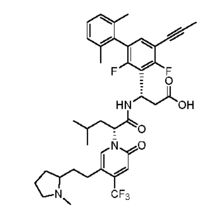 | 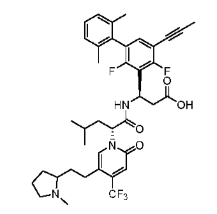 |
| 176 | 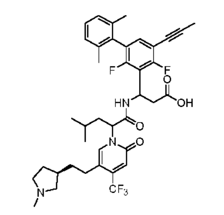 | 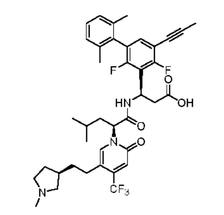 | 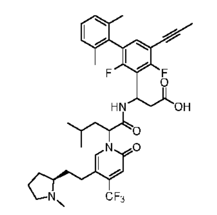 | 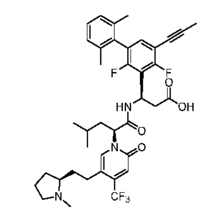 | 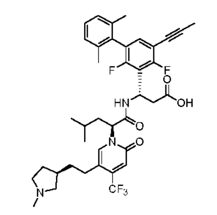 |
| 177 | 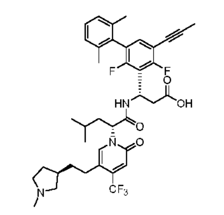 | 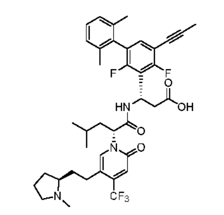 | 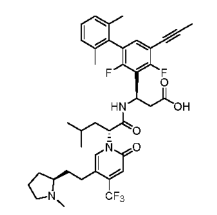 | 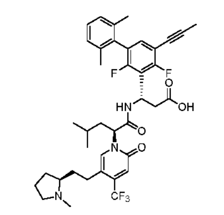 | 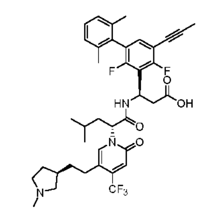 |
| 178 | 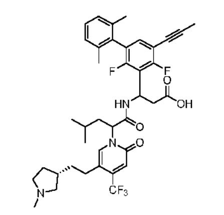 | 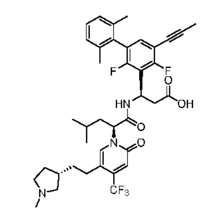 | 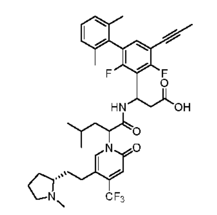 | 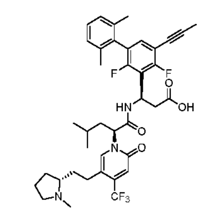 | 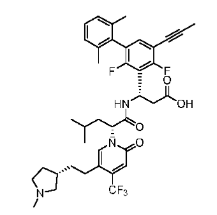 |
| 179 | 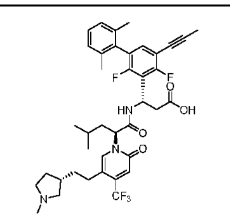 | 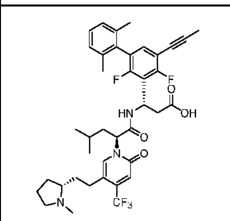 | 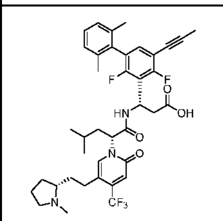 | 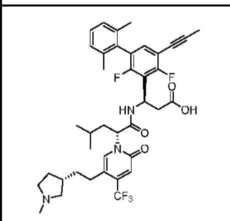 | 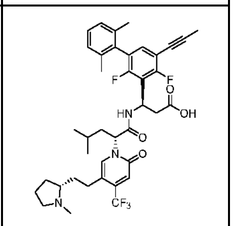 |
| 180 | 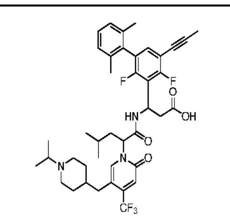 | 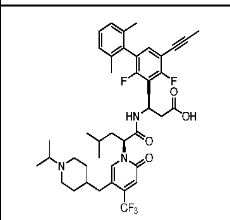 | 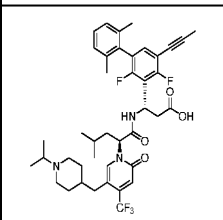 | 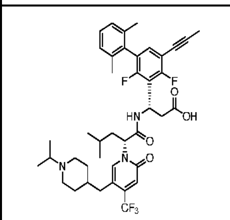 | 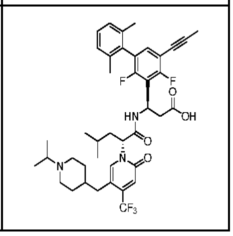 |

(continued)

| | | | | | |
|---|---|---|---|---|---|
| 181 | 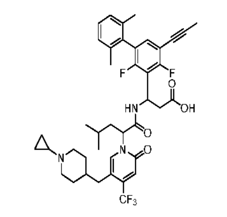 | 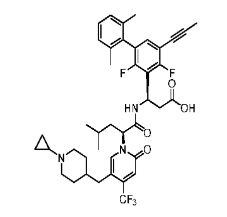 | 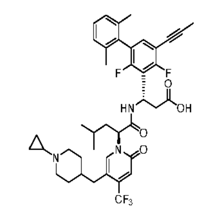 | 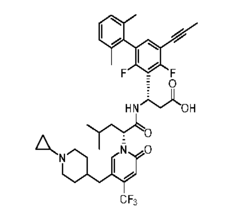 | 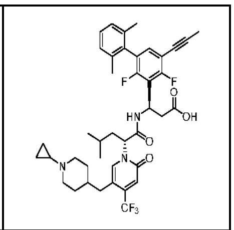 |
| 182 | 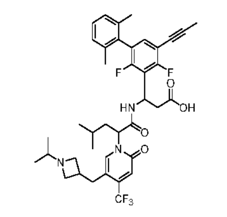 | 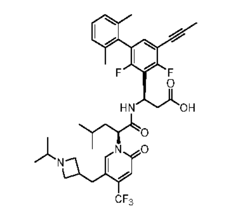 | 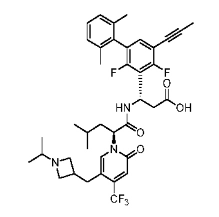 | 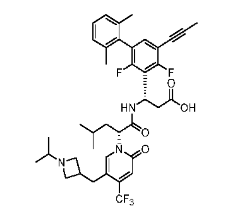 | 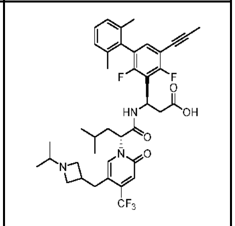 |
| 183 | 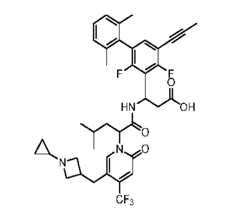 | 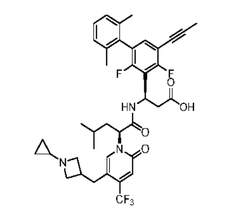 | 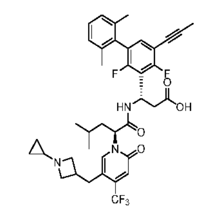 | 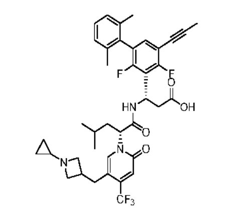 | 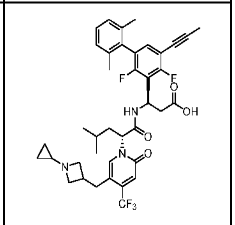 |
| 184 | 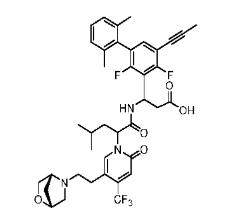 | 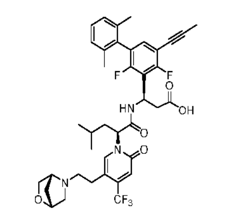 | 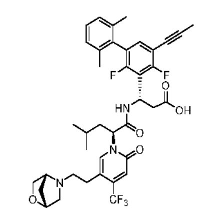 | 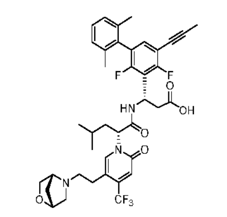 | 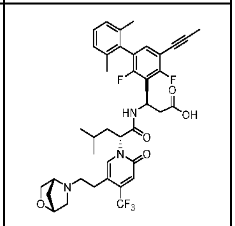 |
| 185 | 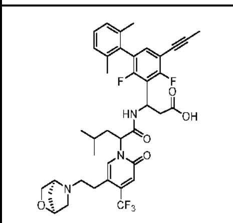 | 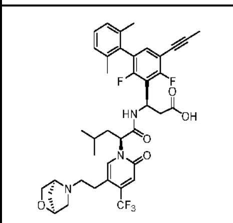 | 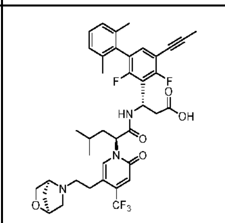 | 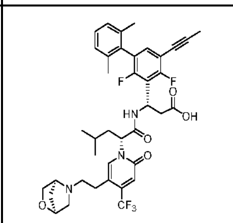 | 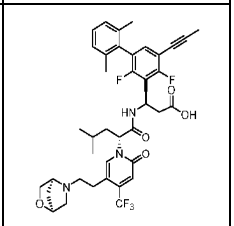 |
| 186 | 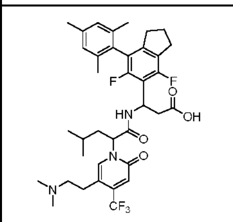 | 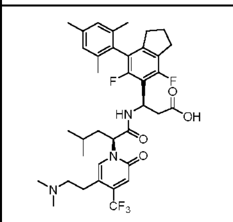 | 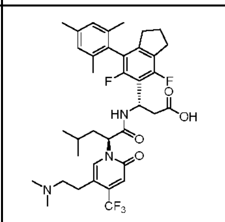 | 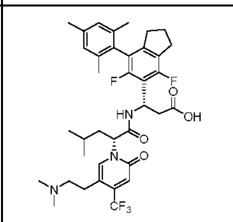 | 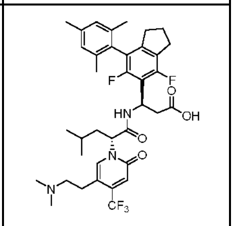 |
| 187 | 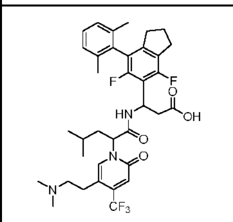 | 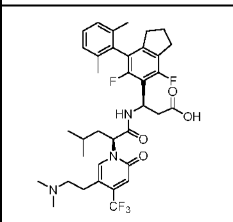 | 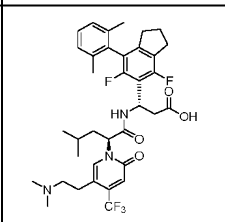 | 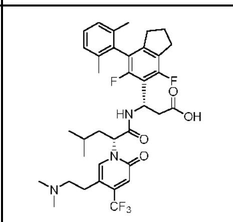 | 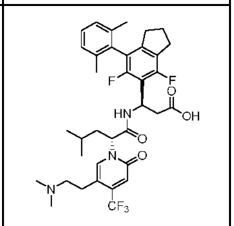 |
| 188 | 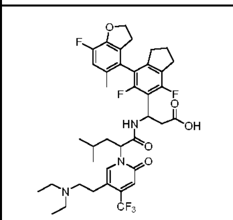 | 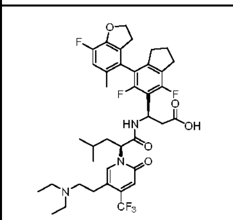 | 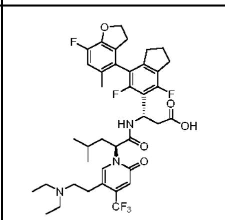 | 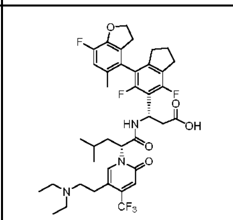 | 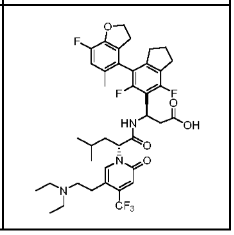 |

(continued)

| | | | | | |
|---|---|---|---|---|---|
| 189 | 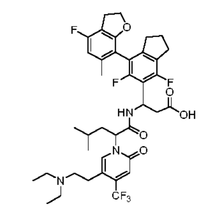 | 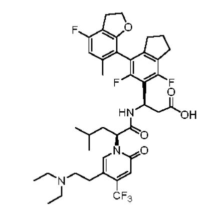 | 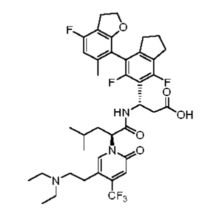 | 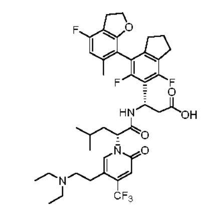 | 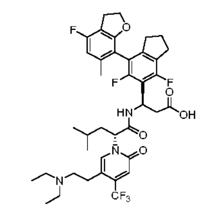 |
| 190 | 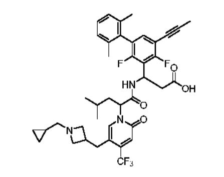 | 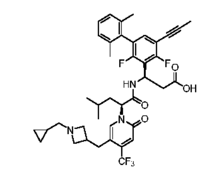 | 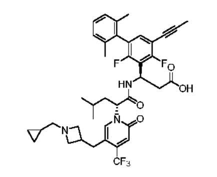 | 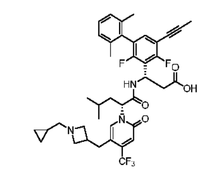 | 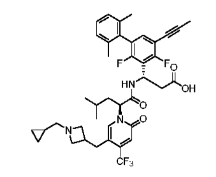 |
| 191 | 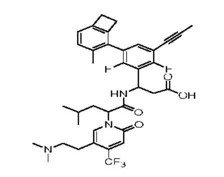 | 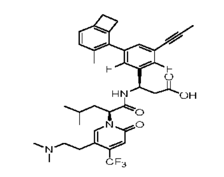 | 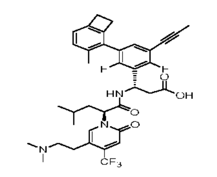 | 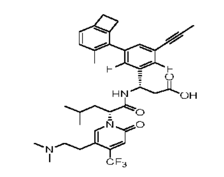 | 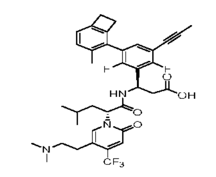 |
| 192 | 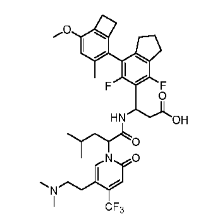 | 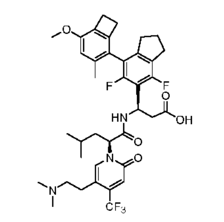 | 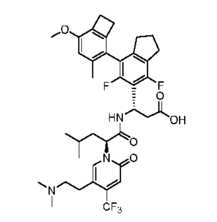 | 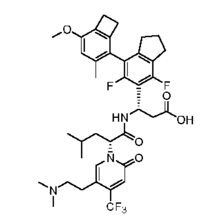 | 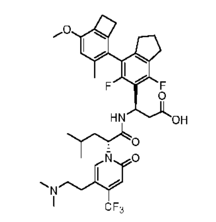 |
| 193 | 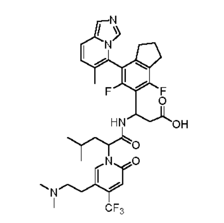 | 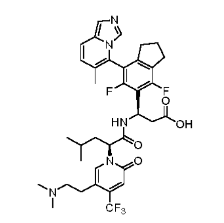 | 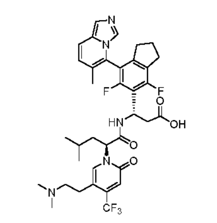 | 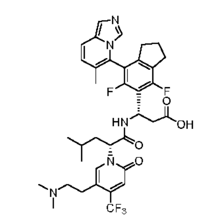 | 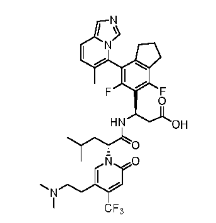 |
| 194 | 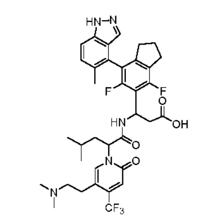 | 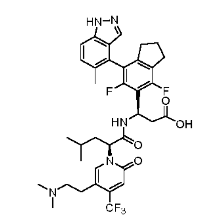 | 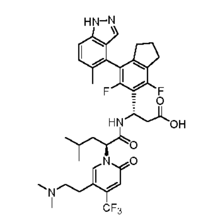 | 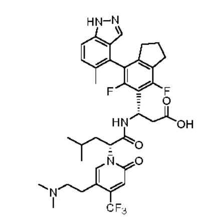 | 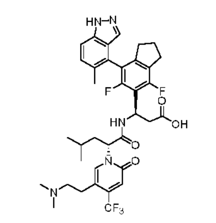 |
| 195 | 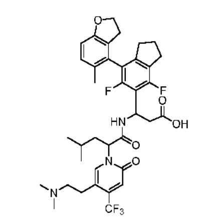 | 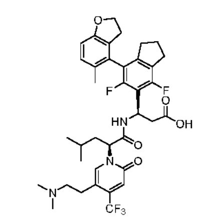 | 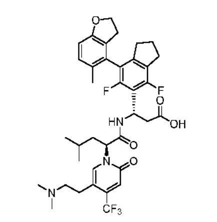 | 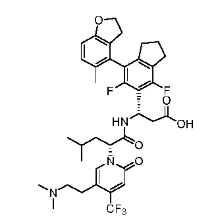 | 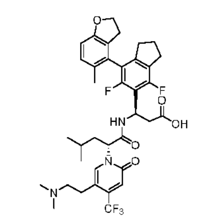 |
| 196 | 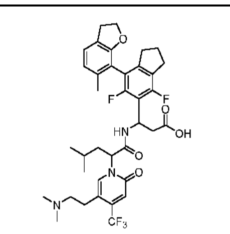 | 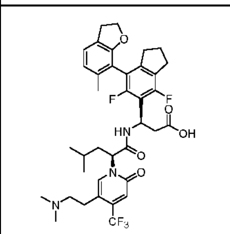 | 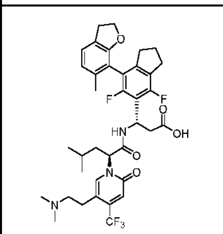 | 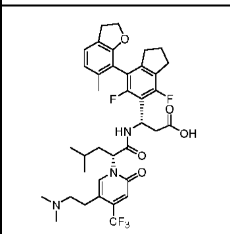 | 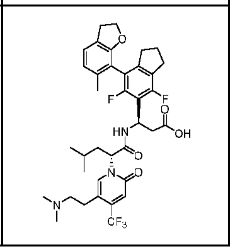 |

(continued)

| | | | | | |
|---|---|---|---|---|---|
| 197 | 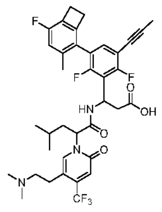 | 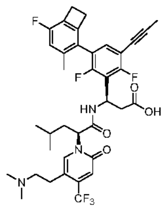 | 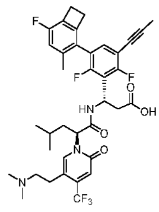 | 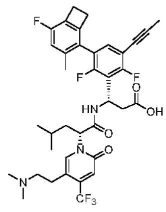 | 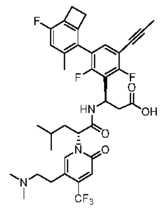 |
| 198 | 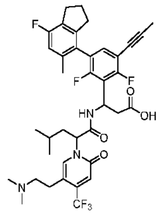 | 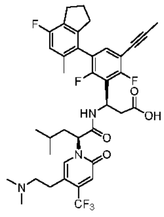 | 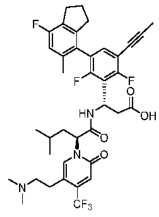 | 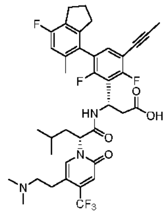 | 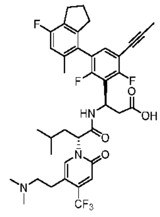 |
| 199 | 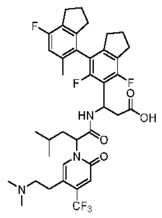 | 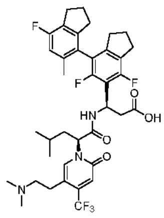 | 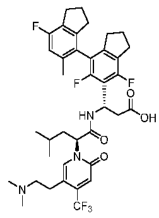 | 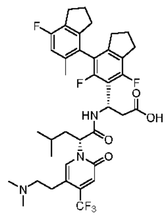 | 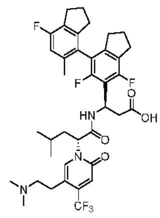 |
| 200 | 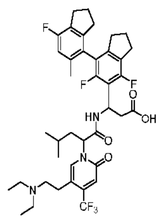 | 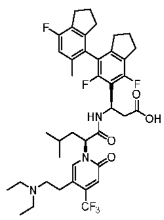 | 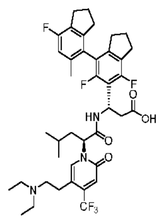 | 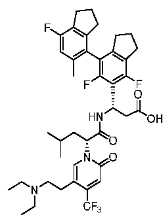 | 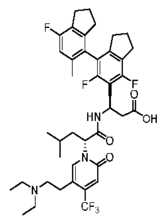 |
| 201 | 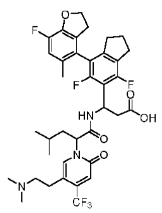 | 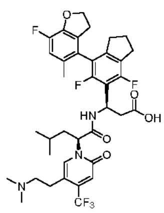 | 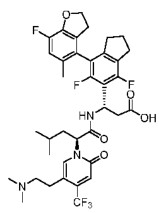 | 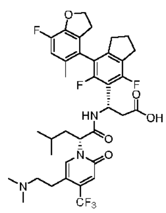 | 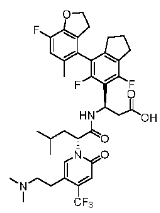 |
| 202 | 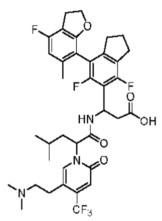 | 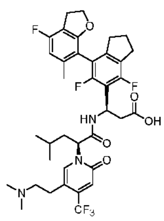 | 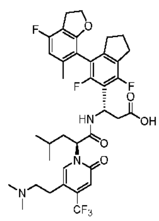 | 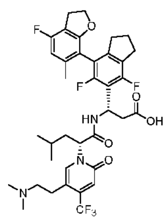 | 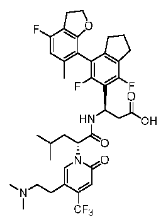 |
| 203 | 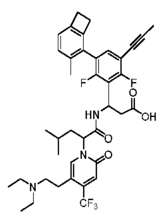 | 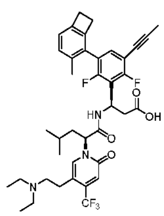 | 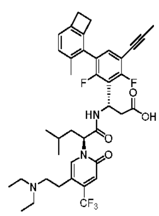 | 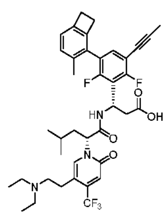 | 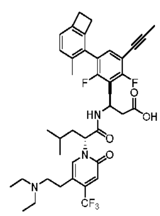 |

(continued)

| | | | | | |
|---|---|---|---|---|---|
| 204 | 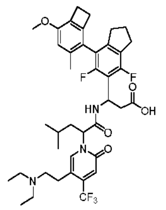 | 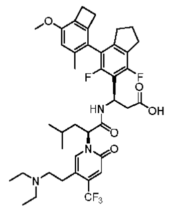 | 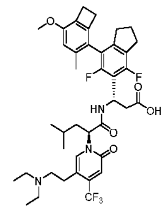 | 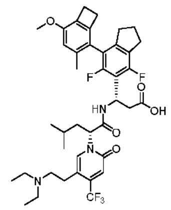 | 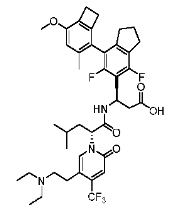 |
| 205 | 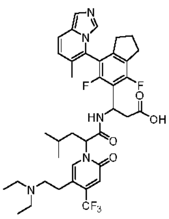 | 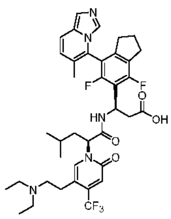 | 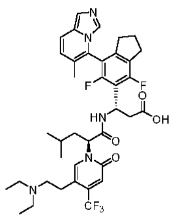 | 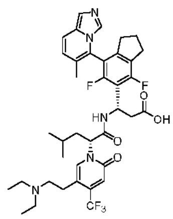 | 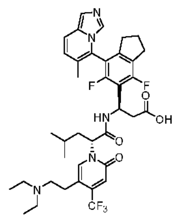 |
| 206 | 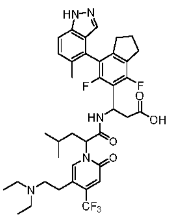 | 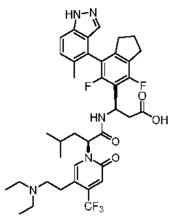 | 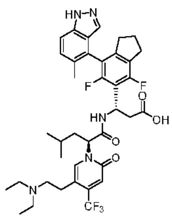 | 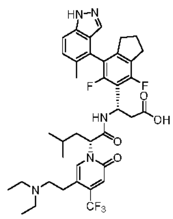 | 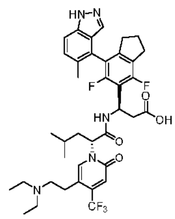 |
| 207 | 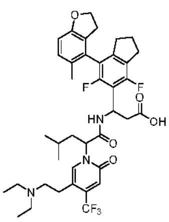 | 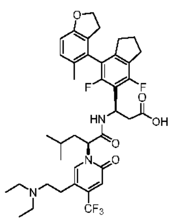 | 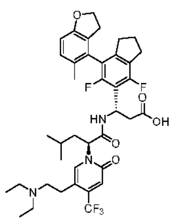 | 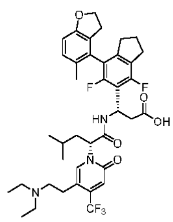 | 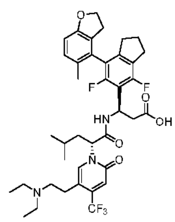 |
| 208 | 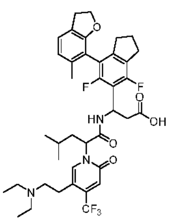 | 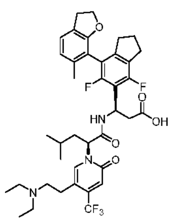 | 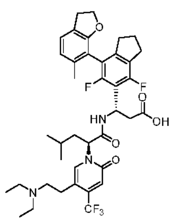 | 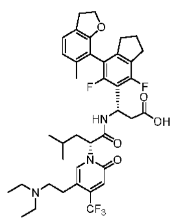 | 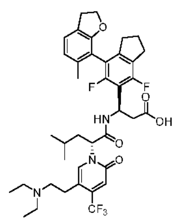 |
| 209 | 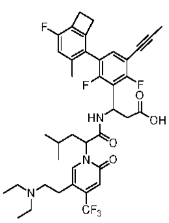 | 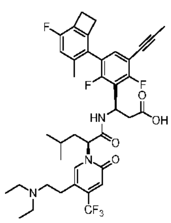 | 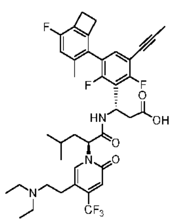 | 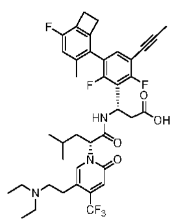 | 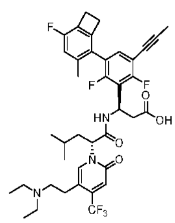 |
| 210 | 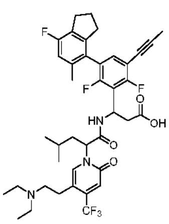 | 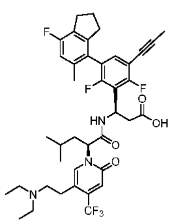 | 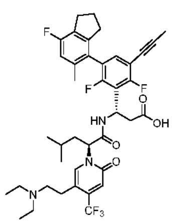 | 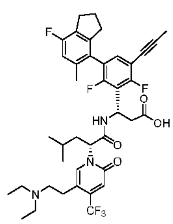 | 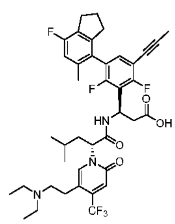 |

(continued)

| | | | | | |
|---|---|---|---|---|---|
| 211 | 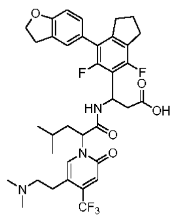 | 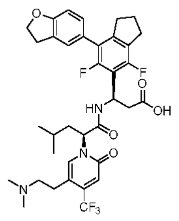 | 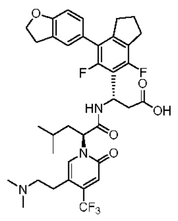 | 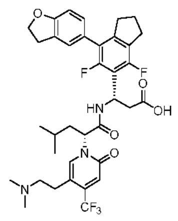 | 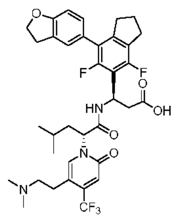 |
| 212 | 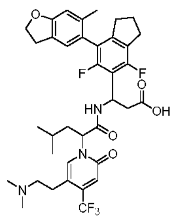 | 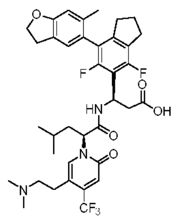 | 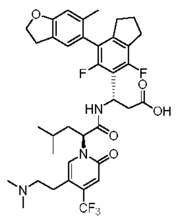 | 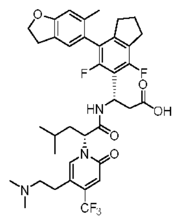 | 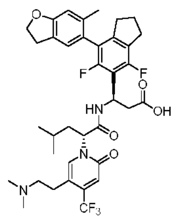 |
| 213 | 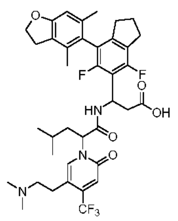 | 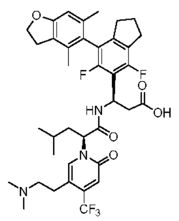 | 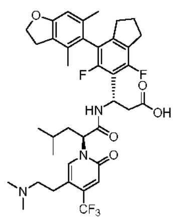 | 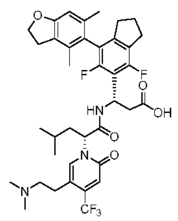 | 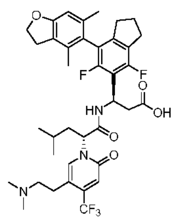 |
| 214 | 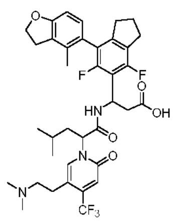 | 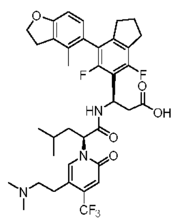 | 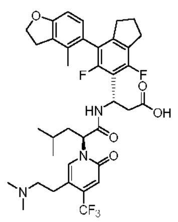 | 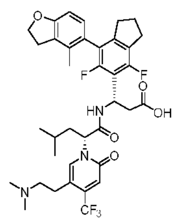 | 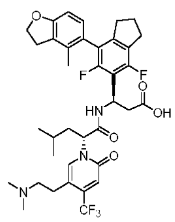 |
| 215 | 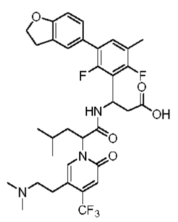 | 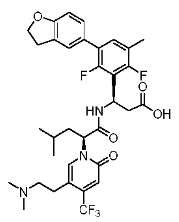 | 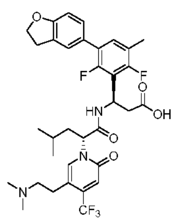 | 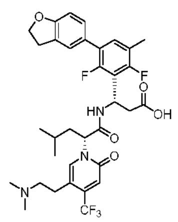 | 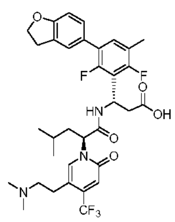 |
| 216 | 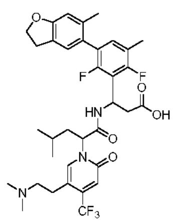 | 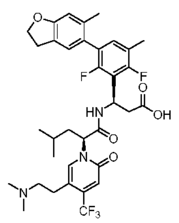 | 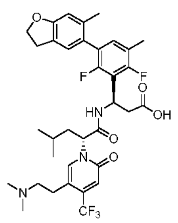 | 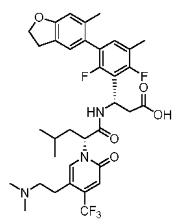 | 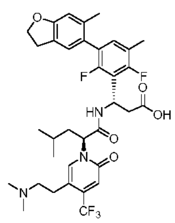 |
| 217 | 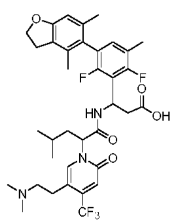 | 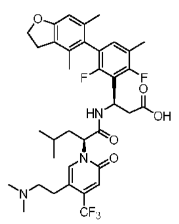 | 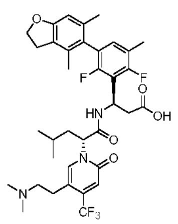 | 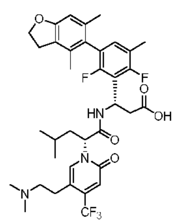 | 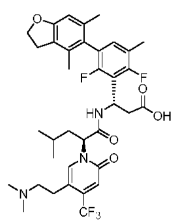 |

(continued)

| | | | | | |
|---|---|---|---|---|---|
| 218 | 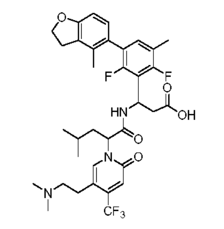 | 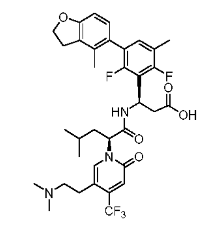 | 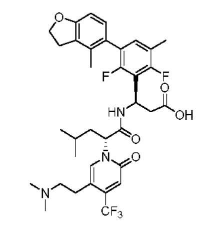 | 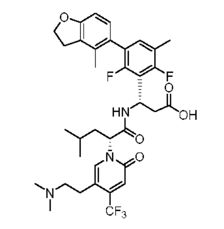 | 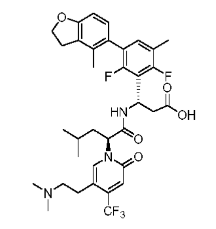 |
| 219 | 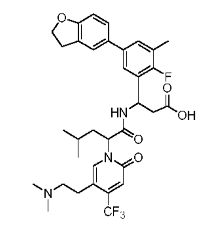 | 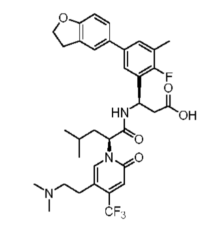 | 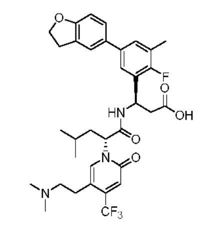 | 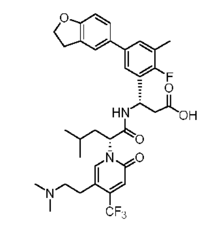 | 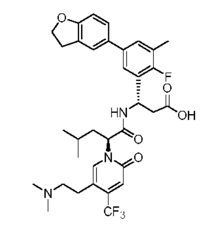 |
| 220 | 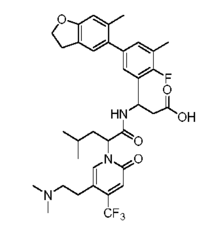 | 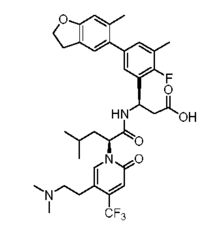 | 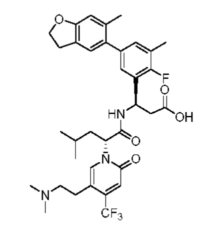 | 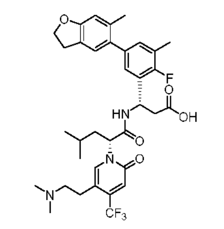 | 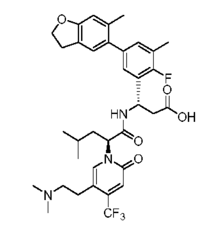 |
| 221 | 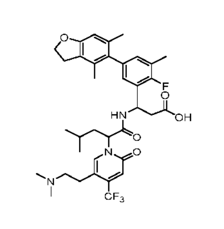 | 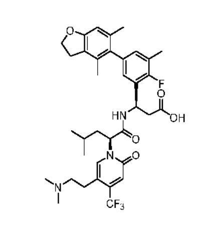 | 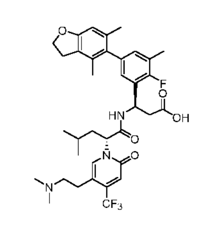 | 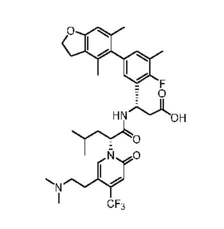 | 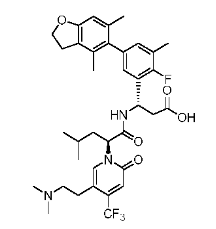 |
| 222 | 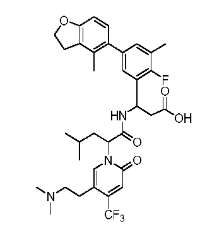 | 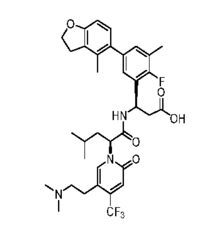 | 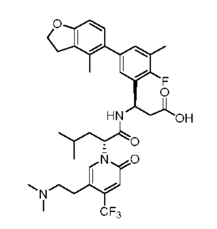 | 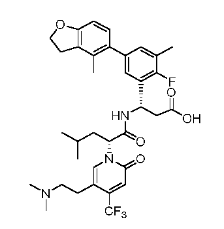 | 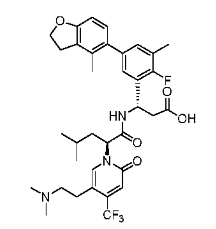 |
| 223 | 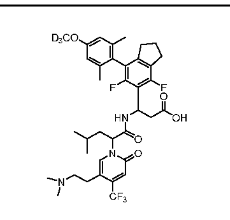 | 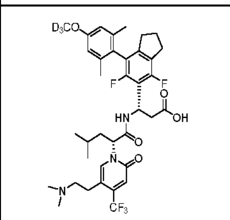 | 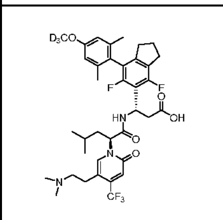 | 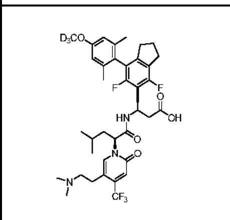 | 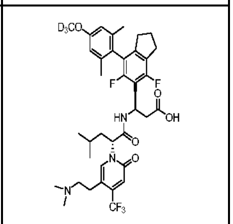 |
| 224 | 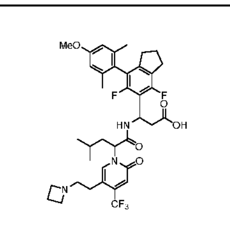 | 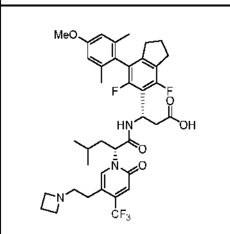 | 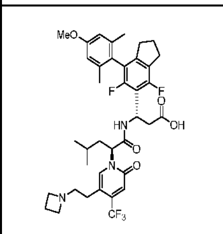 | 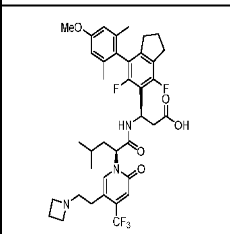 | 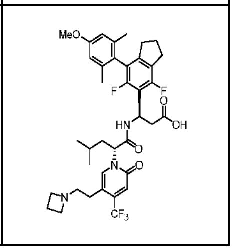 |

(continued)

| | | | | | |
|---|---|---|---|---|---|
| 225 | 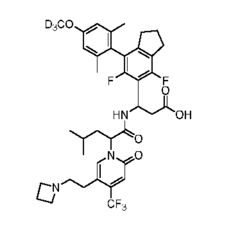 | 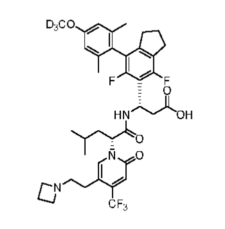 | 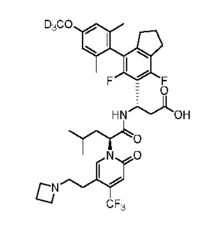 | 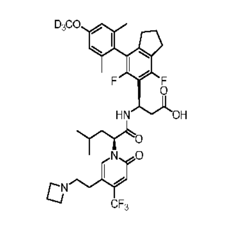 | 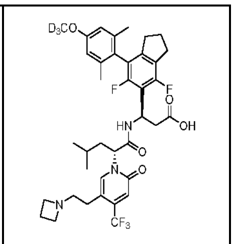 |
| 226 | 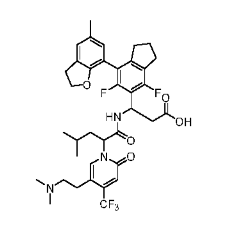 | 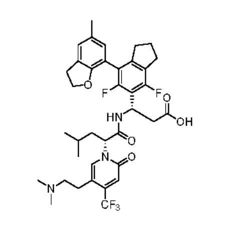 | 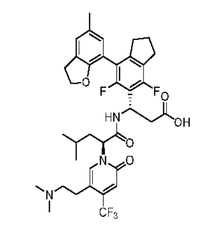 | 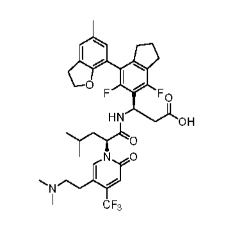 | 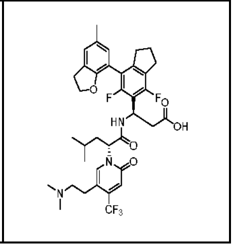 |

Table E-2

| | | | | | |
|---|---|---|---|---|---|
| 227 | 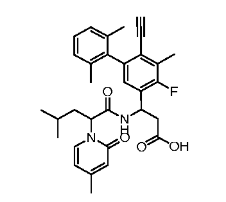 | 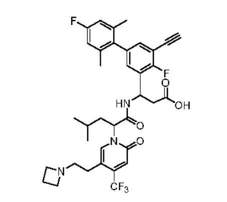 | 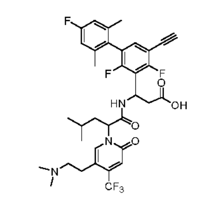 | 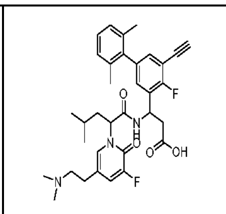 | 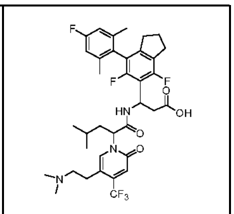 |
| 228 | 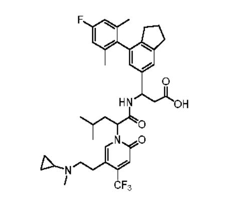 | 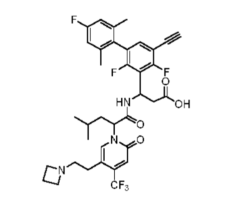 | 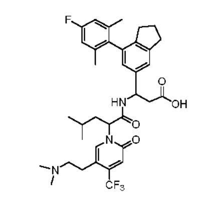 | 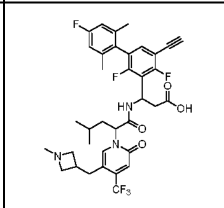 | 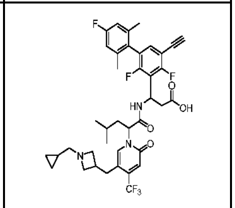 |
| 229 | 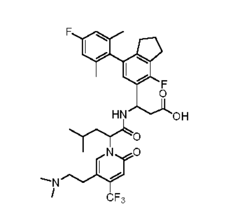 | 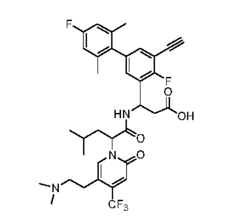 | 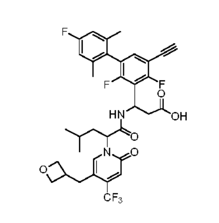 | 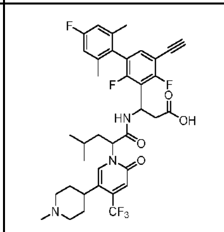 | 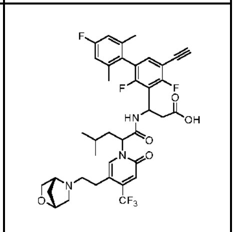 |
| 230 | 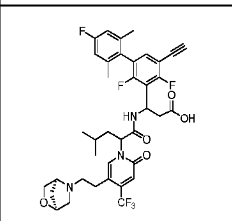 | 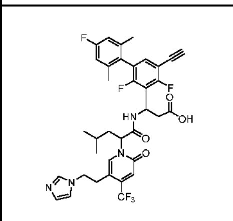 | 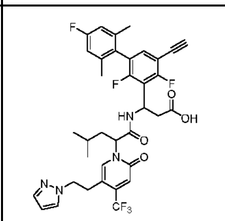 | 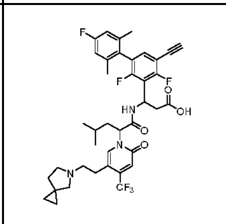 | 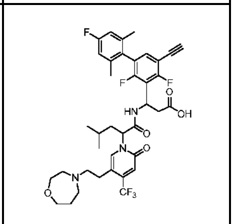 |
| 231 | 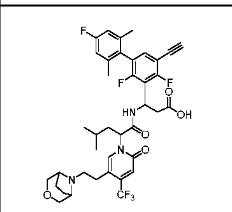 | 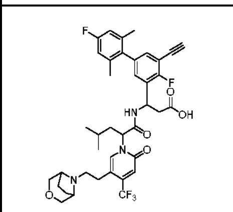 | 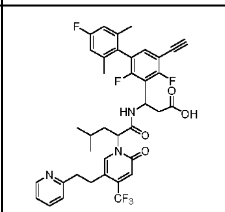 | 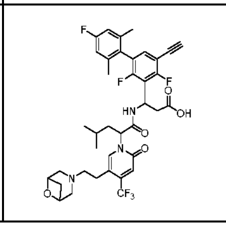 | 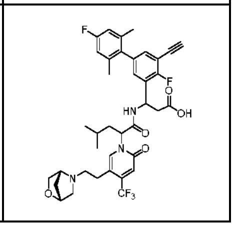 |

(continued)

| | | | | | |
|---|---|---|---|---|---|
| 232 | 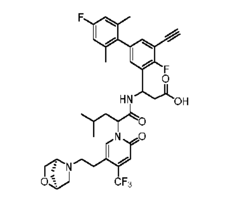 | 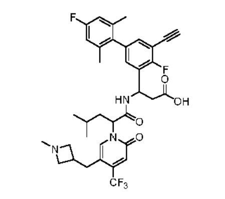 | 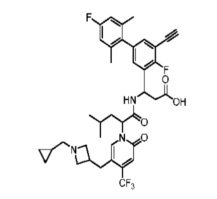 | 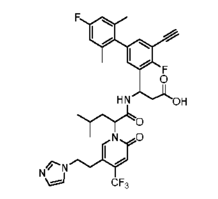 | 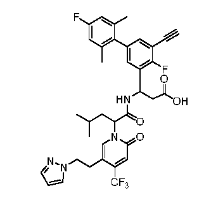 |
| 233 | 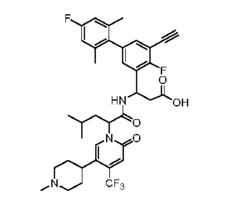 | 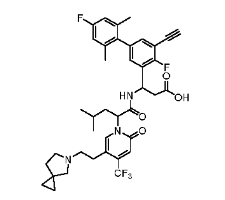 | 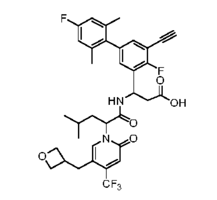 | 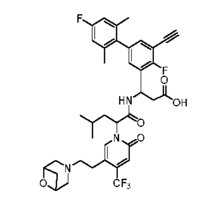 | 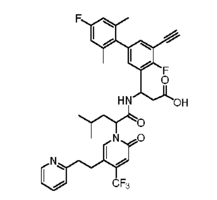 |
| 234 | 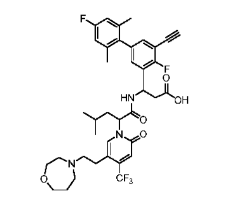 | 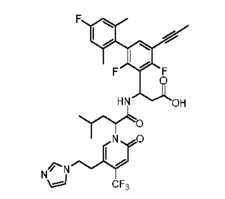 | 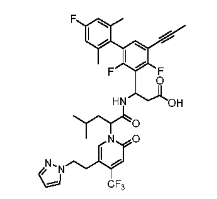 | 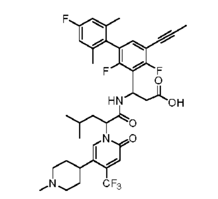 | 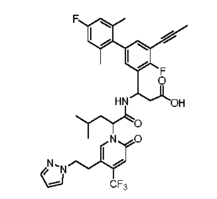 |
| 235 | 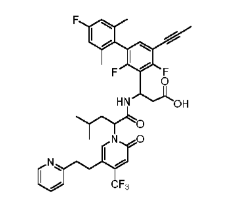 | 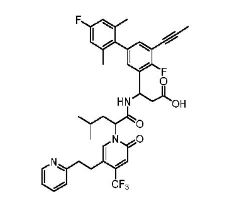 | 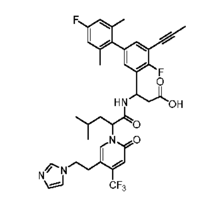 | 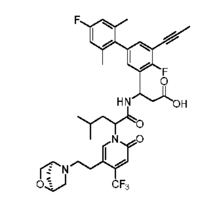 | 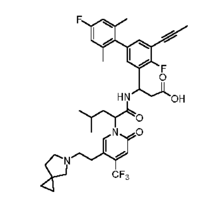 |
| 236 | 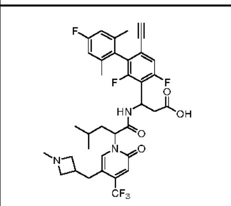 | 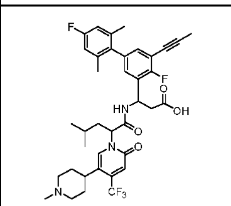 | 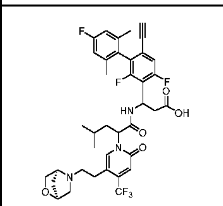 | 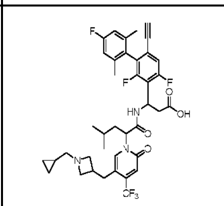 | 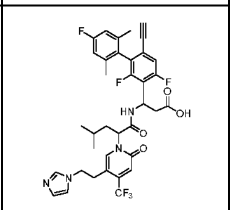 |
| 237 | 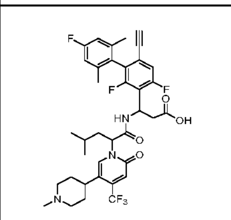 | 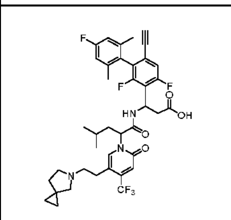 | 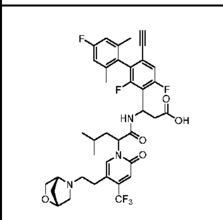 | 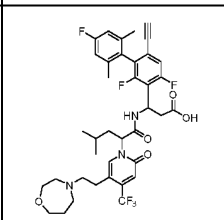 | 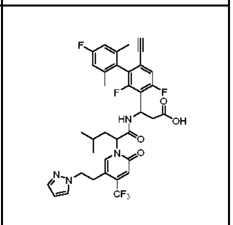 |
| 238 | 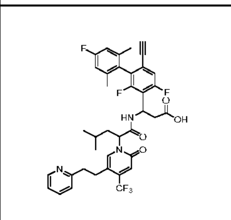 | 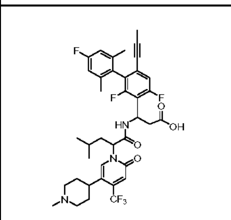 | 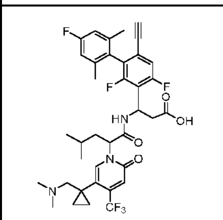 | 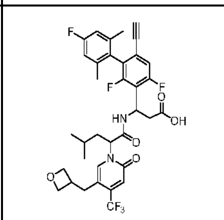 | 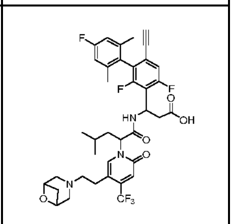 |
| 239 | 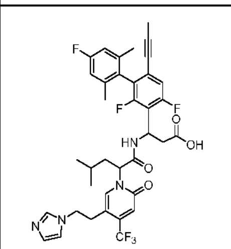 | 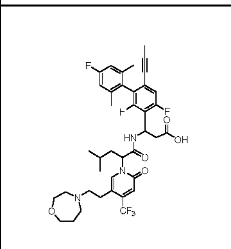 | 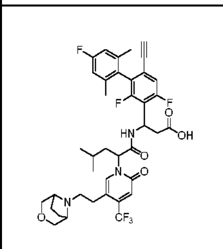 | 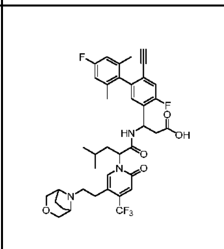 | 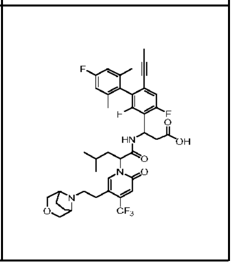 |

(continued)

| | | | | | |
|---|---|---|---|---|---|
| 240 | 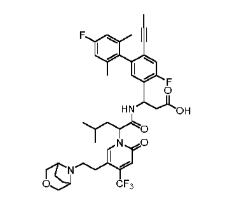 | 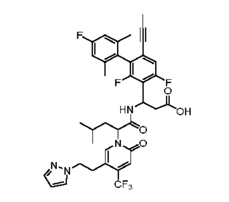 | 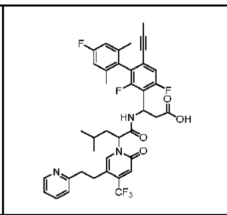 | 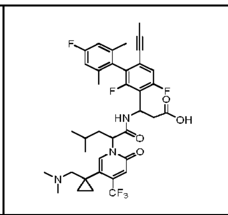 | 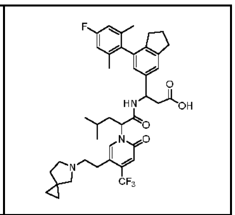 |
| 241 | 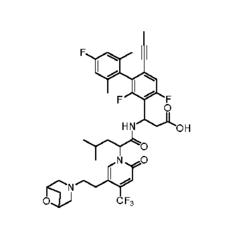 | 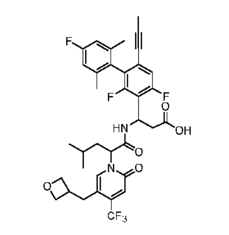 | 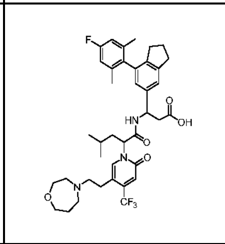 | 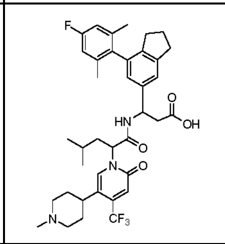 | 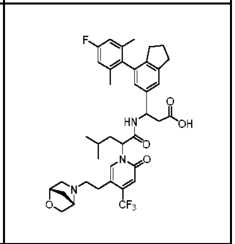 |
| 242 | 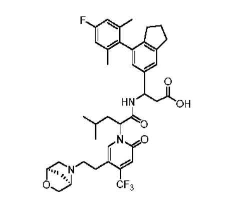 | 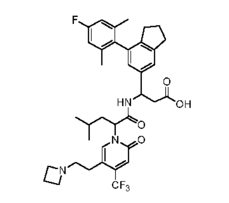 | 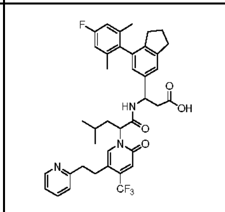 | 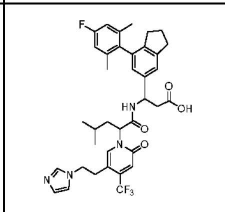 | 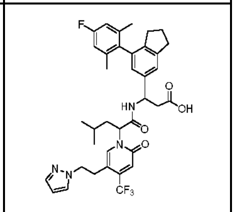 |
| 243 | 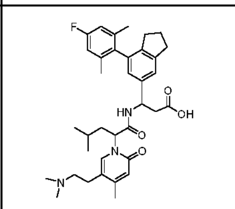 | 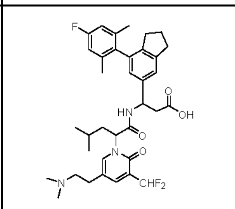 | 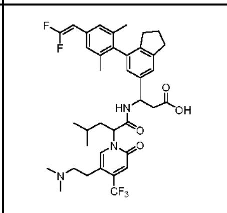 | 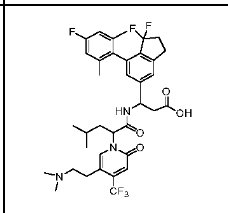 | 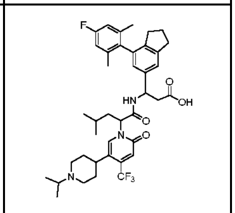 |
| 244 | 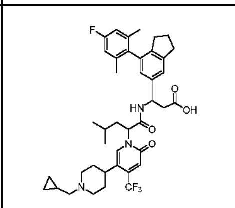 | 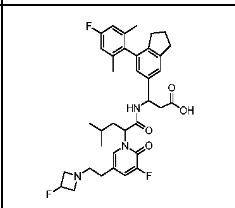 | 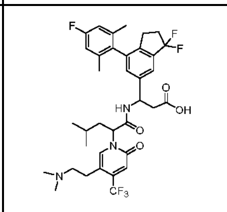 | 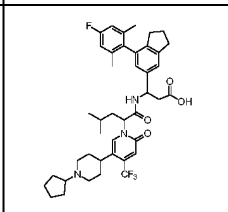 | 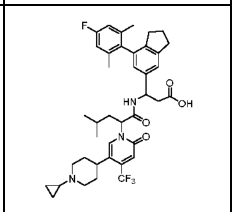 |
| 245 | 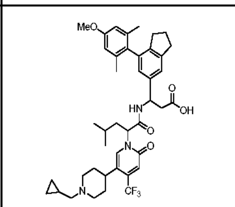 | 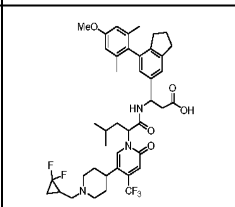 | 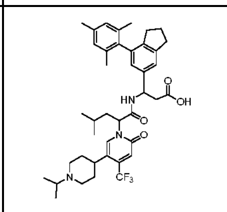 | 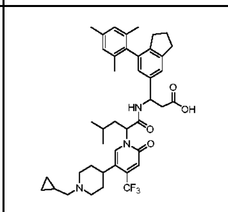 | 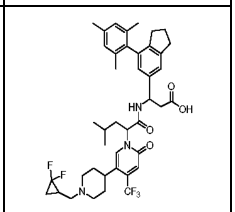 |
| 246 | 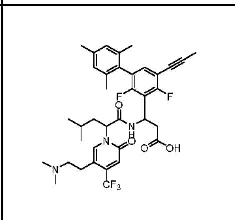 | 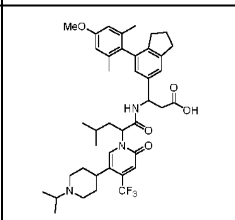 | 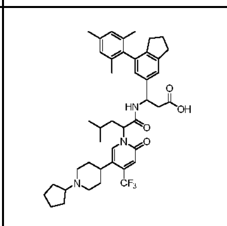 | 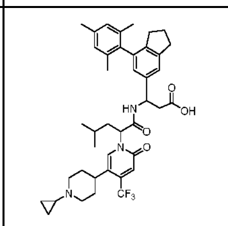 | 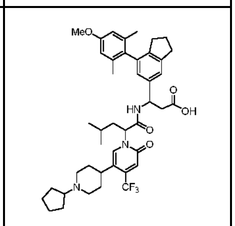 |
| 247 | 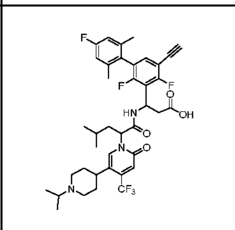 | 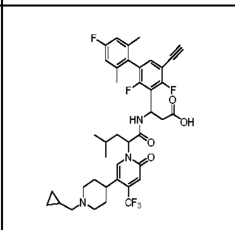 | 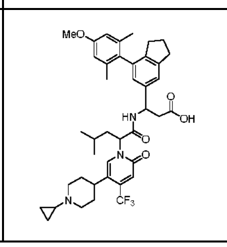 | 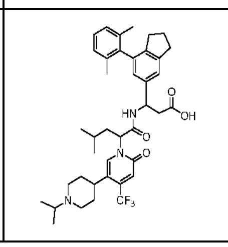 | 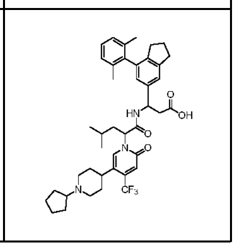 |

(continued)

| | | | | | |
|---|---|---|---|---|---|
| 248 | 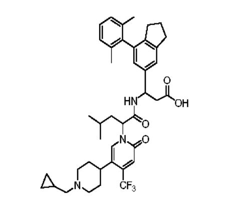 | 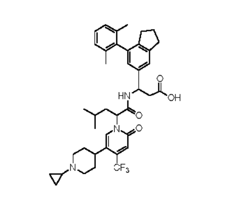 | 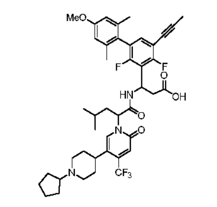 | 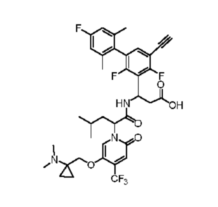 | 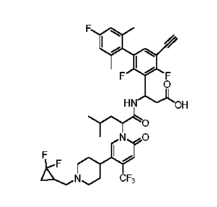 |
| 249 | 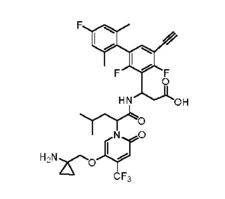 | 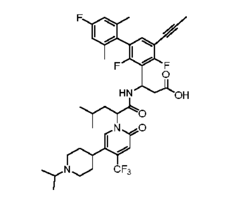 | 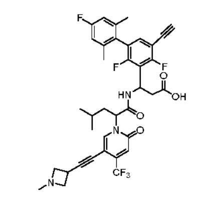 | 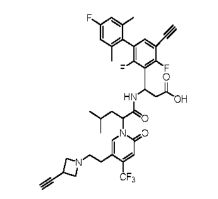 | 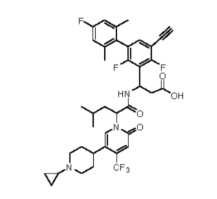 |
| 250 | 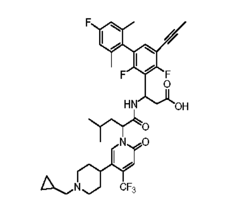 | 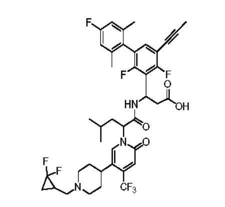 | 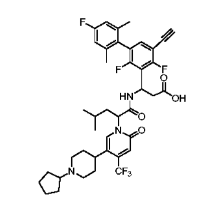 | 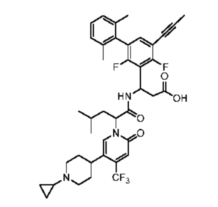 | 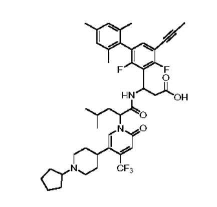 |
| 251 | 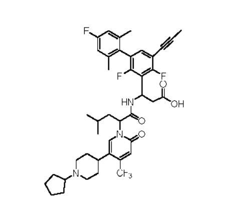 | 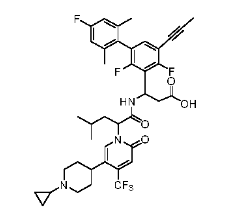 | 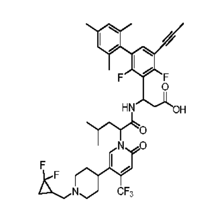 | 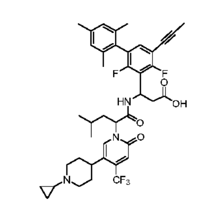 | 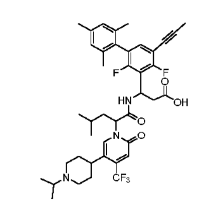 |
| 252 | 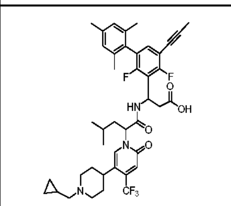 | 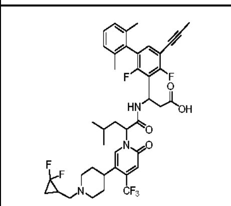 | 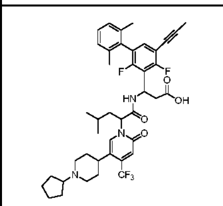 | 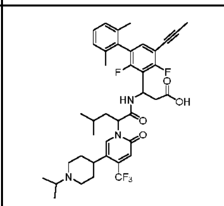 | 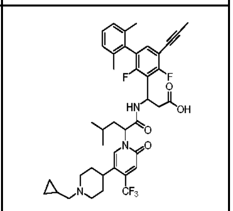 |
| 253 | 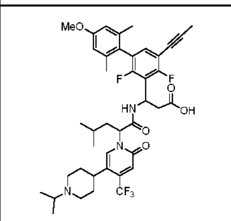 | 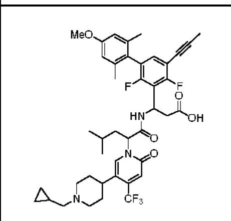 | 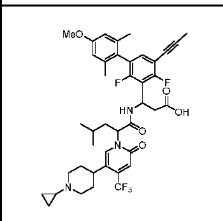 | 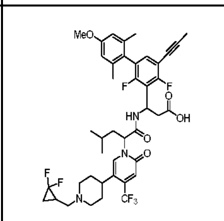 | 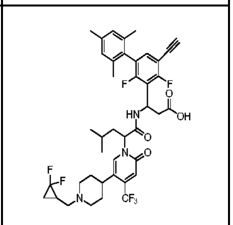 |
| 254 | 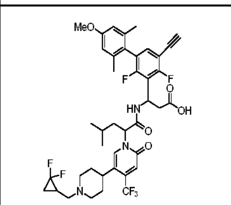 | 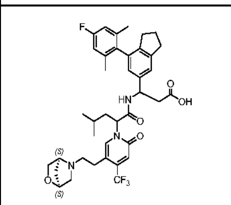 | 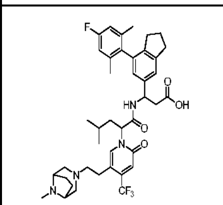 | 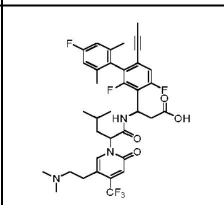 | 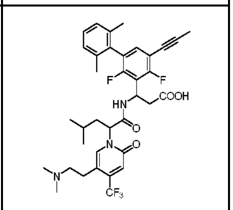 |
| 255 | 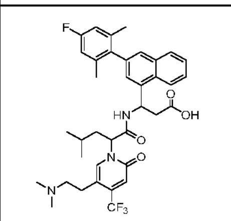 | 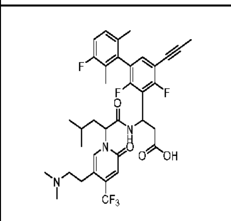 | 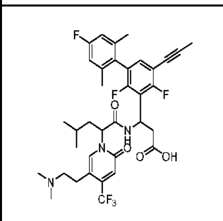 | 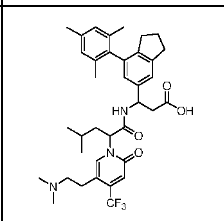 | 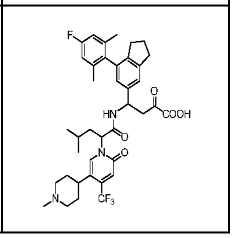 |

Table E-3

| | | | | | |
|---|---|---|---|---|---|
| 256 | 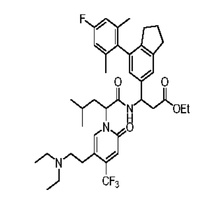 | 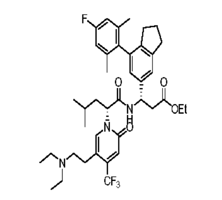 | 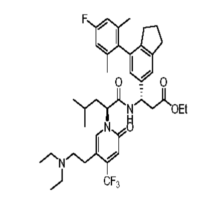 | 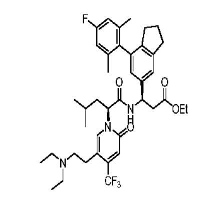 | 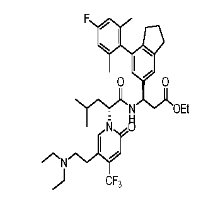 |
| 257 | 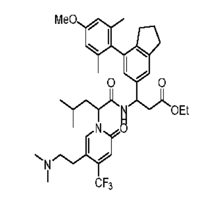 | 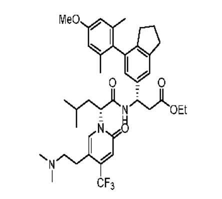 | 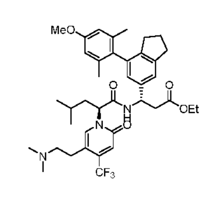 | 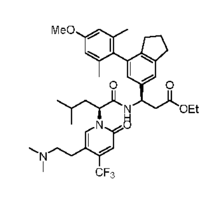 | 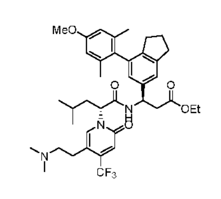 |
| 258 | 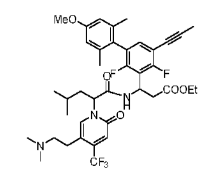 | 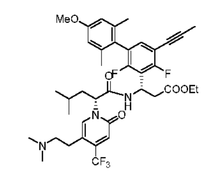 | 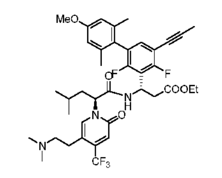 | 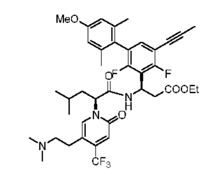 | 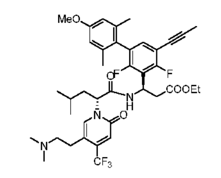 |
| 259 | 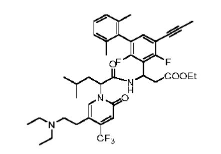 | 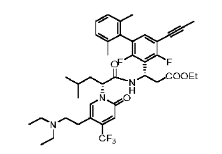 | 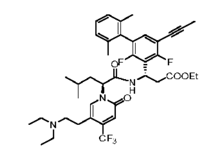 | 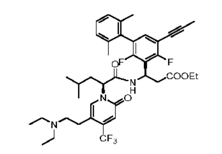 | 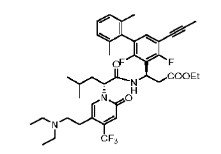 |
| 260 | 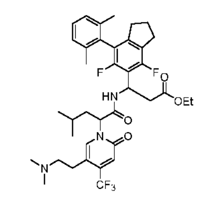 | 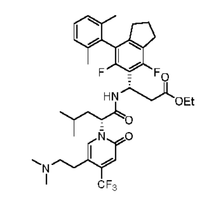 | 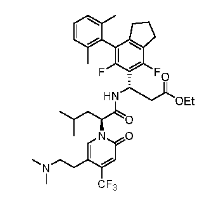 | 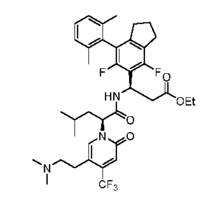 | 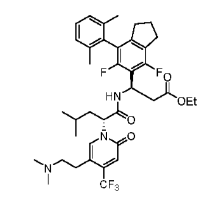 |
| 261 | 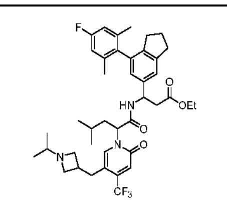 | 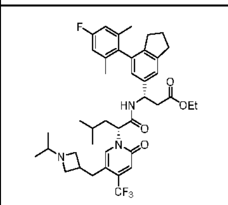 | 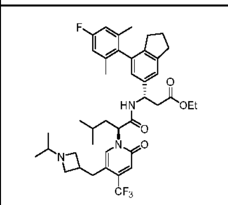 | 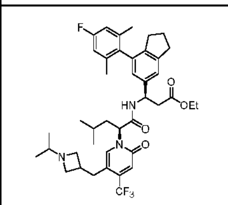 | 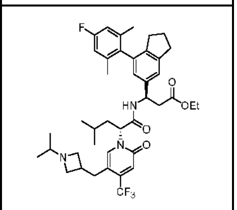 |
| 262 | 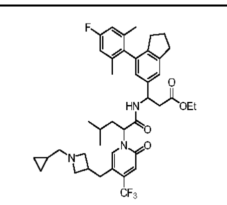 | 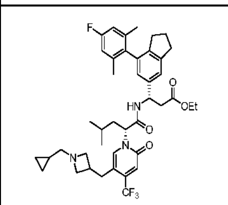 | 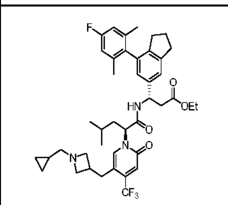 | 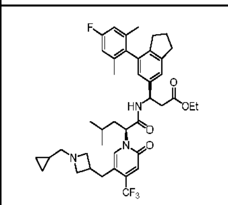 | 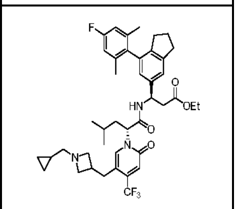 |
| 263 | 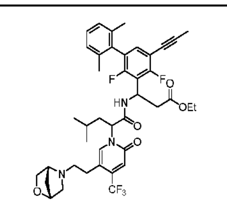 | 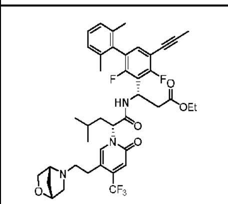 | 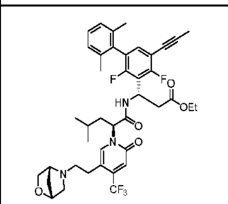 | 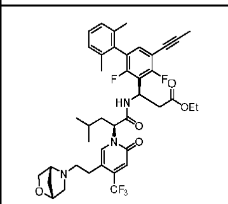 | 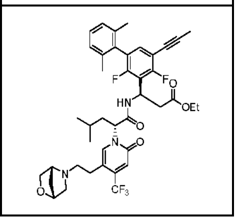 |

(continued)

| | | | | | |
|---|---|---|---|---|---|
| 264 | 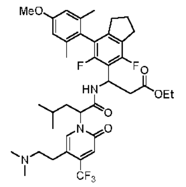 | 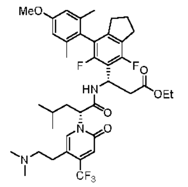 | 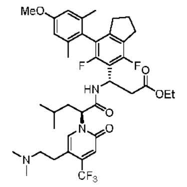 | 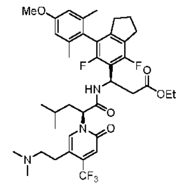 | 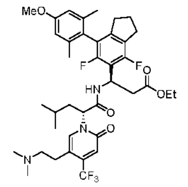 |
| 265 | 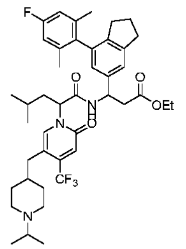 | 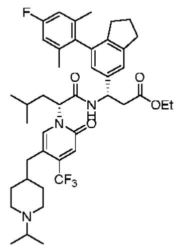 | 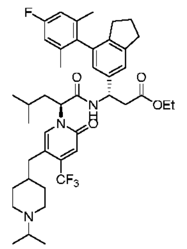 | 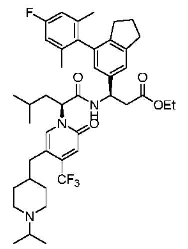 | 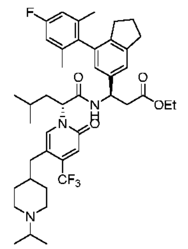 |
| 266 | 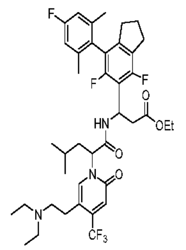 | 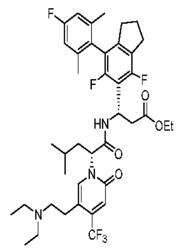 | 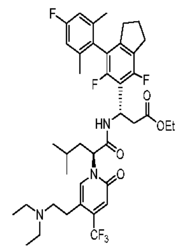 | 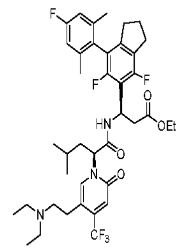 | 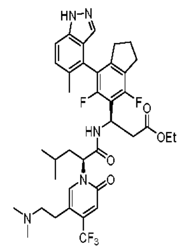 |
| 267 | 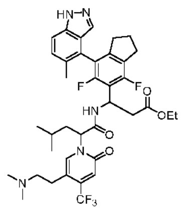 | 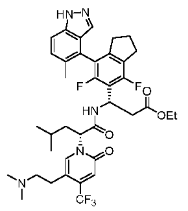 | 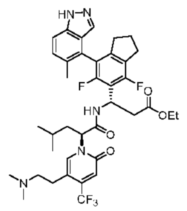 | 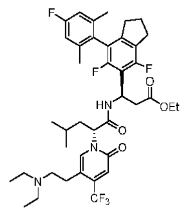 | 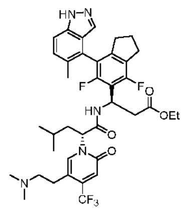 |
| 268 | 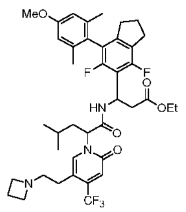 | 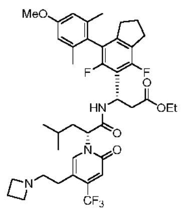 | 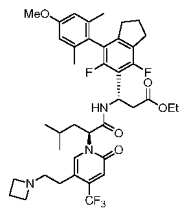 | 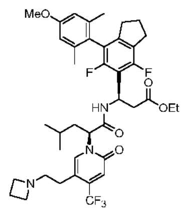 | 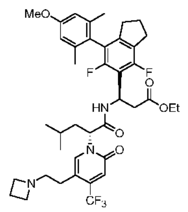 |
| 269 | 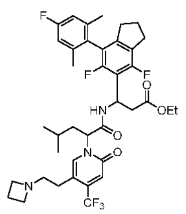 | 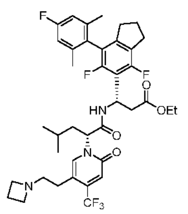 | 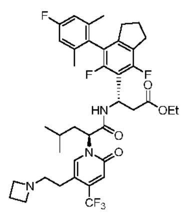 | 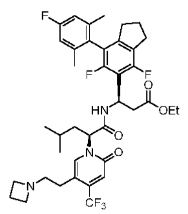 | 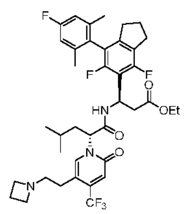 |
| 270 | 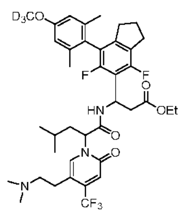 | 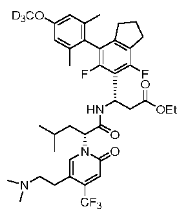 | 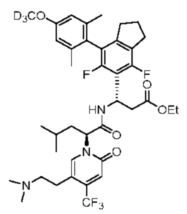 | 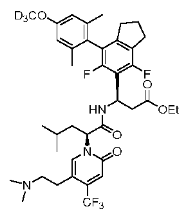 | 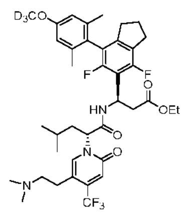 |

(continued)

| | | | | | |
|---|---|---|---|---|---|
| 271 | 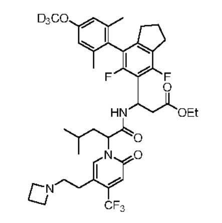 | 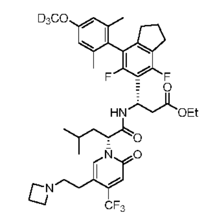 | 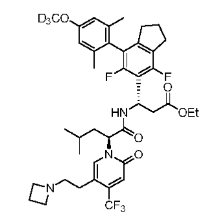 | 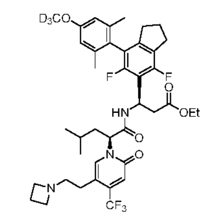 | 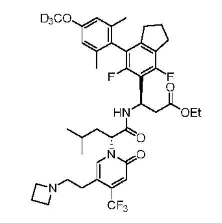 |
| 272 | 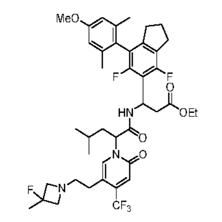 | 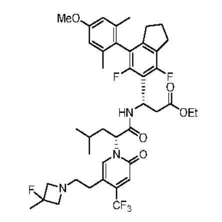 | 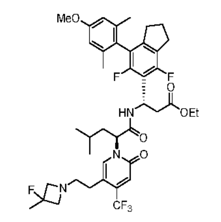 | 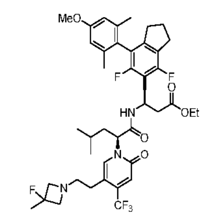 | 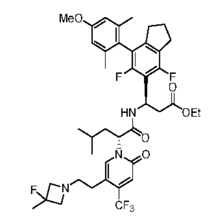 |
| 273 | 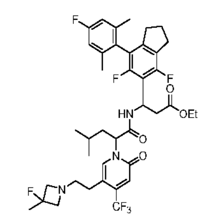 | 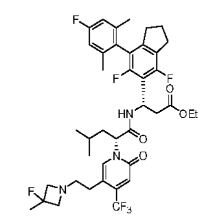 | 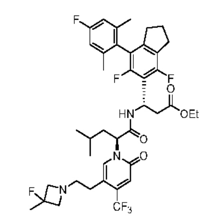 | 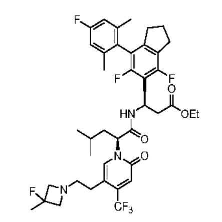 | 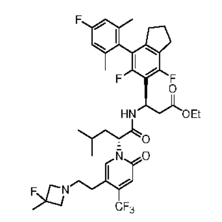 |
| 274 | 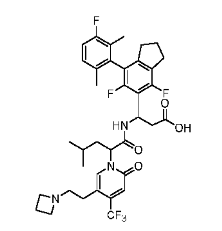 | 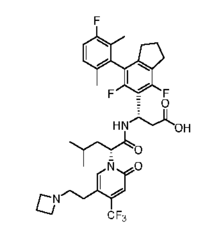 | 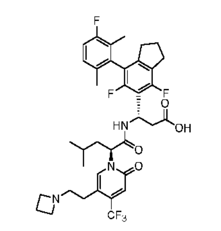 | 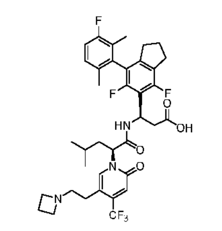 | 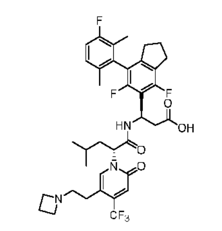 |
| 275 | 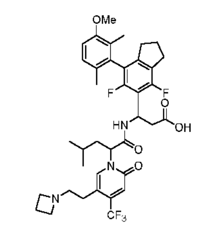 | 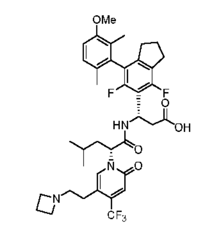 | 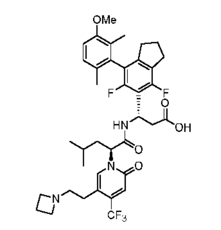 | 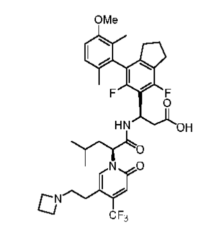 | 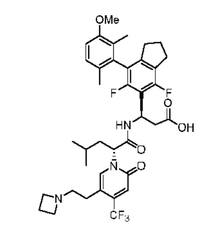 |
| 276 | 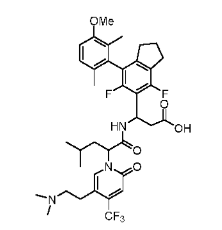 | 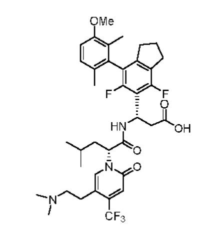 | 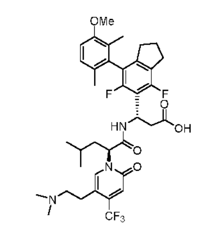 | 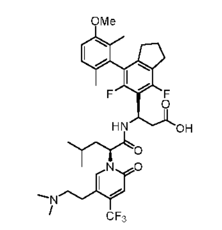 | 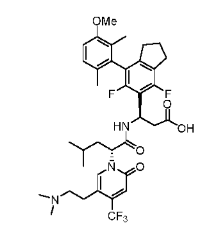 |
| 277 | 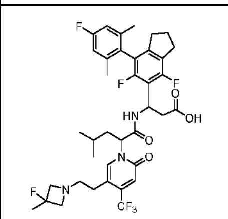 | 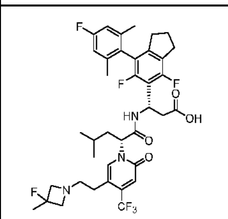 | 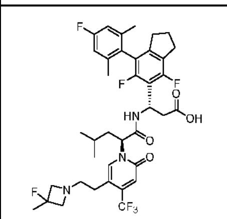 | 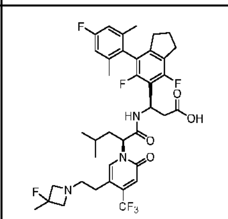 | 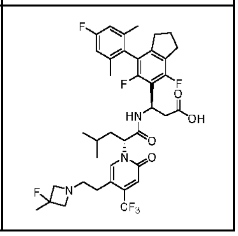 |

(continued)

| | | | | | |
|---|---|---|---|---|---|
| 278 | 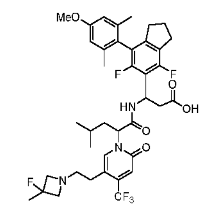 | 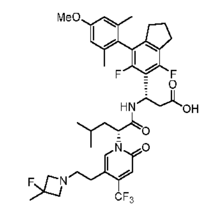 | 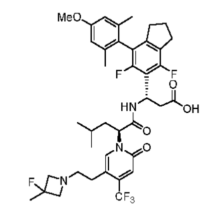 | 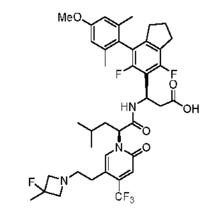 | 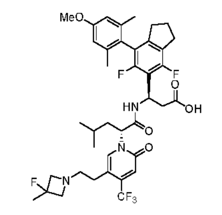 |

Table E-4

| | | | | | |
|---|---|---|---|---|---|
| 279 | 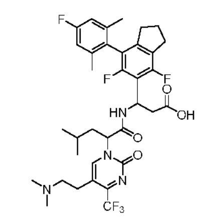 | 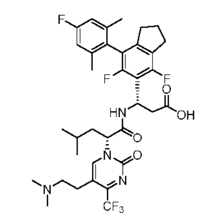 | 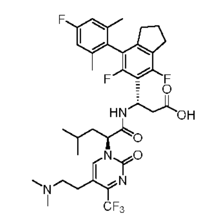 | 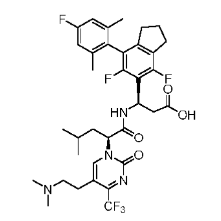 | 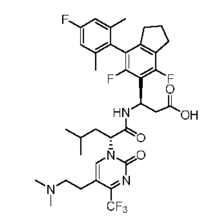 |
| 280 | 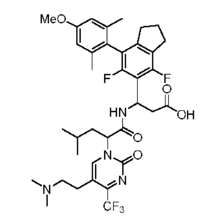 | 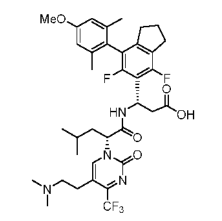 | 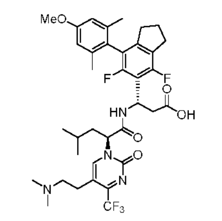 | 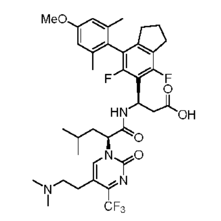 | 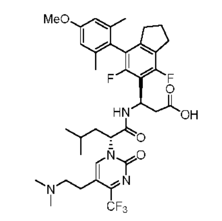 |
| 281 | 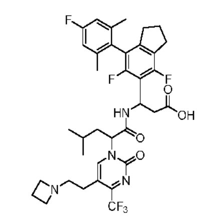 | 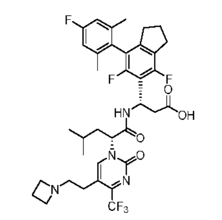 | 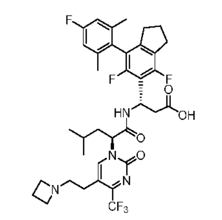 | 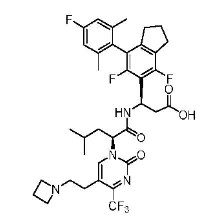 | 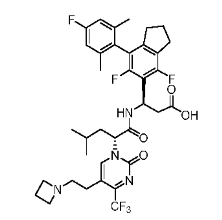 |
| 282 | 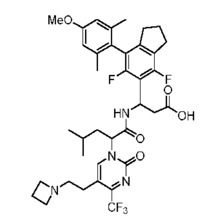 | 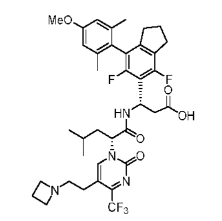 | 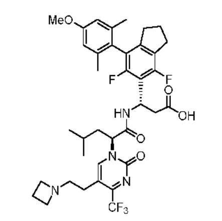 | 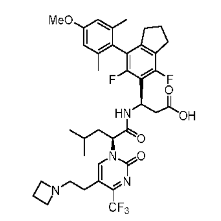 | 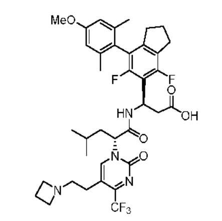 |
| 283 | 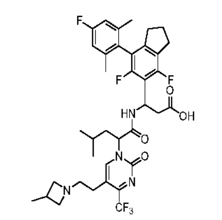 | 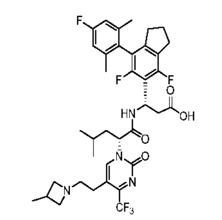 | 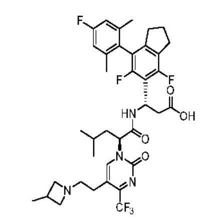 | 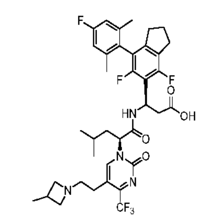 | 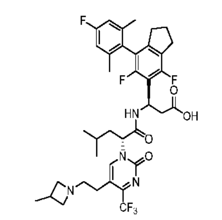 |
| 284 | 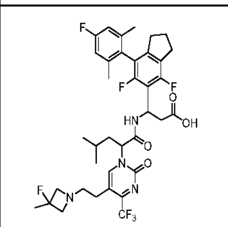 | 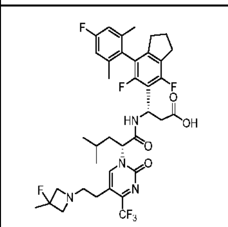 | 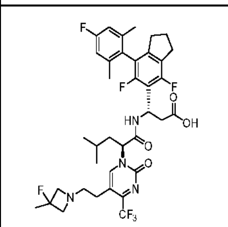 | 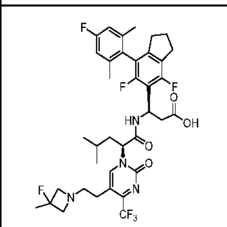 | 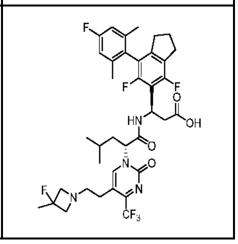 |

(continued)

| | | | | | |
|---|---|---|---|---|---|
| 285 | 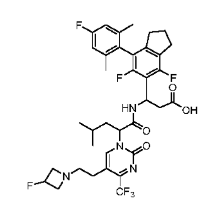 | 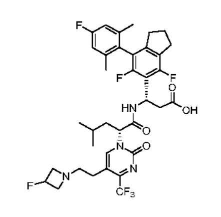 | 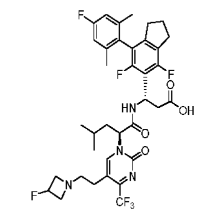 | 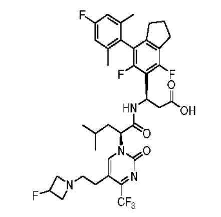 | 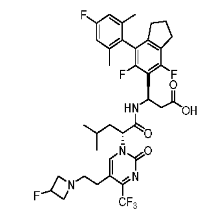 |
| 286 | 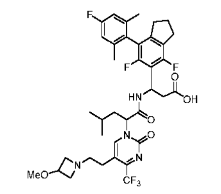 | 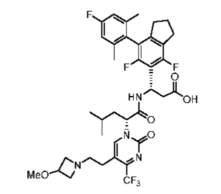 | 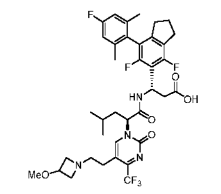 | 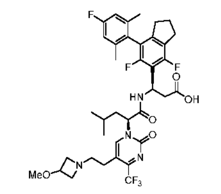 | 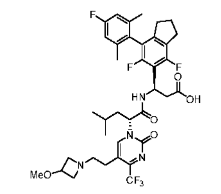 |
| 287 | 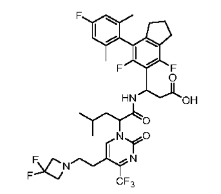 | 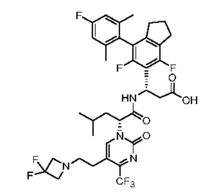 | 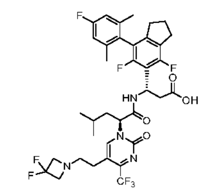 | 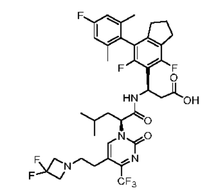 | 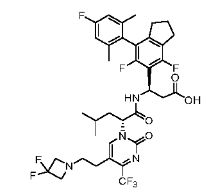 |
| 288 | 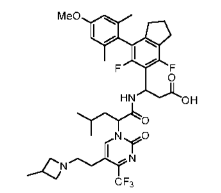 | 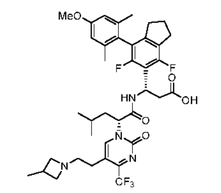 | 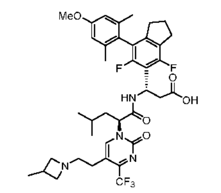 | 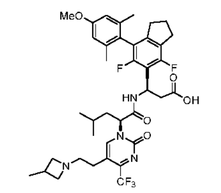 | 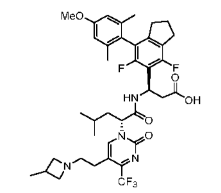 |
| 289 | 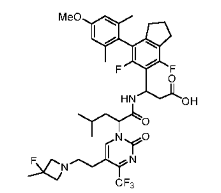 | 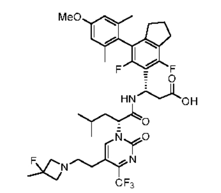 | 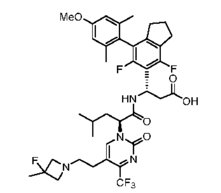 | 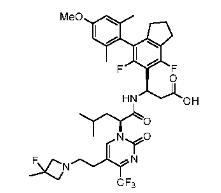 | 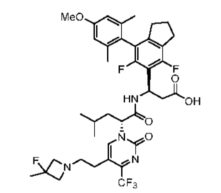 |
| 290 | 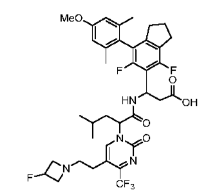 | 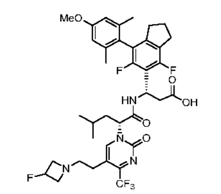 | 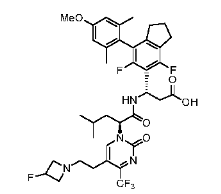 | 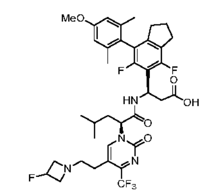 | 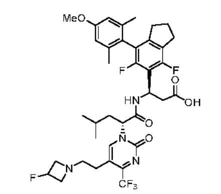 |
| 291 | 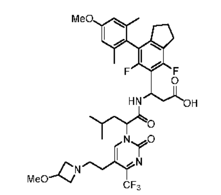 | 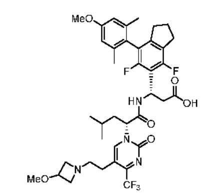 | 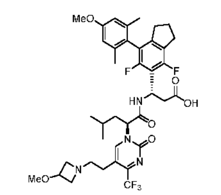 | 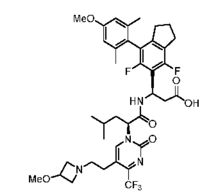 | 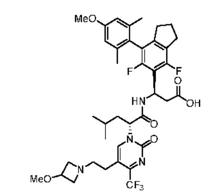 |
| 292 | 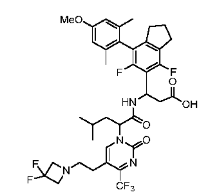 | 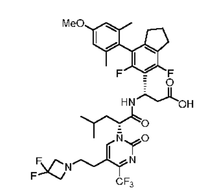 | 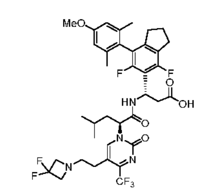 | 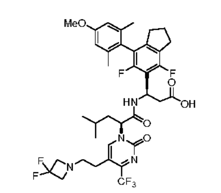 | 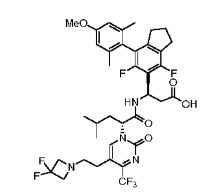 |

(continued)

| | | | | | |
|---|---|---|---|---|---|
| 293 | 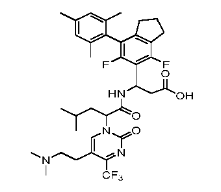 | 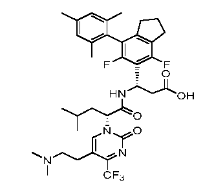 | 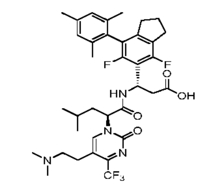 | 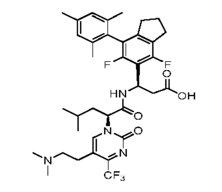 | 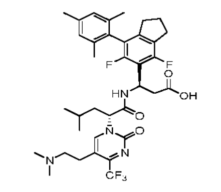 |
| 294 | 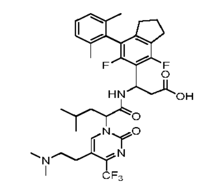 | 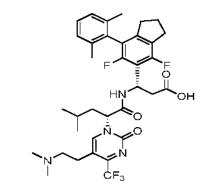 | 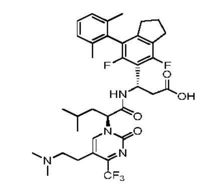 | 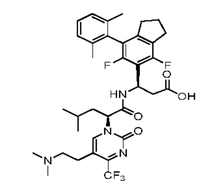 | 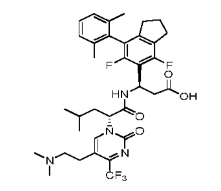 |
| 295 | 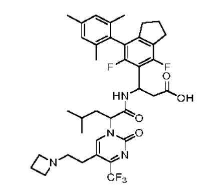 | 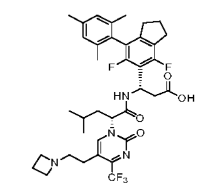 | 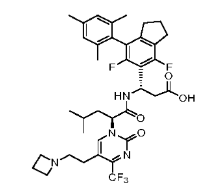 | 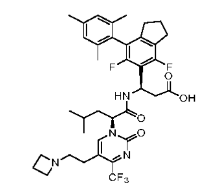 | 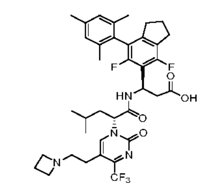 |
| 296 | 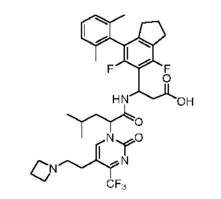 | 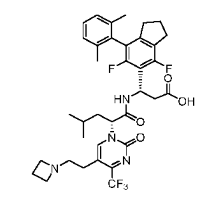 | 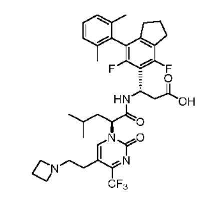 | 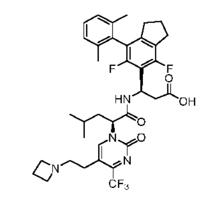 | 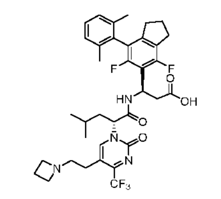 |
| 297 | 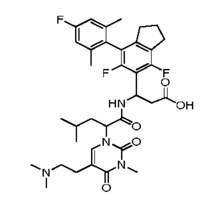 | 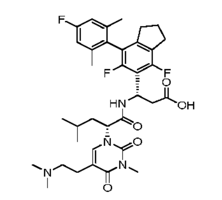 | 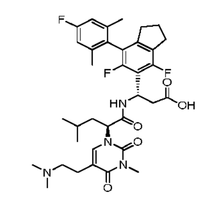 | 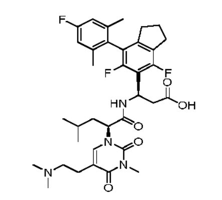 | 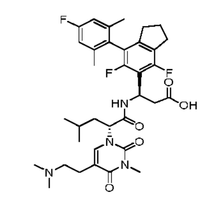 |
| 298 | 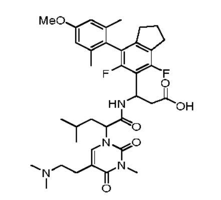 | 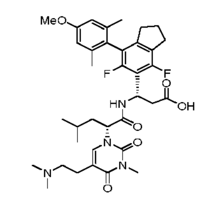 | 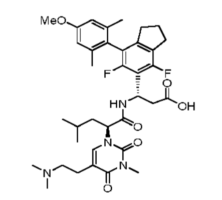 | 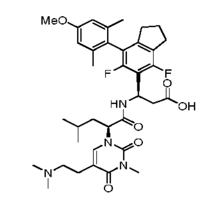 | 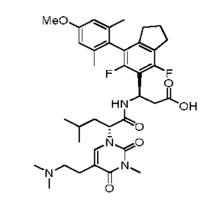 |
| 299 | 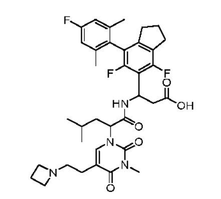 | 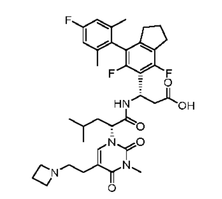 | 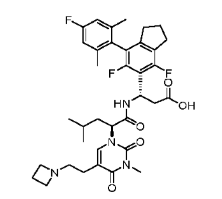 | 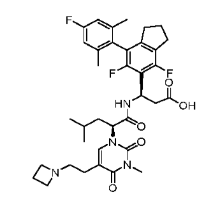 | 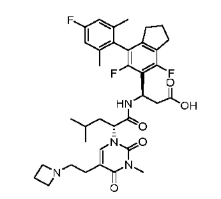 |
| 300 | 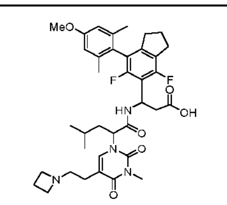 | 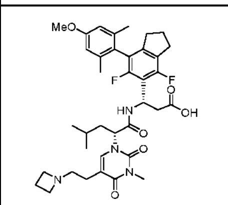 | 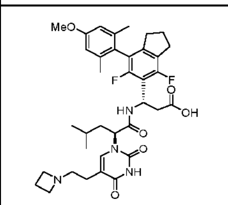 | 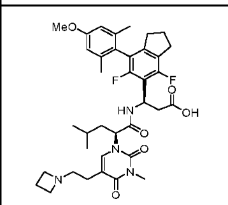 | 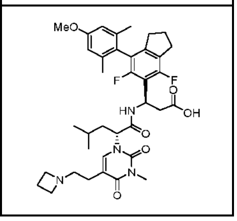 |

(continued)

| | | | | | |
|---|---|---|---|---|---|
| 301 | 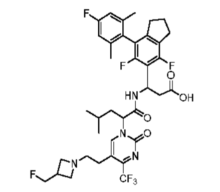 | 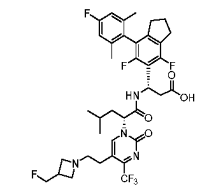 | 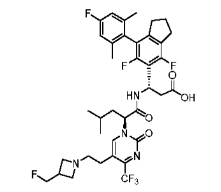 | 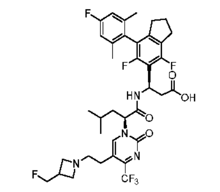 | 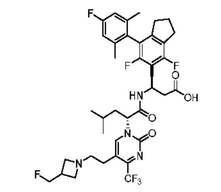 |
| 302 | 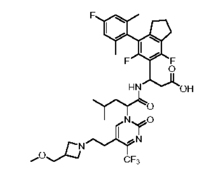 | 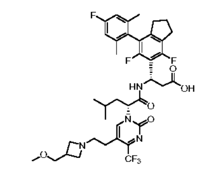 | 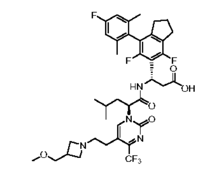 | 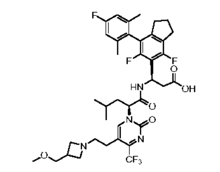 | 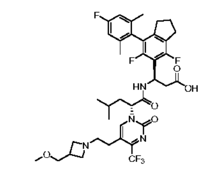 |
| 303 | 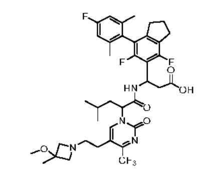 | 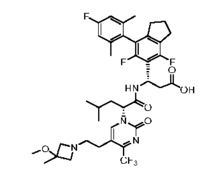 | 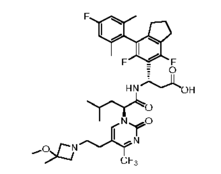 | 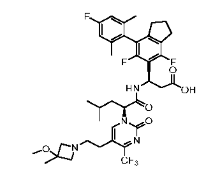 | 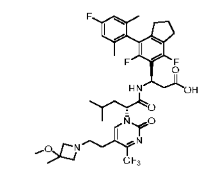 |
| 304 | 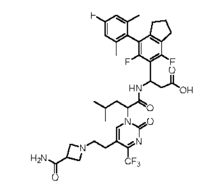 | 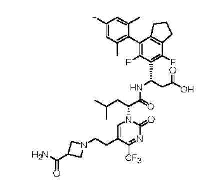 | 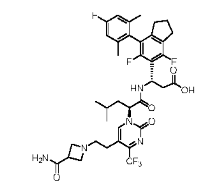 | 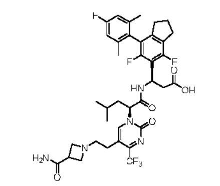 | 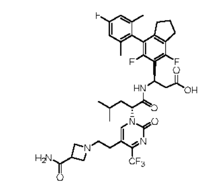 |
| 305 | 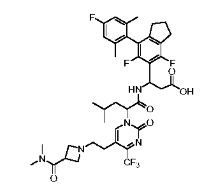 | 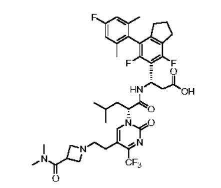 | 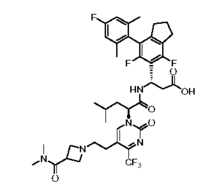 | 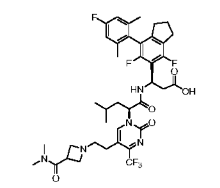 | 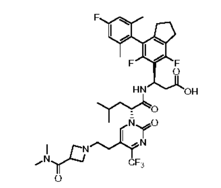 |
| 306 | 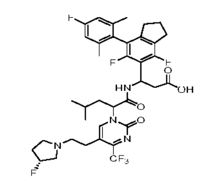 | 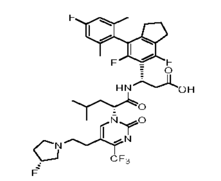 | 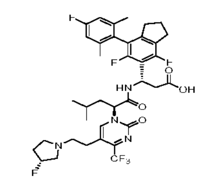 | 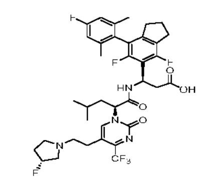 | 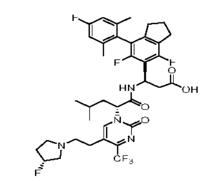 |
| 307 | 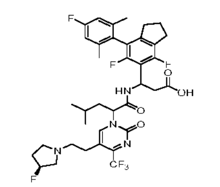 | 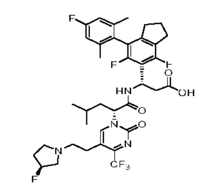 | 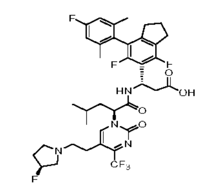 | 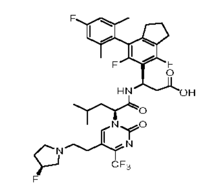 | 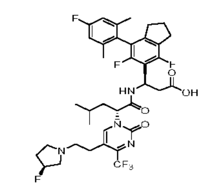 |
| 308 | 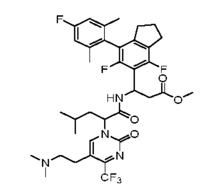 | 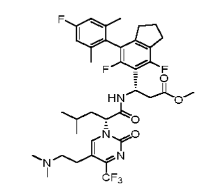 | 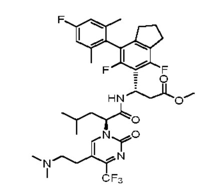 | 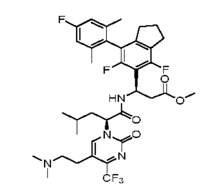 | 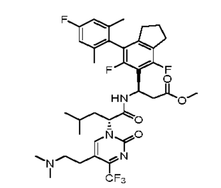 |
| 309 | | | | | |

(continued)

| | | | | | |
|---|---|---|---|---|---|
| 310 | 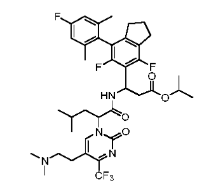 | 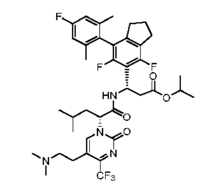 | 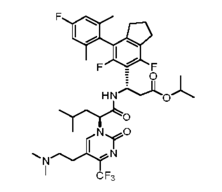 | 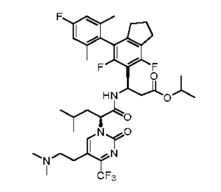 | 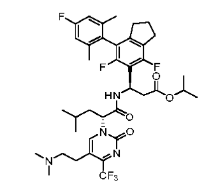 |
| 311 | 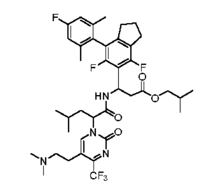 | 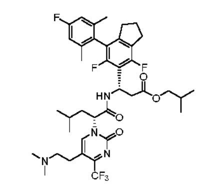 | 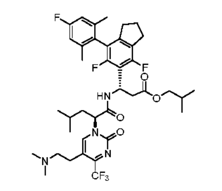 | 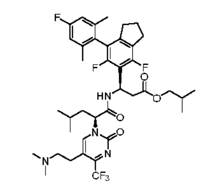 | 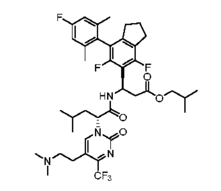 |
| 312 | 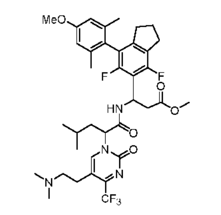 | 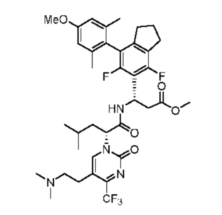 | 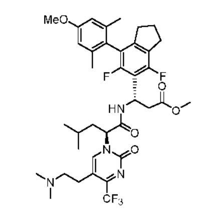 | 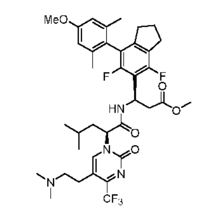 | 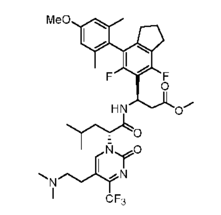 |
| 313 | 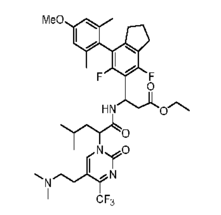 | 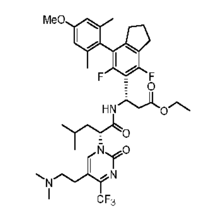 | 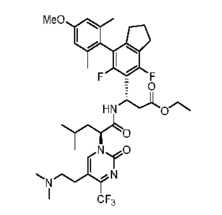 | 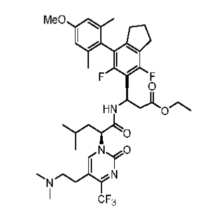 | 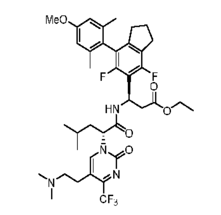 |
| 314 | 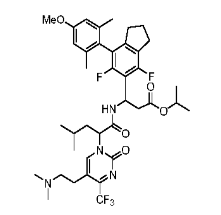 | 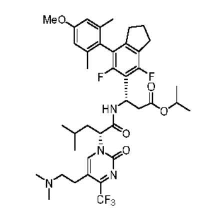 | 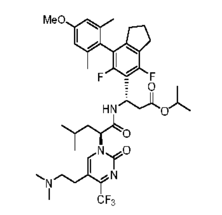 | 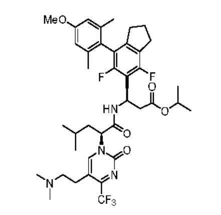 | 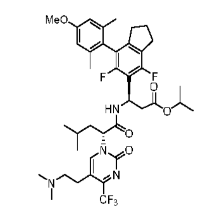 |
| 315 | 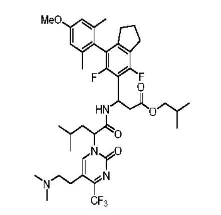 | 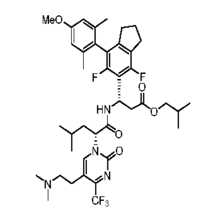 | 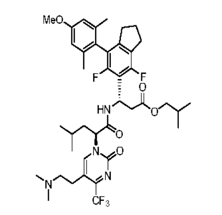 | 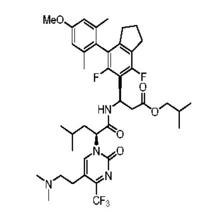 | 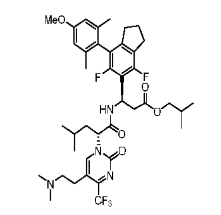 |
| 316 | 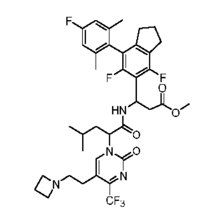 | 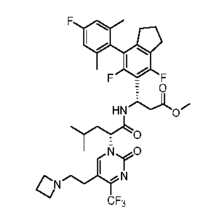 | 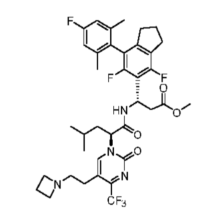 | 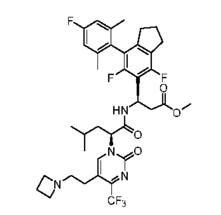 | 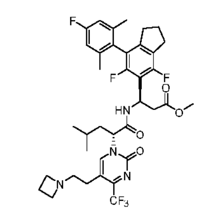 |
| 317 | 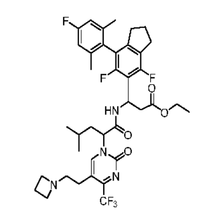 | 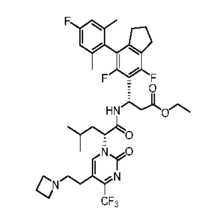 | 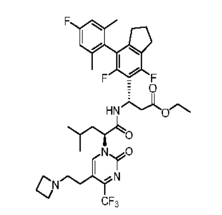 | 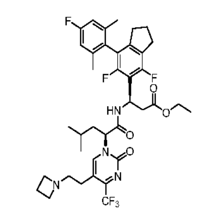 | 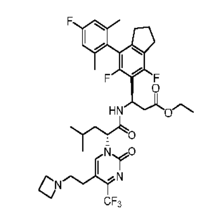 |

(continued)

| | | | | | |
|---|---|---|---|---|---|
| 318 | 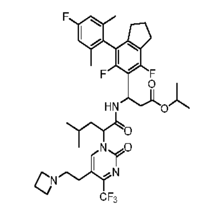 | 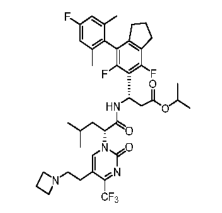 | 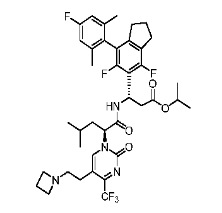 | 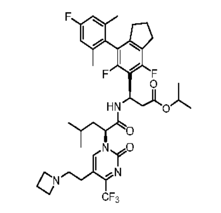 | 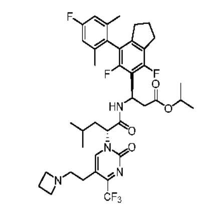 |
| 319 | 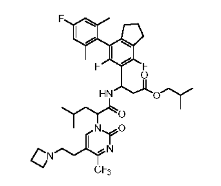 | 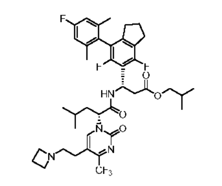 | 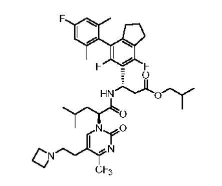 | 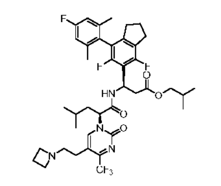 | 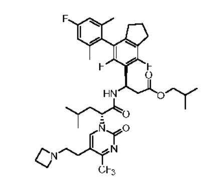 |
| 320 | 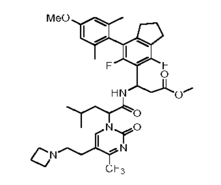 | 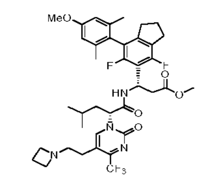 | 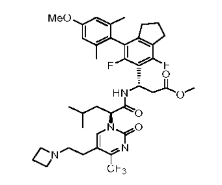 | 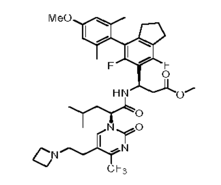 | 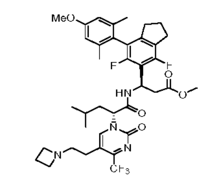 |
| 321 | 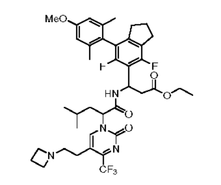 | 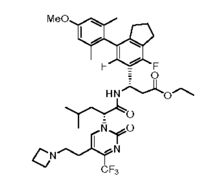 | 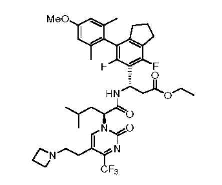 | 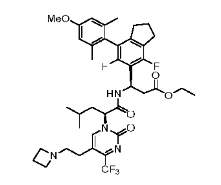 | 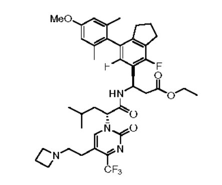 |
| 322 | 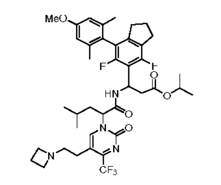 | 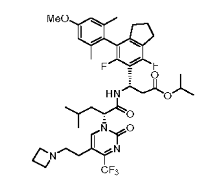 | 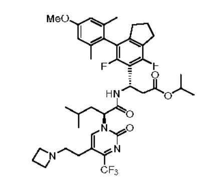 | 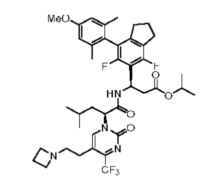 | 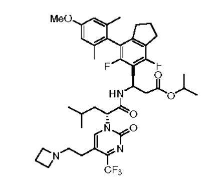 |
| 323 | 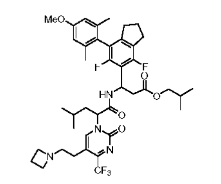 | 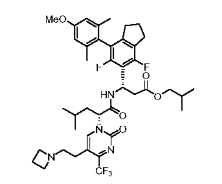 | 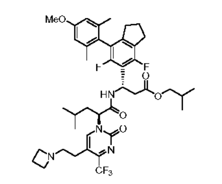 | 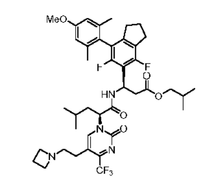 | 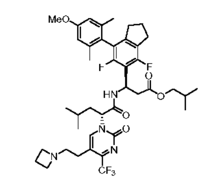 |
| 324 | 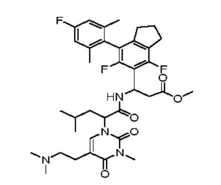 | 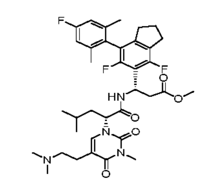 | 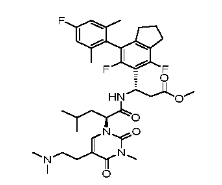 | 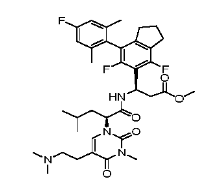 | 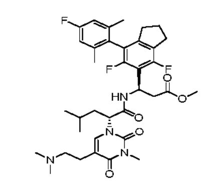 |
| 325 | 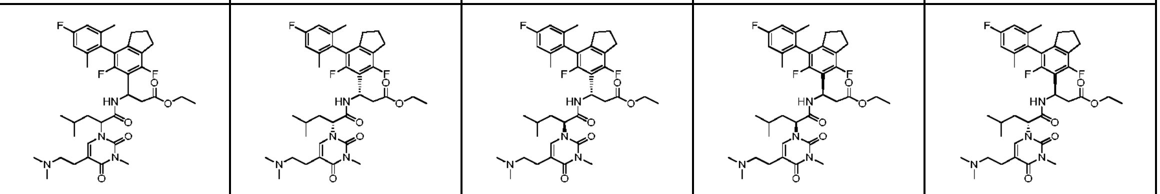 | | | | |
| 326 | 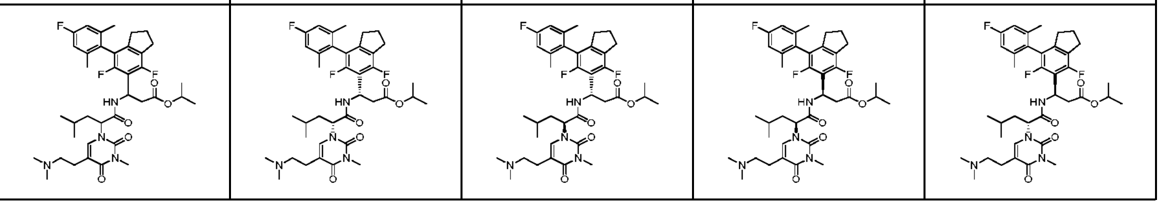 | | | | |

(continued)

| | | | | | |
|---|---|---|---|---|---|
| 327 | 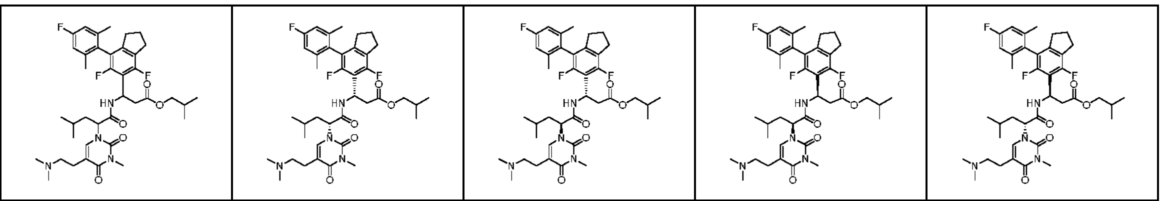 | | | | |
| 328 | 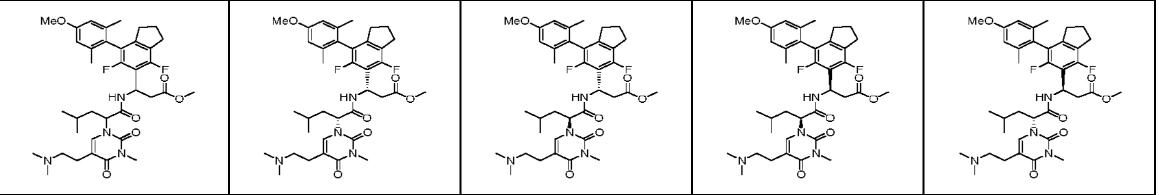 | | | | |
| 329 | 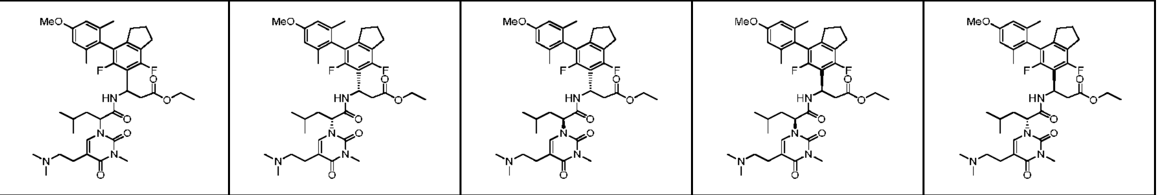 | | | | |
| 330 | 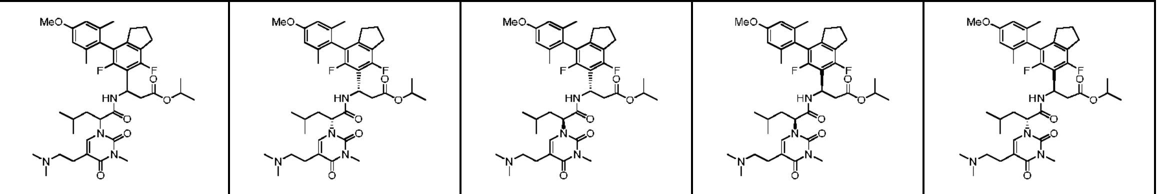 | | | | |
| 331 | 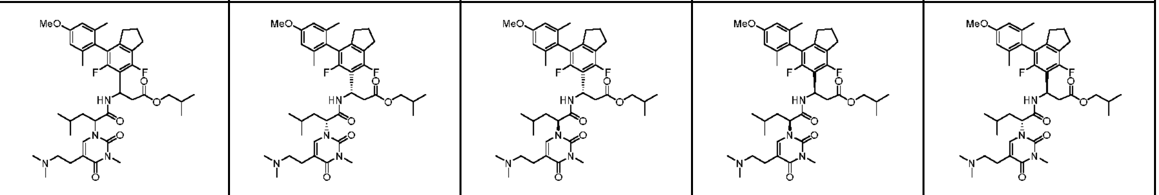 | | | | |
| 332 | 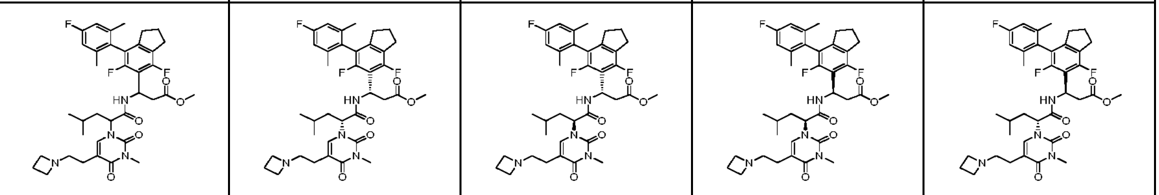 | | | | |
| 333 | 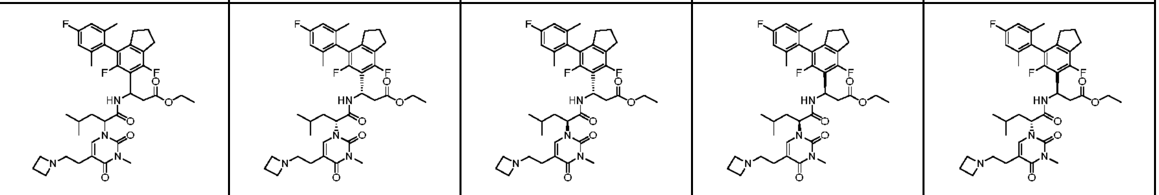 | | | | |
| 334 | 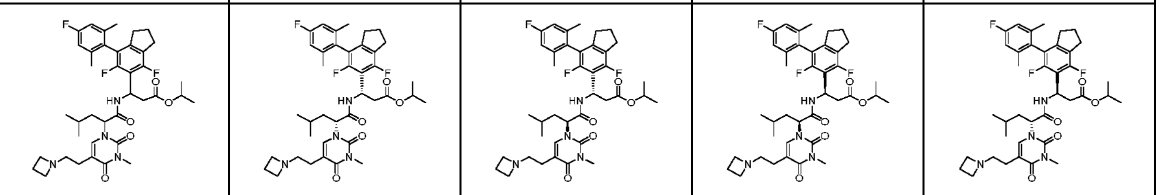 | | | | |
| 335 | 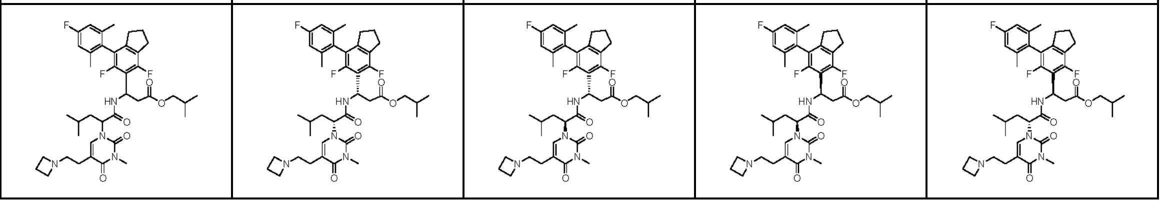 | | | | |

(continued)

| | | | | | |
|---|---|---|---|---|---|
| 336 | 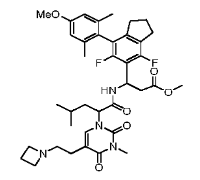 | 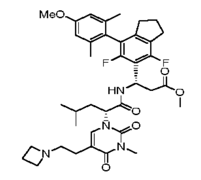 | 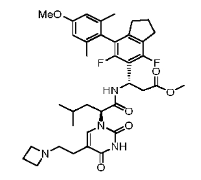 | 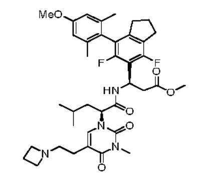 | 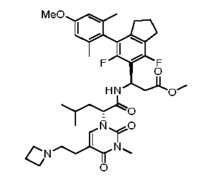 |
| 337 | 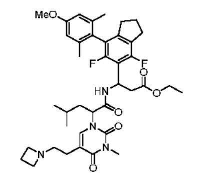 | 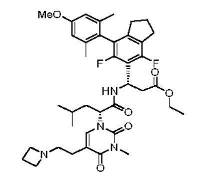 | 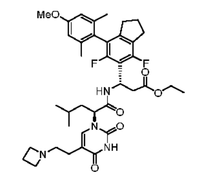 | 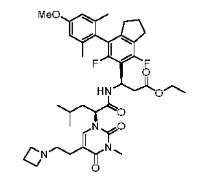 | 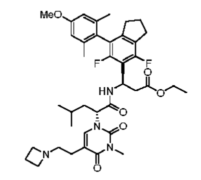 |
| 338 | 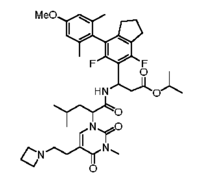 | 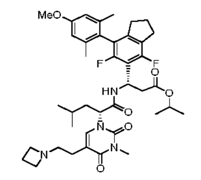 | 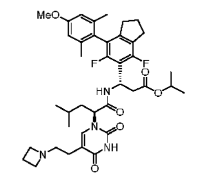 | 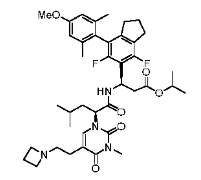 | 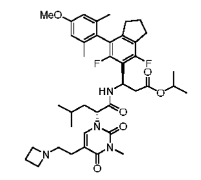 |
| 339 | 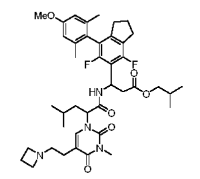 | 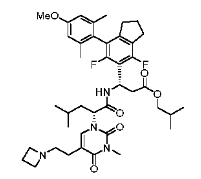 | 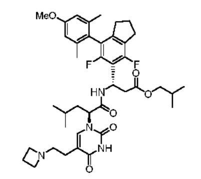 | 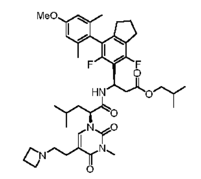 | 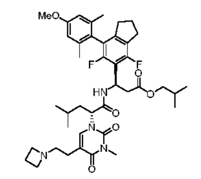 |
| 340 | 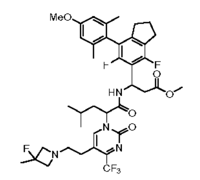 | 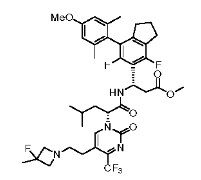 | 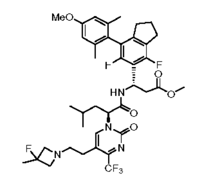 | 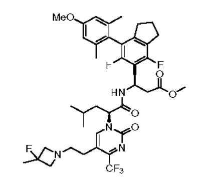 | 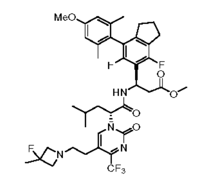 |
| 341 | 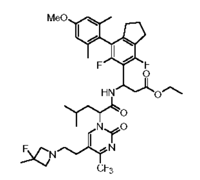 | 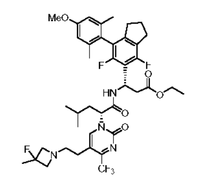 | 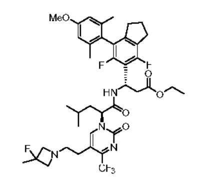 | 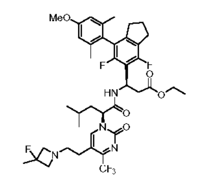 | 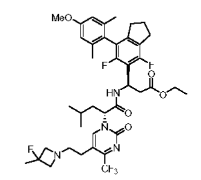 |
| 342 | 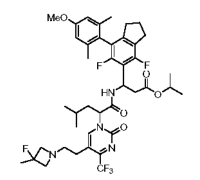 | 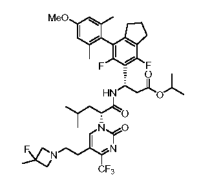 | 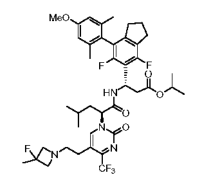 | 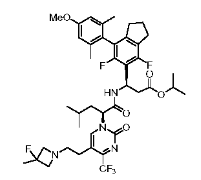 | 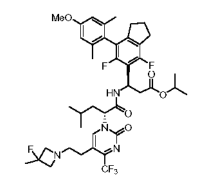 |
| 343 | 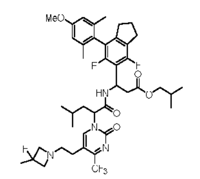 | 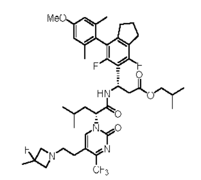 | 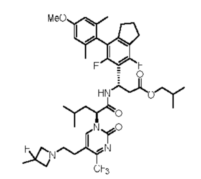 | 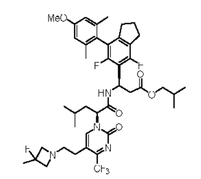 | 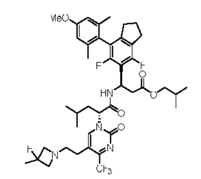 |
| 344 | | | | | |

(continued)

| | | | | | |
|---|---|---|---|---|---|
| 345 | 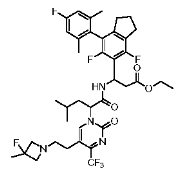 | 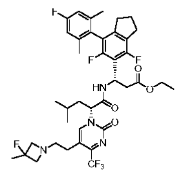 | 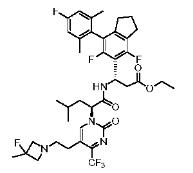 | 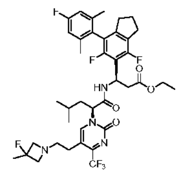 | 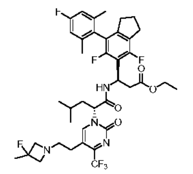 |
| 346 | 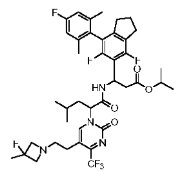 | 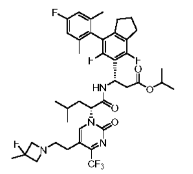 | 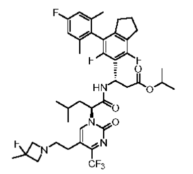 | 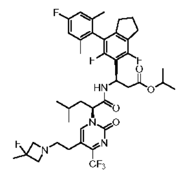 | 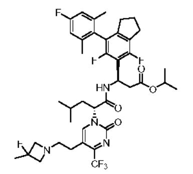 |
| 347 | 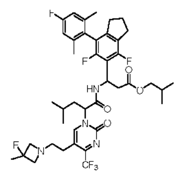 | 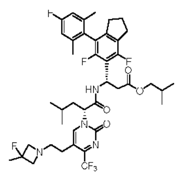 | 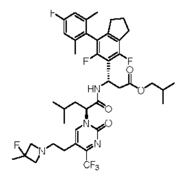 | 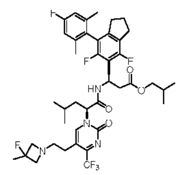 | 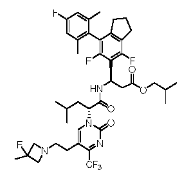 |
| 348 | 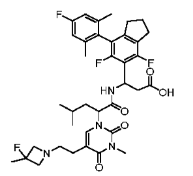 | 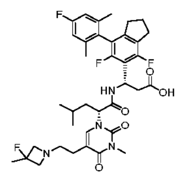 | 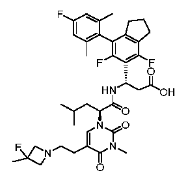 | 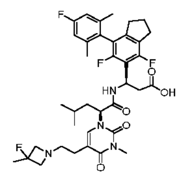 | 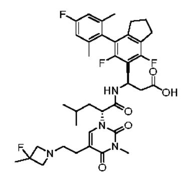 |
| 349 | 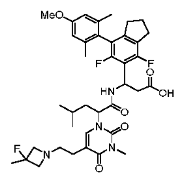 | 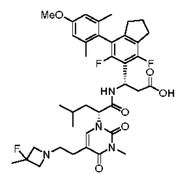 | 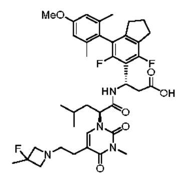 | 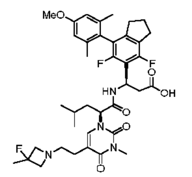 | 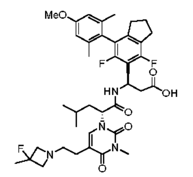 |
| 350 | 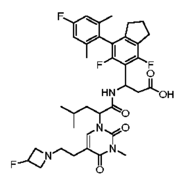 | 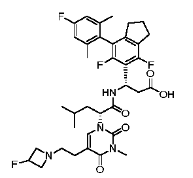 | 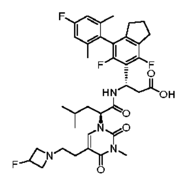 | 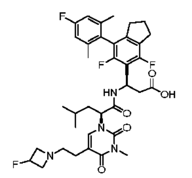 | 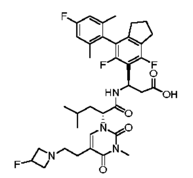 |
| 351 | 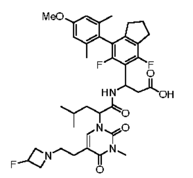 | 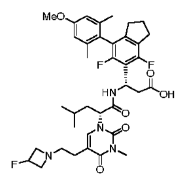 | 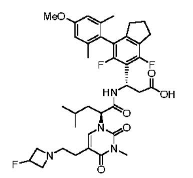 | 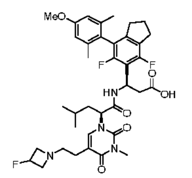 | 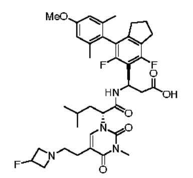 |
| 352 | 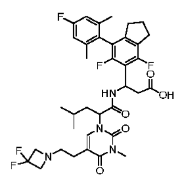 | 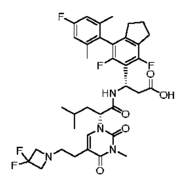 | 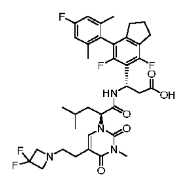 | 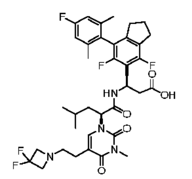 | 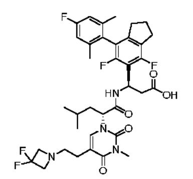 |
| 353 | | | | | |

(continued)

| | | | | | |
|---|---|---|---|---|---|
| 354 | 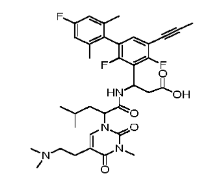 | 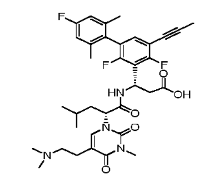 | 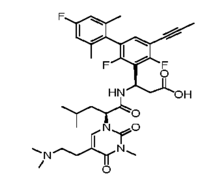 | 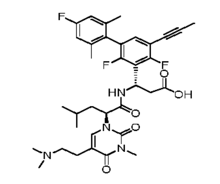 | 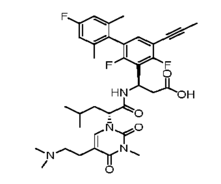 |
| 355 | 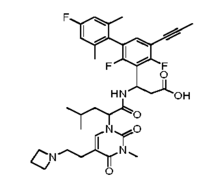 | 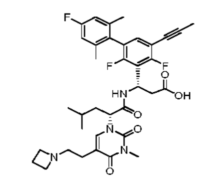 | 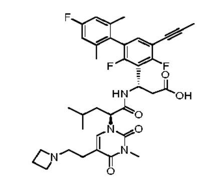 | 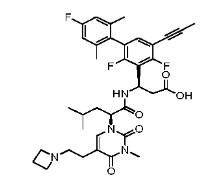 | 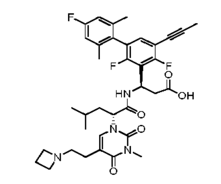 |
| 356 | 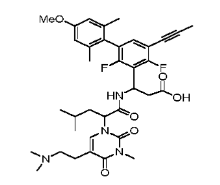 | 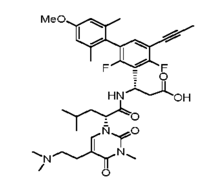 | 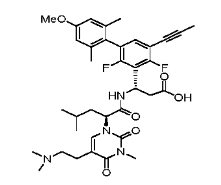 | 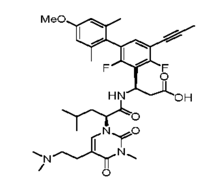 | 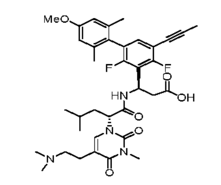 |
| 357 | 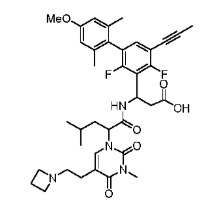 | 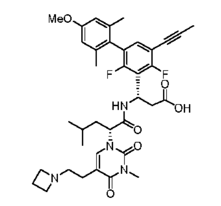 | 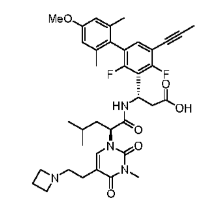 | 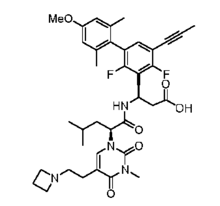 | 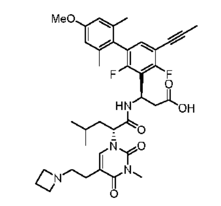 |
| 358 | 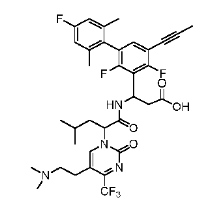 | 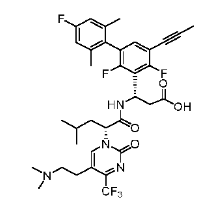 | 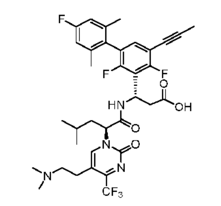 | 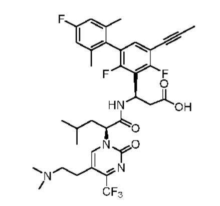 | 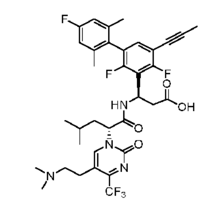 |
| 359 | 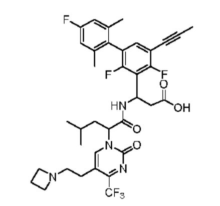 | 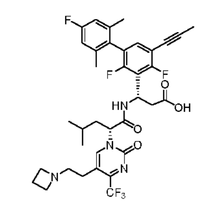 | 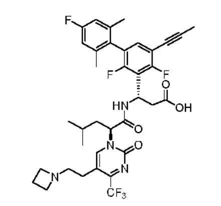 | 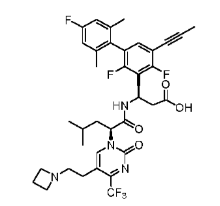 | 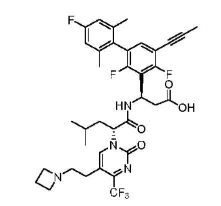 |
| 360 | 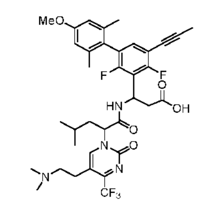 | 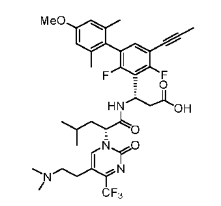 | 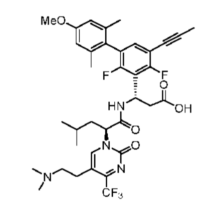 | 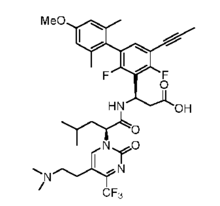 | 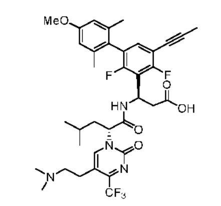 |
| 361 | 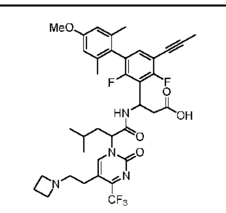 | 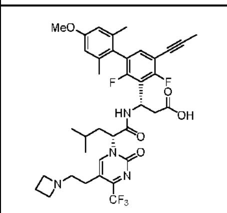 | 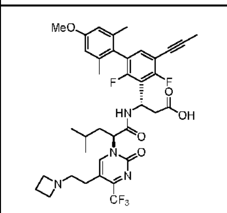 | 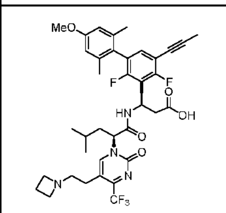 | 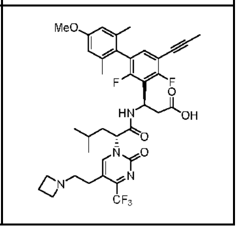 |

Table E-5

| | | | | | |
|---|---|---|---|---|---|
| 362 | 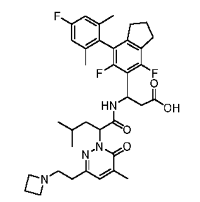 | 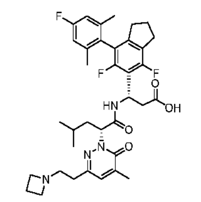 | 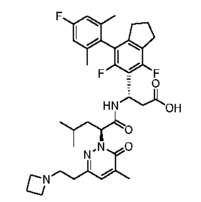 | 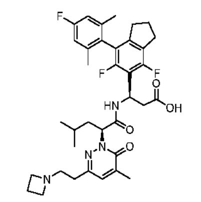 | 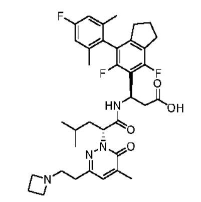 |
| 363 | 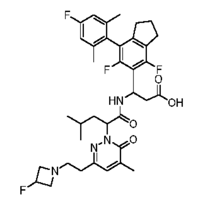 | 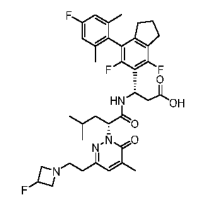 | 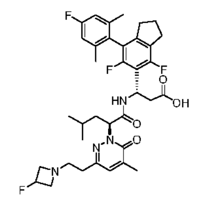 | 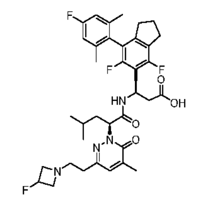 | 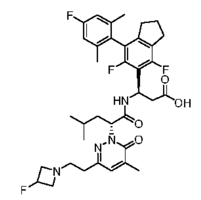 |
| 364 | 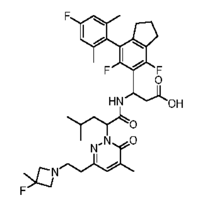 | 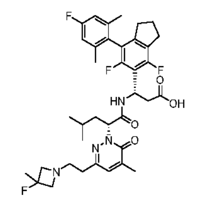 | 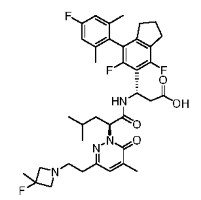 | 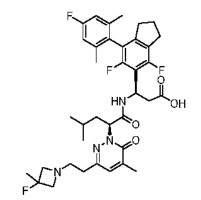 | 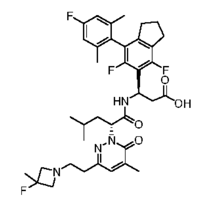 |
| 365 | 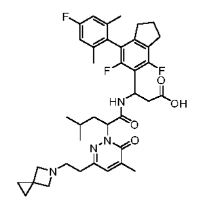 | 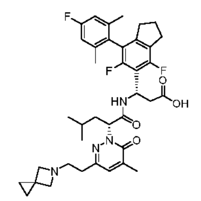 | 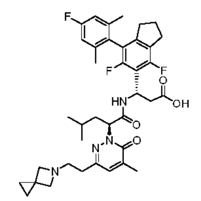 | 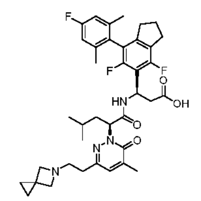 | 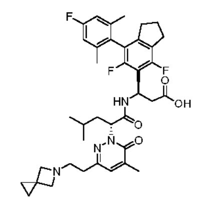 |
| 366 | 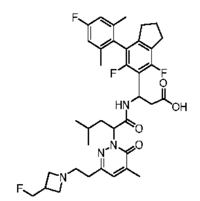 | 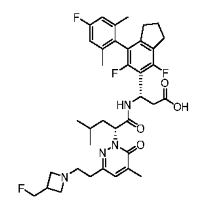 | 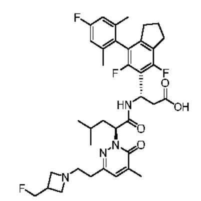 | 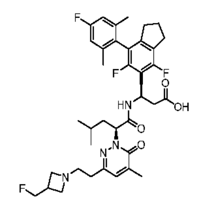 | 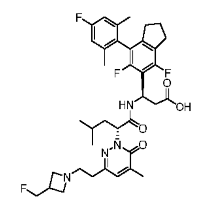 |
| 367 | 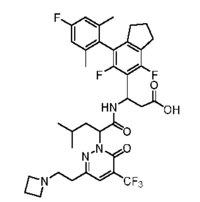 | 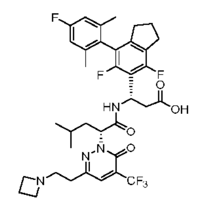 | 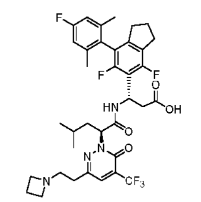 | 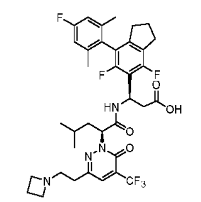 | 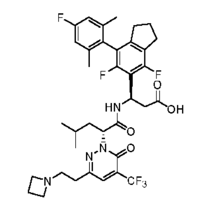 |
| 368 | 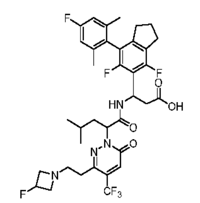 | 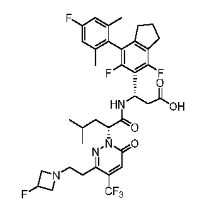 | 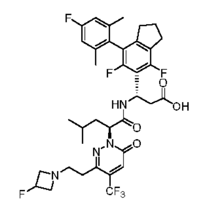 | 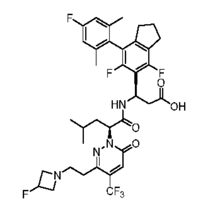 | 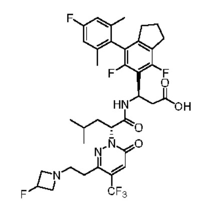 |
| 369 | 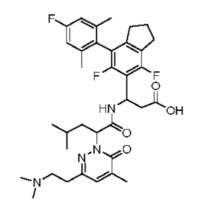 | 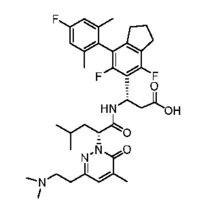 | 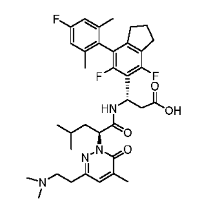 | 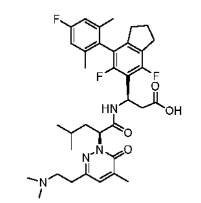 | 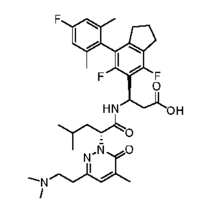 |

(continued)

| | | | | | |
|---|---|---|---|---|---|
| 370 | 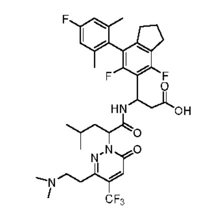 | 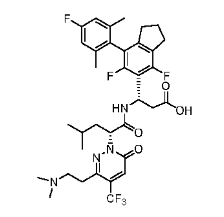 | 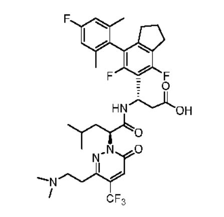 | 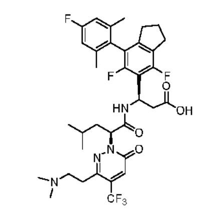 | 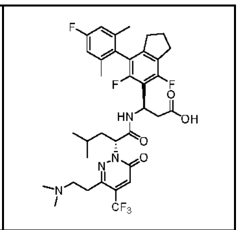 |
| 371 | 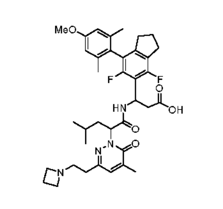 | 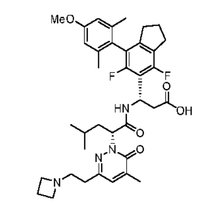 | 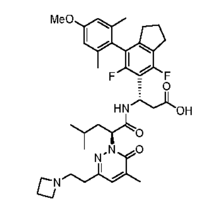 | 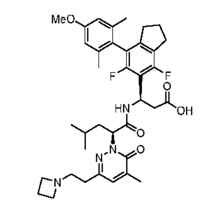 | 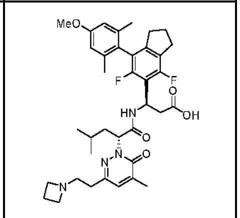 |
| 372 | 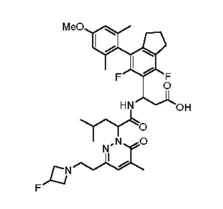 | 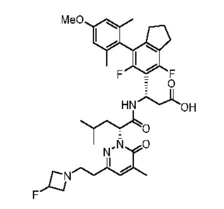 | 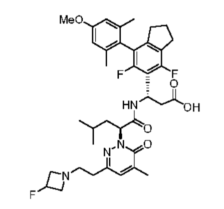 | 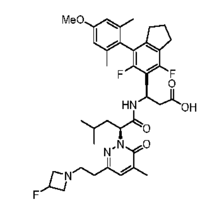 | 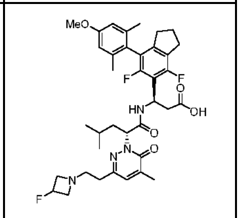 |
| 373 | 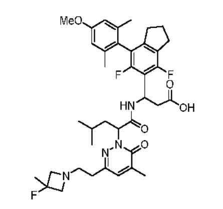 | 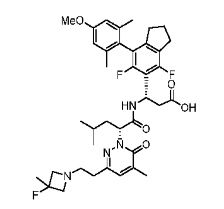 | 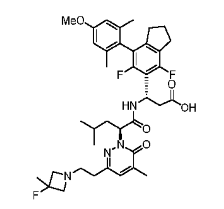 | 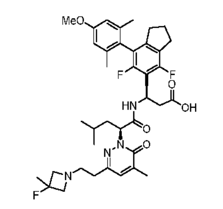 | 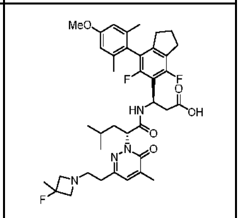 |
| 374 | 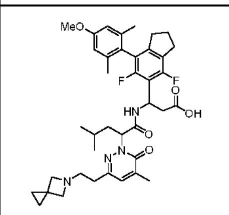 | 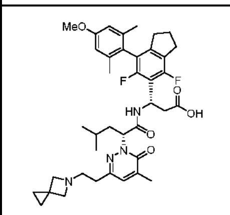 | 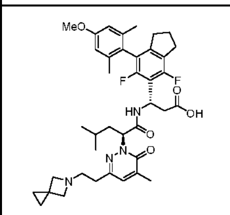 | 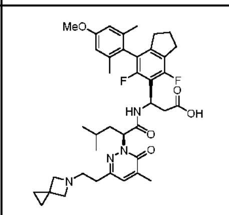 | 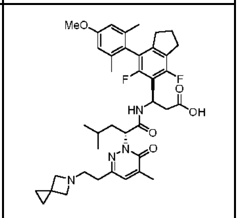 |
| 375 | 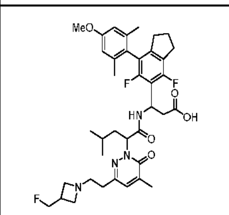 | 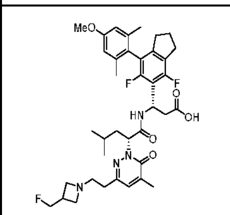 | 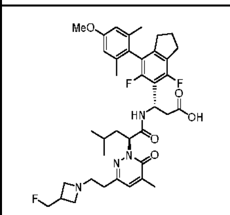 | 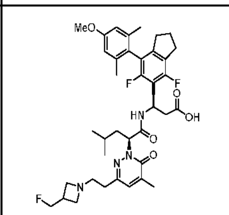 | 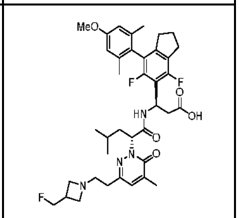 |
| 376 | 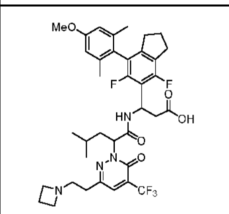 | 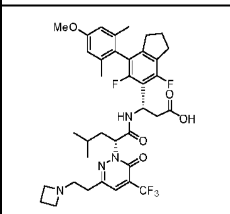 | 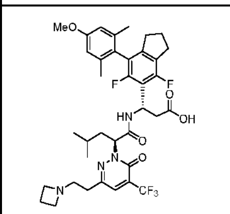 | 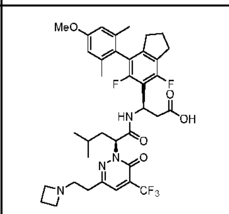 | 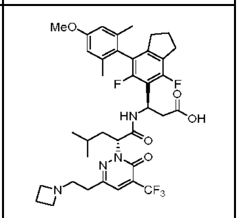 |
| 377 | 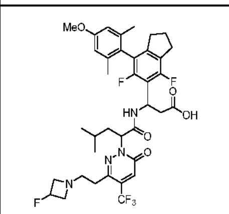 | 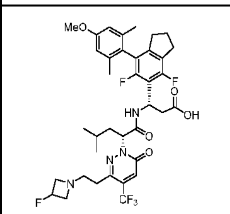 | 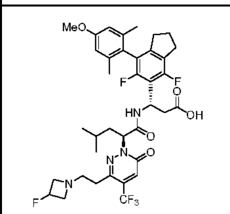 | 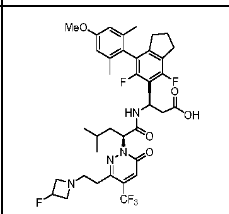 | 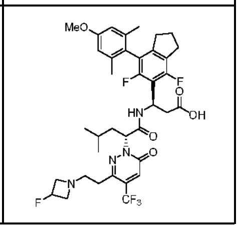 |

(continued)

| | | | | | |
|---|---|---|---|---|---|
| 378 | 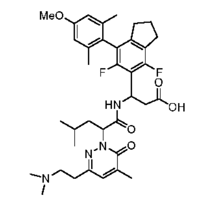 | 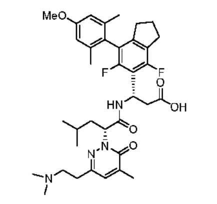 | 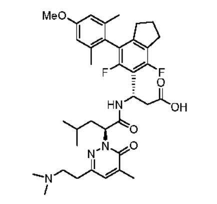 | 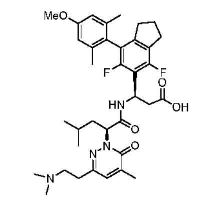 | 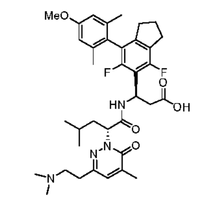 |
| 379 | 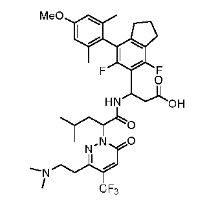 | 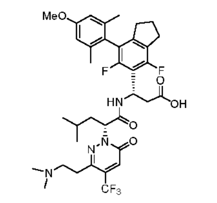 | 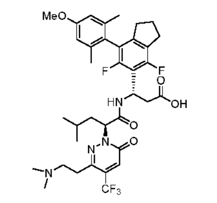 | 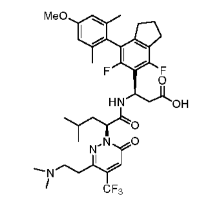 | 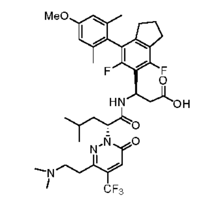 |
| 380 | 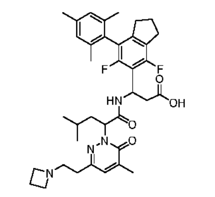 | 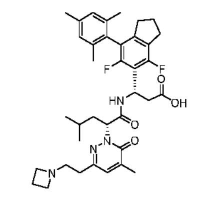 | 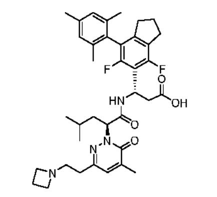 | 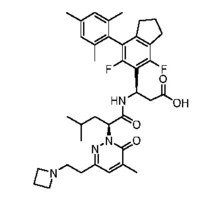 | 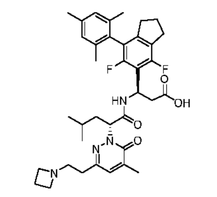 |
| 381 | 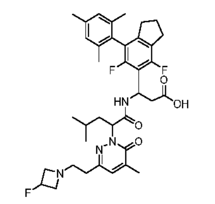 | 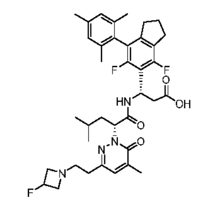 | 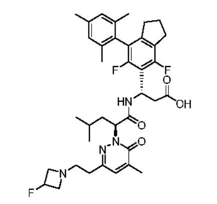 | 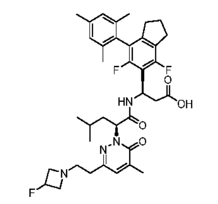 | 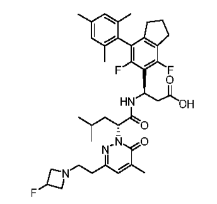 |
| 382 | 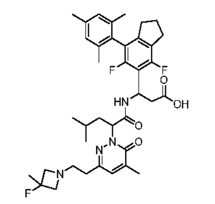 | 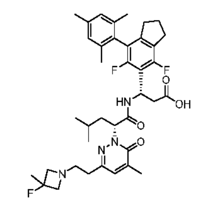 | 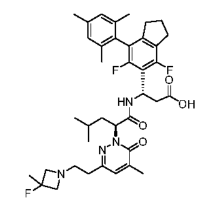 | 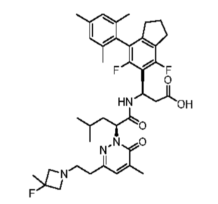 | 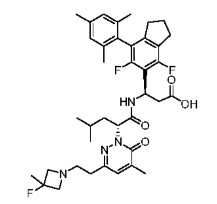 |
| 383 | 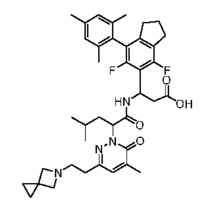 | 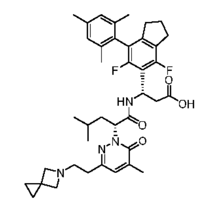 | 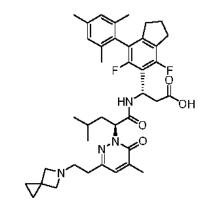 | 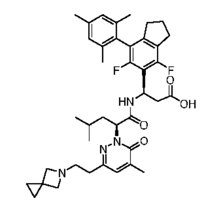 | 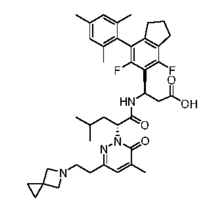 |
| 384 | 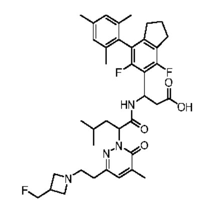 | 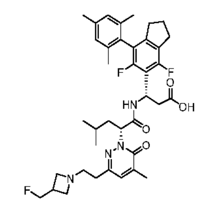 | 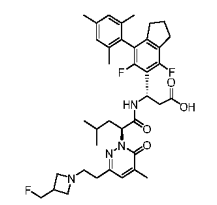 | 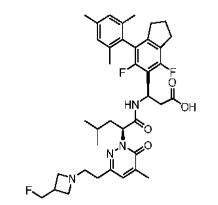 | 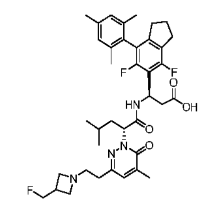 |
| 385 | 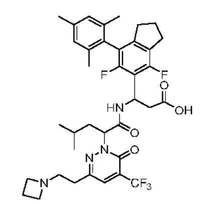 | 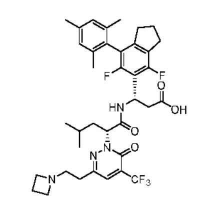 | 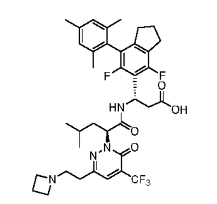 | 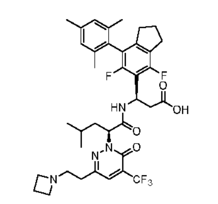 | 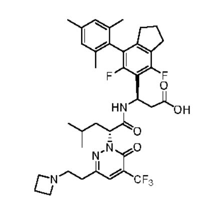 |

(continued)

| | | | | | |
|---|---|---|---|---|---|
| 386 | 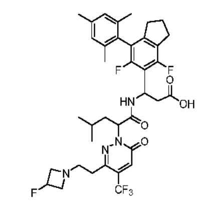 | 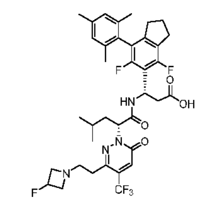 | 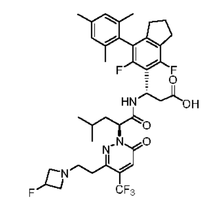 | 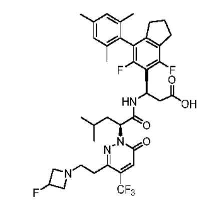 | 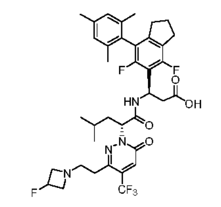 |
| 387 | 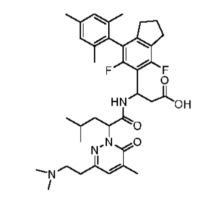 | 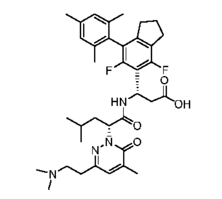 | 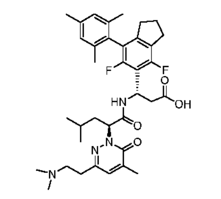 | 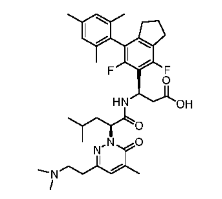 | 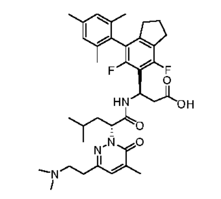 |
| 388 | 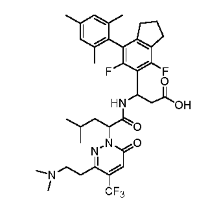 | 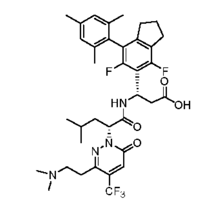 | 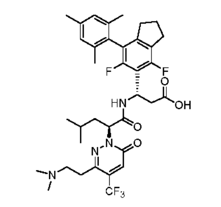 | 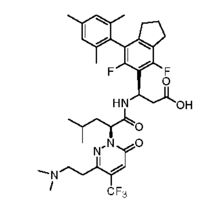 | 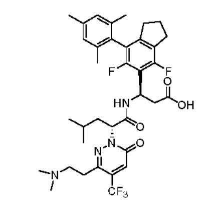 |
| 389 | 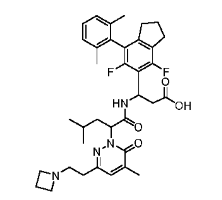 | 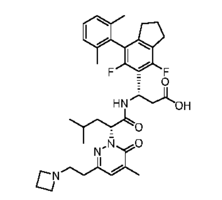 | 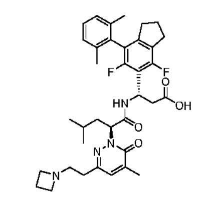 | 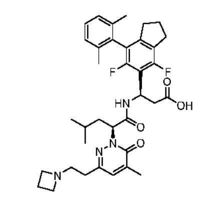 | 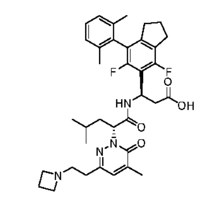 |
| 390 | 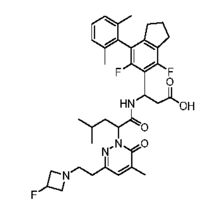 | 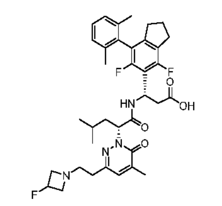 | 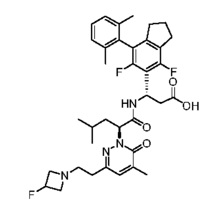 | 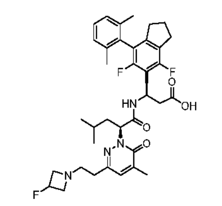 | 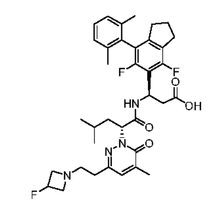 |
| 391 | 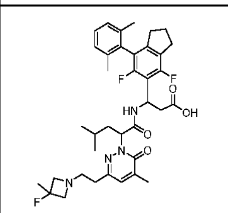 | 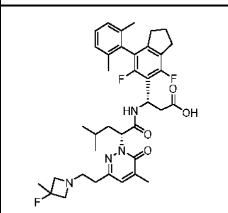 | 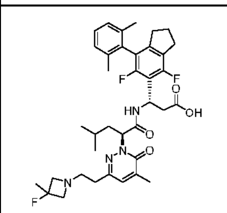 | 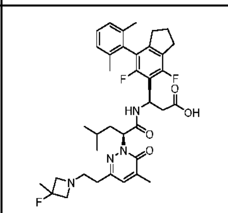 | 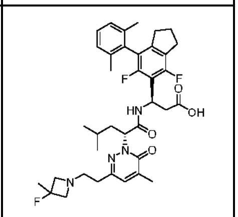 |
| 392 | 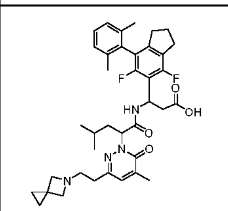 | 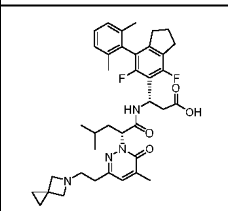 | 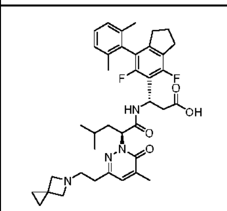 | 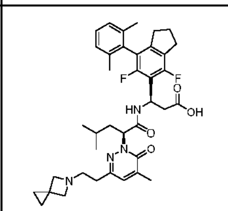 | 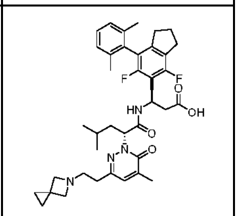 |
| 393 | 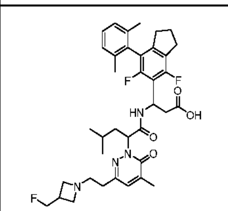 | 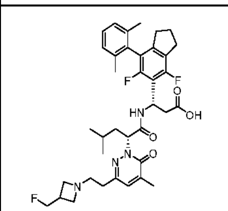 | 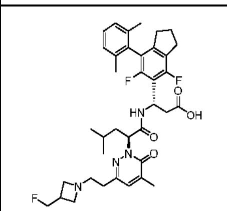 | 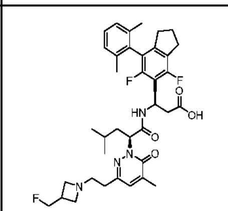 | 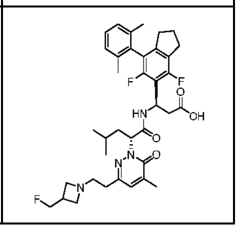 |

(continued)

| | | | | | |
|---|---|---|---|---|---|
| 394 | 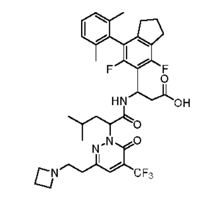 | 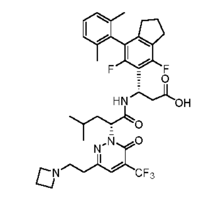 | 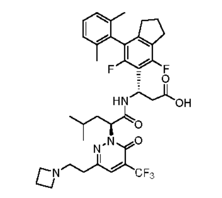 | 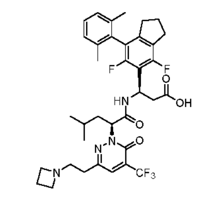 | 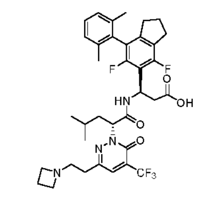 |
| 395 | 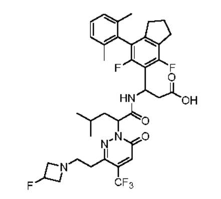 | 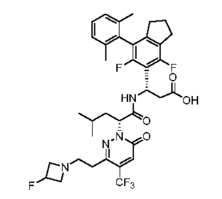 | 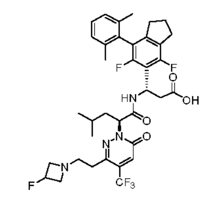 | 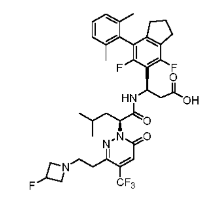 | 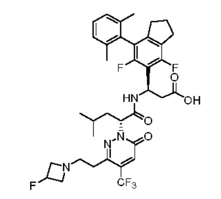 |
| 396 | 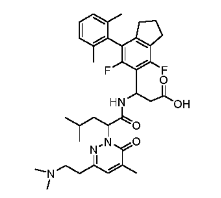 | 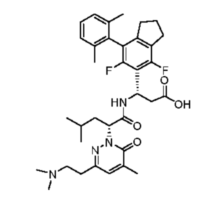 | 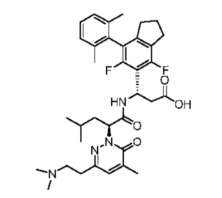 | 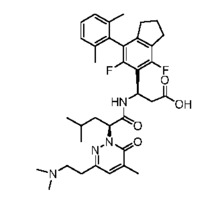 | 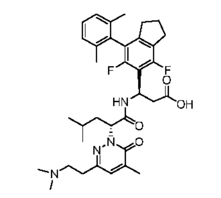 |
| 397 | 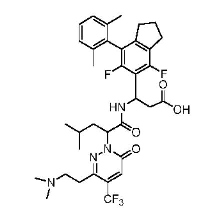 | 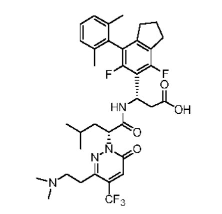 | 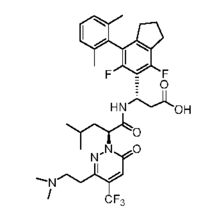 | 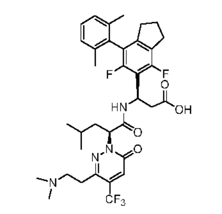 | 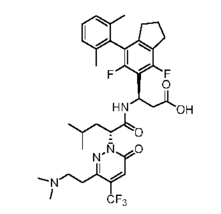 |
| 398 | 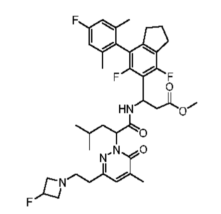 | 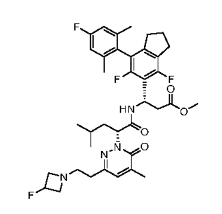 | 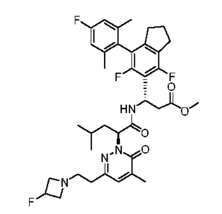 | 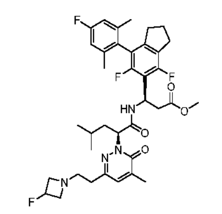 | 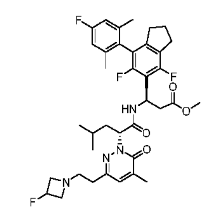 |
| 399 | 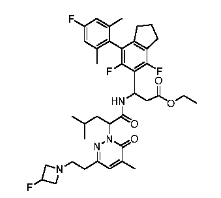 | 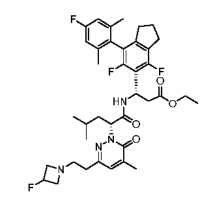 | 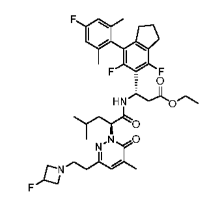 | 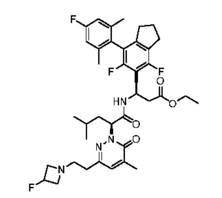 | 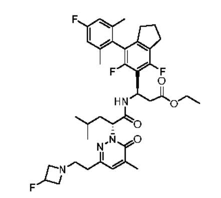 |
| 400 | 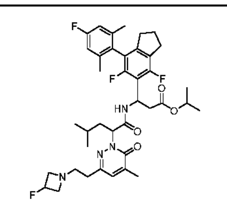 | 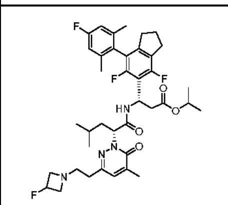 | 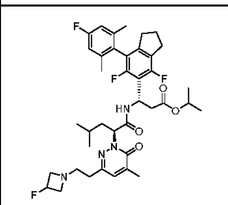 | 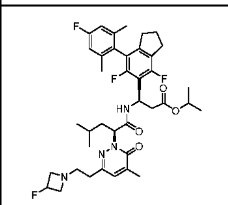 | 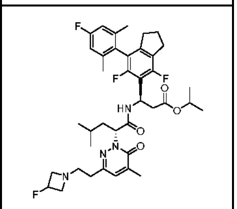 |
| 401 | 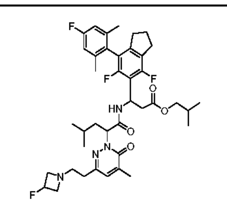 | 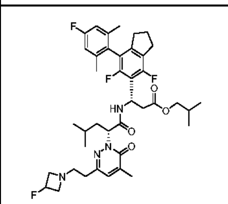 | 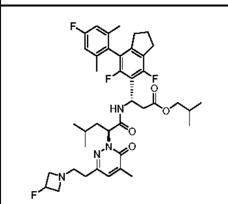 | 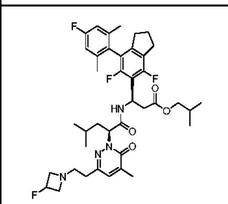 | 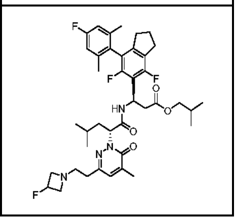 |

(continued)

| | | | | | |
|---|---|---|---|---|---|
| 402 | 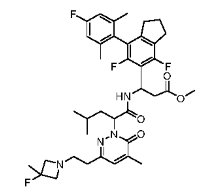 | 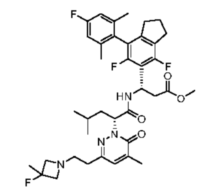 | 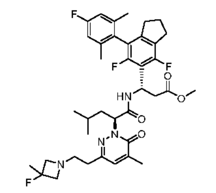 | 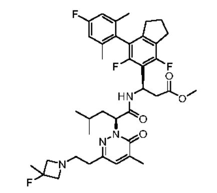 | 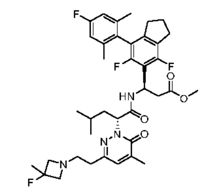 |
| 403 | 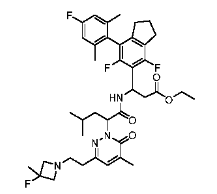 | 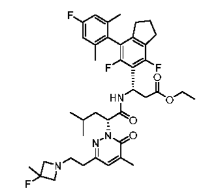 | 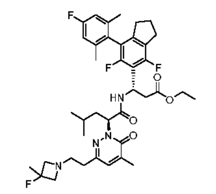 | 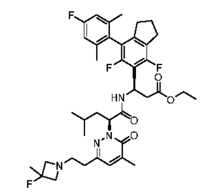 | 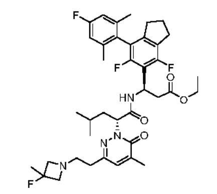 |
| 404 | 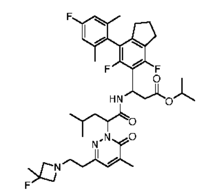 | 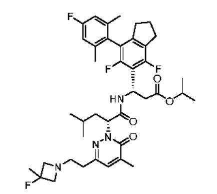 | 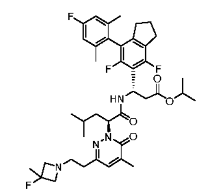 | 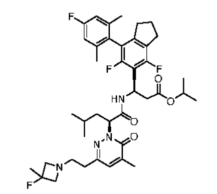 | 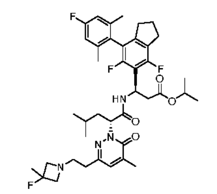 |
| 405 | 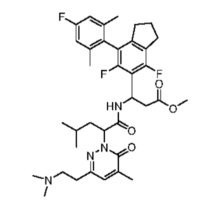 | 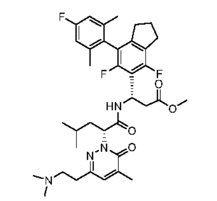 | 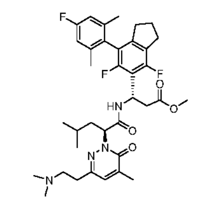 | 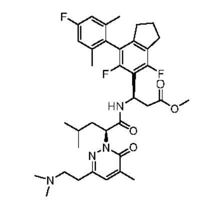 | 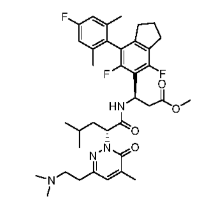 |
| 406 | 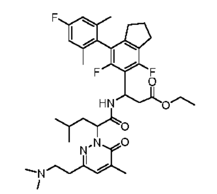 | 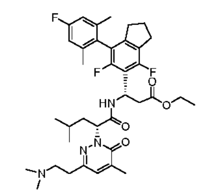 | 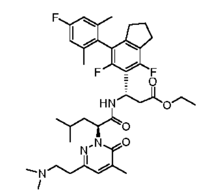 | 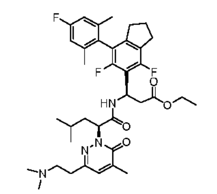 | 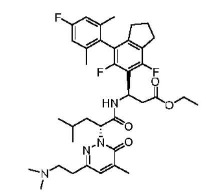 |
| 407 | 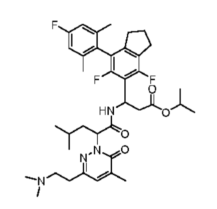 | 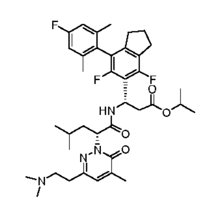 | 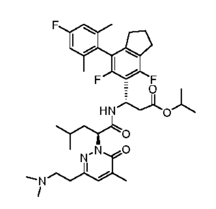 | 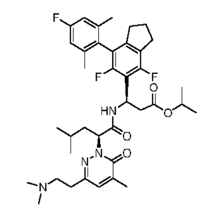 | 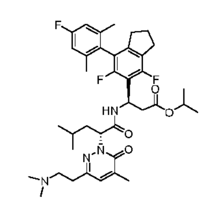 |
| 408 | 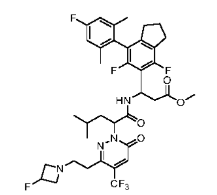 | 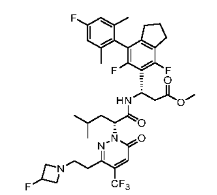 | 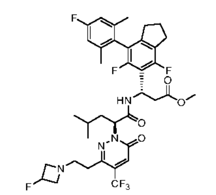 | 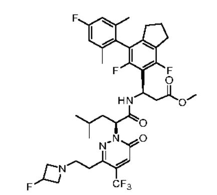 | 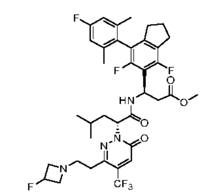 |
| 409 | 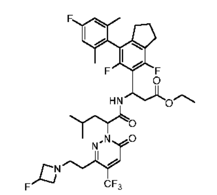 | 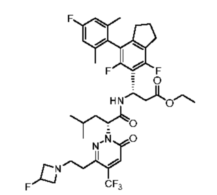 | 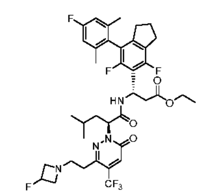 | 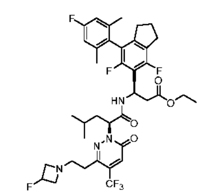 | 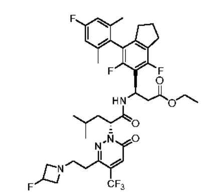 |

(continued)

| | | | | | |
|---|---|---|---|---|---|
| 410 | 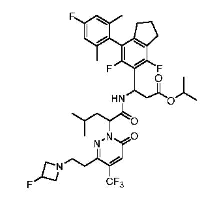 | 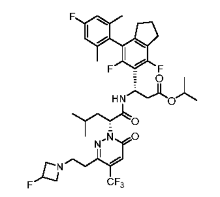 | 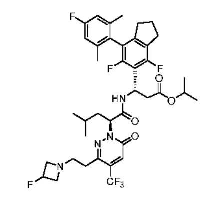 | 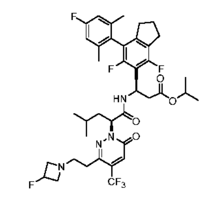 | 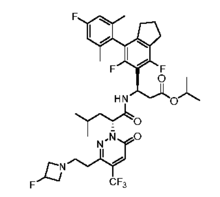 |
| 411 | 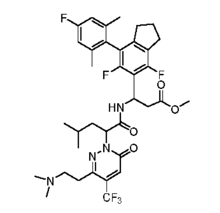 | 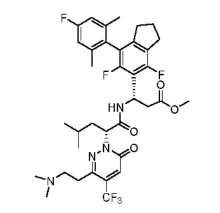 | 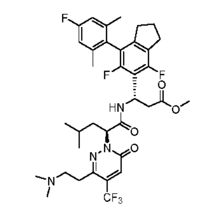 | 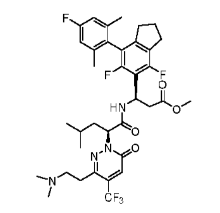 | 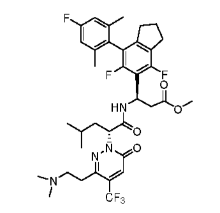 |
| 412 | 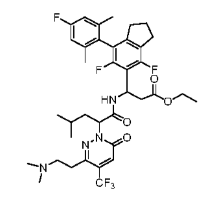 | 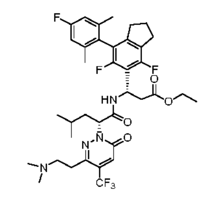 | 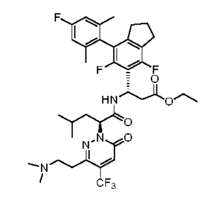 | 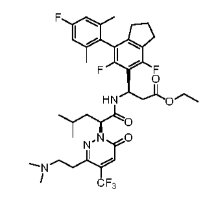 | 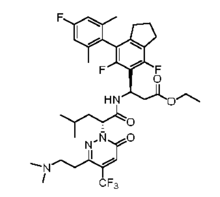 |
| 413 | 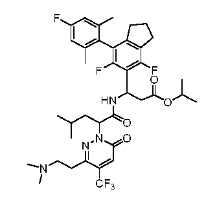 | 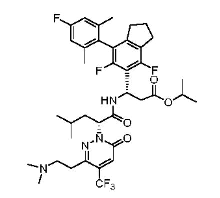 | 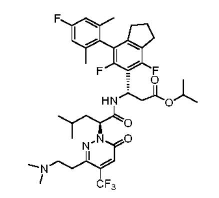 | 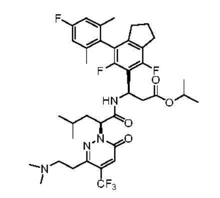 | 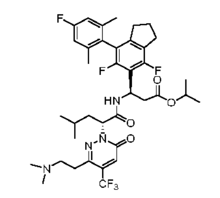 |
| 414 | 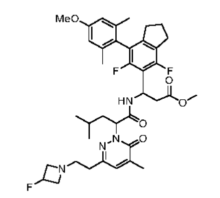 | 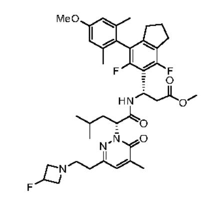 | 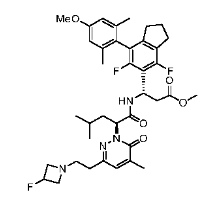 | 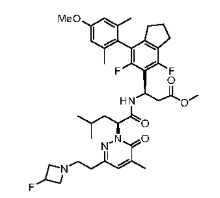 | 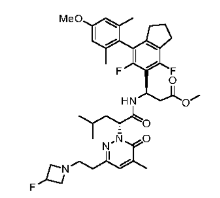 |
| 415 | 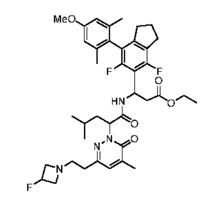 | 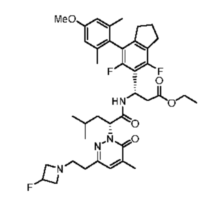 | 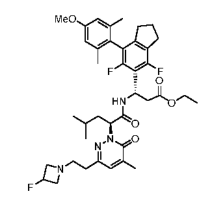 | 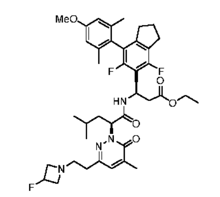 | 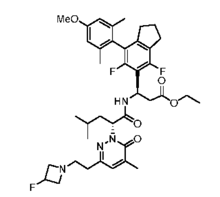 |
| 416 | 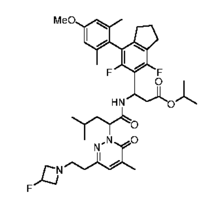 | 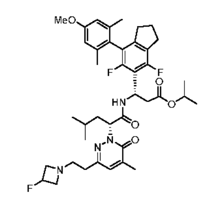 | 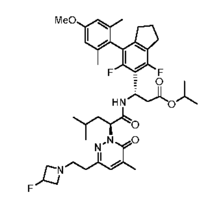 | 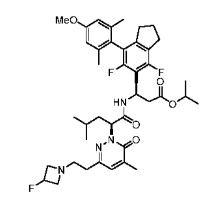 | 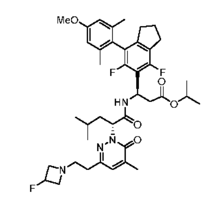 |
| 417 | 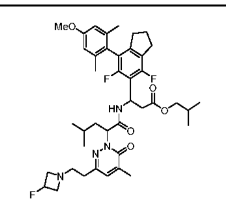 | 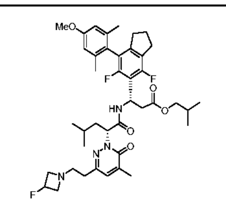 | 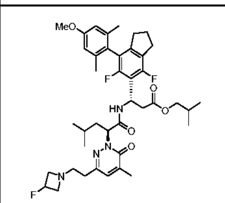 | 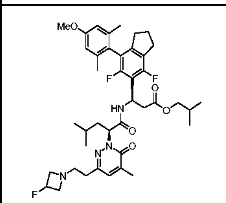 | 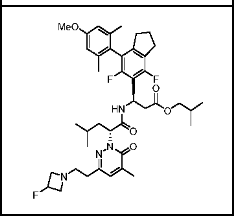 |

(continued)

| | | | | | |
|---|---|---|---|---|---|
| 418 | 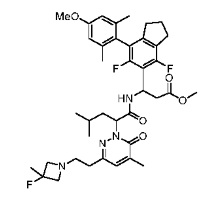 | 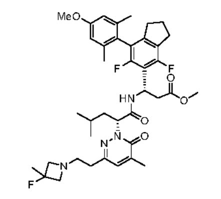 | 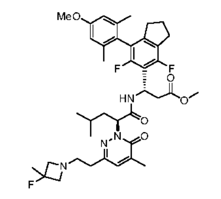 | 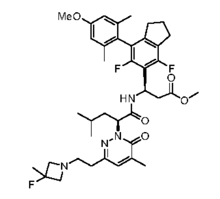 | 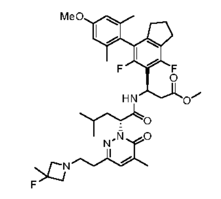 |
| 419 | 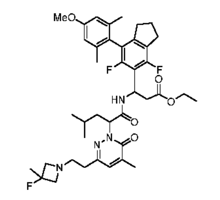 | 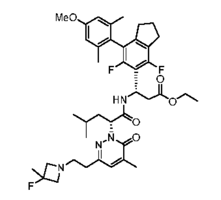 | 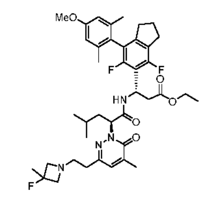 | 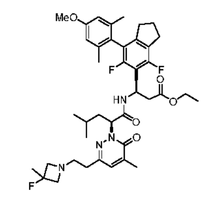 | 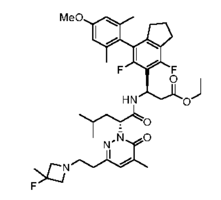 |
| 420 | 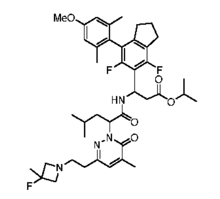 | 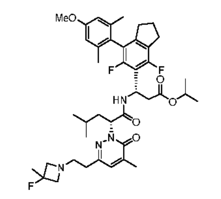 | 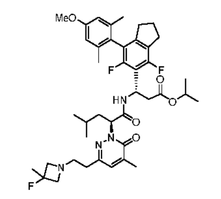 | 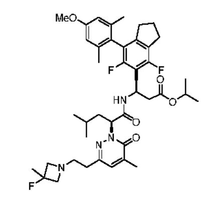 | 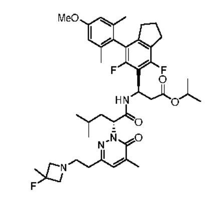 |
| 421 | 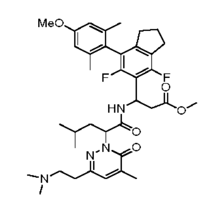 | 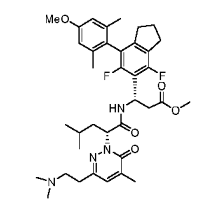 | 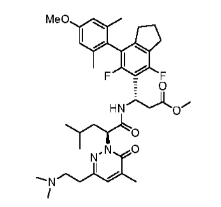 | 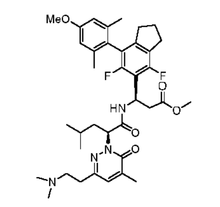 | 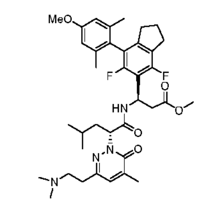 |
| 422 | 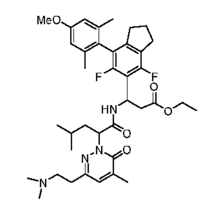 | 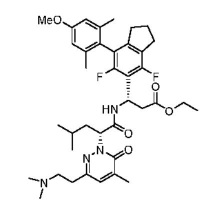 | 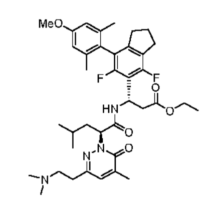 | 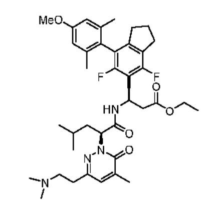 | 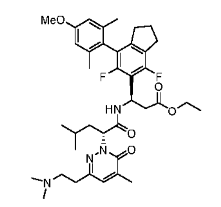 |
| 423 | 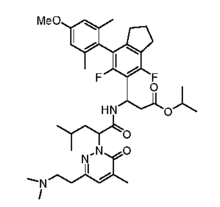 | 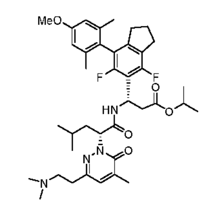 | 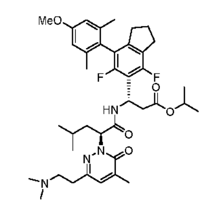 | 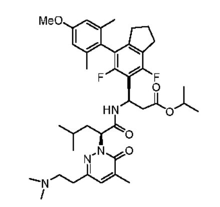 | 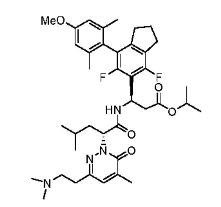 |
| 424 | 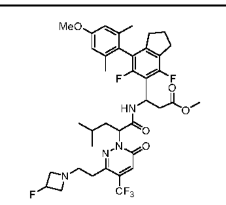 | 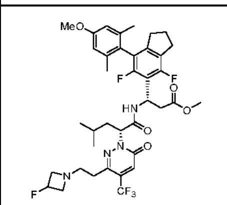 | 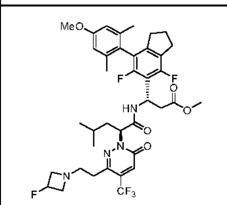 | 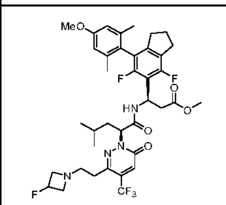 | 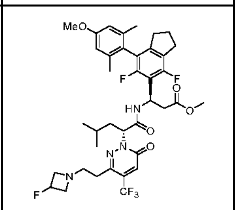 |
| 425 | 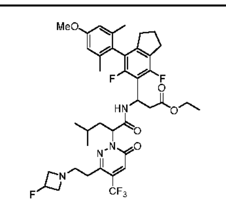 | 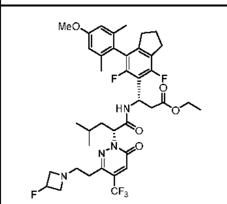 | 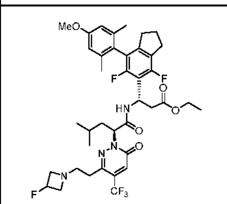 | 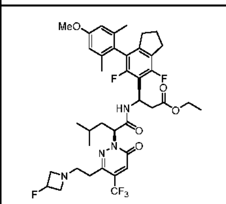 | 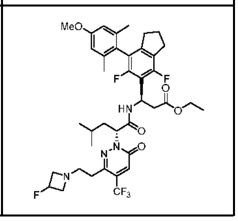 |

(continued)

| | | | | | |
|---|---|---|---|---|---|
| 426 | 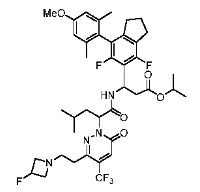 | 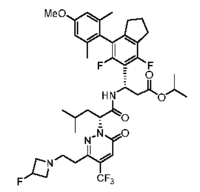 | 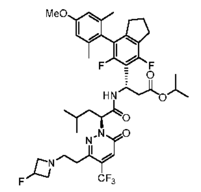 | 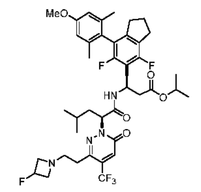 | 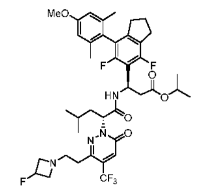 |
| 427 | 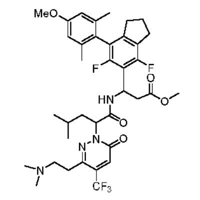 | 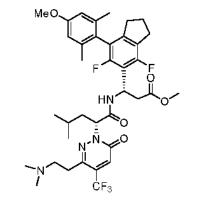 | 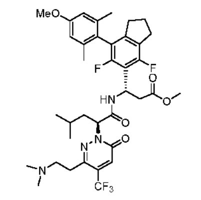 | 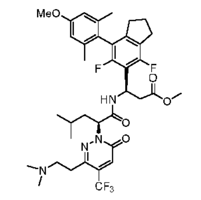 | 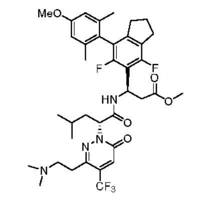 |
| 428 | 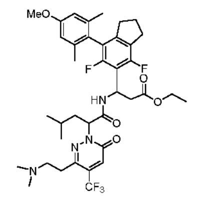 | 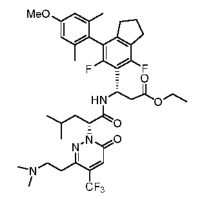 | 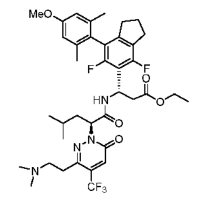 | 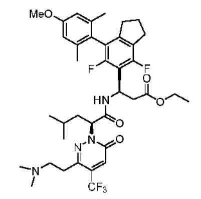 | 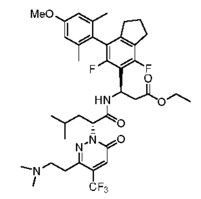 |
| 429 | 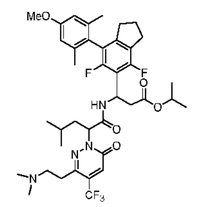 | 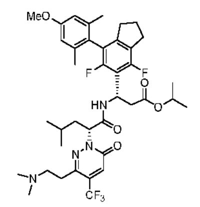 | 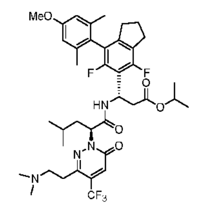 | 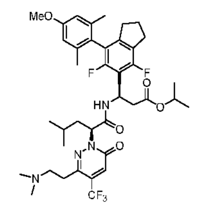 | 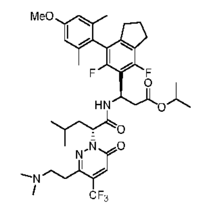 |
| 430 | 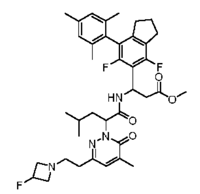 | 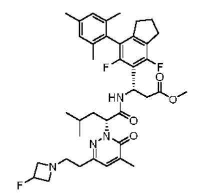 | 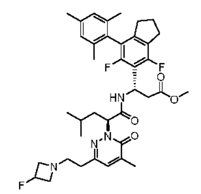 | 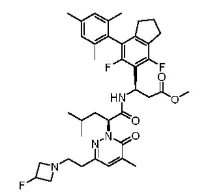 | 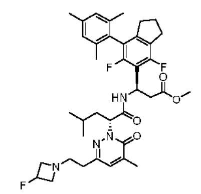 |
| 431 | 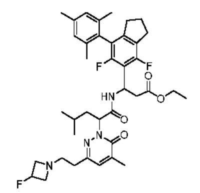 | 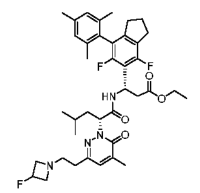 | 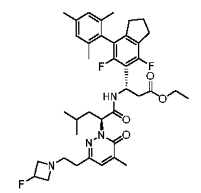 | 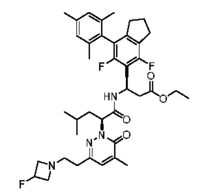 | 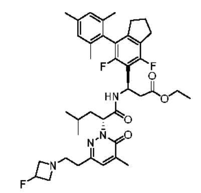 |
| 432 | 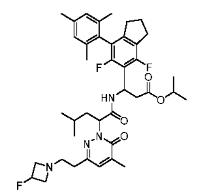 | 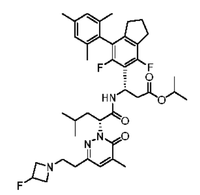 | 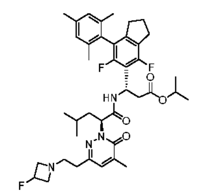 | 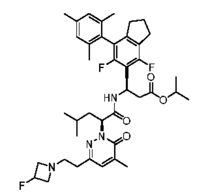 | 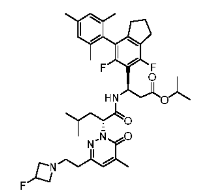 |
| 433 | 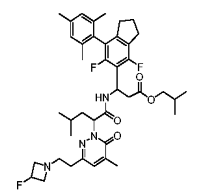 | 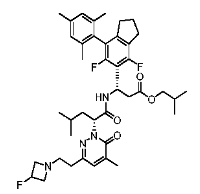 | 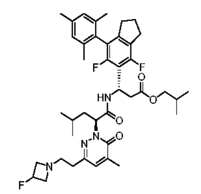 | 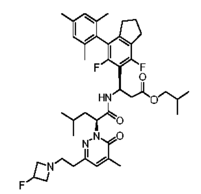 | 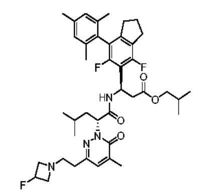 |

(continued)

| | | | | | |
|---|---|---|---|---|---|
| 434 | 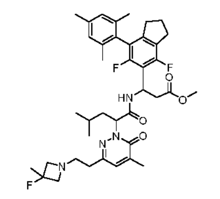 | 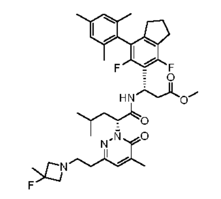 | 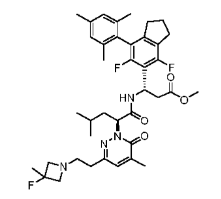 | 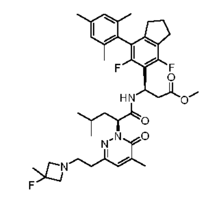 | 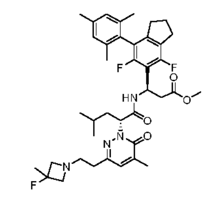 |
| 435 | 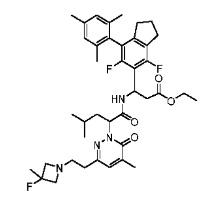 | 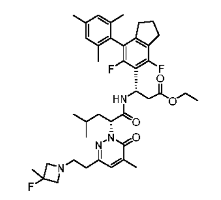 | 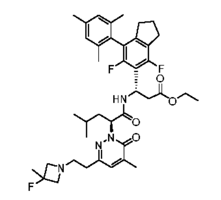 | 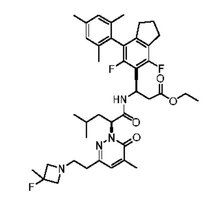 | 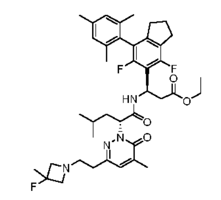 |
| 436 | 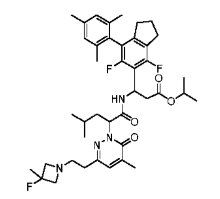 | 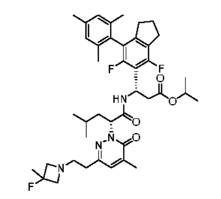 | 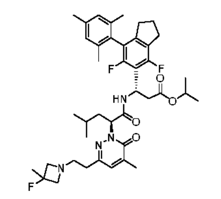 | 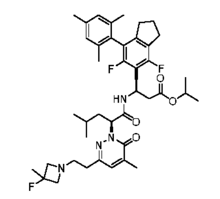 | 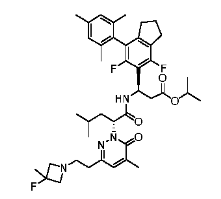 |
| 437 | 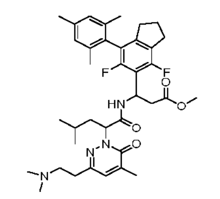 | 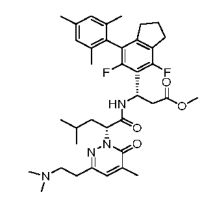 | 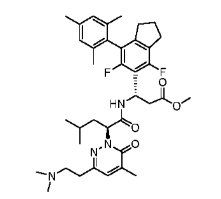 | 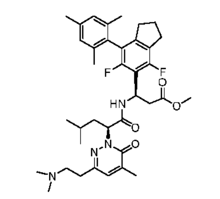 | 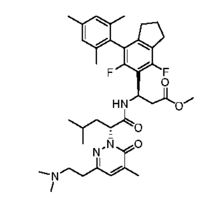 |
| 438 | 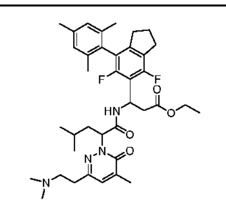 | 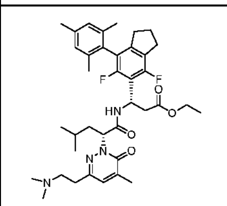 | 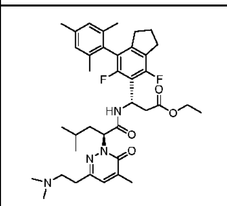 | 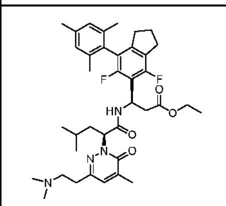 | 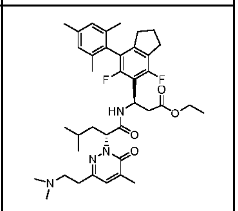 |
| 439 | 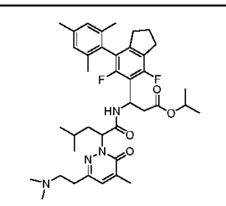 | 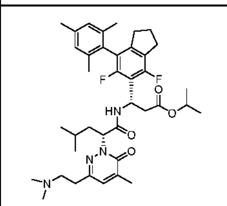 | 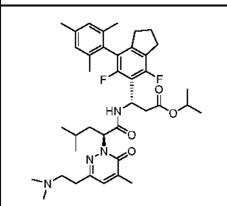 | 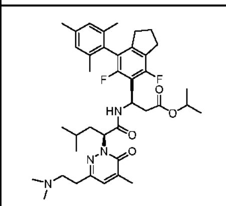 | 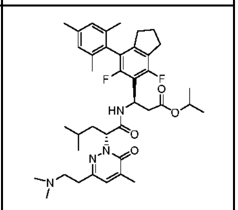 |
| 440 | 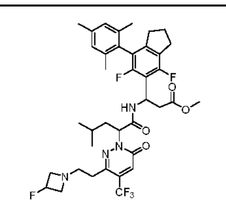 | 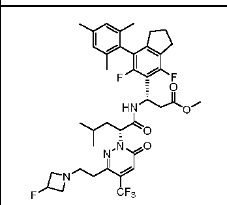 | 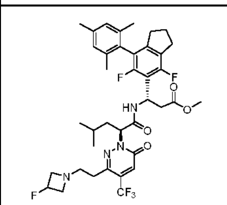 | 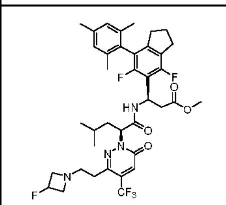 | 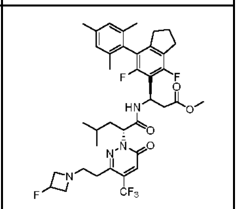 |
| 441 | 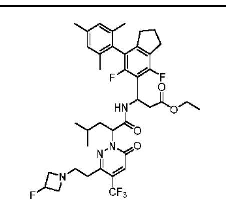 | 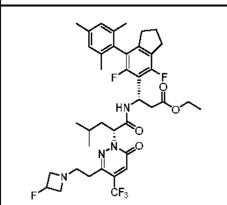 | 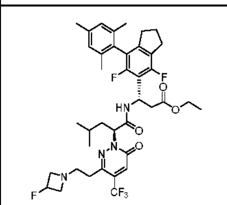 | 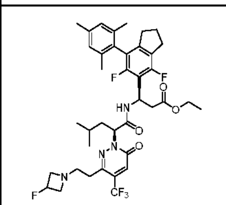 | 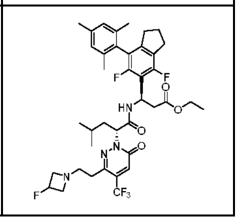 |

(continued)

| | | | | | |
|---|---|---|---|---|---|
| 442 | 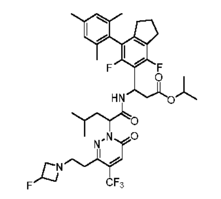 | 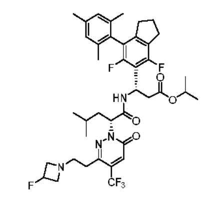 | 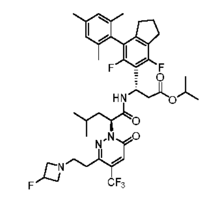 | 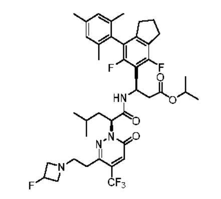 | 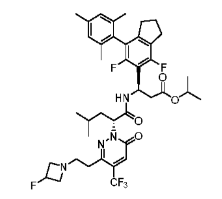 |
| 443 | 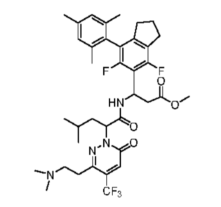 | 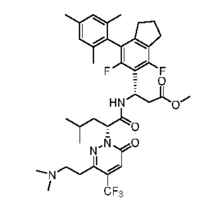 | 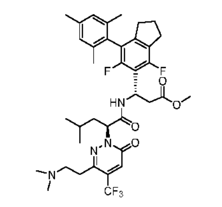 | 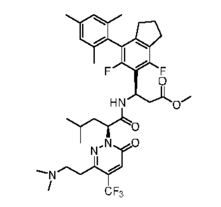 | 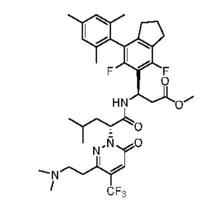 |
| 444 | 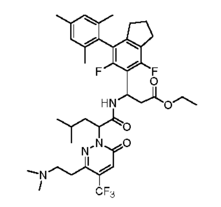 | 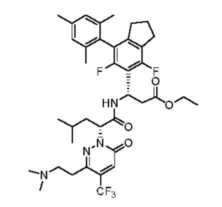 | 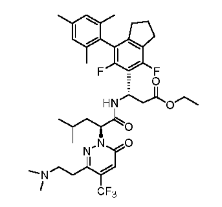 | 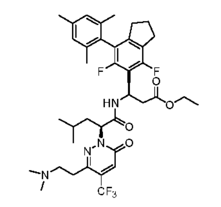 | 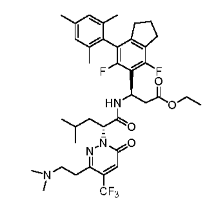 |
| 445 | 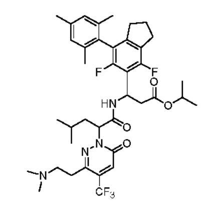 | 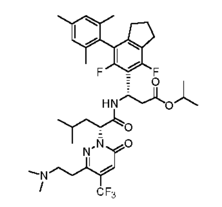 | 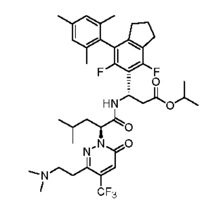 | 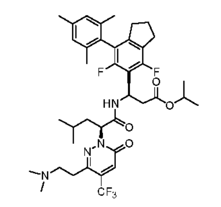 | 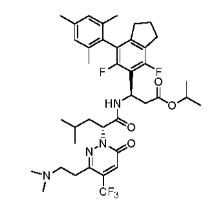 |
| 446 | 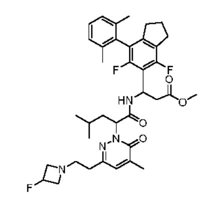 | 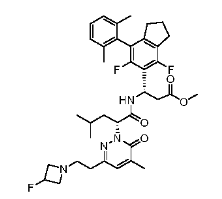 | 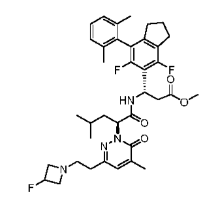 | 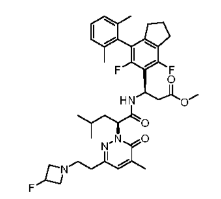 | 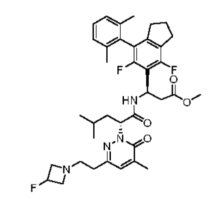 |
| 447 | 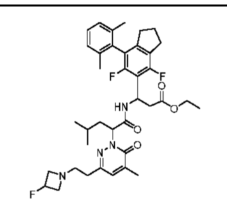 | 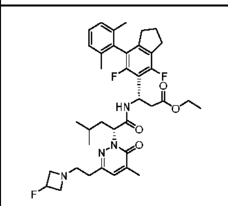 | 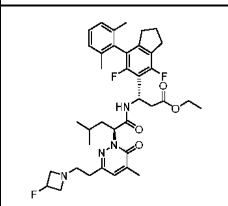 | 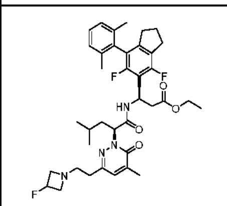 | 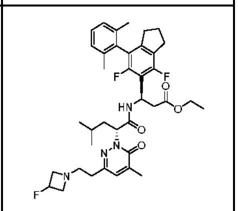 |
| 448 | 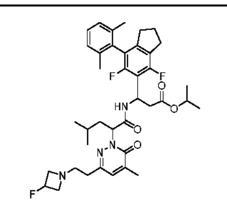 | 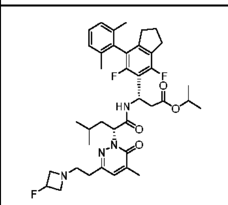 | 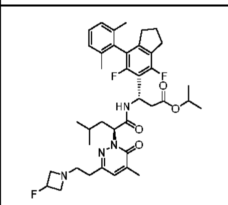 | 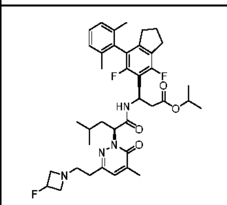 | 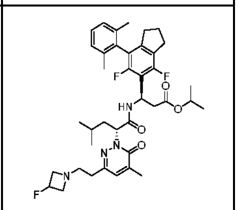 |
| 449 | 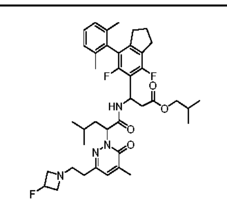 | 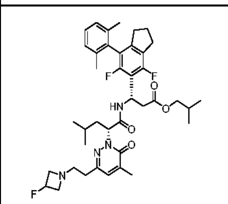 | 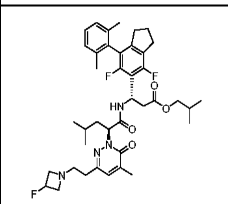 | 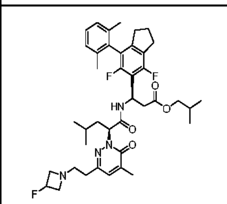 | 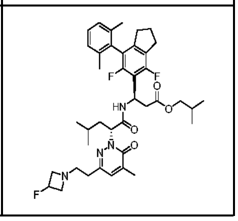 |

(continued)

| | | | | | |
|---|---|---|---|---|---|
| 450 | 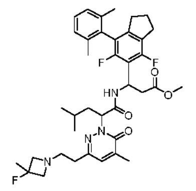 | 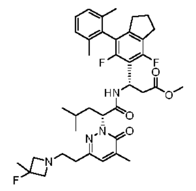 | 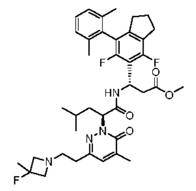 | 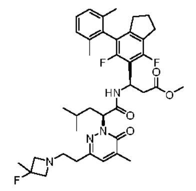 | 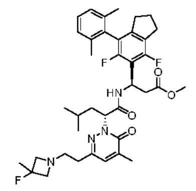 |
| 451 | 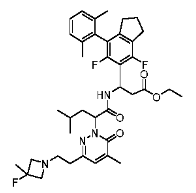 | 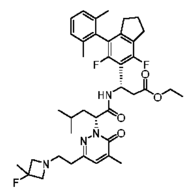 | 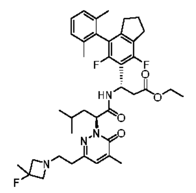 | 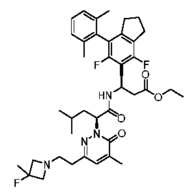 | 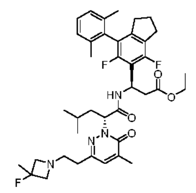 |
| 452 | 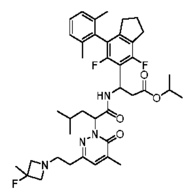 | 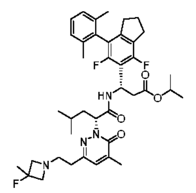 | 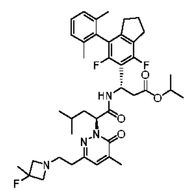 | 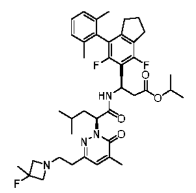 | 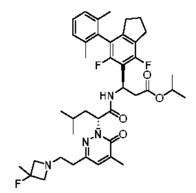 |
| 453 | 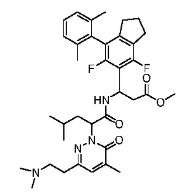 | 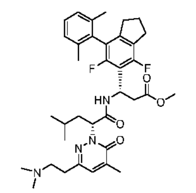 | 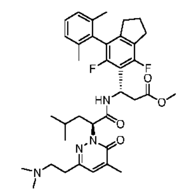 | 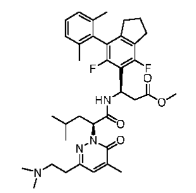 | 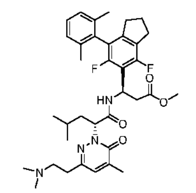 |
| 454 | 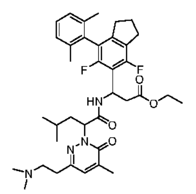 | 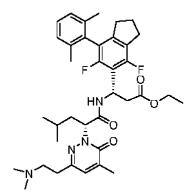 | 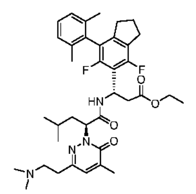 | 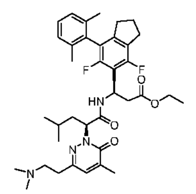 | 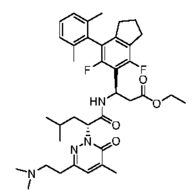 |
| 455 | 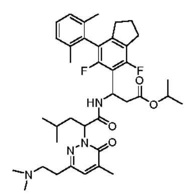 | 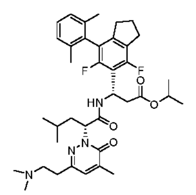 | 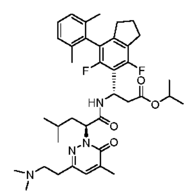 | 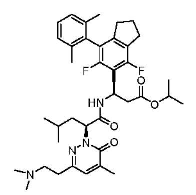 | 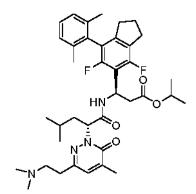 |
| 456 | 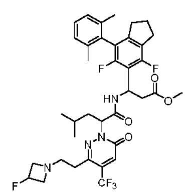 | 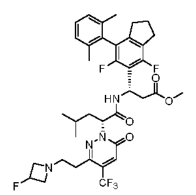 | 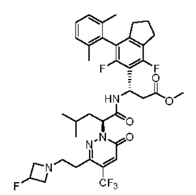 | 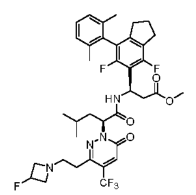 | 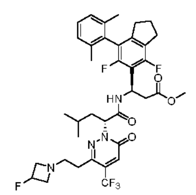 |
| 457 | 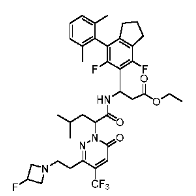 | 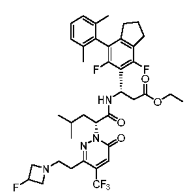 | 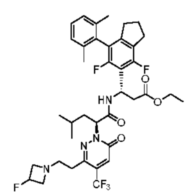 | 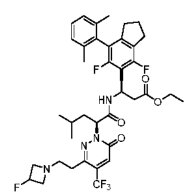 | 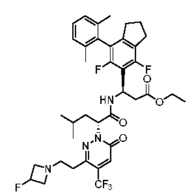 |

(continued)

| | | | | | |
|---|---|---|---|---|---|
| 458 | 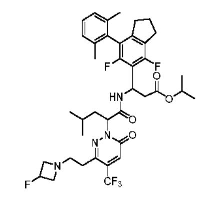 | 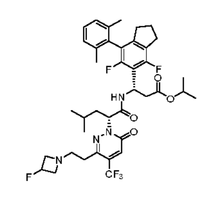 | 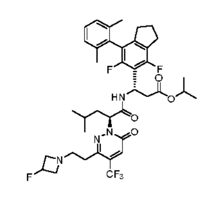 | 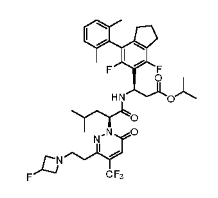 | 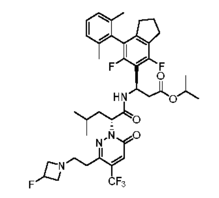 |
| 459 | 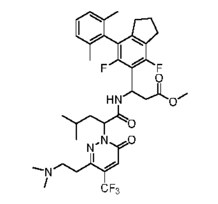 | 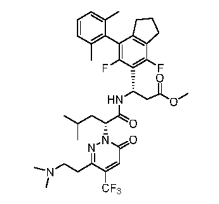 | 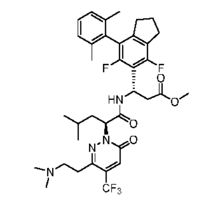 | 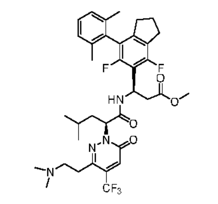 | 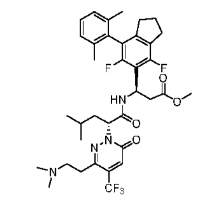 |
| 460 | 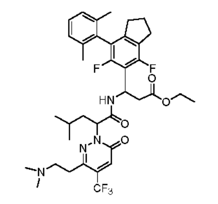 | 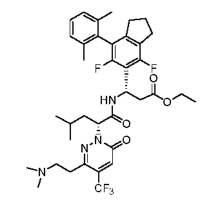 | 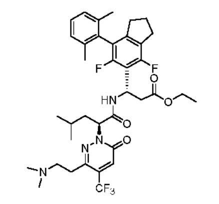 | 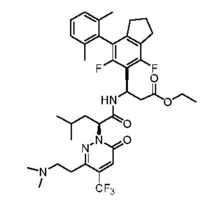 | 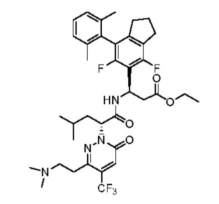 |
| 461 | 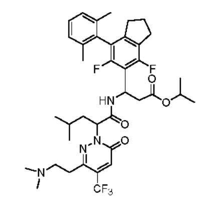 | 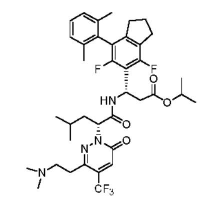 | 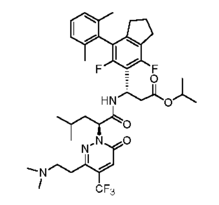 | 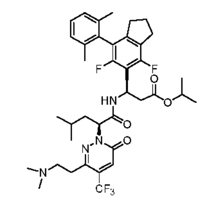 | 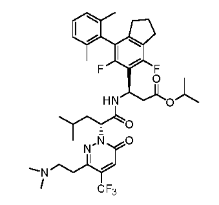 |

The present invention relates to a pharmaceutical composition, comprising the compound or the stereoisomer, mesomer/racemate, deuterated product, solvate, prodrug, metabolite, pharmaceutically acceptable salt, or co-crystal thereof according to the present invention, and a pharmaceutically acceptable carrier.

The present invention relates to the use of the compound or the stereoisomer, mesomer/racemate, deuterated product, solvate, prodrug, metabolite, pharmaceutically acceptable salt, or co-crystal thereof according to the present invention in the preparation of a drug for the treatment of diseases related to $\alpha 4\beta 7$ activity or expression, preferably the use in the preparation of a drug for inflammatory bowel diseases.

The present invention relates to a pharmaceutical composition or pharmaceutical preparation comprising a therapeutically effective amount of the compound or the stereoisomer, mesomer/racemate, deuterated product, solvate, prodrug, metabolite, pharmaceutically acceptable salt, or co-crystal thereof according to the present invention, and a pharmaceutical excipient. The pharmaceutical composition can be in a unit preparation form (the amount of the active drug in the unit preparation is also referred to as "preparation specification").

The present invention further provides a method for treating a disease in a mammal, comprising administering to the mammal a therapeutically effective amount of the compound or the stereoisomer, mesomer/racemate, deuterated product, solvate, prodrug, metabolite, pharmaceutically acceptable salt, or co-crystal thereof or the pharmaceutical composition according to the present invention. In some embodiments, the mammal mentioned in the present invention includes human.

The term "effective amount" or "therapeutically effective amount" in the present application refers to a sufficient amount of the compound disclosed in the present application being administered which will relieve to some extent one or more of the symptoms of the disease or condition being treated, e.g., inflammatory bowel diseases. In some embodiments, the outcome is the reduction and/or remission of signs, symptoms, or causes of the disease, or any other desired change in the biological system. For example, an "effective amount" in terms of the therapeutic use is an amount comprising the compound disclosed in the present application that is required to provide clinically significant reduction of the symptoms of the disease. Examples of the therapeutically effective amount includes, but are not limited to 1-1500 mg, 1-1200 mg, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500

mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1000 mg, or 80-800 mg.

In some embodiments, the pharmaceutical composition comprises the compound or the stereoisomer, mesomer/racemate, deuterated product, solvate, prodrug, metabolite, pharmaceutically acceptable salt, or co-crystal thereof according to the present invention in an amount including but not limited to 1-1000mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg and 840 mg.

The present invention relates to a method for treating a disease in a mammal, the method comprising administering to a subject a therapeutically effective amount of the compound or the stereoisomer, mesomer/racemate, deuterated product, solvate, prodrug, metabolite, pharmaceutically acceptable salt, or co-crystal thereof according to the present invention, wherein the therapeutically effective amount is preferably 1-1500 mg, and the disease is preferably inflammatory bowel disease.

The present invention relates to a method for treating a disease in a mammal, the method comprising administering to a subject a drug, i.e., the compound or the stereoisomer, mesomer/racemate, deuterated product, solvate, prodrug, metabolite, pharmaceutically acceptable salt, or co-crystal thereof according to the present invention in a daily dose of 1-1000 mg/day, wherein the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, or 1000 mg/day.

The present invention relates to a kit, wherein the kit may comprise a composition in the form of a single dose or multiple doses and comprises the compound or the stereoisomer, mesomer/racemate, deuterated product, solvate, prodrug, metabolite, pharmaceutically acceptable salt, or co-crystal thereof according to the present invention, and the amount of the compound or the stereoisomer, mesomer/racemate, deuterated product, solvate, prodrug, metabolite, pharmaceutically acceptable salt, or co-crystal thereof according to the present invention is identical to the amount of same in the above-mentioned pharmaceutical composition.

In the present invention, the amount of the compound or the stereoisomer, mesomer/racemate, deuterated product, solvate, prodrug, metabolite, pharmaceutically acceptable salt, or co-crystal thereof according to the present invention is calculated in the form of free base in each case.

The term "preparation specification" refers to the weight of the active drug contained in each vial, tablet, or other unit preparation.

Synthetic method of the compound according to the present invention:

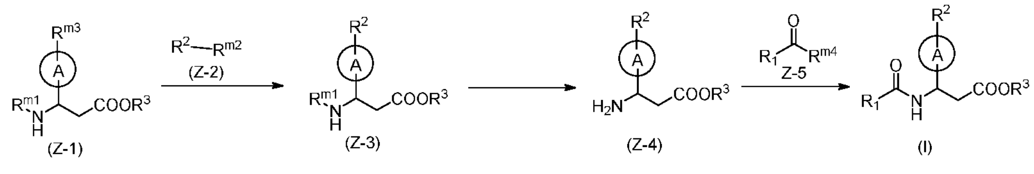


a compound of general formula (Z-1) and a compound of general formula (Z-2) are subjected to coupling or substitution reaction to obtain a compound of general formula (Z-3);

the protecting group of the compound of general formula (Z-3) is removed to obtain a compound of general formula (Z-4); and

the compound of general formula (Z-4) and a compound of general formula (Z-5) are subjected to coupling or substitution reaction to obtain the compound of general formula (I), wherein

$R^{m1}$ is selected from Boc; Cbz; tert-butylsulfinyl, etc.;

$R^{m2}$ and $R^{m3}$ are each independently selected from H, Cl, Br, I, OTf, $B(OH)_2$, boronic ester group, alkyl-substituted stannyl, etc.;

$R^{m4}$ is selected from Cl, Br, I, OH, etc.; and

the definitions of the remaining groups are the same as those in any one of the embodiments of the above general formula (I).

Unless otherwise stated, the terms used in the description and the claims have the following meanings.

The carbon, hydrogen, oxygen, sulfur, nitrogen or F, Cl, Br, I involved in the groups and compounds of the present invention all comprise their isotopes, and the carbon, hydrogen, oxygen, sulfur or nitrogen involved in the groups and compounds of the present invention is optionally replaced with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulfur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"Alkyl" refers to a linear or branched saturated aliphatic hydrocarbon group containing 1 to 20 carbon atoms, preferably alkyl containing 1 to 8 carbon atoms, more preferably alkyl containing 1 to 6 carbon atoms, further preferably alkyl containing 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The alkyl is optionally substituted with 0 to 6 substituents selected from F, Cl, Br, I, hydroxyl, sulfhydryl, nitro, cyano, amino, alkylamino, amido, alkenyl, alkynyl, $C_{1-6}$ alkyl, $C_{1-6}$ hydroxyalkyl, $C_{1-6}$ alkoxy, 3- to 8-membered carbocyclyl, 3- to 8-membered heterocyclyl, 3- to 8-membered carbocyclyloxy, 3- to 8-membered heterocyclyloxy, carboxyl or a carboxylate group.

"Alkylene" refers to linear and branched divalent saturated hydrocarbon groups, including $-(CH_2)_v-$ (v is an integer from 1 to 10), and examples of alkylene include, but are not limited to, methylene, ethylene, propylene, butylene, etc. The alkylene is optionally substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, heterocyclyl, carbocyclyloxy, heterocyclyloxy, carboxyl or a carboxylate group.

"Cycloalkyl" refers to a monovalent saturated carbocyclic hydrocarbon group, usually having from 3 to 12 carbon atoms, and including monocyclic cycloalkyl, fused cycloalkyl, spiro cycloalkyl or bridged cycloalkyl, and non-limiting examples include cyclopropyl, cyclobutyl, cyclopentyl, cyclohexyl, cycloheptyl, etc. The cycloalkyl is optionally substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, heterocyclyl, carbocyclyloxy, heterocyclyloxy, carboxyl or a carboxylate group.

"Alkenyl" refers to linear and branched monovalent unsaturated hydrocarbon groups having at least 1, usually 1, 2 or 3 carbon-carbon double bonds, in which the main chain comprises 2 to 10 carbon atoms, further preferably 2 to 6 carbon atoms, and more preferably 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene and 1,4-hexadiene. The alkenyl is optionally substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, heterocyclyl, carbocyclyloxy, heterocyclyloxy, carboxyl or a carboxylate group.

"Alkynyl" refers to linear and branched unsaturated hydrocarbon groups having at least 1, usually 1, 2 or 3 carbon-carbon triple bonds, in which the main chain comprises 2 to 10 carbon atoms, further preferably 2 to 6 carbon atoms, and more preferably 2 to 4 carbon atoms. Examples of alkynyl include but are not limited to ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5- hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl and 4-decynyl. The alkynyl is optionally substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, heterocyclyl, carbocyclyloxy, heterocyclyloxy, carboxyl or a carboxylate group. The definition of the alkynyl described herein is consistent with this definition.

"Alkoxy" refers to -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy. The alkoxy is optionally substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, heterocyclyl, carbocyclyloxy, heterocyclyloxy, carboxyl or a carboxylate group.

"Carbocyclyl" or "carbocyclic ring" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, benzene ring, naphthalene ring,

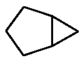, 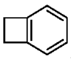, 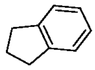 or 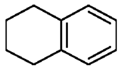.

The carbocyclic ring is optionally substituted with 0 to 5 substituents selected from F, Cl, Br, I, =O, hydroxyl, sulfhydryl, nitro, cyano, amino, alkylamino, amido, alkenyl, alkynyl, alkyl, hydroxyalkyl, alkoxy, carbocyclyl, heterocyclyl, carbocyclyloxy, heterocyclyloxy, carboxyl or a carboxylate group.

"Heterocyclyl" or "heterocyclic ring" refers to a substituted or unsubstituted saturated or unsaturated aromatic ring or non-aromatic ring, and the aromatic ring or non-aromatic ring can be a 3- to 8-membered monocyclic ring, a 4- to 12-membered bicyclic ring or a 10- to 15-membered tricyclic ring system, and contains 1 to 3 heteroatoms selected from N, O or S, preferably 3- to 8-membered heterocyclyl, and the optionally substituted N, S in the ring of heterocyclyl can be oxidized into various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidinyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidinyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

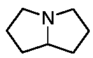,

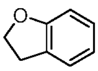, 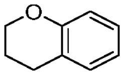, 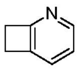, 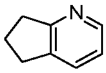, 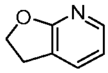, 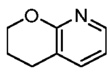, 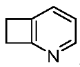, 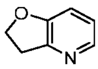, 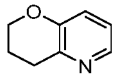,

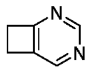, 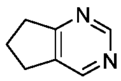, 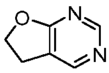 or 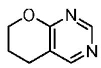.

The heterocyclyl is optionally substituted with 0 to 5 substituents selected from F, Cl, Br, I, =O, hydroxyl, sulfhydryl, nitro, cyano, amino, alkylamino, amido, alkenyl, alkynyl, alkyl, hydroxyalkyl, alkoxy, carbocyclyl, heterocyclyl, carbocyclyloxy, heterocyclyloxy, carboxyl or a carboxylate group.

"Heterocycloalkyl" refers to substituted or unsubstituted saturated heterocyclyl, and can be a 3- to 8-membered monocyclic ring, a 4- to 12-membered bicyclic ring or a 10- to 15-membered tricyclic ring system, and contains 1 to 3 heteroatoms selected from N, O or S, preferably 3- to 8-membered heterocyclyl, and the optionally substituted N, S in the ring of heterocycloalkyl can be oxidized into various oxidation states. Heterocycloalkyl can be connected to a heteroatom or a carbon atom. Non-limiting examples include oxiranyl, aziridinyl, oxetanyl, azetidinyl, 1,3-dioxolanyl, 1,4-dioxolanyl, 1,3-dioxanyl, azepanyl, piperidyl, or morpholinyl. The heterocycloalkyl is optionally substituted with 0 to 5 substituents selected from F, Cl, Br, I, =O, hydroxyl, sulfhydryl, nitro, cyano, amino, alkylamino, amido, alkenyl, alkynyl, alkyl, hydroxyalkyl, alkoxy, carbocyclyl, heterocyclyl, carbocyclyloxy, heterocyclyloxy, carboxyl or a carboxylate group.

"Spiro ring" refers to a 5- to 20-membered polycyclic group sharing one atom (referred to as a spiro atom) between substituted or unsubstituted monocyclic rings, which may contain 0 to 5 double bonds, and may contain 0 to 5 heteroatoms selected from N, O or S(=O)n (n is selected from 0, 1 or 2). The spiro ring is preferably 6- to 14-membered, further preferably 6- to 12-membered, and more preferably 6- to 10-membered. Its non-limiting examples include:

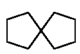 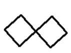 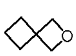 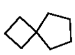 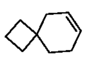 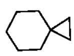 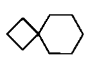

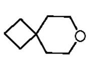 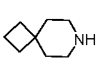 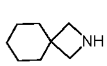 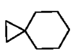 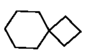 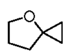 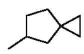

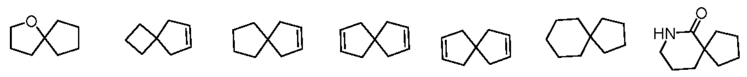

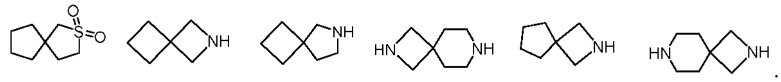

"Fused ring" refers to a polycyclic group in which each ring in the system shares an adjacent pair of atoms with other rings in the system, wherein one or more of the rings may contain 0 or more double bonds, which may be substituted or unsubstituted, and each ring in the fused ring system may contain 0 to 5 heteroatoms selected from N, S(=O)n or O (n is selected from 0, 1 or 2). The fused ring is preferably 5- to 20-membered, further preferably 5- to 14-membered, more preferably 5- to 12-membered, and still further preferably 5- to 10-membered. Non-limiting examples include:

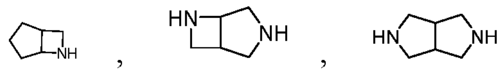

or

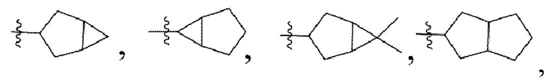

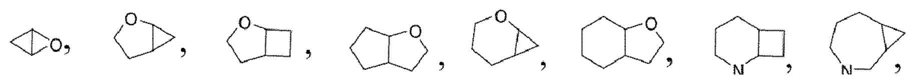

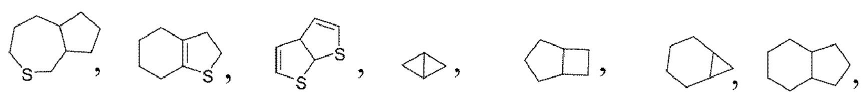

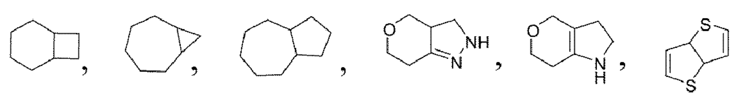 or 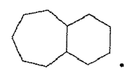.

"Bridged ring" refers to a polycyclic group containing any two atoms that are not directly connected, which group may contain 0 or more double bonds and can be substituted or unsubstituted, and any ring in the bridged ring system may contain 0 to 5 heteroatoms or groups selected from N, S(=O)n or O (wherein n is 0, 1 or 2). The ring atoms contain 5 to 20 atoms, preferably 5 to 14 atoms, further preferably 5 to 12 atoms, and still further preferably 5 to 10 atoms. Non-limiting examples include

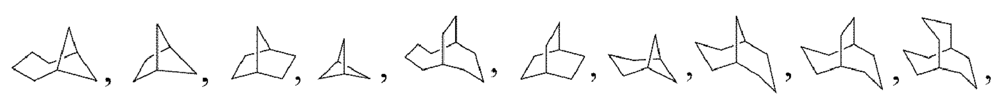

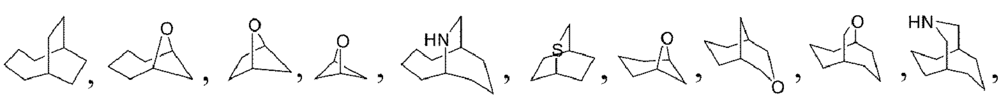

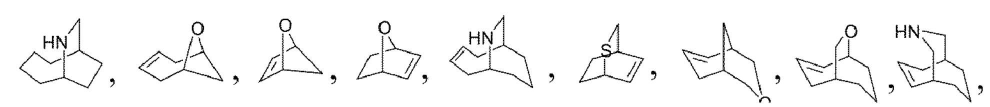

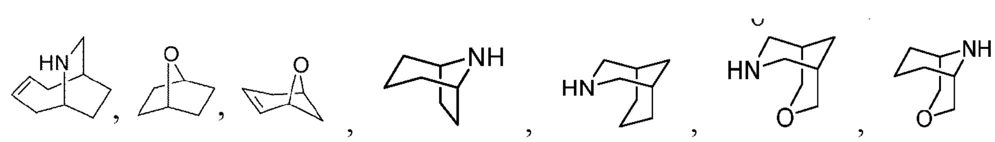

and adamantane. The definition of the bridged ring described herein is consistent with this definition.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Heteromonocyclic ring", "monocyclic heterocyclyl" or "heteromonocyclyl" means "heterocyclyl" or "heterocyclic ring" with a monocyclic system.

"Fused-heterocyclic ring", "fused-heterocyclyl", "fused ring heterocyclyl" or "fused-heterocyclic ring group" refers to a "fused ring" containing a heteroatom.

"Spiro-heterocyclic ring", "spiro-heterocyclyl", "spiro ring heterocyclyl" or "spiro-heterocyclic ring group" refers to a "spiro ring" containing a heteroatom.

"Bridged-heterocyclic ring", "bridged-heterocyclyl" or "bridged ring heterocyclyl" refers to a "bridged ring" containing a heteroatom.

"Aryl" or "aromatic ring" refers to a monovalent aromatic hydrocarbon group with a monocyclic ring or a fused ring, which generally has 6 to 12 carbon atoms and can be substituted or unsubstituted. The definition of the aryl or aromatic ring described herein is consistent with this definition.

"Heteroaryl" refers to substituted or unsubstituted 5- to 15-membered aromatic ring, contains 1 to 5 heteroatoms or groups selected from N, O or S(=O)n, and is preferably 5- to 10-membered heteraromatic ring, and further preferably 5- to 6-membered heteraromatic ring. Non-limiting examples of heteroaryl include, but are not limited to pyridyl, furyl, thienyl, pyridinyl, pyranyl, N-alkylpyrrolyl, pyrimidinyl, pyrazinyl, pyridazinyl, imidazolyl, benzopyrazole, benzimidazolyl, benzopyridine, pyrrolopyridine, etc. The heteroaryl ring can be fused to an aryl, heterocyclyl or cycloalkyl ring, wherein the ring connected to the parent structure is a heteroaryl ring, and non-limiting examples include

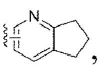, 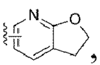, 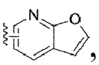, 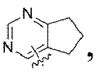, 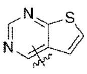, 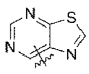, 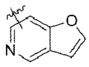 and 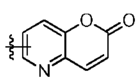.

"Containing 1 to 4 heteroatoms selected from O, S or N" refers to contains 1, 2, 3 or 4 heteroatoms selected from O, S or N.

"Substitution" or "substituted" refers to substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl or $-NR^bR^c$ and the like, wherein $R^b$ and $R^c$ are independently selected from H, hydroxyl, amino, carbonyl, alkyl, alkoxy, cycloalkyl, heterocyclyl, aryl, heteroaryl, sulfonyl, or trifluoromethylsulfonyl. As an option, $R^b$ and $R^c$ may form a five- or six-membered cycloalkyl or heterocyclyl; $R^a$ and $R^d$ are each independently selected from aryl, heteroaryl, alkyl, alkoxy, cycloalkyl, heterocyclyl, carbonyl, ester group, bridged ring group, spiro ring group or fused ring group.

"Substituted with 0 to X substituents" refers to substituted with 0, 1, 2, 3 ... X substituents, wherein X is selected from any integer between 1 and 10. For example, "substituted with 0 to 4 substituents" refers to substituted with 0, 1, 2, 3 or 4 substituents. For example, "substituted with 0 to 5 substituents" refers to substituted with 0, 1, 2, 3, 4 or 5 substituents. For example, "bridged-heterocyclic ring is optionally substituted with 0 to 4 substituents selected from H or F" means that the bridged-heterocyclic ring is optionally substituted with 0, 1, 2, 3 or 4 substituents selected from H or F.

An X- to Y-membered ring (X is selected from an integer less than Y and greater than or equal to 3, and Y is selected from any integer between 4 and 12) includes X-, X+1-, X+2-, X+3-, X+4-, ..., to Y-membered rings. Rings include heterocyclic ring, carbocyclic ring, aromatic ring, aryl, heteroaryl, cycloalkyl, mono-heterocyclic ring, fused-heterocyclic ring, spiro-heterocyclic ring or bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

The term "optional" or "optionally" means that the events or circumstances described subsequently may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound according to the present invention, which salt maintains the biological effectiveness and characteristics of a free acid or a

free base, and is obtained by reacting the free acid with a non-toxic inorganic base or organic base, or reacting the free base with a non-toxic inorganic acid or organic acid.

"Pharmaceutical composition" refers to a mixture of one or more of the compounds of the present invention, a pharmaceutically acceptable salt or a prodrug thereof, and other chemical components, wherein "other chemical components" refer to pharmaceutically acceptable carriers, excipients and/or one or more other therapeutic agents.

"Carrier" refers to a material that does not cause significant irritation to an organism and does not eliminate the biological activity and characteristics of a compound administered.

"Animal" refers to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers and conformational isomers.

## Detailed Description of Embodiments

To achieve the objectives of the present invention, according to organic synthesis techniques known to those skilled in the art, and starting from commercially available chemicals and/or compounds described in chemical documents, the prepared compounds, "commercially available chemicals", for use in the reactions described herein are obtained from standard commercial sources, including Shanghai Aladdin Bio-Chem Technology Co., Ltd., Shanghai Macklin Biochemical Co., Ltd., Sigma-Aldrich, Alfa Aesar (China) Chemical Co., Ltd., Tokyo Chemical Industry (Shanghai) Co., Ltd., Energy Chemical Co., Ltd., Shanghai Titan Scientific Co., Ltd., Kelong Chemical Co., Ltd., J&K Scientific and the like.

The technical solutions of the present invention will be described in detail by the following examples, but the scope of protection of the present invention includes but is not limited thereto.

The structures of the compounds are determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvent for determination is deuterated dimethyl sulfoxide (DMSO-d6), deuterated chloroform (CDCl3) and deuterated methanol (CD3OD); and the internal standard is tetramethylsilane (TMS);

MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

Boc: tert-butoxycarbonyl; Ts: p-toluenesulfonyl; Cbz: benzyloxycarbonyl; TMS: trimethylsilyl;

TIPS: triisopropylsilyl; Bpin: pinacol boronate; MOM: methylenemethyl ether; THF: Tetrahydrofuran
HATU: 2-(7-azabenzotriazole)-N,N,N',N'-tetramethyluronium hexafluorophosphate;
PdCl2(dppf): CAS 72287-26-4; XPhos Pd G2: CAS 1310584-14-5
* next to a chemical bond represents that the chirality of the chiral atom is R or S;
Rt, retention time: the retention time of the analytical method, not specifically stated in the examples, is as follows:

Detection wavelength: 254nm/210nm Flow rate: 1.0 ml/min Column temperature: 35°C
Injection volume: 2μl Acquisition time: 10min

Gradient elution procedure:

| Time (min) | Mobile phase A (%) | Mobile phase B (%) |
|---|---|---|
| 0 | 95 | 5 |
| 5 | 5 | 95 |
| 7 | 5 | 95 |
| 7.01 | 95 | 5 |

(continued)

| Time (min) | Mobile phase A (%) | Mobile phase B (%) |
|---|---|---|
| 10 | 95 | 5 |

Acidic conditions: Mobile phase A: 0.05% TFA solution, mobile phase B: acetonitrile
Chromatographic column brand: Welch, chromatographic column model: Xtimate C18 4.6*50mm, 3μm
Instrument model 1: Shimadzu LC-20AT, Instrument code: CH-Y-J0460
Instrument model 2: Agilent 1260Infinity, Instrument code: CH-Y-J0692

**The specific method** for preparative HPLC (prep-HPLC) **is as follows: Method 1:** instrument: waters 2767 preparative chromatographic column: SunFire@ Prep C18 (19 mm×150 mm); composition of mobile phases: mobile phase A: acetonitrile, and mobile phase B: water (containing 0.1% ammonium acetate). **Method 2:** instrument: waters 2767 preparative chromatographic column: SunFire@ Prep C18 (19 mm×150 mm); composition of mobile phases: mobile phase A: acetonitrile, and mobile phase B: water (containing 0.1% trifluoroacetic acid).

**Intermediate 1:** Preparation of intermediate 1

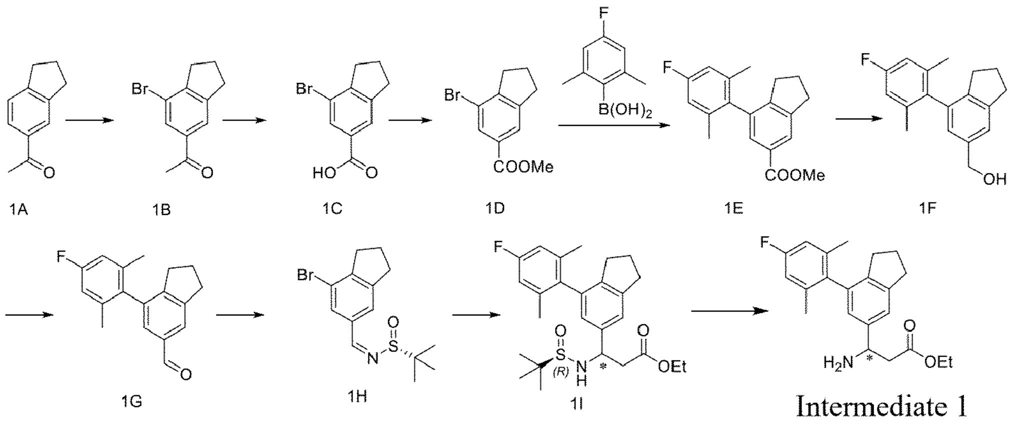


Intermediate 1

Step 1: Synthesis of 1B

Under nitrogen protection, intermediate 1a (6.3 g, 39.34 mmol) was dissolved in dichloromethane (150 mL), and anhydrous aluminum chloride (13.11 g, 98.35 mmol) was added. The reaction mixture was reacted at room temperature for 5 min, a solution of bromine (8.80 g, 55.08 mmol) in dichloromethane (50 mL) was added, and the resulting mixture was reacted at room temperature for 2 h. The reaction liquid was poured into ice water (200 mL), dichloromethane (100 mL) was added, and the resulting mixture was washed with saturated sodium chloride aqueous solution (80 mL), dried over anhydrous sodium sulfate, filtered and concentrated. The residue was subjected to silica gel column chromatography to afford 1B (8.0 g, 85.05%).
LC-Ms m/z (ESI): 241.1 $[M+H]^+$

Step 2: Synthesis of 1C

1B (8.1 g, 33.88mmol) was dissolved in DME (100 mL), 1 M sodium hypochlorite solution (188 mL) and 10 N sodium hydroxide solution (18.5 mL) were added, and the mixture was reacted at 50°C for 1 h. Water (150 mL) was added, the resulting mixture was extracted with diethyl ether (200 mL), and the aqueous phase was adjusted with hydrochloric acid to pH 1-2 and extracted with diethyl ether (150 ml × 2). The organic phases were combined, dried over anhydrous sodium sulfate and concentrated to afford 1C (7.6 g, 93.05%).
LC-Ms m/z (ESI): 240.9 $[M+H]^+$

Step 3: Synthesis of 1D

1C (7.6 g, 31.52 mmol) was dissolved in methanol (50 mL), thionyl chloride (11.25 g, 94.56 mmol) was added, and

the mixture was reacted at 70°C for 2 h. The reaction mixture was concentrated under reduced pressure and subjected to silica gel column chromatography to afford 1D (8.0 g, 99.49%).
LC-Ms m/z (ESI): 255.0 $[M+H]^+$

Step 4: Synthesis of 1E

Under nitrogen protection, 1D (2 g, 7.84 mmol), 4-fluoro2,6-dimethylphenylboronic acid (2.63 g, 15.68 mmol) and cesium carbonate (7.66 g, 23.52 mmol) were added to 1,4-dioxane (30.0 mL) and water (3.0 mL), followed by $Pd(PPh_3)_4$ (1.81 g, 1.57 mmol), and the mixture was reacted at 100°C in a sealed tube for 5 h. The reaction system was cooled to room temperature and filtered through celite. Ethyl acetate (50 mL) was added and the mixture was successively washed with water (30 mL) and saturated sodium chloride aqueous solution (30 mL), dried over anhydrous sodium sulfate, filtered and concentrated under reduced pressure. The residue was subjected to flash chromatography on a silica gel column to afford 1E (2.2 g, yield: 94.05%).

Step 5: Synthesis of 1F

Under nitrogen protection, 1E (2.13 g, 7.14 mmol) was dissolved in dry THF (30 mL) and at 0°C, lithium aluminum hydride (406 mg, 10.71 mmol) was slowly added. The resulting mixture was reacted at room temperature for 1 h. 10% sodium sulfate aqueous solution (20 mL) was added and the mixture was filtered. The solvent was removed under reduced pressure to afford crude 1F.

Step 6: Synthesis of 1g

Under nitrogen protection, crude 1F (1.87 g, 6.92 mmol) was dissolved in dry dichloromethane (35 mL), sodium bicarbonate (1.16 g, 13.84 mmol) and Dess-Martin periodinane (3.81 g, 9.00 mmol) were added at room temperature, and the resulting mixture was reacted at room temperature for 1 h. Saturated sodium thiosulfate solution (10 mL) and saturated sodium bicarbonate solution (10 mL) were added and the mixture was extracted with dichloromethane (50 mL×3), dried over anhydrous sodium sulfate and filtered. The solvent was removed under reduced pressure and the residue was subjected to silica gel column chromatography to afford 1G (1.63 g, 87.68%).

Step 7: Synthesis of 1H

Under nitrogen protection, 1G (1.61 g, 6.00 mmol) and R-tert-butylsulfinamide (1.01 g, 9.00 mmol) were dissolved in THF (35 mL), tetraethyl titanate (2.05 g, 9.00 mmol) was slowly added, and the resulting mixture was reacted at 45°C for 15 h and concentrated under reduced pressure to afford a crude, which was subjected to flash column chromatography on a silica gel column to afford 1H (1.73 g, yield: 77.61%).
LC-Ms m/z (ESI): 329.3 $[M+H]^+$

Step 8: Synthesis of 1I

Zinc powder (915 mg, 14 mmol) was added to dry THF (5 mL), the mixture was subjected to nitrogen replacement three times, CuCl (297 mg, 3 mmol) was added, and the resulting mixture was reacted at 60°C for 2 h. The reaction mixture was cooled to room temperature, ethyl bromoacetate (835 mg, 5 mmol) was slowly added, and the resulting mixture was reacted at 60°C for 1 h and cooled to 0°C. A solution of 1H (371 mg, 1 mmol) in THF (1 mL) was added and the mixture was stirred at 0°C for 3 h. The reaction mixture was filtered through celite and saturated ammonium chloride solution (20 mL) was added. The resulting mixture was extracted with ethyl acetate (30 ml × 3), washed with saturated sodium chloride aqueous solution (20 mL), dried over anhydrous sodium sulfate, filtered and concentrated under reduced pressure. The residue was subjected to flash column chromatography to afford 1I (386 mg, yield: 83.98%).
LC-Ms m/z (ESI): 460.2 $[M+H]^+$

Step 9: Synthesis of intermediate 1

1I (272 mg, 0.27 mmol) was dissolved in THF (2 mL), 4 N hydrogen chloride dioxane solution (2 mL) was added, and the resulting mixture was stirred at room temperature for 3 h. The reaction mixture was concentrated under reduced pressure to afford crude intermediate 1.
LC-Ms m/z (ESI): 356.3 $[M+H]^+$

**Intermediate 2:** Preparation of intermediate 2

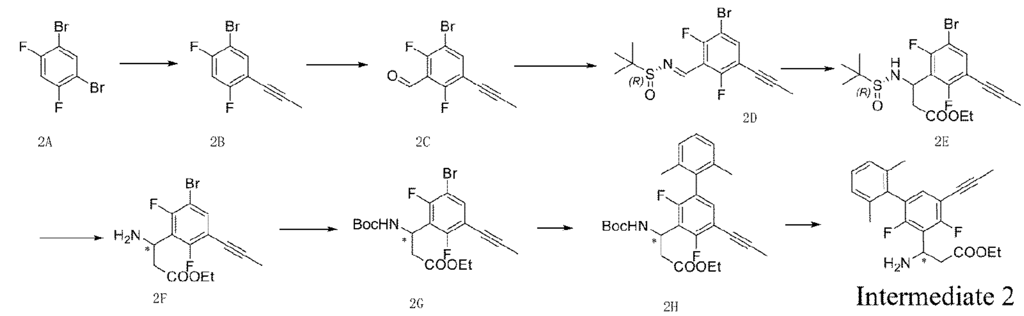

Step 1: Synthesis of 2B

Under nitrogen protection, 2A (25 g, 91.94 mmol), CuI (3.5 g, 18.39 mmol), $PdCl_2(PPh_3)_2$ (6.45 g, 9.19 mmol), 1-(trimethylsilyl)propyne (9.29 g, 82.75 mmol) and cesium fluoride (41.90 g, 275.82 mmol) were dissolved in THF (250 mL) and the resulting mixture was reacted at 60°C for 72 h, concentrated under reduced pressure and then subjected to silica gel column chromatography to afford 2B (8.5 g, 40.02%).

Step 2: Synthesis of 2C

Under nitrogen protection, 2B (8.5 g, 36.79 mmol) was dissolved in THF (75 mL). At -78°C, lithium diisopropylamide (5.91 g, 55.19 mmol) was added and the resulting mixture was stirred for 1 h. DMF (5.38 g, 73.58 mmol) was added dropwise and the mixture was stirred for 30 min. Saturated aqueous ammonium chloride solution (150 mL) was added and the resulting mixture was extracted with ethyl acetate (200 mL×3). The organic phases were combined, dried over anhydrous sodium sulfate and concentrated to afford 2C (7.2 g, 75.55%).

Step 3: Synthesis of 2D

Under nitrogen protection, 2C (7.2 g, 27.79 mmol) and R-tert-butylsulfinamide (4.04 g, 33.35 mmol) were dissolved in THF (75 mL), tetraethyl titanate (9.51 g, 41.69 mmol) was slowly added, and the resulting mixture was reacted at 45°C for 3 h and concentrated under reduced pressure to afford a crude, which was subjected to flash column chromatography on a silica gel column to afford 2D (6.80 g, yield: 67.55%).

Step 4: Synthesis of 2E

Zinc powder (17.19 g, 262.78 mmol) was added to dry THF (45 mL), the mixture was subjected to nitrogen replacement three times, CuCl (5.57 g, 56.31 mmol) was added, and the resulting mixture was reacted at 60°C for 2 h. The reaction mixture was cooled to room temperature, ethyl bromoacetate (15.67 g, 93.85 mmol) was slowly added, and the resulting mixture was reacted at 60°C for 1 h and cooled to 0°C. A solution of 2D (6.8 g, 18.77 mmol) in THF (10 mL) was added and the mixture was stirred at 0°C for 3 h. The reaction mixture was filtered through celite and saturated ammonium chloride solution (200 mL) was added. The resulting mixture was extracted with ethyl acetate (300 ml × 3), washed with saturated sodium chloride aqueous solution (200 mL), dried over anhydrous sodium sulfate, filtered and concentrated under reduced pressure. The residue was subjected to flash column chromatography to afford 2E (7.0 g, yield: 82.81%).

Step 5: Synthesis of 2F

2E (3.0 g, 6.66 mmol) was dissolved in dichloromethane (10 mL), 4 N hydrogen chloride dioxane solution (5 mL) was added, and the resulting mixture was stirred at room temperature for 3 h. The mixture was concentrated under reduced pressure to afford crude 2F.

Step 6: Synthesis of 2G

Under nitrogen protection, crude 2F (2.0 g, 5.78 mmol) was dissolved in dry dichloromethane (35 mL), triethylamine (1.75g, 17.34 mmol) and di-tert-butyl dicarbonate (1.51 g, 6.94 mmol) were added at room temperature,

and the resulting mixture was reacted at room temperature for 3 h. The solvent was removed under reduced pressure and the residue was subjected to column chromatography on a silica gel column to afford 2G (1. 3 g, 50.40%).

Step 7: Synthesis of 2H

Under nitrogen protection, 2G (0.45 g, 1.11 mmol), 2,6-dimethylphenylboronic acid (0.50 g, 3.33 mmol), potassium phosphate (0.47 g, 2.21 mmol), and RuPhos Pd G3 (0.19 g, 0.22 mmol) were dissolved in DMF (8 mL) and water (0.8 mL) and the mixture was stirred at 75°C for 3 h. Water (20 mL) was added and the resulting mixture was extracted with ethyl acetate (30 ml × 3), washed with saturated sodium chloride aqueous solution (20 mL×2), dried over anhydrous sodium sulfate, filtered and concentrated under reduced pressure. The residue was subjected to flash column chromatography to afford 2H (0.3 g, yield: 57.38%).

Step 8: Synthesis of intermediate 2

2H (300 mg, 0.64 mmol) was dissolved in dichloromethane (2 mL), 4 N hydrogen chloride dioxane solution (2 mL) was added, and the resulting mixture was stirred at room temperature for 3 h. The reaction mixture was concentrated under reduced pressure to afford crude intermediate 2.
LC-Ms m/z (ESI): 372.2 $[M+H]^+$

**Intermediate 4:**

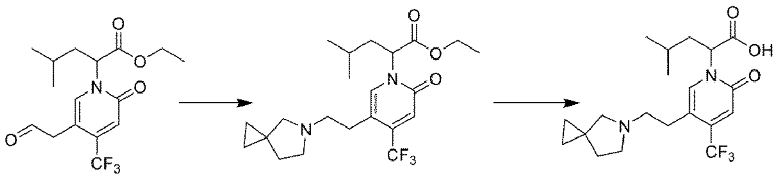

1a 4A Intermediate 4

Step 1: Synthesis of 4A

Under nitrogen protection, 1a (1.5 g, 4.32 mmol, referring to WO 2021076890 A1 for synthetic step) and 5-azaspiro[2.4]heptane (0.42 g, 4.32 mmol) were dissolved in 1,2-dichloroethane (10 mL), acetic acid (0.5 mL) was added dropwise, and the mixture was stirred at room temperature for 2 h. Sodium triacetoxyborohydride (1.82 g, 8.64 mmol) was added and the resulting mixture was stirred for 16 h, concentrated under reduced pressure and then subjected to flash column chromatography on a silica gel column to afford 4A (1.0 g, yield: 54.05%).
LC-Ms m/z (ESI): 429.2 $[M+H]^+$

Step 2: Synthesis of intermediate 4

4A (1.0 g, 2.34 mmol) was dissolved in THF (3 mL) and water (1 mL), lithium hydroxide monohydrate (112 mg, 4.68 mmol) was added, and the resulting mixture was reacted at room temperature for 5 h. The reaction mixture was adjusted with 1 N hydrochloric acid to pH 5-6, concentrated under reduced pressure and then subjected to column chromatography to afford intermediate 4 (650 mg, 69.55%).
LC-Ms m/z (ESI): 401.2 $[M+H]^+$

**Intermediate 5:** Preparation of intermediate 5

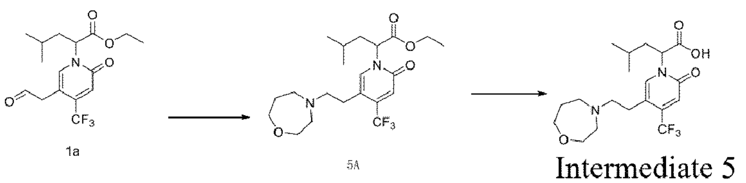

1a 5A Intermediate 5

Step 1: Synthesis of 5A

Under nitrogen protection, 1,4-oxazepane hydrochloride salt (360 mg, 2.59 mmol) was dissolved in 1,2-dichloroethane (8 mL), DIPEA was added, and the resulting mixture was reacted at room temperature for 15 min. 1a (0.9 g, 2.59 mmol, referring to WO 2021076890 A1 for synthetic step) and acetic acid (0.15 mL) were added and the mixture was stirred at room temperature for 1 h. Sodium triacetoxyborohydride (820 mg, 3.86 mmol) was added and the mixture was stirred for 16 h and concentrated under reduced pressure to afford a crude, which was subjected to flash column chromatography on a silica gel column to afford 5A (0.49 g, yield: 43.75%).
LC-Ms m/z (ESI): 433.7 $[M+H]^+$

Step 2: Synthesis of intermediate 5

5A (0.72 g, 1.66 mmol) was dissolved in ethanol (6 mL) and water (0.6 mL), lithium hydroxide monohydrate (210 mg, 4.98 mmol) was added, and the resulting mixture was reacted at room temperature for 3 h. The reaction mixture was adjusted with 1 N hydrochloric acid to pH 4, concentrated under reduced pressure and then subjected to column chromatography to afford intermediate 5 (530 mg, yield: 78.95%).
LC-Ms m/z (ESI): 405.6 $[M+H]^+$

**Intermediate 6:** Preparation of intermediate 6

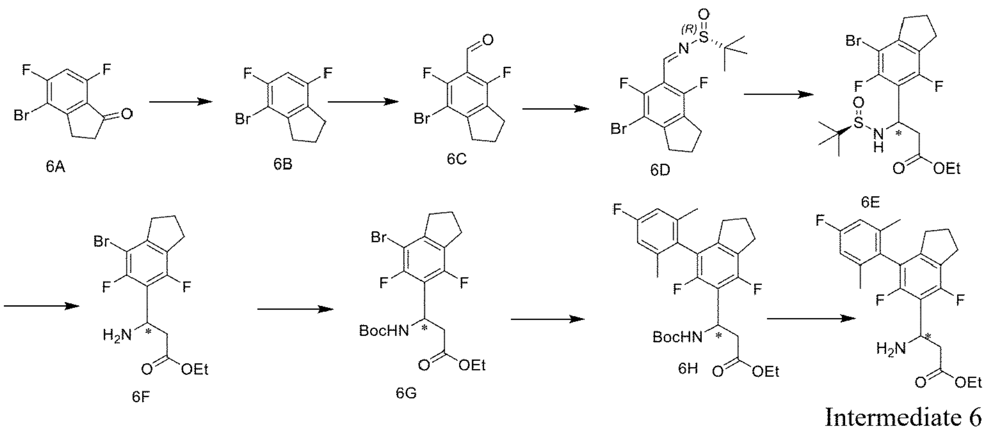

6A 6B 6C 6D 6E 6F 6G 6H

Intermediate 6

Step 1: Synthesis of 6B

6A (1720 mg, 6.96 mmol) was dissolved in trifluoroacetic acid (25 mL), triethylsilane (25 mL) was added, and the mixture was reacted at room temperature for 48 h. Triethylsilane (15 mL) was then added and the resulting mixture was stirred and reacted overnight. The mixture was concentrated under reduced pressure at 30 °C, and the residue was separated and purified by column chromatography on a silica gel column to afford crude compound 6B (1700 mg).

Step 2: Synthesis of 6C

Under nitrogen protection, the crude 6B (1700 mg, 7.29 mmol) was dissolved in dry THF (70 mL), and at -78°C, lithium diisopropylamide (1.56 g, 14.58 mmol) was slowly added. The mixture was stirred at this temperature for 0.5 h and dry DMF (2.6 g, 36.45 mmol) was then added. At -78°C, the mixture was reacted for another 1 h, and the reaction was quenched with ammonium chloride solution (50 mL). The reaction mixture was extracted with ethyl acetate (80 mL×3), dried over anhydrous sodium sulfate and filtered. The solvent was removed under reduced pressure and the residue was separated and purified by column chromatography on a silica gel column to afford 6C (1.58 g, two-step yield: 83.02%).

Step 3: Synthesis of 6D

Under nitrogen protection, 6C (1580 mg, 6.05 mmol) and R-tert-butylsulfinamide (1100 mg, 9.7 mmol) were dissolved in THF (35 mL), tetraethyl titanate (2070 mg, 9.07 mmol) was slowly added, and the resulting mixture was reacted at 45°C for 15 h and concentrated under reduced pressure to afford a crude, which was subjected to flash column

chromatography on a silica gel column to afford 6D (2100 mg, yield: 95.29%).
LC-Ms m/z (ESI): 364.2[M+H]+

Step 4: Synthesis of 6E

Zinc powder (1.2 g, 18.34 mmol) was added to dry THF (8 mL), the mixture was subjected to nitrogen replacement three times, CuCl (389 mg, 3.93 mmol) was added, and the resulting mixture was reacted at 60°C for 2 h. The reaction mixture was cooled to room temperature, ethyl bromoacetate (1.09 g, 6.55 mmol) was slowly added, and the resulting mixture was reacted at 60°C for 1 h and cooled to 0°C. A solution of 6D (478 mg, 1.31 mmol) in THF (3 mL) was added and the mixture was stirred at 0°C for 3 h. The reaction mixture was filtered through celite and saturated ammonium chloride solution (30 mL) was added. The resulting mixture was extracted with ethyl acetate (40 mL × 3), washed with saturated sodium chloride aqueous solution (20 mL), dried over anhydrous sodium sulfate, filtered and concentrated under reduced pressure. The residue was subjected to flash column chromatography to afford 6E (498 mg, yield: 84.04%).
LC-Ms m/z (ESI): 452.4[M+H]+

Step 5: Synthesis of 6F

6E (448 mg, 0.99 mmol) was dissolved in dichloromethane (3 mL), 4 *N* hydrogen chloride dioxane solution (2.5 mL) was added, and the resulting mixture was stirred at room temperature for 3 h. The reaction mixture was concentrated under reduced pressure to afford the hydrochloride salt of crude 6F.
LC-Ms m/z (ESI): 348.3 [M+H]+

Step 6: Synthesis of 6G

6F was dissolved in THF (4 mL) and water (4 mL), and sodium carbonate (210 mg, 1.98 mmol) and $Boc_2O$ (240 mg, 1.09 mmol) were added and stirred at room temperature for 3h. Water (10 mL) was added and the resulting mixture was extracted with ethyl acetate (20 mL×3), dried over anhydrous sodium sulfate, filtered and concentrated under reduced pressure. The residue was subjected to flash column chromatography to afford 6G (420 mg, two-step yield: 86.31%).
LC-Ms m/z (ESI): 350.0 [M+H-Boc]+

Step 7: Synthesis of 6H

Under nitrogen protection, 6G (230 mg, 0.51 mmol), 2,6-dimethyl-4-fluorophenylboronic acid (128 mg, 0.77 mmol) and potassium phosphate (325 mg, 1.53 mmol) were added to 1,4-dioxane (2.5 mL) and water (0.25 mL), followed by Xphos-G2-Pd (40 mg, 0.051 mmol), and the resulting mixture was reacted at 100°C in a sealed tube for 24 h. The reaction system was cooled to room temperature. Ethyl acetate (40 mL) was added and the mixture was successively washed with water (20 mL) and saturated sodium chloride aqueous solution (20 mL), dried over anhydrous sodium sulfate, filtered and concentrated under reduced pressure. The residue was subjected to flash chromatography on a silica gel column to afford 6H (210 mg, yield: 83.77%).
LC-Ms m/z (ESI): 392.6 [M+H-Boc]+

Step 8: Synthesis of intermediate 6

6H (210 mg, 0.43 mmol) was dissolved in THF (3 mL), 4 N hydrogen chloride dioxane solution (2.5 mL) was added, and the resulting mixture was stirred at room temperature for 3 h. The reaction mixture was concentrated under reduced pressure to afford the hydrochloride salt of crude intermediate 6.
LC-Ms m/z (ESI): 392.6[M+H]+

**Intermediate 7:** Preparation of intermediate 7

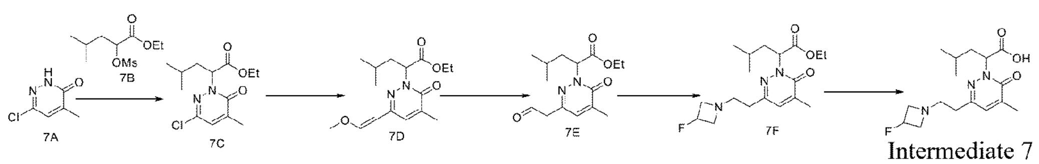

Intermediate 7

Step 1: Synthesis of 7C

Under nitrogen protection, 7A (1.0 g, 6.92 mmol) and 7B (2.14 g, 9.0 mmol) were dissolved in ultra-dry DMF (42 mL) and potassium carbonate (1.91 g, 13.84 mmol) was added. The mixture was reacted under a nitrogen atmosphere at 80 °C for 4 h. 100 mL of ethyl acetate was added to dilute the system, and the organic phase was washed with water (50 mL x 3), followed by saturated sodium chloride aqueous solution (50 mL), and dried over anhydrous sodium sulfate, filtered and concentrated. The residue was subjected to silica gel column chromatography to afford 7C (2.18 g, 99%).
LC-Ms m/z (ESI): 287.1 $[M+H]^+$

Step 2: Synthesis of 7D

7C (2 g , 6.92 mmol) and (E)-2-(2-ethoxyvinyl)-4,4,5,5-tetramethyl-1,3,2-dioxaborolane (2.06 g, 10.38 mmol) were dissolved in a mixed solution of toluene (30 mL) and water (6 mL), and sodium carbonate (2.2 g, 20.76 mmol) and PdCl2(dppf) (506 mg, 0.692 mmol) were added. Reaction was carried out under nitrogen atmosphere at 100°C overnight. 50 mL of water was added to dilute the system, and extraction was performed by addition of ethyl acetate (80 mL x 3). The organic phase was washed with saturated sodium chloride aqueous solution (50 mL), dried over anhydrous sodium sulfate, filtrated, concentrated and subjected to silica gel column chromatography to afford 7D (2.27 g, 99%).
LC-Ms m/z (ESI): 309.1 $[M+H]^+$

Step 3: Synthesis of 7E

7D (2.27 g, 6.92 mmol) was dissolved in dichloromethane (50 mL), trifluoroacetic acid (15 mL) was added, and the resulting mixture was reacted at room temperature for 2 h. The reaction mixture was concentrated under reduced pressure to afford crude intermediate 7E.
LC-Ms m/z (ESI): 295.1 $[M+H]^+$

Step 4: Synthesis of 7F

Under nitrogen protection, the crude 7E was dissolved in 1,2-dichloroethane (20 mL) and methanol (20 mL). 3-Fluoroazetidine hydrochloride salt (780 mg, 10.38 mmol) and acetic acid (0.5 mL) were added, and the mixture was stirred at room temperature for 1 h. Sodium triacetoxyborohydride (4.38 g, 20.76 mmol) was added and the reaction was carried out overnight. The system was diluted with 100 mL of water, and saturated sodium bicarbonate solution was added to adjusted pH = 9-10. The mixture was extracted with dichloromethane (100 mL × 3), and the organic phase was washed with saturated sodium chloride aqueous solution (50 mL). The mixture was dried over anhydrous sodium sulfate, filtrated, concentrated, and subjected to silica gel column chromatography to afford 7F (1.25 g, 48% over two steps).
LC-Ms m/z (ESI): 354.2 $[M+H]^+$

Step 5: Synthesis of intermediate 7

7F (1.25 g, 3.54 mmol) was dissolved in ethanol (15 mL) and water (1.5 mL), lithium hydroxide monohydrate (446 mg, 10.62 mmol) was added, and the resulting mixture was reacted at room temperature for 3 h. The reaction mixture was adjusted with 1 N hydrochloric acid to pH 4, concentrated under reduced pressure and then subjected to column chromatography to afford intermediate 7 (700 mg, 61%).
LC-Ms m/z (ESI): 362.2 $[M+H]^+$

Example 1 Preparation of compound 1

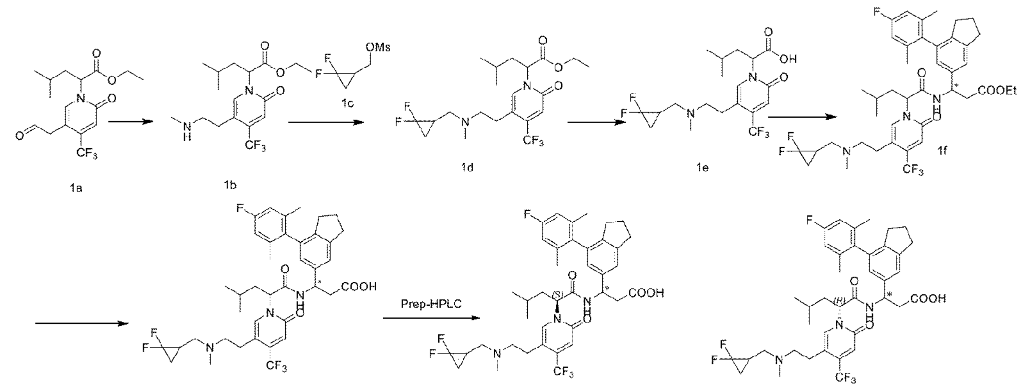

Compound 1
Compound 1-1 and Compound 1-2

Step 1: preparation of 1b

Under nitrogen protection, 1a (3.0 g, 8.64 mmol, referring to WO 2021076890 A1 for synthetic step) and methanamine hydrochloride salt (1.16 g, 17.28 mmol) were dissolved in 1,2-dichloroethane (30 mL). 5 drops of acetic acid were added dropwise and the mixture was stirred at room temperature for 2h. Sodium triacetoxyborohydride (3.65 g, 17.28 mmol) was added and the resulting mixture was stirred for another 16 h, and concentrated under reduced pressure to afford a crude, which was then subjected to flash column chromatography on a silica gel column to afford 1b (1.0 g, yield: 31.95%).
LC-Ms m/z (ESI): 363.2 $[M+H]^+$

Step 2: Preparation of 1d

Under nitrogen protection, 1b (1.0 g, 2.76 mmol) and 1c (1.02 g, 5.52 mmol) were dissolved in DMF (30 mL), potassium carbonate (1.14 g, 8.28 mmol) was added, and the resulting mixture was stirred at 60°C for 24 h and concentrated under reduced pressure to afford a crude, which was subjected to flash column chromatography on a silica gel column to afford 1d (1.0 g, yield: 80.08%).
LC-Ms m/z (ESI): 453.2 $[M+H]^+$

Step 3: Preparation of 1e

1d (1.0 g, 2.21 mmol) was dissolved in THF (3 mL) and water (1 mL), lithium hydroxide monohydrate (112 mg, 4.68 mmol) was added, and the resulting mixture was reacted at room temperature for 5 h. The reaction mixture was adjusted with 1 N hydrochloric acid to pH 5-6, concentrated under reduced pressure and then subjected to column chromatography to afford 1e (700 mg, 74.62%).
LC-Ms m/z (ESI): 425.2 $[M+H]^+$

Step 4: Preparation of 1f

Under nitrogen protection, 1e (280 mg, 0.66 mmol) was dissolved in dry DMF (1.5 mL), and HATU (376 mg, 0.99 mmol) and DIPEA (225 mg, 1.74 mmol) were added. At room temperature, the reaction mixture was stirred for 40 min, and then intermediate 1 (234 mg, 0.66 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 1f (300 mg).
LC-Ms m/z (ESI): 762.4 $[M+H]^+$

Step 5: Preparation of compounds 1-1 and 1-2

1f (300 mg) was dissolved in THF (3 mL) and water (1 mL), lithium hydroxide monohydrate (45 mg, 1.08 mmol) was added, and the resulting mixture was reacted at room temperature for 5 h. PH was adjusted to 5-6 with 1 N hydrochloric acid, and the resulting solution was concentrated under reduced pressure to afford **crude compound 1,** which was separated and purified by **prep-**HPLC **(method 1)** to afford compound 1-1 (15 mg, Rt = 4.325 min, yield: 5.19%) and

compound 1-2 (15 mg, Rt = 4.429 min, yield: 5.19%).

compound 1-1:

LC-Ms m/z (ESI): 734.3 [M+H]$^+$

$^1$H NMR (400 MHz, Methanol-$d_4$) δ 7.93 (s, 1H), 7.17 (s, 1H), 6.84 - 6.72 (m, 4H), 5.75 (t, 1H), 5.34 - 5.28 (m, 1H), 3.54 - 3.43 (m, 1H), 3.40 - 3.33 (m, 2H), 3.09 - 2.95 (m, 5H), 2.95 - 2.89 (m, 2H), 2.86 - 2.80 (m, 2H), 2.44 - 2.36 (m, 2H), 2.19 - 2.08 (m, 1H), 2.02 - 1.93 (m, 4H), 1.88 (s, 3H), 1.80 (s, 4H), 1.55 - 1.41 (m, 2H), 1.01 - 0.91 (m, 6H).

Compound 1-2:

LC-Ms m/z (ESI): 734.3 [M+H]$^+$

$^1$H NMR (400 MHz, Methanol-$d_4$) δ 7.93 (s, 1H), 7.22 (s, 1H), 6.89 (s, 1H), 6.85 (s, 1H), 6.84 - 6.79 (m, 2H), 5.73 (t, 1H), 5.31 (t, 1H), 3.53 - 3.45 (m, 1H), 3.41 - 3.33 (m, 3H), 3.08 - 2.95 (m, 7H), 2.89 - 2.76 (m, 2H), 2.49 - 2.42 (m, 2H), 2.21 - 2.09 (m, 1H), 2.06 - 2.00 (m, 2H), 1.94 (s, 6H), 1.85 - 1.78 (m, 3H), 1.54 - 1.45 (m, 1H), 1.38 - 1.27 (m, 1H), 0.92 - 0.83 (m, 6H).

**Example 2:** Preparation of compound 2

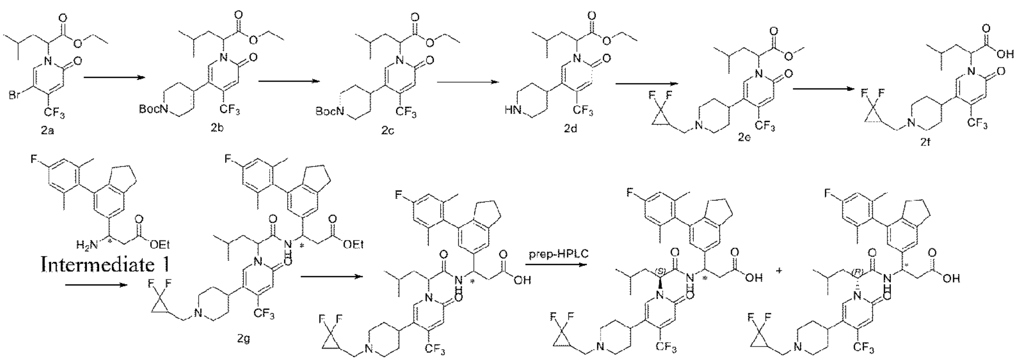


Compound 2 Compound 2-1 and Compound 2-2

Step 1: Synthesis of 2b

Under nitrogen protection, 2a (6.0 g, 15.62 mmol, referring to WO 2021076890 A1 for synthetic step) was dissolved in 1,4-dioxane (60 mL) and water (6 mL), N-Boc-1,2,5,6-tetrahydropyridine-4-boronic acid pinacol ester (5.8 g, 18.74 mmol), $Pd(PPh_3)_4$ (1.8 g, 1.56 mmol) and potassium carbonate (6.48 g, 46.86 mmol) were successively added, and the mixture was reacted at 70°C for 30 h. After the reaction was complete, extraction was performed by addition of ethyl acetate (180 mL) and the resulting mixture was washed with water (50 mL×2) and saturated sodium chloride aqueous solution (50 mL×1), dried over anhydrous sodium sulfate and filtered and the filtrate was concentrated under reduced pressure and subjected to column chromatography on a silica gel column to afford 2b (5.0 g, yield: 65.79%).
LC-Ms m/z (ESI): 487.3 [M+H]$^+$

Step 2: Synthesis of 2c

Under hydrogen conditions, 2b (5.0 g, 10.28 mmol) was dissolved in methanol (50 mL), an equal mass of Pd/C was added, and the reaction was carried out for 2 h at room temperature. The solid was removed by filtration and the filtrate was concentrated and purified by column chromatography to afford 2c (2.5 g, yield: 49.78%).
LC-Ms m/z (ESI): 489.3 [M+H]$^+$

Step 3: Synthesis of 2d

2c (480.0 mg, 0.98 mmol) was dissolved in dichloromethane (5 mL), hydrogen chloride-1,4-dioxane (3 mL) was added, and the reaction was carried out for 1 h. After the reaction was complete, the solvent was removed under reduced pressure to afford crude compound 2d.
LC-Ms m/z (ESI): 389.2 $[M+H]^+$

Step 4: Synthesis of 2e

2d (350.0 mg, 0.90 mmol) was dissolved in methanol (4 mL), triethylamine (273.21 mg, 2.7 mmol) and methyl (2,2-difluorocyclopropyl)methanesulfonate (201.07 mg, 1.08 mmol) were added sequentially and the reaction was carried out at 60 °C for 16 h. After the reaction was complete, the reaction solution was purified by column chromatography to afford 2e (280.0 mg, yield: 65.02%).
LC-Ms m/z (ESI): 465.90 $[M+H]^+$

Step 5: Synthesis of 2f

2e (280.0 mg, 0.60 mmol) was dissolved in methanol (2 mL) and water (1 mL), lithium hydroxide (28.47 mg, 1.20 mmol) was added, and the reaction was carried out at room temperature for 1-2 h. After the reaction was complete, the reaction solution was purified by reverse phase column chromatography to afford 2f (100.0 mg, yield: 37.0%).
LC-Ms m/z (ESI): 451.70 $[M+H]^+$

Step 6: Synthesis of 2g

Under nitrogen protection, intermediate 1 (80.0 mg, 0.23 mmol) was dissolved in dry DMF (1.5 mL), and HOBt (62.16 mg, 0.46 mmol), EDCI (88.18 mg, 0.46 mmol) and DIPEA (118.90 mg, 0.92 mmol) were successively added. At room temperature, the reaction mixture was stirred for 40 min, and then 2e (100.0 mg, 0.23 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 2g (50.0 mg, yield: 28.59%).
LC-Ms m/z (ESI): 788.4 $[M+H]^+$

Step 7: Synthesis of compound 2-1 and compound 2-2

Compound 2g (50.0 mg, 0.063 mmol) was dissolved in methanol (1 mL) and water (0.5 mL), lithium hydroxide monohydrate (3.02 mg, 0.13 mmol) was added, and the resulting mixture was reacted at room temperature for 30 min. PH was adjusted to 5-6 with 1 N hydrochloric acid and concentrated under reduced pressure to afford crude compound 2, which was separated and purified by prep-HPLC **(Method 2)** to afford the trifluoroacetate salt of compound 2-1 (4.0 mg, Rt = 5.955 min, yield: 8.36%) and the trifluoroacetate salt of compound 2-2 (3.0 mg, Rt = 5.989 min, yield: 6.27%).

Trifluoroacetate salt of compound 2-1:

LC-Ms m/z (ESI): 760.3 $[M+H]^+$
$^1$H NMR (400 MHz, Methanol-$d_4$) δ 9.16 (d, 1H), 7.81 (s, 1H), 7.16 (d, 1H), 6.83 - 6.74 (m, 3H), 6.72 (d, 1H), 5.76 (t, 1H), 5.38 - 5.29 (m, 1H), 4.55 - 4.45 (m, 1H), 3.77 - 3.61 (m, 2H), 3.50 - 3.39 (m, 1H), 3.26 - 3.10 (m, 3H), 3.04 - 2.79 (m, 6H), 2.45 - 2.35 (m, 2H), 2.22 - 2.07 (m, 2H), 2.04 - 1.93 (m, 4H), 1.88 - 1.81 (m, 4H), 1.78 (s, 3H), 1.65 (d, 1H), 1.53 - 1.41 (m, 2H), 1.04 - 0.91 (m, 6H).

Trifluoroacetate salt of compound 2-2:

LC-Ms m/z (ESI): 760.3 $[M+H]^+$
$^1$H NMR (400 MHz, Methanol-$d_4$) δ 7.87 (s, 1H), 7.23 (d, 1H), 6.91 - 6.74 (m, 4H), 5.77 - 5.70 (m, 1H), 5.36 - 5.24 (m 1H), 4.56 - 4.45 (m, 1H), 3.79 - 3.64 (m, 2H), 3.48 - 3.39 (m, 1H), 3.26 - 3.12 (m, 2H), 3.00 - 2.93 (m, 2H), 2.90 - 2.76 (m, 2H),

2.49 - 2.38 (m, 2H), 2.22 - 1.99 (m, 5H), 1.94 (s, 6H), 1.88 - 1.78 (m, 2H), 1.77 - 1.68 (m, 1H), 1.68 - 1.61 (m, 2H), 1.55 - 1.43 (m, 1H), 1.37 - 1.26 (m, 2H), 0.96 - 0.78 (m, 6H).

**Example 3:** Preparation of compound 3

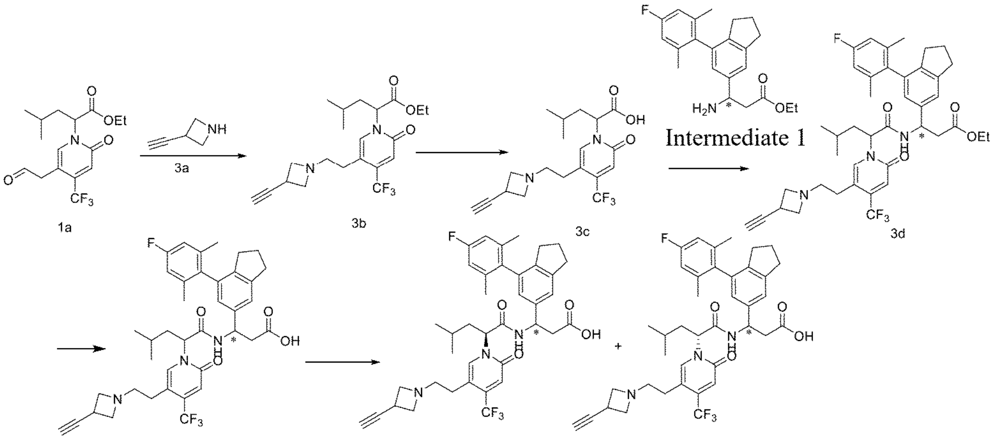


1a 3b 3c 3d

Compound 3

Compound 3-1 and Compound 3-2

Step 1: Preparation of 3b

1a (1.4 g crude), 3a (1.2 g, 7.2 mmol) and glacial acetic acid (220 mg, 3.6 mmol) were dissolved in 1,2-dichloroethane (20 mL), stirred at room temperature under nitrogen protection for 30 min, and sodium triacetoxyborohydride (1.5 g, 7.2 mmol) was added. The mixture was stirred at room temperature overnight. 50 mL of ethyl acetate was added, and the mixed solution was successively washed with water (30 mL×1) and saturated sodium chloride aqueous solution (30 mL×1). The organic layer was dried over anhydrous sodium sulfate and filtered, and the filtrate was concentrated under reduced pressure, and the residue was subjected to column chromatography to afford 3b (1.05 g, yield: 71%).
LCMS m/z = 413.2 $[M+H]^+$

Step 2: Preparation of 3c

3b (1.05 g, 2.55 mmol) was dissolved in ethanol (15 mL) and water (1.5 mL), lithium hydroxide monohydrate (321 mg, 7.65 mmol) was added, and the reaction was carried out at room temperature for 5 h. The solvent was removed by concentration under reduced pressure, 20 mL of water was added to the residue, and the pH was adjusted to around 2-3 with 1N hydrochloric acid. The aqueous phase was extracted with ethyl acetate (50 mL × 2), and the organic phases are combined. The organic phase was washed with saturated sodium chloride aqueous solution (30 mL x 1), the organic layer was dried over anhydrous sodium sulfate and filtered, and the filtrate was concentrated under reduced pressure. The residue was subjected to reverse phase (0.1% trifluoroacetic acid) preparative chromatography to afford 3c (410 mg, yield: 42%).
LCMS m/z = 385.2 $[M+H]^+$;

Step 3: Preparation of 3d

3c (0.200 g, 0.52 mmol), intermediate 1 (0.18 g, 0.52 mmol) were dissolved in 5 mL DMF, and HOBT (0.14 g, 1.04 mmol), DIPEA (0.27 g, 2.05 mmol) and EDCI (0.20 g, 1.04 mmol) were then added, and the mixed solution was stirred at room temperature for 2 h. 30 ml of ethyl acetate were added, and the resulting solution was washed twice with water (20 ml x 2), dried over anhydrous sodium sulfate,and concentrated under reduced pressure, and the residue was subjected to column chromatography to afford the title compound 3d as a yellow oil (0.21 g, yield: 55.95%).
LCMS m/z = 722.90 $[M+H]^+$;

Step 4: Preparation of compounds 3-1 and 3-2

3d (0.18 g, 0.25 mmol) was dissolved in THF (3 mL) and water (1 mL), lithium hydroxide monohydrate (31.5 mg, 0.75 mmol) was added, and the resulting mixture was reacted at room temperature for 5 h. PH was adjusted to 5-6 with 1 N hydrochloric acid, and the resulting solution was concentrated under reduced pressure to afford crude compound 3, which was separated and purified by prep-HPLC (method 1) to afford compound 3-1 (25 mg, Rt=4.300 min, yield: 14%) and compound 3-2 (30 mg, Rt = 4.398 min, yield: 17%).

Compound 3-1:

LC-Ms m/z (ESI): 694.3 $[M+H]^+$
$^1$H NMR (400 MHz, Methanol-$d_4$) δ 9.16 (d, 1H), δ 7.89 (s, 1H), 7.19 (d, 1H), 6.87 - 6.73 (m, 4H), 5.80 - 5.69 (m, 1H), 5.40 - 5.29 (m, 1H), 4.54 - 4.33 (m, 2H), 4.29 - 4.12 (m, 2H), 3.79 - 3.70 (m, 1H), 3.49 - 3.35 (m, 2H), 2.98 - 2.90 (m, 3H), 2.88 - 2.76 (m, 4H), 2.44 - 2.37 (m, 2H), 2.04 - 1.93 (m, 4H), 1.89 (s, 3H), 1.82 (s, 3H), 1.52 - 1.39 (m, 1H), 1.01 - 0.92 (m, 6H).

Compound 3-2:

LC-Ms m/z (ESI): 694.3 $[M+H]^+$
$^1$H NMR (400 MHz, Methanol-$d_4$) δ 7.89 (s, 1H), 7.24 (d, 1H), 6.93 - 6.79 (m, 4H), 5.78 - 5.69 (m, 1H), 5.38 - 5.29 (m, 1H), 4.57 - 4.35 (m, 2H), 4.31 - 4.16 m, 2H), 3.85 - 3.69 (m, 1H), 3.54 - 3.35 (m, 2H), 3.07 - 2.94 (m, 3H), 2.94 - 2.77 (m, 4H), 2.49 - 2.41 (m, 2H), 2.08 - 1.98 (m, 2H), 1.94 (s, 6H), 1.85 - 1.76 (m, 2H), 1.39 - 1.27 (m, 1H), 0.93 - 0.81 (m, 6H).

**Example 4:** Preparation of compound 4

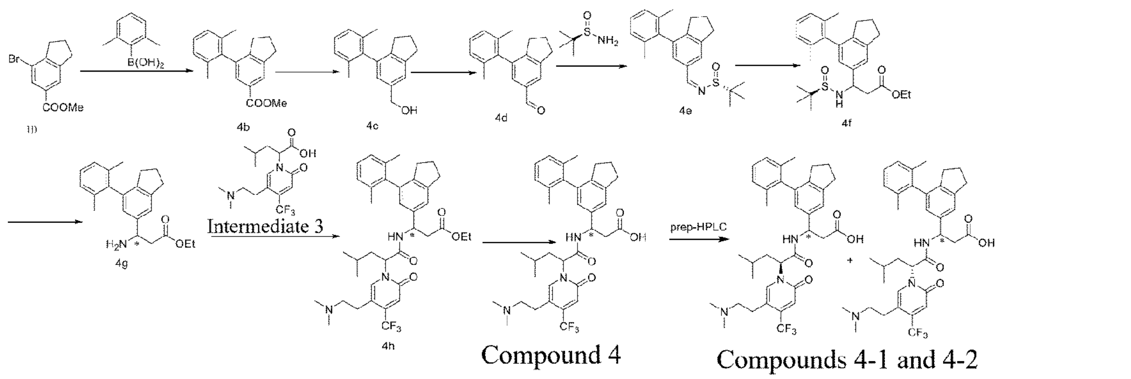


Step 1: Synthesis of 4b

Under nitrogen protection, 1D (1.8 g, 7.06 mmol), 2,6-dimethylphenylboronic acid (1.27 g, 8.47 mmol) and cesium carbonate (6.90 g, 21.18 mmol) were added to 1,4-dioxane (40.0 mL) and water (4.0 mL), followed by $Pd(PPh_3)_4$ (1.63 g, 1.41 mmol), and the mixture was reacted at 100°C in a sealed tube for 5 h. The reaction system was cooled to room temperature and then filtered through celite. Ethyl acetate (50 mL) was added and the mixture was successively washed with water (30 mL) and saturated sodium chloride aqueous solution (30 mL), dried over anhydrous sodium sulfate, filtered and concentrated under reduced pressure. The residue was subjected to flash chromatography on a silica gel column to afford 4b (1.4 g, yield: 70.73%).

Step 2: Synthesis of 4c

Under nitrogen protection, 4b (1.4 g, 4.99 mmol) was dissolved in dry THF (30 mL) and at 0°C, lithium aluminum hydride (380 mg, 10.03 mmol) was slowly added. The resulting mixture was reacted at room temperature for 1 h. 10%

sodium sulfate aqueous solution (20 mL) was added and the mixture was filtered. The solvent was removed under reduced pressure to afford 4c (800 mg, 67.27%).

Step 3: Synthesis of 4d

Under nitrogen protection, 4c (800 mg, 3.17 mmol) was dissolved in dry dichloromethane (20 mL), sodium bicarbonate (0.53 g, 6.31 mmol) and Dess-Martin periodinane (1.75 g, 4.13 mmol) were added, and the resulting mixture was reacted at room temperature for 1 h. Saturated sodium thiosulfate solution (10 mL) and saturated sodium bicarbonate solution (10 mL) were added and the mixture was extracted with dichloromethane (50 mL×3), dried over anhydrous sodium sulfate and filtered. The solvent was removed under reduced pressure and the residue was subjected to column chromatography on a silica gel column to afford 4d (650 mg, 81.91%).

Step 4: Synthesis of 4e

Under nitrogen protection, 4d (600 mg, 2.40 mmol) and R-tert-butylsulfinamide (0.35 g, 2.88 mmol) were dissolved in THF (20 mL), tetraethyl titanate (1.64 g, 7.22 mmol) was slowly added, and the resulting mixture was reacted at 45°C for 15 h and concentrated under reduced pressure to afford a crude, which was subjected to flash column chromatography on a silica gel column to afford 4e (800 mg, yield: 94.29%).
LC-Ms m/z (ESI): 354.5 $[M+H]^+$

Step 5: Synthesis of 4f

Zinc powder (1.81 g, 27.72 mmol) was added to dry THF (5 mL), the mixture was subjected to nitrogen replacement three times, CuCl (590 mg, 5.94 mmol) was added, and the resulting mixture was reacted at 60°C for 2 h. The reaction mixture was cooled to room temperature, ethyl bromoacetate (1.65 g, 9.9 mmol) was slowly added, and the resulting mixture was reacted at 60°C for 1 h and cooled to 0°C. A solution of 4e (700 mg, 1.98 mmol) in THF (1 mL) was added and the mixture was stirred at 0°C for 3 h. The reaction mixture was filtered through celite and saturated ammonium chloride solution (20 mL) was added. The resulting mixture was extracted with ethyl acetate (30 ml × 3), washed with saturated sodium chloride aqueous solution (20 mL), dried over anhydrous sodium sulfate, filtered and concentrated under reduced pressure. The residue was subjected to flash column chromatography to afford 4f (750 mg, yield: 85.77%).
LC-Ms m/z (ESI): 442.6 $[M+H]^+$

Step 6: Synthesis of 4g

4f (600 mg, 1.36 mmol) was dissolved in THF (2 mL), 4 N hydrogen chloride dioxane solution (2 mL) was added, and the resulting mixture was stirred at room temperature for 3 h. The reaction mixture was concentrated under reduced pressure to afford crude 4g.
LC-Ms m/z (ESI): 338.4 $[M+H]^+$

Step 7: Synthesis of compound 4h

Under nitrogen protection, intermediate 3 (260 mg, 0.74 mmol, referring to WO 2021076890 A1 for synthetic step) was dissolved in dry DMF (10 mL), and EDCI (280 mg, 1.46 mmol), HOBT (200 mg, 1.48 mmol) and DIPEA (290 mg, 2.24 mmol) were added. At room temperature, the reaction mixture was stirred for 40 min, and then crude 4g (250 mg, 0.74 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 4h (350 mg, yield: 70.83%).
LC-Ms m/z (ESI): 668.8 $[M+H]^+$

Step 8: Synthesis of compounds 4-1 and 4-2

4h (350 mg, 0.52 mmol) was dissolved in THF (6 mL) and water (2 mL), lithium hydroxide monohydrate (31 mg, 1.29 mmol) was added, and the resulting mixture was reacted at room temperature for 5 h. PH was adjusted to 5-6 with 1 N hydrochloric acid, and the resulting solution was concentrated under reduced pressure to afford crude compound 4, which was separated and purified by prep-HPLC (method 2) to afford the trifluoroacetate salts of compound 4-1 (16 mg, Rt=4.135 min, yield: 4.81%) and compound 4-2 (18 mg, Rt = 4.039 min, yield: 5.41%).

Trifluoroacetate salt of compound 4-1:

LC-Ms m/z (ESI): 640.7 [M+H]+
$^1$H NMR (400 MHz, Methanol-$d_4$) δ 9.15 (d, 1H), 7.92 (s, 1H), 7.22 (s, 1H), 7.15 - 7.05 (m, 3H), 6.88 (s, 1H), 6.84 - 6.79 (m, 1H), 5.74 (t, 1H), 5.35 - 5.28 (m, 1H), 3.66 - 3.27 (m, 2H), 3.05 - 2.91 (m, 10H), 2.90 - 2.77 (m, 2H), 2.49 - 2.40 (m, 2H), 2.07 - 1.98 (m, 2H), 1.93 (s, 6H), 1.86 - 1.78 (m, 2H), 1.39 - 1.28 (m, 1H), 0.93 - 0.81 (m, 6H).

Trifluoroacetate salt of compound 4-2:

LC-Ms m/z (ESI): 640.7 [M+H]+
$^1$H NMR (400 MHz, Methanol-$d_4$) δ 9.17 (d, 1H), 7.92 (s, 1H), 7.16 (d, 1H), 7.13 - 6.99 (m, 3H), 6.79 (s, 1H), 6.76 (d, 1H), 5.75 (t, 1H), 5.38 - 5.27 (m, 1H), 3.31 - 3.19 (m, 2H), 3.06 - 2.87 (m, 10H), 2.87 - 2.75 (m, 2H), 2.44 - 2.34 (m, 2H), 2.04 - 1.92 (m, 4H), 1.87 (s, 3H), 1.80 (s, 3H), 1.50 - 1.41 (m, 1H), 1.01 - 0.90 (m, 6H).

**Example 5:** Preparation of compound 5

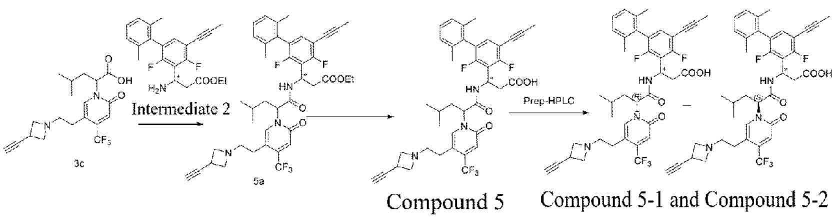


3c 5a Compound 5 Compound 5-1 and Compound 5-2

Referring to the preparation method of compound 3, crude compound 5 was obtained, which was separated and purified by prep-HPLC (method 1) to afford compound 5-1 (2 mg, Rt=4.184 min) and compound 5-2 (2 mg, Rt = 4.283 min).

Compound 5-1:

LC-Ms m/z (ESI): 710.3 [M+H]+
$^1$H NMR (400 MHz, Methanol-$d_4$) δ 7.85 (s, 1H), 7.17 (t, 1H), 7.12 - 7.02 (m, 3H), 6.83 (s, 1H), 5.83 - 5.76 (m, 1H), 5.73 - 5.67 (m, 1H), 5.36 - 5.32 (m, 1H), 4.46 - 4.37 (m, 1H), 4.26 - 4.15 (m, 2H), 3.79 - 3.72 (m, 1H), 3.49 - 3.38 (m, 2H), 3.15 - 3.08 (m, 1H), 2.95 - 2.93 (m, 1H), 2.89 - 2.81 (m, 2H), 2.19 (t, 1H), 2.09 - 2.00 (m, 4H), 1.99 - 1.91 (m, 4H), 1.89 (s, 3H), 1.65 - 1.55 (m, 1H), 1.00 - 0.91 (m, 6H).

Compound 5-2:

LC-Ms m/z (ESI): 710.3 [M+H]+
$^1$H NMR (400 MHz, Methanol-$d_4$) δ 7.88 (s, 1H), 7.23 - 7.03 (m, 4H), 6.89 (s, 1H), 5.84 - 5.77 (m, 1H), 5.71 (t, 1H), 5.34 (t, 1H), 4.51 - 4.37 (m, 1H), 4.27 - 4.16 (m, 1H), 3.82 - 3.71 (m, 1H), 3.52 - 3.39 (m, 3H), 3.15 - 3.03 (m, 2H), 2.97 - 2.83 (m, 3H), 2.24 - 2.14 (m, 2H), 2.10 - 1.95 (m, 7H), 1.92 - 1.78 (m, 2H), 1.66 - 1.53 (m, 1H), 0.95 - 0.79 (m, 6H).

**Example 6:** Preparation of compound 6

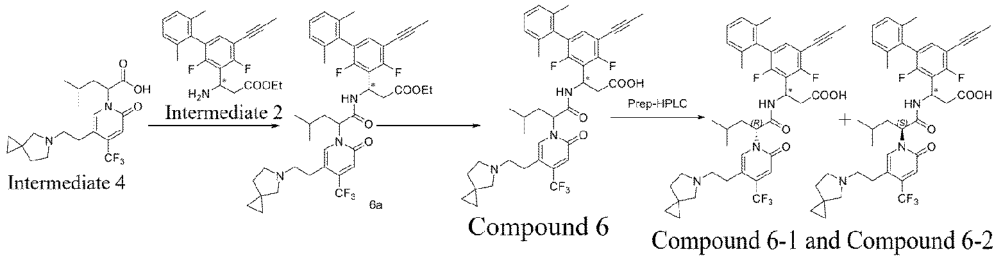

Intermediate 4
6a
Compound 6
Compound 6-1 and Compound 6-2

Referring to the preparation method of compound 3, crude compound 6 was obtained, which was separated and purified by prep-HPLC (method 1) to afford compound 6-1 (20 mg, Rt=4.255 min) and compound 6-2 (20 mg, Rt = 4.342 min).

Compound 6-1:

LC-Ms m/z (ESI): 726.3 $[M+H]^+$

$^1$H NMR (400 MHz, Methanol-$d_4$) δ 7.75 (s, 1H), 7.14 (t, 1H), 7.10 - 7.01 (m, 2H), 6.96 (t, 1H), 6.78 (s, 1H), 5.77 - 5.70 (m, 1H), 5.68 - 5.62 (m, 1H), 2.89 - 2.80 (m, 1H), 2.81 - 2.73 (m, 3H), 2.74 - 2.62 (m, 3H), 2.61 - 2.51 (m, 4H), 2.02 (s, 3H), 1.99 (s, 3H), 1.97 - 1.89 (m, 2H), 1.86 - 1.79 (m, 4H), 1.44 - 1.36 (m, 1H), 0.98 - 0.91 (m, 6H), 0.62 - 0.52 (m, 4H).

Compound 6-2:

LC-Ms m/z (ESI): 726.3 $[M+H]^+$

$^1$H NMR (400 MHz, Methanol-$d_4$) δ 8.51 (s, 1H), 7.83 (s, 1H), 7.20 - 7.15 (m, 1H), 7.13 - 7.05 (m, 3H), 6.90 (s, 1H), 5.84 - 5.75 (m, 1H), 5.64 (t, 1H), 3.56 - 3.47 (m, 2H), 3.44 - 3.37 (m, 1H), 3.25 (s, 2H), 3.03 - 2.94 (m, 2H), 2.88 - 2.78 (m, 1H), 2.60 - 2.51 (m, 1H), 2.09 - 1.97 (m, 11H), 1.94 -1.84 (m, 1H), 1.77 - 1.63 (m, 1H), 1.40 - 1.32 (m, 1H), 0.94 - 0.85 (m, 6H), 0.85 - 0.70 (m, 4H).

**Example 7:** Preparation of compound 7

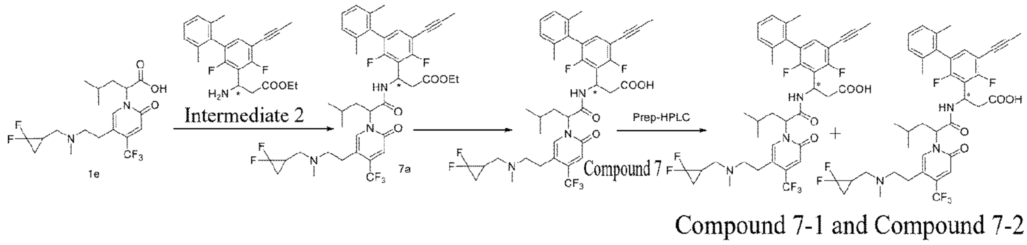

1e
7a
Compound 7
Compound 7-1 and Compound 7-2

Referring to the preparation method of compound 1, crude compound 7 was obtained, which was separated and purified by prep-HPLC (method 1) to afford compound 7-1 (20 mg, Rt=4.323 min) and compound 7-2 (20 mg, Rt = 4.240 min).

Compound 7-1:

LC-Ms m/z (ESI): 750.3 $[M+H]^+$

$^1$H NMR (400 MHz, Methanol-$d_4$) δ 7.85 (d, 1H), 7.20 - 7.13 (m, 1H), 7.12 - 7.01 (m, 3H), 6.80 (s, 1H), 5.77 (t, 1H), 5.65 (t, 1H), 3.34 (s, 3H), 2.88 - 2.55 (m, 6H), 2.49 - 2.38 (m, 1H), 2.36 - 2.30 (m, 2H), 2.10 - 1.94 (m, 9H), 1.82 - 1.75 (m, 2H), 1.55 - 1.44 (m, 1H), 1.32 - 1.23 (m, 1H), 1.10 - 1.01 (m, 1H), 0.93 - 0.80 (m, 6H).

Compound 7-2:

LC-Ms m/z (ESI): 750.3 [M+H]+
$^1$H NMR (400 MHz, Methanol-$d_4$) δ 7.75 (d, 1H), 7.14 (t, 1H), 7.09 - 7.01 (m, 2H), 6.96 (t, 1H), 6.78 (s, 1H), 5.74 (t, 1H), 5.69 - 5.60 (m, 1H), 3.34 (s, 3H), 2.91 - 2.82 (m, 1H), 2.74 - 2.51 (m, 5H), 2.48 - 2.34 (m, 1H), 2.35 - 2.28 (m, 2H), 2.02 (s, 3H), 1.98 (s, 3H), 1.93 (t, 1H), 1.84 (s, 3H), 1.77 - 1.68 (m, 1H), 1.53 - 1.35 (m, 2H), 1.09 - 0.99 (m, 1H), 0.98 - 0.85 (m, 6H).

**Example 8:** Preparation of compound 8

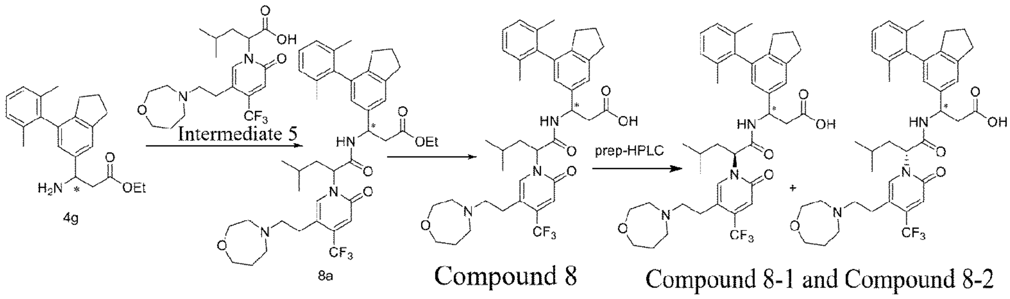


Referring to the preparation method of compound 4, crude compound 8 was obtained, which was separated and purified by prep-HPLC (method 2) to afford the trifluoroacetate salt of compound 8-1 (25 mg, Rt=4.269 min, yield: 6.53%) and the trifluoroacetate salt of compound 8-2 (28 mg, Rt = 4.171 min, yield: 7.32%).

Trifluoroacetate salt of compound 8-1:

LC-Ms m/z (ESI): 696.7 [M+H]+
$^1$H NMR (400 MHz, Methanol-$d_4$) δ 7.90 (s, 1H), 7.21 (s, 1H), 7.15 - 7.02 (m, 3H), 6.87 (s, 1H), 6.83 (d, 1H), 5.74 - 5.66 (m, 1H), 5.34 (t, 1H), 3.93 - 3.87 (m, 2H), 3.83 (t, 2H), 3.49 - 3.42 (m, 2H), 3.42 - 3.37 (m, 2H), 3.35 - 3.30 (m, 2H), 3.05 - 2.90 (m, 4H), 2.80 - 2.74 (m, 2H), 2.45 (t, 2H), 2.19 - 2.09 (m, 2H), 2.08 - 1.96 (m, 2H), 1.93 (s, 6H), 1.89 - 1.74 (m, 2H), 1.39 - 1.28 (m, 1H), 0.92 - 0.84 (m, 6H).

Trifluoroacetate salt of compound 8-2:

LC-Ms m/z (ESI): 696.7 [M+H]+
$^1$H NMR (400 MHz, Methanol-$d_4$) δ 7.92 (s, 1H), 7.16 (s, 1H), 7.13 - 6.99 (m, 3H), 6.81 - 6.72 (m, 2H), 5.78 - 5.70 (m, 1H), 5.37 - 5.28 (m, 1H), 3.95 - 3.87 (m, 2H), 3.86 - 3.77 (m, 2H), 3.48 - 3.42 (m, 2H), 3.42 - 3.37 (m, 2H), 3.30 - 3.20 (m, 2H), 3.06 - 2.88 (m, 4H), 2.87 - 2.77 (m, 2H), 2.43 - 2.36 (m, 2H), 2.17 - 2.09 (m, 2H), 2.03 - 1.91 (m, 4H), 1.87 (s, 3H), 1.80 (s, 3H), 1.51 - 1.39 (m, 1H), 0.99 - 0.91 (m, 6H).

**Example 9:** Preparation of compound 9

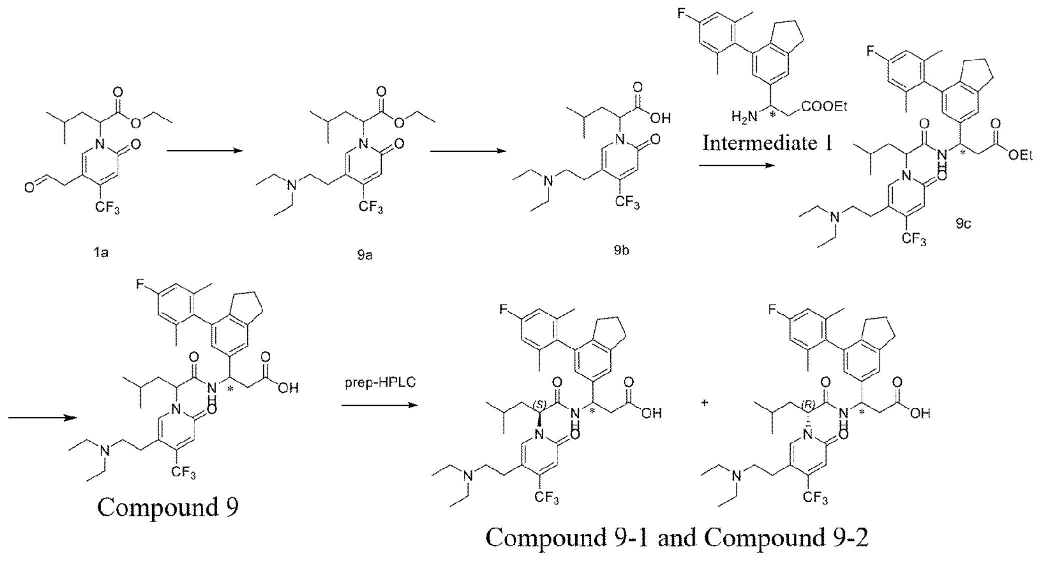


1a 9a 9b 9c

Compound 9

Compound 9-1 and Compound 9-2

Step 1: Synthesis of 9a

1a (3.0 g, 8.64 mmol) was dissolved in 30 mL of ultra-dry DCE, and diethylamine (0.95 g, 12.96 mmol) and AcOH (0.10 mg, 1.73 mmol) were successively added. The reaction was carried out for 1h at room temperature, sodium triacetoxyborohydride (3.66 g, 17.28 mmol) was added and the reaction was continued for 16 h. Ethyl acetate (200 mL) and saturated $NaHCO_3$ aqueous solution (100 mL) were added for separation by extraction, and the aqueous phase was extracted again with ethyl acetate (100 mL×1). The ethyl acetate layers were combined, and the ethyl acetate layers were washed with saturated sodium chloride aqueous solution (100 mL×1), dried over anhydrous sodium sulfate, and concentrated under reduced pressure. The residue was purified by column chromatography to afford 9a (2.8 g, yield: 80.12%).
LC-Ms m/z (ESI): 405.3 $[M+H]^+$

Step 2: Synthesis of 9b

9a (2.8 g, 6.92 mmol) was dissolved in THF (30 mL) and water (10 mL), lithium hydroxide (331.55 mg, 13.85 mmol) was added, and the reaction was continued for 30 min. The reaction solution was purified by reverse phase column chromatography to afford 9b (1.2 g, yield: 46.05%).
LC-Ms m/z (ESI): 377.2 $[M+H]^+$

Step 3: Synthesis of 9c

Under nitrogen protection, intermediate 1 (300.0 mg, 0.84 mmol) was dissolved in dry DMF (5 mL), and HOBt (227.00 mg, 1.68 mmol), EDCI (322.06 mg, 1.68 mmol) and DIPEA (434.25 mg, 3.36 mmol) were successively added. At room temperature, the reaction mixture was stirred for 40 min, and then compound 9b (316.18 mg, 0.84 mmol) was added. The resulting mixture was reacted at room temperature overnight. Ethyl acetate (100 mL) was added and the resulting mixture was successively washed with water (40 mL×2) and saturated sodium chloride aqueous solution (40 mL×1), dried over anhydrous sodium sulfate and filtered and the filtrate was concentrated under reduced pressure and subjected to column chromatography on a silica gel column to afford 9c (400.0 mg, yield: 66.71%).
LC-Ms m/z (ESI): 714.4 $[M+H]^+$

Step 4: Synthesis of compound 9-1 and compound 9-2

Compound 9d (400.0 mg, 0.56 mmol) was dissolved in methanol (3 mL) and water (1 mL), lithium hydroxide monohydrate (27.0 mg, 1.12 mmol) was added, and the resulting mixture was reacted at room temperature for 30 min. PH was adjusted to 5-6 with 1 N hydrochloric acid and concentrated under reduced pressure to afford crude compound 9, which was separated and purified by prep-HPLC (Method 2) to afford the trifluoroacetate salt of compound 9-1 (76.0 mg, yield: 8.36%) and the trifluoroacetate salt of compound 9-2 (81.0 mg, yield: 6.27%).

Trifluoroacetate salt of compound 9-1:

LC-Ms m/z (ESI): 686.2 [M+H]+

$^1$H NMR (400 MHz, $CD_3OD$) δ 7.91 (s, 1H), 7.19 (s, 1H), 6.82 - 6.73 (m, 4H), 5.74 - 5.66 (m, 1H), 5.35 - 5.27 (m, 1H), 3.21 - 3.06 (m, 6H), 3.00 - 2.85 (m, 4H), 2.73 - 2.63 (m, 2H), 2.43 - 2.35 (m, 2H), 2.02 - 1.92 (m, 4H), 1.89 (s, 3H), 1.82 (s, 3H), 1.48 - 1.36 (m, 1H), 1.26 (t, 6H), 0.99 - 0.90 (m, 6H).

Trifluoroacetate salt of compound 9-2:

LC-Ms m/z (ESI): 686.2 [M+H]+

$^1$H NMR (400 MHz, $CD_3OD$) δ 7.86 (s, 1H), 7.22 (s, 1H), 6.90 - 6.77 (m, 4H), 5.66 - 5.57 (m, 1H), 5.41 - 5.33 (m, 1H), 3.30 - 3.17 (m, 6H), 3.03 - 2.90 (m, 4H), 2.68 - 2.53 (m, 2H), 2.48 - 2.38 (m, 2H), 2.07 - 1.89 (m, 9H), 1.78 - 1.67 (m, 1H), 1.43 - 1.34 (m, 1H), 1.29 (t, 6H), 0.92 - 0.82 (m, 6H).

**Example 10:** Preparation of compound 10

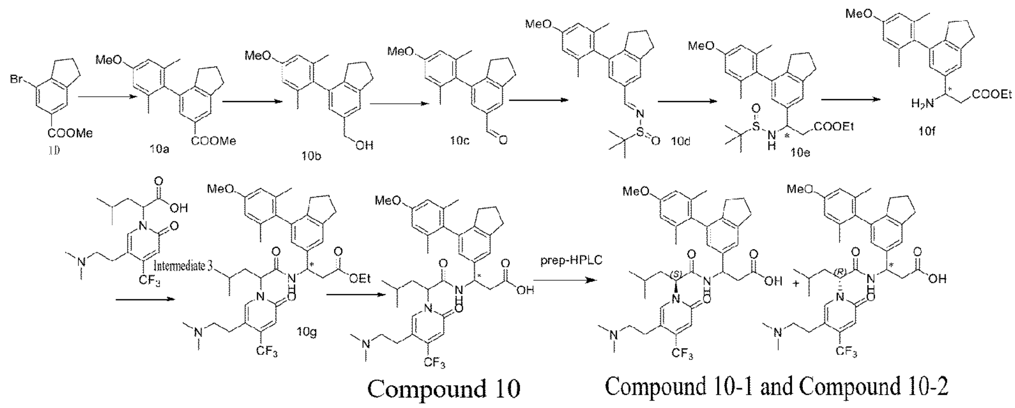


Compound 10

Compound 10-1 and Compound 10-2

Step 1: Synthesis of 10a

Under nitrogen protection, 1D (2.0 g, 7.84 mmol), 2.6-dimethyl-4-methoxy phenylboronic acid (2.82 g, 15.68 mmol) and cesium carbonate (7.66 g, 23.52 mmol) were added to 1,4-dioxane (30.0 mL) and water (3.0 mL), followed by $Pd(PPh_3)_4$ (0.29 g, 0.78 mmol), and the mixture was reacted at 100°C in a sealed tube for 12 h. The reaction system was cooled to room temperature and then filtered through celite. Ethyl acetate (50 mL) was added and the mixture was successively washed with water (30 mL) and saturated sodium chloride aqueous solution (30 mL), dried over anhydrous sodium sulfate, filtered and concentrated under reduced pressure. The residue was subjected to flash chromatography on a silica gel column to afford 10a (1.6 g, yield: 65.75%).

Step 2: Synthesis of 10b

Under nitrogen protection, 10a (1.6 g, 5.15 mmol) was dissolved in dry THF (10 mL) and at 0°C, lithium aluminum hydride (8 mL) was slowly added. The resulting mixture was reacted at room temperature for 1 h. 10% sodium sulfate aqueous solution (20 mL) was added and the mixture was filtered. The solvent was removed under reduced pressure and the residue was subjected to flash chromatography on a silica gel column to afford 10b (1.0 g, yield: 68.76%).

Step 3: Synthesis of 10c

Under nitrogen protection, the compound 10b (1.0 g, 3.54 mmol) was dissolved in dry dichloromethane (10 mL), sodium bicarbonate (0.6 g, 7.08 mmol) and Dess-Martin periodinane (2.25 g, 5.31 mmol) were added at room temperature, and the resulting mixture was reacted at room temperature for 1 h. Saturated sodium thiosulfate solution (10 mL) and

saturated sodium bicarbonate solution (10 mL) were added and the mixture was extracted with dichloromethane (50 mL×3), dried over anhydrous sodium sulfate and filtered. The solvent was removed under reduced pressure and the residue was subjected to column chromatography on a silica gel column to afford 10c (0.8 g, yield: 80.58%).

Step 4: Synthesis of 10d

Under nitrogen protection, 10c (1.4 g, 4.99 mmol) and R-tert-butylsulfinamide (0.82 g, 5.99 mmol) were dissolved in THF (15 mL), tetraethyl titanate (1.71 g, 7.50 mmol) was slowly added, and the resulting mixture was reacted at 45°C for 15 h and concentrated under reduced pressure to afford a crude, which was subjected to flash column chromatography on a silica gel column to afford 10d (1.9 g, yield: 99.27%).

Step 5: Synthesis of 10e

Zinc powder (4.77 g, 72.94 mmol) was added to dry THF (5 mL), the mixture was subjected to nitrogen replacement three times, CuCl (1.55 g, 15.63 mmol) was added, and the resulting mixture was reacted at 60°C for 2 h. The reaction mixture was cooled to room temperature, ethyl bromoacetate (4.35 g, 26.05 mmol) was slowly added, and the resulting mixture was reacted at 60°C for 1 h and cooled to 0°C. A solution of 10d (2.0 g, 5.21 mmol) in THF (1 mL) was added and the mixture was stirred at 0°C for 3 h. The reaction mixture was filtered through celite and saturated ammonium chloride solution (80 mL) was added. The resulting mixture was extracted with ethyl acetate (100 ml × 3), washed with saturated sodium chloride aqueous solution (80 mL), dried over anhydrous sodium sulfate, filtered and concentrated under reduced pressure. The residue was subjected to flash column chromatography to afford 10e (2.0 g, yield: 81.39%).

Step 6: Synthesis of 10f

10e (500.0 mg, 1.06 mmol) was dissolved in THF (5 mL), 4 N hydrogen chloride dioxane solution (5 mL) was added, and the resulting mixture was stirred at room temperature for 3 h. The reaction mixture was concentrated under reduced pressure to afford crude 10f.
LC-Ms m/z (ESI): 368.0 $[M+H]^+$

Step 7: Synthesis of 10g

Under nitrogen protection, intermediate 3 (189.6 mg, 0.54 mmol) was dissolved in dry DMF (1.5 mL), and HOBt (147.08 mg, 1.090 mmol), EDCI (168.98 mg, 1.09 mmol) and DIPEA (220.3 mg, 2.18 mmol) were successively added. At room temperature, the reaction mixture was stirred for 40 min, and then crude 10f (200.0 mg, 0.54 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 10g (180.0 mg, yield: 47.39%).
LC-Ms m/z (ESI): 698.3 $[M+H]^+$

Step 8: Synthesis of compound 10-1 and compound 10-2

10g (180.0 mg, 0.26 mmol) was dissolved in THF (3 mL) and water (1 mL), lithium hydroxide monohydrate (12.45 mg, 0.52 mmol) was added, and the resulting mixture was reacted at room temperature for 5 h. PH was adjusted to 5-6 with 1 N hydrochloric acid and concentrated under reduced pressure to afford crude compound 10, which was separated and purified by prep-HPLC (Method 2) to afford the trifluoroacetate salt of compound 10-1 (17 mg, yield: 9.76%) and the trifluoroacetate salt of compound 10-2 (15 mg, yield: 8.61%).

Trifluoroacetate salt of compound 10-1:

LC-Ms m/z (ESI): 670.3 $[M+H]^+$
$^1$H NMR (400 MHz, $CD_3OD$) δ 7.92 (s, 1H), 7.14 (s, 1H), 6.80 (s, 1H), 6.74 (s, 1H), 6.67 - 6.56 (m, 2H), 5.79 - 5.70 (m, 1H), 5.37 - 5.27 (m, 1H), 3.77 (s, 3H), 3.29 - 3.20 (m, 2H), 3.05 - 2.88 (m, 10H), 2.87 - 2.76 (m, 2H), 2.46 - 2.35 (m, 2H), 2.04 - 1.89 (m, 4H), 1.85 (s, 3H), 1.78 (s, 3H), 1.51 - 1.39 (m, 1H), 1.02 - 0.91 (m, 6H).

Trifluoroacetate salt of compound 10-2:

LC-Ms m/z (ESI): 670.3 [M+H]$^+$
$^1$H NMR (400 MHz, $CD_3OD$) δ 7.92 (s, 1H), 7.20 (s, 1H), 6.88 (s, 1H), 6.82 (s, 1H), 6.66 (s, 2H), 5.78 - 5.69 (m, 1H), 5.37 - 5.24 (m, 1H), 3.78 (s, 3H), 3.35 - 3.24 (m, 2H), 3.05 - 2.92 (m, 10H), 2.89 - 2.76 (m, 2H), 2.50 - 2.42 (m, 2H), 2.09 - 1.96 (m, 2H), 1.91 (s, 6H), 1.86 - 1.77 (m, 2H), 1.39 - 1.25 (m, 1H), 0.95 - 0.81 (m, 6H).

**Example 11:** Preparation of compound 11

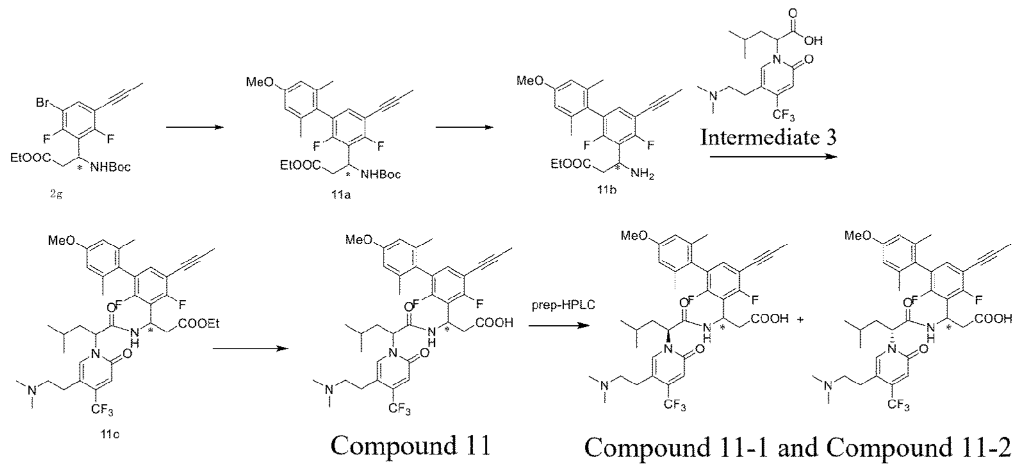


2g 11a 11b 11c

Compound 11

Compound 11-1 and Compound 11-2

Step 1: Synthesis of 11a

Under nitrogen protection, 2g (300.0 mg, 0.67 mmol), 2.6-dimethyl-4-methoxy phenylboronic acid (242.0 mg, 1.34 mmol) and cesium carbonate (0.65 g, 2.01 mmol) were added to 1,4-dioxane (5.0 mL) and water (0.5 mL), followed by $Pd(PPh_3)_4$ (77.0 mg, 0.067 mmol), and the mixture was reacted at 100°C in a sealed tube for 12 h. The reaction system was cooled to room temperature and then filtered through celite. Ethyl acetate (50 mL) was added and the mixture was successively washed with water (30 mL) and saturated sodium chloride aqueous solution (30 mL), dried over anhydrous sodium sulfate, filtered and concentrated under reduced pressure. The residue was subjected to flash chromatography on a silica gel column to afford 11a (300.0 mg, yield: 92.22%).

Step 2: Synthesis of 11b

11a (300.0 mg, 0.60 mmol) was dissolved in acetonitrile (3 mL), hydrogen chloride-1,4-dioxane solution (2 mL) was added, and the resulting mixture was stirred at room temperature for 30 min. The solvent was removed by drying under reduced pressure to afford crude 11b.
LC-Ms m/z (ESI):402.7 [M+H]$^+$

Step 3: Synthesis of 11c

Under nitrogen protection, intermediate 3 (174.18 mg, 0.50 mmol) was dissolved in dry DMF (5 mL), and HOBt (135.12 mg, 1.00 mmol), EDCI (191.70 mg, 1.00 mmol) and DIPEA (258.48 mg, 2.00 mmol) were successively added. At room temperature, the reaction mixture was stirred for 40 min, and then crude 11b (200.0 mg, 0.50 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 11c (300.0 mg, yield: 81.99%).
LC-Ms m/z (ESI): 732.3 [M+H]$^+$

Step 4: Synthesis of compound 11-1 and compound 11-2

11c (300.0 mg, 0.41 mmol) was dissolved in THF (3 mL) and water (1 mL), lithium hydroxide monohydrate (19.46 mg, 0.82 mmol) was added, and the resulting mixture was reacted at room temperature for 5 h. PH was adjusted to 5-6 with 1 N hydrochloric acid, and the resulting solution was concentrated under reduced pressure to afford crude compound 11, which was separated and purified by prep-HPLC (method 2) to afford the trifluoroacetate salt of compound 11-1 (23 mg, Rt=4.024 min, yield: 7.97%) and the trifluoroacetate salt of compound 11-2 (27 mg, Rt = 4.165 min, yield: 9.36%).

Trifluoroacetate salt of compound 11-1:

LC-Ms m/z (ESI):704.3 $[M+H]^+$
$^1H$ NMR (400 MHz, $CD_3OD$) δ 7.87 (s, 1H), 7.03 (t, 1H), 6.84 (s, 1H), 6.69 - 6.63 (m, 2H), 5.76 - 5.62 (m, 2H), 3.78 (s, 3H), 3.24 - 3.14 (m, 2H), 3.03 - 2.89 (m, 3H), 2.85 (s, 6H), 2.81 - 2.70 (m, 1H), 2.03 (s, 3H), 2.01 - 1.92 (m, 5H), 1.87 (s, 3H), 1.46 - 1.32 (m, 1H), 0.99 - 0.88 (m, 6H).

Trifluoroacetate salt of compound 11-1:

LC-Ms m/z (ESI):704.3 $[M+H]^+$
$^1H$ NMR (400 MHz, $CD_3OD$) δ 7.83 (s, 1H), 7.08 (t, 1H), 6.90 (s, 1H), 6.69 (s, 2H), 5.81 - 5.74 (m, 1H), 5.70 - 5.61 (m, 1H), 3.79 (s, 3H), 3.30 - 3.17 (m, 2H), 3.05 - 2.95 (m, 2H), 2.91 - 2.78 (m, 7H), 2.69 - 2.58 (m, 1H), 2.06 (s, 3H), 2.02 - 1.96 (m, 6H), 1.94 - 1.82 (m, 1H), 1.81 - 1.70 (m, 1H), 1.41 - 1.25 (m, 1H), 0.94 - 0.82 (m, 6H).

**Example 12:** Synthesis of compound 12

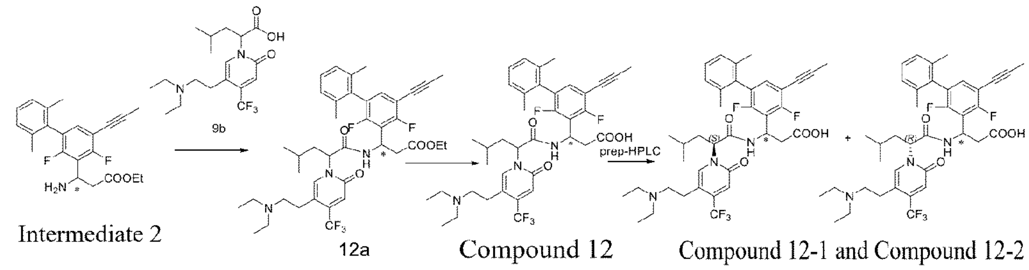

Intermediate 2 12a Compound 12 Compound 12-1 and Compound 12-2

Referring to the preparation method of compound 9, crude compound 12 was obtained, which was separated and purified by prep-HPLC (method 2) to afford the trifluoroacetate salt of compound 12-1 (11 mg, Rt=4.191 min) and the trifluoroacetate salt of compound 12-2 (17 mg, Rt = 4.332 min).

Trifluoroacetate salt of compound 12-1:

LC-Ms m/z (ESI):702.3 $[M+H]^+$
$^1H$ NMR (400 MHz, $CD_3OD$) δ 7.88 (s, 1H), 7.19 - 7.11 (m, 1H), 7.10 - 6.98 (m, 3H), 6.84 (s, 1H), 5.77 - 5.64 (m, 2H), 3.22 - 3.10 (m, 6H), 3.03 - 2.87 (m, 3H), 2.72 - 2.63 (m, 1H), 2.03 (s, 3H), 2.00 - 1.93 (m, 5H), 1.89 (s, 3H), 1.46 - 1.34 (m, 1H), 1.27 (t, 6H), 0.98 - 0.87 (m, 6H).

Trifluoroacetate salt of compound 12-2:

LC-Ms m/z (ESI):702.3 $[M+H]^+$

$^1$H NMR (400 MHz, $CD_3OD$) δ 7.89 - 7.83 (m, 1H), 7.20 - 7.14 (m, 1H), 7.13 - 7.02 (m, 3H), 6.89 (s, 1H), 5.80 - 5.72 (m, 1H), 5.69 - 5.62 (m, 1H), 3.29 - 3.18 (m, 6H), 3.01- 2.92 (m, 2H), 2.90 - 2.80 (m, 1H), 2.68 - 2.57 (m, 1H), 2.06 (s, 3H), 2.03 - 1.98 (m, 6H), 1.94 - 1.83 (m, 1H), 1.78 - 1.67 (m, 1H), 1.39 - 1.24 (m, 7H), 0.93 - 0.82 (m, 6H).

**Example 13:** Preparation of compound 13

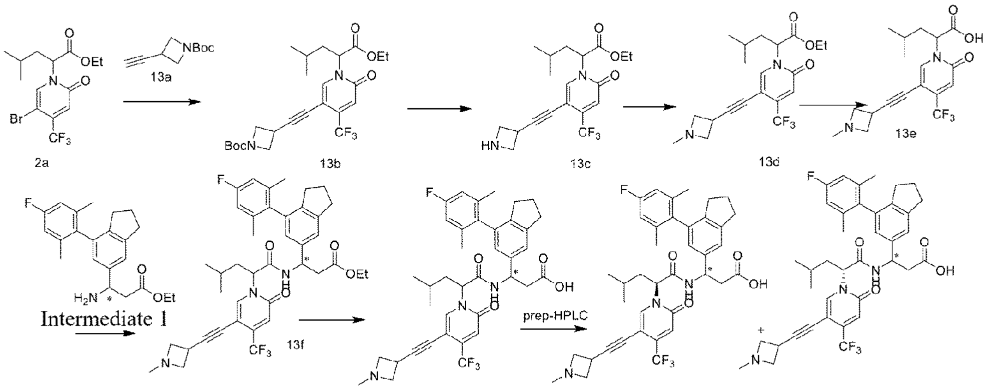


2a 13b 13c 13d 13e 13f

Compound 13

Compound 13-1 and Compound 13-2

Step 1: Preparation of 13b

2a (2.0 g, 5.2 mmol), 13a (1.9 g, 10.4 mmol), triethylamine (1.4 mL, 10.4 mmol), cuprous iodide (4.0 g, 20.8 mmol), anhydrous lithium chloride (1.76 g, 41.6 mmol) and $(PPh_3)_2PdCl_2$(730 mg, 1.04 mmol) were placed in a sealed tube, and DMF (30 mL) and water (3 mL) were added. The mixture was stirred at 80°C under nitrogen protection for 3 h. The reaction solution was cooled to room temperature. 100 mL of ethyl acetate was added for dilution, and the mixed solution was successively washed with water (50 mL×3) and saturated sodium chloride aqueous solution (50 mL×1). The organic layer was dried over anhydrous sodium sulfate and filtered, and the filtrate was concentrated under reduced pressure, and the residue was subjected to column chromatography to afford 13b (1.86 g, yield: 74%).
LCMS m/z = 485.2 $[M+H]^+$

Step 2: Preparation of 13c

13b (1.86 g, 3.84 mmol) was dissolved in 20 mL of dichloromethane, and 5 mL of trifluoroacetic acid was added. The mixture was stirred at room temperature for 3 h. The reaction mixture was concentrated under reduced pressure to afford crude 13c (1.8 g).
LCMS m/z = 385.2 $[M+H]^+$

Step 3: Preparation of 13d

The crude 13c (1.8 g), aqueous carbaldehyde (5 mL) and glacial acetic acid (230 mg, 3.84 mmol) were dissolved in methanol (20 mL), stirred at room temperature under nitrogen protection for 30 min, and sodium triacetoxyborohydride (1.62 g, 7.68 mmol) was added. The stirring was continued overnight at room temperature. The solvent was removed by concentration under reduced pressure, 30 mL of water was added, and the aqueous phase was extracted with ethyl acetate (50 mL × 2), and the organic phases were combined. The organic phase was washed with saturated sodium chloride aqueous solution (30 mL x 1), the organic layer was dried over anhydrous sodium sulfate and filtered, and the filtrate was concentrated under reduced pressure. The residue was subjected to column chromatography to afford 13d (0.91 g, two-step yield: 60%).
LCMS m/z = 399.2 $[M+H]^+$

Step 4: Preparation of 13e

13d (0.91 g, 2.29 mmol) was dissolved in ethanol (15 mL) and water (1.5 mL), lithium hydroxide monohydrate (288 mg, 6.86 mmol) was added, and the reaction was carried out at room temperature for 5 h. The solvent was removed by concentration under reduced pressure, 20 mL of water was added to the residue, and the pH was adjusted to around 2-3

with 1N hydrochloric acid. The aqueous phase was extracted with ethyl acetate (50 mL × 2), and the organic phases are combined. The organic phase was washed with saturated sodium chloride aqueous solution (30 mL x 1), the organic layer was dried over anhydrous sodium sulfate and filtered, and the filtrate was concentrated under reduced pressure. The residue was subjected to reverse phase (0.1% trifluoroacetic acid) preparative chromatography to afford 13e (200 mg, yield: 24%).
LCMS m/z = 371.2 $[M+H]^+$;

Step 5: Preparation of 13f

13e (0.160 g, 0.43 mmol) and intermediate 1 (0.15 g, 0.43 mmol) were dissolved in 5 mL DMF. HOBT (0.12 g, 0.86 mmol), DIPEA (0.22 g, 1.72 mmol) and EDCI (0.16 g, 0.86 mmol) were added, and stirred at room temperature for 2h. After the reaction was complete, 30 ml of ethyl acetate was added, and the reaction solution was washed with water twice (20 mL ×2), dried over anhydrous sodium sulfate, concentrated under reduced pressure, and subjected to column chromatography to afford 13f (0.13 g, yield: 42.71%).
LCMS m/z = 708.9 $[M+H]^+$;

Step 6: Preparation of compounds 13-1 and 13-2

13f (0.13 g, 0.18 mmol) was dissolved in THF (3 mL) and water (1 mL), lithium hydroxide monohydrate (23 mg, 0.55 mmol) was added, and the resulting mixture was reacted at room temperature for 5 h. PH was adjusted to 5-6 with 1 N hydrochloric acid, and the resulting solution was concentrated under reduced pressure to afford crude compound 13, which was separated and purified by prep-HPLC (method 1) to afford compound 13-1 (0.55 mg, Rt=4.271 min) and compound 13-2 (0.6 mg, Rt = 4.358 min).

Compound 13-1:

LC-Ms m/z (ESI): 680.2 $[M+H]^+$
$^1$H NMR (400 MHz, Methanol-$d_4$) δ 8.03 (s, 1H), 7.15 (s, 1H), 6.84 - 6.70 (m, 4H), 5.72 - 5.65 (m, 1H), 5.31 (t, 1H), 3.93 - 3.81 (m, 1H), 3.58 - 3.48 (m, 2H), 3.15 - 3.12 (m, 1H), 2.95 - 2.85 (m, 2H), 2.70 (d, 2H), 2.52 (s, 3H), 2.42 -2.35 (m, 2H), 2.03 - 1.91 (m, 4H), 1.89 (s, 3H), 1.79 (s, 3H), 1.48 - 1.29 (m, 2H), 1.00 - 0.90 (m, 6H)

Compound 13-2:

LC-Ms m/z (ESI): 680.2$[M+H]^+$
$^1$H NMR (400 MHz, Methanol-$d_4$) δ 8.08 (s, 1H), 7.23 (s, 1H), 6.86 - 6.78 (m, 4H), 5.69 - 5.58 (m, 1H), 5.40 - 5.29 (m, 1H), 3.96 - 3.87 (m, 1H), 3.70 -3.47 (m, 2H), 3.14 - 3.10 (m, 1H), 2.99 - 2.91 (m, 2H), 2.72 - 2.64 (m, 2H), 2.55 (s, 3H), 2.48 - 2.39 (m, 2H), 2.22 - 2.14 (m, 1H), 2.06 - 1.98 (m, 3H), 1.96 - 1.90 (m, 6H), 1.77 - 1.69 (m, 1H), 1.66 - 1.55 (m, 1H), 0.91 - 0.83 (m, 6H).

**Example 14:** Preparation of compound 14

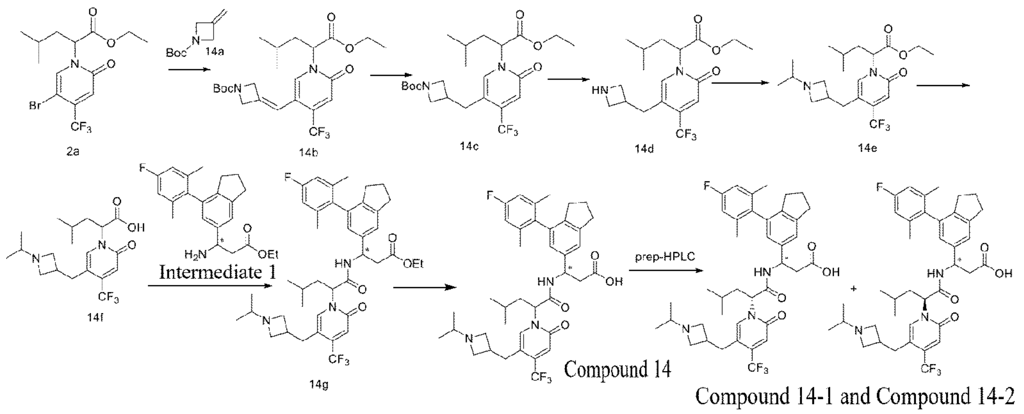


Step 1: Synthesis of 14b

Under nitrogen protection, 2a (20.0 g, 52.04 mmol) was dissolved in DMF (60 mL). 14a (10.56 g, 62.44 mmol), palladium acetate (3.32 g, 10.4 mmol), tris(2-methylphenyl)phosphine (6.32 g, 20.8 mmol) and triethylamine (15.8 g, 156.12 mmol) were successively added, and the resulting mixture was reacted at 100°C for 24 h. Extraction was performed by addition of ethyl acetate (180 mL) and the resulting mixture was washed with water (50 mL×2) and saturated sodium chloride aqueous solution (50 mL×1), dried over anhydrous sodium sulfate and filtered and the filtrate was concentrated under reduced pressure and subjected to column chromatography on a silica gel column to afford 14b (4.0 g, yield: 16.27%).
LC-Ms m/z (ESI): 473.5 $[M+H]^+$

Step 2: Synthesis of 14c

14b (4.0 g, 8.46 mmol) was dissolved in methanol (10 mL), Pd/C(2.0 g) was added, and the mixed solution was reacted under hydrogen atmosphere at room temperature for 0.5 h. The solid was removed by filtration and the filtrate was concentrated and purified by column chromatography to afford 14c (3.6 g, yield: 89.68%).
LC-Ms m/z (ESI): 475.5 $[M+H]^+$

Step 3: Synthesis of 14d

14c (3.6 g, 7.59 mmol) was dissolved in dichloromethane (5 mL), hydrogen chloride-1,4-dioxane (3 mL) was added, and the reaction was carried out for 1 h. The solvent was removed under reduced pressure to afford the hydrochloride salt of crude 14d.

Step 4: Synthesis of 14e

The hydrochloride salt of 14d (1.3 g, 3.47 mmol) was dissolved in ultra-dry 1,2-dichloroethane (30 mL), acetone (400 mg, 6.87 mmol) and glacial acetic acid (21.0 mg, 0.35 mmol) were successively added, and the reaction was carried out at 60 °C for 2 h. The reaction liquid was cooled to room temperature, and sodium triacetoxyborohydride (1.84 g, 8.68 mmol) was then added. The resulting mixture was reacted at room temperature overnight. Extraction was performed by addition of ethyl acetate (20 mL) and the resulting mixture was washed with water (30 mL×2) and saturated sodium chloride aqueous solution (30 mL×1), dried over anhydrous sodium sulfate and filtered and the filtrate was concentrated under reduced pressure and subjected to column chromatography on a silica gel column to afford 14e (1.0 g, yield: 69.20%).
LC-Ms m/z (ESI): 417.4 $[M+H]^+$

Step 5: Synthesis of 14f

14e (1.0 g, 2.40 mmol) was dissolved in THF (9 mL) and water (3 mL), lithium hydroxide (170 mg, 7.10 mmol) was added, and the reaction was carried out at room temperature for 2 h. The reaction solution was purified by reverse phase column chromatography to afford 14f (800 mg, yield: 85.82%).
LC-Ms m/z (ESI): 389.4 $[M+H]^+$

Step 6: Synthesis of 14g

Under nitrogen protection, intermediate 1 (250.0 mg, 0.70 mmol) was dissolved in dry DMF (1.5 mL), and HOBt (190 mg, 1.41 mmol), EDCI (270 mg, 1.41 mmol) and DIPEA (270 mg, 2.09 mmol) were successively added. At room temperature, the reaction mixture was stirred for 40 min, and then 14f (270 mg, 0.70 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 14g (368 mg, yield: 72.43%).
LC-Ms m/z (ESI): 726.8 $[M+H]^+$

Step 7: Synthesis of compound 14-1 and compound 14-2

Compound 14g (368 mg, 0.51 mmol) was dissolved in methanol (1.5 mL) and water (0.5 mL), lithium hydroxide monohydrate (31 mg, 1.27 mmol) was added, and the resulting mixture was reacted at room temperature for 30 min. PH was adjusted to 5-6 with 1 *N* hydrochloric acid, and the resulting solution was concentrated under reduced pressure to afford crude compound 14, which was separated and purified by prep-HPLC (method 2) to afford the trifluoroacetate salt of compound 14-1 (20 mg, Rt=4.295 min, yield: 5.62 %) and the trifluoroacetate salt of compound 14-2 (25 mg, Rt = 4.378 min, yield: 7.02 %).

Trifluoroacetate salt of compound 14-1:

LC-Ms m/z (ESI): 698.8 $[M+H]^+$
$^1$H NMR (400 MHz, $CD_3OD$) δ 7.90 - 7.68 (m, 1H), 7.20 (s, 1H), 6.88 - 6.73 (m, 4H), 5.84 - 5.75 (m, 1H), 5.39 - 5.27 (m, 1H), 4.32 - 3.83 (m, 4H), 3.52 - 3.34 (m, 1H), 3.20 - 2.81 (m, 7H), 2.49 - 2.34 (m, 2H), 2.10 - 1.92 (m, 4H), 1.90 (m, 3H), 1.83 (s, 3H), 1.52 - 1.35 (m, 1H), 1.30 - 1.11 (m, 6H), 1.05 - 0.93 (m, 6H).

Trifluoroacetate salt of compound 14-2:

LC-Ms m/z (ESI): 698.8 $[M+H]^+$
$^1$H NMR (400 MHz, $CD_3OD$) δ 7.91 - 7.76 (m, 1H), 7.25 (s, 1H), 6.92 - 6.78 (m, 4H), 5.84 - 5.73 (m, 1H), 5.39 - 5.26 (m, 1H), 4.34 - 3.85 (m, 4H), 3.53 - 3.35 (m, 1H), 3.24 - 2.95 (m, 4H), 2.94 - 2.77 (m, 3H), 2.53 - 2.43 (m, 2H), 2.12 - 2.00 (m, 2H), 1.96 (s, 6H), 1.91 - 1.78 (m, 2H), 1.40 - 1.20 (m, 7H), 0.96 - 0.85 (m, 6H).

**Example 15:** Preparation of compound 15

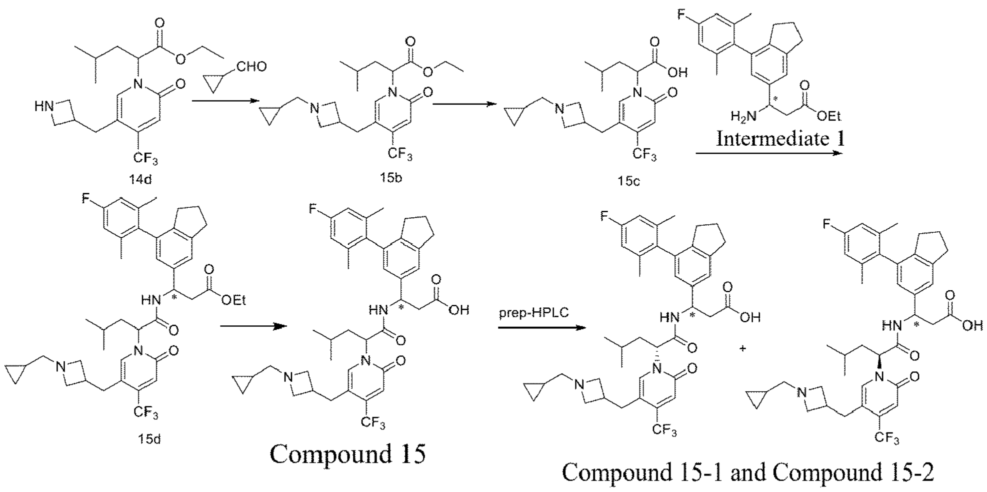

14d 15b 15c 15d
Compound 15
Compound 15-1 and Compound 15-2

Referring to the preparation method of compound 14, crude compound 15 was obtained, which was separated and purified by prep-HPLC (method 2) to afford the trifluoroacetate salt of compound 15-1 (15 mg, Rt=4.354 min) and the trifluoroacetate salt of compound 15-2 (17 mg, Rt = 4.455 min).

Trifluoroacetate salt of compound 15-1:

LC-Ms m/z (ESI): 710.8 $[M+H]^+$
$^1$H NMR (400 MHz, $CD_3OD$) δ 7.89 - 7.72 (s, 1H), 7.20 (s, 1H), 6.89 - 6.72 (m, 4H), 5.84 - 5.75 (m, 1H), 5.38 - 5.28 (m, 1H), 4.36 - 3.84 (m, 4H), 3.27 - 2.79 (m, 9H), 2.48 - 2.37 (m, 2H), 2.09 - 1.93 (m, 4H), 1.92 - 1.81 (m, 6H), 1.51 - 1.38 (m, 1H), 1.07 - 0.94 (m, 7H), 0.74 - 0.63 (m, 2H), 0.44 - 0.34 (m, 2H).

Trifluoroacetate salt of compound 15-2:

LC-Ms m/z (ESI): 710.8 $[M+H]^+$
$^1$H NMR (400 MHz, $CD_3OD$) δ 7.92 - 7.74 (s, 1H), 7.25 (s, 1H), 6.93 - 6.77 (m, 4H), 5.83 - 5.73 (m, 1H), 5.38 - 5.28 (m, 1H), 4.36 - 3.86 (m, 4H), 3.30 - 2.76 (m, 9H), 2.52 - 2.43 (m, 2H), 2.12 - 2.01 (m, 2H), 1.96 (s, 6H), 1.93 - 1.74 (m, 2H), 1.41 - 1.27 (m, 1H), 1.11 - 0.84 (m, 7H), 0.76 - 0.61 (m, 2H), 0.47 - 0.38 (m, 2H).

**Example 16:** Preparation of compound 16

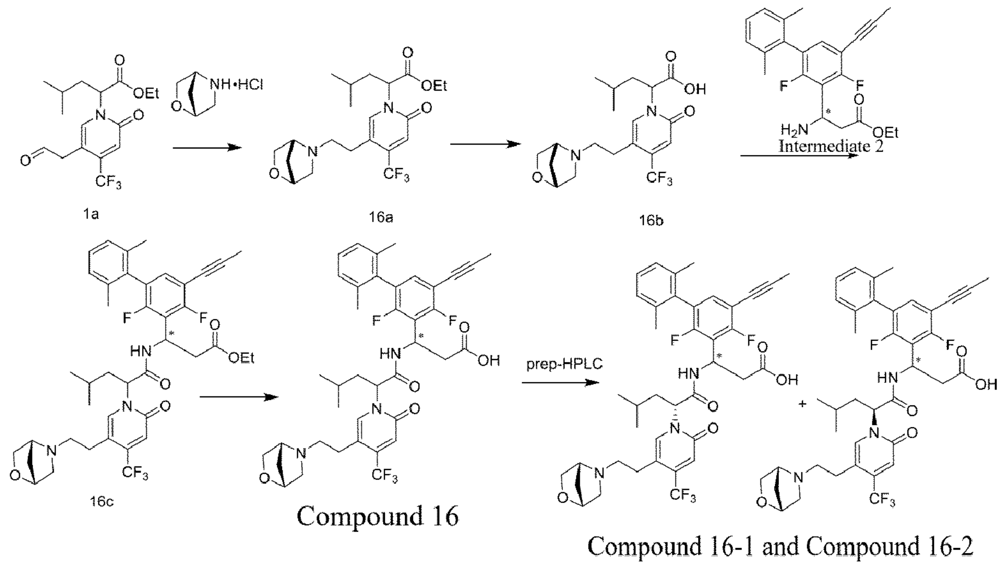

1a
16a
16b
16c
Compound 16
Compound 16-1 and Compound 16-2

Referring to the preparation method of compound 3, crude compound 16 was obtained, which was separated and purified by prep-HPLC (method 2) to afford the trifluoroacetate salt of compound 16-1 (15 mg, Rt=4.077 min) and the trifluoroacetate salt of compound 16-2 (16 mg, Rt = 4.178 min).

Trifluoroacetate salt of compound 16-1:

LC-Ms m/z (ESI): 728.7 $[M+H]^+$
$^1$H NMR (400 MHz, $CD_3OD$) δ 7.92 (s, 1H), 7.23 - 7.15 (m, 1H), 7.14 - 7.03 (m, 3H), 6.86 (s, 1H), 5.87 - 5.76 (m, 1H), 5.75 - 5.64 (m, 1H), 4.77 - 4.68 (m, 1H), 4.61 - 4.47 (m, 1H), 4.23 - 4.02 (m, 1H), 3.97 - 3.84 (m, 1H), 3.77 - 3.35 (m, 4H), 3.19 - 2.86 (m, 4H), 2.38 - 2.18 (m, 2H), 2.12 - 1.84 (m, 11H), 1.50 - 1.35 (m, 1H), 1.05 - 0.90 (m, 6H).

Trifluoroacetate salt of compound 16-2:

LC-Ms m/z (ESI): 728.7 $[M+H]^+$
$^1H$ NMR (400 MHz, $CD_3OD$) δ 7.95 (s, 1H), 7.25 - 7.07 (m, 4H), 6.92 (s, 1H), 5.87 - 5.78 (m, 1H), 5.75 - 5.66 (m, 1H), 4.77 - 4.70 (m, 1H), 4.62 - 4.51 (m, 1H), 4.26 - 4.04 (m, 1H), 3.94 - 3.85 (m, 1H), 3.75 - 3.36 (m, 4H), 3.14 - 2.89 (m, 4H), 2.39 - 2.18 (m, 2H), 2.12 - 1.98 (m, 9H), 1.97 - 1.73 (m, 2H), 1.41 - 1.25 (m, 1H), 0.94 - 0.85 (m, 6H).

**Example 17:** Preparation of compound 17

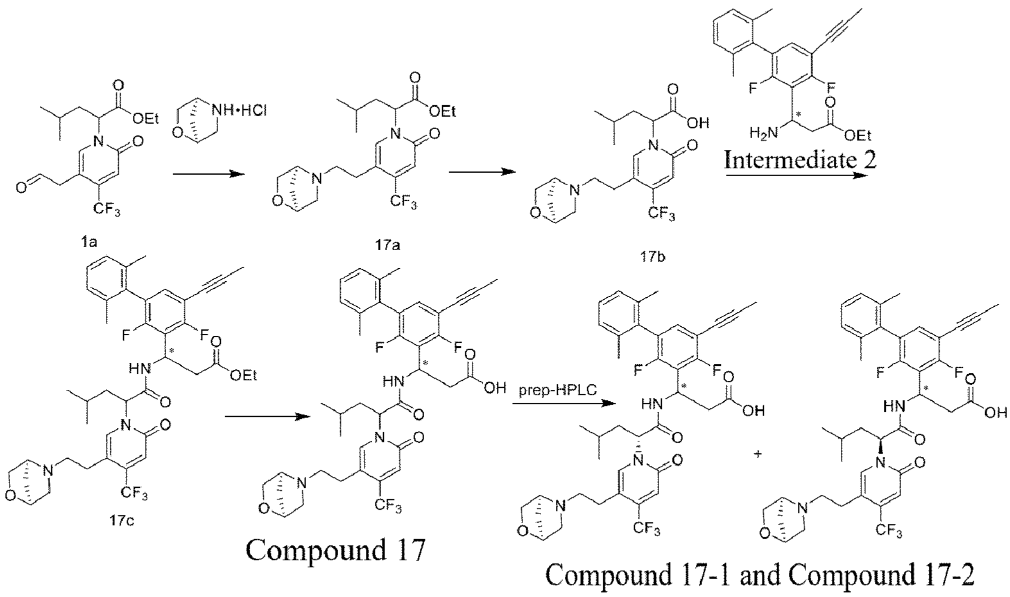

1a
17a
17b
17c
Compound 17
+
Compound 17-1 and Compound 17-2

Referring to the preparation method of compound 3, crude compound 17 was obtained, which was separated and purified by prep-HPLC (method 2) to afford the trifluoroacetate salt of compound 17-1 (17 mg, Rt=4.035 min) and the trifluoroacetate salt of compound 17-2 (18 mg, Rt = 4.118 min).

Trifluoroacetate salt of compound 17-1:

LC-Ms m/z (ESI): 728.7 $[M+H]^+$
$^1H$ NMR (400 MHz, $CD_3OD$) δ 8.01 - 7.80 (s, 1H), 7.23 - 7.15 (m, 1H), 7.14 - 7.03 (m, 3H), 6.86 (s, 1H), 5.88 - 5.78 (m, 1H), 5.76 - 5.64 (m, 1H), 4.79 - 4.65 (m, 1H), 4.62 - 4.46 (m, 1H), 4.41 - 3.97 (m, 1H), 3.96 - 3.64 (m, 2H), 3.60 - 3.40 (m, 2H), 3.32 - 3.22 (m, 1H), 3.19 - 2.85 (m, 4H), 2.45 - 2.13 (m, 2H), 2.11 - 1.85 (m, 11H), 1.50 - 1.37 (m, 1H), 1.04 - 0.90 (m, 6H).

Trifluoroacetate salt of compound 17-2:

LC-Ms m/z (ESI): 728.7 $[M+H]^+$
$^1H$ NMR (400 MHz, $CD_3OD$) δ 7.93 (s, 1H), 7.25 - 7.06 (m, 4H), 6.92 (s, 1H), 5.88 -5.77 (m, 1H), 5.75 - 5.64 (m, 1H), 4.79 - 4.68 (m, 1H), 4.62 - 4.48 (m, 1H), 4.24 - 3.98 (m, 1H), 3.97 - 3.84 (m, 1H), 3.82 - 3.62 (m, 1H), 3.62 - 3.37 (m, 3H), 3.15 - 2.89 (m, 4H), 2.44 - 2.16 (m, 2H), 2.09 (s, 3H), 2.04 (s, 6H), 1.95 - 1.71 (m, 2H), 1.38 - 1.26 (m, 1H), 0.98 - 0.84 (m, 6H).

**Example 18:** Preparation of compound 18

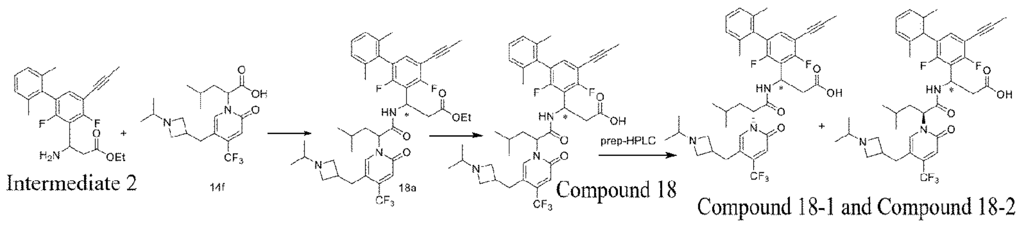


Referring to the preparation method of compound 14, crude compound 18 was obtained, which was separated and purified by prep-HPLC (method 2) to afford the trifluoroacetate salt of compound 18-1 (12 mg, Rt=4.164 min) and the trifluoroacetate salt of compound 18-2 (13 mg, Rt = 4.268 min).

Trifluoroacetate salt of compound 18-1:

LC-Ms m/z (ESI): 714.7 $[M+H]^+$
$^1$H NMR (400 MHz, $CD_3OD$) δ 7.97 - 7.77 (m, 1H), 7.25 - 7.18 (m, 1H), 7.18 - 7.09 (m, 3H), 6.89 (s, 1H), 5.89 - 5.79 (m, 1H), 5.77 - 5.65 (m, 1H), 4.34 - 4.05 (m, 2H), 4.03 - 3.85 (m, 2H), 3.55 - 3.36 (m, 1H), 3.24 - 3.03 (m, 2H), 3.00 - 2.79 (m, 3H), 2.08 (s, 3H), 2.04 (s, 6H), 1.93 - 1.71 (m, 2H), 1.39 - 1.20 (m, 7H), 0.97 - 0.85 (m, 6H).

Trifluoroacetate salt of compound 18-2:

LC-Ms m/z (ESI): 714.7 $[M+H]^+$
$^1$H NMR (400 MHz, $CD_3OD$) δ 7.91 - 7.72 (m, 1H), 7.22 - 7.15 (m, 1H), 7.14 - 7.04 (m, 3H), 6.84 (s, 1H), 5.88 - 5.79 (m, 1H), 5.75 - 5.65 (m, 1H), 4.33 - 4.14 (m, 1H), 4.10 - 3.81 (m, 3H), 3.51 - 3.35 (m, 1H), 3.22 - 3.07 (m, 2H), 3.06 - 2.81 (m, 3H), 2.14 - 2.04 (m, 4H), 2.02 - 1.83 (m, 7H), 1.48 - 1.35 (m, 1H), 1.32 - 1.17 (m, 6H), 1.04 - 0.93 (m, 6H).

**Example 19:** Preparation of compound 19

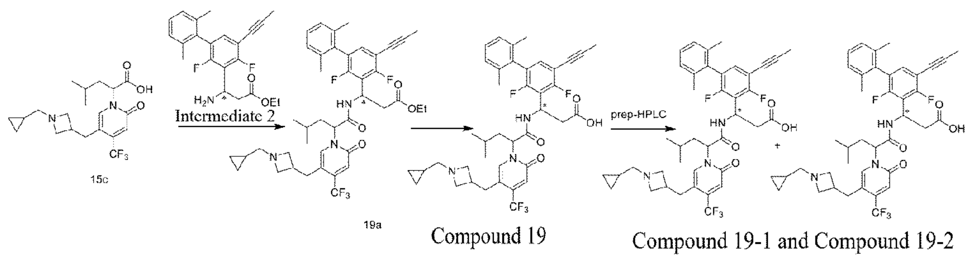


Referring to the preparation method of compound 14, crude compound 19 was obtained, which was separated and purified by prep-HPLC (method 2) to afford the trifluoroacetate salt of compound 19-1 (18 mg, Rt=4.218 min) and the trifluoroacetate salt of compound 19-2 (19 mg, Rt = 4.297 min).

Trifluoroacetate salt of compound 19-1:

LC-Ms m/z (ESI): 726.8 $[M+H]^+$
$^1$H NMR (400 MHz, Methanol-$d_4$) δ 9.36 - 9.20 (m, 1H), 7.88 -7.69 (m, 1H), 7.23 - 7.04 (m, 4H), 6.88 -6.77 (m, 1H), 5.87 -

5.76 (m, 1H), 5.75 -5.65 (m, 1H), 4.34 - 3.85 (m, 4H), 3.26 - 2.87 (m, 7H), 2.12 -2.01 (m, 4H), 2.01 - 1.93 (m, 4H), 1.92 -1.84 (m, 3H), 1.46 - 1.35 (m, 1H), 1.06 -1.93 (m, 7H), 0.74 -0.64 (m, 2H), 0.47 - 0.35 (m, 2H).

Trifluoroacetate salt of compound 19-2:

LC-Ms m/z (ESI): 726.8 [M+H]+

$^1$H NMR (400 MHz, Methanol-$d_4$) δ 9.51 - 9.33 (m, 1H), 7.94 - 7.77 (m, 1H), 7.27 -7.04 (m, 4H), 6.89 (d, 1H), 5.88 -5.78 (m, 1H), 5.76 -5.65 (m, 1H), 4.37 - 3.90 (m, 4H), 3.29 - 2.82 (m, 7H), 2.08 (s, 3H), 2.04 (s, 6H), 1.92 - 1.69 (m, 2H), 1.39 -1.25 (m, 1H), 1.11 -0.96 (m, 1H), 0.96 - 0.86 (m, 6H), 0.75 -0.64 (m, 2H), 0.45 -0.36 (m, 2H).

**Example 20:** Preparation of compound 20

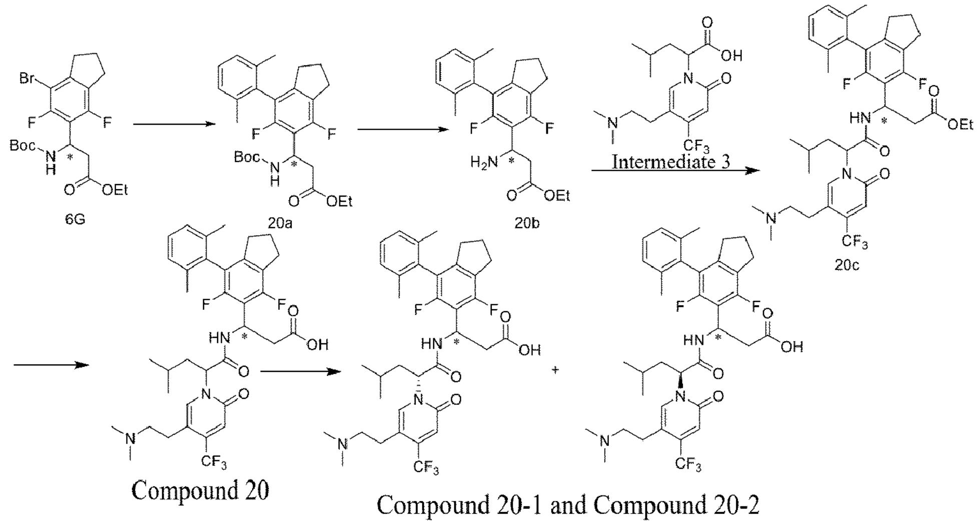

6G
20a
20b
20c

Compound 20

Compound 20-1 and Compound 20-2

Referring to the preparation method of Example 21, crude compound 20 was obtained, which was separated and purified by prep-HPLC (method 2) to afford the trifluoroacetate salt of compound 20-1 (16 mg, Rt=4.007 min, yield: 5.51%) and the trifluoroacetate salt of compound 20-2 (17 mg, Rt = 4.095 min, yield: 5.85%).

Trifluoroacetate salt of compound 20-1:

LC-Ms m/z (ESI): 676.7 [M+H]+

$^1$H NMR (400 MHz, Methanol-$d_4$) δ 9.20 (d, 1H), 7.89 (s, 1H), 7.20 - 7.04 (m, 3H), 6.82 (s, 1H), 5.82 -5.73 (m, 1H), 5.74 -5.64 (m, 1H), 3.29 - 3.23 (m, 2H), 3.16 - 2.82 (m, 12H), 2.50 -2.35 (m, 2H), 2.08 - 1.88 (m, 7H), 1.86 (s, 3H), 1.47 - 1.34 (m, 1H), 1.00- 0.89 (m, 6H).

Trifluoroacetate salt of compound 20-2:

LC-Ms m/z (ESI): 676.7 [M+H]+

$^1$H NMR (400 MHz, Methanol-$d_4$) δ 9.33 (d, 1H), 7.91 (s, 1H), 7.20 - 7.07 (m, 3H), 6.88 (s, 1H), 5.82 - 5.66 (m, 2H), 3.37 - 3.30 (m, 2H), 3.08 - 2.86 (m, 12H), 2.48 (t, 2H), 2.14 - 2.01 (m, 2H), 1.98 (s, 6H), 1.86 - 1.73 (m, 2H), 1.36 -1.23 (m, 1H), 0.93 -0.81 (m, 6H).

**Example 21:** Preparation of compound 21

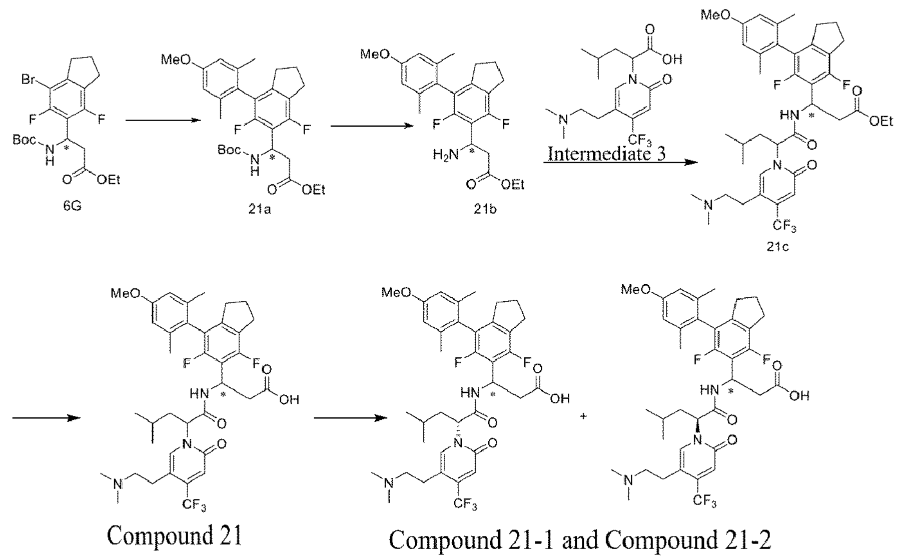


6G 21a 21b 21c

Compound 21

Compound 21-1 and Compound 21-2

Step 1: Synthesis of 21a

Under nitrogen protection, 6G (400 mg, 0.89 mmol) was dissolved in 1,4-dioxane (6 mL) and water (0.6 mL). 2.6-Dimethyl -4-methoxy phenylboronic acid (240 mg, 1.33 mmol), XPhos Pd G2 (110 mg, 0.13 mmol) and potassium phosphate (570 mg, 2.67 mmol) were successively added, and the resulting mixture was reacted at 100°C for 24 h. Extraction was performed by addition of ethyl acetate (80 mL) and the resulting mixture was washed with water (50 mL×2) and saturated sodium chloride aqueous solution (50 mL×1), dried over anhydrous sodium sulfate and filtered and the filtrate was concentrated under reduced pressure and subjected to column chromatography on a silica gel column to afford 21a (230 mg, yield: 51.32%).
LC-Ms m/z (ESI): 404.5 $[M+H-Boc]^+$

Step 2: Synthesis of 21b

21a (230 mg, 0.46 mmol) was dissolved in dichloromethane (6 mL), hydrogen chloride-1,4-dioxane (6 mL) was added, and the reaction was carried out for 1 h. The solvent was removed under reduced pressure to afford the hydrochloride salt of crude 21b.

Step 3: Synthesis of 21c

Under nitrogen protection, intermediate 3 (160 mg, 0.46 mmol) was dissolved in dry DMF (8 mL), and HATU (350 mg, 0.92 mmol) and DIPEA (230 mg, 1.81 mmol) were successively added. At room temperature, the reaction mixture was stirred for 40 min, and then the hydrochloride salt of 21b (185 mg, 0.46 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 21c (240 mg, yield: 71.10%).
LC-Ms m/z (ESI): 734.8$[M+H]^+$

Step 4: Synthesis of compound 21-1 and compound 21-2

Compound 21c (240 mg, 0.33 mmol) was dissolved in THF (6 mL) and water (2 mL), lithium hydroxide monohydrate (20 mg, 0.83 mmol) was added, and the resulting mixture was reacted at room temperature for 30 min. PH was adjusted to 5-6 with 1 *N* hydrochloric acid, and the resulting solution was concentrated under reduced pressure to afford crude compound 21, which was separated and purified by prep-HPLC (method 2) to afford the trifluoroacetate salt of compound 21-1 (19 mg, Rt=3.973 min, yield: 8.16 %) and the trifluoroacetate salt of compound 21-2 (20 mg, Rt = 4.051

min, yield: 8.59 %).

Trifluoroacetate salt of compound 21-1:

LC-Ms m/z (ESI): 706.7 [M+H]$^+$

$^1$H NMR (400 MHz, Methanol-$d_4$) δ 7.89 (s, 1H), 6.82 (s, 1H), 6.67 (s, 2H), 5.82 -5.74 (m, 1H), 5.72 -5.63 (m, 1H), 3.78 (s, 3H), 3.32 - 3.24 (m, 2H), 3.14 - 2.83 (m, 12H), 2.49 -2.38 (m, 2H), 2.08 - 1.92 (m, 4H), 1.90 (s, 3H), 1.83 (s, 3H), 1.47 - 1.35 (m, 1H), 1.01 -0.86 (m, 6H).

Trifluoroacetate salt of compound 21-2:

LC-Ms m/z (ESI): 706.7 [M+H]$^+$

$^1$H NMR (400 MHz, Methanol-$d_4$) δ 9.33 (d, 1H), 7.91 (s, 1H), 6.88 (s, 1H), 6.70 (s, 2H), 5.82 -5.75 (m, 1H), 5.74 -5.63 (m, 1H), 3.79 (s, 3H), 3.36 -3.29 (m, 2H), 3.10 - 2.83 (m, 12H), 2.53 -2.43 (m, 2H), 2.13 - 2.01 (m, 2H), 1.95 (s, 6H), 1.87 - 1.74 (m, 2H), 1.35 -1.23 (m, 1H), 0.93 -0.82 (m, 6H)

Compound 21-A has the structure of

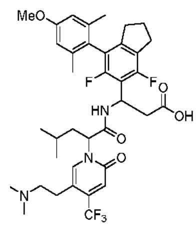
,

which is the racemate of 4 chiral isomers mixed in equal proportions.

**Example 22:** Preparation of compound 22

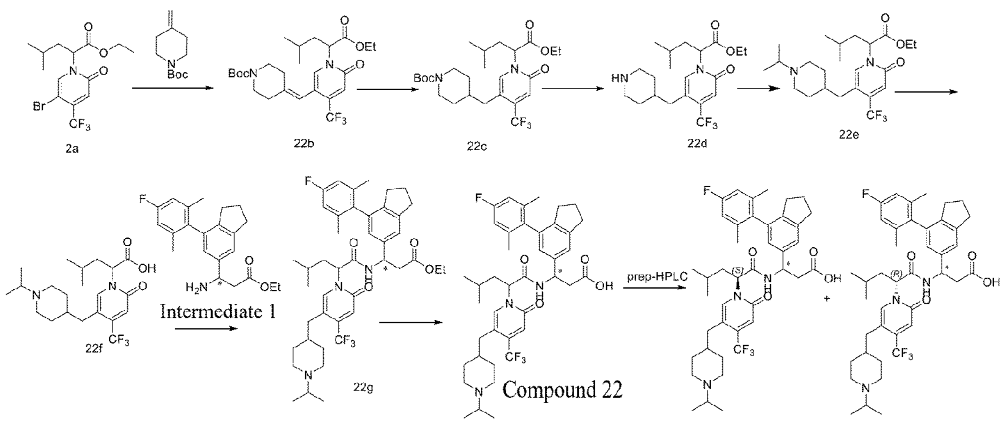


Compound 22-1 and Compound 22-2

Referring to the preparation method of compound 14, crude compound 22 was obtained, which was separated and purified by prep-HPLC (method 2) to afford the trifluoroacetate salt of compound 22-1 (10 mg, Rt=4.334 min) and the trifluoroacetate salt of compound 22-2 (10 mg, Rt = 4.411 min).

Trifluoroacetate salt of compound 22-1:

LC-Ms m/z (ESI): 726.4 [M+H]+
$^1$H NMR (400 MHz, Methanol-$d_4$) δ 7.74 (s, 1H), 7.18 (s, 1H), 6.86 - 6.70 (m, 4H), 5.76 (t, 1H), 5.33 (t, 1H), 3.50 - 3.35 (m, 3H), 3.06 - 2.97 (m, 1H), 2.96 - 2.81 (m, 5H), 2.61 - 2.49 (m, 2H), 2.40 (t, 2H), 2.07 - 1.90 (m, 6H), 1.86 (s, 3H), 1.81 (s, 3H), 1.55 - 1.37 (m, 4H), 1.34 (s, 3H), 1.32 (s, 3H), 1.00 - 0.92 (m, 6H).

Trifluoroacetate salt of compound 22-2:

LC-Ms m/z (ESI): 726.4 [M+H]+
$^1$H NMR (400 MHz, Methanol-$d_4$) δ 9.12 (d, 1H), 7.79 (s, 1H), 7.23 (d, 1H), 6.88 - 6.79 (m, 4H), 5.76 (t, 1H), 5.31 (q, 1H), 3.52 - 3.41 (m, 3H), 3.10 - 2.94 (m, 4H), 2.86 - 2.78 (m, 2H), 2.65 - 2.42 (m, 4H), 2.11 - 1.98 (m, 4H), 1.96 - 1.90 (m, 6H), 1.83 - 1.77 (m, 2H), 1.57 - 1.26 (m, 10H), 0.92 - 0.83 (m, 6H).

**Example 23:** Preparation of compound 23

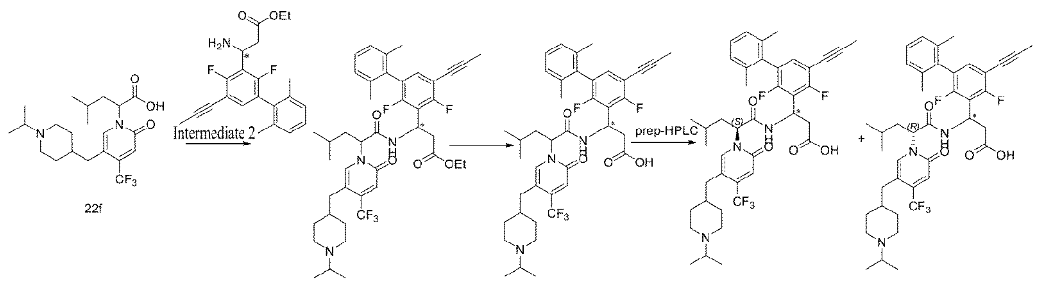


22f

23a

Compound 23

Compound 23-1 and Compound 23-2

Referring to the preparation method of compound 14, crude compound 23 was obtained, which was separated and purified by prep-HPLC (method 2) to afford the trifluoroacetate salt of compound 23-1 (10 mg, Rt=4.233 min) and the trifluoroacetate salt of compound 23-2 (10 mg, Rt = 4.411 min).

Trifluoroacetate salt of compound 23-1:

LC-Ms m/z (ESI): 742.4 [M+H]+
$^1$H NMR (400 MHz, Methanol-$d_4$) δ 9.29 (d, 1H), 7.74 (s, 1H), 7.20 - 7.03 (m, 4H), 6.81 (s, 1H), 5.84 - 5.75 (m, 1H), 5.73 - 5.63 (m, 1H), 3.51 - 3.38 (m, 2H), 3.13 - 2.87 (m, 5H), 2.60 - 2.48 (m, 2H), 2.04 (s, 3H), 2.02 - 1.89 (m, 6H), 1.88 - 1.71 (m, 5H), 1.55 - 1.25 (m, 10H), 1.00 - 0.89 (m, 6H).

Trifluoroacetate salt of compound 23-2:

LC-Ms m/z (ESI): 742.4 [M+H]+
$^1$H NMR (400 MHz, Methanol-$d_4$) δ 7.79 (s, 1H), 7.24 - 7.07 (m, 4H), 6.86 (s, 1H), 5.83 - 5.74 (m, 1H), 5.76 - 5.64 (m, 1H), 3.55 - 3.40 (m, 3H), 3.12 - 2.87 (m, 4H), 2.65 - 2.47 (m, 2H), 2.15 - 1.96 (m, 11H), 1.90 - 1.72 (m, 3H), 1.58 - 1.44 (m, 2H), 1.41 - 1.21 (m, 7H), 0.95 - 0.81 (m, 6H).

**Example 24:** Preparation of compound 24

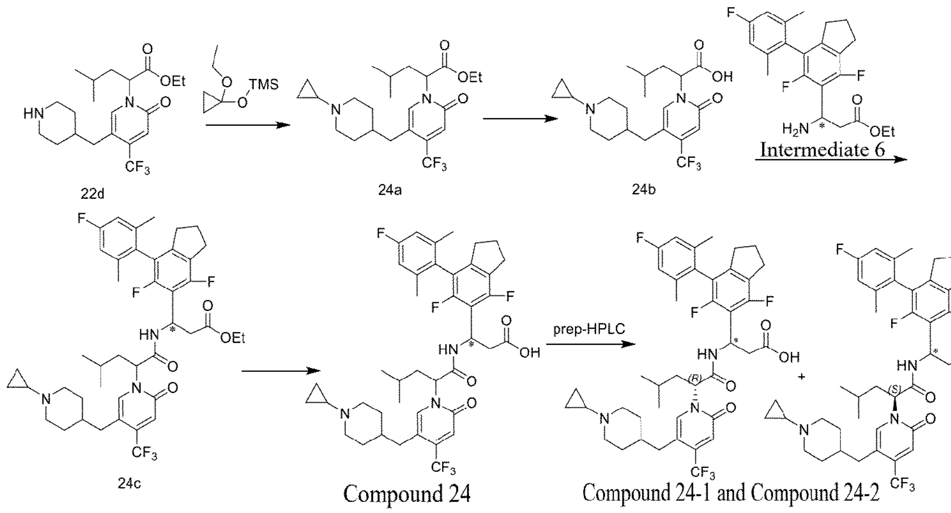


22d 24a 24b 24c Compound 24 Compound 24-1 and Compound 24-2

Referring to the preparation method of compound 14, crude compound 24 was obtained, which was separated and purified by prep-HPLC (method 1) to afford compound 24-1 (34 mg, Rt = 4.300 min) and compound 24-2 (35 mg, Rt = 4.374 min).

Compound 24-1:

LC-Ms m/z (ESI): 760.3 $[M+H]^+$
$^1$H NMR (400 MHz, Methanol-$d_4$) δ 7.71 (s, 1H), 6.89 - 6.82 (m 2H), 6.81 (s, 1H), 5.81 - 5.74 (m, 1H), 5.73 - 5.67 (m, 1H), 3.31 - 3.24 (m, 2H), 3.04 - 2.93 (m, 3H), 2.80 - 2.73 (m, 1H), 2.70 - 2.53 (m, 2H), 2.54 - 2.43 (m, 4H), 2.23 - 2.16 (m, 1H), 2.12 - 2.02 (m, 2H), 2.01 - 1.91 (m, 5H), 1.86 (s, 3H), 1.82 - 1.77 (m, 2H), 1.70 - 1.61 (m, 1H), 1.41 - 1.25 (m, 3H), 0.98 - 0.93 (m, 6H), 0.77 - 0.65 (m, 4H).

Compound 24-2:

LC-Ms m/z (ESI): 760.3 $[M+H]^+$
$^1$H NMR (400 MHz, Methanol-$d_4$) δ 7.78 (s, 1H), 6.91 (s, 1H), 6.89 - 6.85 (m, 2H), 5.81 - 5.75 (m, 1H), 5.75 - 5.68 (m, 1H), 3.47 - 3.38 (m, 2H), 3.01 (t, 2H), 2.94 - 2.84 (m, 1H), 2.82 - 2.58 (m, 4H), 2.57 - 2.47 (m, 3H), 2.33 - 2.26 (m, 1H), 2.15 - 2.05 (m, 2H), 2.02 - 1.96 (m, 6H), 1.89 - 1.71 (m, 4H), 1.59 - 1.50 (m, 1H), 1.40 - 1.24 (m, 3H), 0.91 - 0.85 (m, 6H), 0.84 - 0.79 (m, 2H), 0.77 - 0.71 (m, 2H).

**Example 25:** Preparation of compound 25

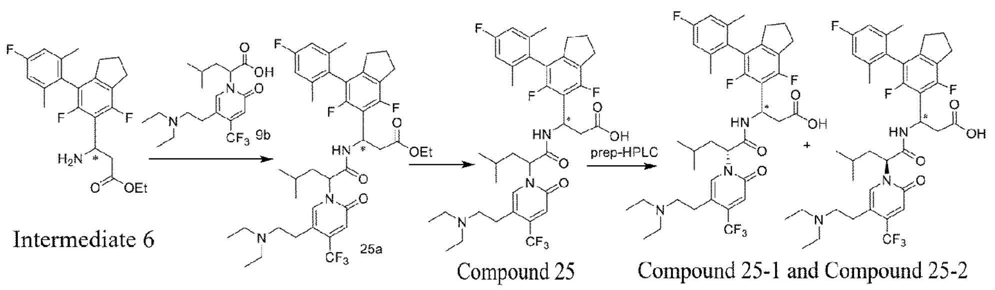


9b Intermediate 6 25a Compound 25 Compound 25-1 and Compound 25-2

Step 1: Synthesis of 25a

Under nitrogen protection, 9b (140 mg, 0.33 mmol) and intermediate 6 (124 mg, 0.33 mmol) were dissolved in dry DMF (3.0 mL). EDCI (126 mg, 0.66 mmol), HOBT (90 mg, 0.66 mmol) and DIPEA (128 mg, 0.99 mmol) were added, and the resulting mixture was reacted at room temperature overnight. Ethyl acetate (80 mL) was added, and the resulting mixture was successively washed with water (20 mL×2) and saturated sodium chloride aqueous solution (20 mL×1), dried over anhydrous sodium sulfate and filtered, and the filtrate was concentrated under reduced pressure and then subjected to column chromatography on a silica gel column to afford crude 25a (250 mg).
LC-Ms m/z (ESI): 750.2 $[M+H]^+$

Step 2: Preparation of compounds 25-1 and 25-2

The above-mentioned crude 25a (250 mg) was dissolved in THF (4 mL) and water (1 mL), lithium hydroxide monohydrate (45 mg, 1.08 mmol) was added, and the resulting mixture was reacted at room temperature for 5 h. PH was adjusted to 5-6 with 1 *N* hydrochloric acid, and the resulting solution was concentrated under reduced pressure. The residue was separated and purified by prep-HPLC (method 1) to afford compound 25-1 (29 mg, Rt=4.237 min, two-step yield: 10.86%) and compound 25-2 (36 mg, Rt = 4.294 min, two-step yield: 13.48%).

Compound 25-1:

LC-Ms m/z (ESI): 722.3 $[M+H]^+$
$^1$H NMR (400 MHz, Methanol-$d_4$) δ 7.91 (s, 1H), 6.90 - 6.76 (m, 3H), 5.78 - 5.68 (m, 2H), 3.26 - 3.11 (m, 6H), 3.02 - 2.89 (m, 5H), 2.71 - 2.62 (m, 1H), 2.50 - 2.43 (m, 2H), 2.11 - 1.98 (m, 4H), 1.97 (s, 3H), 1.90 (s, 3H), 1.45 - 1.34 (m, 1H), 1.34 - 1.28 (m, 6H), 0.99 - 0.90 (m, 6H).

Compound 25-2:

LC-Ms m/z (ESI): 722.3 $[M+H]^+$
$^1$H NMR (400 MHz, Methanol-$d_4$) δ 7.88 (s, 1H), 6.93 - 6.86 (m, 3H), 5.83 - 5.75 (m, 1H), 5.70 - 5.61 (m, 1H), 3.29 - 3.15 (m, 6H), 3.05 - 2.94 (m, 4H), 2.89 - 2.79 (m, 1H), 2.64 - 2.55 (m, 1H), 2.56 -2.46 (m, 2H), 2.14 - 2.04 (m, 2H), 2.02 - 1.97 (m, 6H), 1.95 - 1.87 (m, 1H), 1.78 - 1.69 (m, 1H), 1.39 - 1.28 (m, 7H), 0.93 - 0.86 (m, 6H).

**Example 26:** Preparation of compound 26

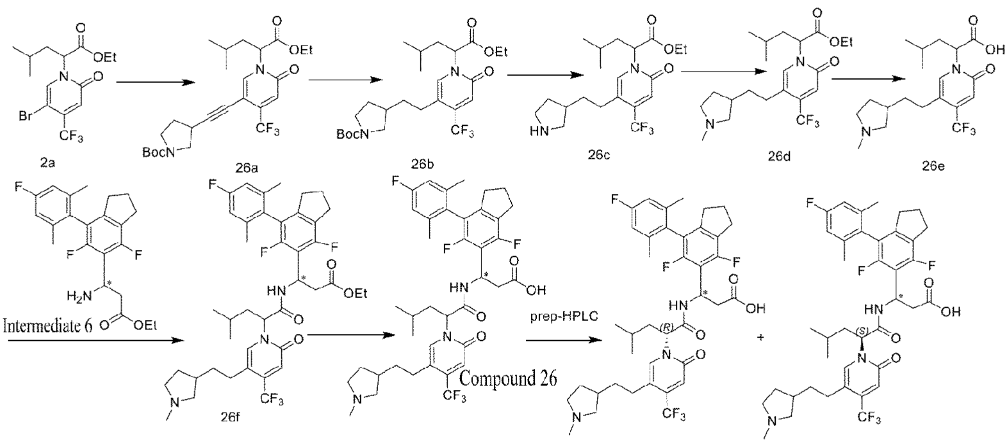


Compounds 26-1 and 26-2

Step 1: Synthesis of 26a

Under nitrogen protection, 2a (1.68 g, 4.72 mmol) was dissolved in DMF (29 mL) and water (2.9 mL). 1-BOC-3-ethynyl pyrrolidine (1.84 g, 9.44 mmol), Copper iodide (3.6 g, 18.88 mmol), lithium chloride (1.6 g, 37.76 mmol) and PdCl2(dppf) (578 mg, 0.71 mmol) were successively added, and the resulting mixture was reacted at 80 °C for 5 h. The reaction system was filtered through celite, ethyl acetate (100 mL) was added and the resulting mixture was successively washed with water (30 mL×2) and saturated sodium chloride aqueous solution (30 mL×1), dried over anhydrous sodium sulfate and filtered and the filtrate was concentrated under reduced pressure and subjected to column chromatography on a silica gel column to afford 26a (1.7 g, yield: 72.24%).
LC-Ms m/z (ESI): 443.2 $[M+H-t\text{-}Bu]^+$

Step 2: Synthesis of 26b

Under nitrogen protection, 26a (1.3 g, 2.61 mmol) was dissolved in methanol (22 mL), and 10%w/w Pd/C (2.2 g, 2.09 mmol) was added. The mixture was subjected to hydrogen replacement three times, and reacted at room temperature under the protection of hydrogen balloon for 0.5 h. The solid was removed by filtration and the filtrate was concentrated to afford crude 26b (1.3 g).
LC-Ms m/z (ESI): 447.2 $[M+H-t\text{-}Bu]^+$

Step 3: Synthesis of 26c

The crude 26b (1.3 g) from the previous step was dissolved in dichloromethane (14 mL), hydrogen chloride-1,4-dioxane (10 mL) was added, and the reaction was carried out for 3 h. The solvent was removed under reduced pressure to afford crude 26c (1.4 g).
LC-Ms m/z (ESI): 403.6 $[M+H]^+$

Step 4: Synthesis of 26d

The crude 26c (1.4 g, 3.48 mmol) was dissolved in DCE (23 mL), paraformaldehyde (209 mg, 6.96 mmol), acetic acid (105 mg, 1.74 mmol) and sodium triacetoxyborohydride (1475 mg, 6.96 mmol) were successively added, and the resulting mixture was reacted at 60°C for 16 h. Dichloromethane (80 mL) and water (30 mL) were added, and the resulting mixture was washed with saturated sodium chloride aqueous solution (30 mL×1), dried over anhydrous sodium sulfate and filtered and the filtrate was concentrated under reduced pressure and subjected to column chromatography on a silica gel column to afford 26d (750 mg, three-step yield: 51.75%).
LC-Ms m/z (ESI): 417.6 $[M+H]^+$

Step 5: Synthesis of 26e

26d (780 mg, 1.87 mmol) was dissolved in THF (8 mL) and water (2 mL), lithium hydroxide monohydrate (235 mg, 5.61 mmol) was added, and the resulting mixture was reacted at room temperature for 3 h. The reaction mixture was adjusted with 1 *N* hydrochloric acid to pH 5-6 and concentrated under reduced pressure to afford crude compound, which was separated by C18 column (mobile phase A: acetonitrile, and mobile phase B: 0.5% aqueous $NH_4HCO_3$, gradient elution from 10% to 40% of A in B) to afford 26e (620 mg, 85.36%).
LC-Ms m/z (ESI): 389.4 $[M+H]^+$

Step 6: Synthesis of 26f

Under nitrogen protection, 26e (155 mg, 0.40 mmol) and intermediate 6 (157 mg, 0.40 mmol) were dissolved in dry DMF (3.0 mL). EDCI (153 mg, 0.80 mmol), HOBT (108 mg, 0.80 mmol) and DIPEA (155 mg, 1.20 mmol) were added, and the resulting mixture was reacted at room temperature overnight. Ethyl acetate (80 mL) was added, and the resulting mixture was successively washed with water (20 mL×2) and saturated sodium chloride aqueous solution (20 mL×1), dried over anhydrous sodium sulfate and filtered, and the filtrate was concentrated under reduced pressure and then subjected to column chromatography on a silica gel column to afford 26f (189 mg, 62.02%).
LC-Ms m/z (ESI): 762.7 $[M+H]^+$

Step 7: Preparation of compounds 26-1 and 26-2

26f (189 mg, 0.25 mmol) was dissolved in THF (4 mL) and water (1 mL), lithium hydroxide monohydrate (32 mg,

0.75 mmol) was added, and the resulting mixture was reacted at room temperature for 5 h. PH was adjusted to 5-6 with 1 *N* hydrochloric acid, and the resulting solution was concentrated under reduced pressure to afford crude compound 26, which was separated and purified by prep-HPLC (method 2) to afford the trifluoroacetate salt of compound 26-1 (30 mg, Rt=4.218 min, two-step yield: 16.35%) and the trifluoroacetate salt of compound 26-2 (30 mg, Rt = 4.305 min, two-step yield: 16.35%).

Trifluoroacetate salt of compound 26-1:

LC-Ms m/z (ESI): 734.3 $[M+H]^+$
$^1$H NMR (400 MHz, Methanol-$d_4$) δ 9.16 (t, 1H), 7.76 (d, 1H), 6.91 - 6.83 (m, 2H), 6.81 (s, 1H), 5.84 - 5.76 (m, 1H), 5.75 - 5.67 (m, 1H), 3.87 - 3.63 (m, 2H), 3.29 - 3.01 (m, 3H), 3.01 - 2.86 (m, 5H), 2.81 - 2.53 (m, 3H), 2.53 - 2.42 (m, 2H), 2.38 - 2.18 (m, 2H), 2.12 - 2.03 (m, 3H), 2.02 - 1.99 (m, 1H), 1.94 (d, 3H), 1.89 (d, 3H), 1.82 - 1.69 (m, 2H), 1.45 - 1.33 (m, 2H), 1.01 - 0.93 (m, 6H).

Trifluoroacetate salt of compound 26-2:

LC-Ms m/z (ESI): 734.3 $[M+H]^+$
$^1$H NMR (400 MHz, Methanol-$d_4$) δ 9.32 (d, 1H), 7.85 (s, 1H), 6.96 - 6.84 (m, 3H), 5.80 (t, 1H), 5.75 - 5.66 (m, 1H), 3.88 - 3.64 (m, 2H), 3.25 - 2.83 (m, 8H), 2.82 - 2.56 (m, 3H), 2.54 - 2.45 (m, 2H), 2.41 - 2.18 (m, 1H), 2.17 - 2.06 (m, 2H), 2.00 (s, 6H), 1.90 - 1.70 (m, 5H), 1.40 - 1.23 (m, 2H), 0.95 - 0.83 (m, 6H).

**Example 27:** Preparation of compound 27

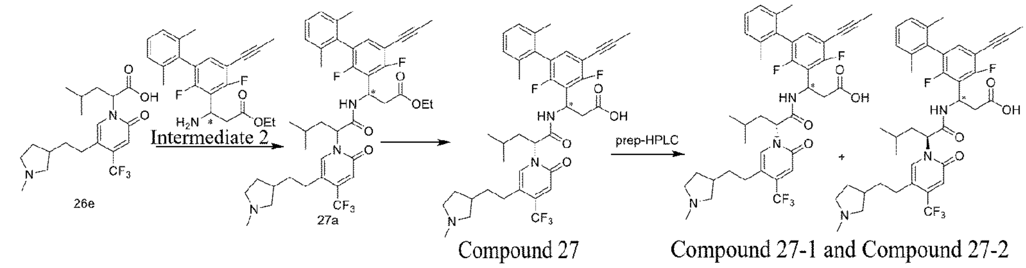


Referring to the preparation method of compound 26, crude compound 27 was obtained, which was separated and purified by prep-HPLC (method 2) to afford the trifluoroacetate salt of compound 27-1 (58 mg, Rt=4.134 min) and the trifluoroacetate salt of compound 27-2 (86 mg, Rt = 4.243 min).

Trifluoroacetate salt of compound 27-1:

LC-Ms m/z (ESI): 714.3 $[M+H]^+$
$^1$H NMR (400 MHz, Methanol-$d_4$) δ 9.27 (t, 1H), 7.75 (s, 1H), 7.19 (t, 1H), 7.13 - 7.04 (m, 3H), 6.82 (s, 1H), 5.83 - 5.74 (m, 1H), 5.74 - 5.63 (m, 1H), 3.87 - 3.55 (m, 2H), 3.26 - 3.02 (m, 2H), 3.00 - 2.88 (m, 4H), 2.81 - 2.46 (m, 3H), 2.46 - 2.15 (m, 2H), 2.06 (s, 3H), 2.00 - 1.91 (m, 4H), 1.90 - 1.84 (m, 4H), 1.79 - 1.66 (m, 2H), 1.47 - 1.33 (m, 2H), 1.01 - 0.92 (m, 6H).

Trifluoroacetate salt of compound 27-2:

LC-Ms m/z (ESI): 714.3 $[M+H]^+$

$^1$H NMR (400 MHz, Methanol-$d_4$) δ 9.47 - 9.37 (m, 1H), 7.84 (d, 1H), 7.26 - 7.09 (m, 4H), 6.87 (s, 1H), 5.86 - 5.76 (m, 1H), 5.76 - 5.65 (m, 1H), 3.90 - 3.54 (m, 2H), 3.25 - 3.03 (m, 2H), 3.02 - 2.88 (m, 4H), 2.84 - 2.51 (m, 3H), 2.52 - 2.18 (m, 2H), 2.14 - 2.00 (m, 9H), 1.92 - 1.60 (m, 5H), 1.42 - 1.22 (m, 2H), 0.96 - 0.82 (m, 6H).

**Example 28:** Preparation of compound 28

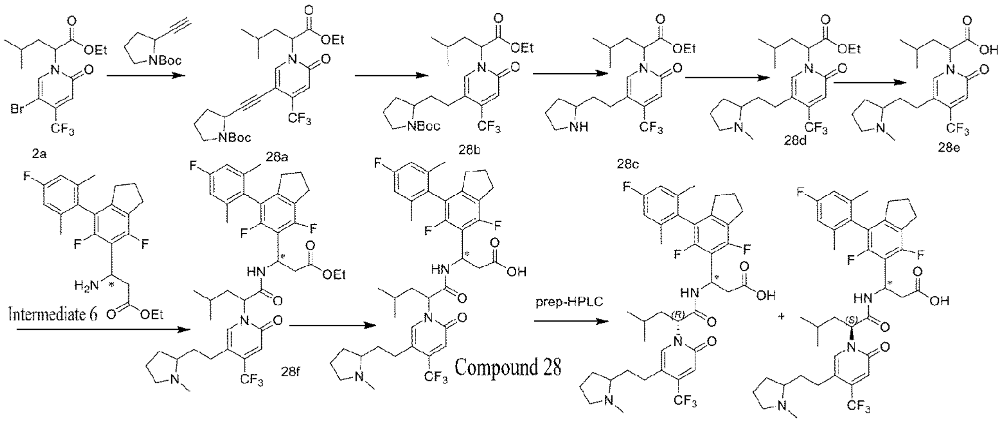


Compound 28-1 and Compound 28-2

Referring to the preparation method of compound 26, crude compound 28 was obtained, which was separated and purified by prep-HPLC (method 2) to afford the trifluoroacetate salt of compound 28-1 (37 mg, Rt=4.218 min) and the trifluoroacetate salt of compound 28-2 (50 mg, Rt = 4.343 min).

Trifluoroacetate salt of compound 28-1:

LC-Ms m/z (ESI): 734.2 [M+H]$^+$
$^1$H NMR (400 MHz, Methanol-$d_4$) δ 9.20 (t, 1H), 7.84 (d, 1H), 6.91 - 6.78 (m, 3H), 5.86 - 5.78 (m, 1H), 5.76 - 5.65 (m, 1H), 3.75 - 3.63 (m, 1H), 3.41 - 3.37 (m, 1H), 3.21 - 3.06 (m, 2H), 3.01 - 2.94 (m, 3H), 2.93 - 2.86 (m, 2H), 2.78 - 2.61 (m, 2H), 2.56 - 2.44 (m, 2H), 2.41 - 2.15 (m, 3H), 2.13 - 2.00 (m, 4H), 1.99 - 1.92 (m, 4H), 1.89 (d, 3H), 1.85 - 1.74 (m, 2H), 1.47 - 1.33 (m, 2H), 1.01 - 0.94 (m, 6H).

Trifluoroacetate salt of compound 28-2:

LC-Ms m/z (ESI): 734.2 [M+H]$^+$
$^1$H NMR (400 MHz, Methanol-$d_4$) δ 9.35 (d, 1H), 7.87 (s, 1H), 6.94 - 6.81 (m, 3H), 5.81 (t, 1H), 5.74 - 5.65 (m, 1H), 3.75 - 5.65 (m, 1H), 3.23 - 3.14 (m, 1H), 3.11 - 2.98 (m, 4H), 2.94 - 2.86 (m, 3H), 2.85 - 2.74 (m, 1H), 2.70 - 2.61 (m, 1H), 2.52 (t, $J$ = 7.4 Hz, 3H), 2.25 - 2.04 (m, 5H), 2.00 (s, 6H), 1.87 - 1.76 (m, 4H), 1.41 - 1.26 (m, 2H), 0.95 - 0.84 (m, 6H).

**Example 29:** Preparation of compound 29

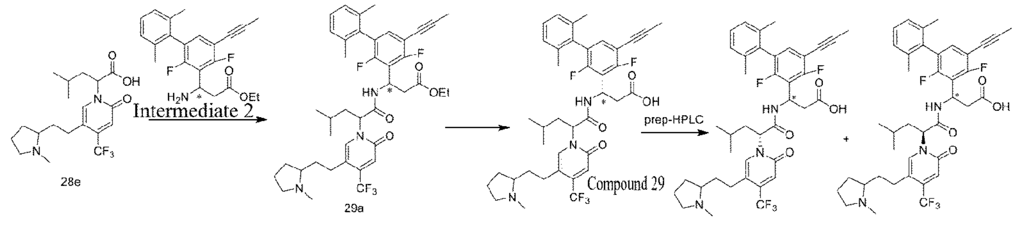

28e
29a
Compound 29
Compound 29-1 and Compound 29-2

Referring to the preparation method of compound 26, crude compound 29 was obtained, which was separated and purified by prep-HPLC (method 2) to afford the trifluoroacetate salt of compound 29-1 (60 mg, Rt=4.181 min) and the trifluoroacetate salt of compound 29-2 (88 mg, Rt = 4.279 min).

Trifluoroacetate salt of compound 29-1:

LC-Ms m/z (ESI): 714.2 $[M+H]^+$
$^1$H NMR (400 MHz, Methanol-$d_4$) δ 9.31 (t, 1H), 7.83 (d, 1H), 7.19 (t, 1H), 7.14 - 7.03 (m, 3H), 6.83 (s, 1H), 5.86 - 5.78 (m, 1H), 5.73 - 5.64 (m, 1H), 3.75 - 3.64 (m, 1H), 3.22 - 3.10 (m, 2H), 2.98 (s, 1H), 2.96 - 2.88 (m, 1H), 2.86 (s, 1H), 2.81 - 2.51 (m, 3H), 2.36 - 2.33 (m, 1H), 2.24 - 2.09 (m, 2H), 2.08 - 2.01 (m, 5H), 2.01 - 1.91 (m, 4H), 1.88 (d, 3H), 1.85 - 1.74 (m, 2H), 1.49 - 1.31 (m, 2H),1.01 - 0.93 (m, 6H).

Trifluoroacetate salt of compound 29-2:

LC-Ms m/z (ESI): 714.2 $[M+H]^+$
$^1$H NMR (400 MHz, Methanol-$d_4$) δ 9.45 (d, 1H), 7.88 (d, 1H), 7.25 - 7.09 (m, 4H), 6.88 (d, 1H), 5.86 - 5.78 (m, 1H), 5.73 - 5.64 (m, 1H), 3.75 - 3.64 (m, 1H), 3.43 - 3.37 (m, 1H), 3.24 - 3.03 (m, 2H), 3.01 - 2.90 (m, 4H), 2.86 - 2.50 (m, 3H), 2.41 - 2.29 (m, 1H), 2.25 - 2.11 (m, 2H), 2.11 - 1.97 (m, 9H), 1.89 - 1.73 (m, 4H), 1.39 - 1.26 (m, 2H), 0.95 - 0.86 (m, 6H).

**Example 30:** Preparation of compound 30 and isomer thereof

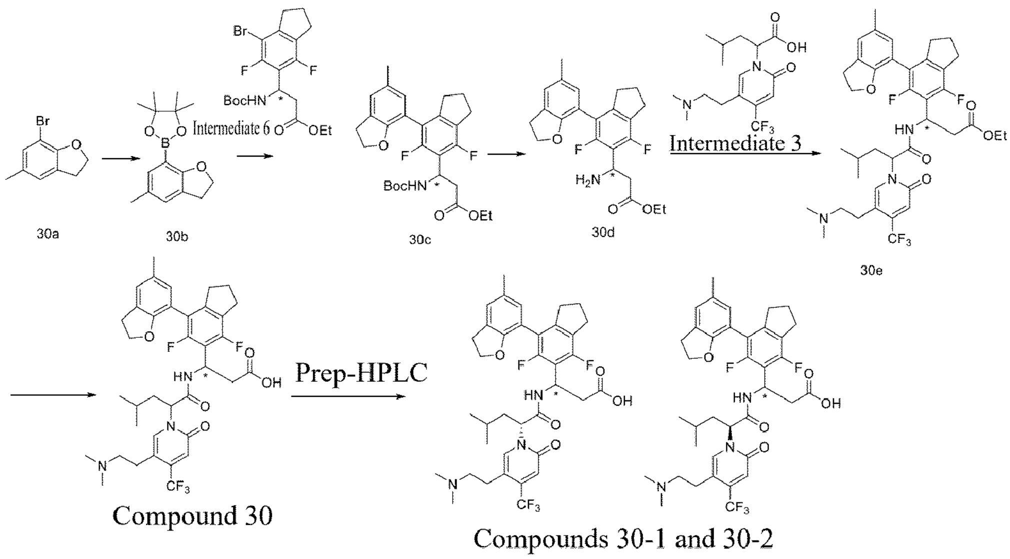

30a
30b
30c
30d
30e
Compound 30
Compounds 30-1 and 30-2

Step 1: Synthesis of 30b

Under nitrogen protection, 30a (1.76 g, 8.26 mmol) was dissolved in 1,4-dioxane (60 mL), pinacol diboronate (3.15 g, 12.39 mmol), PdCl2(dppf) (0.60 g, 0.83 mmol) and potassium acetate (2.43 g, 24.78 mmol) were successively added, and the resulting mixture was reacted at 100°C for 2h. Extraction was performed by addition of ethyl acetate (180 mL) and the resulting mixture was washed with water (50 mL×2) and saturated sodium chloride aqueous solution (50 mL×1), dried over anhydrous sodium sulfate and filtered and the filtrate was concentrated under reduced pressure and subjected to column chromatography on a silica gel column to afford 22b (1.35 g, yield: 62.86%).

Referring to the preparation method of compound 20, and taking 30b and intermediate 6 as starting materials, crude compound 30 was obtained, which was separated and purified by prep-HPLC (method 2) to afford the trifluoroacetate salt of compound 30-1 (10 mg, Rt=4.121 min, yield: 10.4%) and the trifluoroacetate salt of compound 30-2 (10 mg, Rt = 4.175 min, yield: 10.4%).

Trifluoroacetate salt of compound 30-1:

LC-Ms m/z (ESI): 704.3 [M+H]+

$^1$H NMR (400 MHz, $CD_3OD$) δ 7.90 (d, 1H), 7.04 (s, 1H), 6.84 (s, 1H), 6.76 (d, 1H), 5.85 - 5.76 (m, 1H), 5.75 - 5.65 (m, 1H), 4.52 - 4.42 (m, 2H), 3.30 - 3.25 (m, 1H), 3.24 - 3.15 (m, 2H), 3.05 - 3.05 (m, 1H), 3.05 - 2.87 (m, 10H), 2.87 - 2.74 (m, 2H), 2.69 - 2.55 (m, 1H), 2.30 - 2.22 (m, 3H), 2.11 - 1.86 (m, 4H), 1.47 - 1.33 (m, 1H), 1.00 - 0.89 (m, 6H).

Trifluoroacetate salt of compound 30-2:

LC-Ms m/z (ESI): 704.3 [M+H]+

$^1$H NMR (400 MHz, $CD_3OD$) δ 7.90 (s, 1H), 7.06 (s, 1H), 6.88 (s, 1H), 6.77 (d, 1H), 5.84 - 5.74 (m, 1H), 5.73 - 5.61 (m, 1H), 4.54 - 4.44 (m, 2H), 3.37 - 3.31 (m, 1H), 3.26 - 3.18 (m, 2H), 3.14 - 2.91 (m, 11H), 2.90 - 2.75 (m, 2H), 2.73 - 2.54 (m, 1H), 2.32 - 2.24 (m, 3H), 2.14 - 2.00 (m, 2H), 1.87 - 1.77 (m, 2H), 1.38 - 1.24 (m, 1H), 0.98 - 0.83 (m, 6H).

**Example 32:** Preparation of compound 32 and isomer thereof

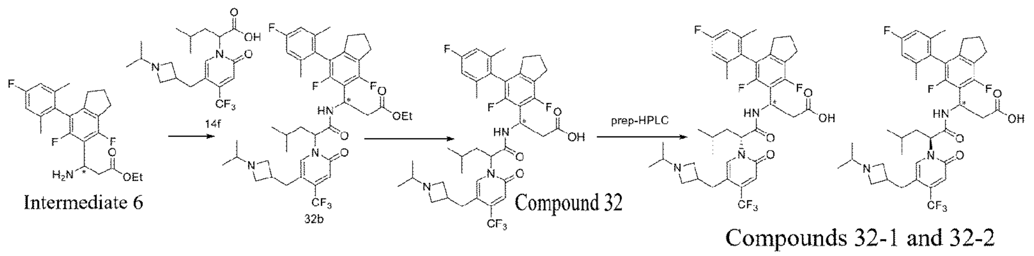

Intermediate 6
32b
Compound 32
Compounds 32-1 and 32-2

Referring to the preparation method of Example 21, crude compound 32 was obtained, which was separated and purified by prep-HPLC (method 2) to afford the trifluoroacetate salt of compound 32-1 (19 mg, Rt=4.790 min, yield: 8.93%) and the trifluoroacetate salt of compound 32-2 (20 mg, Rt = 4.862 min, yield: 8.46%).

Trifluoroacetate salt of compound 32-1:

LC-Ms m/z (ESI): 734.8 [M+H]+

$^1$H NMR (400 MHz, $CD_3OD$) δ 9.23 - 9.07 (m, 1H), 7.82 (s, 1H), 6.89 - 6.78 (m, 3H), 5.85 - 5.76 (m, 1H), 5.74 - 5.64 (m, 1H), 4.33 - 3.83 (m, 4H), 3.51 - 3.35 (m, 1H), 3.18 - 2.81 (m, 7H), 2.50 - 2.39 (m, 2H), 2.13 - 1.99 (m, 3H), 1.94 - 1.83 (m, 6H), 1.40 - 1.29 (m, 2H), 1.27 - 1.16 (m, 6H), 1.02 - 0.88 (m, 6H).

Trifluoroacetate salt of compound 32-2:

LC-Ms m/z (ESI): 734.8 [M+H]$^+$
$^1$H NMR (400 MHz, $CD_3OD$) $\delta$ 7.94 - 7.73 (m, 1H), 6.93 - 6.83 (m, 3H), 5.84 - 5.65 (m, 2H), 4.26 - 4.07 (m, 2H), 4.00 - 3.83 (m, 2H), 3.46 - 3.35 (m, 1H), 3.19 - 2.77 (m, 7H), 2.53 - 2.43 (m, 2H), 2.16 - 2.03 (m, 2H), 1.98 (s, 6H), 1.87 - 1.70 (m, 2H), 1.38 - 1.18 (m, 7H), 0.97 - 0.81 (m, 6H).

**Example 33:** Preparation of compound 33 and isomer thereof

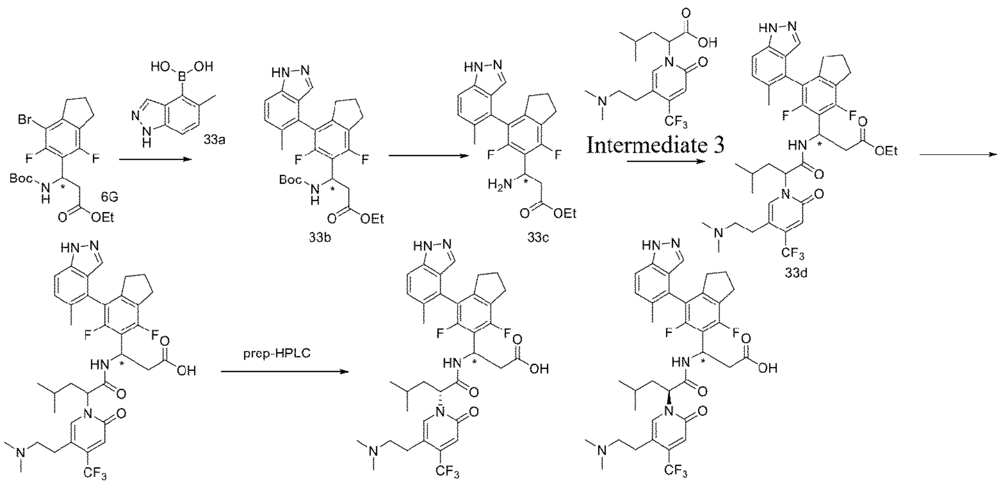


6G 33a 33b 33c 33d

Compound 33

Compounds 33-1 and 33-2

Referring to the preparation method of Example 21, crude compound 33 was obtained, which was separated and purified by prep-HPLC (method 1) to afford compound 33-1 (20 mg, Rt=3.714 min, yield: 7.50%) and compound 33-2 (25 mg, Rt = 3.765 min, yield: 9.38%).

Compound 33-1:

LC-Ms m/z (ESI): 702.7 [M+H]$^+$
$^1$H NMR (400 MHz, $CD_3OD$) $\delta$ 7.84 (d, 1H), 7.52 - 7.42 (m, 2H), 7.36 - 7.30 (m, 1H), 6.87 - 6.80 (m, 1H), 5.76 - 5.61 (m, 2H), 3.05 - 2.83 (m, 7H), 2.76 - 2.58 (m, 7H), 2.54 - 2.41 (m, 2H), 2.19 - 1.91 (m, 7H), 1.43 - 1.31 (m, 1H), 0.97 - 0.85 (m, 6H).

Compound 33-2:

LC-Ms m/z (ESI): 702.7 [M+H]$^+$
$^1$H NMR (400 MHz, $CD_3OD$) $\delta$ 7.83 - 7.78 (m, 1H), 7.53 - 7.45 (m, 2H), 7.40 - 7.33 (m, 1H), 6.92 - 6.87 (m, 1H), 5.87 - 5.79 (m, 1H), 5.68 - 5.58 (m, 1H), 3.28 - 3.14 (m, 2H), 3.06 - 2.94 (m, 4H), 2.85 - 2.75 (m, 7H), 2.62 - 2.42 (m, 3H), 2.21 - 2.16 (m, 3H), 2.13 - 2.02 (m, 2H), 1.97 - 1.85 (m, 1H), 1.81 - 1.70 (m, 1H), 1.40 - 1.27 (m, 1H), 0.94 - 0.81 (m, 6H).

**Example 35:** Preparation of compound 35

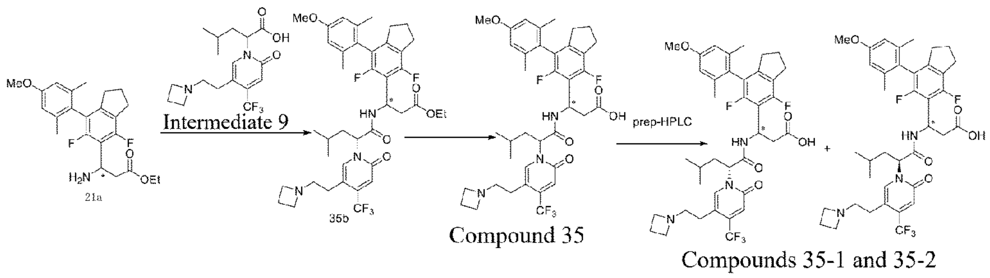

21a
35b
Compound 35
Compounds 35-1 and 35-2

Step 1: Synthesis of 35b

Under nitrogen protection, intermediate 9 (referring to WO 2021076902 for synthesis) (390 mg, 1.08 mmol) was dissolved in dry DMF (5 mL), and HATU (750 mg, 1.97 mmol) and DIPEA (510 mg, 3.98 mmol) were successively added. At room temperature, the reaction mixture was stirred for 40 min, and then intermediate 21a (400 mg, 0.99 mmol) was added. The resulting mixture was reacted at room temperature overnight. Ethyl acetate (80 mL) was added and the resulting mixture was successively washed with water (20 mL×2) and saturated sodium chloride aqueous solution (20 mL×1), dried over anhydrous sodium sulfate and filtered and the filtrate was concentrated under reduced pressure and subjected to column chromatography on a silica gel column to afford 35b (500 mg, yield: 67.72%).
LC-Ms m/z (ESI): 746.8$[M+H]^+$

Step 2: Synthesis of compound 35-1 and compound 35-2

35b (500 mg, 0.67 mmol) was dissolved in THF (9 mL) and water (3 mL), lithium hydroxide monohydrate (40 mg, 1.68 mmol) was added, and the resulting mixture was reacted at room temperature for 30 min. PH was adjusted to 5-6 with 1 *N* hydrochloric acid, and the resulting solution was concentrated under reduced pressure to afford crude compound 35, which was separated and purified by prep-HPLC (method 1) to afford compound 35-1 (21 mg, Rt=4.109 min, yield: 4.37%) and compound 35-2 (22 mg, Rt = 4.204 min, yield: 4.57%).

Compound 35-1:

LC-Ms m/z (ESI): 718.7 $[M+H]^+$
$^1$H NMR (400 MHz, $CD_3OD$) δ 7.77 (s, 1H), 6.88 (s, 1H), 6.68 (s, 2H), 5.75 - 5.68 (m, 1H), 5.68 - 5.60 (m, 1H), 4.06 - 3.95 (m, 4H), 3.78 (s, 3H), 3.00 - 2.82 (m, 5H), 2.69 - 2.59 (m, 1H), 2.51 - 2.37 (m, 4H), 2.11 - 2.01 (m, 2H), 2.00 - 1.86 (s, 9H), 1.46 - 1.34 (m, 1H), 0.97 - 0.86 (m, 6H).

Compound 35-2:

LC-Ms m/z (ESI): 718.7 $[M+H]^+$
$^1$H NMR (400 MHz, $CD_3OD$) δ 7.66 (s, 1H), 6.92 (s, 1H), 6.70 (s, 2H), 5.92 - 5.85 (m, 1H), 5.63 - 5.56 (m, 1H), 4.17 - 4.06 (m, 4H), 3.79 (s, 3H), 3.46 - 3.32 (m, 2H), 3.02 - 2.88 (m, 3H), 2.87 - 2.72 (m, 2H), 2.53 - 2.39 (m, 5H), 2.14 - 2.03 (m, 2H), 2.00 - 1.90 (m, 7H), 1.77 - 1.68 (m, 1H), 1.43 - 1.31 (m, 1H), 0.93 - 0.84 (m, 6H).

**Example 36:** Preparation of compound 36

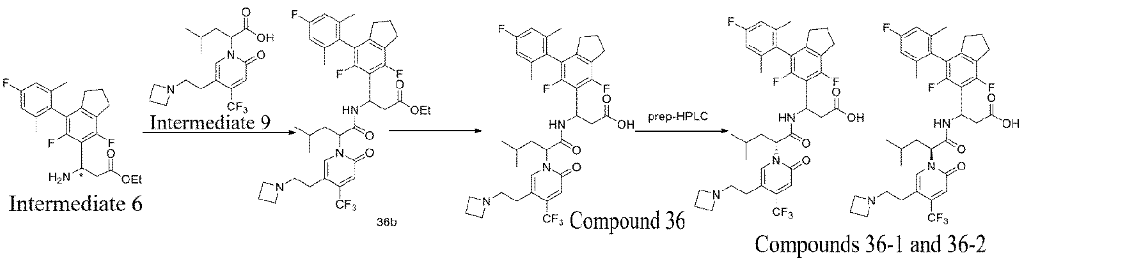

Intermediate 6 36b Compound 36 Compounds 36-1 and 36-2

Referring to the preparation method of Example 35, crude compound 36 was obtained, which was separated and purified by prep-HPLC (method 1) to afford compound 36-1 (19 mg, Rt=5.540 min, yield: 6.57%) and compound 36-2 (21 mg, Rt = 5.599 min, yield: 7.26%).

Compound 36-1:

LC-Ms m/z (ESI): 706.7 $[M+H]^+$
$^1$H NMR (400 MHz, $CD_3OD$) δ 7.86 (s, 1H), 6.91 - 6.78 (m, 3H), 5.83 - 5.66 (m, 2H), 4.43 - 4.01 (m, 4H), 3.45 - 3.33 (m, 2H), 3.15 - 3.04 (m, 1H), 2.99 - 2.76 (m, 5H), 2.68 - 2.54 (m, 1H), 2.53 - 2.37 (m, 3H), 2.11 - 1.99 (m, 3H), 1.97 - 1.83 (m, 7H), 1.47 - 1.33 (m, 1H), 1.00 - 0.89 (m, 6H).

Compound 36-2:

LC-Ms m/z (ESI): 706.7 $[M+H]^+$
$^1$H NMR (400 MHz, $CD_3OD$) δ 7.67 (s, 1H), 6.95 - 6.84 (m, 3H), 5.94 - 5.85 (m, 1H), 5.64 - 5.55 (m, 1H), 4.17 - 4.04 (m, 4H), 3.46 - 3.33 (m, 2H), 3.04 - 2.71 (m, 5H), 2.53 - 2.39 (m, 5H), 2.16 - 2.03 (m, 2H), 2.01 - 1.87 (m, 7H), 1.77 - 1.67 (m, 1H), 1.42 - 1.30 (m, 1H), 0.93 - 0.82 (m, 6H).

**Example 37:** Preparation of compound 37

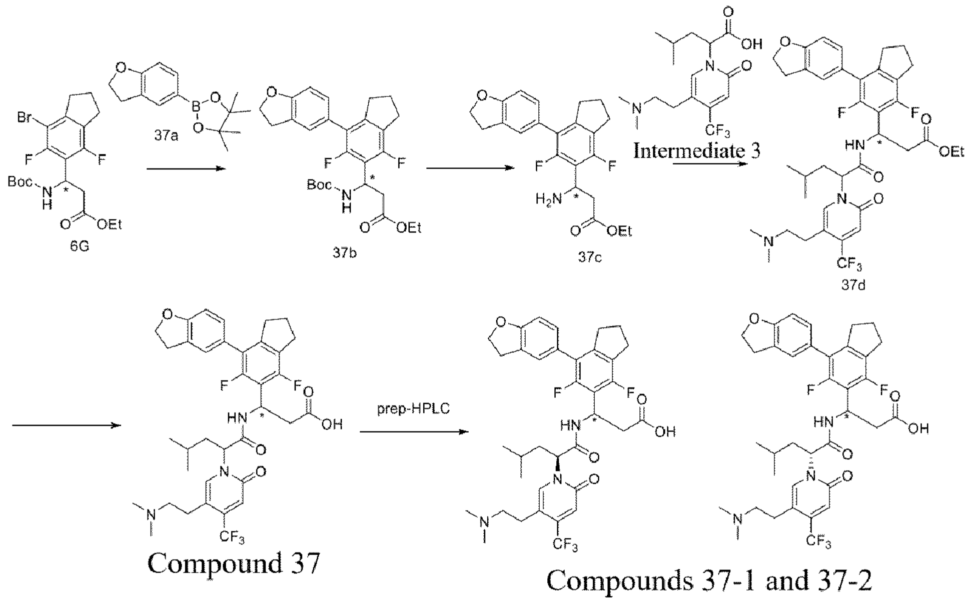

6G 37b 37c 37d Compound 37 Compounds 37-1 and 37-2

Referring to the preparation method of Example 20, crude compound 37 was obtained, which was separated and purified by prep-HPLC (method 1) to afford compound 37-1 (18 mg, Rt=5.359 min, yield: 6.21%) and compound 37-2 (19

mg, Rt = 5.401 min, yield: 6.56%).

Compound 37-1:

LC-Ms m/z (ESI): 690.7 $[M+H]^+$
$^1$H NMR (400 MHz, $CD_3OD$) δ 7.86 (s, 1H), 7.12 (s, 1H), 6.98 (d, 1H), 6.83 (s, 1H), 6.73 (d, 1H), 5.74 - 5.64 (m, 2H), 4.56 (t, 2H), 3.26 - 3.17 (m, 2H), 3.12 - 3.02 (m, 2H), 2.98 - 2.84 (m, 5H), 2.79 - 2.65 (m, 9H), 2.08 - 1.94 (m, 4H), 1.46 - 1.35 (m, 1H), 0.97 - 0.91 (m, 6H).

Compound 37-2:

LC-Ms m/z (ESI): 690.7 $[M+H]^+$
$^1$H NMR (400 MHz, $CD_3OD$) δ 7.80 (s, 1H), 7.16 (s, 1H), 7.02 (d, 1H), 6.91 (s, 1H), 6.77 (d, 1H), 5.85 - 5.77 (m, 1H), 5.69 - 5.60 (m, 1H), 4.58 (t, 2H), 3.29 - 3.12 (m, 4H), 3.05 - 2.89 (m, 4H), 2.83 - 2.72 (m, 9H), 2.57 - 2.48 (m, 1H), 2.11 - 2.00 (m, 2H), 1.96 - 1.88 (m, 1H), 1.81 - 1.70 (m, 1H), 1.41 - 1.28 (m, 1H), 0.93 - 0.82 (m, 6H).

**Example 38:** Preparation of compound 38

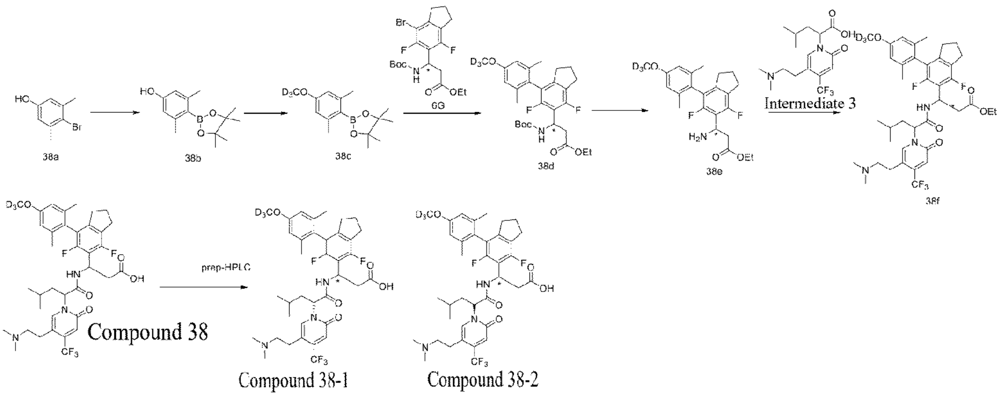

38a
38b
38c
38d
38e
38f
Compound 38
Compound 38-1
Compound 38-2

Step 1: Synthesis of 38b

Under nitrogen protection, 38a (3 g, 14.91 mmol), pinacol diboronate (4.54 g, 17.88 mmol), potassium carbonate (2.06 g, 14.91 mmol) and $PdCl_2$(dppf).DCM (CAS: 95464-05-4) (1.22 g, 1.49 mmol) were dissolved in 1.4-dioxane (30 mL) and water (3 mL), and the mixture was refluxed and reacted at 100°C overnight. Water (20 mL) was added, and the reaction solution was extracted with ethyl acetate (30 ml × 3), washed with saturated sodium chloride aqueous solution (20mL× 2), dried over anhydrous sodium sulfate, filtered, concentrated under reduced pressure, and then subjected to flash column chromatography to afford 38b (2.8 g, yield: 75.68%).

Step 2: Synthesis of 38c

38b (2.8 g, 11.28 mmol) was dissolved in a solution of dichloromethane (20 mL), and NaH (0.54 g, 22.5 mmol) was added slowly in portions under ice bath conditions. After stirring for 20 minutes, deuterated iodomethane (3.27 g, 22.56 mmol) was added, and the reaction was carried out overnight. Water (20 mL) was added and the resulting mixture was extracted with ethyl acetate (30 ml × 3), washed with saturated sodium chloride aqueous solution (20 mL×2), dried over anhydrous sodium sulfate, filtered and concentrated under reduced pressure. The residue was subjected to flash column chromatography to afford 38c (1.9 g, yield: 63.52%).

For step 3 to step 6, referring to the preparation method of Example 21, crude compound 38 was obtained, which was separated and purified by prep-HPLC (method 1) to afford compound 38-1 (20 mg, Rt=4.048 min, yield: 9.41%) and compound 38-2 (25 mg, Rt = 4.301 min, yield: 11.76%).

Compound 38-1:

LC-Ms m/z (ESI): 709.7 [M+H]+
$^1$H NMR (400 MHz, $CD_3OD$) δ 7.83 (s, 1H), 6.82 (s, 1H), 6.68 - 6.62 (m, 2H), 5.74 - 5.63 (m, 2H), 3.00 - 2.83 (m, 7H), 2.71 - 2.58 (m, 7H), 2.48 - 2.39 (m, 2H), 2.08 - 1.93 (m, 4H), 1.91 (s, 3H), 1.83 (s, 3H), 1.46 - 1.34 (m, 1H), 0.97 - 0.88 (m, 6H).

Compound 38-2:

LC-Ms m/z (ESI): 709.7 [M+H]+
$^1$H NMR (400 MHz, $CD_3OD$) δ 7.85 (s, 1H), 6.90 (s, 1H), 6.69 (s, 2H), 5.81 - 5.74 (m, 1H), 5.71 - 5.64 (m, 1H), 3.29 - 3.18 (m, 2H), 3.06 - 2.82 (m, 11H), 2.73 - 2.62 (m, 1H), 2.53 - 2.43 (m, 2H), 2.13 - 2.01 (m, 2H), 1.94 (s, 6H), 1.91 - 1.82 (m, 1H), 1.82 - 1.72 (m, 1H), 1.41 - 1.26 (m, 1H), 0.94 - 0.82 (m, 6H).

**Example 39:** Preparation of compound 39

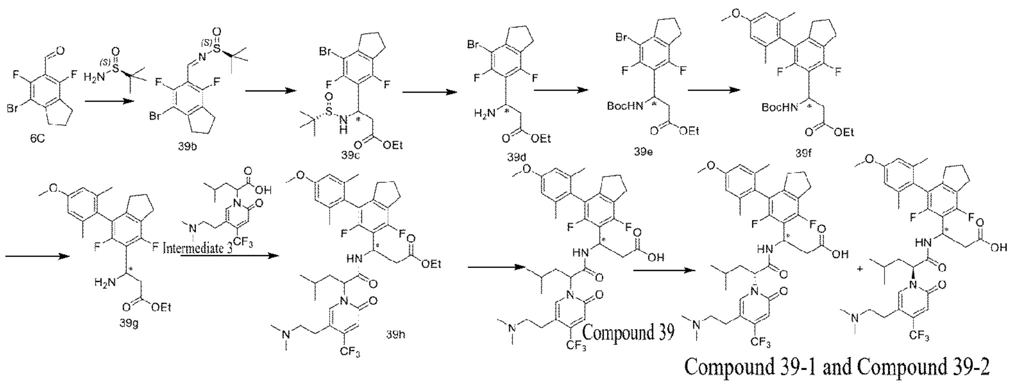


Referring to the preparation method of intermediate 6 and compound 21, crude compound 39 was obtained, which was separated and purified by prep-HPLC (method 1) to afford compound 39-1 (28 mg, Rt=4.070 min) and compound 39-2 (30 mg, Rt = 4.143 min).

Compound 39-1:

LC-Ms m/z (ESI): 706.7 [M+H]+
$^1$H NMR (400 MHz, $CD_3OD$) δ 7.85 (s, 1H), 6.83 (s, 1H), 6.67 (s, 2H), 5.72 - 5.63 (m, 2H), 3.78 (s, 3H), 3.11 - 2.99 (m, 2H), 2.98 - 2.86 (m, 5H), 2.75 - 2.62 (m, 7H), 2.48 - 2.40 (m, 2H), 2.09 - 2.00 (m, 2H), 1.99 - 1.94 (m, 2H), 1.92 (s, 3H), 1.85 (s, 3H), 1.47 - 1.33 (m, 1H), 0.99 - 0.87 (m, 6H).

Compound 39-2:

LC-Ms m/z (ESI): 706.7 [M+H]+
$^1$H NMR (400 MHz, $CD_3OD$) δ 7.80 (s, 1H), 6.90 (s, 1H), 6.69 (s, 2H), 5.86 - 5.79 (m, 1H), 5.65 - 5.59 (m, 1H), 3.79 (s, 3H), 3.28 - 3.10 (m, 2H), 3.02 - 2.93 (m, 4H), 2.83 - 2.72 (m, 7H), 2.58 - 2.44 (m, 3H), 2.13 - 2.01 (m, 2H), 1.97 - 1.87 (m, 7H), 1.79 - 1.70 (m, 1H), 1.41 - 1.29 (m, 1H), 0.93 - 0.83 (m, 6H).

**Example 40:** Preparation of compound 40

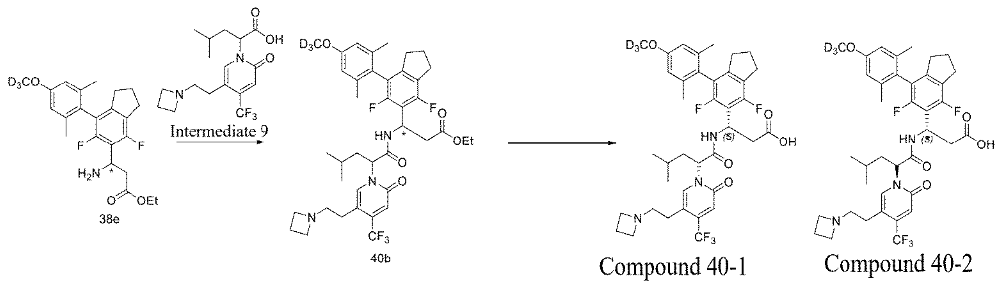


38e

40b

Compound 40-1 Compound 40-2

Referring to the preparation method of compound 38, crude compound 40 was obtained, which was separated and purified by prep-HPLC (method 1) to afford compound 40-1 (11 mg, Rt=4.152 min) and compound 40-2 (10 mg, Rt = 4.238 min).

Compound 40-1:

LC-Ms m/z (ESI): 721.7 $[M+H]^+$
$^1$H NMR (400 MHz, $CD_3OD$) δ 7.76 (s, 1H), 6.88 (s, 1H), 6.67 (s, 2H), 5.75 - 5.68 (m, 1H), 5.67 - 5.59 (m, 1H), 4.03 - 3.90 (m, 4H), 3.35 - 3.22 (m, 2H), 3.00 - 2.92 (m, 2H), 2.92 - 2.80 (m, 3H), 2.67 - 2.59 (m, 1H), 2.51 - 2.36 (m, 4H), 2.11 - 2.00 (m, 2H), 1.99 - 1.92 (m, 5H), 1.89 (s, 3H), 1.47 - 1.35 (m, 1H), 0.98 - 0.87 (m, 6H).

Compound 40-2:

LC-Ms m/z (ESI): 721.7 $[M+H]^+$
$^1$H NMR (400 MHz, $CD_3OD$) δ 7.66 (s, 1H), 6.92 (s, 1H), 6.69 (s, 2H), 5.89 (dd, 1H), 5.59 (t, 1H), 4.16 - 4.03 (s, 4H), 3.45 - 3.33 (m, 2H), 3.03 - 2.70 (m, 5H), 2.53 - 2.38 (m, 5H), 2.13 - 2.03 (m, 2H), 2.00 - 1.88 (m, 7H), 1.79 - 1.68 (m, 1H), 1.43 - 1.33 (m, 1H), 0.95 - 0.83 (m, 6H).

**Example 41:** Preparation of compound 41

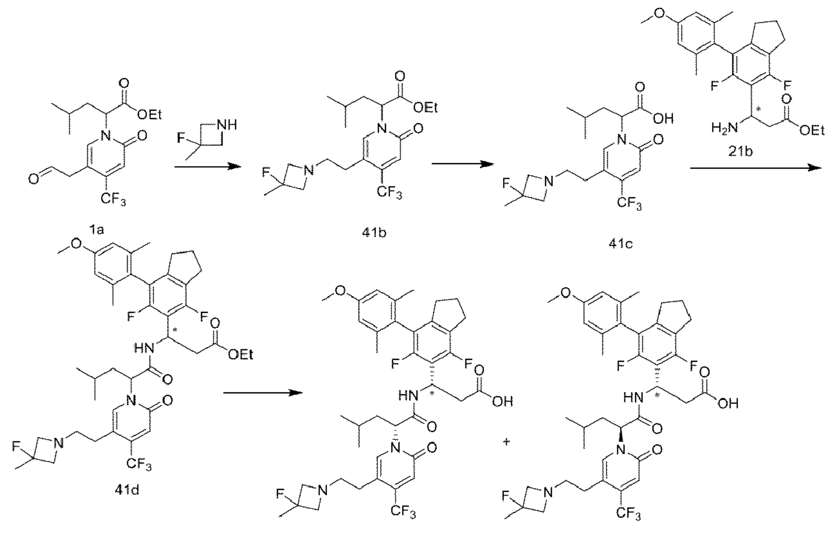


Compound 41-1 and Compound 41-2

Referring to the preparation method of compound 9, crude compound 41 was obtained, which was separated and purified by prep-HPLC (method 1) to afford compound 41-1 (20 mg, Rt=4.286 min) and compound 41-2 (25 mg, Rt = 4.371 min).

Compound 41-1:

LC-Ms m/z (ESI): 750.3 $[M+H]^+$

$^1$H NMR (400 MHz, $CD_3OD$) δ 7.80 (s, 1H), 6.80 (s, 1H), 6.69 - 6.63 (m, 2H), 5.74 - 5.66 (m, 2H), 3.78 (s, 3H), 3.73 - 3.47 (m, 4H), 3.05 - 2.87 (m, 5H), 2.82 - 2.74 (m, 1H), 2.73 - 2.60 (m, 2H), 2.48 - 2.40 (m, 2H), 2.09 - 1.98 (m, 2H), 1.98 - 1.88 (m, 5H), 1.83 (s, 3H), 1.63 - 1.52 (m, 3H), 1.47 - 1.36 (m, 1H), 0.99 - 0.90 (m, 6H).

Compound 41-2:

LC-Ms m/z (ESI): 750.3 $[M+H]^+$

$^1$H NMR (400 MHz, $CD_3OD$) δ 7.74 (s, 1H), 6.88 (s, 1H), 6.69 (s, 2H), 5.84 - 5.76 (m, 1H), 5.70 - 5.62 (m, 1H), 4.03 - 3.75 (m, 7H), 3.20 - 3.13 (m, 2H), 3.02 - 2.94 (m, 2H), 2.91 - 2.70 (m, 3H), 2.68 - 2.59 (m, 1H), 2.53 - 2.45 (m, 2H), 2.13 - 2.02 (m, 2H), 1.97 - 1.83 (m, 7H), 1.81 - 1.71 (m, 1H), 1.66 - 1.55 (m, 3H), 1.40 - 1.29 (m, 1H), 0.95 - 0.83 (m, 6H).

**Example 42:** Preparation of compound 42

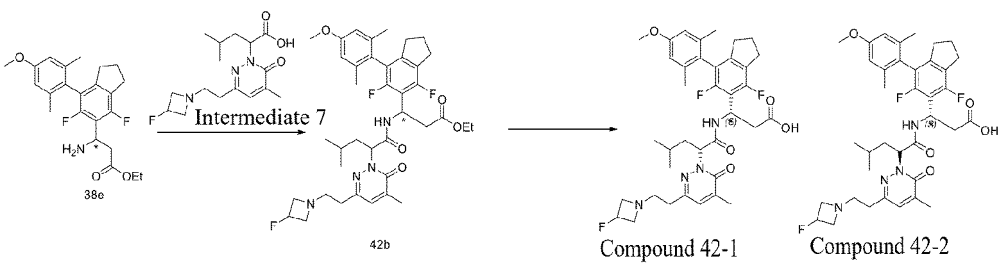


Compound 42-1 Compound 42-2

Referring to the preparation method of compound 35, crude compound 42 was obtained, which was separated and purified by prep-HPLC (method 1) to afford compound 42-1 (15 mg, Rt=4.071 min) and compound 42-2 (18 mg, Rt = 4.228 min).

Compound 42-1:

LC-Ms m/z (ESI): 683.3 [M+H]+

$^1$H NMR (400 MHz, $CD_3OD$) δ 7.29 (s, 1H), 6.69 (s, 2H), 5.56 (dd, 1H), 5.49 (t, 1H), 5.34 - 5.12 (m, 1H), 4.32 - 4.08 (m, 2H), 3.93 - 3.82 (m, 1H), 3.80 - 3.69 (m, 4H), 3.49 - 3.33 (m, 2H), 3.00 - 2.85 (m, 3H), 2.85 - 2.75 (m, 1H), 2.66 - 2.43 (m, 4H), 2.24 - 2.14 (m, 4H), 2.12 - 2.02 (m, 2H), 1.96 - 1.90 (m, 6H), 1.79 - 1.70 (m, 1H), 1.41 - 1.28 (m, 1H), 0.91 - 0.83 (m, 6H).

Compound 42-2:

LC-Ms m/z (ESI): 683.3 [M+H]+

$^1$H NMR (400 MHz, $CD_3OD$) δ 7.28 - 7.24 (m, 1H), 6.69 (s, 2H), 5.73 (dd, 1H), 5.66 - 5.59 (m, 1H), 5.40 - 5.17 (m, 1H), 4.43 - 4.26 (m, 2H), 4.10 - 3.88 (m, 2H), 3.79 (s, 3H), 3.49 - 3.40 (m, 2H), 3.02 - 2.83 (m, 4H), 2.70 - 2.60 (m, 1H), 2.55 - 2.44 (m, 3H), 2.21 - 2.15 (m, 3H), 2.14 - 2.02 (m, 3H), 1.96 - 1.91 (m, 6H), 1.88 - 1.78 (m, 1H), 1.41 - 1.27 (m, 1H), 0.91 - 0.82 (m, 6H).

**Example 43:** Preparation of compound 43

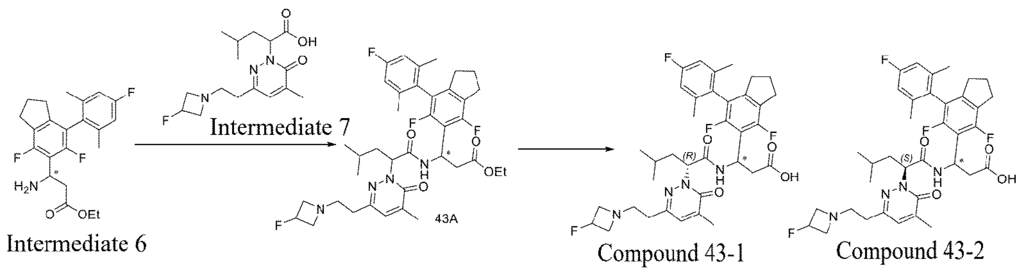


Referring to the preparation method of compound 35, crude compound 42 was obtained, which was separated and purified by prep-HPLC (method 1) to afford compound 43-1 (98 mg, Rt=4.126 min) and compound 43-2 (103 mg, Rt = 4.303 min).

Compound 43-1:

LC-Ms m/z (ESI): 671.3 [M+H]+

$^1$H NMR (400 MHz, $CD_3OD$) δ 7.32 - 7.27 (m, 1H), 6.92 - 6.82 (m, 2H), 5.60 - 5.47 (m, 2H), 5.33 - 5.08 (m, 1H), 4.19 - 3.96 (m, 2H), 3.81 - 3.61 (m, 2H), 3.37 - 3.23 (m, 2H), 3.02 - 2.92 (m, 2H), 2.90 - 2.70 (m, 2H), 2.67 - 2.54 (m, 2H), 2.52 - 2.41 (m, 2H), 2.24 - 2.13 (m, 4H), 2.13 - 2.02 (m, 2H), 1.99 - 1.94 (m, 6H), 1.80 - 1.70 (m, 1H), 1.42 - 1.26 (m, 1H), 0.92 - 0.81 (m, 6H).

Compound 43-2:

LC-Ms m/z (ESI): 671.3 [M+H]+

$^1$H NMR (400 MHz, $CD_3OD$) δ 7.29 - 7.25 (m, 1H), 6.90 - 6.84 (m, 2H), 5.76 - 5.69 (m, 1H), 5.65 - 5.58 (m, 1H), 5.41 - 5.19 (m, 1H), 4.44 - 4.31 (m, 2H), 4.12 - 3.92 (m, 2H), 3.50 - 3.44 (m, 2H), 3.02 - 2.84 (m, 4H), 2.70 - 2.61 (m, 1H), 2.54 - 2.45 (m, 3H), 2.20 - 2.16 (m, 3H), 2.14 - 2.03 (m, 3H), 1.99 - 1.93 (m, 6H), 1.88 - 1.79 (m, 1H), 1.40 - 1.27 (m, 1H), 0.90 - 0.83 (m, 6H).

**Example 44:** Preparation of compound 44

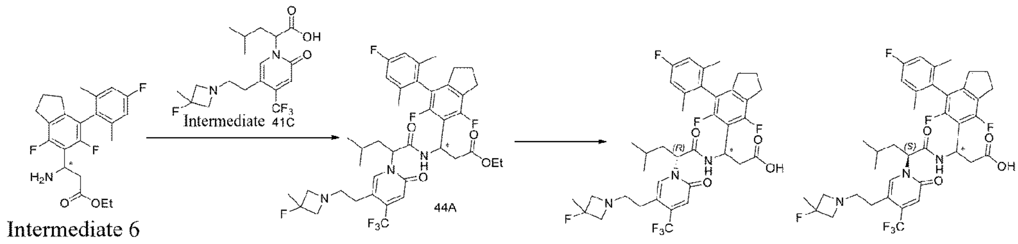


Intermediate 6 44A Compound 44-1 Compound 44-2

Referring to the preparation method of compound 35, crude compound 44 was obtained, which was separated and purified by prep-HPLC (method 1) to afford compound 44-1 (84 mg, Rt=4.315 min) and compound 44-2 (96 mg, Rt = 4.398 min).

Compound 44-1:

LC-Ms m/z (ESI): 738.3 $[M+H]^+$
$^1H$ NMR (400 MHz, $CD_3OD$) δ 7.79 (s, 1H), 6.88 - 6.80 (m, 2H), 6.79 (s, 1H), 5.74 - 5.64 (m, 2H), 3.63 - 3.38 (m, 4H), 3.02 - 2.90 (m, 3H), 2.88 - 2.80 (m, 2H), 2.80 - 2.71 (m, 1H), 2.71 - 2.57 (m, 2H), 2.49 - 2.39 (m, 2H), 2.10 - 2.01 (m, 2H), 2.00 - 1.91 (m, 5H), 1.85 - 1.82 (m, 3H), 1.56 (d, 3H), 1.47 - 1.36 (m, 1H), 0.99 - 0.89 (m, 6H).

Compound 44-2:

LC-Ms m/z (ESI): 738.3 $[M+H]^+$
$^1H$ NMR (400 MHz, $CD_3OD$) δ 7.74 (s, 1H), 6.91 - 6.84 (m, 3H), 5.83 - 5.76 (m, 1H), 5.70 - 5.61 (m, 1H), 4.02 - 3.75 (m, 4H), 3.20 - 3.13 (m, 2H), 3.04 - 2.94 (m, 2H), 2.91 - 2.70 (m, 3H), 2.69 - 2.60 (m, 1H), 2.53 - 2.44 (m, 2H), 2.14 - 2.03 (m, 2H), 1.98 (s, 6H), 1.94 - 1.84 (m, 1H), 1.83 - 1.71 (m, 1H), 1.63 - 1.58 (m, 3H), 1.42 - 1.28 (m, 1H), 0.94 - 0.84 (m, 6H).

**Example 45:** Preparation of compound 45

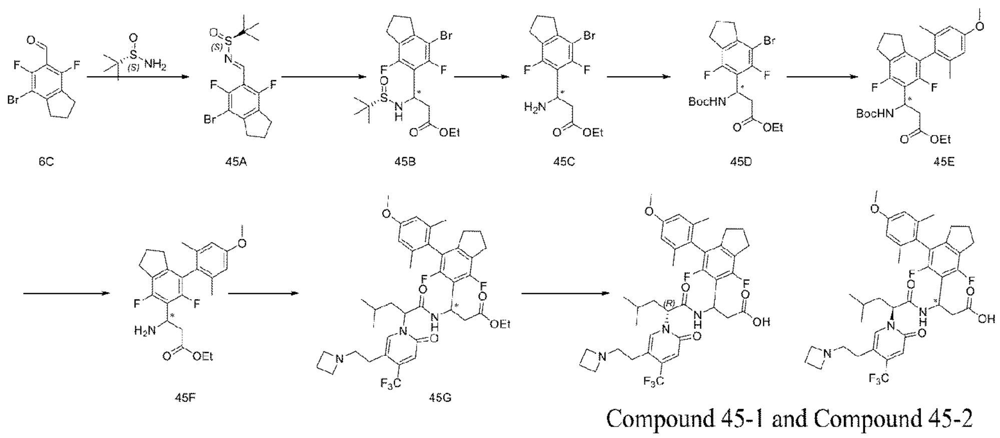

6C 45A 45B 45C 45D 45E 45F 45G

Compound 45-1 and Compound 45-2

Taking (S)-tert-butylsulfinamide and 6c as starting materials, and referring to the preparation method of intermediate 6 and compound 35, crude compound 45 was obtained, which was separated and purified by prep-HPLC (method 1) to afford compound 45-1 (5 mg, retention time=3.918 min) and compound 45-2 (6 mg, retention time = 4.001

min).

Compound 45-1: LC-Ms m/z (ESI): 718.3 [M+H]+
Compound 45-2: LC-Ms m/z (ESI): 718.3 [M+H]+

**Example 46:** Synthesis of compound 46

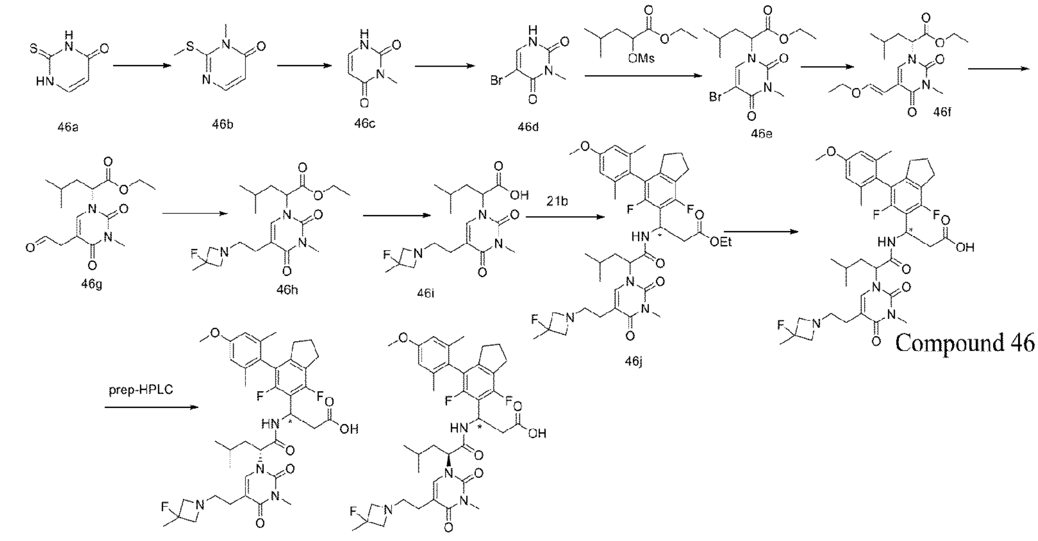

46a 46b 46c 46d 46e 46f 46g 46h 46i 46j Compound 46

46-1 and 46-2

Step 1: Synthesis of 46b

At room temperature, 2-thiouracil (10 g, 78.03 mmoL) and potassium carbonate (32 g, 234.02 mmoL) were dissolved in tetrahydrofuran solution (500 mL), iodomethane (15 mL, 234.04 mmoL) was added, and the resulting mixture was reacted for 3 hours. Water was added, and the reaction solution was extracted with ethyl acetate (50 mL× 3 times). The organic phases were combined, dried over anhydrous sodium sulfate and filtered. The filtrate was concentrated under reduced pressure and the residue was purified by silica gel column chromatography to afford 46b (6.8 g, yield: 55.79%).
LC-Ms m/z (ESI): 157.2 [M+H]+

Step 2: Synthesis of 46c

At room temperature, 46b (6.8 g, 43.54 mmoL) was dissolved in a mixed solution of 1,4-dioxane and water (5:1, 200 mL), potassium peroxymonosulfonate (40.15 g, 65.31 mmoL) was added, and the mixture was reacted at 100°C for 4 hours. Water was added, and the reaction solution was extracted with ethyl acetate (50 mL× 3 times). The organic phases were combined, dried over anhydrous sodium sulfate and filtered. The filtrate was concentrated under reduced pressure and the residue was purified by silica gel column chromatography to afford compound 46c (4.9 g, yield: 89.24%).
LC-Ms m/z (ESI): 127.2 [M+H]+

Step 3: Synthesis of 46d

At room temperature, 46c (4.9 g, 38.87 mmoL) was dissolved in 50 mL acetonitrile, N-bromosuccinimide (6.92 g, 38.87 mmoL) and glacial acetic acid (0.22 mL, 3.85 mmoL) were separately added, and the resulting mixture was reacted for 4 hours. Sodium bicarbonate aqueous solution was added to quench the reaction, and the reaction solution was extracted with ethyl acetate (50 milliliter× 3 times). The organic phases were combined, dried over anhydrous sodium sulfate and filtered. The filtrate was concentrated under reduced pressure and the residue was purified by silica gel column chromatography to afford compound 46d (5.5 g, yield: 69.02%).
LC-Ms m/z (ESI): 206.1 [M+H]+

Step 4: Synthesis of 46e

At room temperature, 46d (5.5 g, 26.83 mmoL), and intermediate ethyl 2-(methanesulfonyloxy)-4-methylpentanoate (7.67 g, 32.20 mmoL) were dissolved in acetonitrile (80 mL), and potassium carbonate (11.12 g, 80.53mmoL) was added. The reaction system was then warmed to 80°C and allowed to react overnight. The reaction liquid was filtered over celite, and extracted with ethyl acetate (30 mL× 3 times). The organic phases were combined, dried over anhydrous sodium sulfate and filtered. The filtrate was concentrated under reduced pressure and the residue was purified by silica gel column chromatography to afford compound 46e (4.7 g, yield: 50.45%).
LC-Ms m/z (ESI): 348.2 $[M+H]^+$

Step 5: Synthesis of 46f

46e (4.7 g, 13.54 mmoL), and (E)-1-ethoxyvinyl-2-boronic acid pinacol ester (3.22 g, 16.25 mmoL) were dissolved in 1,4-dioxane and water (v/v=10:1, 110 mL). Potassium carbonate (5.61 g, 40.62 mmoL) and tetrakistriphenylphosphine palladium (1.56 g, 1.35 mmoL) were added, and the reaction system was subjected to nitrogen replacement 3 times and reacted at 70°C for 24 hours. Water was added to quench the reaction, and the reaction solution was extracted with ethyl acetate (30 mL× 3 times). The organic phases were combined, washed with saturated sodium chloride aqueous solution (50 mL), dried over anhydrous sodium sulfate and filtered. The filtrate was concentrated under reduced pressure and the residue was purified by silica gel column chromatography to afford 46f (1.8 g, yield: 39.28%).
LC-Ms m/z (ESI): 339.4 $[M+H]^+$

Step 6: Synthesis of compound 46g

46f (1.8 g, 5.32 mmoL) was dissolved in dichloromethane (20 mL), trifluoroacetic acid (10 mL, 134.19 mmoL) was added slowly and the reaction was allowed to proceed overnight at room temperature. The reaction solution was concentrated under reduced pressure, the pH was adjusted to neutral by adding aqueous sodium bicarbonate solution, and the reaction solution was extracted with ethyl acetate (20 mL× 2). The organic phases were combined, dried over anhydrous sodium sulfate, filtered and concentrated under reduced pressure to obtain crude 46g, which was directly used in the next reaction without purification.

Steps 7 to 10: Synthesis of compound 46

Taking 46g as starting material and referring to the synthetic route and preparation method of compound 3, crude compound 46 was obtained, which was separated and purified by prep-HPLC (preparation method 1) to afford compound 46-1 (20 mg, retention time=4.122 min) and compound 46-2 (18 mg, retention time = 4.250 min).

Compound 46-1:

LC-Ms m/z (ESI): 713.3 $[M+H]^+$
$^1$H NMR (400 MHz, $CD_3OD$) δ 7.70 (s, 1H), 6.68 (s, 2H), 5.73 - 5.65 (m, 1H), 5.45 - 5.37 (m, 1H), 4.47 - 4.26 (m, 4H), 3.79 (s, 3H), 3.56 - 3.38 (m, 2H), 3.25 (s, 3H), 3.12 - 3.02 (m, 1H), 2.99 - 2.91 (m, 2H), 2.89 - 2.81 (m, 1H), 2.80 - 2.71 (m, 1H), 2.66 - 2.56 (m, 1H), 2.52 - 2.39 (m, 2H), 2.10 - 2.01 (m, 2H), 2.00 - 1.90 (m, 4H), 1.89 - 1.75 (m, 4H), 1.71 - 1.61 (m, 3H), 1.54 - 1.43 (m, 1H), 1.00 - 0.91 (m, 6H).

Compound 46-2:

LC-Ms m/z (ESI): 713.3 $[M+H]^+$
$^1$H NMR (400 MHz, $CD_3OD$) δ 7.71 (s, 1H), 6.70 (s, 2H), 5.74 - 5.64 (m, 1H), 5.48 - 5.40 (m, 1H), 4.52 - 4.26 (m, 4H), 3.79 (s, 3H), 3.58 - 3.38 (m, 2H), 3.32 (s, 3H), 3.09 - 3.01 (m, 1H), 3.00 - 2.93 (m, 2H), 2.91 - 2.72 (m, 2H), 2.66 - 2.56 (m, 1H), 2.53 - 2.44 (m, 2H), 2.12 - 2.01 (m, 2H), 1.94 (s, 6H), 1.83 - 1.73 (m, 1H), 1.71 - 1.60 (m, 4H), 1.45 - 1.32 (m, 1H), 0.95 - 0.85 (t, 6H).

Example 47 Preparation of compound 47

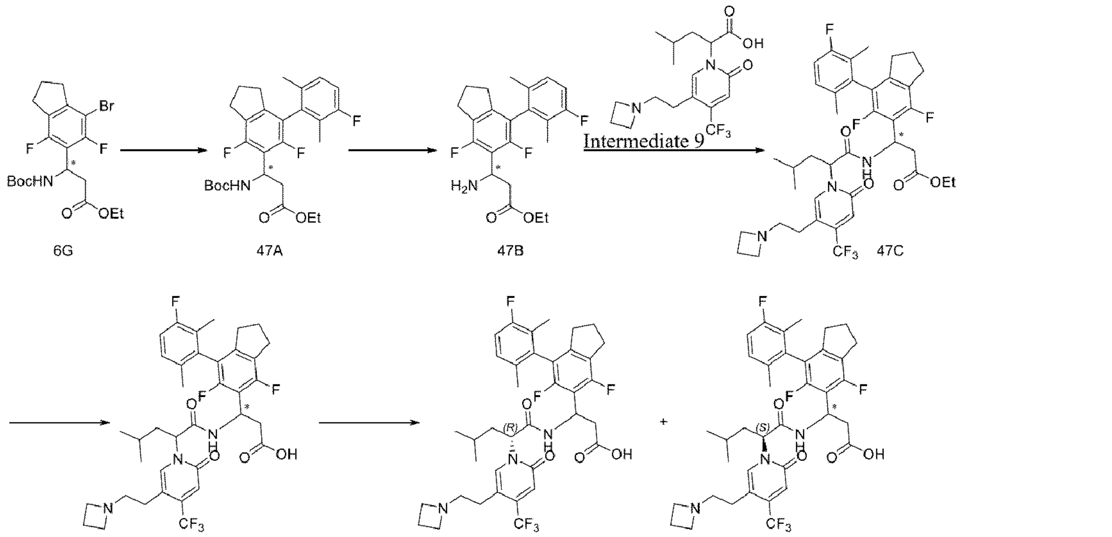


6G 47A 47B 47C

Compound 47

Compound 47-1 and Compound 47-2

Referring to the synthetic route and preparation method of compound 35, crude compound 47 was obtained, which was separated and purified by prep-HPLC (preparation method 2) to afford compound 47-1 (40 mg, retention time=4.179 min) and compound 47-2 (45 mg, retention time = 4.271 min).

Trifluoroacetate salt of compound 47-1:

LC-Ms m/z (ESI): 706.3 $[M+H]^+$
$^1$H NMR (400 MHz, $CD_3OD$) δ 9.26 (d, 1H), 7.86 (s, 1H), 7.15 - 7.05 (m, 1H), 7.02 - 6.93 (m, 1H), 6.82 (s, 1H), 5.85 - 5.65 (m, 2H), 4.42 - 4.03 (m, 4H), 3.44 - 3.33 (m, 2H), 3.15 - 3.05 (m, 1H), 3.00 - 2.78 (m, 5H), 2.67 - 2.53 (m, 1H), 2.51 - 2.36 (m, 3H), 2.13 - 1.99 (m, 3H), 1.97 - 1.76 (m, 7H), 1.47 - 1.35 (m, 1H), 1.00 - 0.90 (m, 6H).

Trifluoroacetate salt of compound 47-2:

LC-Ms m/z (ESI): 706.3 [M+H]+
$^1$H NMR (400 MHz, $CD_3OD$) δ 9.38 (d, 1H), 7.88 (s, 1H), 7.17 - 7.10 (m, 1H), 7.04 - 6.96 (m, 1H), 6.88 (s, 1H), 5.83 - 5.66 (m, 2H), 4.42 - 4.05 (m, 4H), 3.50 - 3.33 (m, 2H), 3.11 - 2.95 (m, 3H), 2.95 - 2.80 (m, 3H), 2.71 - 2.56 (m, 1H), 2.53 - 2.39 (m, 3H), 2.15 - 2.04 (m, 2H), 1.95 (s, 3H), 1.90 (d, 3H), 1.87 - 1.71 (m, 2H), 1.37 - 1.23 (m, 1H), 0.98 - 0.83 (m, 6H).

**Example 48:** Preparation of compound 48

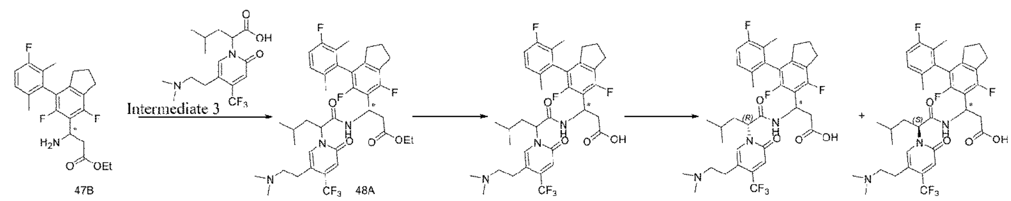


47B 48A

Compound 48

Compound 48-1 and Compound 48-2

Referring to the synthetic route and preparation method of compound 35, crude compound 48 was obtained, which was separated and purified by prep-HPLC (preparation method 2) to afford compound 48-1 (54 mg, retention time=4.179 min) and compound 48-2 (62 mg, retention time = 4.271 min).

Trifluoroacetate salt of compound 48-1:

LC-Ms m/z (ESI): 694.3 $[M+H]^+$
$^1$H NMR (400 MHz, $CD_3OD$) δ 9.23 (d, 1H), 7.90 (s, 1H), 7.13 - 7.04 (m, 1H), 7.01 - 6.91 (m, 1H), 6.82 (s, 1H), 5.82 - 5.74 (m, 1H), 5.73 - 5.64 (m, 1H), 3.36 - 3.22 (m, 2H), 3.15 - 2.83 (m, 12H), 2.52 - 2.36 (m, 2H), 2.13 - 1.98 (m, 3H), 1.97 - 1.74 (m, 7H), 1.48 - 1.35 (m, 1H), 1.01 - 0.94 (m, 6H).

Trifluoroacetate salt of compound 48-2:

LC-Ms m/z (ESI): 694.3 $[M+H]^+$
$^1$H NMR (400 MHz, $CD_3OD$) δ 9.35 (d, 1H), 7.92 (s, 1H), 7.16 - 7.08 (m, 1H), 7.04 - 6.94 (m, 1H), 6.88 (s, 1H), 5.82 - 5.64 (m, 2H), 3.39 - 3.24 (m, 2H), 3.10 - 2.84 (m, 12H), 2.55 - 2.45 (m, 2H), 2.16 - 2.03 (m, 2H), 1.95 (s, 3H), 1.90 (d, 3H), 1.87 - 1.71 (m, 2H), 1.38 - 1.23 (m, 1H), 0.98 - 0.81 (m, 6H).

**Example 49:** Preparation of compound 49

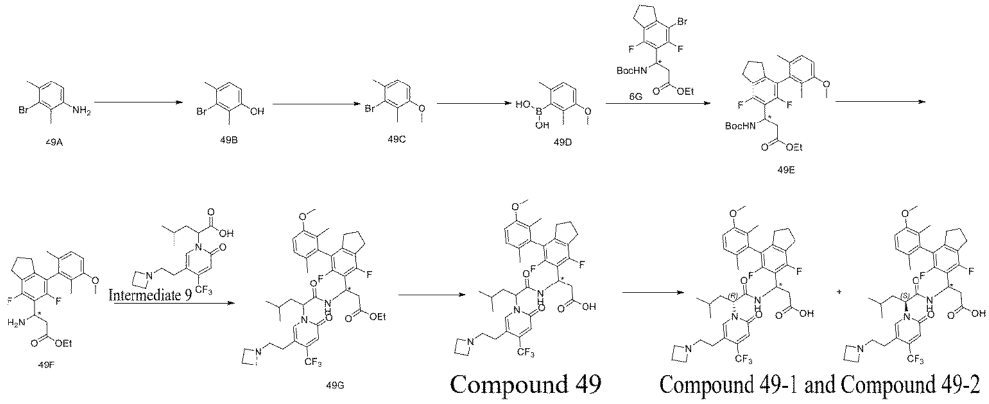


Step 1: Synthesis of compound 49B

Substrate 49A (5.00 g, 24.99 mmo) was dissolved in 10% aqueous sulfuric acid (50 mL) under ice bath conditions, then sodium nitrite (1.72 g, 24.99 mmol) was dissolved in water (10 mL) and then added dropwise to the system. After the addition was complete, the reaction was continued under ice bath conditions for 1 hour; 50% aqueous sulfuric acid solution (50 mL) was added dropwise to the system, and after the addition was complete, the reaction system was warmed to 100°C and reacted for 1 hour. The reaction system was cooled to room temperature, and the reaction liquid was slowly added to a stirred ice water (300 mL). The stirring was continued for 30 minutes, and the aqueous phase was extracted with ethyl acetate (50 mL×3). The organic phases were combined, dried over anhydrous sodium sulfate, filtered and concentrated to afford a crude product, which was purified by silica gel column chromatography to afford 49B (2.89 g, yield: 57.52%).

Step 2: Synthesis of compound 49C

Substrate 49B (2.89 g, 14.37 mmo) was dissolved in acetonitrile (30 mL), and potassium carbonate (3.97 g, 28.72 mmol) and iodomethane (3.06 g, 21.55 mmol) were added. After the addition was complete, the reaction system was warmed to 60°C and reacted for 18 hours under nitrogen atmosphere. The reaction was cooled to room temperature and filtered, and the filter cake was washed with ethyl acetate (5 mL×2). The filtrate was collected and concentrated to obtain a

crude product, which was purified by silica gel column chromatography to afford 49C (2.65 g, yield: 85.74%).

Step 3: Synthesis of compound 49D

Substrate 49C (2.65 g, 12.32 mmol) was dissolved in ultra-dry tetrahydrofuran (40 mL), and pre-cooled under nitrogen atmosphere at -78°C for 10 minutes. N-butyllithium (0.89 g, 13.91 mmol) was slowly added dropwise, and after the addition was complete, stirring was continued at -78°C for 1 hour. Trimethyl borate (1.71 g, 16.47 mmol) was added dropwise to the system, and after the addition was complete, the reaction system was naturally warmed to room temperature and reacted for 18 hours under nitrogen atmosphere. The reaction was poured into 10% aqueous hydrochloric acid solution (50 mL) under ice bath conditions, stirred for 30 minutes under ice bath conditions, and the reaction solution was extracted with ethyl acetate (30 mL 3). The organic phases were combined, dried over anhydrous sodium sulfate, filtered and concentrated to afford a crude product, which was purified by silica gel column chromatography to afford 49D (0.84 g, yield: 37.88%).

Referring to the synthetic route and preparation method of compound 35, crude compound 49 was obtained, which was separated and purified by prep-HPLC (preparation method 1) to afford compound 49-1 (32 mg, retention time=4.218 min) and compound 49-2 (35 mg, retention time = 4.303 min).

Compound 49-1:

LC-Ms m/z (ESI): 718.3 $[M+H]^+$

$^1$H NMR (400 MHz, $CD_3OD$) δ 7.76 (s, 1H), 7.06 (d, 1H), 6.88 (s, 1H), 6.85 (d, 1H), 5.75 - 5.68 (m, 1H), 5.67 - 5.59 (m, 1H), 4.05 - 4.39 (m, 4H), 3.82 (s, 3H), 3.37 - 3.31 (m, 2H), 2.96 (t, 2H), 2.92 - 2.82 (m, 3H), 2.69 - 2.60 (m, 1H), 2.49 - 2.36 (m, 4H), 2.12 - 2.01 (m, 2H), 2.00 - 1.92 (m, 2H), 1.89 - 1.76 (m, 6H), 1.47 - 1.34 (m, 1H), 0.97 - 0.87 (m, 6H).

Compound 49-2:

LC-Ms m/z (ESI): 718.3 [M+H]+

$^1$H NMR (400 MHz, $CD_3OD$) δ 7.66 (s, 1H), 7.08 (d, 1H), 6.92 (s, 1H), 6.86 (d, 1H), 5.92 - 5.86 (m, 1H), 5.60 (t, 1H), 4.11 (t, 4H), 3.83 (s, 3H), 3.46 - 3.32 (m, 2H), 3.03 - 2.87 (m, 3H), 2.86 - 2.71 (m, 2H), 2.53 - 2.39 (m, 5H), 2.13 - 2.02 (m, 2H), 1.99 - 1.92 (m, 1H), 1.91 - 1.81 (m, 6H), 1.78 - 1.67 (m, 1H), 1.43 - 1.32 (m, 1H), 0.93 - 0.84 (m, 6H).

**Example 50:** Preparation of compound 50

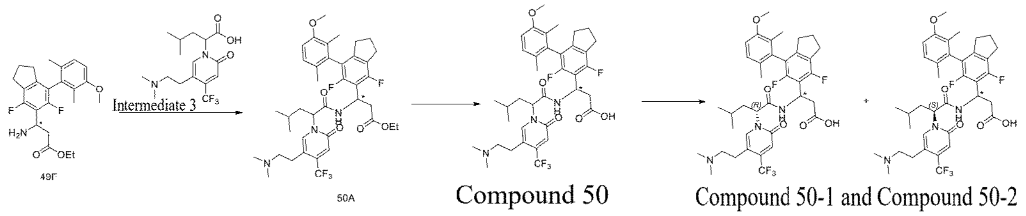


49F 50A Compound 50 Compound 50-1 and Compound 50-2

Referring to the synthetic route and preparation method of compound 35, crude compound 50 was obtained, which was separated and purified by prep-HPLC (preparation method 1) to afford compound 50-1 (35 mg, retention time=4.184 min) and compound 50-2 (37 mg, retention time = 4.260 min).

Compound 50-1:

LC-Ms m/z (ESI): 706.4 $[M+H]^+$

1H NMR (400 MHz, $CD_3OD$) δ 7.85 (s, 1H), 7.08 - 7.02 (m, 1H), 6.87 - 6.81 (m, 2H), 5.72 - 5.63 (m, 2H), 3.82 (s, 3H), 3.14 - 3.00 (m, 2H), 2.99 - 2.87 (m, 5H), 2.71 (m, 6H), 2.70 - 2.63 (m, 1H), 2.46 - 2.39 (m, 2H), 2.09 - 2.00 (m, 2H), 1.99 -

1.91 (m, 2H), 1.89 - 1.72 (m, 6H), 1.47 - 1.35 (m, 1H), 0.97 - 0.88 (m, 6H).

Compound 50-2:

LC-Ms m/z (ESI): 706.4 $[M+H]^+$
1H NMR (400 MHz, $CD_3OD$) δ 7.79 (s, 1H), 7.08 (d, 1H), 6.90 (s, 1H), 6.86 (d, 1H), 5.87 - 5.78 (m, 1H), 5.61 (t, 1H), 3.83 (s, 3H), 3.25 - 3.11 (m, 2H), 3.03 - 2.92 (m, 4H), 2.83 - 2.72 (m, 7H), 2.58 - 2.50 (m, 1H), 2.46 (t, 2H), 2.14 - 2.02 (m, 2H), 1.98 - 1.91 (m, 1H), 1.90 - 1.81 (m, 6H), 1.79 - 1.69 (m, 1H), 1.42 - 1.31 (m, 1H), 0.94 - 0.81 (m, 6H).

**Biological test:**

**1. ELISA detection assay for integrin α4β7:**

MAdCAM was formulated with TBS buffer to a final concentration of 2.5 ug/ml, 50 ul of the solution was transferred to a 96-well plate, and the plate was coated with same at 4°C overnight. The plate was washed with TBS buffer three times, then 150 ul of a blocking solution (TBS buffer containing 1% BSA) was added, and the plate was blocked at 37°C for 1 h. The plate was washed with TBS buffer three times. 1 ug/ml α4β7 integrin was formulated using TBS buffer containing 0.1% BSA. 50 ul of integrin was transferred to a 96-well plate, then 1 ul of the compound at different concentrations or DMSO was added, and the plate was incubated at normal temperature for 2 h. 1 ug/ml biotinylated anti-β7 antibody was formulated using TBS buffer containing 0.1% BSA. The plate was washed with TBS buffer three times, 50 ul of the antibody was added, and the plate was incubated at normal temperature for 1 h. The plate was washed with TBS buffer three times, 50 ul of streptavidin-HRP was added, and the plate was incubated at normal temperature for 20 min. The plate was washed with TBS buffer three times, 50 ul of a TMB substrate was added, and the plate was incubated at normal temperature for 5-30 min. Finally, 25 ul of a termination buffer (phosphate solution at a high concentration) was added. The OD value of the plate was read at 450 nm with a microplate reader. The $IC_{50}$ value was calculated using GraphPad Prism 6 software.

Conclusion: The compounds of the present invention, for example, the compounds of the Examples have good inhibitory activity against **integrin α4β7**.

2. **Integrin α4β7 cell adhesion assay**

MAdCAM-1 was formulated with TBS buffer to a final concentration of 2 μg/ml, 50 μl of the solution was transferred to a 96-well plate, and the plate was coated with same at 4°C overnight. The plate was washed with TBS buffer three times, then 150 μl of a blocking solution (TBS buffer containing 1% BSA) was added, and the plate was blocked at 37°C for 1 h. RPMI8866 cells were collected, washed twice with DPBS buffer, and then resuspended to $4\times10^5$ cells/ml with TBS buffer. 50 μl of the cell suspension was transferred to a 96-well plate so that the cell density reached $2\times10^5$ cells/well. Then 1 μl of the compound at different concentrations or DMSO was added and the plate was incubated at 37°C for 1 h or 2 h. The plate was washed with TBS buffer to remove non-adherent cells. 50 μl of a substrate (4-nitrophenyl-N-acetyl-β-D-glucosaminide) was added and the plate was incubated at 37°C for 2 h. Finally, 90 μl of a termination solution (50 mM glycine and 5 mM EDTA, pH 10.4) was added. The OD value of the plate was read at 405 nm with a microplate reader. The $IC_{50}$ value was calculated using GraphPad Prism 6 software. The results are shown in Table 1.

**3. Integrin α4β1-mediated cell adhesion assay**

VCAM was formulated into a final concentration of 0.5 μg/ml with a coating buffer, 50 μl of the solution was transferred to a 96-well plate, and the plate was coated with same at 4°C overnight. The plate was washed with coating buffer three times, then 150 μl of a blocking solution (coating buffer containing 1% BSA) was added, and the plate was blocked at 37°C for 1 h. Jurkat cells were collected, washed twice using DPBS buffer, and then resuspended to $4 \times 10^6$ cells/ml with an action buffer. 50 μl of the cell suspension was transferred to a 96-well plate so that the cell density reached $2 \times 10^5$ cells/well. Then 1 μl of the test compound at different concentration or DMSO was added and the plate was incubated at 37°C for 1 h. The plate was washed with coating buffer to remove non-adherent cells. 50 μl of a substrate (4-nitrophenyl-N-acetyl-β-D-glucosaminide) was added and the plate was incubated at 37°C for 2 h. Finally, 90 μl of a termination solution (50 mM glycine and 5 mM EDTA, pH 10.4) was added. The OD value of the plate was read at 405 nm.

Coating buffer: DMEM+ 20 mM HEPES+ 0.4 mM $MnCl_2$
Action buffer: DMEM+ 20 mM HEPES+ 0.1% BSA+0.4 mM $MnCl_2$

DMEM: dulbecco's modified eagle medium;
HEPES: 2-[4-(2-hydroxyethyl)piperazin-1-yl]ethanesulfonic acid

Table 1 Results of effects of compounds on α4β7- and α4β1-mediated cell adhesion

| Compound No. | RPMI-8866 (α4β7)/MadCAM-1 | Jurkat (α4β1)/VCAM-1 | α4β1/α4β7 |
|---|---|---|---|
| | $IC_{50}$ (nM) | IC50 (nM) | |
| Compound 1-2 | A | >100 | >50 |
| Trifluoroacetate salt of compound 2-2 | A | >300 | 106 |
| Compound 3-2 | A | / | / |
| Trifluoroacetate salt of compound 4-2 | A | >500 | 648 |
| Compound 6-2 | A | / | / |
| Compound 7-2 | A | >500 | 182 |
| Trifluoroacetate salt of compound 9-2 | <0.5 | >30 | 269 |
| Trifluoroacetate salt of compound 10-2 | <0.5 | >100 | 1423 |
| Trifluoroacetate salt of compound 11-2 | <0.5 | >100 | 269 |
| Trifluoroacetate salt of compound 12-2 | <0.5 | >100 | 354 |
| Trifluoroacetate salt of compound 14-2 | <0.5 | >100 | 454 |
| Trifluoroacetate salt of compound 15-2 | <1 | >100 | 272 |
| Trifluoroacetate salt of compound 16-2 | <1 | >200 | 308 |
| Trifluoroacetate salt of compound 17-2 | <1 | >100 | 165 |
| Trifluoroacetate salt of compound 18-2 | A | >500 | 258 |
| Trifluoroacetate salt of compound 19-2 | A | >500 | 323 |
| Trifluoroacetate salt of compound 20-2 | A | >500 | 648 |
| Trifluoroacetate salt of compound 21-2 | <1 | >500 | 1586 |
| Trifluoroacetate salt of compound 22-2 | <0.5 | >100 | 307 |
| Trifluoroacetate salt of compound 23-2 | A | >500 | 310 |
| Compound 24-2 | A | / | / |
| Compound 25-2 | <0.5 | >50 | 194 |
| Trifluoroacetate salt of compound 26-2 | <1 | >100 | 177 |
| Trifluoroacetate salt of compound 27-2 | A | >100 | 133 |
| Trifluoroacetate salt of compound 28-2 | A | >100 | 130 |
| Trifluoroacetate salt of compound 29-2 | <1 | >200 | 292 |
| Trifluoroacetate salt of compound 32-2 | <1 | >100 | 220 |
| Compound 33-2 | <0.5 | >100 | 643 |
| Compound 35-2 | <0.5 | >300 | 3651 |
| Compound 36-2 | 0.5 | >100 | 240 |
| Compound 38-2 | <0.5 | >100 | 862 |
| Compound 39-2 | A | >500 | 213 |
| Compound 21-A | A | >100 | 119 |
| Compound 40-2 | <0.5 | >300 | 1143 |
| Compound 43-2 | <1 | 90.7 | >100 |

(continued)

| Compound No. | RPMI-8866 (α4β7)/MadCAM-1 | Jurkat (α4β1)/VCAM-1 | α4β1/α4β7 |
|---|---|---|---|
| | $IC_{50}$ (nM) | IC50 (nM) | |
| Compound 46-2 | A | >4000 | >1200 |
| Trifluoroacetate salt of compound 48-2 | <1 | | |
| Compound 49-2 | A | | |
| Compound 50-2 | A | | |
| Control compound A | <0.5 | <5 | 50.4 |
| A < 10 nM; control compound A is 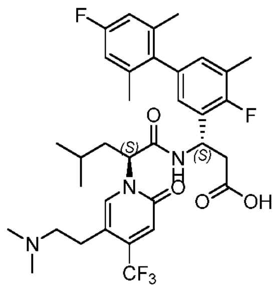 | | | |

Conclusion: The compounds of the present invention, such as the compounds of the Examples, have a good inhibitory effect on integrin α4β7-mediated cell adhesion and are more selective than the control compound A.

**4. CYP450 enzyme inhibition test**

The purpose of this study was to evaluate the effect of the test compound on the activity of five isoenzymes (CYP1A2, CYP2C9, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and IC50 value was calculated to evaluate the inhibitory potential of the test compound on each CYP enzyme subtype CYP1A2, CYP2C9, CYP2D6, or CYP3A4-M (midazolam was used as the substrate).

Conclusion: The compounds of the present invention, such as the compounds of the Examples, have no significant inhibitory effect on various subtypes of CYP enzyme.

**5. Pharmacokinetic test in mice**

**Experimental animals:** male BALB/c mice, 20-25 g, 6 mice/compound. purchased from Chengdu Ddossy Experimental Animals Co., Ltd.

**Experimental design:** on the day of the test, 6 BALB/c mice were randomly grouped according to their body weights. The animals were fasted with water available for 12 to 14 h one day before the administration and were fed 4 h after the administration.

Table 2. Administration information

| Group | Number | Administration information | | | | | |
|---|---|---|---|---|---|---|---|
| | Male | Test compound | Administra tion dosage (mg/kg) | Administra tion concentrati on (mg/mL) | Administra tion volume (mL/kg) | Collected samples | Mode of administratio n |
| G1 | 3 | Compound | 1 | 0.2 | 5 | Plasma | Intravenous administratio n |
| G2 | 3 | | 10 | 1 | 10 | Plasma | Intragastric administratio n |
| Note: solvent for intravenous administration: 5% DMA + 5% Solutol + 90% Saline; solvent for intragastric administration: 5% DMSO+95% (20% SBE-β-CD in saline)<br>(DMA: dimethylacetamide; Solutol: polyethylene glycol-15-hydroxystearate; Saline: physiological saline; DMSO: dimethyl sulfoxide; SBE-β-CD: sulfobutyl-β-cyclodextrin) | | | | | | | |

Before and after the administration, 0.06 mL of blood was taken from the orbit of the animals under isoflurane anesthesia and placed in an EDTAK2 centrifuge tube. The blood was centrifuged at 5000 rpm and 4°C for 10 min to collect plasma. The blood collection time points for the intravenous group and intragastric group were both 0 min, 5 min, 15 min, 30 min, 1 h, 2 h, 4 h, 6 h, 8 h, and 24 h. Before analysis and detection, all samples were stored at -80°C, and the samples were quantitatively analyzed by LC-MS/MS.

Table 3

| Compound No. | IV: 1 mg/kg | PO: 10 mg/kg | |
|---|---|---|---|
| | CL ($mL \cdot kg^{-1} \cdot min^{-1}$) | AUC ($h \cdot ng \cdot mL^{-1}$) | F |
| Compound 21-2 | 22.6 | 1380 | / |
| Compound 35-2 | 13.3 | 1259 | / |
| Compound 36-2 | 14.0 | 5501 | 45.9% |
| Control compound A | 56.0 | 830 | 28.2% |

Conclusion: The compounds of the present invention, such as the compounds of the Examples, have good oral absorption performance and lower clearance rate in mice.

6. Pharmacokinetic test in rats

**Experimental animals:** Male SD rats, about 220 g, 6-8 weeks old, 6 rats/compound, purchased from Chengdu Ddossy Experimental Animals Co., Ltd.

**Experimental design:** on the day of the test, 6 SD rats/compound were randomly grouped according to their body weights. The animals were fasted with water available for 12 to 14 h one day before the administration and were fed 4 h after the administration.

Table 4. Administration information

| Group | Number | Administration information | | | | | |
|---|---|---|---|---|---|---|---|
| | Male | Test compound | Administrati on dosage (mg/kg) | Administrat ion concentrati on (mg/mL) | Administra tion volume (mL/kg) | Collected samples | Mode of administratio n |
| G1 | 3 | Compound | 1 | 0.2 | 5 | Plasma | Intravenous |

(continued)

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| | | of the present invention | | | | | administration |
| G2 | 3 | | 7.5 | 0.75 | 10 | Plasma | Intragastric administration |
| G3 | 3 | Control compound A | 2.5 | 0.5 | 5 | Plasma | Intravenous administration |
| G4 | 3 | | 10 | 1 | 10 | Plasma | Intragastric administration |
| Note: solvent for intravenous administration: 5% DMA + 5% Solutol + 90% Saline; vehicle for intragastric administration: 5% DMSO+95% (20% SBE-β-CD in saline)<br>(DMA: dimethylacetamide; Solutol: polyethylene glycol-15-hydroxystearate; Saline: physiological saline; DMSO: dimethyl sulfoxide; SBE-β-CD: sulfobutyl-β-cyclodextrin) | | | | | | | |

Before and after administration, 0.10 mL of blood was taken from the orbit under isoflurane anesthesia and placed in an EDTAK2 centrifuge tube for centrifugation at 5000 rpm at 4°C for 10 min, followed by plasma collection. The blood collection time points for the intravenous administration group and intragastric administration group were: 0, 5 min, 15 min, 30 min, 1 h, 2 h, 4 h, 6 h, 8 h and 24 h. Before analysis and detection, all samples were stored at -80°C, and the samples were quantitatively analyzed by LC-MS/MS.

Table 5

| Compound No. | IV: 1 mg/kg | PO: 7.5 mg/kg | |
|---|---|---|---|
| | CL ($mL \cdot kg^{-1} \cdot min^{-1}$) | AUC ($h \cdot ng \cdot mL^{-1}$) | F |
| Compound 21-2 | 17.2 | 2264 | 31.2% |
| Compound 32-2 | 20.2 | / | / |
| Compound 35-2 | 8.76 | 3084 | / |
| Compound 36-2 | 8.84 | 3172 | / |
| Control compound A* | 45.8 | 900 | 23.9% |
| *Control compound A iv 2.5 mg/kg; PO: 10 mg/kg<br>Conclusion: The compounds of the present invention, such as the compounds of the Examples, have good oral absorption performance and/or lower clearance rate in rats. | | | |

7. Pharmacokinetic test in beagle dogs

**Experimental animals:** male beagle dogs, about 8-11 kg, 5-6 beagle dogs/compound, purchased from Beijing Marshall Biotechnology Co., Ltd.

**Experimental method:** on the day of the test, 5-6 beagle dogs/compound were randomly grouped according to their body weights. The animals were fasted with water available for 12 to 14 h one day before the administration and were fed 4 h after the administration.

Table 6 Administration information

| Group | Number | Administration information | | | | | |
|---|---|---|---|---|---|---|---|
| | Male | Test compound | Administra tion dosage (mg/kg) | Administra tion concentrati on (mg/mL) | Administra tion volume (mL/kg) | Collected samples | Mode of administratio n |
| G1 | 2 or 3 | Test compound | 1 | 0.5 | 2 | Plasma | Intravenous administratio n |
| G2 | 3 | | 5 | 1 | 5 | Plasma | Intragastric administratio n |
| Note: solvent for intravenous administration: 5% DMA + 5% Solutol + 90% Saline; vehicle for intragastric administration: 5% DMSO+95% (20% SBE-β-CD in saline)<br>(DMA: dimethylacetamide; Solutol: polyethylene glycol-15-hydroxystearate; Saline: physiological saline; DMSO: dimethyl sulfoxide; SBE-β-CD: sulfobutyl-β-cyclodextrin) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for groups G1 and G2 (the intravenous group and intragastric administration group) were: 0 min, 5 min, 15 min, 30 min, 1 h, 2 h, 4 h, 6 h, 8 h, 10 h, 12 h, 24 h, 48 h, and 72 h. The blood collection time points for groups G3 and G4 (the intravenous group and intragastric administration group) were: 0 min, 5 min, 15 min, 30 min, 1 h, 2 h, 4 h, 6 h, 8 h, 10 h, 12 h, and 24 h. Before analysis and detection, all samples were stored at -80°C and the samples were quantitatively analyzed by LC-MS/MS.

**Conclusion:** The compounds of the present invention, such as the compounds of the Examples, have good oral absorption performance in beagle dogs.

## 8. Pharmacokinetic test in monkeys

**Experimental animals:** male cynomolgus monkeys, 3-5 kg, 3-6 years old, 4-6 monkeys/compound. purchased from Suzhou Xishan Biotechnology Inc.

**Experimental method:** on the day of the test, 4-6 monkeys/compound were randomly grouped according to their body weights. The animals were fasted but with water available for 14 to 18 h one day before the administration and were fed 4 h after the administration.

Table 7. Administration information

| Group | Number | Administration information | | | | | |
|---|---|---|---|---|---|---|---|
| | Male | Test compound | Administra tion dosage (mg/kg) | Administra tion concentrati on (mg/mL) | Administra tion volume (mL/kg) | Collected samples | Mode of administration |
| G1 | 2 | Compound | 1 | 1 | 1 | Plasma | Intravenous administration |
| G2 | 2 | | 10 | 2 | 5 | Plasma | Intragastric administration |
| G3 | 3 | Compound | 1 | 1 | 1 | Plasma | Intravenous administration |
| G4 | 3 | Compound | 10 | 2 | 5 | Plasma | Intragastric administration |
| Note: solvent for intravenous administration: 5% DMA + 5% Solutol + 90% Saline; vehicle for intragastric administration: 5% DMSO+95% (20% SBE-β-CD in saline)<br>(DMA: dimethylacetamide; Solutol: polyethylene glycol-15-hydroxystearate; Saline: physiological saline; DMSO: dimethyl sulfoxide; SBE-β-CD: sulfobutyl-β-cyclodextrin)<br>*Dosage is calculated based on free base. | | | | | | | |

Before and after the administration, 1.0 mL of blood was taken from the limb veins and placed in an EDTAK2 centrifuge tube. Centrifugation was performed at 5000 rpm at 4°C for 10 min, and the plasma was collected. The blood collection time points for the intravenous group and intragastric administration group were: 0 min, 5 min, 15 min, 30 min, 1 h, 2 h, 4 h, 6 h, 8 h, 10 h, 12 h, and 24 h. Before analysis and detection, all samples were stored at -80°C, and the samples were quantitatively analyzed by LC-MS/MS.

Conclusion: The compounds of the present invention, such as the compounds of the Examples, have good oral absorption performance in monkeys.

**9. Test for hERG potassium ion channel**

Experimental platform: electrophysiological manual patch-clamp system

Cell line: Chinese hamster ovary (CHO) cell line stably expressing hERG potassium ion channel

Experimental method: in CHO (Chinese Hamster Ovary) cells stably expressing hERG potassium channel, the whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current (I hERG) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

Data processing: data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition } \% = [1 - (I / Io)] \times 100\%$$

where Inhibition% represents the percentage of inhibition of hERG potassium current by the compound, and I and Io represent the amplitude of hERG potassium current after and before the administration, respectively.

Compound IC50 was calculated using GraphPad Prism 5 software by fitting according to the following equation:

$$Y = \text{Bottom} + (\text{Top-Bottom})/(1+10\text{^}((\text{LogIC}_{50}\text{-X}) \times \text{HillSlope}))$$

Among the equation, X represents the Log value of the tested concentration of the test sample, Y represents the inhibition percentage at the corresponding concentration, and Bottom and Top represent the minimum and maximum inhibition percentage, respectively.

Conclusion: The compounds of the present invention, for example, the compounds of the Examples, has no apparent inhibitory activity against hERG.

**10. Test of stability in liver microsomes**

In this experiment, liver microsomes of three species, including monkey, rat and mouse, were used as in vitro models to evaluate the metabolic stability of the test compound.

At 37°C, 1 μM of the test compound was co-incubated with microsomal protein and coenzyme NADPH. At given time points of the reaction (5 min, 10 min, 20 min, 30 min, and 60 min), the reaction was terminated by adding ice-cold acetonitrile containing an internal standard. The LC-MS/MS method was used to measure the concentration of the test compound in the sample. $T_{1/2}$ was calculated using the natural logarithm (ln) of the residual rate of the drug in the incubation system and the incubation time. In addition, the intrinsic clearance in liver microsomes CLint(mic) and the intrinsic clearance in liver CLint(Liver) were further calculated.

Table 8 Results of stability in liver microsomes

| Test compound | Species | T1/2 (min) | Test compound after 1h incubation Remaining% (T=60min) | Hepatic microsomal intrinsic clearance CLint (mic) (μL/min/mg) | Hepatic intrinsic clearance CLint(liver) (mL/min/kg) |
|---|---|---|---|---|---|
| Compound 21-2 | Monkey | 53.6 | 45.1 | 25.8 | 34.9 |
| Compound 35-2 | | 59.8 | 47.2 | 23.2 | 31.3 |
| Compound 36-2 | | 18.9 | 11.6 | 73.5 | 99.2 |
| Control compound A | | 10.6 | 1.8 | 130 | 176 |
| Compound 21-2 | Rat | 168 | 80.4 | 8.24 | 14.8 |
| Compound 35-2 | | 128 | 74.5 | 10.8 | 19.5 |
| Compound 36-2 | | 165 | 77.3 | 8.38 | 15.1 |
| Control compound A | | 35.7 | 31.9 | 38.8 | 69.9 |
| Compound 21-2 | Mice | 92.8 | 63.7 | 14.9 | 59.1 |
| Compound 35-2 | | >186 | 83.8 | <7.45 | <29.5 |
| Compound 36-2 | | 159 | 80.8 | 8.74 | 34.6 |
| Control compound A | | 49.3 | 42.6 | 28.1 | 111 |
| Conclusion: The compounds of the present invention, such as the compounds of the Examples, have good stability in the liver microsomes of monkeys, mice and rats. | | | | | |

**Claims**

**1.** A compound or a stereoisomer, mesomer/racemate, deuterated product, solvate, prodrug, metabolite, pharmaceutically acceptable salt, or co-crystal thereof, which compound is selected from a compound of general formula (I), **characterized in that**

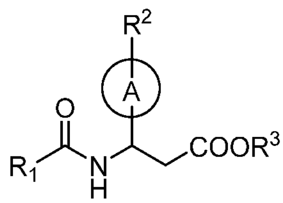
(I)

$R^1$ is selected from $-CHR^{1a}R^{1b}$ or $-NR^{1a}R^{1b}$;
$R^{1a}$ is selected from $C_{1-6}$ alkyl, wherein the alkyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ cycloalkyl;
$R^{1b}$ is selected from $C_{4-10}$ carbocyclic ring or 5- to 10-membered heterocyclic ring, wherein the carbocyclic ring or heterocyclic ring is optionally substituted with 0 to 4 $R^b$, and the heterocyclic ring contains 1 to 4 heteroatoms selected from O, S or N;
each $R^b$ is independently selected from deuterium, halogen, OH, =O, cyano, COOH, $NH_2$, $-C_{0-4}$ alkyl-$NHC_{1-6}$ alkyl, $-C_{0-4}$ alkyl-$N(C_{1-6}$ alkyl$)_2$, $C_{1-6}$ alkyl, $C_{2-6}$ alkynyl, $C_{1-6}$ alkoxy, $-C_{0-4}$ alkyl-$C_{3-10}$ carbocyclic ring, $-C_{0-4}$ alkyl-3- to 10-membered heterocyclic ring or $R^{ba}$, wherein the alkyl, alkynyl, alkoxy, carbocyclic ring or heterocyclic ring is optionally substituted with 0 to 4 substituents selected from deuterium, halogen, OH, =O, cyano, COOH, $CONH_2$, $CONHC_{1-6}$ alkyl, $CON(C_{1-6}$ alkyl$)_2$, $NH_2$, $NHC_{1-6}$ alkyl, $N(C_{1-6}$ alkyl$)_2$, $N(C_{1-6}$ alkyl$)(C_{3-6}$ cycloalkyl$)$, $NH(C_{3-6}$ cycloalkyl$)$, $C_{1-6}$ alkyl, $C_{2-6}$ alkynyl, $C_{1-6}$ alkoxy, halogen-substituted $C_{1-6}$ alkyl, halogen-substituted $C_{1-6}$ alkoxy, $C_{1-6}$ alkoxyalkyl or $R^k$, and the heterocyclic ring contains 1 to 4 heteroatoms selected from O, S or N;
$R^{ba}$ is selected from $-C_{0-4}$ alkyl-7- to 12-membered heterocyclic ring, $-C_{0-4}$ alkyl-4- to 6-membered heterocyclic ring connected via a carbon atom

, 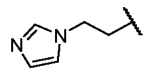,

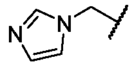, 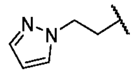, 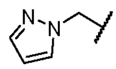 or 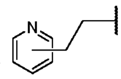,

and the $R^{ba}$ is optionally substituted with 1 to 4 substituents selected from H, halogen, OH, =O, cyano, COOH, $CONH_2$, $CONHC_{1-6}$ alkyl, $CON(C_{1-6}$ alkyl$)_2$, $NH_2$, $NHC_{1-6}$ alkyl, $N(C_{1-6}$ alkyl$)_2$, $C_{1-6}$ alkyl, $C_{2-6}$ alkynyl, $C_{1-6}$ alkoxy, halogen-substituted $C_{1-6}$ alkyl, halogen-substituted $C_{1-6}$ alkoxy, $C_{1-6}$ alkoxyalkyl or $R^k$, wherein the heterocyclic ring contains 1 to 4 heteroatoms selected from O, S or N;

$R^k$ is selected from $-C_{1-4}$ alkyl-$NH_2$, $-C_{1-4}$ alkyl-$NHC_{1-6}$ alkyl, $-C_{1-4}$ alkyl-$N(C_{1-6}$ alkyl$)_2$, $-C_{0-4}$ alkyl-$C_{3-10}$ carbocyclic ring or $-C_{0-4}$ alkyl-3- to 10-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy, halogen-substituted $C_{1-6}$ alkyl, halogen-substituted $C_{1-6}$ alkoxy or $C_{1-6}$ alkoxyalkyl, and the heterocyclic ring contains 1 to 4 heteroatoms selected from O, S or N;

$R^2$ is selected from $C_{1-6}$ alkyl, $C_{6-10}$ aromatic ring, 5- to 10-membered heteroaromatic ring, $C_{3-10}$ carbocyclic ring or 5- to 10-membered heterocyclic ring, and the $R^2$ is optionally substituted with 0 to 4 $R^{2a}$, wherein the heteroaromatic ring or heterocyclic ring contains 1 to 4 heteroatoms selected from O, S or N;

each $R^{2a}$ is independently selected from deuterium, halogen, OH, cyano, =O, $C_{1-6}$ alkyl, $C_{2-6}$ alkenyl, $C_{2-6}$ alkynyl, $C_{1-6}$ alkoxy, $C_{3-6}$ cycloalkyl or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl or $C_{1-6}$ alkoxy, and the heterocyclyl contains 1 to 3 heteroatoms selected from N, O or S;

as an option, $R^{2a}$ and $R^{2a}$ are directly linked to form $C_{4-7}$ carbocyclic ring or 4-to 7-membered heterocyclic ring, wherein the carbocyclic ring or heterocyclic ring is optionally substituted with 1 to 4 substituents selected from deuterium, halogen, OH, cyano, =O, $C_{1-6}$ alkyl, $C_{2-6}$ alkenyl, $C_{2-6}$ alkynyl, $C_{1-6}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;

$R^3$ is selected from H or $C_{1-6}$ alkyl, wherein the alkyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ carbocyclic ring;

ring A is selected from $C_{8-10}$ fused carbocyclic ring, and the ring A is optionally substituted with 0 to 4 $R^{a5}$;

or ring A is selected from

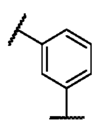,

and the ring A is substituted with one substituent selected from $C_{2-6}$ alkenyl or $C_{2-6}$ alkynyl and optionally substituted with 1 to 3 $R^{a5}$;

$R^{a5}$ is selected from halogen, OH, cyano, $C_{1-6}$ alkyl, $C_{2-6}$ alkenyl, $C_{2-6}$ alkynyl, $C_{1-6}$ alkoxy, $C_{3-6}$ cycloalkyl, or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-6}$ alkyl, $N(C_{1-6}$ alkyl$)_2$, $C_{1-6}$ alkyl, $C_{1-6}$ alkoxy or $C_{3-6}$ cycloalkyl, and the heterocyclyl contains 1 to 3 heteroatoms selected from N, O or S.

**2.** The compound or the stereoisomer, mesomer/racemate, deuterated product, solvate, prodrug, metabolite, pharmaceutically acceptable salt, or co-crystal thereof according to claim 1, characterized in that$R^1$ is selected from

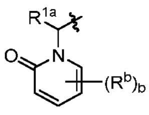, 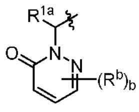, 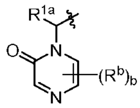, 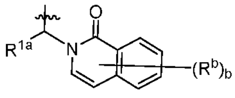, 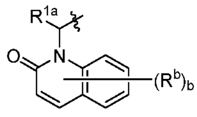, 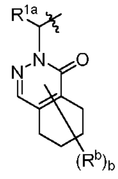,

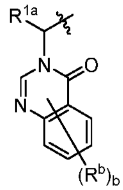, 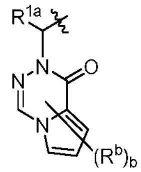, 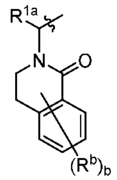, 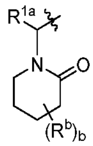, 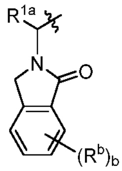, 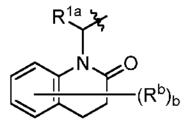, 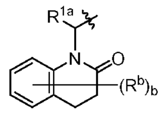,

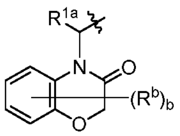, 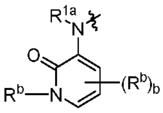, 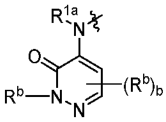, 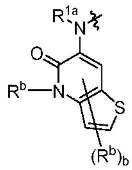, 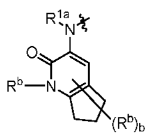, 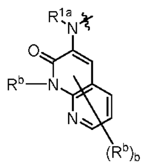,

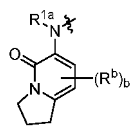, 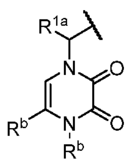, 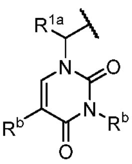 or 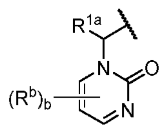;

b is selected from 0, 1, 2 or 3;
each $R^b$ is independently selected from deuterium, halogen, OH, =O, cyano, COOH, $NH_2$, -$C_{0-4}$ alkyl-NH$C_{1-4}$ alkyl, -$C_{0-4}$ alkyl-N($C_{1-4}$ alkyl)$_2$, $C_{1-4}$ alkyl, $C_{2-4}$ alkynyl, $C_{1-4}$ alkoxy, -$C_{0-4}$ alkyl-$C_{3-6}$ carbocyclic ring, -$C_{0-4}$ alkyl-3- to 7-membered heterocyclic ring or $R^{ba}$, wherein the alkyl, alkynyl, alkoxy, carbocyclic ring or heterocyclic ring is optionally substituted with 0 to 4 substituents selected from deuterium, halogen, OH, =O, cyano, COOH, $CONH_2$, CONH$C_{1-4}$ alkyl, CON($C_{1-4}$ alkyl)$_2$, $NH_2$, NH$C_{1-4}$ alkyl, N($C_{1-4}$ alkyl)$_2$, N($C_{1-4}$ alkyl)($C_{3-6}$ cycloalkyl), NH($C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heterocyclic ring contains 1 to 4 heteroatoms selected from O, S or N;
$R^{ba}$ is selected from -$C_{0-2}$ alkyl-7- to 8-membered monocyclic heterocycloalkyl, -$C_{0-2}$ alkyl-7- to 11-membered spiro heterocycloalkyl, -$C_{0-2}$ alkyl-7- to 11-membered bridged heterocycloalkyl, -$C_{0-2}$ alkyl-4- to 6-membered monocyclic heterocycloalkyl connected via a carbon atom,

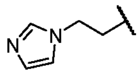, 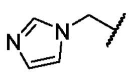,

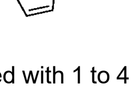, 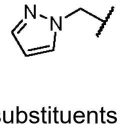 or 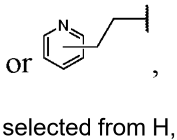,

and the $R^{ba}$ is optionally substituted with 1 to 4 substituents selected from H, halogen, OH, =O, cyano, COOH, $CONH_2$, CONH$C_{1-4}$ alkyl, CON($C_{1-4}$ alkyl)$_2$, $NH_2$, NH$C_{1-4}$ alkyl, N($C_{1-4}$ alkyl)$_2$, $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, wherein the heterocycloalkyl contains 1 to 4 heteroatoms selected from O, S or N;
$R^k$ is selected from -$C_{1-2}$ alkyl-$NH_2$, -$C_{1-2}$ alkyl-NH$C_{1-4}$ alkyl, -$C_{1-2}$ alkyl-N($C_{1-4}$ alkyl)$_2$, -$C_{0-2}$ alkyl-$C_{3-6}$ carbocyclic ring or -$C_{0-2}$ alkyl-3- to 6-membered heterocyclic ring, wherein the alkyl, carbocyclic ring or heterocyclic ring is optionally substituted with 1 to 4 substituents selected from deuterium, halogen, OH, =O, cyano, COOH, $NH_2$, NH$C_{1-4}$ alkyl, N($C_{1-4}$ alkyl)$_2$, $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl, and the heterocyclic ring contains 1 to 4 heteroatoms selected from O, S or N;

$R^2$ is selected from $C_{1-4}$ alkyl, benzene ring, naphthalene ring, 5- to 6-membered heteroaromatic ring, 9- to 10-membered heteroaromatic ring, $C_{3-10}$ non-aromatic carbocyclic ring, 5- to 10-membered non-aromatic heterocyclic ring, benzo $C_{4-6}$ carbocyclyl or benzo 4- to 6-membered heterocyclyl, and the $R_2$ is optionally substituted with 0 to 4 $R^{2a}$, wherein the heteroaromatic ring or heterocyclic ring contains 1 to 4 heteroatoms selected from O, S or N;

each $R^{2a}$ is independently selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocyclyl, wherein the alkyl, alkenyl, alkynyl, alkoxy, cycloalkyl, or heterocyclyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl or $C_{1-4}$ alkoxy, and the heterocyclyl contains 1 to 3 heteroatoms selected from N, O or S;

as an option, $R^{2a}$ and $R^{2a}$ are directly linked to form $C_{4-7}$ carbocyclic ring or 4-to 7-membered heterocyclic ring, wherein the carbocyclic ring or heterocyclic ring is optionally substituted with 1 to 4 substituents selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;

$R^3$ is selected from H or $C_{1-4}$ alkyl, wherein the alkyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, $C_{1-4}$ alkoxy or $C_{3-6}$ carbocyclic ring;

ring A is selected from benzo $C_{4-6}$ carbocyclic ring, and the ring A is optionally substituted with 0 to 4 $R^{a5}$; or ring A is selected from

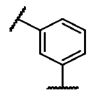,

and the ring A is substituted with one substituent selected from $C_{2-4}$ alkenyl or $C_{2-4}$ alkynyl and optionally substituted with 1 to 3 substituents selected from $R^{a5}$;

$R^{a5}$ is selected from halogen, OH, cyano, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocyclyl, wherein the alkyl, alkenyl, alkynyl, alkoxy, cycloalkyl or heterocyclyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, halogen-substituted $C_{1-4}$ alkyl, cyano-substituted $C_{1-4}$ alkyl, $C_{1-4}$ alkoxy or $C_{3-6}$ cycloalkyl, and the heterocyclyl contains 1 to 3 heteroatoms selected from N, O or S.

**3.** The compound or the stereoisomer, mesomer/racemate, deuterated product, solvate, prodrug, metabolite, pharmaceutically acceptable salt, or co-crystal thereof according to claim 2, **characterized in that**

each $R^b$ is independently selected from deuterium, halogen, OH, =O, cyano, $C_{1-4}$ alkyl, $C_{2-4}$ alkynyl, $C_{1-4}$ alkoxy, phenyl, 5- to 6-membered heteroaryl, - $CH_2NHC_{1-4}$ alkyl, $-CH_2N(C_{1-4}$ alkyl$)_2$, $-CH_2CH_2-NHC_{1-4}$ alkyl, $-CH_2CH_2-N(C_{1-4}$ alkyl$)_2$, phenyl, 5- to 6-membered heteroaryl, $C_{3-6}$ cycloalkyl, 3- to 7-membered heterocycloalkyl, $-CH_2$-phenyl, $-CH_2$-5- to 6-membered heteroaryl, $-CH_2-C_{3-6}$ cycloalkyl, $-CH_2$-3- to 7-membered heterocycloalkyl, $-CH_2CH_2$-phenyl, $-CH_2CH_2$-5- to 6-membered heteroaryl, $-CH_2CH_2-C_{3-6}$ cycloalkyl, $-CH_2CH_2$-3- to 7-membered heterocycloalkyl or $R^{ba}$, wherein the $CH_2$, alkyl, alkynyl, phenyl, heteroaryl, cycloalkyl or heterocycloalkyl is optionally substituted with 0 to 4 substituents selected from deuterium, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heteroaryl or heterocycloalkyl contains 1 to 4 heteroatoms selected from O, S or N;

$R^{ba}$ is selected from 7- to 8-membered monocyclic heterocycloalkyl, 7- to 11-membered spiro heterocycloalkyl, 7- to 11-membered bridged heterocycloalkyl, 4-to 6-membered monocyclic heterocycloalkyl connected via a carbon atom, $-CH_2$-7-to 8-membered monocyclic heterocycloalkyl, $-CH_2$-7- to 11-membered spiro heterocycloalkyl, $-CH_2$-7- to 11-membered bridged heterocycloalkyl, $-CH_2$-4- to 6-membered monocyclic heterocycloalkyl connected via a carbon atom, $-CH_2CH_2$-7-to 8-membered monocyclic heterocycloalkyl, $-CH_2CH_2$-7- to 11-membered spiro heterocycloalkyl, $-CH_2CH_2$-7- to 11-membered bridged heterocycloalkyl, - $CH_2CH_2$-4- to 6-membered monocyclic heterocycloalkyl connected via a carbon atom,

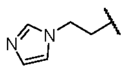, 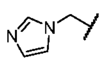, 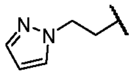, 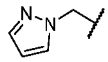 or 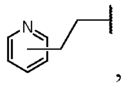,

and the $R^{ba}$ is optionally substituted with 1 to 4 substituents selected from H, halogen, OH, =O, cyano, COOH, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, wherein the heterocycloalkyl contains 1 to 4 heteroatoms selected from O, S or N;

$R^k$ is selected from -$C_{1-4}$ alkyl-$NH_2$, -$C_{1-4}$ alkyl-$NHC_{1-4}$ alkyl, -$C_{1-4}$ alkyl-$N(C_{1-4}$ alkyl$)_2$, -$C_{0-4}$ alkyl-$C_{3-6}$ carbocyclic ring or -$C_{0-4}$ alkyl-3- to 6-membered heterocyclic ring, wherein the alkyl, carbocyclic ring or heterocyclic ring is optionally substituted with 1 to 4 substituents selected from deuterium, halogen, OH, =O, cyano, COOH, $NH_2$, $NHC_{1-4}$ alkyl, $N(C_{1-4}$ alkyl$)_2$, $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl, and the heterocyclic ring contains 1 to 4 heteroatoms selected from O, S or N;

$R^2$ is selected from benzene ring, naphthalene ring, 5- to 6-membered heteroaromatic ring, 9- to 10-membered heteroaromatic ring, $C_{3-6}$ cycloalkyl, 3- to 7-membered heterocycloalkyl, benzo $C_{4-6}$ carbocyclyl or benzo 4- to 6-membered heterocyclyl, and the $R^2$ is optionally substituted with 0 to 4 $R^{2a}$, wherein the heteroaromatic ring or heterocycloalkyl contains 1 to 4 heteroatoms selected from O, S or N;

each $R^{2a}$ is independently selected from deuterium, halogen, OH, cyano, =O, $C_{1-4}$ alkyl, $C_{2-4}$ alkenyl, $C_{2-4}$ alkynyl, $C_{1-4}$ alkoxy or $C_{3-6}$ cycloalkyl, wherein the alkyl, alkenyl, alkynyl, alkoxy or cycloalkyl is optionally substituted with 0 to 4 substituents selected from deuterium, halogen, OH, =O, cyano, $C_{1-4}$ alkyl or $C_{1-4}$ alkoxy;

as an option, $R^{2a}$ and $R^{2a}$ are directly linked to form 4-membered carbocyclic ring, 5-membered carbocyclic ring, 6-membered carbocyclic ring, 4-membered heterocyclic ring, 5-membered heterocyclic ring or 6-membered heterocyclic ring, wherein the carbocyclic ring or heterocyclic ring is optionally substituted with 1 to 4 substituents selected from deuterium, halogen, OH, cyano, =O, $C_{1-4}$ alkyl, $C_{2-4}$ alkenyl, $C_{2-4}$ alkynyl, $C_{1-4}$ alkoxy, $C_{3-6}$ cycloalkyl or 3- to 7-membered heterocycloalkyl, and the alkyl, alkenyl, alkynyl, alkoxy, cycloalkyl or heterocycloalkyl is optionally substituted with 1 to 4 substituents selected from deuterium, halogen, OH or cyano;

$R^3$ is selected from H or $C_{1-4}$ alkyl, wherein the alkyl is optionally substituted with 0 to 4 substituents selected from deuterium, halogen, $C_{1-4}$ alkyl, $C_{1-4}$ alkoxy or benzene ring;

ring A is selected from

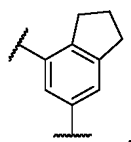,

and the ring A is optionally substituted with 0 to 4 $R^{a5}$;

or ring A is selected from

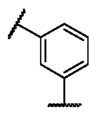,

and the ring A is substituted with one substituent selected from $C_{2-4}$ alkenyl or $C_{2-4}$ alkynyl and optionally substituted with 1 to 3 substituents selected from $R^{a5}$;

$R^{a5}$ is selected from halogen, OH, cyano, $C_{1-4}$ alkyl, $C_{2-4}$ alkynyl, $C_{1-4}$ alkoxy or $C_{3-6}$ cycloalkyl, wherein the alkyl, alkynyl, alkoxy, cycloalkyl or heterocyclyl is optionally substituted with 0 to 4 substituents selected from deuterium, halogen, OH, =O, cyano, $C_{1-4}$ alkyl, $C_{1-4}$ alkoxy or $C_{3-6}$ cycloalkyl.

**4.** The compound or the stereoisomer, mesomer/racemate, deuterated product, solvate, prodrug, metabolite, pharmaceutically acceptable salt, or co-crystal thereof according to claim 3, characterized in that$R^{1a}$ is selected from methyl, ethyl, propyl, butyl, isobutyl, sec-butyl, tert-butyl, -$CH_2$-cyclopropyl or - $CH_2$-cyclobutyl;

each $R^b$ is independently selected from deuterium, F, Cl, Br, I, OH, =O, cyano or $R^{ba}$, or each $R^b$ is independently selected from one of the following substituted or unsubstituted groups: methyl, ethyl, ethynyl, methoxy, ethoxy, cyclopropyl, cyclobutyl, cyclopentyl, cyclohexyl, azetidinyl, azolidinyl, azinanyl, oxetanyl, oxolanyl, oxanyl, morpholinyl, phenyl, pyridine, -$CH_2NH(CH_2CH_3)$, - $CH_2N(CH_2CH_3)_2$, -$CH_2CH_2NH(CH_3)$, -$CH_2CH_2N(CH_3)_2$,

$-CH_2CH_2NH(CH_2CH_3)$, $-CH_2CH_2N(CH_2CH_3)_2$, $-CH_2CH_2N(CH_3)(CH_2CH_3)$, $-CH_2$-cyclopropyl, $-CH_2$-cyclobutyl, $-CH_2$-cyclopentyl, $-CH_2$-cyclohexyl, $-CH_2$-azetidinyl, $-CH_2$-azolidinyl, $-CH_2$-azinanyl, $-CH_2$-oxetanyl, $-CH_2$-oxolanyl, $-CH_2$-oxanyl, $-CH_2$-morpholinyl, $-CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, $-CH_2CH_2$-oxetanyl, $-CH_2CH_2$-oxolanyl, $-CH_2CH_2$-oxanyl, $-CH_2CH_2$-morpholinyl, $-CH_2$-phenyl, $-CH_2$-pyridyl, $-CH_2CH_2$-phenyl or $-CH_2CH_2$-pyridyl, which, when substituted, is substituted with 0 to 4 substituents selected from deuterium, halogen, OH, =O, cyano, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$;

$R^{ba}$ is selected from one of the following optionally substituted groups:

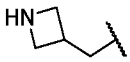, 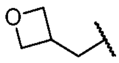, 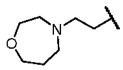, 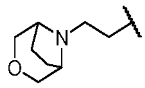, 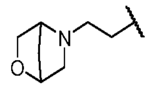, 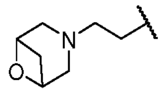, 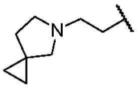,

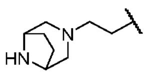, 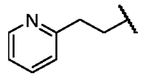, 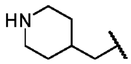, 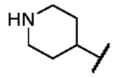, 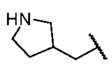, 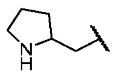, 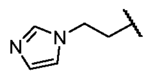,

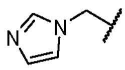, 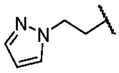, 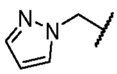, 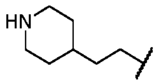, 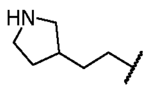 or 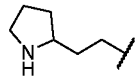,

which, when substituted, is optionally substituted with 1 to 4 substituents selected from deuterium, halogen, OH, =O, cyano, $CONH_2$, $CONHC_{1-4}$ alkyl, $CON(C_{1-4}$ alkyl$)_2$, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$;

$R^k$ is selected from $-CH_2N(CH_3)_2$, $-CH_2$-cyclopropyl, $-CH_2$-cyclobutyl, cyclopropyl, cyclobutyl, cyclopentyl, cyclohexyl, azetidinyl, azolidinyl, azinanyl or oxetanyl, wherein the cyclopropyl, cyclobutyl, cyclopentyl, cyclohexyl, azetidinyl, azolidinyl or azinanyl is optionally substituted with 1 to 4 substituents selected from deuterium, halogen, OH, =O, cyano, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{1-4}$ alkoxy, halogen-substituted $C_{1-4}$ alkyl, halogen-substituted $C_{1-4}$ alkoxy or $C_{1-4}$ alkoxyalkyl;

$R^2$ is selected from benzene ring, pyridyl, pyridonyl, pyrazinyl, pyrimidyl, thienyl, thiazolyl, furyl, oxazolyl, pyrrolyl, pyrazolyl, imidazolyl, cyclopropyl, cyclobutyl, cyclopentyl, cyclohexyl, azetidinyl, azolidinyl, azinanyl, oxetanyl, oxolanyl, oxanyl, morpholinyl,

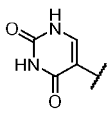, 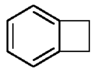, 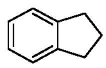, 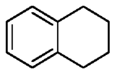, 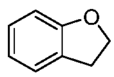,

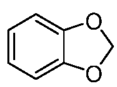, 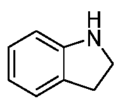, 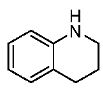, 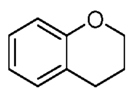, 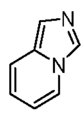 or 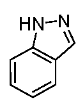,

and the $R^2$ is optionally substituted with 0 to 4 $R^{2a}$;

each $R^{2a}$ is independently selected from deuterium, F, Cl, Br, I, OH, =O, cyano, methyl, ethyl, ethenyl, ethynyl, methoxy, ethoxy, cyclopropyl or cyclobutyl, wherein the methyl, ethyl, ethenyl, ethynyl, methoxy, ethoxy, cyclopropyl or cyclobutyl is optionally substituted with 0 to 4 substituents selected from deuterium, halogen, OH, =O, cyano, $C_{1-4}$ alkyl or $C_{1-4}$ alkoxy;

$R^3$ is selected from H, methyl, ethyl, propyl, butyl, isobutyl, sec-butyl, tert-butyl or benzyl;

ring A is selected from

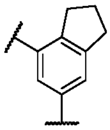,

and the ring A is optionally substituted with 0 to 4 $R^{a5}$;
or ring A is selected from

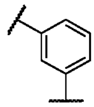,

and the ring A is substituted with one substituent selected from ethenyl, ethynyl, propynyl or propargyl and optionally substituted with 1 to 3 substituents selected from $R^{a5}$;
each $R^{a5}$ is independently selected from F, Cl, Br, I, OH, CN, ethynyl, propynyl, propargyl, methyl, ethyl, cyclopropyl, methoxy or ethoxy, wherein the ethynyl, propynyl, propargyl, methyl, ethyl, cyclopropyl, methoxy or ethoxy is optionally substituted with 1 to 4 substituents selected from deuterium, halogen, OH, =O, cyano, $C_{1-4}$ alkyl, $C_{1-4}$ alkoxy or $C_{3-6}$ cycloalkyl.

**5.** The compound or the stereoisomer, mesomer/racemate, deuterated product, solvate, prodrug, metabolite, pharmaceutically acceptable salt, or co-crystal thereof according to claim 4, characterized in thatR$^1$ is selected from

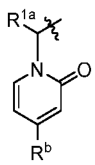, 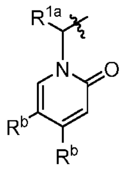, 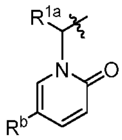, 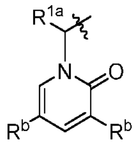, 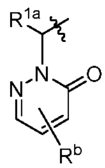, 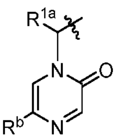, 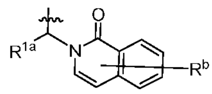,

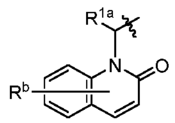, 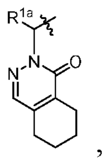, 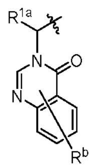, 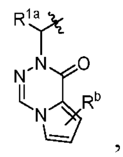, 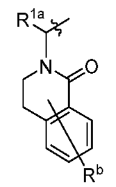, 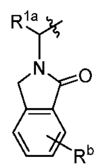, 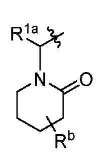, 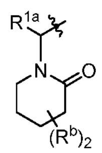,

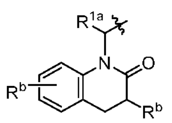, 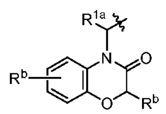, 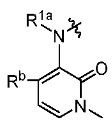, 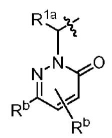, 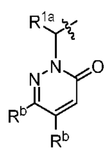, 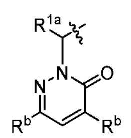, 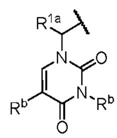,

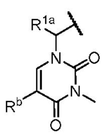, 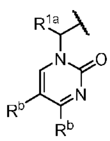, 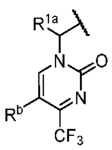, 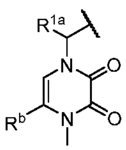, 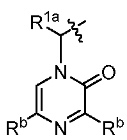, 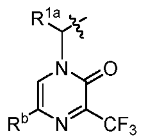, 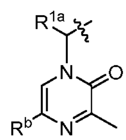, 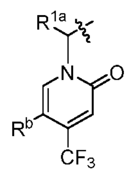,

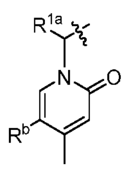, 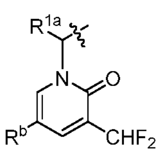 or 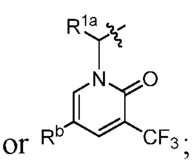;

each $R^b$ is independently selected from deuterium, F, Cl, Br, OH, cyano or $R^{ba}$, or each $R^b$ is independently selected from one of the following substituted or unsubstituted groups: methyl, ethyl, ethynyl, methoxy, ethoxy, cyclopropyl, cyclobutyl, cyclopentyl, cyclohexyl, azetidinyl, azolidinyl, azinanyl, oxetanyl, oxolanyl, oxanyl, morpholinyl, phenyl, pyridyl, $-CH_2NH(CH_2CH_3)$, - $CH_2N(CH_2CH_3)_2$, $-CH_2CH_2NH(CH_3)$, $-CH_2CH_2N(CH_3)_2$, $-CH_2CH_2NH(CH_2CH_3)$, - $CH_2CH_2N(CH_2CH_3)_2$, $-CH_2CH_2N(CH_3)(CH_2CH_3)$, $-CH_2$-cyclopropyl, $-CH_2$-cyclobutyl, $-CH_2$-cyclopentyl, $-CH_2$-cyclohexyl, $-CH_2$-azetidinyl, $-CH_2$-azolidinyl, $-CH_2$-azinanyl, $-CH_2$-oxetanyl, $-CH_2$-oxolanyl, $-CH_2$-oxanyl, $-CH_2$-morpholinyl, - $CH_2CH_2$-cyclopropyl, $-CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, - $CH_2CH_2$-oxetanyl, $-CH_2CH_2$-oxolanyl, $-CH_2CH_2$-oxanyl, $-CH_2CH_2$-morpholinyl, - $CH_2$-phenyl, $-CH_2$-pyridyl, $-CH_2CH_2$-phenyl, $-CH_2CH_2$-pyridyl or

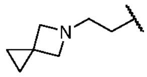,

which, when substituted, is substituted with 0 to 4 substituents selected from deuterium, F, Cl, Br, OH, =O, cyano, $CONH_2$, $CONHCH_3$, $CON(CH_3)_2$, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;

$R^{ba}$ is selected from one of the following optionally substituted groups:

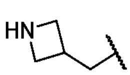, 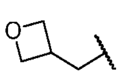, 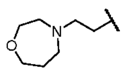, 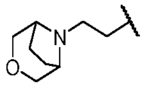, 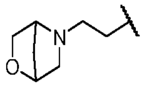, 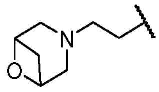, 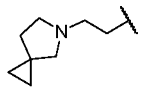,

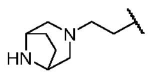, 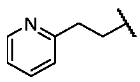, 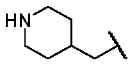, 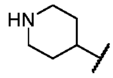, 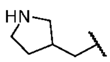, 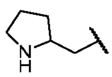, 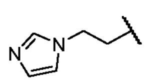,

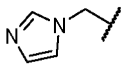, 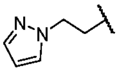, 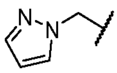, 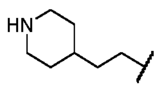, 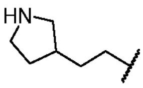 or 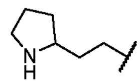,

which, when substituted, is optionally substituted with 1 to 4 substituents selected from deuterium, F, Cl, Br, OH, =O, cyano, $CONH_2$, $CONHCH_3$, $CON(CH_3)_2$, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$;

$R^k$ is selected from $-CH_2N(CH_3)_2$, $-CH_2$-cyclopropyl, $-CH_2$-cyclobutyl, cyclopropyl, cyclobutyl, cyclopentyl, cyclohexyl, azetidinyl, azolidinyl, azinanyl or oxetanyl, wherein the cyclopropyl, cyclobutyl, cyclopentyl, cyclohexyl, azetidinyl, azolidinyl, azinanyl or oxetanyl is optionally substituted with 1 to 4 substituents selected from deuterium, F, Cl, Br, OH, =O, cyano, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, methoxy, ethoxy, methoxymethyl, ethoxymethyl or methoxyethyl;

$R^2$ is selected from phenyl, pyridyl, pyridonyl, azolidinyl, morpholinyl or

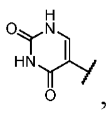,

and the $R^2$ is optionally substituted with 0 to 4 substituents selected from deuterium, F, Cl, Br, OH, $CF_3$, cyano, methyl, ethyl, methoxy, ethoxy, cyclopropyl or cyclobutyl;

or $R^2$ is selected from

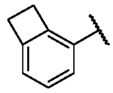, 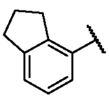, 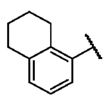, 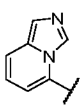, 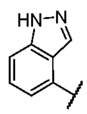, 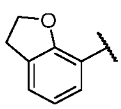,

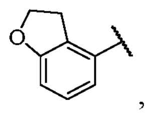, 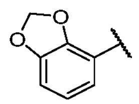 or 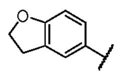,

and the $R^2$ is optionally substituted with 1 to 4 substituents selected from deuterium, $CD_3$, $-OCD_3$, F, Cl, Br, OH, $CF_3$, cyano, methyl, ethyl, methoxy, ethoxy, cyclopropyl or cyclobutyl;

ring A is selected from 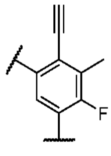, 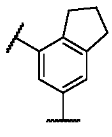, 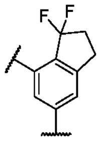, 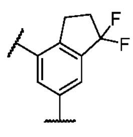, 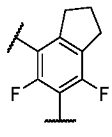,

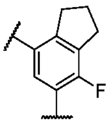, 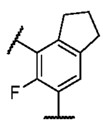, 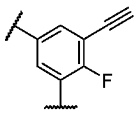, 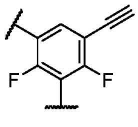, 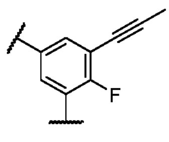, 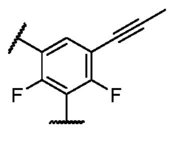, 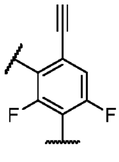,

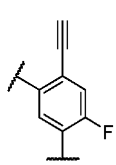, 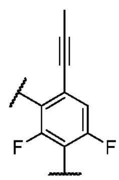 or 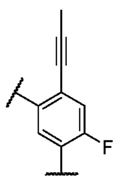.

**6.** The compound or the stereoisomer, mesomer/racemate, deuterated product, solvate, prodrug, metabolite, pharmaceutically acceptable salt, or co-crystal thereof according to claim 5, **characterized in that**

$R^1$ is selected from

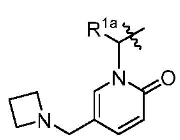, 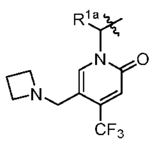, 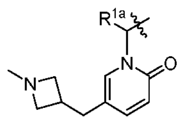, 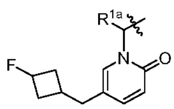,

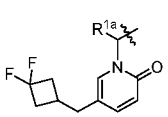, 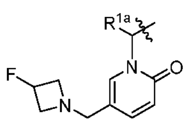, 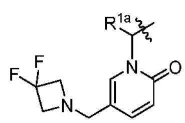, 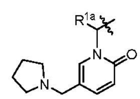, 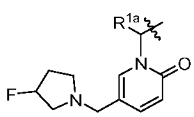,

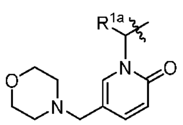, 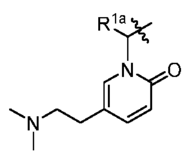, 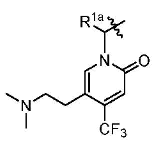, 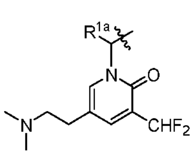, 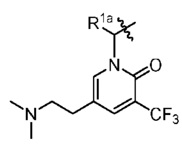,

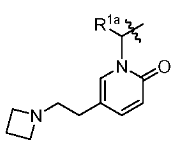, 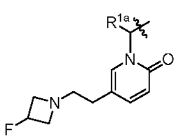, 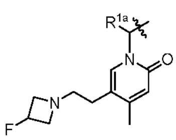, 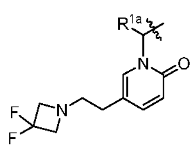, 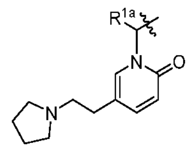,

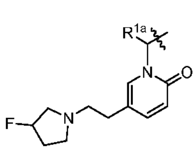, 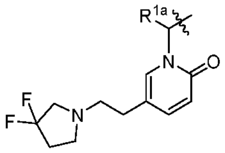, 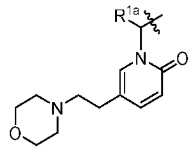, 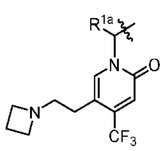, 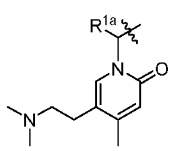,

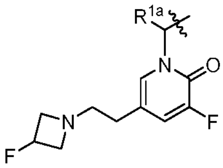, 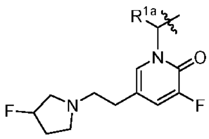 or 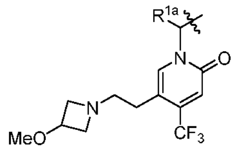;

or $R^1$ is selected from

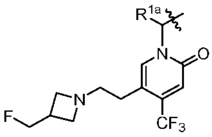 or 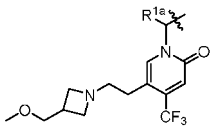;

or $R^1$ is selected from

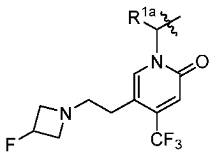 or 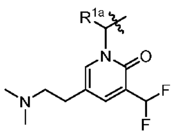;

or $R^1$ is selected from

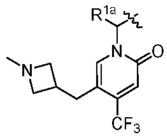, 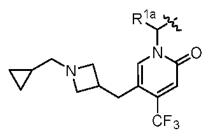, 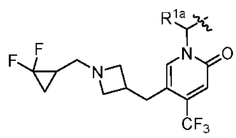,

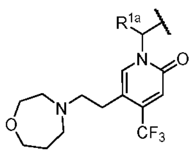, 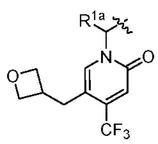, 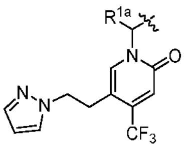, 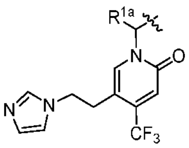, 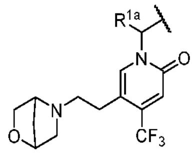,

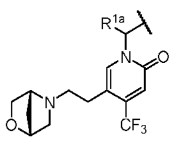, 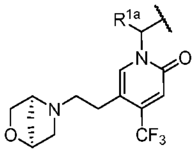, 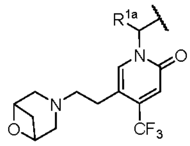, 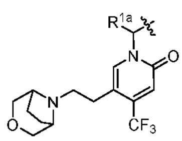, 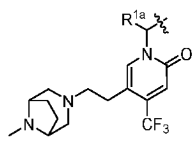,

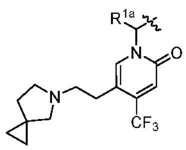, 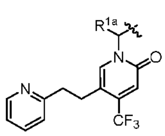, 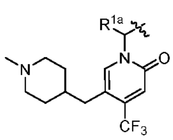, 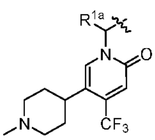, 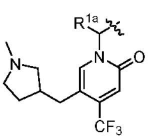, 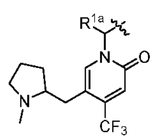,

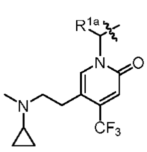, 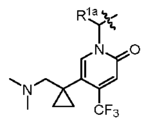, 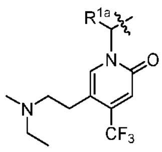, 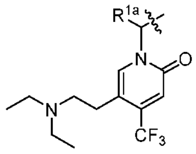, 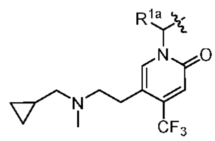

or

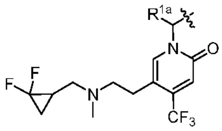;

or $R^1$ is selected from

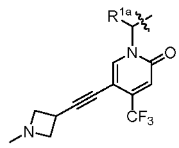, 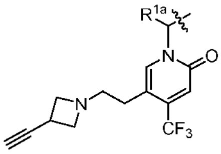, 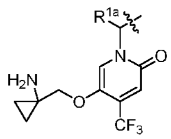,

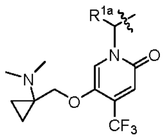, 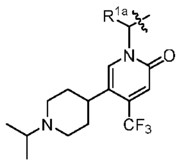, 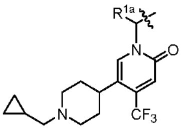, 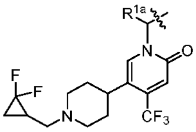, 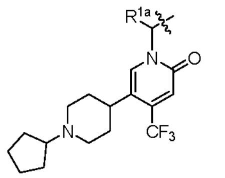

or

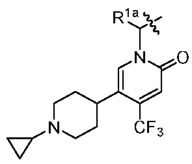;

or $R^1$ is selected from

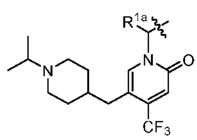, 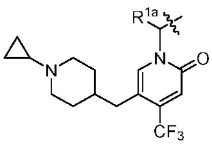, 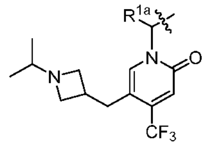,

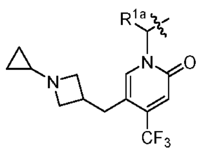, 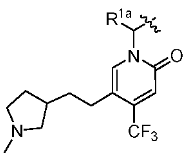 or 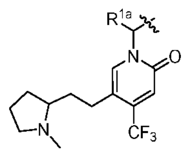;

or $R^1$ is selected from

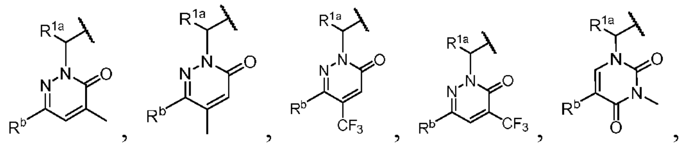
, , , , ,

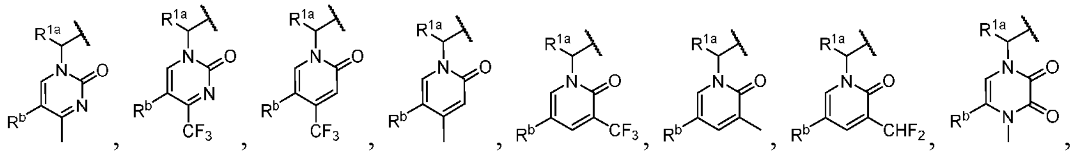
, , , , , , , ,

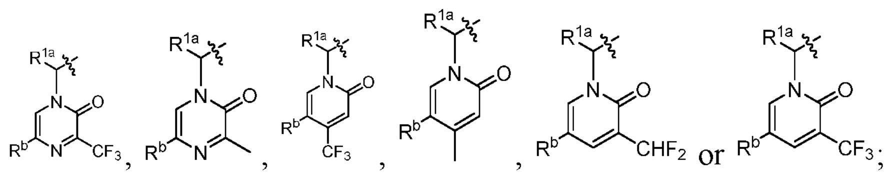
, , , , or ;

$R^2$ is selected from

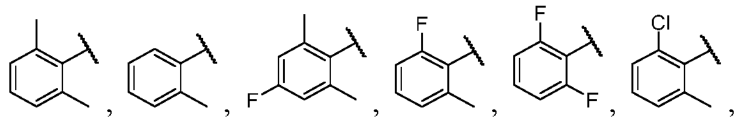
, , , , , ,

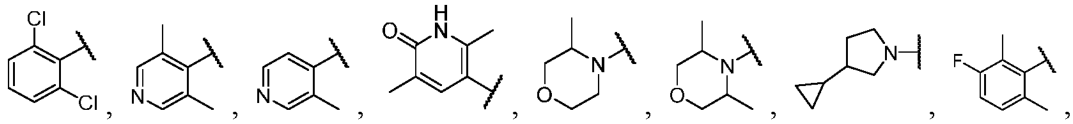
, , , , , , , ,

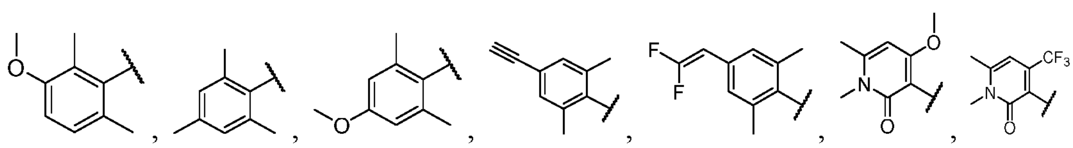
, , , , , , ,

or

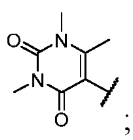
;

or $R^2$ is selected from

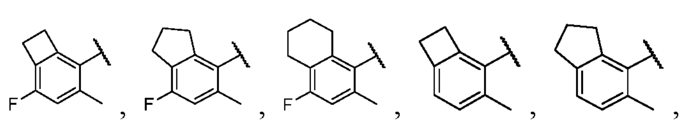
, , , , ,

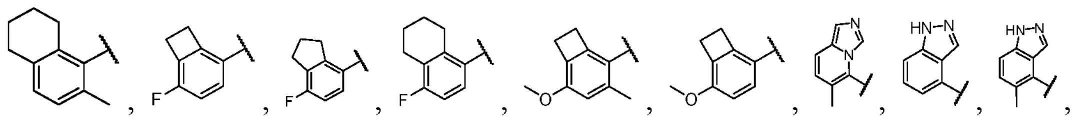
, , , , , , , , ,

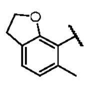, 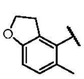, 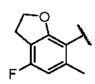 or 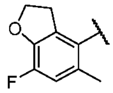;

or $R^2$ is selected from

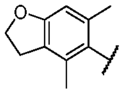, 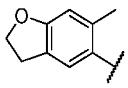, 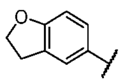, 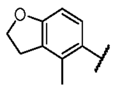 or 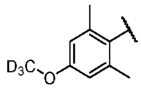;

$R^{1a}$ is selected from

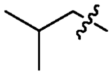, 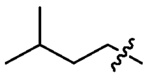 or 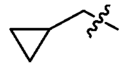.

**7.** The compound or the stereoisomer, mesomer/racemate, deuterated product, solvate, prodrug, metabolite, pharmaceutically acceptable salt, or co-crystal thereof according to claim 1, **characterized in that** the compound is selected from a compound of general formula (I-a), (I-b), (I-a-1) or (I-b-1),

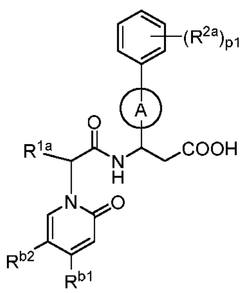
(I-a)
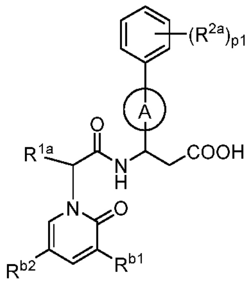
(I-b)

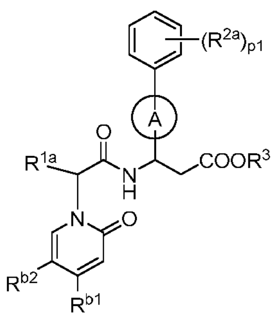
(I-a-1)
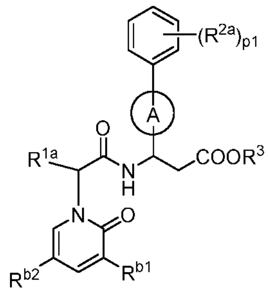
(I-b-1)

the definitions of ring A and $R^3$ are the same as those in any one of claims 2 to 6;
p1 is selected from 0, 1, 2, 3 or 4;
$R^{2a}$ is selected from deuterium, halogen, OH, CN, $C_{1-4}$ alkyl, $C_{1-4}$ alkoxy, $C_{3-6}$ cycloalkyl or 4- to 6-membered heterocycloalkyl, wherein the alkyl, alkoxy, cycloalkyl or heterocycloalkyl is optionally substituted with 0 to 4 substituents selected from deuterium, halogen, OH, CN, $C_{1-4}$ alkyl or $C_{1-4}$ alkoxy, and the heterocycloalkyl contains 1 to 4 heteroatoms selected from O, S or N;
$R^{1a}$ is selected from

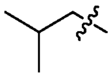, 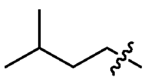 or 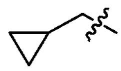;

$R^{b1}$ is selected from H, $C_{1-4}$ alkyl or $C_{3-6}$ cycloalkyl, wherein the alkyl or cycloalkyl is optionally substituted with 0 to 4 substituents selected from deuterium, halogen, OH, CN, $NHC_{1-4}$ alkyl, $N(C_{1-4}$ alkyl$)_2$, $C_{1-4}$ alkyl or $C_{1-4}$ alkoxy;
$R^{b2}$ is selected from H, $R^{ba}$, or one of the following substituted or unsubstituted groups: $C_{1-4}$ alkyl, $C_{2-4}$ alkynyl,

$C_{1-4}$ alkoxy, phenyl, 5- to 6-membered heteroaryl, $-CH_2CH_2$-phenyl, $-CH_2CH_2$-5- to 6-membered heteroaryl, -$CH_2CH_2$-$NHC_{1-4}$ alkyl, $-CH_2CH_2-N(C_{1-4}$ alkyl$)_2$, $-CH_2CH_2-C_{3-6}$ cycloalkyl or - $CH_2CH_2$-3- to 7-membered heterocycloalkyl, wherein the $CH_2$, alkyl, cycloalkyl or heterocycloalkyl is optionally substituted with 0 to 4 substituents selected from deuterium, halogen, OH, =O, cyano, COOH, $NH_2$, $NHC_{1-4}$ alkyl, $N(C_{1-4}$ alkyl$)_2$, $N(C_{1-4}$ alkyl$)(C_{3-6}$ cycloalkyl), $NH(C_{3-6}$ cycloalkyl), $C_{1-4}$ alkyl, $C_{2-4}$ alkynyl, $C_{1-4}$ alkoxy, halogen-substituted $C_{1-4}$ alkyl, halogen-substituted $C_{1-4}$ alkoxy, $C_{1-4}$ alkoxyalkyl or $R^k$, and the heteroaryl or heterocycloalkyl contains 1 to 4 heteroatoms selected from O, S or N;

the definitions of $R^{ba}$ and $R^k$ are the same as those in any one of claims 2 to 6.

**8.** The compound or the stereoisomer, mesomer/racemate, deuterated product, solvate, prodrug, metabolite, pharmaceutically acceptable salt, or co-crystal thereof according to claim 7, characterized in that$R^{2a}$ is selected from deuterium, $-CD_3$, $-OCD_3$, F, Cl, Br, I, OH, cyano, methyl, ethyl, methoxy or ethoxy;

$R^{b1}$ is selected from H, F, $CH_2F$, $CHF_2$, $CF_3$, methyl or $-CH_2CH_2N(CH_3)_2$;
$R^{b2}$ is selected from H, $R^{ba}$, or one of the following substituted or unsubstituted groups: methyl, ethyl, ethynyl, methoxy, ethoxy, $-CH_2NH(CH_2CH_3)$, $-CH_2N(CH_2CH_3)_2$, $-CH_2CH_2NH(CH_3)$, $-CH_2CH_2N(CH_3)_2$, $-CH_2CH_2NH(CH_2CH_3)$, $-CH_2CH_2N(CH_2CH_3)_2$, $-CH_2CH_2N(CH_3)(CH_2CH_3)$, $-CH_2CH_2$-cyclopropyl, - $CH_2CH_2$-cyclobutyl, $-CH_2CH_2$-cyclopentyl, $-CH_2CH_2$-cyclohexyl, $-CH_2CH_2$-azetidinyl, $-CH_2CH_2$-azolidinyl, $-CH_2CH_2$-azinanyl, $-CH_2CH_2$-oxetanyl, $-CH_2CH_2$-oxolanyl, $-CH_2CH_2$-oxanyl, $-CH_2CH_2$-morpholinyl, $-CH_2CH_2$-phenyl or $-CH_2CH_2$-pyridyl, which, when substituted, is substituted with 1, 2 or 3 substituents selected from H, F, Cl, Br, OH, =O, cyano, $NH_2$, $NHCH_3$, $N(CH_3)_2$, $N(CH_3)$(cyclopropyl), $NHCH_2CH_3$, $N(CH_2CH_3)_2$, $CH_2F$, $CHF_2$, $CF_3$, methyl, ethyl, isopropyl, ethynyl, methoxy, ethoxy, methoxymethyl, ethoxymethyl, methoxyethyl or $R^k$.

**9.** The compound or the stereoisomer, mesomer/racemate, deuterated product, solvate, prodrug, metabolite, pharmaceutically acceptable salt, or co-crystal thereof according to claim 1, **characterized in that** the compound is selected from a compound of general formula (I-f), (I-g), (I-f-1), (I-g-1), (I-h), (I-i), (I-h-1) or (I-i-1),

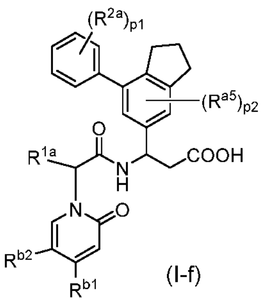
(I-f)
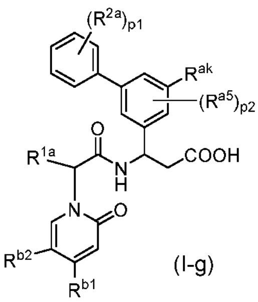
(I-g)
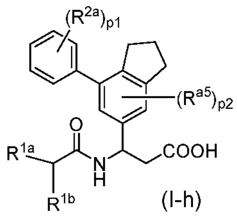
(I-h)
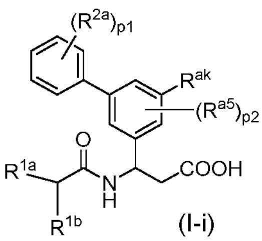
(I-i)

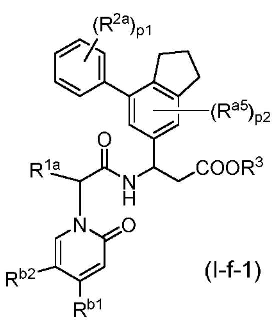
(I-f-1)
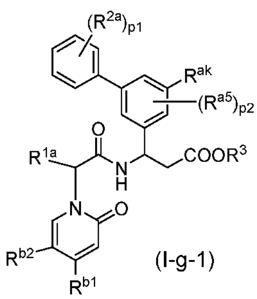
(I-g-1)
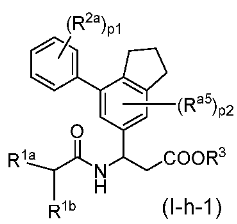
(I-h-1)
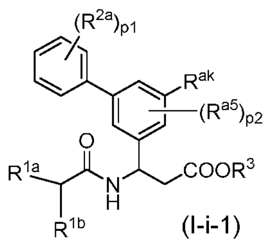
(I-i-1)

$R^{1b}$ is selected from

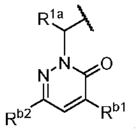 , 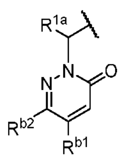 , 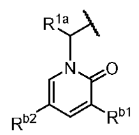 , 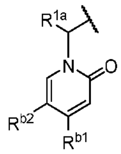 , 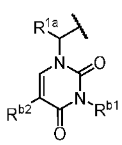 ,

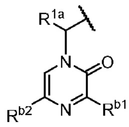 or 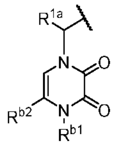 ;

wherein p2 is selected from 0, 1 or 2;
$R^{ak}$ is selected from $C_{2-4}$ alkynyl;
the definition of $R^{a5}$ is the same as that in any one of claims 1 to 4;
the definitions of the remaining groups are the same as those in claim 7 or 8.

**10.** The compound or the stereoisomer, mesomer/racemate, deuterated product, solvate, prodrug, metabolite, pharmaceutically acceptable salt, or co-crystal thereof according to claim 9, characterized in that$R^{ak}$ is selected from ethynyl, propynyl or propargyl;
$R^{a5}$ is selected from F, Cl, Br, I, OH, cyano, methyl, ethyl, methoxy, ethoxy or ethynyl.

**11.** The compound or the stereoisomer, mesomer/racemate, deuterated product, solvate, prodrug, metabolite, pharmaceutically acceptable salt, or co-crystal thereof according to claim 1, **characterized in that** the compound is selected from one of the structures shown in Table E-1, Table E-3, Table E-4 or Table E-5.

**12.** A pharmaceutical composition, **characterized by** comprising the compound or the stereoisomer, mesomer/racemate, deuterated product, solvate, prodrug, metabolite, pharmaceutically acceptable salt, or co-crystal thereof according to any one of claims 1-11, and a pharmaceutically acceptable carrier, wherein preferably, the pharmaceutical composition comprises 1-1500 mg of the compound or the stereoisomer, mesomer/racemate, deuterated product, solvate, prodrug, metabolite, pharmaceutically acceptable salt, or co-crystal thereof according to any one of claims 1-11.

**13.** Use of the compound or the stereoisomer, mesomer/racemate, deuterated product, solvate, prodrug, metabolite, pharmaceutically acceptable salt, or co-crystal thereof according to any one of claims 1-12, or the pharmaceutical composition according to claim 12 in the preparation of a drug for the treatment of diseases related to $\alpha4\beta7$ activity or expression.

**14.** The use according to claim 13, **characterized in that** the diseases are selected from inflammatory bowel diseases.

**15.** A method for treating a disease in a mammal, **characterized in that** the method comprises administering to a subject a therapeutically effective amount of the compound or the stereoisomer, mesomer/racemate, deuterated product, solvate, prodrug, metabolite, pharmaceutically acceptable salt, or co-crystal thereof according to any one of claims 1-11 or the pharmaceutical composition according to claim 12, wherein the therapeutically effective amount is preferably 1-1500 mg, and the disease is preferably inflammatory bowel disease.

## INTERNATIONAL SEARCH REPORT

International application No.
**PCT/CN2023/117732**

**A. CLASSIFICATION OF SUBJECT MATTER**

C07D213/64(2006.01)i; C07D401/02(2006.01)i; C07D205/02(2006.01)i; A61K31/397(2006.01)i; A61K31/4412(2006.01)i; A61P29/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNABS, CNTXT, ENTXT, DWPI, 万方, WANFANG, Web of Science, 百度学术, Baidu Scholar, Registry, Caplus, Marpat, 海思科医药, 张晨, 何平, 王乐, 宣兆利, 魏琦, 唐平明, 何海清, 钟亚军, 余彦, 李瑶, 倪佳, 严庞科, 炎症性肠病, 肠道炎症疾病, 溃疡性结肠炎, 克罗恩病, 整联蛋白, 整合素, 丙酸, α4β7, inflammatory bowel disease, IBD, ulcerative colitis, Crohn's disease, integrin, propan+

**C. DOCUMENTS CONSIDERED TO BE RELEVANT**

| Category* | Citation of document, with indication, where appropriate, of the relevant passages | Relevant to claim No. |
|---|---|---|
| PX | WO 2023125182 A1 (HAISCO PHARMACEUTICAL GROUP CO., LTD.) 06 July 2023 (2023-07-06)<br>description, pages 52-53, 56-86, table E2, page 1 paragraphs 3-5, page 86 paragraphs 1-2 | 1-14 |
| A | WO 2021076890 A1 (MORPHIC THERAPEUTIC, INC.) 22 April 2021 (2021-04-22)<br>entire document | 1-14 |
| A | CN 110914241 A (AVIARA PHARMACEUTICALS, INC.) 24 March 2020 (2020-03-24)<br>entire document | 1-14 |
| A | CN 112312910 A (MORPHIC THERAPEUTIC, INC.) 02 February 2021 (2021-02-02)<br>entire document | 1-14 |
| A | CN 1412181 A (TEXAS BIOTECHNOLOGY CORP.) 23 April 2003 (2003-04-23)<br>entire document | 1-14 |
| A | CN 112969504 A (GILEAD SCIENCES, INC.) 15 June 2021 (2021-06-15)<br>entire document | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 December 2023** | **06 December 2023** |

| Name and mailing address of the ISA/CN | Authorized officer |
|---|---|
| **China National Intellectual Property Administration (ISA/CN)<br>China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/117732**

**C. DOCUMENTS CONSIDERED TO BE RELEVANT**

| Category* | Citation of document, with indication, where appropriate, of the relevant passages | Relevant to claim No. |
|---|---|---|
| A | US 2004063955 A1 (ENCYSIVE PHARMACEUTICALS, INC.) 01 April 2004 (2004-04-01) entire document | 1-14 |
| A | US 2004235848 A1 (AJINOMOTO KK) 25 November 2004 (2004-11-25) entire document | 1-14 |
| A | US 2003199692 A1 (TEXAS BIOTECHNOLOGY CORP.) 23 October 2003 (2003-10-23) entire document | 1-14 |

Form PCT/ISA/210 (second sheet) (July 2022)

INTERNATIONAL SEARCH REPORT

International application No.
**PCT/CN2023/117732**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

The solution of claim 15 relates to a method for treatment of the human or animal body, which falls within subject matter for which a search is not required; therefore, said claim does not comply with PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/117732**

| Patent document cited in search report | | | Publication date (day/month/year) | Patent family member(s) | | | Publication date (day/month/year) |
|---|---|---|---|---|---|---|---|
| WO | 2023125182 | A1 | 06 July 2023 | TW | 202330524 | A | 01 August 2023 |
| WO | 2021076890 | A1 | 22 April 2021 | TW | 202222793 | A | 16 June 2022 |
| | | | | TWI | 791304 | B | 01 February 2023 |
| | | | | TW | 202126624 | A | 16 July 2021 |
| | | | | TWI | 775182 | B | 21 August 2022 |
| | | | | JP | 2023036958 | A | 14 March 2023 |
| | | | | AU | 2020366435 | A1 | 21 April 2022 |
| | | | | AU | 2021361031 | A1 | 08 June 2023 |
| | | | | WO | 2021076902 | A1 | 22 April 2021 |
| | | | | PE | 20221829 | A1 | 29 November 2022 |
| | | | | TW | 202244040 | A | 16 November 2022 |
| | | | | TWI | 802477 | B | 11 May 2023 |
| | | | | TW | 202315868 | A | 16 April 2023 |
| | | | | DOP | 2022000081 | A | 31 July 2022 |
| | | | | CR | 20220205 | A | 22 July 2022 |
| | | | | EP | 4045039 | A1 | 24 August 2022 |
| | | | | UY | 38926 | A | 31 May 2021 |
| | | | | BR | 112022007284 | A2 | 05 July 2022 |
| | | | | ECSP | 22038978 | A | 30 June 2022 |
| | | | | JP | 2022542184 | A | 29 September 2022 |
| | | | | JP | 2023500437 | A | 06 January 2023 |
| | | | | KR | 20230088376 | A | 19 June 2023 |
| | | | | US | 11104661 | B1 | 31 August 2021 |
| | | | | MX | 2022004406 | A | 25 August 2022 |
| | | | | CA | 3154269 | A1 | 22 April 2021 |
| | | | | CL | 2022000959 | A1 | 28 April 2023 |
| | | | | US | 11370773 | B1 | 28 June 2022 |
| | | | | WO | 2022081983 | A1 | 21 April 2022 |
| | | | | TW | 202332675 | A | 16 August 2023 |
| | | | | CU | 20220027 | A7 | 12 December 2022 |
| | | | | AR | 120244 | A1 | 09 February 2022 |
| | | | | EP | 4228634 | A1 | 23 August 2023 |
| | | | | WO | 2021076890 | A4 | 10 June 2021 |
| | | | | CO | 2022005759 | A2 | 20 May 2022 |
| | | | | JP | 2022548809 | A | 22 November 2022 |
| | | | | JP | 7209116 | B2 | 19 January 2023 |
| | | | | IL | 292296 | A | 01 June 2022 |
| | | | | KR | 20220102669 | A | 20 July 2022 |
| CN | 110914241 | A | 24 March 2020 | AU | 2018266966 | A1 | 19 December 2019 |
| | | | | AU | 2018266966 | B2 | 01 September 2022 |
| | | | | EP | 3615515 | A2 | 04 March 2020 |
| | | | | EP | 3615515 | A4 | 02 September 2020 |
| | | | | US | 2019225602 | A1 | 25 July 2019 |
| | | | | US | 10494367 | B2 | 03 December 2019 |
| | | | | CA | 3061363 | A1 | 15 November 2018 |
| | | | | WO | 2018209363 | A2 | 15 November 2018 |
| | | | | WO | 2018209363 | A3 | 03 January 2019 |
| | | | | US | 2018312498 | A1 | 01 November 2018 |
| | | | | US | 10246451 | B2 | 02 April 2019 |
| CN | 112312910 | A | 02 February 2021 | WO | 2019200202 | A1 | 17 October 2019 |

Form PCT/ISA/210 (patent family annex) (July 2022)

**INTERNATIONAL SEARCH REPORT**
**Information on patent family members**

International application No.
**PCT/CN2023/117732**

| Patent document cited in search report | Publication date (day/month/year) | Patent family member(s) | Publication date (day/month/year) |
|---|---|---|---|
| | | US 2019315692 A1 | 17 October 2019 |
| | | US 10759756 B2 | 01 September 2020 |
| | | US 2020385352 A1 | 10 December 2020 |
| | | US 11174228 B2 | 16 November 2021 |
| | | EP 3773573 A1 | 17 February 2021 |
| | | EP 3773573 A4 | 06 April 2022 |
| | | TW 202003466 A | 16 January 2020 |
| | | MA 52249 A | 05 May 2021 |
| | | AR 114489 A1 | 09 September 2020 |
| | | JP 2021531234 A | 18 November 2021 |
| | | JP 7365358 B2 | 19 October 2023 |
| | | US 2022340529 A1 | 27 October 2022 |
| CN 1412181 A | 23 April 2003 | PL 356532 A1 | 22 April 2003 |
| | | PL 223151 B1 | 31 October 2016 |
| | | SI 21096 A1 | 30 June 2003 |
| | | SI 21096 B | 31 May 2012 |
| | | SG 107574 A1 | 29 December 2004 |
| | | SG 161741 A1 | 29 June 2010 |
| | | JP 2003119181 A | 23 April 2003 |
| | | JP 4149172 B2 | 10 September 2008 |
| | | CA 2366800 A1 | 10 April 2003 |
| | | CA 2366800 C | 10 August 2010 |
| CN 112969504 A | 15 June 2021 | CA 3115830 A1 | 07 May 2020 |
| | | CA 3115830 C | 12 September 2023 |
| | | US 2022152014 A1 | 19 May 2022 |
| | | JP 2022513401 A | 07 February 2022 |
| | | JP 7189369 B2 | 13 December 2022 |
| | | US 2020163953 A1 | 28 May 2020 |
| | | US 11179383 B2 | 23 November 2021 |
| | | TW 202323238 A | 16 June 2023 |
| | | AU 2019373245 A1 | 13 May 2021 |
| | | AU 2019373245 B2 | 24 February 2022 |
| | | AU 2019373245 C1 | 27 October 2022 |
| | | EP 3873605 A1 | 08 September 2021 |
| | | WO 2020092401 A1 | 07 May 2020 |
| | | JP 2022140725 A | 27 September 2022 |
| | | TW 202033491 A | 16 September 2020 |
| | | TWI 777100 B | 11 September 2022 |
| | | KR 20210087960 A | 13 July 2021 |
| US 2004063955 A1 | 01 April 2004 | NZ 515252 A | 30 January 2004 |
| | | US 6972296 B2 | 06 December 2005 |
| | | TR 200103179 A2 | 21 June 2002 |
| | | EP 1203766 A2 | 08 May 2002 |
| | | EP 1203766 A3 | 08 December 2004 |
| | | EP 1203766 B1 | 29 February 2012 |
| | | EP 1203766 B8 | 11 April 2012 |
| | | HK 1046275 A1 | 03 January 2003 |
| | | NO 20015394 D0 | 05 November 2001 |
| | | NO 20015394 L | 07 May 2002 |
| | | NO 324496 B1 | 29 October 2007 |

Form PCT/ISA/210 (patent family annex) (July 2022)

**INTERNATIONAL SEARCH REPORT**
**Information on patent family members**

International application No.
**PCT/CN2023/117732**

| Patent document cited in search report | Publication date (day/month/year) | Patent family member(s) | Publication date (day/month/year) |
|---|---|---|---|
| | | IL 146313 A0 | 25 July 2002 |
| | | IL 146313 A | 30 December 2010 |
| | | DK 1203766 T3 | 10 April 2012 |
| | | ES 2379945 T3 | 07 May 2012 |
| | | ATE 547406 T1 | 15 March 2012 |
| | | KR 20020035471 A | 11 May 2002 |
| | | KR 100851766 B1 | 13 August 2008 |
| | | US 2006276476 A1 | 07 December 2006 |
| | | US 7812038 B2 | 12 October 2010 |
| US 2004235848 A1 | 25 November 2004 | AU 2002354224 A1 | 09 July 2003 |
| | | JPWO 2003053926 A1 | 28 April 2005 |
| | | JP 4452899 B2 | 21 April 2010 |
| | | WO 03053926 A1 | 03 July 2003 |
| | | US 7250516 B2 | 31 July 2007 |
| | | EP 1454898 A1 | 08 September 2004 |
| | | EP 1454898 A4 | 13 December 2006 |
| | | US 2008280909 A1 | 13 November 2008 |
| | | US 7884204 B2 | 08 February 2011 |
| US 2003199692 A1 | 23 October 2003 | HK 1048469 A1 | 04 April 2003 |
| | | HK 1048469 B | 14 August 2009 |
| | | ES 2321059 T3 | 02 June 2009 |
| | | ATE 420876 T1 | 15 January 2009 |
| | | DE 60137399 D1 | 05 March 2009 |
| | | US 6723711 B2 | 20 April 2004 |
| | | EP 1213288 A1 | 12 June 2002 |
| | | EP 1213288 B1 | 14 January 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021076890 A1
- WO 2021076902 A


### Non-patent literature cited in the description


- *CHEMICAL ABSTRACTS*, 72287-26-4
- *CHEMICAL ABSTRACTS*, 1310584-14-5
- *CHEMICAL ABSTRACTS*, 95464-05-4